# EUROPEAN PATENT APPLICATION

(11) **EP 2 270 192 A1**
(43) Date of publication of application: **05.01.2011**
(21) Application number: 10172218.9
(22) Date of filing: 25.04.2002
(51) Int. Cl.: C12P 19/28, A61K 35/00, C12N 1/20, C07K 7/64

(54) **Glycopeptide antibiotics**

(30) Priority: 25.04.2001 US 286396 P
(62) Divisional of application: 02764346.9
(71) Applicant: Wyeth Holdings Corporation, Madison, NJ 07940 (US)
(72) Inventor: Abbanat, Darren Robert, Cornwall, NY 12518 (US); Bailey, Arthur Emery, Bethel, CT 06801 (US); Bernan, Valerie Sue, New City, NY 10956 (US); Greenstein, Michael, Suffern, NY 10901 (US); Lotvin, Jason Arnold, Union, NJ 07083 (US); Ruppen, Mark Edward, Garnerville, NY 10953 (US); Sutherland, Alan Gordon, New City, 10956 (US); He, Haiyin, Washington Township, NJ 07675 (US)
(74) Representative: Kingsbury, Oliver William

(57) **Abstract**

The invention provides compounds of formula (I), wherein R¹, R², R³, R⁴, R⁵, R^{6a}, R^{6b}, R^{6c}, R^{6d}, R^{6e} and R⁷ are defined in the specification. These compounds are useful as antibiotic agents.

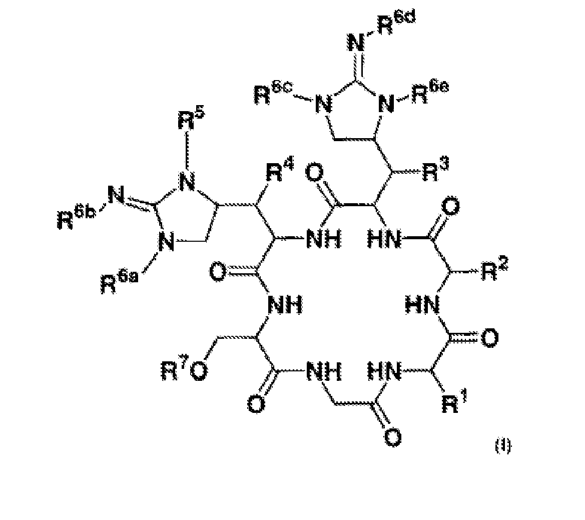

## Description

### FIELD OF THE INVENTION

The present invention relates to novel glycopeptides, which are useful as antibiotics.

### BACKGROUND OF THE INVENTION

New improved antibiotics are continually in demand, for the treatment of human diseases. Antibiotic resistant organisms are continually a problem, particularly in hospitals. Vancomycin has been the last defense. However, especially in hospitals, isolates which are vancomycin resistant are becoming more common. A recent survey found 7.9% of Enterococci in United States hospitals are now vancomycin resistant. "Nosocomial Enterococci Resistant to Vancomycin" Morbidity and Mortality Weekly Report 42(30):597-598(1993). Further resistance of Vancomycin and other antibiotics to *Enterococcus faecium* is reported, Handwergers. et al., Clin. Infect. Dis. 1993(16),750-755. Additional resistance to enterococci is reported, Boyle, JF, Clin. Microbiol. 1993(31),1280-1285. Vancomycin resistance has been reported against *Staphylococcus aureus,* F.A. Waldvogel, The New England Journal of Medicine, 340(7), 1999. Additional reports include: Murry, B. E. The New England Journal of Medicine, 342(10), 710-721(2000) and Y. Cetinkaya et al, Clinical Microbiology Reviews, 13(4) 686-707(2000). Clearly, antibiotic resistance is a growing public health problem. Having new antibiotics available could provide additional options for physicians in treatment regimens.

The search for new antibiotics which exhibit improved antibacterial activity against vancomycin-resistant isolates and having structures which are not derivatives of vancomycin are particularly appealing targets for screening and synthetic efforts. Structural similarity to existing antibiotics could facilitate the emergence of resistance.
The AC98-antibiotic complex isolated as an unseparated mixture of compounds of undetermined structure produced from cultures of *Streptomyces hygroscopicus* (strain NRRL 3085) is described in U.S. Patent. No. 3,495,004. It is an object of this invention to provide a novel family of glycopeptide antibiotics which are shown to possess antibacterial activity, especially against vancomycin resistant bacterial isolates and in particular having chemical structures unlike vancomycin.

### SUMMARY OF THE INVENTION

This invention is concerned with novel glycopeptides which have antibacterial activity; with methods of treating infectious disease in mammals employing these novel glycopeptides; with pharmaceutical preparations containing these glycopeptides and processes for the production of glycopeptides of the invention. More particularly, this invention is concerned with glycopeptides which have enhanced antibacterial activity against vancomycin, penicillin and methicillin resistant strains. Compounds according to the invention comprise compounds of the formula: 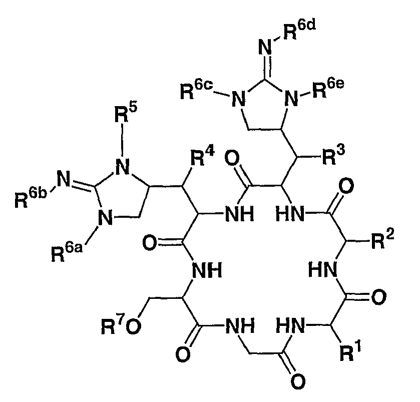 wherein:
R¹ is a moiety selected from: 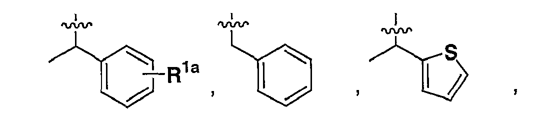 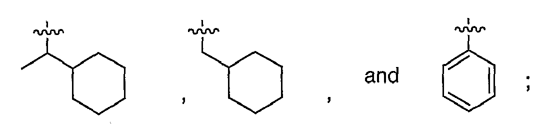
R^{1a} is H or halogen;
R² is a moiety selected from: 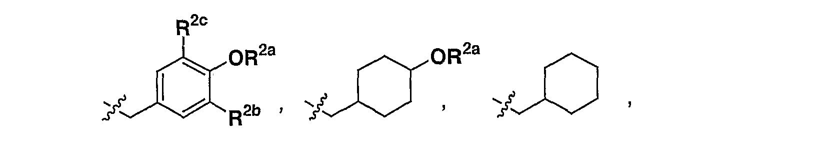 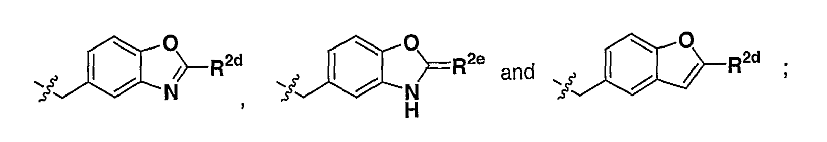
provided when R² is selected from the moieties: 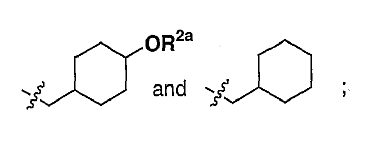
that R¹ is selected from the moieties: 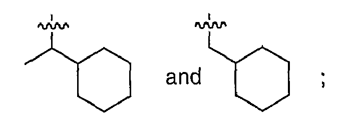
R^{2a} is selected from H, alkyl(C₁-C₂₀), cycloalkyl(C₃-C₂₀), alkenyl(C₃-C₂₀), alkynyl(C₃-C₂₀), the group -C(O)-Y-Z, and a moiety selected from: 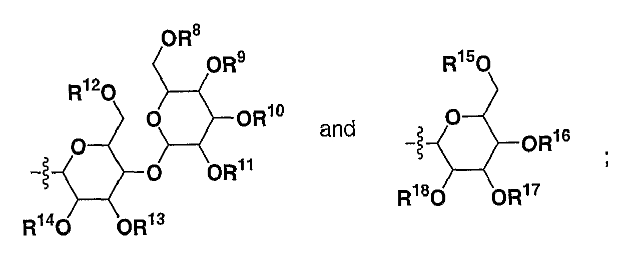
Y is selected from a single bond, -O- and -NR^{8a}-;
Z is selected from alkyl(C₁-C₂₀), cycloalkyl(C₃-C₂₀), alkenyl(C₃-C₂₀), alkynyl(C₃-C₂₀), aryl and heteroaryl;
and when Y is a single bond then Z is also selected from H, alkenyl(C₂-C₂₀) and alkynyl (C₂-C₂₀);
R^{2b} and R^{2c} are independently selected from H, halogen, alkyl(C₁-C₂₀), cycloalkyl(C₃-C₂₀), alkenyl(C₂-C₂₀), alkynyl(C₂-C₂₀), aryl, heteroaryl, -NH₂, -NR^{2f}R^{2g}, and -NO₂, provided when R^{2b} is -NO₂, that R^{2c} must be H;
R^{2d} is selected from alkyl (C₁-C₂₀), alkenyl(C₂-C₂₀), alkynyl(C₂-C₂₀), aryl, heteroaryl, and when R² is 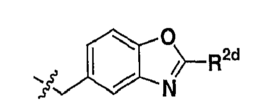
R^{2d} may also be the group L-M, wherein;
L is selected from -NH-, -S-, -SCH₂C(O)-, and a group of the formula: 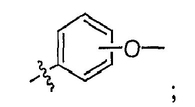
M is selected from alkyl(C₁-C₂₀), cycloalkyl(C₃-C₂₀), alkenyl(C₃-C₂₀) and alkynyl(C₃-C₂₀);
and when:
   a) L is not -S-, M may also be aryl or heteroaryl;
   b) L is -SCH₂C(O)-, M may also be H, alkenyl(C₂-C₂₀) or alkynyl(C₂-C₂₀); or
   c) L is -NH- or a group of the formula: 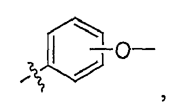
M may also be a moiety of the formula: 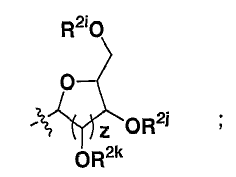
z is an integer of 1 or 2;
R²ⁱ, R^{2j} and R^{2k} are independently selected from H, alkyl(C₁-C₂₀), cycloalkyl(C₃-C₂₀), alkenyl(C₃-C₂₀), alkynyl(C₃-C₂₀), acyl, -Si(alkyl(C₁-C₂₀))₃, -Si(alkyl(C₁-C₂₀))₂(aryl), -Si(alkyl(C₁-C₂₀))(aryl)₂, -Si(aryl)₃, and aroyl;
R^{2e} is S or O;
R^{2f} is selected from H, alkyl(C₁-C₂₀), cycloalkyl(C₃-C₂₀), alkenyl(C₃-C₂₀), alkynyl(C₃-C₂₀), aryl and heteroaryl;
R^{2g} is H, alkyl(C₁-C₂₀), cycloalkyl(C₃-C₂₀), alkenyl(C₃-C₂₀), alkynyl(C₃-C₂₀), aryl and heteroaryl, and the group D-E-G, wherein;
D is selected from -C(O)-, -C(S)- and -S(O)₂-;
E is selected from a single bond, and, when D is -C(O)- or -C(S)-, E is also selected from -0- and -NR^{2h}-;
G is selected from alkyl(C₁-C₂₀), cycloalkyl(C₃-C₂₀), alkenyl(C₃-C₂₀), alkynyl(C₃-C₂₀), aryl and heteroaryl;
and when:
   a) E is a single bond, then G may also be alkenyl(C₂-C₂₀) or alkynyl(C₂-C₂₀); or
   b) D is -C(O)- or -C(S)- and E is a single bond, then G may also be H;
R^{2f} and R^{2g} may optionally when taken together with the nitrogen atom to which each is attached form a monocyclic ring having three to seven atoms independently selected from the elements C, N, 0 and S where said monocyclic ring optionally contains up to three nitrogen atoms, up to two oxygen atoms and up to two sulfur atoms provided said monocyclic ring does not contain -0-0-, -S-S- or -S-O- bonds;
R^{2h} is selected from H, alkyl(C₁-C₂₀), cycloalkyl(C₃-C₂₀), alkenyl(C₃-C₂₀), alkynyl(C₃-C₂₀), aryl and heteroaryl;
R^{2h} and G may optionally when taken together with the nitrogen atom to which each is attached form a monocyclic ring having three to seven atoms independently selected from the elements C, N, O and S where said monocyclic ring optionally contains up to three nitrogen atoms, up to two oxygen atoms and up to two sulfur atoms provided said monocyclic ring does not contain -0-0-, -S-S- or -S-O- bonds;
R³ and R⁴ are independently H, OH, -Si(alkyl(C₁-C₂₀))₃, -Si(alkyl(C₁-C₂₀))₂(aryl), -Si(alkyl(C₁-C₂₀))(aryl)₂, -Si(aryl)₃ or the group -C(O)-Y-Z;
R⁵ is selected from H, alkyl(C₁-C₂₀), cycloalkyl(C₃-C₂₀), alkenyl(C₃-C₂₀), alkynyl(C₃-C₂₀), the group -C(O)-Y-Z and moieties of the formulae; 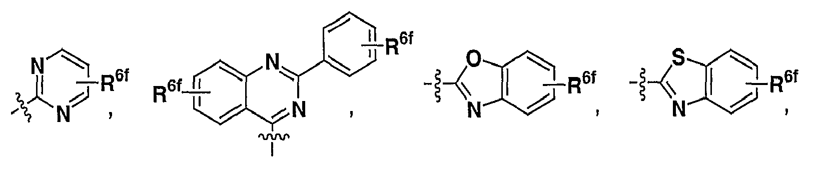 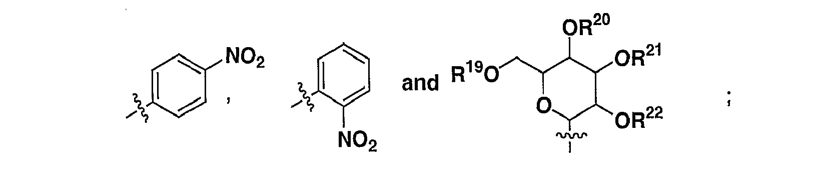
R^{6a}, R^{6b}, R^{6c}, R^{6d} and R^{6e} are independently selected from H, alkyl(C₁-C₂₀), cycloalkyl(C₃-C₂₀), alkenyl(C₃-C₂₀), alkynyl(C₃-C₂₀), the group -C(O)-Y-Z and moieties of the formulae; 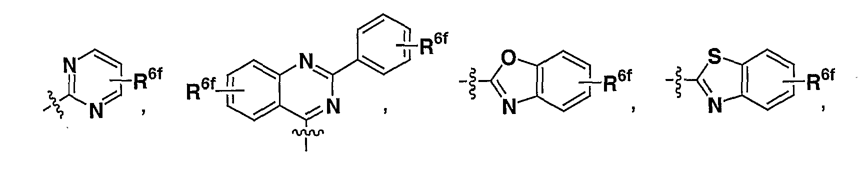 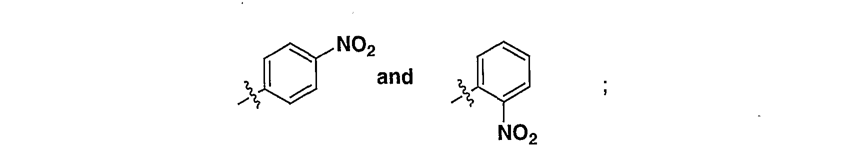
R^{6f} is selected from H, alkyl(C₁-C₂₀), cycloalkyl(C₃-C₂₀), alkenyl(C₃-C₂₀), alkynyl(C₃-C₂₀), aryl, heteroaryl, halogen, hydroxy, alkoxy(C₁-C₂₀), aryloxy, amino, monoalkyl(C₁-C₂₀)amino, dialkyl(C₁-C₂₀)amino, carboxy, carboxyalkyl(C₁-C₂₀), carboxyaryl and carboxyamido;
R⁷ is selected from H, alkyl(C₁-C₂₀), cycloalkyl(C₃-C₂₀), alkenyl(C₃-C₂₀), alkynyl(C₃-C₂₀), -Si(alkyl(C₁-C₂₀))₃, -Si(alkyl(C₁-C₂₀))₂(aryl), -Si(alkyl(C₁-C₂₀))(aryl)₂, -Si(aryl)₃ and the group -C(O)-Y-Z;
R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently selected from H, alkyl(C₁-C₂₀), cycloalkyl(C₃-C₂₀), alkenyl(C₃-C₂₀), alkynyl(C₃-C₂₀), -Si(alkyl(C₁-C₂₀))₃, -Si(alkyl(C₁-C₂₀))₂(aryl), -Si(alkyl(C₁-C₂₀))(aryl)₂, -Si(aryl)₃ and the group -C(O)-Y-Z or, optionally, R⁸ and R⁹, R⁹ and R¹⁰, R¹⁰ and R¹¹, R¹³ and R¹⁴, R¹⁵ and R¹⁶, R¹⁶ and R¹⁷, R¹⁷ and R¹⁸, R¹⁹ and R²⁰, R²⁰ and R²¹ or R²¹ and R²² may independently be taken together forming moieties of the formulae: 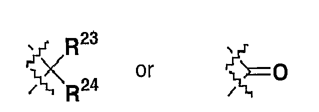 provided when;
   a) R⁸ and R⁹ are so joined, R⁹ and R¹⁰ may not be so joined;
   b) R⁹ and R¹⁰ are so joined, R⁸ and R⁹ and R¹⁰ and R¹¹ may not be so joined;
   c) R¹⁵ and R¹⁶ are so joined, R¹⁶ and R¹⁷ may not be so joined;
   d) R¹⁶ and R¹⁷ are so joined, R¹⁵ and R¹⁶, R¹⁷ and R¹⁸ may not be so joined;
   e) R¹⁹ and R²⁰ are so joined, R²⁰ and R²¹ may not be so joined;
   f) R²⁰ and R²¹ are so joined, R¹⁹ and R²⁰, and R²¹ and R²² may not be so joined;
R^{8a} is selected from H, alkyl(C₁-C₂₀), cycloalkyl(C₃-C₂₀), alkenyl(C₃-C₂₀), alkynyl(C₃-C₂₀), aryl and heteroaryl;
R^{8a} and Z may optionally when taken together with the nitrogen atom to which each is attached form a monocyclic ring having three to seven atoms independently selected from the elements C, N, 0 and S where said monocyclic ring optionally contains up to three nitrogen atoms, up to two oxygen atoms and up to two sulfur atoms provided said monocyclic ring does not contain -0-0-, -S-S- or -S-O- bonds;
R²³ and R²⁴ are independently selected from H, alkyl(C₁-C₂₀), cycloalkyl(C₃-C₂₀), alkenyl(C₂-C₂₀), alkynyl(C₂-C₂₀), aryl, and heteroaryl;
R²³ and R²⁴ may optionally when taken together with the carbon atom to which each is attached form carbocyclic, monocyclic, fused, bridged, spirocyclic or polycyclic rings from three to twenty ring atoms optionally selected from the elements C, N, 0 and S where said monocyclic ring optionally contains up to three nitrogen atoms, up to two oxygen atoms and up to two sulfur atoms provided said monocyclic ring does not contain -O-O-, -S-S- or -S-O- bonds;
provided when:
R¹ is 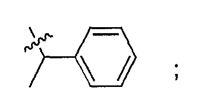
R³ and R⁴ are -OH;
R⁵ is 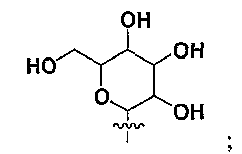 and
R^{2b}, R^{2c}, R^{6a}, R^{6b}, R^{6c}, R^{6d}, R^{6e}, R⁷, are H;
that:
   R^{2a} is not H or moieties selected from the formulae: 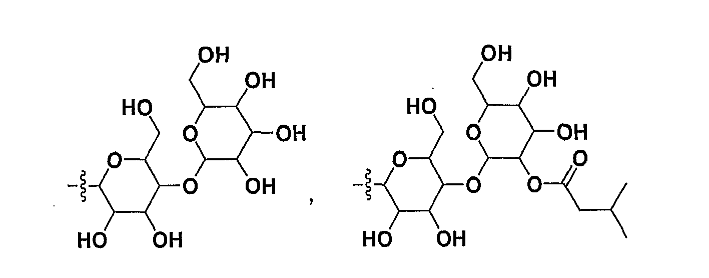 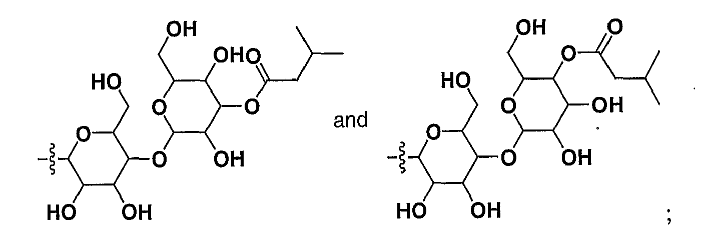 or a pharmaceutically acceptable salt thereof.

Among the preferred groups of compounds of this invention including pharmaceutically acceptable salts thereof are those in the subgroups below, wherein other variables are as defined above:
a)
   R¹ is a moiety selected from the group: 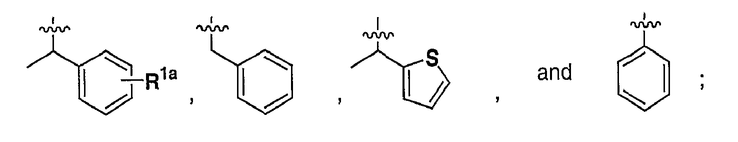
b)
   R¹ is a moiety of the formula: 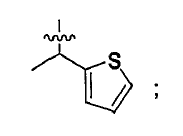
c)
   R¹ is a moiety selected from the group: 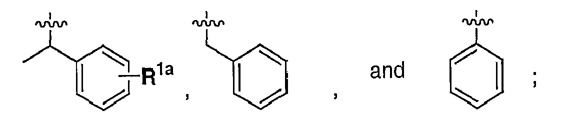
d)
   R¹ is a moiety selected from the group: 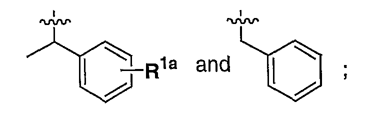 or
e)
   R¹ is a moiety selected from the group:

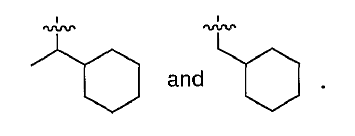

Also among the preferred groups of compounds of this invention including pharmaceutically acceptable salts thereof are those in the subgroups below, wherein other variables are as defined above:
a)
   R² is a moiety of the formula: 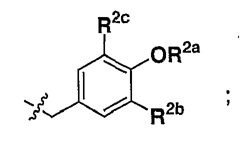
b)
   R² is a moiety of the formula: 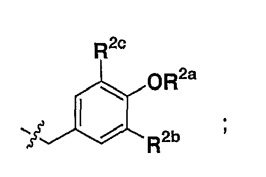
   R^{2b} is selected from -NH₂, and -NR^{2f}R^{2g}; and
   R^{2c} is H;
c)
   R² is a moiety of the formula: 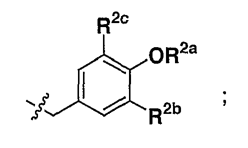
   R^{2a} is H;
   R^{2b} is selected from -NH₂, and -NR^{2f}R^{2g}; and
   R^{2c} is H;
d)
   R² is a moiety of the formula: 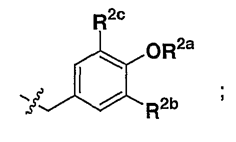 and
   R^{2b} and R^{2c} are independently selected from H, halogen, alkyl(C₁-C₂₀), cycloalkyl(C₃-C₂₀), alkenyl(C₂-C₂₀), alkynyl(C₂-C₂₀), aryl and heteroaryl;
e)
   R² is a moiety of the formula: 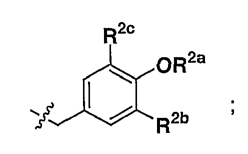 and
   R^{2b} and R^{2c} are independently selected from H and halogen;
f)
   R² is a moiety of the formula: 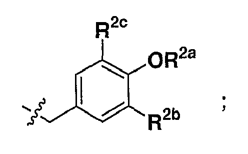 R^{2a} is a moiety of the formula: 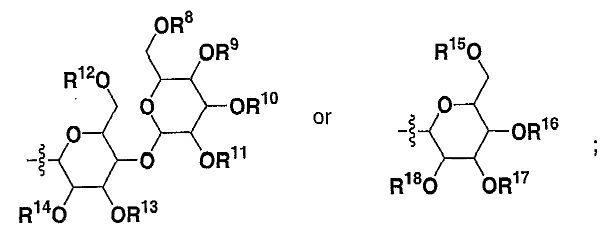 and R^{2b} and R^{2c} are independently selected from H and halogen;
g)
   R² is a moiety selected from the group: 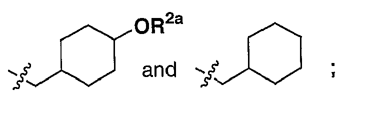
h)
   R² is a moiety of the formula: 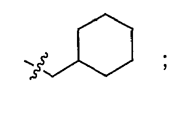 or
i)
   R² is a moiety selected from the group:

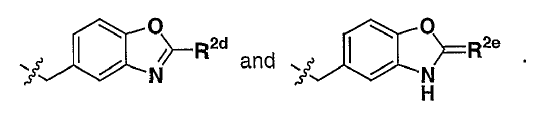

Additionally preferred groups of compounds of this invention including pharmaceutically acceptable salts thereof are those in the subgroups below, wherein other variables are as defined above:
a)
   R³ and R⁴ are independently selected from H and OH; or
b)
   R³ and R⁴ are OH.

Another preferred group of compounds of this invention including pharmaceutically acceptable salts thereof are those in the subgroup below, wherein other variables are as defined above:
R⁵ is selected from H or a moiety of the formula:

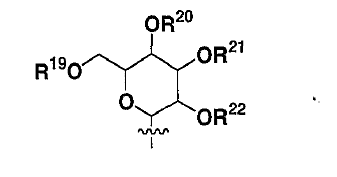

Also among the preferred groups of compounds of this invention including pharmaceutically acceptable salts thereof are those in the subgroups below, wherein other variables are as defined above:
a)
   R^{6a}, R^{6b}, R^{6c}, R^{6d} and R^{6e} are independently selected from H, alkyl(C₁-C₂₀), cycloalkyl(C₃-C₂₀), alkenyl(C₃-C₂₀) and alkynyl(C₃-C₂₀);
b)
   R^{6a}, R^{6b}, R^{6c}, R^{6d} and R^{6e} are independently selected from H and moieties of the formulae: 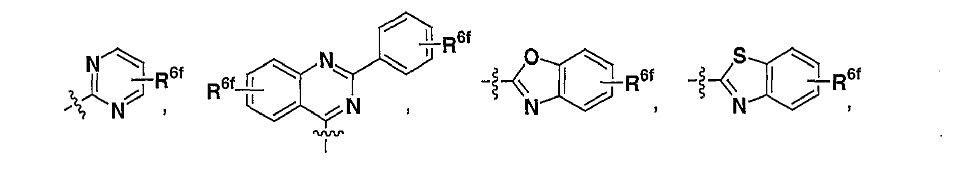 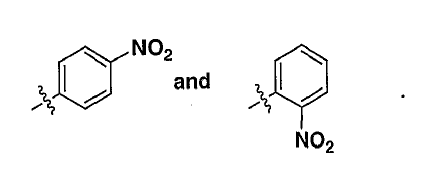
c)
   R^{6a}, R^{6b}, R^{6c}, R^{6d} and R^{6e} are H.

Preferred groups of compounds of this invention including pharmaceutically acceptable salts thereof are those in the subgroups below, wherein other variables are as defined above:
a)
   R⁷ is H; and/or
b)
   R⁷ is -C(O)-Y-Z; and/or
c)
   R⁷ is alkyl(C₁-C₂₀), cycloalkyl(C₃-C₂₀), alkenyl(C₃-C₂₀) and alkynyl(C₃-C₂₀).

Also among the preferred groups of compounds of this invention including pharmaceutically acceptable salts thereof are those in the subgroups below, wherein other variables are as defined above:
a)
   R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently selected from H and -C(O)-Y-Z;
b)
   R¹³, R¹⁴, R¹⁹, R²⁰, R²¹ and R²² are H;
   R⁸, R⁹, R¹⁰, R¹¹, R¹², R¹⁵, R¹⁶, R¹⁷ and R¹⁸ are independently selected from H and -C(O)-Y-Z;
c)
   R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently selected from H, alkyl(C₁-C₂₀), cycloalkyl(C₃-C₂₀), alkenyl(C₃-C₂₀) and alkynyl(C₃-C₂₀);
d)
   R¹³, R¹⁴, R¹⁹, R²⁰, R²¹ and R²² are H;
   R⁸, R⁹, R¹⁰, R¹¹, R¹², R¹⁵, R¹⁶, R¹⁷ and R¹⁸ are independently selected from H, alkyl(C₁-C₂₀), cycloalkyl(C₃-C₂₀), alkenyl(C₃-C₂₀) and alkynyl(C₃-C₂₀);
e)
   R⁸ and R⁹, R⁹ and R¹⁰, R¹⁰ and R¹¹, R¹³ and R¹⁴, R¹⁵ and R¹⁶, R¹⁶ and R¹⁷, R¹⁷ and R¹⁸, R¹⁹ and R²⁰, R²⁰ and R²¹ or R²¹ and R²² may independently be joined forming a moiety of the formula: 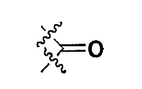 provided when;
   i) R⁸ and R⁹ are so joined, R⁹ and R¹⁰ may not be so joined;
   ii) R⁹ and R¹⁰ are so joined, R¹⁰ and R¹¹ and R⁸ and R⁹ may not be so joined;
   iii) R¹⁵ and R¹⁶ are so joined, R¹⁶ and R¹⁷ may not be so joined;
   iv) R¹⁶ and R¹⁷ are so joined, R¹⁵ and R¹⁶ and R¹⁷ and R¹⁸ may not be so joined;
   v) R¹⁹ and R²⁰ are so joined, R²⁰ and R²¹ may not be so joined;
   vi) R²⁰ and R²¹ are so joined, R¹⁹ and R²⁰ and R²¹ and R²² may not be so joined;
f)
   R¹², R¹³, R¹⁴, R¹⁹, R²⁰, R²¹, and R²² are H;
   R⁸ and R⁹, R⁹ and R¹⁰, R¹⁰ and R¹¹, R¹⁵ and R¹⁶, R¹⁶ and R¹⁷ or R¹⁷ and R¹⁸ may independently be joined forming a moiety of the formula: 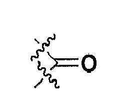 provided when;
   i) R⁸ and R⁹ are so joined, R⁹ and R¹⁰ may not be so joined;
   ii) R⁹ and R¹⁰ are so joined, R⁸ and R⁹ and R¹⁰ and R¹¹ may not be so joined;
   iii) R¹⁵ and R¹⁶ are so joined, R¹⁶ and R¹⁷ may not be so joined;
   iv) R¹⁶ and R¹⁷ are so joined, R¹⁵ and R¹⁶ and R¹⁷ and R¹⁸ may not be so joined;
g)
   R⁸ and R⁹, R⁹ and R¹⁰, R¹⁰ and R¹¹, R¹³ and R¹⁴, R¹⁵ and R¹⁶, R¹⁶ and R¹⁷, R¹⁷ and R¹⁸, R¹⁹ and R²⁰, R²⁰ and R²¹ or R²¹ and R²² may independently be joined forming a moiety of the formula: 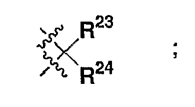 provided when;
   i) R⁸ and R⁹ are so joined, R⁹ and R¹⁰ may not be so joined;
   ii) R⁹ and R¹⁰ are so joined, R⁸ and R⁹ and R¹⁰ and R¹¹ may not be so joined;
   iii) R¹⁵ and R¹⁶ are so joined, R¹⁶ and R¹⁷ may not be so joined;
   iv) R¹⁶ and R¹⁷ are so joined, R¹⁵ and R¹⁶ and R¹⁷ and R¹⁸ may not be so joined;
   v) R¹⁹ and R²⁰ are so joined, R²⁰ and R²¹ may not be so joined;
   vi) R²⁰ and R²¹ are so joined, R¹⁹ and R²⁰ and R²¹ and R²² may not be so joined;
h)
   R¹², R¹³, R¹⁴, R¹⁹, R²⁰, R²¹, and R²² are H;
   R⁸ and R⁹, R⁹ and R¹⁰, R¹⁰ and R¹¹, R¹⁵ and R¹⁶, R¹⁶ and R¹⁷ or R¹⁷ and R¹⁸ may independently be joined forming a moiety of the formula: 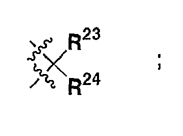 provided when;
   i) R⁸ and R⁹ are so joined, R⁹ and R¹⁰ may not be so joined;
   ii) R⁹ and R¹⁰ are so joined, R⁸ and R⁹ and R¹⁰ and R¹¹ may not be so joined;
   iii) R¹⁵ and R¹⁶ are so joined, R¹⁶ and R¹⁷ may not be so joined;
   iv) R¹⁶ and R¹⁷ are so joined, R¹⁵ and R¹⁶ and R¹⁷ and R¹⁸ may not be so joined;
      and
i)
   R¹², R¹³, R¹⁴, R¹⁵, R¹⁶, R¹⁷, R¹⁸, R¹⁹, R²⁰, R²¹, and R²² are H; R⁸ and R⁹, R⁹ and R¹⁰ or R¹⁰ and R¹¹ may independently be joined forming a moiety of the formula: 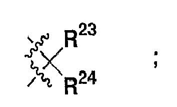 provided when;
   i) R⁸ and R⁹ are so joined, R⁹ and R¹⁰ may not be so joined;
   ii) R⁹ and R¹⁰ are so joined, R⁸ and R⁹ and R¹⁰ and R¹¹ may not be so joined;

Among the most preferred groups of compounds of this invention including pharmaceutically acceptable salts thereof are those in the subgroups below, wherein other variables are as defined above:
a)
   R¹ is a moiety selected from the group: 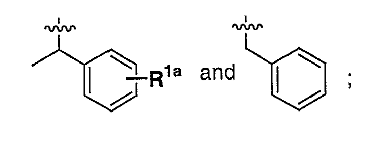
   R² is a moiety of the formula: 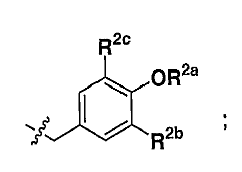
   R^{2a} is a moiety of the formula: 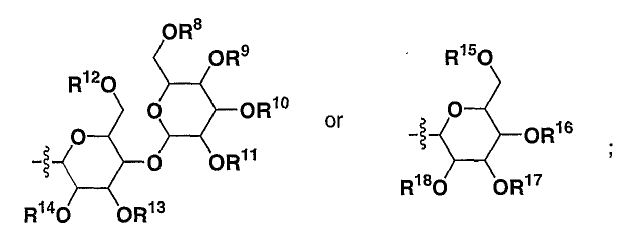
   R^{2b} and R^{2c} are H;
   R³ and R⁴ are independently H or OH;
   R⁵ is a moiety of the formula: 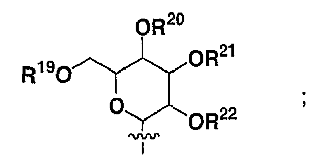
   R^{6a}, R^{6b}, R^{6c}, R^{6d} and R^{6e} are H;
   R⁷ is H;
   R¹³, R¹⁴, R¹⁹, R²⁰, R²¹ and R²² are H; and
   R⁸, R⁹, R¹⁰, R¹¹, R¹², R¹⁵, R¹⁶, R¹⁷ and R¹⁸ are independently selected from H and -C(O)-Y-Z;
b)
   R¹ is a moiety selected from the group: 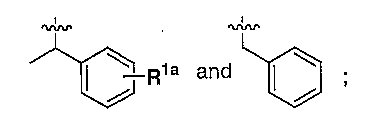
   R² is a moiety of the formula: 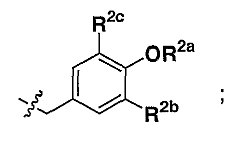
   R^{2a} is a moiety of the formula: 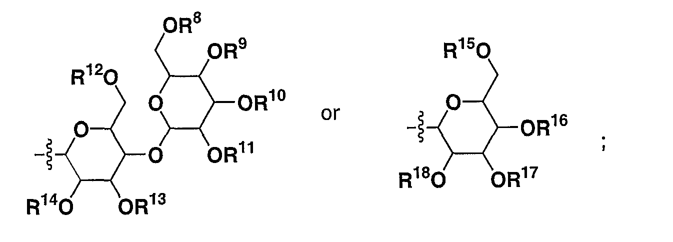
   R^{2b} and R^{2c} are H;
   R³ and R⁴ are independently H or OH;
   R⁵ is a moiety of the formula: 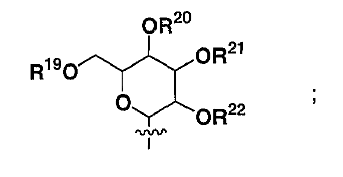
   R^{6a}, R^{6b}, R^{6c}, R^{6d} and R^{6e} are H;
   R⁷ is H;
   R¹³, R¹⁴, R¹⁹, R²⁰, R²¹ and R²² are H; and
   R⁸, R⁹, R¹⁰, R¹¹, R¹², R¹⁵, R¹⁶, R¹⁷ and R¹⁸ are independently selected from H, alkyl(C₁-C₂₀), cycloalkyl(C₃-C₂₀), alkenyl(C₃-C₂₀) and alkynyl(C₃-C₂₀);
c)
   R¹ is a moiety selected from the group: 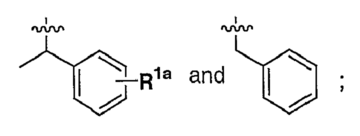
   R² is a moiety of the formula: 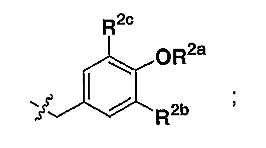
   R^{2a} is a moiety of the formula: 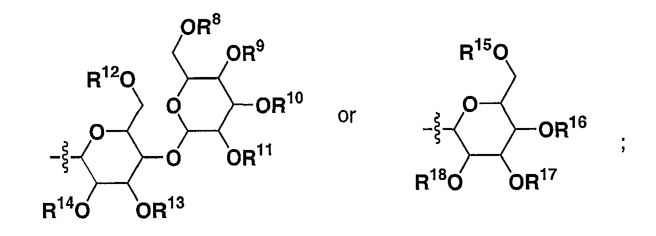
   R^{2b} and R^{2c} are H;
   R³ and R⁴ are independently H or OH;
   R⁵ is a moiety of the formula: 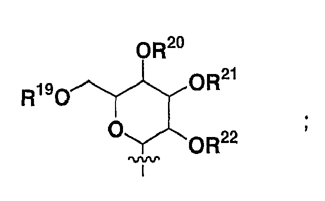
   R^{6a}, R^{6b}, R^{6c}, R^{6d} and R^{6e} are H;
   R⁷ is H;
   R¹², R¹³, R¹⁴, R¹⁹, R²⁰, R²¹ and R²² are H;
   R⁸ and R⁹, R⁹ and R¹⁰, R¹⁰ and R¹¹, R¹⁵ and R¹⁶, R¹⁶ and R¹⁷ or R¹⁷ and R¹⁸ may independently be joined forming a moiety of the formula: 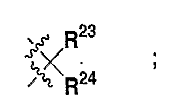 provided when;
      i) R⁸ and R⁹ are so joined, R⁹ and R¹⁰ may not be so joined;
      ii) R⁹ and R¹⁰ are so joined, R⁸ and R⁹ and R¹⁰ and R¹¹ may not be so joined;
      iii) R¹⁵ and R¹⁶ are so joined, R¹⁶ and R¹⁷ may not be so joined;
      iv) R¹⁶ and R¹⁷ are so joined, R¹⁵ and R¹⁶ and R¹⁷ and R¹⁸ may not be so joined;
d)
   R¹ is a moiety selected from the group: 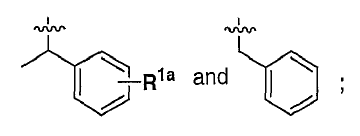
   R² is a moiety of the formula: 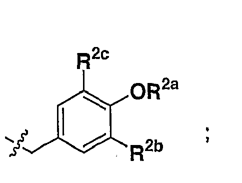
   R^{2a} is a moiety of the formula: 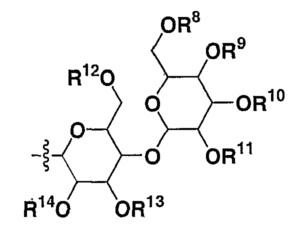
   R^{2b} and R^{2c} are H;
   R³ and R⁴ are independently H or OH;
   R⁵ is a moiety of the formula: 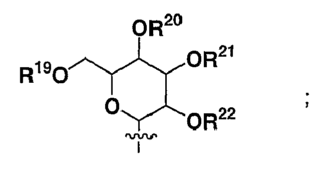
   R^{6a}, R^{6b}, R⁶, R^{6d} and R^{6e} are H;
   R⁷ is H;
   R¹², R¹³, R¹⁴, R¹⁹, R²⁰, R²¹, and R²² are H;
   R⁸ and R⁹, R⁹ and R¹⁰ or R¹⁰ and R¹¹ may independently be joined forming a moiety of the formula: 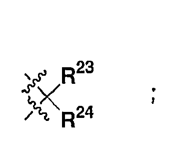 provided when;
      i) R⁸ and R⁹ are so joined, R⁹ and R¹⁰ may not be so joined;
      ii) R⁹ and R¹⁰ are so joined, R⁸ and R⁹ and R¹⁰ and R¹¹ may not be so joined;
e)
   R¹ is a moiety selected from the group: 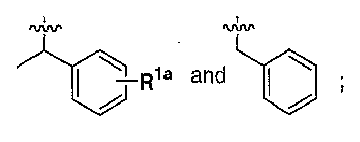
   R² is a moiety of the formula: 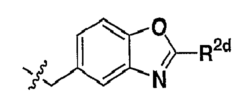
   R³ and R⁴ are independently H or OH;
   R⁵ is H or a moiety of the formula: 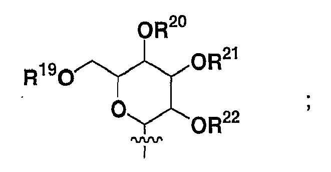
   R¹⁹, R²⁰, R²¹ and R²² are H;
f)
   R¹ is a moiety selected from the group: 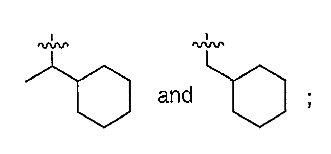
   R² is a moiety of the formula: 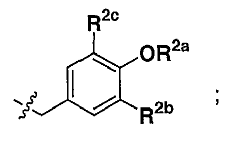
   R^{2a} is a moiety of the formula: 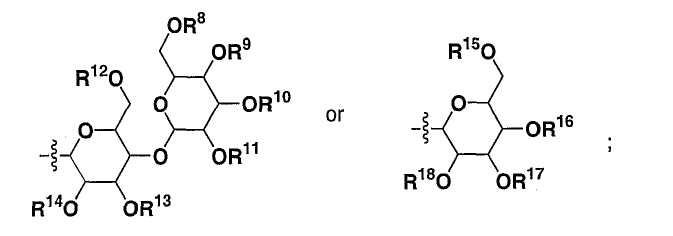
   R^{2b} and R^{2c} are H;
   R³ and R⁴ are independently H or OH;
   R⁵ is a moiety of the formula: 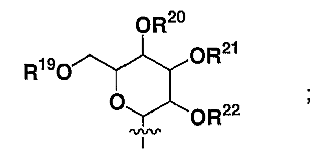
   R^{6a}, R^{6b}, R^{6c}, R^{6d} and R^{6e} are H;
   R⁷ is H;
   R¹³, R¹⁴, R¹⁹, R²⁰, R²¹ and R²² are H; and
   R⁸, R⁹, R¹⁰, R¹¹, R¹², R¹⁵, R¹⁶, R¹⁷ and R¹⁸ are independently selected from H and -C(O)-Y-Z;
g)
   R¹ is a moiety selected from the group: 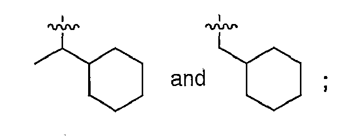
   R² is a moiety of the formula: 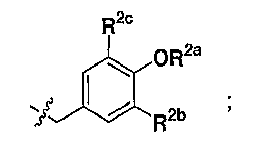
   R^{2a} is a moiety of the formula: 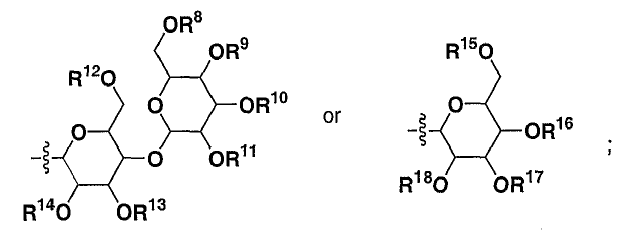
   R^{2b} and R^{2c} are H;
   R³ and R⁴ are independently H or OH;
   R⁵ is a moiety of the formula: 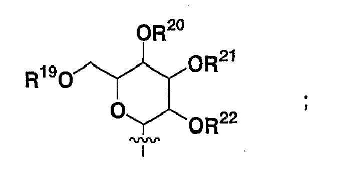
   R^{6a}, R^{6b}, R^{6c}, R^{6d} and R^{6e} are H;
   R⁷ is H;
   R¹³, R¹⁴, R¹⁹, R²⁰ R²¹ and R²² are H; and
   R⁸, R⁹_{,} R¹⁰, R¹¹, R¹², R¹⁵, R¹⁶, R¹⁷ and R¹⁸ are independently selected from H, alkyl(C₁-C₂₀), cycloalkyl(C₃-C₂₀), alkenyl(C₃-C₂₀) and alkynyl(C₃-C₂₀);
h)
   R¹ is a moiety selected from the group: 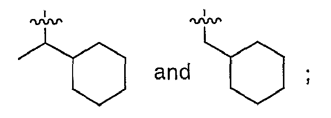
   R² is a moiety of the formula: 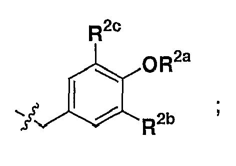
   R^{2a} is a moiety of the formula: 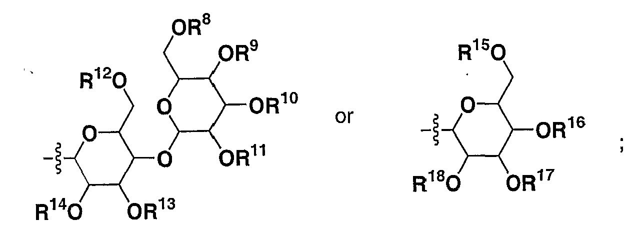
   R^{2b} and R^{2c} are H;
   R³ and R⁴ are independently H or OH;
   R⁵ is a moiety of the formula: 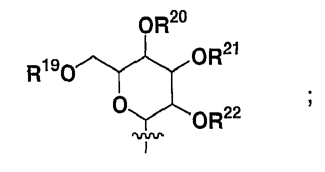
   R^{6a}, R^{6b}, R^{6c}, R^{6d} and R^{6e} are H;
   R⁷ is H;
   R¹², R¹³, R¹⁴, R¹⁹, R²⁰, R²¹ and R²² are H;
   R⁸ and R⁹, R⁹ and R¹⁰, R¹⁰ and R¹¹, R¹⁵ and R¹⁶, R¹⁶ and R¹⁷ or R¹⁷ and R¹⁸ may independently be joined forming a moiety of the formula: 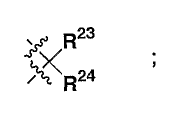 provided when;
      i) R⁸ and R⁹ are so joined, R⁹ and R¹⁰ may not be so joined;
      ii) R⁹ and R¹⁰ are so joined, R⁸ and R⁹ and R¹⁰ and R¹¹ may not be so joined;
      iii) R¹⁵ and R¹⁶ are so joined, R¹⁶ and R¹⁷ may not be so joined;
      iv) R¹⁶ and R¹⁷ are so joined, R¹⁵ and R¹⁶ and R¹⁷ and R¹⁸ may not be so joined;
i)
   R¹ is a moiety selected from the group: 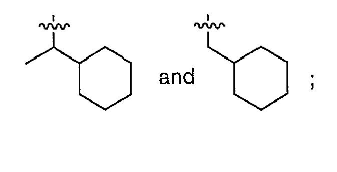
   R² is a moiety of the formula: 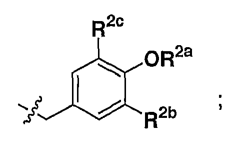
   R^{2a} is a moiety of the formula: 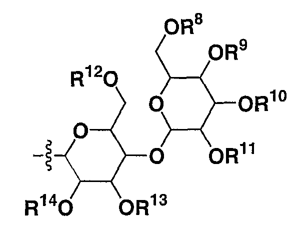
   R^{2b} and R^{2c} are H;
   R³ and R⁴ are independently H or OH;
   R⁵ is a moiety of the formula: 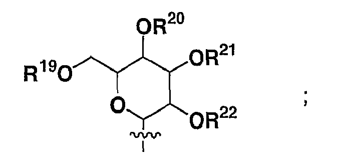
   R^{6a}, R^{6b}, R^{6c}, R^{6d} and R^{6e} are H;
   R⁷ is H;
   R¹², R¹³, R¹⁴, R¹⁹, R²⁰, R²¹, and R²² are H;
   R⁸ and R⁹, R⁹ and R¹⁰ or R¹⁰ and R¹¹ may independently be joined forming a moiety of the formula: 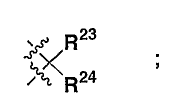 provided when;
      i) R⁸ and R⁹ are so joined, R⁹ and R¹⁰ may not be so joined;
      ii) R⁹ and R¹⁰ are so joined, R⁸ and R⁹ and R¹⁰ and R¹¹ may not be so joined;
j)
   R¹ is a moiety selected from the group: 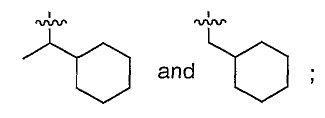
   R² is a moiety of the formula: 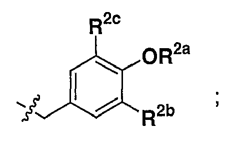
   R^{2a} is a moiety of the formula: 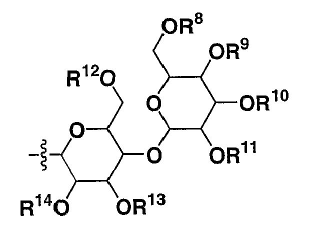
   R^{2b} and R^{2c} are H;
   R³ and R⁴ are independently H or OH;
   R⁵ is a moiety of the formula: 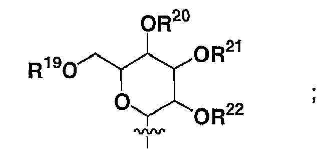
   R^{6a}, R^{6b}, R^{6c}, R^{6d} and R^{6e} are H;
   R⁷ is H;
   R¹², R¹³, R¹⁴, R¹⁹, R²⁰, R²¹, and R²² are H;
   R⁸ and R⁹, R⁹ and R¹⁰ or R¹⁰ and R¹¹ may independently be joined forming a moiety of the formula: 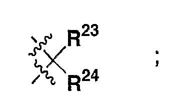 provided when;
      i) R⁸ and R⁹ are so joined, R⁹ and R¹⁰ may not be so joined;
      ii) R⁹ and R¹⁰ are so joined, R⁸ and R⁹ and R¹⁰ and R¹¹ may not be so joined;
   R²³ and R²⁴ when taken together with the carbon atom to which each is attached may optionally form carbocyclic, monocyclic, fused, bridged, spirocyclic or polycyclic rings of from three to twenty ring atoms;
      provided when;
      i) R⁸ and R⁹ are so joined, R⁹ and R¹⁰ may not be so joined;
      ii) R⁹ and R¹⁰ are so joined, R⁸ and R⁹ and R¹⁰ and R¹¹ may not be so joined;
k)
   R¹ is a moiety selected from the group: 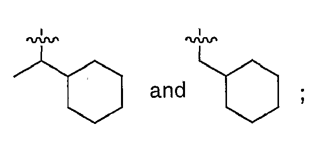
   R² is a moiety of the formula:
   R³ and R⁴ are independently H or OH;
   R⁵ is H or a moiety of the formula: 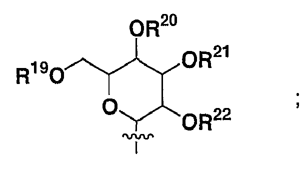 and
   R¹⁹, R²⁰, R²¹ and R²² are H;
   and
l)
   R¹ is a moiety selected from the group: 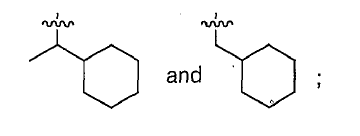
   R² is a moiety of the formula: 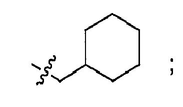
   R³ and R⁴ are independently H or OH;
   R⁵ is H or a moiety of the formula: 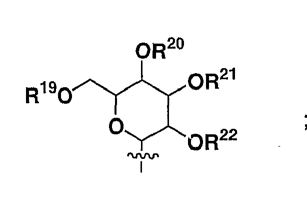 and
   R¹⁹, R²⁰, R²¹ and R²² are H.

Specifically preferred compounds of the invention are the following compounds or a pharmaceutically acceptable salt thereof:
Cyclo[glycyl-(*S*)-β-methyl-L-phenylalanyl-3-bromo-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-3-bromo-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-3-bromo-D-tyrosyl-3-(2-iminoimidazolidin-4-yl)-L-seryl-3-(2-imino-3α-mannopyranosylimidazolidin-4-yl)-D-seryl-L-seryl];
Cyclo[glycyl-β-methylphenylalanyl-3-bromotyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-3,5-dibromo-D-tyrosyl-3-(2-iminoimidazolidin-4-yl)-L-seryl-3-(2-imino-3a-mannopyranosylimidazolidin-4-yl)-D-seryl-L-seryl];
Cyclo[glycyl-β-methylphenylalanyl-3,5-dibromotyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-3,5-dibromo-D-tyrosyl-3-(2-iminoimidazolidin-4-yl)-L-seryl-3-(2-iminoimidazolidin-4-yl)-D-seryl-L-seryl];
Cyclo[glycyl-β-methylphenylalanyl-3,5-dibromotyrosyl-3-(2-iminoimidazolidin-4-yl)seryl-3-(2-iminoimidazolidin-4-yl)serylseryl];
Cyclo[glycyl-(*S*)-β-methyl-L-phenylalanyl-3-iodo-D-tyrosyl-3-(2-iminoimidazolidin-4-yl)-L-seryl-3-(2-imino-3α-mannopyranosylimidazolidin-4-yl)-D-seryl-L-seryl];
Cyclo[glycyl-β-methylphenylalanyl-3-iodotyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-3-iodo-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-(4-O-hexopyranosylhexopyranosyl)-3-iodo-tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-3,5-diiodo-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-(4-O-hexopyranosylhexopyranosyl)-3,5-diiodo-tyrosyl-3-(2-iminoimidazolidin-4-yl)seryl-3-(3-hexopyranosyl-2-iminoimidazolidin-4-yl)sexylseryl];
Cyclo[glycyl-(*S*)-β-methyl-L-phenylalanyl-3-nitro-D-tyrosyl-3-(2-iminoimidazolidin-4-yl)-L-seryl-3-(2-imino-3α-mannopyranosylimidazolidin-4-yl)-D-seryl-L-seryl];
Cyclo[glycyl-β-methylphenylalanyl-3-mtrotyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-3-nitro-D-tyrosyl-3-(2-iminoimidazolidin-4-yl)-L-seryl-3-(2-iminoimidazolidin-4-yl)-D-seryl-L-seryl];
Cyclo[glycyl-β-methylphenylalanyl-3-nitrotyrosyl-3-(2-iminoimidazolidin-4-yl)seryl-3-(2-iminoimidazolidin-4-yl)serylseryl];
Cyclo[3-(2-iminoimidazolidin-4-yl)-L-alanyl-3-(2-imino-3-α-D-mannopyranosylimidazolidin-4-yl)-D-alanyl-L-serylglycyl-(*S*)-β-methyl-L-phenylalanyl-3-nitro-D-tyrosyl];
Cyclo[3-(2-iminoimidazolidin-4-yl)alanyl-3-(3-hexopyranosyl-2-iminoimidazolidin-4-yl)-alanylserylglycyl-β-methylphenylalanyl-3-nitrotyrosyl];
Cyclo[glycyl-(*S*)-β-methyl-L-phenylalanyl-3-amino-D-tyrosyl-3-(2-iminoimidazolidin-4-yl)-L-seryl-3-(2-imino-3α-mannopyranosylimidazolidin-4-yl)-D-seryl-L-seryl];
Cyclo[glycyl-β-methylphenylalanyl-3-aminotyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-3-(dimethylamino)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-3-(dimethylamino)tyrosyl-3-(2-iminoimidazolidin-4-yl)-seryl-3-(3-hexopyranosyl-2-iminoimidazolidin-4-yl)serylseryl];
Cyclo[glycyl-(*S*)-β-methyl-L-phenylalanyl-3-acetamido-D-tyrosyl-3-(2-iminoimidazolidin-4-yl)-L-seryl-3-(2-imino-3-α-D-mannopyranosylimidazolidin-4-yl)-D-seryl-L-seryl] ;
Cyclo[glycyl-β-methylphenylalanyl-3-acetamidotyrosyl-3-(2-iminoimidazolidin-4-yl)seryl-3-(3-hexopyranosyl-2-iminoimidazolodin-4-yl)serylseryl];
Cyclo[glycyl-(*S*)-β-methyl-L-phenylalanyl-3-(propanamido)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-3-(propanamido)tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-3-(2-methylpropanamido)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-3-(2-methylpropanamido)tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-3-(heptanamido)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-3-(heptanamido)tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-3-(benzamido)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-3-(benzamido)tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-3-formamido-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-3-formamidotyrosyl-3-(2-iminoimidazolidin-4-yl)seryl-3-(3-hexopyranosyl-2-iminoimidazolidin-4-yl)serylseryl];
Cyclo[glycyl-(5')-β-methyl-L-phenylalanyl-3-[[(4-methylphenoxy)carbonyl]amino]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-3-[[(4-methylphenoxy)carbonyl]amino]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-3-[(methoxycarbonyl)amino]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-3-[(methoxycarbonyl)amino]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-3-[(phenylmethoxycarbonyl)amino]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-3-[(phenylmethoxycarbonyl)amino]tyrosyl-3-(2-iminoimidazolidin-4-yl)seryl-3-(3-hexopyranosyl-2-iminoimidazolidin-4-yl)serylseryl];
Cyclo[3-(2,3-dihydro-2-oxo-1,3-benzoxazol-5-yl)-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-(2,3-dihydro-2-oxo-1,3-benzoxazol-5-yl)alanyl-3-(2-iminoimidazolidin-4-yl)seryl-3-(3-hexopyranosyl-2-iminoimidazolidin-4-yl)serylserylglycyl-β-methylphenylalanyl];
   23
Cyclo[3-(2,3-dihydro-2-thio-1,3-benzoxazol-5-yl)-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-(2,3-dihydro-2-thio-1,3-benzoxazol-5-yl)alanyl-3-(2-iminoimidazolidin-4-yl)seryl-3-(3-hexopyranosyl-2-iminoimidazolidin-4-yl)serylserylglycyl-β-methylphenylalanyl];
Cyclo[glycyl-(*S*)-β-methyl-L-phenylalanyl-3-[[[3,5-bis(trifluoromethyl)phenyl]carbamothioyl]-amino]-D-tyrosyl-3-(2-iminoimidazolidin-4-yl)-L-seryl-3-(2-imino-3-α-D-mannopyranosyl-imidazolidin-4-yl)-D-seryl-L-seryl];
Cyclo[glycyl-β-methylphenylalanyl-3-[[[3,5-bis(trifluoromethyl)phenyl]carbamothioyl]amino]-tyrosyl-3-(2-iminoimidazolidin-4-yl)seryl-3-(3-hexopyranosyl-2-iminoimidazolidin-4-yl)-serylseryl];
Cyclo[3-[2-(4-carboxyphenyl)-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-(4-carboxyphenyl)-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenylalanyl];
Cyclo[3-[2-(3-nitrophenyl)-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-(3-nitrophenyl)-1,3-benzoxazol-5-yl)alanyl-3-(2-iminoimidazolidin-4-yl)seryl-3-(3-hexopyranosyl-2-iminoimidazolidin-4-yl)serylserylglycyl-β-methylphenylalanyl];
Cyclo[3-[2-(4-bromophenyl)-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-(4-bromophenyl)-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenylalanyl];
Cyclo[3-[2-[3-(4-methylphenoxy)phenyl]-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-[3-(4-methylphenoxy)phenyl]-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenyl-alanyl];
Cyclo[3-[2-[4-(dimethylamino)phenyl]-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-[4-(dimethylamino)phenyl]-1,3-benzoxazol-5-yl]alanyl-3-(2-iminoimidazolidin-4-yl)-seryl-3-(3-hexopyranosyl-2-iminoimidazolidin-4-yl)serylserylglycyl-β-methylphenyl-alanyl];
Cyclo[3-[2-(3-fluorophenyl)-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-(3-fluorophenyl)-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenylalanyl];

Cyclo[3-[2-[4-(phenylmethoxy)phenyl]-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-[4-(phenylmethoxy)phenyl]-1,3-benzoxazol-5-yl]alanyl-3-(2-iminoimidazolidin-4-yl)seryl-3-(3-hexopyranosyl-2-iminoimidazolidin-4-yl)serylserylglycyl-β-methyl-phenylalanyl];
Cyclo[3-[2-(4-tert-butylphenyl)-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-(4-tert-butylphenyl)-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenylalanyl];
Cyclo[3-[2-([1,1-biphenyl]-4-yl)-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-([1,1-biphenyl]-4-yl)-1,3-benzoxazol-5-yl]alanyl-3-(2-iminoimidazolidin-4-yl)-seryl-3-(3-hexopyranosyl-2-iminoimidazolidin-4-yl)serylserylglycyl-β-methylphenylalanyl];
Cyclo[3-[2-(3,4,5-trimethoxyphenyl)-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-(3,4,5-trimethoxyphenyl)-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenyl-alanyl];
Cyclo[3-[2-[3-(4-methoxyphenoxy)phenyl]-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-[3-(4-methoxyphenoxy)phenyl]-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenyl-alanyl];
Cyclo[3-[2-[2-(α-D-glucopyranosyloxy)phenyl]-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-[2-(hexopyranosyloxy)phenyl]-1,3-benzoxazol-5-yl]alanyl-3-(2-iminoimidazolidin-4-yl)seryl-3-(3-hexopyranosyl-2-iminoimidazolidin-4-yl)serylserylglycyl-β-methyl-phenylalanyl];
Cyclo[3-[2-(9H-fluroren-2-yl)-1,3-benzoxazol-5-yl]-D-alanyl-3-(2-iminoimidazolidin-4-yl)-L-seryl-3-(2-imino-3-(α-D-mannopyranosylimidazolidin-4-yl)-D-seryl-L-serylglycyl-(*S*)-β-methyl-L-phenylalanyl];
Cyclo[3-[2-(9H-fluoren-2-yl)-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenylalanyl];
Cyclo[3-[2-(3-furyl)-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-(3-furyl)-1,3-benzoxazol-5-y]lalanyl-3-(2-iminoimidazolidin-4-yl)seryl-3-(3-hexopyranosyl-2-iminoimidazolidin-4-yl)serylserylglycyl-β-methylphenylalanyl];
Cyclo[3-[2-(2,2-diphenylethenyl)-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-(2,2-diphenylethenyl)-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenylalanyl];
Cyclo[3-[2-(2-methylprop-1-en-1-yl)-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-(2-methylprop-1-en-1-yl)-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenyl-alanyl];
Cyclo[3-[2-[3-(4-methylphenoxy)phenyl]-1,3-benzoxazol-5-yl]-D-alanyl-3-(2-iminoimidazolidin-4-yl)-L-seryl-3-(2-iminoimidazolidin-4-yl)-D-seryl-L-serylglycyl-(*S*)-β-methyl-L-phenyl-alanyl];
Cyclo[3-[2-[4-(3-methylphenoxy)phenyl]benzoxazol-5-yl]alanyl-3-(2-iminoimidazolidin-4-yl)-seryl-3-(2-iminoimidazolidin-4-yl)serylserylglycyl-β-methylphenylalanyl];
Cyclo[3-[2-(9H-fluoren-2-yl)-1,3-benzoxazol-5-yl]-D-alanyl-3-(2-iminoimidazolidin-4-yl)-L-alanyl-3-(2-imino-3-α-D-mannopyranosylimidazolidin-4-yl)-D-alanyl-L-serylglycyl-(*S*)-β-methyl-L-phenylalanyl];
Cyclo[3-[2-(9H-fluoren-2-yl)-1,3-benzoxazol-5-yl]alanyl-3-(2-iminoimidazolidin-4-yl)alanyl-3-(3-hexopyranosyl-2-iminoimidazolidin-4-yl)alanylserylglycyl-β-methylphenylalanyl];
Cyclo[3-[2-(6-methoxynaphth-2-yl)-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-(6-methoxynaphth-2-yl)-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenylalanyl];
Cyclo[3-[[2-(2,3,4,6-tetra-O-benzoyl-glucopyranosyl)amino]-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[[2-(2,3,4,6-tetra-O-benzoylhexopyranosyl)amino]-1,3-benzoxazol-5-yl]alanyl-3-(2-iminoimidazolidin-4-yl)seryl-3-(3-hexopyranosyl-2-iminoimidazolidin-4-yl)seryl-serylglycyl-β-methylphenylalanyl];
Cyclo[3-[2-(benzylthio)-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-(benzylthio)-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenylalanyl];
Cyclo[3-[2-[(2-naphthylmethyl)thio]-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-[(naphthylmethyl)thio]-1,3-benzoxazol-5-yl]alanyl-3-(2-iminoimidazolidin-4-yl)-seryl-3-(3-hexopyranosyl-2-iminoimidazolidin-4-yl)serylserylglycyl-β-methylphenylalanyl];
Cyclo[3-[2-[(4-phenylbenzyl)thio]-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-[(4-phenylbenzyl)thio]-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenylalanyl];
Cyclo[3-[2-[(2-oxo-2-phenylethyl)thio]-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-[(2-oxo-2-phenylethyl)thio]-1,3-benzoxazol-5-yl]alanyl-3-(2-iminoimidazolidin-4-yl)-seryl-3-(3-hexopyranosyl-2-iminoimidazolidin-4-yl)serylserylglycyl-β-methylphenyl-alanyl];

Cyclo[3-[2-[[2-(4-chlorophenyl)-2-oxoethyl]thio]-1,3-benzoxazol-5-yl]-D-alanyl-3-(2-iminoimidazolidin-4-yl)-L-seryl-3-(2-imino-3-α-D-mannopyranosylimidazolidin-4-yl)-D-seryl-L-serylglycyl-(*S*)-β-methyl-L-phenylalanyl];
Cyclo[3-[2-[[2-(4-chlorophenyl)-2-oxoethyl]thio]-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenylalanyl];
Cyclo[3-cyclohexyl-L-alanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-serylglycyl];
Cyclo[3-cyclohexylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylserylglycyl];
Cyclo[(3S)-3-cyclohexyl-L-2-aminobutanoyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-serylglycyl];
Cyclo[3-cyclohexyl-2-aminobutanoyl-O-(4-O-hexopyranosylhexopyranosyl)tyrosyl-3-(2-iminoimidazolidin-4-yl)seryl-3-(3-hexopyranosyl-2-iminoimidazolidin-4-yl)-serylserylglycyl];
Cyclo[3-cyclohexyl-L-alanyl-3-cyclohexyl-D-alanyl-3-(2-iminoimidazolidin-4-yl)-L-seryl-3-(2-imino-3-α-D-mannopyranosylimidazolidin-4-yl)-D-seryl-L-serylglycyl];
Cyclo[3-cyclohexylalanyl-3-cyclohexylalanyl-3-(2-iminoimidazolidin-4-yl)seryl-3-(3-hexopyranosyl-2-iminoimidazolidin-4-yl)serylserylglycyl];
Cyclo[3-cyclohexyl-L-alanyl-3-[4-[(4-O-α-D-mannopyranosyl-α-D-mannopyranosyl)oxy]cyclohexyl]-D-alanyl-3-(2-iminoimidazolidin-4-yl)-L-seryl-3-(2-imino-3-α-D-mannopyranosylimidazolidin-4-yl)-D-seryl-L-serylglycyl];
Cyclo[3-cyclohexylalanyl-3-[4-[(4-O-hexopyranosylhexopyranosyl)oxy]cyclohexyl]alanyl-3-(2-iminoimidazolidin-4-yl)seryl-3-(3-hexopyranosyl-2-iminoimidazolidin-4-yl)serylserylglycyl);
Cyclo[3-cyclohexyl-L-alanyl-3-[4-[(4-O-α-D-mannopyranosyl-α-D-mannopyranosyl)oxy]cyclohexyl]-D-alanyl-3-(2-iminoimidazolidin-4-yl)-L-seryl-3-(2-imino-3-α-D-mannopyranosylimidazolidin-4-yl)-D-seryl-L-serylglycyl];
Cyclo[3-cyclohexylalanyl-3-[4-[(4-O-hexopyranosylhexopyranosyl)oxy]cyclohexyl]alanyl-3-(2-iminoimidazolidin-4-yl)seryl-3-(3-hexopyranosyl-2-iminoimidazolidin-4-yl)serylserylglycyl];
Cyclo[3-cyclohexyl-D-alanyl-3-(2-iminoimidazolidin-4-yl)-L-seryl-3-(2-imino-3-α-D-mannopyranosylimidazolidin-4-yl)-D-seryl-L-serylglycyl-(3S)-3-cyclohexyl-L-2-aminobutanoyl]
Cyclo[3-cyclohexylalanyl-3-(2-iminoimidazolidin-4-yl)seryl-3-(3-hexopyranosyl-2-iminoimidazolidin-4-yl)serylserylglycyl-3-cyclohexyl-2-aminobutanoyl];
Cyclo[3-[4-[(4-O-α-D-mannopyranosyl-α-D-mannopyranosyl)oxy]cyclohexyl]-D-alanyl-3-(2-iminoimidazolidin-4-yl)-L-seryl-3-(2-imino-3-α-D-mannopyranosylimidazolidin-4-yl)-D-seryl-L-serylglycyl-(3S)-3-cyclohexyl-L-2-aminobutanoyl];
Cyclo[3-[4-[(4-O-hexopyranosylhexopyranosyl)oxy]cyclohexyl]alanyl-3-(2-iminoimidazolidin-4-yl)seryl-3-(3-hexopyranosyl-2-iminoimidazolidin-4-yl)serylserylglycyl-3-cyclohexyl-2-aminobutanoyl];
Cyclo[3-(syn-4-hydroxycyclohexyl)-D-alanyl-3-(2-iminoimidazolidin-4-yl)-L-seryl-3-(2-imino-3-α-D-mannopyranosylimidazolidin-4-yl)-D-seryl-L-serylglycyl-(3S)-3-cyclohexyl-L-2-aminobutanoyl];
Cyclo[3-(syn-4-hydroxycyclohexyl)alanyl-3-(2-iminoimidazolidin-4-yl)seryl-3-(3-hexopyranosyl-2-iminoimidazolidin-4-yl)serylserylglycyl-3-cyclohexyl-2-aminobutanoyl] ;
Cyclo[3-(anti-4-hydroxycyclohexyl)-D-alanyl-3-(2-iminoimidazolidin-4-yl)-L-seryl-3-(2-imino-3-α-D-mannopyranosylimidazolidin-4-yl)-D-seryl-L-serylglycyl-(3S)-3-cyclohexyl-L-2-aminobutanoyl];
Cyclo[3-(anti-4-hydroxycyclohexyl)alanyl-3-(2-iminoimidazolidin-4-yl)seryl-3-(3-hexopyranosyl-2-iminoimidazolidin-4-yl)serylserylglycyl-3-cyclohexyl-2-aminobutanoyl];
Cyclo[glycyl-(*S*)-β-methyl-L-phenylalanyl-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyltyrosyl-3-(2-iminoimidazolidin-4-yl)seryl-3-(3-hexopyranosyl-2-iminoimidazolidin-4-yl)serylseryl];
Cyclo[glycyl-L-phenylalanyl-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycylphenylalanyltyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-D-tyrosyl-3-(2-iminoimidazolidin-4-yl)-L-seryl-3-(2-iminoimidazolidin-4-yl)-D-seryl-L-seryl];
Cyclo[glycyl-β-methylphenylalanyltyrosyl-3-(2-iminoimidazolidin-4-yl)seryl-3-(2-iminoimidazolidin-4-yl)serylseryl];
Cyclo[3-cyclohexyl-L-alanyl-3-cyclohexyl-D-alanyl-3-(2-iminoimidazolidin-4-yl)-L-seryl-3-(2-iminoimidazolidin-4-yl)-D-seryl-L-serylglycyl];
Cyclo[3-cyclohexylalanyl-3-cyclohexylalanyl-3-(2-iminoimidazolidin-4-yl)seryl-3-(2-iminoimidazolidin-4-yl)-serylserylglycyl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-[1-benzyl-2-imino-3-α-D-mannopyranosyl-imidazolidin-4-yl]-D-seryl-L-seryl];
Cyclo[glycyl-β-methylphenylalanyl-O-(4-O-hexopyranosylhexopyranosyl)tyrosyl-(2-iminoimidazolidin-4-yl)seryl-3-[1-benzyl-3-hexopyranosyl-2-iminoimidazolidin-4-yl]serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-[1-(4-tert-butylbenzyl)-2-imino-3-α-D-mannopyranosylimidazolidin-4-yl]-D-seryl-L-seryl];
Cyclo[glycyl-β-methylphenylalanyl-O-(4-O-hexopyranosylhexopyranosyl)tyrosyl-(2-iminoimidazolidin-4-yl)seryl-3-[1-(4-tert-butylbenzyl)-3-hexopyranosyl-2-iminoimidazolidin-4-yl]-serylseryl];
Di-N-(4-tert-butylbenzyl)-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Di-N-(4-tert-butylbenzyl)-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-[1-(12-hydroxydodecyl)-2-imino-3-α-D-mannopyranosylimidazolidin-4-yl]-D-seryl-L-seryl];
Cyclo[glycyl-β-methylphenylalanyl-O-(4-O-hexopyranosylhexopyranosyl)tyrosyl-3-(2-iminoimidazolidin-4-yl)seryl-3-[3-hexopyranosyl-1-(12-hydroxydodecyl)-2-iminoimidazolidin-4-yl]-serylseryl];
Cyclo[glycyl-(*S*)-β-methyl-L-phenylalanyl-O-(4-O-α-D-mannopyranosyl-α-D-mannopyranosyl)-D-tyrosyl-3-[1,3-dibenzyl-2-(benzylimino)imidazolidin-4-yl]-L-seryl-3-[1-benzyl-2-(benzyl-imino)-3-α-D-mannopyranosylimidazolidin-4-yl]-D-seryl-L-seryl];
Cyclo[glycyl-β-methylphenylalanyl-O-(4-O-hexopyranosylhexopyranosyl)tyrosyl-3-[1,3-dibenzyl-2-(benzylimino)imidazolidin-4-yl]seryl-3-[1-benzyl-2-(benzylimino)-3-hexopyranosylimidazolidin-4-yl]serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-[1-butyl-2-imino-3-α-D-mannopyranosyl-imidazolidin-4-yl]-D-seryl-L-seryl];

Cyclo[glycyl-β-methylphenylalanyl-O-(4-O-hexopyranosylhexopyranosyl)tyrosyl-3-(2-iminoimidazolidin-4-yl)seryl-3-[1-butyl-3-hexopyranosyl-2-iminoimidazolidin-4-yl]serylseryl];
Cyclo[glycyl-(*S*)-β-methyl-L-phenylalanyl-O-(4-O-α-D-mannopyranosyl-α-D-mannopyranosyl)-D-tyrosyl-3-[1,3-dimethyl-2-(methylimino)imidazolidin-4-yl]-L-seryl-3-[3-α-D-mannopyranosyl-l-methyl-2-(methylimino)imidazolidin-4-yl]-D-seryl-L-seryl] bis-methidodide
Cyclo[glycyl-β-methylphenylalanyl-O-(4-O-hexopyranosylhexopyranosyl)tyrosyl-3-(1,3-dimethyl-2-(methylimino)imidazolidin-4-yl)seryl-3-[3-hexopyranosyl-1-methyl-2-(methylimino)imidazolidin-4-yl]serylseryl] bis-methiodide
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-[2-imino-3-α-D-mannopyranosyl-1-(2-phenylbenzyl)-imidazolidin-4-yl]-D-seryl-L-seryl];
Cyclo[glycyl-β-methylphenylalanyl-O-(4-O-hexopyranosylhexopyranosyl)tyrosyl-3-(2-iminoimidazolidin-4-yl)seryl]-3-[3-hexopyranosyl-2-imino-1-(2-phenylbenzyl)-imidazolidin-4-yl]-serylseryl];
Cyclo[glycyl-(*S*)-β-methyl-L-phenylalanyl-O-(4-O-α-D-mannopyranosyl-α-D-mannopyranosyl)-D-tyrosyl-3-[3-(4-phenylbenzyl)-2-[(4-phenylbenzyl)imino]imidazolidin-4-yl]-L-seryl-3-[2-imino-3-α-D-mannopyranosyl-1-(4-phenylbenzyl)-imidazolidin-4-yl]-D-seryl-L-seryl];
Cyclo[glycyl-β-methylphenylalanyl-O-(4-O-hexopyranosylhexopyranosyl)tyrosyl-3-[3-(4-phenylbenzyl)-2-[(4-phenylbenzyl)imino]imidazolidin-4-yl]seryl-3-[3-hexopyranosyl-2-imino-1-(4-phenylbenzyl)-imidazolidin-4-yl] serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-[2-imino-3-α-D-mannopyranosyl-1-(4-phenylbenzyl)-imidazolidin-4-yl]-D-seryl-L-seryl];
Cyclo[glycyl-β-methylphenylalanyl-O-(4-O-hexopyranosylhexopyranosyl)tyrosyl-3-(2-iminoimidazolidin-4-yl)seryl]-3-[3-hexopyranosyl-2-imino-1-(4-phenylbenzyl)-imidazolidin-4-yl]-serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-[2-imino-3-α-D-mannopyranosyl-1-(2-naphthylmethyl)-imidazolidin-4-yl]-D-seryl-L-seryl];
Cyclo[glycyl-β-methylphenylalanyl-O-(4-O-hexopyranosylhexopyranosyl)tyrosyl-3-(2-iminoimidazolidin-4-yl)seryl-3-[3-hexopyranosyl-2-imino-1-(2-naphthylmethyl)-imidazolidin-4-yl]-serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-[2-imino-3-α-D-mannopyranosyl-1-(4-trifluoromethylbenzyl)-imidazolidin-4-yl]-D-seryl-L-seryl];
Cyclo[glycyl-β-methylphenylalanyl-O-(4-O-hexopyranosylhexopyranosyl)tyrosyl-3-(2-iminoimidazolidin-4-yl)seryl]-3-[3-hexopyranosyl-2-imino-1-(4-trifluoromethylbenzyl)-imidazolidin-4-yl]-serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-[1-(4-carboxybenzyl)-2-imino-3-α-D-mannopyranosylimidazolidin-4-yl]-D-seryl-L-seryl];
Cyclo[glycyl-β-methylphenylalanyl-O-(4-O-hexopyranosylhexopyranosyl)tyrosyl-3-(2-iminoimidazolidin-4-yl)seryl-3-[1-(4-carboxybenzyl)-2-imino-3-hexopyranosylimidazolidin-4-yl]-serylseryl];
Di-N-(4-carboxybenzyl)-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Di-N-(4-carboxybenzyl)-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
Tri-N-(4-carboxybenzyl)-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl]
Tri-N-(4-carboxybenzyl)-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-[2-imino-3-α-D-mannopyranosyl-1-(3-methyl-but-2-enyl)-imidazolidin-4-yl]-D-seryl-L-seryl];
Cyclo[glycyl-β-methylphenylalanyl-O-(4-O-hexopyranosylhexopyranosyl)tyrosyl-3-(2-iminoimidazolidin-4-yl)seryl-3-[3-hexopyranosyl-2-imino-1-(3-methyl-but-2-enyl)imidazolidin-4-yl]serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-[1-heptyl-2-imino-3-α-D-mannopyranosyl-imidazolidin-4-yl]-D-seryl-L-seryl];
Cyclo[glycyl-β-methylphenylalanyl-O-(4-O-hexopyranosylhexopyranosyl)tyrosyl-3-(2-iminoimidazolidin-4-yl)seryl-3-[1-heptyl-3-hexopyranosyl-2-iminoimidazolidin-4-yl]serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-[1-(10-carboxydecyl)-2-imino-3-α-D-mannopyranosylimidazolidin-4-yl]-D-seryl-L-seryl];
Cyclo[glycyl-β-methylphenylalanyl-O-(4-O-hexopyranosylhexopyranosyl)tyrosyl-3-(2-iminoimidazolidin-4-yl)seryl-3-[1-(10-carboxydecyl)-3-hexopyranosyl-2-iminoimidazolidin-4-yl]serylseryl];
Di-N-(10-carboxydecyl)-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Di-N-(10-carboxydecyl)-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
Cyclo[3-[1,3-dibenzyl-2-(benzylimino)imidazolidin-4-yl]-L-alanyl-3-[1-benzyl-2-(benzylimino)-3-α-D-mannopyranosylimidazolidin-4-yl]-D-alanyl-L-serylglycyl-(*S*)-β-methyl-L-phenylalanyl-O-(4-O-α-D-mannopyranosyl-α-D-mannopyranosyl)-D-tyrosyl];
Cyclo[3-[1,3-benzyl-2-(benzylimino)imidazolidin-4-yl]alanyl-3-[1-benzyl-2-(benzylimino)-3-hexopyranosylimidazolidin-4-yl]alanylserylglycyl-β-methylphenylalanyl-O-(4-O-hexopyranosylhexopyranosyl)tyrosyl];
Cyclo[3-[1,3-dimethyl-2-(methylimino)imidazolidin-4-yl]-L-alanyl-3-[3-α-D-mannopyranosyl-1-methyl-2-(methylimino)imidazolidin-4-yl]-D-alanyl-L-serylglycyl-(*S*)-β-methyl-L-phenylalanyl-O-(4-O-α-D-mannopyranosyl-α-D-mannopyranosyl)-D-tyrosyl] bis-methiodide
Cyclo[3-[1,3-dimethyl-2-(methylimino)imidazolidin-4-yl]alanyl-3-[3-hexopyranosyl-1-methyl-2-(methylimino)imidazolidin-4-yl]alanylserylglycyl-β-methylphenylalanyl-O-(4-O-hexopyranosylhexopyranosyl)tyrosyl] bis-methiodide
Cyclo[glycyl-(*S*)-β-methyl-L-phenylalanyl-O-methyl-D-tyrosyl-3-[1,3-dimethyl-2-(methylimino)imidazolidin-4-yl]-L-seryl-3-[3-α-D-mannopyranosyl-1-methyl-2-(methylimino)imidazolidin-4-yl]-D-seryl-L-seryl] bis-methiodide
Cyclo[glycyl-β-methylphenylalanyl-O-(methyl)tyrosyl-3-(1,3-dimethyl-2-(methylimino)imidazolidin-4-yl)seryl-3-[3-hexopyranosyl-1-methyl-2-(methylimino)imidazolidin-4-yl]serylseryl] bis-methiodide
Cyclo[glycyl-(*S*)-β-methyl-L-phenylalanyl-O-[4-O-[4,6-O-(2-adamantylidene)-α-D-mannopyranosyl]-α-D-mannopyranosyl]-D-tyrosyl-3-(2-iminoimidazolidin-4-yl)-L-seryl-3-[1-benzyl-2-imino-3-α-D-mannopyranosyl-imidazolidin-4-yl]-D-seryl-L-seryl]
Cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-[1-benzyl-3-hexopyranosyl-2-iminoimidazolidin-4-yl]serylseryl]

Cyclo[glycyl-(*S*)-β-methyl-L-phenylalanyl-O-[4-O-[4,6-O-(2-adamantylidene)-α-D-mannopyranosyl]-α-D-mannopyranosyl]-D-tyrosyl-3-(2-iminoimidazolidin-4-yl)-L-seryl-3-[1-(4-tert-butylbenzyl)-2-imino-3-α-D-mannopyranosylimidazolidin-4-yl]-D-seryl-L-seryl]
Cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-[2-[1-(4-tert-butylbenzyl)-3-hexopyranosyl-2-iminoimidazolidin-4-yl]serylseryl]
Cyclo[glycyl-(*S*)-β-methyl-L-phenylalanyl-O-[4-O-[4,6-O-(2-adamantylidene)-α-D-mannopyranosyl]-α-D-mannopyranosyl]-D-tyrosyl-3-(2-iminoimidazolidin-4-yl)-L-seryl-3-[2-imino-3-α-D-mannopyranosyl-1-(2-naphthylmethyl)-imidazolidin-4-yl]-D-seryl-L-seryl]
Cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-[3-hexopyranosyl-2-imino-1-(2-naphthylmethyl)-imidazolidin-4-yl]serylseryl]
Cyclo[glycyl-(*S*)-β-methyl-L-phenylalanyl-O-[4-O-[4,6-O-(2-adamantylidene)-α-D-mannopyranosyl]-α-D-mannopyranosyl]-D-tyrosyl-3-[1,3-dibenzyl-2-(benzylimino)imidazolidin-4-yl]-L-seryl-3-[1-benzyl-2-(benzylimino)-3-α-D-mannopyranosylimidazolidin-4-yl]-D-seryl-L-seryl];
Cyclo[glycyl-β-methylphenylalanyl-O-[4-O-[4,6-O-(2-adamantylidene)hexopyranosyl]hexopyranosyl]tyrosyl-3-[1,3-dibenzyl-2-(benzylimino)imidazolidin-4-yl]seryl-3-[1-benzyl-2-(benzylimino)-3-hexopyranosylimidazolidin-4-yl]serylseryl];
Cyclo[3-(2-iminoimidazolidin-4-yl)-L-alanyl-3-(1-benzyl-2-imino-3-α-D-mannopyranosyl-imidazolidin-4-yl)-D-alanyl-L-serylglycyl-(*S*)-β-methyl-L-phenylalanyl-O-[4-O-[4,6-O-(2-adamantylidene)-α-D-mannopyranosyl]-α-D-mannopyranosyl]-D-tyrosyl];
Cyclo[3-(2-iminoimidazolidin-4-yl)alanyl-3-(1-benzyl-3-hexopyranosyl-2-iminoimidazolidin-4-yl)alanylserylglycyl-β-methylphenylalanyl-O-[4-O-[4,6-O-(2-adamantylidene)hexopyranosyl]hexopyranosyl]tyrosyl];
Cyclo[glycyl-(*S*)-β-methyl-L-phenylalanyl-O-[4-O-[4,6-O-(2-adamantylidene)-α-D-mannopyranosyl]-α-D-mannopyranosyl]-D-tyrosyl-3-[1,3-dimethyl-2-(methylimino)imidazolidin-4-yl]-L-seryl-3-[3-α-D-mannopyranosyl-1-methyl-2-(methylimino)imidazolidin-4-yl]-D-seryl-L-seryl] bis-methiodide
Cyclo[glycyl-β-methylphenylalanyl-O-[4-O-[4,6-O-(2-adamantylidene)hexopyranosyl]hexopyranosyl]tyrosyl-3-[1,3-dimethyl-2-(methylimino)imidazolidin-4-yl]seryl-3-[3-hexopyranosyl-1-methyl-2-(methylimino)imidazolidin-4-yl]serylseryl] bis-methiodide
Cyclo[glycyl-(*S*)-β-methyl-L-phenylalanyl-O-[4-O-[4,6-(2-adamantylidene)-α-D-mannopyranosyl]-α-D-mannopyranosyl]-D-tyrosyl-3-(2-iminoimidazolidin-4-yl)-L-seryl-3-[1-[(2*E*)-3,7-dimethylocta-2,6-dien-1-yl]-2-imino-3-α-D-mannopyranosylimidazolidin-4-yl]-D-seryl-L-seryl]
Cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-[1-(3,7-dimethylocta-2,6-dien-1-yl)-3-hexopyranosyl-2-iminoimidazolidin-4-yl]serylseryl]
Cyclo[glycyl-(*S*)-β-methyl-L-phenylalanyl-O-[4-O-[4,6-O-(2-adamantylidene)-α-D-mannopyranosyl]-α-D-mannopyranosyl]-D-tyrosyl-3-(2-iminoimidazolidin-4-yl)-L-seryl-3-[1-heptyl-2-imino-3-α-D-mannopyranosyl-imidazolidin-4-yl]-D-seryl-L-seryl]
Cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-[1-heptyl-3-hexopyranosyl-2-iminoimidazolidin-4-yl]serylseryl]
Cyclo[glycyl-(*S*)-β-methyl-L-phenylalanyl-O-[4-O-[4,6-O-(2-adamantylidene)-α-D-mannopyranosyl]-α-D-mannopyranosyl]-D-tyrosyl-3-(2-iminoimidazolidin-4-yl)-L-seryl-3-[1-hexyl-2-imino-3-α-D-mannopyranosylimidazolidin-4-yl]-D-seryl-L-seryl]
Cyclo[glycyl-β-methylphenylalanyl-O-[4-O-[4,6-O-(2-adamantylidene)hexopyranosyl]-hexopyranosyl]tyrosyl-3-(2-iminoimidazolidin-4-yl)seryl-3-[3-hexopyranosyl-l-hexyl-2-iminoimidazolidin-4-yl]serylseryl]
Cyclo[3-[2-[2-(α-D-glucopyranosyloxy)phenyl]-1,3-benzoxazol-5-yl]-D-alanyl-3-(2-iminoimidazolidin-4-yl)-L-seryl-3-(1-benzyl-2-imino-3-α-D-mannopyranosylimidazolidin-4-yl)-D-seryl-L-serylglycyl-(*S*)-β-methyl-L-phenylalanyl];
Cyclo[3-[2-[2-(hexopyranosyloxy)phenyl]-1,3-benzoxazol-5-yl]alanyl-3-(2-iminoimidazolidin-4-yl)seryl-3-(1-benzyl-3-hexopyranosyl-2-iminoimidazolidin-4-yl)serylserylglycyl-β-methyl-phenylalanyl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-decanoylimino-3-α-D-mannopyranosylimidazolidin-4-yl)-D-seryl-L-seryl];
Cyclo[glycyl-β-methylphenylalanyl-O-(4-O-hexopyranosylhexopyranosyl)tyrosyl-3-(2-iminoimidazolidin-4-yl)seryl-3-(2-decanoylimino-3-hexopyranosylimidazolidin-4-yl)serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(3-β-D-mannopyranosyl-2-(3-methyl-butyrylimino)-imidazolidin-4-yl)-D-seryl-L-seryl];
Cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-(3-methyl-butyrylimino)-imidazolidin-4-yl)serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-(2-ethyl-butyrylimino)-3-α-D-mannopyranosylimidazolidin-4-yl)-D-seryl-L-seryl];
Cyclo[glycyl-β-methylphenylalanyl-O-(4-O-hexopyranosylhexopyranosyl)tyrosyl-3-(2-iminoimidazolidin-4-yl)seryl-3-(2-(2-ethyl-butyrylimino)-3-hexopyranosylimidazolidin-4-yl)serylseryl];
Tri-N-[(phenylmethoxy)carbonyl]-cyclo[glycyl-(*S*)-β-methyl-L-phenylalanyl-D-tyrosyl-3-(2-iminoimidazolidin-4-yl)-L-seryl-3-(2-imino-3-α-D-mannopyranosylimidazolidin-4-yl)-D-seryl-L-seryl];
Tri-N-[(phenylmethoxy)carbonyl]-cyclo[glycyl-β-methylphenylalanyltyrosyl-3-(2-iminoimidazolidin-4-yl)seryl-3-(3-hexopyranosyl-2-iminoimidazolidin-4-yl)serylseryl];
N-[(Phenylmethoxy)carbonyl]-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
N-[(Phenylmethoxy)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
Tri-N-[(phenylmethoxy)carbonyl]-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Tri-N-[(phenylmethoxy)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-(4-O-α-D-mannopyranosyl-α-D-mannopyranosyl)-D-tyrosyl-3-[2-(pyrimidin-2-ylimino)imidazolidin-4-yl]-L-seryl-3-[3-α-D-mannopyranosyl-2-imino-1-(pyrimidin-2-yl)imidazolidin-4-yl]-D-seryl-L-seryl];
Cyclo[glycyl-β-methylphenylalanyl-O-(4-O-hexopyranosylhexopyranosyl)tyrosyl-3-[2-(pyrimidin-2-ylimino)imidazolidin-4-yl]seryl-3-[3-hexopyranosyl-2-imino-1-(pyrimdin-2-yl)imidazolidin-4-yl]serylseryl];
Cyclo[glycyl-(*S*)-β-methyl-L-phenylalanyl-O-(4-O-α-D-mannopyranosyl-α-D-mannopyranosyl)-D-tyrosyl-3-[2-(1,3-benzoxazol-2-ylimino)imidazolidin-4-yl]-L-seryl-3-[1-(1,3-benzoxazol-2-yl)-2-imino-3-α-D-mannopyranosylimidazolidin-4-yl]-D-seryl-L-seryl];

Cyclo[glycyl-β-methylphenylalanyl-O-(4-O-hexopyranosylhexopyranosyl)tyrosyl-3-[2-(1,3-benzoxazol-2-ylimino)imidazolidin-4-yl]seryl-3-[1-(1,3-benzoxaxol-2-yl)-3-hexopyranosyl-2-iminoimidazolidin-4-yl]serylseryl];
Cyclo[glycyl-(*S*)-β-methyl-L-phenylalanyl-O-(4-O-α-D-mannopyranosyl-α-D-mannopyranosyl)-D-tyrosyl-3-[2-(1,3-benzothiazol-2-ylimino)imidazolidin-4-yl]-L-seryl-3-[1-(1,3-benzothiazol-2-yl-3-α-D-mannopyranosyl)-2-iminoimidazolidin-4-yl]-D-seryl-L-seryl];
Cyclo[glycyl-β-methylphenylalanyl-O-(4-O-hexopyranosylhexopyranosyl)tyrosyl-3-[2-(1,3-benzothiazol-2-ylimino)imidazolidin-4-yl]seryl-3-[1-(1,3-benzo-thiazol-2-yl)-2-imino-3-hexopyranosylimidazolidin-4-yl]serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-[3-(6-O-hexanoyl-α-D-mannopyranosyl)-2-iminoimidazolidin-4-yl]-D-seryl-L-seryl];
Cyclo[glycyl-β-methylphenylalanyl-O-(4-O-hexopyranosylhexopyranosyl)tyrosyl-3-(2-iminoimidazolidin-4-yl)seryl-3-[3-(6-O-hexanoylhexopyranosyl)-2-iminoimidazolidin-4-yl]serylseryl];
Cyclo[glycyl-(*S*)-β-methyl-L-phenylalanyl-O-(6-O-hexanoyl-4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-(6-O-hexanoyl-4-O-hexopyranosylhexopyranosyl)-tyrosyl-3-(2-iminoimidazolidin-4-yl)seryl-3-(3-hexopyranosyl-2-iminoimidazolidin-4-yl)-serylseryl];
Cyclo[glycyl-(*S*)-β-methyl-L-phenylalanyl-O-[4-O-(6-O-hexanoyl-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-(6-O-hexanoylhexopyranosyl)hexopyranosyl]-tyrosyl-3-(2-iminoimidazolidin-4-yl)seryl-3-(3-hexopyranosyl-2-iminoimidazolidin-4-yl)-serylseryl];
Cyclo[glycyl-(*S*)-β-methyl-L-phenylalanyl-O-[4-O-[6-O-(diphenylacetyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-(diphenylacetyl)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-[3-[6-O-(diphenylacetyl)-α-D-mannopyranosyl]-2-iminoimidazolidin-4-yl]-D-seryl-L-seryl];
Cyclo[glycyl-β-methylphenylalanyl-O-(4-O-hexopyranosylhexopyranosyl)tyrosyl-3-(2-iminoimidazolidin-4-yl)seryl-3-[3-[6-O-(diphenylacetyl)hexopyranosyl]-2-iminoimidazolidin-4-yl]-serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-O-(diphenylacetyl)-L-seryl];
Cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)seryl-O-(diphenylacetyl)-seryl];
Cyclo[glycyl-(*S*)-β-methyl-L-phenylalanyl-O-[6-O-(diphenylacetyl)-4-O-α-D-mannopyranosyl-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[6-O-(diphenylacetyl)-4-O-hexopyranosylhexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-[3-(6-O-heptanoyl-α-D-mannopyranosyl)-2-iminoimidazolidin-4-yl]-D-seryl-L-seryl];
Cyclo[glycyl-β-methylphenylalanyl-O-(4-O-hexopyranosylhexopyranosyl)tyrosyl-3-(2-iminoimidazolidin-4-yl)seryl-3-[3-(6-O-heptanoylhexopyranosyl)-2-iminoimidazolidin-4-yl]seryl-seryl];
Cyclo[glycyl-(*S*)-β-methyl-L-phenylalanyl-O-(6-O-heptanoyl-4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-(6-O-heptanoyl-4-O-hexopyranosylhexopyxanosyl)-tyrosyl-3-(2-iminoimidazolidin-4-yl)seryl-3-(3-hexopyranosyl-2-iminoimidazolidin-4-yl)-serylseryl];
Cyclo[glycyl-(*S*)-β-methyl-L-phenylalanyl-O-[4-O-(6-O-heptanoyl-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-(6-O-heptanoylhexopyranosyl)hexopyranosyl]-tyrosyl-3-(2-iminoimidazolidin-4-yl)seryl-3-(3-hexopyranosyl-2-immoimidazolidin-4-yl)-serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-(6-O-(phenylacetyl)-α-D-mannopyranosylimidazolidin-4-yl)-D-seryl-L-seryl];
Cyclo[glycyl-β-methylphenylalanyl-O-(4-O-hexopyranosylhexopyranosyl)tyrosyl-3-(2-iminoimidazolidin-4-yl)seryl-3-(3-(6-O-(phenylacetyl)hexopyranosyl-2-iminoimidazolidin-4-yl-serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-O-(phenyacetyl)-L-seryl];
Cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl-seryl-O-(phenylacetyl)seryl];
Cyclo[glycyl-(S)-β-methyl-L-phenylalanyl-O-[4-O-α-D-mannopyranosyl-6-O-(phenylacetyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-hexopyranosyl-6-O-(phenylacetyl)hexopyranosyl]-tyrosyl-3-(2-iminoimidazolidin-4-yl)seryl-3-(3-hexopyranosyl-2-iminoimidazolidin-4-yl-serylseryl];
Cyclo[glycyl-(*S*)-β-methyl-L-phenylalanyl-O-[4-O-[6-O-(phenylacetyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-(phenylacetyl)hexopyranosyl]hexopyranosyl]-tyrosyl-3-(2-iminoimidazolidin-4-yl)seryl-3-(3-hexopyranosyl-2-iminoimidazolidin-4-yl)-serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-[6-O-(2-propylpentanoyl)-α-D-mannopyranosyl]-imidazolidin-4-yl)-D-seryl-L-seryl];
Cyclo[glycyl-β-methylphenylalanyl-O-[4-O-[6-O-(2-propylpentanoyl)hexopyranosyl]-hexopyranosyl]tyrosyl-3-(2-iminoimidazolidin-4-yl)seryl-3-[3-(6-O-(2-propylpentanoyl)-hexopyranosyl)-2-iminoimidazolidin-4-yl]serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-O-(2-propylpentanoyl)-L-seryl];
Cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)seryl-O-(2-propylpentanoyl)-seryl];

Cyclo[glycyl-(*S*)-β-methyl-L-phenylalanyl-O-[4-O-α-D-mannopyranosyl-6-O-(2-propyl-pentanoyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-hexopyranosyl-6-O-(2-propylpentanoyl)hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-(2-propylpentanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-(2-propylpentanoyl)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-[2-imino-3-(6-O-(3-cyclopentylpropanoyl)-α-D-mannopyranosyl)-imidazolidin-4-yl]-D-seryl-L-seryl];
Cyclo[glycyl-β-methylphenylalanyl-O-(4-O-hexopyranosylhexopyranosyl)tyrosyl-3-(2-iminoimidazolidin-4-yl)seryl-3-[3-(6-O-(3-cyclopentylpropanoyl)-hexopyranosyl)-2-iminoimidazolidin-4-yl]serylseryl];
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-O-(3-cyclopentylpropanoyl)-L-seryl];
Cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)seryl-O-(3-cyclopentylpropanoyl)seryl];
Cyclo[glycyl-(*S*)-β-methyl-L-phenylalanyl-O-[6-O-(3-cyclopentylpropanoyl)-4-O-α-D-mannopyranosyl-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[6-O-(3-cyclopentylpropanoyl)-4-O-hexopyranosyl-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-(3-cyclopentylpropanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-(3-cyclopentylpropanoyl)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-α-D-mannopyranosyl-6-O-[(phenylmethoxy)-carbonyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-hexopyranosyl-6-O-[(phenylmethoxy)carbonyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-[(phenylmethoxy)carbonyl]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-[(phenylmethoxy)carbonyl]hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-(4,6-O-phenethylidene-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-(4,6-O-phenethylidenehexopyranosyl)-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(4-methoxyphenethylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(4-methoxyphenethylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-(4,6-O-benzylidene-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-(4,6-O-benzylidenehexopyranosyl)hexopyranosyl]-tyrosyl-3-(2-iminoimidazolidin-4-yl)seryl-3-(3-hexopyranosyl-2-iminoimidazolidin-4-yl)-serylseryl];
Cyclo[glycyl-(*S*)-β-methyl-L-phenylalanyl-O-[4-O-(2,3-O-benzylidene-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-(2,3-O-benzylidenehexopyranosyl)hexopyranosyl]-tyrosyl-3-(2-iminoimidazolidin-4-yl)seryl-3-(3-hexopyranosyl-2-iminoimidazolidin-4-yl)-serylseryl];
Cyclo[glycyl-(*S*)-β-methyl-L-phenylalanyl-O-[4-O-(2,3-O-benzylidene-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-(2,3-O-benzylidenehexopyranosyl)hexopyranosyl]-tyrosyl-3-(2-iminoimidazolidin-4-yl)seryl-3-(3-hexopyranosyl-2-iminoimidazolidin-4-yl)-serylseryl];
Cyclo[glycyl-(*S*)-β-methyl-L-phenylalanyl-O-[4-O-[4,6-O-(2-adamantylidene)-α-D-mannopyranosyl]-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[3-cyclohexyl-L-alanyl-O-[4-O-[4,6-O-(2-adamantylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-serylglycyl];
Cyclo[3-cyclohexylalanyl-O-[4-O-[4,6-O-(2-adamantylidene)hexopyranosyl]hexopyranosyl]-tyrosyl-3-(2-iminoimidazolidin-4-yl)seryl-3-(3-hexopyranosyl-2-iminoimidazolidin-4-yl)-serylserylglycyl];
Cyclo[3-cyclohexyl-L-alanyl-O-[4-O-[2,3-O-(2-adamantylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-serylglycyl];
Cyclo[3-cyclohexylalanyl-O-[4-O-[2,3-O-(2-adamantylidene)hexopyranosyl]hexopyranosyl]-tyrosyl-3-(2-iminoimidazolidin-4-yl)seryl-3-(3-hexopyranosyl-2-iminoimidazolidin-4-yl)-serylserylglycyl];
Cyclo[3-cyclohexyl-L-alanyl-O-[4-O-[2,3:4,6-di-O-(2-adamantylidene)-α-D-mannopyranosyl]-α-D-man-nopyranosyl]-D-tyrosyl-3-(2-iminoimidazolidin-4-yl)-L-seryl-3-(2-imino-3-α-D-mannopyranosylimidazolidin-4-yl)-D-seryl-L-serylglycyl];
Cyclo[3-cyclohexylalanyl-O-[4-O-[2,3:4,6-di-O-(2-adamantylidene)hexopyranosyl]hexopyranosyl]tyrosyl-3-(2-iminoimidazolidin-4-yl)seryl-3-(3-hexopyranosyl-2-iminoimidazolidin-4-yl)-serylserylglycyl];
Cyclo[glycyl-(*S*)-β-methyl-L-phenylalanyl-O-[4-O-[4,6-O-(3-methylbutylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(3-methylbutylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-(4,6-O-hexylidene-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-(4,6-O-hexylidenehexopyranosyl)hexopyranosyl]-tyrosyl-3-(2-iminoimidazolidin-4-yl)seryl-3-(3-hexopyranosyl-2-iminoimidazolidin-4-yl)-serylseryl];
Cyclo[glycyl-(*S*)-β-methyl-L-phenylalanyl-O-[4-O-(4,6-O-octylidene-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-(4,6-O-octylidenehexopyranosyl)hexopyranosyl]-tyrosyl-3-(2-iminoimidazolidin-4-yl)seryl-3-(3-hexopyranosyl-2-iminoimidazolidin-4-yl)-serylseryl];
Cyclo[glycyl-(*S*)-β-methyl-L-phenylalanyl-O-[4-O-[4,6-O-(3,3-dimethylbutylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];

Cyclo[glycyl-β-methylphenylalanyl-O-[4-O-[4,6-O-(3,3-dimethylbutylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(2-methylpentylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(2-methylpentylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(cyclohexylmethylene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(cyclohexylmethylene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[2-[1-[(phenylmethoxy)carbonyl]-piperidin-4-yl]ethylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[2-[1-[(phenylmethoxy)carbonyl]piperidin-4-yl]ethylidene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(4-butoxy-4-oxobutylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(4-butoxy-4-oxobutylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(cyclohex-3-en-1-ylmethylene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(cyclohex-3-en-1-ylmethylene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(2-ethylbutylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(2-ethylbutylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(cyclopentylmethylene)α-mannopyranosyl]α-mannopyranosyl]-tyrosyl-3-(2-iminoimidazolidin-4-yl)-seryl-3-(2-imino-3αDmannopyranosylimidazolidin-4-yl)-seryl-seryl];
Cyclo[glycyl-β-methylphenylalanyl-O-[4-O-[4,6-O-(cyclopentylmethylene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(4-bromophenethylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryll;
Cyclo[glycyl-β-methylphenylalanyl-O-[4-O-[4,6-O-(4-bromophenethylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
CycIo[glycyl-(*S*)-β-methyl-L-phenylalanyl-O-[4-O-[4,6-O-(4-methylphenethylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(4-methylphenethylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(4-chlorophenethylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(4-chlorophenethylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(4-fluorophenethylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(4-fluorophenethylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(2-cyclohexylethylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(2-cyclohexylethylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(5-methylhex-4-en-1-ylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(5-methylhex-4-en-1-ylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(3-phenylpropylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(3-phenylpropylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(2,6-dimethylhept-5-en-1-ylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(2,6-dimethylhept-5-en-1-yidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(3,7-dimethyloct-6-en-1-ylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(3,7-dimethyloct-6-en-1-ylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(1-adamantylmethylene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(1-adamantylmethylene)hexopyranosyl]-hexopyranosyl]tyrosyl-3-(2-iminoimidazolidin-4-yl)seryl-3-(3-hexopyranosyl-2-iminoimidazolidin-4-yl)serylseryl];

Cyclo[glycyl-(*S*)-β-methyl-L-phenylalanyl-O-[4-O-[2,3-O-[2-(1-adamantyl)ethylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[2,3-O-[2-(1-adamantyl)ethylidene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[2-(1-adamantyl)ethylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[2-(1-adamantyl)ethylidene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(3α-methoxy-5β-cholan-24-ylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(3-methoxycholan-24-ylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-(4,6-O-(4-phenylbenzylidene)-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-(4,6-O-(4-phenylbenzylidene)-hexopyranosyl)hexopyranosyl]-tyrosyl-3-(2-iminoimidazolidin-4-yl)seryl-3-(3-hexopyranosyl-2-iminoimidazolidin-4-yl)-serylseryl];
Cyclo[glycyl-(*S*)-β-methyl-L-phenylalanyl-O-[4-O-[4,6-O-(2-thienylmethylene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(2-thienylmethylene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(3-thienylmethylene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(3-thienylmethylene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(4-methylbenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(4-methylbenzylidene)hexopyranosyl]-hexopyranosyl]-tyrosyl-3-(2-iminoimidazolidin-4-yl)seryl-3-(3-hexopyranosyl-2-iminoimidazolidin-4-yl)-serylseryl];
Cyclo[glycyl-(*S*)-β-methyl-L-phenylalanyl-O-[4-O-[4,6-O-(3-methylbenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(3-methylbenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(3-hydroxybenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(3-hydroxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(3-methoxybenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(3-methoxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[2,3-O-(3-methoxybenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[2,3-O-(3-methoxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[2,3-O-(3-methoxybenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[2,3-O-(3-methoxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(3-chlorobenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(3-chlorobenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(4-isopropylbenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(4-isopropylbenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(4-propylbenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(4-propylbenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(4-carboxybenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(4-carboxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-(4-O-[4,6-O-(4-ethoxybenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(4-ethoxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];

Cyclo[glycyl-(*S*)-β-methyl-L-phenylalanyl-O-[4-O-[4,6-O-(1,3-benzodioxol-5-ylmethylene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(1,3-benzodioxol-5-ylmethylene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[4-(methylthio)benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[4-(methylthio)benzylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(2-naphthylmethylene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(2-naphthylmethylene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[3-(2-methylprop-1-en-1-yl)-benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glyeyl-β-methylphenylalanyl-O-[4-O-[4,6-O-[3-(2-methylprop-1-en-1-yl)benzylidene]-hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(4-tert-butylbenzylidene)-α-D-mannopyranosyl]-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(4-tert-butylbenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(4-propyloxybenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(4-propyloxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[(2,3-dihydro-1,4-benzodioxan-6-yl)-methylene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[(2,3-dihydro-1,4-benzodioxan-6-yl)-methylene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(3,5-dimethoxybenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(3,5-dimethoxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[2,3-O-(3,5-dimethoxybenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[2,3-O-(3,5-dimethoxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(3-methyl-4-nitrobenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(3-methyl-4-nitrobenzylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(3-ethoxy-4-methoxybenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(3-ethoxy-4-methoxybenzylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(3-methoxy-4-nitrobenzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(3-methoxy-4-nitrobenzylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(3-phenylbenzylidene)-a-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(3-phenylbenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];

cyclo[glycyl-(*S*)-β-methyl-L-phenylalanyl-O-(4-O-α-D-mannopyranosyl-α-D-mannopyranosyl)-D-tyrosyl-3-[2-[(2,2-dimethylpropanoyl)imino]imidazolidin-4-yl]-L-seryl-3-(2-imino-3-α-D-mannopyranosylimidazolidin-4-yl)-D-seryl-L-seryl];
Cyclo[glycyl-β-methylphenylalanyl-O-(4-O-hexopyranosylhexopyranosyl)tyrosyl-3-[2-[(2,2-dimethylpropanoyl)imino]imidazolidin-4-yl]seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[3-(3-pyridyl)benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[3-(3-pyridyl)benzylidene]hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[4-(methylsulfonyl)benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[4-(methylsulfonyl)benzylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[(6-chloro-1,3-benzodioxol-5-yl)-methylene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[(6-chloro-1,3-benzodioxol-5-yl)-methylene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[(6-methoxy-2-naphthyl)-methylene]-a-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[(6-methoxy-2-naphthyl)-methylene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[2,3-O-[(6-methoxy-2-naphthyl)-methylene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[2,3-O-[(6-methoxy-2-naphthyl)-methylene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[(1-acetylindol-3-yl)methylene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[1-(acetylindol-3-yl)methylene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[3-(3-thienyl)benzylidene]-α-D-mannopyranosyl]-α-D-mannopyranosyl]-D-tyrosyl-3-(2-iminoiniidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[3-(3-thienyl)benzylidene]hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[4-(2-thienyl)benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[4-(2-thienyl)benzylidene]hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[4-(3-thienyl)benzylidene]-α-D-mannopyranosyl]-a-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[4-(3-thienyl)benzylidene]hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[2,3-O-[4-(3-thienyl)benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[2,3-O-[4-(3-thienyl)benzylidene]hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[3-[(3-methylbut-2-en-1-yl)oxy]-benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[3-[(3-methylbut-2-en-1-yl)oxy]-benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[3-(carboxymethoxy)benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[3-(carboxymethoxy)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(3-ethoxy-4-mtrobenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(3-ethoxy-4-nitrobenzylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[4-(methylthio)-3-nitrobenzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[4-(methylthio)-3-nitrobenzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(4-hydroxy-3-methoxy-5-nitro-benzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(4-hydroxy-3-methoxy-4-nitrobenzylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(3-phenoxybenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];

Cyclo[glycyl-β-methylphenylalanyl-O-[4-O-[4,6-O-(3-phenoxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(4-phenoxybenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(4-phenoxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[4-[(*E*)-2-phenylethenyl]benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[4-(2-phenylethenyl)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(4-benzoylbenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(4-benzoylbenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[4-(4-methylphenoxy)benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[4-(4-methylphenoxy)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[3-(phenylmethoxy)benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[3-(phenylmethoxy)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[4-(phenylmethoxy)benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[4-(phenylmethoxy)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[3-(4-chlorophenyl)benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[3-(4-chlorophenyl)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[4-(4-chloropheny)benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-α-methylphenylalanyl-O-[4-O-[4,6-O-[4-(4-chlorophenyl)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[3-(1,1,2,2-tetrafluoroethoxy)-benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[3-(1,1,2,2-tetrafluoroethoxy)benzylidene]-hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[3-(4-methoxyphenoxy)benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[3-(4-methoxyphenoxy)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[4-(4-tert-butylphenoxy)benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[4-(4-tert-butylphenoxy)benzylidene]hexopyranosyl]hexopyranosyl]tyrosyl-3-(2-iminoimidazolidin-4-yl)seryl-3-(3-hexopyranosyl-2-iminoimidazolidin-4-yl)seryiseryl]
Cyclo[glycyl-(*S*)-β-methyl-L-phenylalanyl-O-[4-O-[4,6-O-[3-[(4-nitrophenyl)methoxy]benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[3-[(4-nitrophenyl)methoxy]benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[[4-[(2*E*)-3,7-dimethylocta-2,6-dien-1-yl]-oxy]benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[4-[(3,7-dimethylocta-2,6-dien-1-yl)oxy]-benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[3-[3-(trifluoromethyl)phenoxy]-benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[3-[3-(trifluoromethyl)phenoxy]-benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[[3-[(2*E*,6*E*)-3,7,11-trimethyldodeca-2,6,10-trien-1-yl]oxy]benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[[3-(3,7,11-trimethyldodeca-2,6,10-trien-1-yl)oxy]benzylidene]hexopyranosyl]hexopyranosyl]tyrosyl-3-(2-iminoimidazolidin-4-yl)-seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[4-(4-iodobenzoyl)benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[4-(4-iodobenzoyl)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[4-(3,4,5-triiodobenzoyl)benz-ylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[4-(3,4,5-triiodobenzoyl)benzylidene]-hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(3,3-diphenylprop-2-enylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(3,3-diphenylprop-2-enylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(prop-2-enylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];

Cyclo[glycyl-β-methylphenylalanyl-O-[4-O-[4,6-O-(prop-2-enylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[3-(2-iminoimidazolidin-4-yl)-L-alanyl-3-(2-imino-3-α-D-mannopyranosylimidazolidin-4-yl)-D-alanyl-L-serylglycyl-(*S*)-β-methyl-L-phenylalanyl-O-[4-O-[4,6-O-[(6-methoxy-2-naphthyl)methylene]-α-D-mannopyranosyl]-α-D-mannopyranosyl]-D-tyrosyl];
Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[4,6-O-[(6-methoxy-2-naphthyl)-methylene]hexopyranosyl]hexopyranosyl]tyrosyl];
Cyclo[3-(2-iminoimidazolidin-4-yl)-L-alanyl-3-(2-imino-3-α-D-mannopyranosylimidazolidin-4-yl)-D-alanyl-L-serylglycyl-(*S*)-β-methyl-L-phenylalanyl-O-[4-O-[4,6-O-[4-(phenylmethoxy)-benzylidene]-α-D-mannopyranosyl]-α-D-mannopyranosyl]-D-tyrosyl];
Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[4,6-O-[4-(phenylmethoxy)benzylidene]-hexopyranosyl]hexopyranosyl]tyrosyl];
Cyclo[3-(2-iminoimidazolidin-4-yl)-D-alanyl-3-(2-imino-3-α-D-mannopyranosylimidazolidin-4-yl)-D-alanyl-L-serylglycyl-(*S*)-β-methyl-L-phenylalanyl-O-[4-O-[4,6-O-[3-(4-methoxy-phenoxy)benzylidene]-α-D-mannopyranosyl]-α-D-mannopyranosyl]-D-tyrosyl];
Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[4,6-O-[3-(4-methoxyphenoxy)benzylidene]-hexopyranosyl]hexopyranosyl]tyrosyl];
Cyclo[glycyl-(*S*)-4-chloro-β-methyl-L-phenylalanyl-O-[4-O-[4,6-O-(cyclohexylmethylene)-α-mannopyranosyl]α-mannopyranosyl]-D-tyrosyl-3-(2-iminoimidazolidin-4-yl)-L-seryl-3-(2-imino-3α-mannopyranosylimidazolidin-4-yl)-D-seryl-L-seryl];
Cyclo[glycyl-4-chloro-β-methylphenylalanyl-O-[4-O-[4,6-O-(cyclohexylmethylene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-4-chloro-β-methyl-L-phenylalanyl-O-[4-O-[4,6-O-(2-methylpentylidene)-α-mannopyranosyl]α-mannopyranosyl]-D-tyrosyl-3-(2-iminoimidazolidin-4-yl)-L-seryl-3-(2-imino-3α-mannopyranosylimidazolidin-4-yl)-D-seryl-L-seryl];
Cyclo[glycyl-4-chloro-β-methylphenylalanyl-O-[4-O-[4,6-O-(2-methylpentylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-4-chloro-β-methyl-L-phenylalanyl-O-[4-O-(4,6-O-benzylidene-β-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-4-chloro-β-methylphenylalanyl-O-[4-O-(4,6-O-benzylidenehexopyranosyl)-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-(4,6-O-benzylidene-α-D-mannopyranosyl)-α-D-mannopyranosyl]-3-iodo-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-(4,6-O-benzylidenehexopyranosyl)hexopyranosyl]-3-iodotyrosyl-3-(2-iminoimidazolidin-4-yl)seryl-3-(3-hexopyranosyl-2-iminoimidazolidin-4-yl)-serylseryl];
Cyclo[glycyl-(*S*)-β-methyl-L-phenylalanyl-O-[4-O-[4,6-O-[3-(4-methylphenoxy)benzylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-[3-(4-methylphenoxy)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[3-cyclohexyl-L-alanyl-O-[4-O-[4,6-O-(3-phenylpropylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-serylglycyl];
Cyclo[3-cyclohexylalanyl-O-[4-O-[4,6-O-(3-phenylpropylidene)hexopyranosyl]hexopyranosyl]tyrosyl-3-(2-iminoimidazolidin-4-yl)seryl-3-(3-hexopyranosyl-2-iminoimidazolidin-4-yl)serylserylglycyl];
Cyclo[3-cyclohexyl-L-alanyl-O-[4-O-(4,6-O-benzylidene-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-serylglycyl];
Cyclo[3-cyclohexylalanyl-O-[4-O-(4,6-O-benzylidenehexopyranosyl)hexopyranosyl]tyrosyl-3-(2-iminoimidazolidin-4-yl)seryl-3-(3-hexopyranosyl-2-iminoimidazolidin-4-yl)-seryl-serylglycyl];
Cyclo[3-cyclohexyl-L-alanyl-O-[4-O-[4,6-O-phenethylidene-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-serylglycyl];
Cyclo[3-cyclohexylalanyl-O-[4-O-[4,6-O-phenethylidene-hexopyranosyl]hexopyranosyl]tyrosyl-3-(2-iminoimidazolidin-4-yl)seryl-3-(3-hexopyranosyl-2-iminoimidazolidin-4-yl)serylserylglycyl];
Cyclo[(3*S*)-3-cyclohexyl-L-2-aminobutanoyl-O-[4-O-[4,6-O-benzylidene-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-serylglycyl];
Cyclo[3-cyclohexyl-2-aminobutanoyl-O-[4-O-[4,6-O-benzylidenehexopyranosyl]hexopyranosyl]tyrosyl-3-(2-iminoimidazolidin-4-yl)seryl-3-(3-hexopyranosyl-2-iminoimidazolidin-4-yl)serylserylglycyl];
Cyclo[glycyl-(*S*)-4-chloro-β-methyl-L-phenylalanyl-O-[4-O-(4,6-O-cyclopentylidene-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-4-chloro-β-methylphenylalanyl-O-[4-O-(4,6-O-cyclopentylidenehexopyranosyl)-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-(4,6-O-cyclohexylidene-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-(4,6-O-cyclohexylidene-4-O-hexopyranosyl)hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(3*S*)-β-methyl-L-phenylalanyl-O-[4-O-[4,6-O-(2-methylcyclohexylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(2-methylcyclohexylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(2,2-dimethylcyclohexylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(2,2-dimethylcyclohexylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyc1o[glycyl-(*S*)-β-methyl-L-phenylalanyl-O-[4-O-[4,6-O-(2,6-dimethylcyclohexylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(2,6-dimethylcyclohexylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(3,3,5,5-tetramethylcyclohexylidene)-α-D-mannopyranosyl]-a-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(3,3,5,5-tetramethylcyclohexylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(4-tert-butylcyclohexylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylinfidazolidin-4-yl)-D-seryl-L-seryl];
Cyclo[glycyl-β-methylphenylalanyl-O-[4-O-[4,6-O-(4-tert-butylcyclohexylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-(4,6-O-cyclododecylidene-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];

Cyclo[glycyl-β-methylphenylalanyl-O-[4-O-(4,6-O-cyclododecylidenehexopyranosyl)-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-(4,6-O-cyclotridecylidene-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-(4,6-O-cyclotridecylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-(2,3-O-cyclotridecylidene-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-(2,3-O-cyclotridecylidenehexopyranosyl)hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(bicylo-[3.2.1]oct-2-ylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl] ;
Cyclo[glycyl-β-methylphenylalanyl-O-[4-O-[4,6-O-(bicylo-[3.2.1]oct-2-ylidene)-hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-(4,6-O-bicyclo[3.3.1]non-9-ylidene-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl] ;
Cyclo[glycyl-β-methylphenylalanyl-O-[4-O-(4,6-O-bicyclo[3.3.1]non-9-ylidenehexopyranosyl)hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-(4,6-O-tricyclo[5.2.1.0(2,6)]decan-2-ylidene-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-(4,6-O-tricyclo[5.2.1.0(2,6)]decan-2-ylidenehexopyranosyl)hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[(1R,4R)-1,7,7-trimethylbicyclo[2.2.1]hept-2-ylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(1,7,7-trimethylbicyclo[2.2.1]hept-2-ylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-[(1S,4S)-1,7,7-trimethylbicyclo[2.2.1]hept-2-ylidene]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(1,7,7-trimethylbicyclo[2.2.1]hept-2-ylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(4-fluoro-α-methylbenzylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(4-fluoro-α-methylbenzylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(tetrahydrothiopyran-4-ylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4,6-O-(tetrahydrothiopyran-4-ylidene)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4,6-O-(2-adamantylidene)-3-O-(3-methyl-butanoyl)-α-D-mannopyranosyl]-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl] ;
Cyclo[glycyl-β-methylphenylalanyl-O-[4-O-[4,6-O-(2-adamantylidene)-3-O-(3-methyl-butanoyl)-hexopyranosyl]hexopyranosyl]tyrosyl-3-(2-iminoimidazolidin-4-yl)-seryl-3-(3-hexopyranosyl-2-iminoimidazolidin-4-yl)serylseryl];
Cyclo[3-(2-iminoimidazolidin-4-yl)-L-alanyl-3-(2-imino-3-α-D-mannopyranosylimidazolidin-4-yl)-D-alanyl-L-serylglycyl-(*S*)-β-methyl-L-phenylalanyl-O-[4-O-[4,6-O-(2-adamantylidene)-α-D-mannopyranosyl]-α-D-mannopyranosyl]-D-tyrosyl];
Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[4,6-O-(2-adamantylidene)hexopyranosyl]-hexopyranosyl]tyrosyl];
Cyclo[glycyl-L-phenylalanyl-O-[4-O-[4,6-O-(2-adamantylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycylphenylalanyl-O-[4-O-[4,6-O-(2-adamantylidene)hexopyranosyl]hexopyranosyl]-tyrosyl-3-(2-iminoimidazolidin-4-yl)seryl-3-(3-hexopyranosyl-2-iminoimidazolidin-4-yl]-serylseryl];
Cyclo[(3S)-3-cyclohexyl-L-2-aminobutanoyl-O-[4-O-[4,6-O-(2-adamantylidene)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-serylglycyl];
Cyclo[3-cyclohexyl-2-aminobutanoyl-O-[4-O-[4,6-O-(2-adamantylidene)hexopyranosyl]hexopyranosyl]tyrosyl-3-(2-iminoimidazolidin-4-yl)seryl-3-(3-hexopyranosyl-2-iminoimidazolidin-4-yl)serylserylglycyl];
Cyclo[3-cyclohexyl-L-alanyl-3-[cis-4-[[4-O-[4,6-O-(2-adamantylidene)-α-D-mannopyranosyl]-α-D-mannopyranosyl]oxy]cyclohexyl]-D-alanyl-3-(2-iminoimidazolidin-4-yl)-L-seryl-3-(2-imino-3-α-D-mannopyranosylimidazolidin-4-yl)-D-seryl-L-serylglycyl];
Cyclo[3-cyclohexylalanyl-3-[4-[[4-O-[4,6-O-(2-adamantylidene)hexopyranosyl]hexopyranosyl]oxy]cyclohexyl]alanyl-3-(2-iminoimidazolidin-4-yl)seryl-3-(3-hexopyranosyl-2-iminoimidazolidin-4-yl)serylserylglycyl];
Cyclo[3-cyclohexyl-L-alanyl-3-[trans-4-[[4-O-[4,6-O-(2-adamantylidene)-α-D-mannopyranosyl]-α-D-mannopyranosyl]oxy]cyclohexyl]-D-alanyl-3-(2-iminoimidazolidin-4-yl)-L-seryl-3-(2-imino-3-α-D-mannopyranosylimidazolidin-4-yl)-D-seryl-L-serylglycyl];
Cyclo[3-cyclohexylalanyl-3-[4-[[4-O-[4,6-O-(2-adamantylidene)hexopyranosyl]hexopyranosyl]oxy]cyclohexyl]alanyl-3-(2-iminoimidazolidin-4-yl)seryl-3-(3-hexopyranosyl-2-iminoimidazolidin-4-yl)serylserylglycyl];
Cyclo[glycyl-(*S*)-β-methyl-L-phenylalanyl-O-[4-O-[4,6-O-(2-adamantylidene)-β-D-mannopyranosyl]-α-D-mannopyranosyl]-D-tyrosyl-3-(2-iminoimidazolidin-4-yl)-L-seryl-3-(1-butyl-2-imino-3-α-D-mannopyranosylimidazolidin-4-yl)-D-seryl-L-seryl];
Cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-(1-butyl-3-hexopyranosyl-2-iminoimidazolidin-4-yl)serylseryl];
Cyclo[glycyl-(*S*)-β-methyl-L-phenylalanyl-O-[4-O-[4,6-O-(2-adamantylidene)-α-D-mannopyranosyl]-α-D-mannopyranosyl]-D-tyrosyl-3-(2-iminoimidazolidin-4-yl)-L-seryl-3-[2-imino-3-α-D-mannopyranosyl-1-(3-methylbut-2-en-1-yl)imidazolidin-4-yl]-D-seryl-L-seryl];
Cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-[3-hexopyranosyl-2-imino-1-(3-methylbut-2-en-1-yl)imidazolidin-4-yl]serylseryl];
Cyclo[glycyl-(*S*)-β-methyl-L-phenylalanyl-O-[4-O-[4,6-O-(2-adamantylidene)-α-D-mannopyranosyl]-α-D-mannopyranosyl]-D-tyrosyl-3-(2-iminoimidazolidin-4-yl)-L-seryl-3-[2-imino-3-α-D-mannopyranosyl-1-[4-(trifluoromethyl)benzyl]imidazolidin-4-yl]-D-seryl-L-seryl]
Cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-[3-hexopyranosyl-2-imino-1-[4-(trifluoromethyl)benzyl]imidazolidin-4-yl]serylseryl]
Di-N-(2-(1,3-benzoxazolyl))-cyclo[glycyl-(*S*)-β-methyl-L-phenylalanyl-O-[4-O-[4,6-O-(2-adamantylidene)-α-D-mannopyranosyl]-α-D-mannopyranosyl]-D-tyrosyl-3-[2-iminoimidazolidin-4-yl]-L-seryl-3-[2-imino-3-α-D-mannopyranosylimidazolidin-4-yl]-D-seryl-L-seryl]
Di-N-(2-(1,3-benzoxazolyl))-cyclo[glycyl-β-methylphenylalanyl-O-[4-O-[4,6-O-(2-adamantylidene)hexopyranosyl]hexopyranosyl]tyrosyl-3-[2-iminoimidazolidin-4-yl]seryl-3-[3-hexopyranosyl-2-iminoimidazolidin-4-yl]serylseryl]
Di-N-(2-pyrimidinyl)-cyclo[glycyl-(*S*)-β-methyl-L-phenylalanyl-O-[4-O-[4,6-O-(2-adamantylidene)-α-D-mannopyranosyl]-α-D-mannopyranosyl]-D-tyrosyl-3-[2-immoimidazolidin-4-yl]-L-seryl-3-[2-imino-3-a-D-mannopyranosyl-imidazolidin-4-yl]-D-seryl-L-seryl]

Di-N-(2-pyrimidinyl)-cyclo[glycyl-β-methylphenylalanyl-O-[4-O-[4,6-O-(2-adamantylidene)hexopyranosyl]hexopyranosyl]tyrosyl-3-[2-iminoimidazolidin-4-yl]seryl-3-[3-hexopyranosyl-2-iminoimidazolidin-4-yl]serylseryl]
Cyclo[glycyl-(*S*)-β-methyl-L-phenylalanyl-O-(2,3-O-isopropylidene-4-O-α-D-mannopyranosyl-α-D-mannopyranosyl)-D-tyrosyl-3-(2-iminoimidazolidin-4-yl)-L-seryl-3-[2-imino-3-(2,3-O-isopropylidene-α-D-mannopyranosyl)imidazolidin-4-yl]-D-seryl-L-seryl];
Cyclo[glycyl-β-methylphenylalanyl-O-(2,3-O-isopropylidene-4-O-hexopyranosylhexopyranosyl)tyrosyl-3-(2-iminoimidazolidin-4-yl)seryl-3-[2-imino-(2,3-O-isopropylidene-hexopyranosyl)imidazolidin-4-yl]serylseryl];
Cyclo[glycyl-(*S*)-β-methyl-L-phenylalanyl-O-[2,3-O-isopropylidene-4-O-(2,3-isopropylidene-α-D-mannopyranosyl)-α-D-mannopyranosyl]-D-tyrosyl-3-(2-iminoimidazolidin-4-yl)-L-seryl-3-[2-imino-3-(2,3-O-isopropylidene-α-D-mannopyranosyl)imidazolidin-4-y]-D-seryl-L-seryl];
Cyclo[glycyl-β-methylphenylalanyl-O-[2,3-O-isopropylidene-4-O-(2,3-isopropylidenehexopyranosyl)hexopyranosyl]tyrosyl-3-(2-iminoimidazolidin-4-yl)seryl-3-[2-imino-3-(2,3-O-isopropylidenehexopyranosyl)imidazolidin-4-yl]serylseryl];
Cyclo[glycyl-(*S*)-β-methyl-L-phenylalanyl-O-[2,3-O-isopropylidene-4-O-(2,3:4,6-di-O-iso-propylidene-α-D-mannopyranosyl)-α-D-mannopyranosyl]-D-tyrosyl-3-(2-iminoimidazolidin-4-yl)-L-seryl-3-[2-imino-3-(2,3-O-isopropylidene-α-D-mannopyranosyl)imidazolidin-4-yl]-D-seryl-L-seryl];
Cyclo[glycyl-β-methylphenylalanyl-O-[2,3-O-isopropylidene-4-O-(2,3:4,6-di-O-isopropylidene-hexopyranosyl)hexopyranosyl]tyrosyl-3-(2-iminoimidazolidin-4-yl)seryl-3-[2-imino-(2,3-O-isopropylidenehexopyranosyl)imidazolidin-4-yl]serylseryl];
Cyclo[glycyl-(*S*)-β-methyl-L-phenylalanyl-O-[4-O-[6-O-[(6-methoxy-2-naphthyl)methyl]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-[(6-methoxy-2-naphthyl)methyl]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4-O-[(6-methoxy-2-naphthyl)methyl]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4-O-[(6-methoxy-2-naphthyl)methyl]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[3-O-[(6-methoxy-2-naphthyl)methyl]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[3-O-[(6-methoxy-2-naphthyl)methyl]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[2-O-[(6-methoxy-2-naphthyl)methyl]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[2-O-[(6-methoxy-2-naphthyl)methyl]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-[4-(phenylmethoxy)benzyl]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-[4-(phenylmethoxy)benzyl]hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4-O-[4-(phenylinethoxy)benzyl]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4-O-[4-(phenylmethoxy)benzyl]hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[2-O-[4-(phenylmethoxy)benzyl]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[2-O-[4-(phenylmethoxy)benzyl]hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[3-O-[4-(phenylmethoxy)benzyl]-α-D-mannopyranosyll-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[3-O-[4-(phenylmethoxy)benzyl]hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-(4-methoxybenzyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-(4-methoxybenzyl)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-(4-ethoxybenzyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-(4-ethoxybenzyl)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-(4-methylbenzyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-(4-methylbenzyl)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-(4-propoxybenzyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo(glycyl-β-methylphenylalanyl-O-[4-O-[6-O-(4-propoxybenzyl)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-(4-phenoxybenzyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-(4-phenoxybenzyl)hexopyranosyl]hexopyranosyl]tyrosyl-3-(2-iminoimidazolidin-4-yl)seryl-3-(3-hexopyranosyl-2-iminoimidazolidin-4-yl)serylseryl];

Cyclo[glycyl-(*S*)-β-methyl-L-phenylalanyl-O-[4-O-[6-O-(3-fluoro-4-methoxybenzyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-(3-fluoro-4-methoxybenzyl)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-(3-ethoxy-4-methoxybenzyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-(3-ethoxy-4-methoxybenzyl)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-[4-methoxy-3-(phenylmethoxy)-benzyl]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-[4-methoxy-3-(phenylmethoxy)benzyl]-hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4-O-[4-methoxy-3-(phenylmethoxy)-benzyl]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4-O-[4-methoxy-3-(phenylmethoxy)benzyl]-hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-[(4-methoxy-1-naphthyl)methyl]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-[(4-methoxy-1-naphthyl)methyl]hexo-pyranosy]lhexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4-O-[(4-methoxy-1-naphthyl)methyl]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4-O-[(4-methoxy-1-naphthyl)methyl]hexo-pyranosy]lhexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[2-O-[(4-methoxy-1-naphthyl)methyl]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[2-O-[(4-methoxy-1-naphthyl)methyl]hexo-pyranosy]lhexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-(3,3-diphenylprop-2-en-1-yl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryll;
Cyclo[glycyl-β-methylphenylalanyl-O-[4-O-[6-O-(3,3-diphenylprop-2-en-1-yl)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4-O-(3,3-diphenylprop-2-en-1-yl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4-O-(3,3-diphenylprop-2-en-1-yl)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-[(2*E*)-3-phenylprop-2-en-1-yl]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-(3-phenylprop-2-en-1-yl)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-[3-[4-(dimethylamino)phenyl](2*E*)-prop-2-en-1-yl]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-[3-[4-(dimethylamino)phenyl]prop-2-en-1-yl]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4-O-[3-[4-(dimethylamino)phenyl](2*E*)-prop-2-en-l-yl]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4-O-[3-[4-(dimethylamino)phenyl]prop-2-en-1-yl]hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-(2-thienylmethyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-(2-thienylmethyl)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[3-(2-iminoimidazolidin-4-yl)-L-alanyl-3-(2-imino-3-α-D-mannopyranosylimidazolidin-4-yl)-D-alanyl-L-serylglycyl-(*S*)-β-methyl-L-phenylalanyl-O-[4-O-[6-O-[(6-methoxy-2-naphthyl)methyl]-α-D-mannopyranosyl]-α-D-mannopyranosyl]-D-tyrosyl];
Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[6-O-[(6-methoxy-2-naphthyl)methyl]-hexopyranosyl]hexopyranosyl]tyrosyl];
Cyclo[3-(2-iminoimidazolidin-4-yl)-L-alanyl-3-(2-imino-3-α-D-mannopyranosylimidazolidin-4-yl)-D-alanyl-L-serylglycyl-(*S*)-β-methyl-L-phenylalanyl-O-[4-O-[4-O-[(6-methoxy-2-naphthyl)methyl]-α-D-mannopyranosyl]-α-D-mannopyranosyl]-D-tyrosyl];
Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[4-O-[(6-methoxy-2-naphthyl)methyl]-hexopyranosyl]hexopyranosyl]tyrosyl];
Cyclo[3-(2-iminoimidazolidin-4-yl)-L-alanyl-3-(2-imino-3-α-D-mannopyranosylimidazolidin-4-yl)-D-alanyl-L-serylglycyl-(*S*)-β-methyl-L-phenylalanyl-O-[4-O-[3-O-[(6-methoxy-2-naphthyl)methyl]-α-D-mannopyranosyl]-α-D-mannopyranosyl]-D-tyrosyl];
Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[3-O-[(6-methoxy-2-naphthyl)methyl]-hexopyranosyl]hexopyranosyl]tyrosyl];
Cyclo[3-(2-iminoimidazolidin-4-yl)-L-alanyl-3-(2-imino-3-α-D-mannopyranosylimidazolidin-4-yl)-D-alanyl-L-serylglycyl-(*S*)-β-methyl-L-phenylalanyl-O-[4-O-[2-O-[(6-methoxy-2-naphthyl)methyl]-α-D-mannopyranosyl]-α-D-mannopyranosyl]-D-tyrosyl];

Cyclo[3-(2-iminoimidazolidin-4-yl)alanyl-3-(3-hexopyranosyl-2-iminoimidazolidin-4-yl)-alanylserylglycyl-β-methylphenylalanyl-O[4-O-[2-O-[(6-methoxy-2-naphthyl)methyl]-hexopyranosyl]hexopyranosyl]tyrosyl];
Cyclo[3-(2-iminoimidazolidin-4-yl)-L-alanyl-3-(2-imino-3-α-D-mannopyranosylimidazolidin-4-yl)-D-alanyl-L-serylglycyl-(*S*)-β-methyl-L-phenylalanyl-O-[4-O-[6-O-[4-(phenylmethoxy)-benzyl]-α-D-mannopyranosyl]-α-D-mannopyranosyl]-D-tyrosyl];
Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[6-O-[4-(phenylmethoxy)benzyl]hexopyranosyl]hexopyranosyl]tyrosyl];
Cyclo[3-(2-iminoimidazolidin-4-yl)-L-alanyl-3-(2-imino-3-α-D-mannopyranosylimidazolidin-4-yl)-D-alanyl-L-serylglycyl-(*S*)-β-methyl-L-phenylalanyl-O-[4-O-[4-O-[4-(phenylmethoxy)-benzyl]-α-D-mannopyranosyl]-α-D-mannopyranosyl]-D-tyrosyl];
Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[4-O-[4-(phenylmethoxy)benzyl]hexopyranosyl]hexopyranosyl]tyrosyl];
Cyclo[3-(2-iminoimidazolidin-4-yl)-L-alanyl-3-(2-imino-3-α-D-mannopyranosylimidazolidin-4-yl)-D-alanyl-L-serylglycyl-(*S*)-β-methyl-L-phenylalanyl-O-[4-O-[3-O-[4-(phenylmethoxy)-benzyl]-α-D-mannopyranosyl]-α-D-mannopyranosyl]-D-tyrosyl];
Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[3-O-[4-(phenylmethoxy)benzyl]hexopyranosyl]hexopyranosyl]tyrosyl];
Cyclo[3-(2-iminoimidazolidin-4-yl)-L-alanyl-3-(2-imino-3-α-D-mannopyranosylimidazolidin-4-yl)-D-alanyl-L-serylglycyl-(*S*)-β-methyl-L-phenylalanyl-O-[4-O-[2-O-[4-(phenylmethoxy)-benzyl]-α-D-mannopyranosyl]-α-D-mannopyranosyl]-D-tyrosyl];
Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[2-O-[4-(phenylmethoxy)benzyl]hexopyranosyl]hexopyranosyl]tyrosyl];
Cyclo[glycyl-(*S*)-β-methyl-L-phenylalanyl-D-tyrosyl-3-(2-iminoimidazolidin-4-yl)-L-seryl-3-(2,3:4,6-diisopropylidene-α-D-mannopyranosyl)-2-iminoimidazolidin-4-yl-D-seryl-L-seryl];
Cyclo[glycyl-β-methylphonylalanyltyrosyl-3-(2-iminoimidazolidin-4-yl)seryl-3-(2,3:4,6-diisopropylidenehexopyranosyl)-2-iminoimidazolidin-4-ylserylseryl];
Cyclo[glycyl-(*S*)-β-methyl-L-phenylalanyl-D-tyrosyl-3-(2-iminoimidazolidin-4-yl)-L-seryl-3-[3-(2,3:4,6-di-O-isopropylidene-α-D-mannopyranosyl)-2-iminoimidazolidin-4-yl]-D-seryl-O-(tert-butyldimethylsilyl)-L-seryl];
Cyclo[glycyl-β-methylphenylalanyltyrosyl-3-(2-iminoimidazolidin-4-yl)seryl-3-[3-(2,3:4,6-di-O-isopropylidenehexopyranosyl)-2-iminoimidazolidin-4-yl]seryl-O-(tertbutyldimethylsilyl)seryl];
Tri-N-[(phenylmethoxy)carbonyl]-cyclo[glycyl-(*S*)-β-methyl-L-phenylalanyl-D-tyrosyl-3-(2-iminoimidazolidin-4-yl)-L-seryl-3-[3-(2,3:4,6-di-O-isopropylidene-α-D-mannopyranosyl)-2-iminoimidazolidin-4-yl]-D-seryl-O-(tert-butyldimethylsilyl)-L-seryl];
Tri-N-[(phenylmethoxy)carbonyl]-cyclo[glycyl-β-methylphenylalanyltyrosyl-3-(2-iminoimidazolidin-4-yl)seryl-3-[3-(2,3:4,6-di-O-isopropylidenehexopyranosyl)-2-iminoimidazolidin-4-yl]seryl-O-(tert-butyldimethylsilyl)seryl];
Tetra-N-[(phenylmethoxy)carbonyl]-cyclo[glycyl-(*S*)-β-methyl-L-phenylalanyl-D-tyrosyl-3-(2-iminoimidazolidin-4-yl)-L-seryl-3-(2-iminoimidazolidin-4-yl)-D-seryl-O-(tert-butyldimethylsilyl)-L-seryl];
Tetra-N-[(phenylmethoxy)carbonyl]-cyclo[glycyl-β-methylphenylalamyktyrosyl-3-(2-iminoimidazolidin-4-yl)seryl-3-(2-iminoimidazolidin-4-yl)seryl-O-(tertbutyldimethylsilyl)seryl];
α-O-[(Pentylamino)carbonyl]-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
α-O-[(Pentylaimno)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
β-O-[(Pentylamino)carbonyl]-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
β-O-[(Pentylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
γ-O-[(Pentylamino)carbonyl]-cyclo[glycyl-(*S*)-β-methyl-L-phenyla]anyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
γ-O-[(Pentylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
α-O-[(Butylamino)carbonyl]-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
α-O-[(Butylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
β-O-[(Butylamino)carbonyl]-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
β-O-[(Butylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
α-O-[(Heptylamino)carbonyl]-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
α-O-[(Heptylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
β-O-[(Heptylamino)carbonyl]-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
β-O-[(Heptylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
γ-O-[(Heptylamino)carbonyl]-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
γ-O-[(Heptylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
α-O-[(Dodecylamino)carbonyl]-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
α-O-[(Dodecylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];

β-O-[(Dodecylamino)carbonyl]-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
β-O-[(Dodecylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
γ-O-[(Dodecylamino)carbonyl]-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
γ-O-[(Dodecylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
α-O-[(N-Methyl-2-methylpropylamino)carbonyl]-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
α-O-[(N-Methyl-2-methylpropylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
β-O-[(N-Methyl-2-methylpropylamino)carbonyl]-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
β-O-[(N-Methyl-2-methylpropylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
α-O-[(Cyclohexylamino)carbonyl]-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
α-O-[(Cyclohexylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
β-O-[(Cyclohexylamino)carbonyl]-cyclo[glycyl-(*S*)-β-methyl-L-phenylalanyl-O-(4-O-α-D-mannopyranosyl-β-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
β-O-[(Cyclohexylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
α-O-[(Benzylamino)carbonyl]-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
α-O-[(Benzylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
β-O-[(Benzylamino)carbonyl]-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
β-O-[(Benzylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
γ-O-[(Benzylamino)carbonyl]-cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
γ-O-[(Benzylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
Cyclo[glycyl-(*S*)-4-fluoro-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-4-fluoro-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]
Cyclo[glycyl-(*S*)-4-fluoro-β-methyl-L-phenylalanyl-O-[4-O-[2-O-(3-methylbutanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-4-fluoro-β-methylphenylalanyl-O-[4-O-[2-O-(3-methylbutanoyl)hexopyranosyl]-hexopyranosyl]tyrosyl-3-(2-iminoimidazolidin-4-yl)seryl-3-(3-hexopyranosyl-2-imino-imidazolidin-4-yl)serylseryl]
Cyclo[glycyl-(*S*)-4-fluoro-β-methyl-L-phenylalanyl-O-[4-O-[3-O-(3-methylbutanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-4-fluoro-β-methylphenylalanyl-O-[4-O-[3-O-(3-methylbutanoyl)hexopyranosyl]-hexopyranosyl]tyrosyl-3-(2-iminoimidazolidin-4-yl)seryl-3-(3-hexopyranosyl-2-iminoimidazolidin-4-yl)serylseryl]
Cyclo[glycyl-(*S*)-4-chloro-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-4-chloro-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
Cyclo[glycyl-(*S*)-4-chloro-β-methyl-L-phenylalanyl-O-[4-O-[3-O-(3-methylbutanoyl)α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-4-chloro-β-methylphenylalanyl-O-[4-O-[3-O-(3-methylbutanoyl)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-4-chloro-β-methyl-L-phenylalanyl-O-[4-O-[2-O-(3-methylbutanoyl)α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-4-chloro-β-methylphenylalanyl-O-[4-O-[2-O-(3-methylbutanoyl)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[3-cyclohexyl-L-alanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-serylglycyl];
Cyclo[3-cyclohexylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylserylglycyl]
Cyclo[glycyl-(*S*)-3-fluoro-β-methyl-L-phenylalanyl-O-[4-O-[2-O-(3-methylbutanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-3-fluoro-β-methylphenylalanyl-O-[4-O-[2-O-(3-methylbutanoyl)hexopyranosyl]-hexopyranosyl]tyrosyl-3-(2-iminoimidazolidin-4-yl)seryl-3-(3-hexopyranosyl-2-iminoimidazolidin-4-yl)serylseryl]

Cyclo[glycyl-(*S*)-3-fluoro-β-methyl-L-phenylalanyl-O-[4-O-[3-O-(3-methylbutanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-3-fluoro-β-methylphenylalanyl-O-[4-O-[3-O-(3-methylbutanoyl)hexopyranosyl]-hexopyranosyl]tyrosyl-3-(2-iminoimidazolidin-4-yl)seryl-3-(3-hexopyranosyl-2-iminoimidazolidin-4-yl)serylseryl]
Cyclo[glycyl-(*S*)-3-fluoro-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-3-fluoro-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]
Cyclo[(3*R*)-3-(2-thienyl)-L-2-aminobutanoyl-O-[4-O-[2-O-(3-methylbutanoyl)α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[3-(2-thienyl)-2-aminobutanoyl-O-[4-O-[2-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylserylglycyl];
Cyclo[(3*R*)-3-(2-thienyl)-L-2-aminobutanoyl-O-[4-O-[3-O-(3-methylbutanoyl)α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[3-(2-thienyl)-2-aminobutanoyl-O-[4-O-[3-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylserylglycyl]
Cyclo[(3*R*)-3-(2-thienyl)-L-2-aminobutanoyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[3-(2-thienyl)-2-aminobutanoyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylserylglycyl];
Cyclo[glycyl-(*S*)-β-methyl-L-phenylalanyl-3-fluoro-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-3-fluoro-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]
Cyclo[glycyl-(*S*)-β-methyl-L-phenylalanyl-3-fluoro-O-[4-O-[2-O-(3-methylbutanoyl)-α-D-mannopyranosyl]-β-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-3-fluoro-O-[4-O-[2-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-3-fluoro-O-[4-O-[3-O-(3-methylbutanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-3-fluoro-O-[4-O-[3-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-3-amino-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-3-amino-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-3-amino-O-[4-O-[2-O-(3-methylbutanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-3-amino-O-[4-O-[2-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-3-amino-O-[4-O-[3-O-(3-methylbutanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-3-amino-O-[4-O-[3-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-L-phenylglycyl-O-[4-O-[2-O-(3-methylbutanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-phenylglycyl-O-[4-O-[2-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-2-chloro-β-methyl-L-phenylalanyl-O-(4-O-α-D-mannopyranosyl-a-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-2-chloro-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
Cyclo[glycyl-(*S*)-2-chloro-β-methyl-L-phenylalanyl-O-[4-O-[2-O-(3-methylbutanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-2-chloro-β-methylphenylalanyl-O-[4-O-[2-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-2-chloro-β-methyl-L-phenylalanyl-O-[4-O-[3-O-(3-methylbutanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-2-chloro-β-methylphenylalanyl-O-[4-O-[3-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[2-O-(4-methylpentanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[2-O-(4-methylpentanoyl)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-acetyl-3-O-(4-methylpentanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-acetyl-3-O-(4-methylpentanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl];

Cyclo[glycyl-(*S*)-β-methyl-L-phenylalanyl-O-[4-O-[4-O-(4-methylpentanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4-O-(4-methylpentanoyl)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[3-O-(3-methylpentanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[3-O-(3-methylpentanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-acetyl-3-O-(3-methylpentanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl] ;
Cyclo[glycyl-β-methylphenylalanyl-O-[4-O-[6-O-acetyl-3-O-(3-methylpentanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[4-O-(3-methylpentanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4-O-(3-methylpentanoyl)hexopyranosyl]-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-(2-O-butanoyl-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl]; Cyclo[glycyl-β-methylphenylalanyl-O-[4-O-(2-O-butanoyl-hexopyranosyl)-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-(3-O-butanoyl-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-(3-O-butanoyl-hexopyranosyl)-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-(2-O-hexanoyl-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-(2-O-hexanoyl-hexopyranosyl)-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-(3-O-hexanoyl-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-(3-O-hexanoyl-hexopyranosyl)-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-(2-O-heptanoyl-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-(2-O-heptanoyl-hexopyranosyl)-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-(3-O-heptanoyl-α-D-mannopyranosyl)-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-(3-O-heptanoyl-hexopyranosyl)-hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-acetyl-3-O-(3-methylbutanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-acetyl-3-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-acetyl-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl]
Cyclo[glycyl-β-methylphenylalanyl-O-[4-O-[6-O-acetyl-hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[6-O-acetyl-2-O-(3-methylbutanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[6-O-acetyl-2-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[3-(2-iminoimidazolidin-4-yl)-L-alanyl-3-(2-imino-3-α-D-mannopyranosylimidazolidin-4-yl)-D-alanyl-L-serylglycyl-(*S*)-β-methyl-L-phenylalanyl-O-(4-O-α-D-mannopyranosyl-α-D-mannopyranosyl)-D-tyrosyl];
Cyclo[3-(2-iminoimidazolidin-4-yl)alanyl-3-(3-hexopyranosyl-2-iminoimidazolidin-4-yl)-alanylserylglycyl-β-methylphenylalanyl-O-(4-O-hexopyranosylhexopyranosyl)tyrosyl];
Cyclo[3-(2-iminoimidazolidin-4-yl)-L-alanyl-3-(2-imino-3-α-D-mannopyranosylimidazolidin-4-yl)-D-alanyl-L-serylglycyl-(*S*)-β-methyl-L-phenylalanyl-O-[4-O-[2-O-(3-methylbutanoyl)α-D-mannopyranosyl]-α-D-mannopyranosyl]-D-tyrosyl];
Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[2-O-(3-methylbutanoyl)hexopyranosyl]-hexopyranosyl]tyrosyl];
Cyclo[3-(2-iminoimidazolidin-4-yl)-L-alanyl-3-(2-imino-3-α-D-mannopyranosylimidazolidin-4-yl)-D-alanyl-L-serylglycyl-(*S*)-β-methyl-L-phenylalanyl-O-[4-O-[3-O-(3-methylbutanoyl)α-D-mannopyranosyl]-α-D-mannopyranosyl]-D-tyrosyl];
Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[3-O-(3-methylbutanoyl)hexopyranosyl]-hexopyranosyl]tyrosyl];
Cyclo[glycyl-(*S*)-β-methyl-L-phenylalanyl-O-[α-D-mannopyranosyl]-D-tyrosyl-3-(2-iminoimidazolidin-4-yl)-L-seryl-3-(2-iminoimidazolidin-4-yl)-D-seryl-L-seryl];
Cyclo[glycyl-β-methylphenylalanyl-O-hexopyranosyltyrosyl-3-(2-iminoimidazolidin-4-yl)-seryl-3-(2-iminoimidazolidin-4-yl)serylseryl];
Cyclo[glycyl-(*S*)-β-methyl-L-phenylalanyl-O-[4-O-[4-O-[(2*E*)-2-methylbut-2-enoyl]-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];

Cyclo[glycyl-β-methylphenylalanyl-O-[4-O-[4-O-(2-methylbut-2-enoyl)hexopyranosyl]-hexopyranosyl]tyrosyl-3-(2-iminoimidazolidin-4-yl)seryl-3-(3-hexopyranosyl-2-imino-imidazolidin-4-yl)serylseryl];
Cyclo[glycyl-(*S*)-β-methyl-L-phenylalanyl-O-[4-O-[4-O-(2-methylbutanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[4-O-(2-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[4-O-[3-O-(4-methylpentanoyl)-α-D-mannopyranosyl]-α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-[4-O-[3-O-(4-methylpentanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-L-phenylalanyl-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycylphenylalanyltyrosyl-3-(2-iminoimidazolidin-4-yl)seryl-3-(3-hexopyranosyl-2-iminoimidazolidin-4-yl)serylseryl];
Cyclo[glycyl-L-phenylalanyl-D-tyrosyl-3-(2-iminoimidazolidin-4-yl)-L-seryl-3-(2-iminoimidazolidin-4-yl)-D-seryl-L-seryl];
Cyclo[glycylphenylalanyltyrosyl-3-(2-iminoimidazolidin-4-yl)seryl-3-(2-iminoimidazolidin-4-yl)serylseryl];
Cyclo[glycyl-(*S*)-β-methyl-L-phenylalanyl-D-tyrosyl-3-(2-iminoimidazolidin-4-yl)-L-seryl-3-(2-iminoimidazolidin-4-yl)-D-seryl-L-seryl];
Cyclo[glycyl-β-methylphenylalanyltyrosyl-3-(2-iminoimidazolidin-4-yl)seryl-3-(2-iminoimidazolidin-4-yl)serylseryl];
Cyclo[glycyl-L-phenylalanyl-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycylphenylalanyl-O-(4-O-hexopyranosylhexopyranosyl)tyrosyl-3-(2-iminomidazolidin-4-yl)seryl-3-(3-hexopyranosyl-2-iminoimidazolidin-4-yl)serylseryl];
Cyclo[glycyl-L-phenylalanyl-O-(4-O-[6-O-(2-methylpropanoyl)-α-D-mannopyranosyl]-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycylphenylalanyl-O-[4-O-[6-O-(2-methylpropanoyl)-hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-L-phenylalanyl-O-(4-O-[3-O-(2-methylpropanoyl)-α-D-mannopyranosyl]-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycylphenylalanyl-O-[4-O-[3-O-(2-methylpropanoyl)-hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-L-phenylalanyl-O-(4-O-[6-O-(3-methylbutanoyl)-α-D-mannopyranosyl]-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycylphenylalanyl-O-[4-O-[6-O-(3-methylbutanoyl)-hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[3-(2-iminoimidazolidin-4-yl)-L-alanyl-3-(2-imino-3-α-D-mannopyranosylimidazolidin-4-yl)-D-seryl-L-serylglycyl-(*S*)-β-methyl-L-phenylalanyl-O-(4-O-α-D-mannopyranosyl-α-D-mannopyranosyl)-D-tyrosyl];
Cyclo[3-(2-iminoimidazolidin-4-yl)alanyl-3-(3-hexopyranosyl-2-iminoimidazolidin-4-yl)-serylserylglycyl-β-methylphenylalanyl-O-(4-O-hexopyranosylhexopyranosyl)tyrosyl];
Cyclo[3-(2-imino-3-α-D-mannopyranosylimidazolidin-4-yl)-D-alanyl-L-serylglycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl];
Cyclo[3-(3-hexopyranosyl-2-iminoimidazolidin-4-yl)-alanylserylglycyl-β-methylphenylalanyl-O-(4-O-hexopyranosylhexopyranosyl)tyrosyl-3-(2-iminoimidazolidin-4-yl)seryl];
Cyclo[3-(2-iminoimidazolidin-4-yl)-L-alanyl-3-(2-imino-3-α-D-mannopyranosylimidazolidin-4-yl)-D-alanyl-L-serylglycyl-L-phenylalanyl-O-(4-O-α-D-mannopyranosyl-α-D-mannopyranosyl)-D-tyrosyl];
Cyclo[3-(2-iminoimidazolidin-4-yl)alanyl-3-(3-hexopyranosyl-2-iminoimidazolidin-4-yl)-alanylserylglycylphenylalanyl-O-(4-O-hexopyranosylhexopyranosyl)tyrosyl]
Cyclo[glycyl-L-phenylalanyl-O-(4-O-[2-O-(3-methylbutanoyl)-α-D-mannopyranosyl]-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycylphenylalanyl-O-[4-O-[2-O-(3-methylbutanoyl)-hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-L-phenylalanyl-O-(4-O-[3-O-(3-methylbutanoyl)-α-D-mannopyranosyl]-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycylphenylalanyl-O-[4-O-[3-O-(3-methylbutanoyl)-hexopyranosyl]hexopyranosyl]tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-2-amino-O-(4-O-α-D-mannopyranosyl-α-D-mannopyranosyl)-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-2-amino-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl];
Cyclo[glycyl-(*S*)-β-methyl-L-phenylalanyl-O-[α-D-mannopyranosyl]-D-tyrosyl-3-(2-iminoimidazolidin-4-yl)-L-seryl-3-(2-imino-3-α-D-mannopyranosylimidazolidin-4-yl)-D-seryl-L-seryl];
Cyclo[glycyl-β-methylphenylalanyl-O-hexopyranosyltyrosyl-3-(2-iminoimidazolidin-4-yl)-seryl-3-(3-hexopyranosyl-2-iminoimidazolidin-4-yl)serylseryl];
Cyclo[3-cyclohexyl-L-alanyl-D-tyrosyl-3-(2-iminoimidazolidin-4-yl)-L-seryl-3-(2-imino-3-α-D-mannopyranosylimidazolidin-4-yl)-D-seryl-L-serylglycyl];
Cyclo[3-cyclohexylalanyltyrosyl-3-(2-iminoimidazolidin-4-yl)seryl-3-(3-hexopyranosyl-2-iminoimidazolidin-4-yl)serylserylglycyl];
Cyclo[3-(2-iminoimidazolidin-4-yl)-L-alanyl-3-(2-imino-3-α-D-mannopyranosylimidazolidin-4-yl)-D-alanyl-L-serylglycyl-(*S*)-β-methyl-L-phenylalanyl-D-tyrosyl];
Cyclo[3-(2-iminoimidazolidin-4-yl)alanyl-3-(3-hexopyranosyl-2-iminoimidazolidin-4-yl)-alanylserylglycyl-β-methylphenylalanyltyrosyl];
Cyclo[(3S)-3-cyclohexyl-L-2-aminobutanoyl-D-tyrosyl-3-(2-iminoimidazolidin-4-yl)-L-seryl-3-(2-imino-3-α-D-mannopyranosylimidazolidin-4-yl)-D-seryl-L-serylglycyl]; Cyclo[3-cyclohexyl-2-aminobutanoyl-tyrosyl-3-(2-iminoimidazolidin-4-yl)seryl-3-(3-hexopyranosyl-2-iminoimidazolidin-4-yl)-serylserylglycyl];
Cyclo[glycyl-(*S*)-β-methyl-L-phenylalanyl-O-(2,3-O-isopropylidene-α-D-mannopyranosyl)-D-tyrosyl-3-(2-iminoimidazolidin-4-yl)-L-seryl-3-[2-imino-3-(2,3-O-isopropylidene-α-D-mannopyranosyl)imidazolidin-4-yl]-D-seryl-L-seryl];
Cyclo[glycyl-β-methylphenylalanyl-O-(2,3-O-isopropylidene-hexopyranosyl)tyrosyl-3-(2-iminoimidazolidin-4-yl)seryl-3-[2-imino-(2,3-O-isopropylidene-hexopyranosyl)imidazolidin-4-yl]serylseryl];
Cyclo[glycyl-L-phenylalanyl-O-(4-O-α-D-mannopyranosyl-α-D-mannopyranosyl)-D-tyrosyl-3-(2-iminoimidazolidin-4-yl)-L-seryl-3-(2-iminoimidazolidin-4-yl)-D-seryl-L-seryl] ;
Cyclo[glycylphenylalanyl-O-(4-O-hexopyranosylhexopyranosyl)tyrosyl-3-(2-iminoimidazolidin-4-yl)seryl-3-(2-iminoimidazolidin-4-yl)serylseryl]
Cyclo[glycyl-(*S*)-β-methyl-L-phenylalanyl-O-(4-O-α-D-mannopyranosyl-α-D-mannopyranosyl)-D-tyrosyl-3-(2-iminoimidazolidin-4-yl)-L-seryl-3-(2-iminoimidazolidin-4-yl)-D-seryl-L-seryl];
Cyclo[glycyl-β-methylphenylalanyl-O-(4-O-hexopyranosylhexopyranosyl)tyrosyl-3-(2-iminoimidazolidin-4-yl)seryl-3-(2-iminoimidazolidin-4-yl)serylseryl].

An aspect of the invention is a process for producing a compound of the formula: 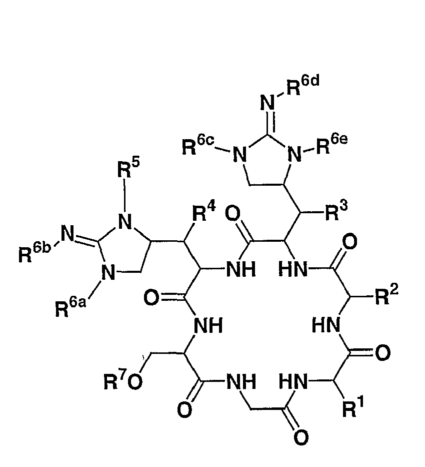 wherein:
R¹ is selected from: 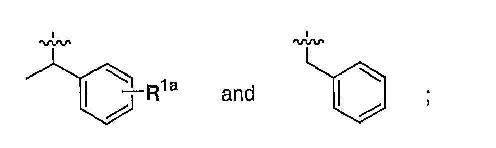
R^{1a} is H;
R² is a moiety 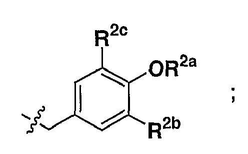
R^{2a} is selected from H, 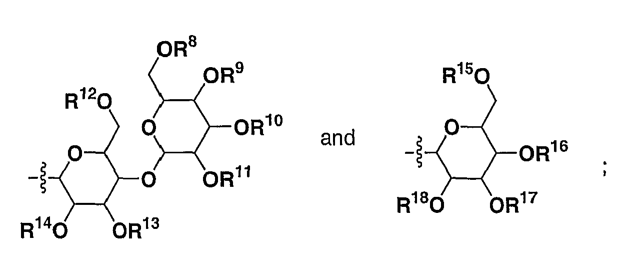
R^{2b} is H;
R^{2c} is H;
R³ and R⁴ are independently H or OH;
R⁵ is H or 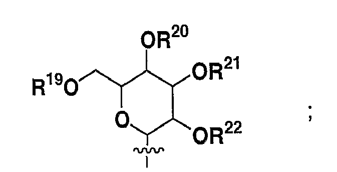
R^{6a}, R^{6b}, R^{6c}, R^{6d}, or R^{6e} are H;
R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently
H, or -C(O)-Y-Z ;
Y is a single bond ;
Z is straight or branched chain alkyl(C₁-C₂₀) or straight or branched chain alkenyl(C₂-C₂₀);
which process comprises:
   cultivating a glycopeptide antibiotic producing strain of *Streptomyces hygroscopicus* selected from the group LL4600, LL4614, LL4666, LL4690, LL4728, LL4741, LL4742, LL4744, LL4773, LL4779, LL4783, LL4902, BD2, BD20 and BD70 or a mutant thereof under aerobic conditions, in a suitable culture medium until a recoverable amount of said antibiotic is formed in said medium and recovering said antibiotic therefrom. Preferred aspects of the process include:
   a.) R⁷, R¹², R¹³, R¹⁴, R¹⁵, R¹⁶, R¹⁷, R¹⁸, R¹⁹, R²⁰, R²¹ and R²² are H; and
      Z is straight or branched chain alkyl(C₁-C₉) or straight or branched chain alkenyl(C₂-C₁₀);
   b.) R⁷, R¹², R¹³, R¹⁴, R¹⁵, R¹⁶, R¹⁷, R¹⁸, R¹⁹, R²⁰, R²¹ and R²² are H;
      Z is straight or branched chain alkyl(C₁-C₉) or straight or branched chain alkenyl(C₂-C₁₀);
      R³ and R⁴ are H;
   c.) R⁷, R¹², R¹³, R¹⁴, R¹⁵, R¹⁶, R¹⁷, R¹⁸, R¹⁹, R²⁰, R²¹ and R²² are H;
      Z is straight or branched chain alkyl(C₁-C₉) or straight or branched chain alkenyl(C₂-C₁₀); and
      R³ and R⁴ are OH; and
   d.) the glycopeptide antibiotic produced has the structure selected from the group 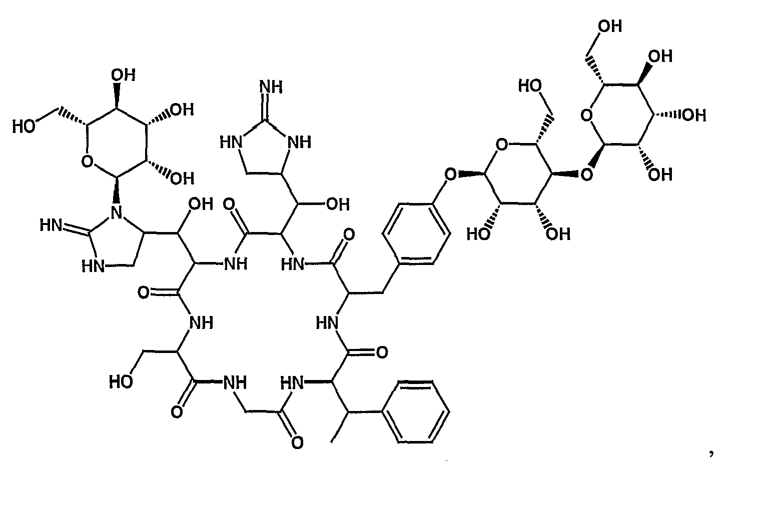 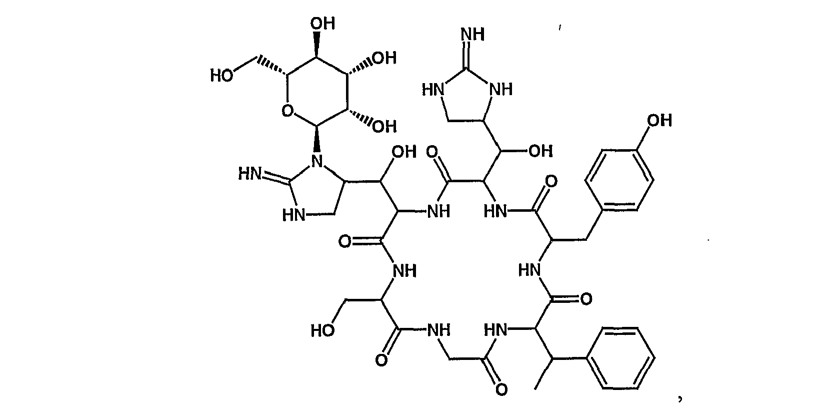 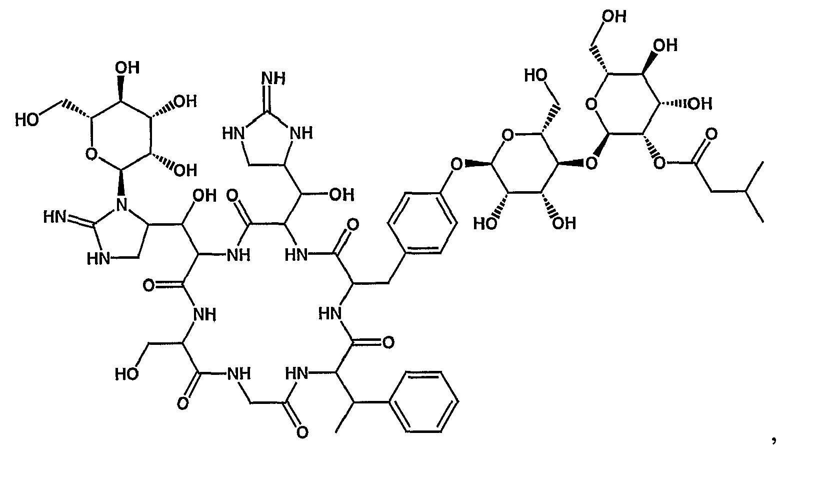 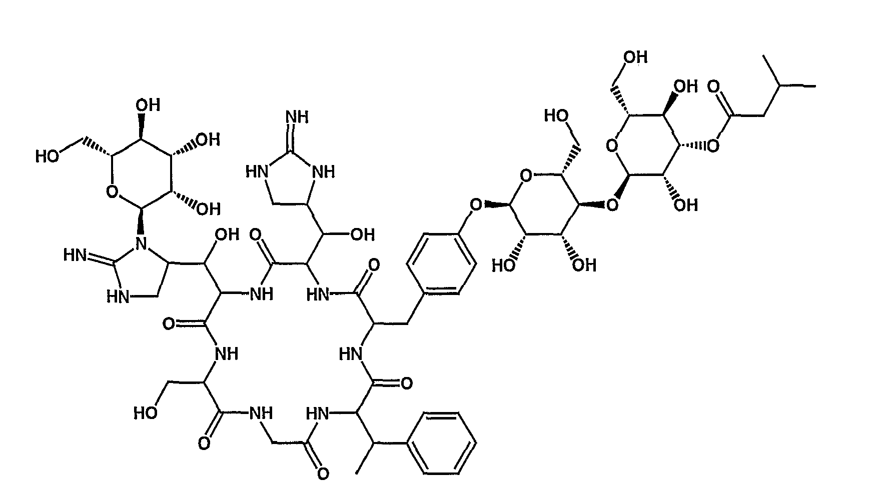 and

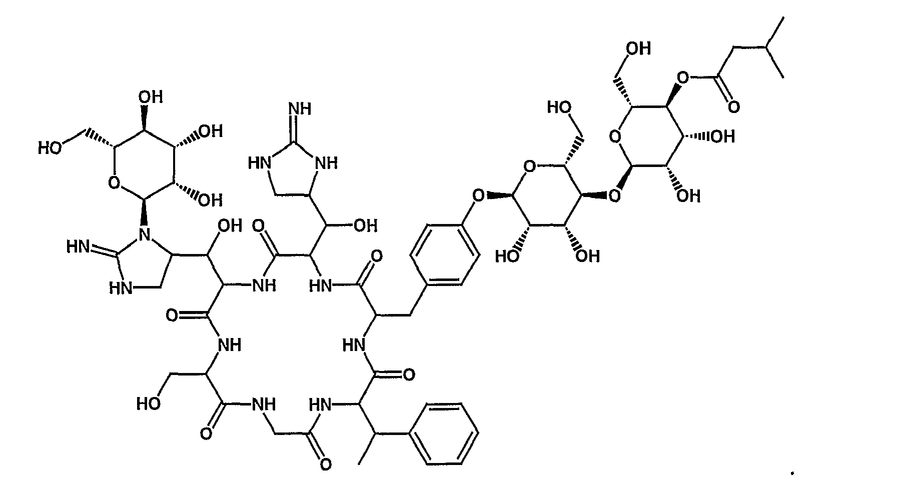

Another aspect of the invention is the preparation by fermentation means in a liquid media using modified strains of *Streptomyces hygroscopicus* selected from the group LL4600, LL4614, LL4666, LL4690, LL4728, LL4741, LL4742, LL4744, LL4773, LL4779, LL4783, LL4902, BD2, BD20 and BD70 or mutants thereof glycopeptide antibiotics of the formula 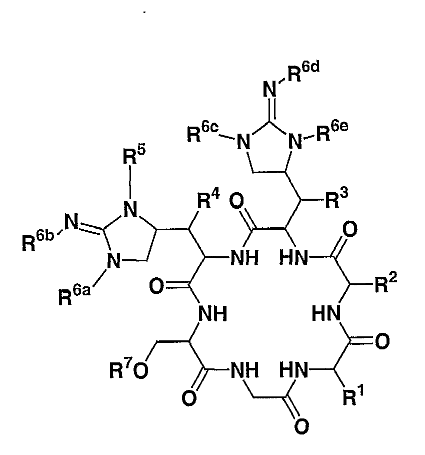 wherein:
R¹ is selected from: 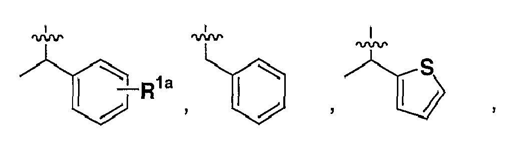 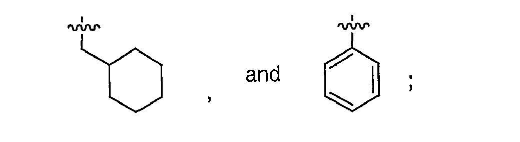
R^{1a} is H or halogen;
R² is a moiety 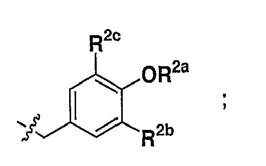
R^{2a} is selected from H, 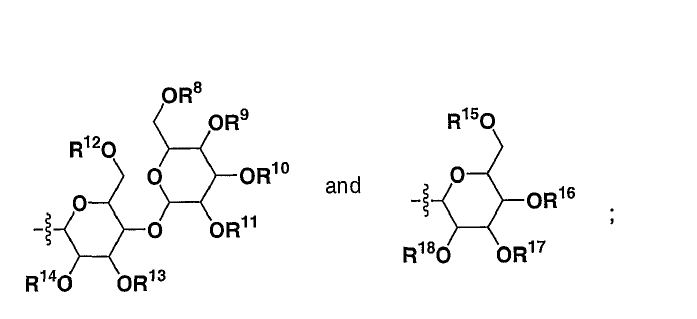
R^{2b} is H, F, or NH₂;
R^{2c} is H;
R³ and R⁴ are independently H or OH;
R⁵ is H or 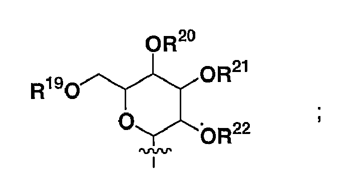
R^{6a}, R^{6b}, R^{6c}, R^{6d}, or R^{6e} are H;
R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently H, or -C(O)-Y-Z ;
Y is a single bond ;
Z is straight or branched chain alkyl(C₁-C₂₀) or straight or branched chain alkenyl(C₂-C₂₀);
by the addition of substrates of the formulae:
   straight or branched chain alkyl(C₁-C₂₀)CO₂H, straight or branched chain alkenyl(C₂-C₂₀)CO₂H, 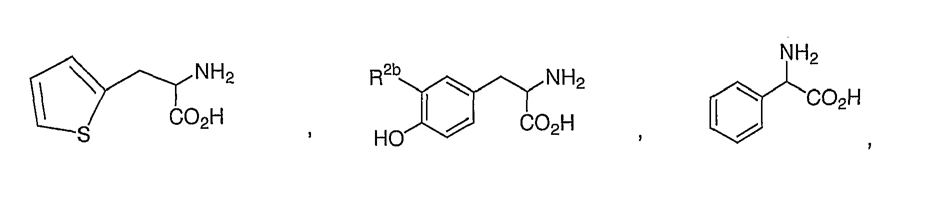 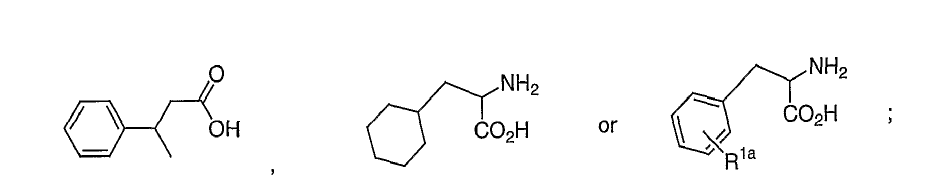 and detecting and/or recovering the antibiotic. In an additional aspect of the process multiple substrates may optionally be added. For example, straight or branched chain alkyl(C₁-C₂₀)CO₂H and 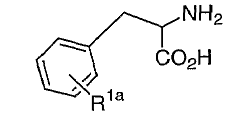 may optionally be added.
Preferred aspects of the process include:
a.) R⁷, R¹², R¹³, R¹⁴, R¹⁵, R¹⁶, R¹⁷, R¹⁸, R¹⁹, R²⁰, R²¹ and R²² are H; and
   Z is straight or branched chain alkyl(C₁-C₉) or straight or branched chain alkenyl(C₂-C₁₀);
b.) R⁷, R¹², R¹³, R¹⁴, R¹⁵, R¹⁶, R¹⁷, R¹⁸, R¹⁹, R²⁰, R²¹ and R²² are H;
   Z is straight or branched chain alkyl(C₁-C₉) or straight or branched chain alkenyl(C₂-C₁₀); and
   R³ and R⁴ are H; or
c.) R⁷, R¹², R¹³, R¹⁴, R¹⁵, R¹⁶, R¹⁷, R¹⁸, R¹⁹, R²⁰, R²¹ and R²² are H;
   Z is straight or branched chain alkyl(C₁-C₉) or straight or branched chain alkenyl(C₂-C₁₀); and
   R³ and R⁴ are OH.

A further aspect of the invention is the preparation by fermentation means in a liquid media using modified strains of *Streptomyces hygroscopicus* selected from the group LL4600, LL4614, LL4666, LL4690, LL4728, LL4741, LL4742, LL4744, LL4773, LL4779, LL4783, LL4902, BD2, BD20 and BD70 or mutants thereof glycopeptide antibiotics of the formula 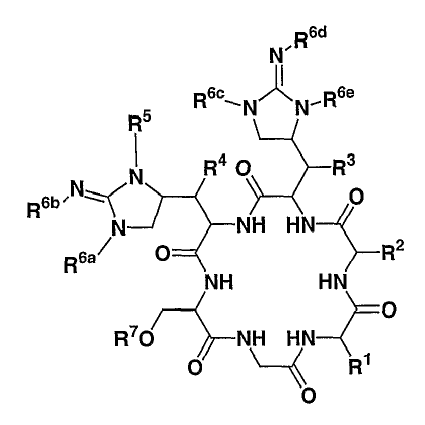 wherein:
R¹ is selected from: 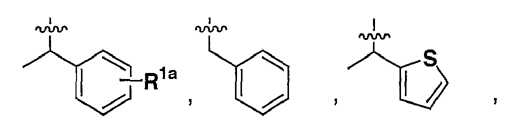 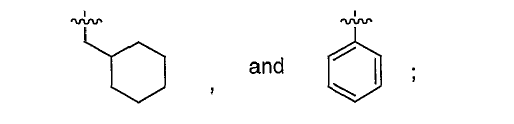
R^{1a} is H or halogen;
R² is a moiety 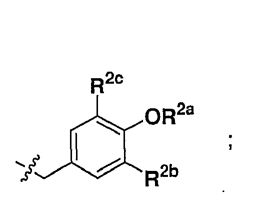
R^{2a} is selected from
H, 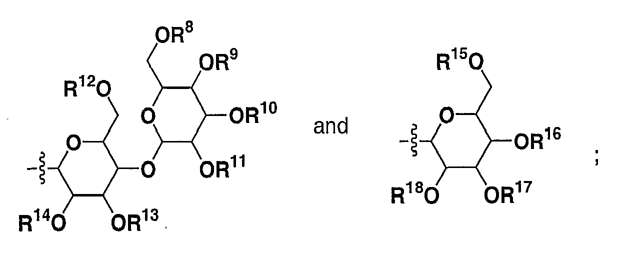
R^{2b} is H, F, or NH₂;
R^{2c} is H;
R³ and R⁴ are independently H or OH;
R⁵ is H or 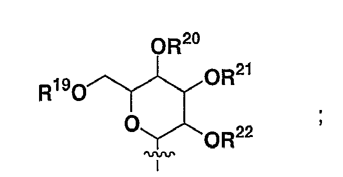
R^{6a}, R^{6b}, R^{6c}, R^{6d}, or R^{6e} are H;
R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently H, or -C(O)-Y-Z ;
Y is a single bond ;
Z is straight or branched chain alkyl(C₁-C₂₀) or straight or branched chain alkenyl(C₂-C₂₀);
by the addition of suitable substrates of the formulae: straight or branched chain alkyl(C₁-C₂₀)CO₂H, straight or branched chain alkenyl(C₂-C₂₀)CO₂H, 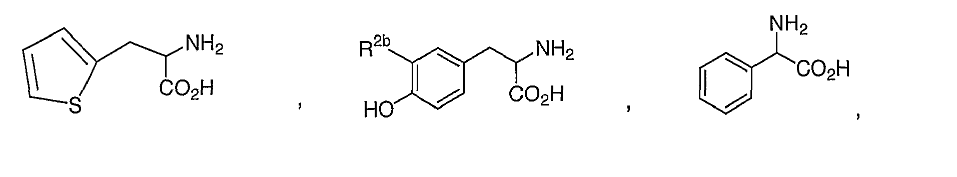 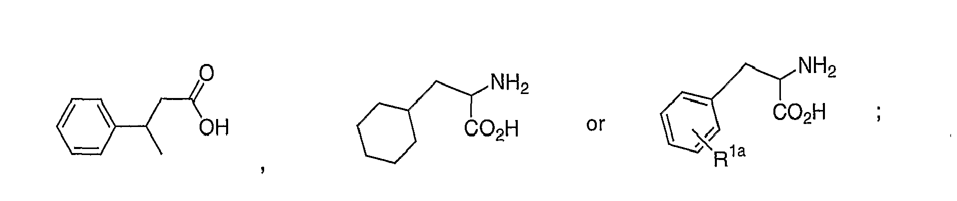 and detecting and/or recovering the antibiotic.

It is understood herein that when a compound of the invention contains asymmetric carbons, that they encompass all possible regioisomers, stereoisomers and mixtures thereof. In particular, the definitions encompass any optical isomers and diastereomers, as well as the racemic and resolved, enantiomerically pure R and S stereoisomers, as well as other mixtures of the R and S stereoisomers and pharmaceutically acceptable salts thereof. Optical isomers may be obtained in pure form by standard separation techniques.

Glycopeptide compounds of the formula according to the invention may contain mobile hydrogen atoms and consequently be present in different tautomeric forms. One skilled in the art will recognize that said tautomers often exist in equilibrium with each other. As these tautomers interconvert under physiological conditions, they provide the same useful antibacterial effects. The present invention includes mixtures of such tautomers as well as the individual tautomers of compounds of the invention.

Compounds of Formula I where R⁵, R^{6a}, R^{6b}, R^{6c}, R^{6d}, or R^{6e} are not H may form regioisomeric products which are represented by the formulae:

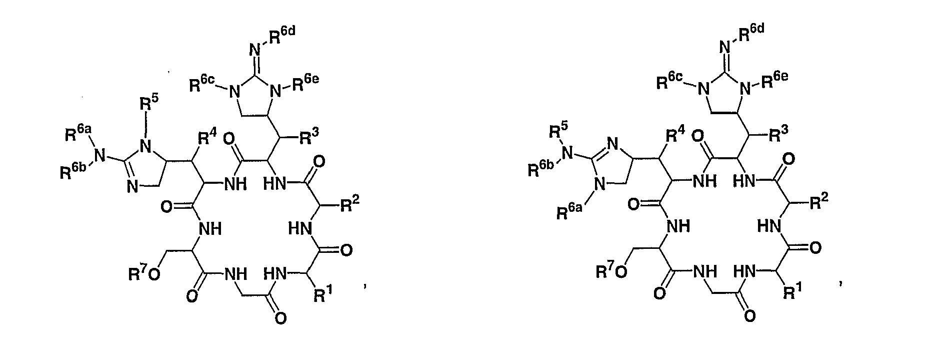

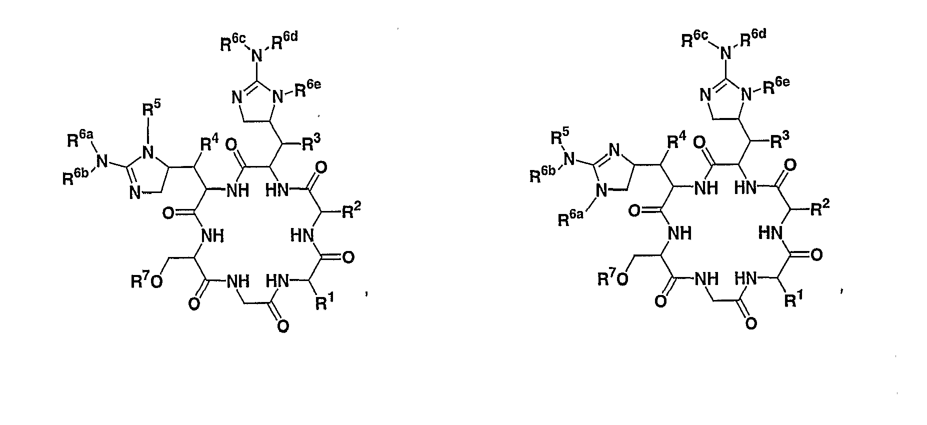

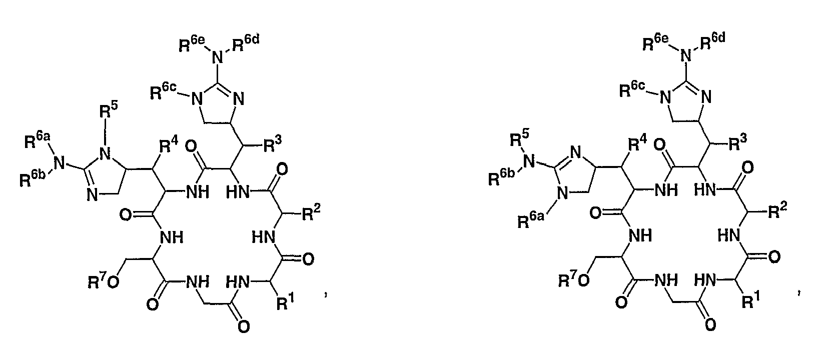

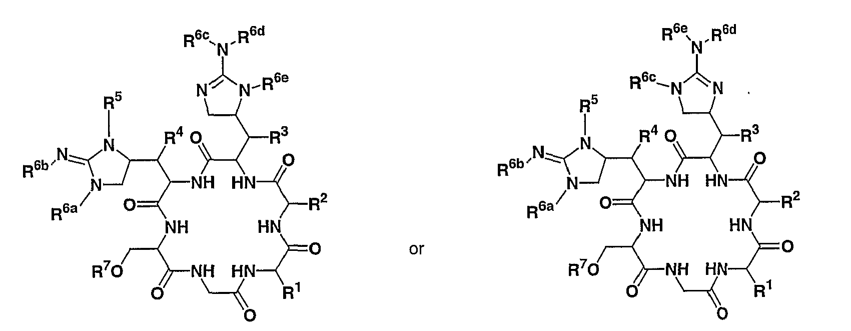

In the same regard, any substituents R¹, R², R³, R⁴, R⁵, R⁶, R⁷, R⁸, R⁹, R¹⁰, R¹¹, R¹², R¹³, R¹⁴, R¹⁶, R¹⁷, R¹⁸, R¹⁹, R²⁰, R²¹, R²², R²³, R²⁴, R²⁵, R²⁶, R²⁷, R²⁸ and R²⁹ of the compounds of the invention above and below, may be represented by their alternative tautomeric forms, where appropriate, as is known to those skilled in the art.

For the compounds of the invention defined above and referred to herein, unless otherwise noted, the following terms are defined:
Halogen, as used herein means fluoro, chloro, bromo and/or iodo.

Alkyl as used herein means a branched or straight chain radical having from 1 to 20 (preferably 1 to 6) carbon atoms optionally substituted with one or more groups selected from halogen, cyano, nitro, hydroxy, sulfhydryl, amino, alkylamino, dialkylamino, alkoxy, aryloxy, thioalkyl, thioaryl, acyl, aroyl, acyloxy, acylamino, carboxy, carboxyalkyl, carboxyaryl, carboxamido, carboxamidoalkyl, carboxamidodialkyl, alkylsulfonamido, arylsulfonamido, aryl, and heteroaryl.. Exemplary alkyl groups include but are not limited to methyl, ethyl, propyl, isopropyl, butyl, isobutyl, t-butyl, pentyl and hexyl, also optionally substituted, as well as perfluoroalkyl.

Alkenyl as used herein means a branched or straight chain radical having from 2 to 20 (preferably 2 to 6) carbon atoms optionally substituted with one or more groups selected from halogen, cyano, nitro, hydroxy, sulfhydryl, amino, alkylamino, dialkylamino, alkoxy, aryloxy, thioalkyl, thioaryl, acyl, aroyl, acyloxy, acylamino, carboxy, carboxyalkyl, carboxyaryl, carboxamido, carboxamidoalkyl, carboxamidodialkyl, alkylsulfonamido, arylsulfonamido, aryl, and heteroaryl, with the chain containing at least one carbon-carbon double bond. Alkenyl, may be used synonymously with the term olefin and includes alkylidenes. Exemplary alkenyl groups include but are not limited to ethylene, propylene and isobutylene.

Alkynyl as used herein means a branched or straight chain radical having from 2 to 20 (preferably 3 to 10) carbon atoms optionally substituted with one or more groups selected from halogen, cyano, nitro, hydroxy, sulfhydryl, amino, alkylamino, dialkylamino, alkoxy, aryloxy, thioalkyl, thioaryl, acyl, aroyl, acyloxy, acylamino, carboxy, carboxyalkyl, carboxyaryl, carboxamido, carboxamidoalkyl, carboxamidodialkyl, alkylsulfonamido, arylsulfonamido, aryl, and heteroaryl. The chain contains at least one carbon-carbon triple bond.

Cycloalkyl as used herein means a saturated monocyclic or polycyclic fused, bridged, or spirocyclic ring system having from 3 to 20 carbon atoms. Exemplary cycloalkyl rings include but are not limited to cyclopropyl, cyclobutyl, cyclopentyl, cyclohexyl, [2.2.1]-bicycloheptanyl, [2.2.2]-bicyclooctanyl, adamantyl, [3.3.1]-bicyclononanyl, spiro-[4.4]-nonanyl, spiro-[4.5]-decanyl, spiro-[5.5]-undecanyl, and the like.

Aryl as used herein means a homocyclic or polycyclic aromatic radical, fused or catenated, having 6 to 20 carbon atoms independently substituted with one to three substituents selected from the group of alkyl, halogen, cyano, nitro, hydroxy, sulfhydryl, amino, alkylamino, dialkylamino, alkoxy, aryloxy, thioalkyl, thioaryl, acyl, aroyl, acyloxy, acylamino, carboxy, carboxyalkyl, carboxyaryl, carboxamido, carboxamidoalkyl, carboxamidodialkyl, alkylsulfonamido, arylsulfonamido, aryl, or heteroaryl. Examples include, but are not limited to, phenyl, biphenyl, naphthyl, fluorenyl, and anthracenyl, optionally substituted with one to three substituents.

Alkoxy as used herein means an alkyl-O- group in which the alkyl group is as previously described. Exemplary alkoxy groups include but are not limited to methoxy, ethoxy, n-propoxy, i-propoxy, n-butoxy, and t-butoxy.

Aryloxy as used herein means an aryl-O- group in which the aryl group is as previously described.

Thioalkyl as used herein means an alkyl-S- group in which the alkyl group is as previously described.

Thioaryl as used herein means an aryl-S- group in which the aryl group is as previously described.

Acyl as used herein means an alkyl-C(O)- group in which the alkyl group is as previously described.

Aroyl as used herein refers to an aryl-C(O)- group in which the aryl group is as previously defined. Examples include but are not limited to benzoyl and naphthoyl.

Acyloxy as used herein means an alkyl-C(O)O- group or an aryl-C(O)O- group in which the alkyl or aryl group is as previously described.

Acylamino as used herein means an alkyl-C(O)N= group or an aryl-C(O)N= group in which the alkyl or aryl group is as previously described.

Carboxyalkyl as used herein means an alkyl-OC(O)- group in which the alkyl group is as previously defined.

Carboxyaryl as used herein means an aryl-OC(O)- group in which the aryl group is as previously defined.

Carboxyamido as used herein means a NH₂C(O)- group.

Carboxyamidoalkyl as used herein means an alkyl-NHC(O)- group in which the alkyl group is as previously defined.

Carboxyamidodialkyl as used herein means a dialkyl-NC(O)- group in which the alkyl groups are as previously defined.

Alkylsulfondamido as used herein means an alkyl-S(O)₂-N= group in which the alkyl group is as previously defined.

Arylsulfonamido as used herein means an aryl-S(O)₂-N= group in which the aryl group is as previously defined.

Heteroaryl denotes a 5- or 6-membered heterocyclic ring, which may be fused to another 5- or 6-membered heterocyclic ring or non-heterocyclic ring, especially heteroaromatic rings which contain 1 to 3 heteroatoms which may be the same or different. Nitrogen, oxygen and sulfur are the preferred heteroatoms provided that the heterocyclic ring does not contain -O-O-, -S-S- and -S-O- bonds. A heteroaryl group may be optionally substituted with 1 to 3 substituents selected from the group halogen, cyano, nitro, hydroxy, sulfhydryl, amino, alkylamino, dialkylamino, alkoxy, aryloxy, thioalkyl, thioaryl, acyl, aroyl, acyloxy, acylamino, carboxy, carboxyalkyl, carboxyaryl, carboxamido, carboxamidoalkyl, carboxamidodialkyl, alkylsulfonamido, arylsulfonamido, aryl, and heteroaryl. Exemplary heteroaryl groups include but are not limited to furan, thiophene, pyrrole, oxazole, thiazole, imidazole, isoxazole, isothiazole, pyridine, pyrimidine, pyrazine, pyridazine, indole, quinoline, isoquinoline, benzimidazole, quinazoline, and the like.

Where terms are used in combination, the definition for each individual part of the combination applies unless defined otherwise. For instance, aralkyl refers to an aryl group, and alkyl refers to the alkyl group as defined above.

The compounds of the invention may be obtained as inorganic or organic salts using methods known to those skilled in the art (Richard C. Larock, Comprehensive Organic Transformations, VCH publishers, 411-415, 1989). It is well known to one skilled in the art that an appropriate salt form is chosen based on physical and chemical stability, flowability, hydroscopicity and solubility.

Pharmaceutically acceptable salts of the compounds of the invention include basic salts of inorganic and organic acids, including but not limited to hydrochloric acid, hydrobromic acid, sulphuric acid, phosphoric acid, nitric acid, methanesulfonic acid, ethanesulfonic acid, benzenesulfonic acid, toluenesulfonic acid, napthalenesulfonic acid, camphorsulfonic acid, malic acid, acetic acid, trifluroacetic acid, oxalic acid, malonic acid, tartaric acid, citric acid, lactic acid, fumaric acid, succinic acid, maleic acid, salycylic acid, benzoic acid, phenylacetic acid, mandelic acid and the like.

When compounds of the invention include an acidic function such as a carboxy group, then suitable pharmaceutically acceptable salts of the compounds of the invention with an acidic moiety can be formed from organic and inorganic bases. Such includes but is not limited to salts formed with alkali metals or alkaline earth metals such as sodium, potassium, lithium, calcium, or magnesium or organic bases such as triethylamine, N,N-diethylmethylamine, N,N-diethylethylenediamine, and N-tetraalkylammonium salts such as N-tetrabutylammonium salts. For additional examples of "pharmaceutically acceptable salts" see Berge et al, J. Pharm. Sci. 66, 1 (1977).

The compounds can also be used in the form of esters, carbonates, carbamates and other conventional prodrug forms, which when administered in such form, convert to the active moiety in vivo.

### DESCRIPTION OF THE PREFERRED EMBODIMENTS

This invention uses as starting materials for the preparation of certain compounds of the invention, a variety of glycopeptide antibiotics prepared by fermentation. In particular, using the fermentation conditions described in U.S. Patent No. 3,495,004 a complex of antibiotics is isolated. Optionally, using hereindescribed fermentation conditions, with *Streptomyces hygroscopicus* strain LL4600, the complex may also be prepared. Further separation of the complex of antibiotics by HPLC into individual components AC-98-1, AC-98-2, AC-98-3, AC-98-4 and AC-98-5 and determination of the chemical structures by spectroscopy is described in copending provisional patent application Docket Number 33447, 60/286,249, filed April 25, 2001. The structures of the individual components are shown below.

| | **R¹** | **R²** | **R³** | **R⁴** | **R⁵** |
|---|---|---|---|---|---|
| AC-98-1 | 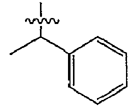 | 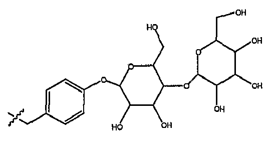 | OH | OH | 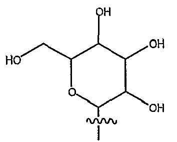 |
| AC-98-2 | 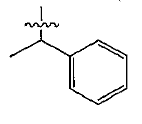 | 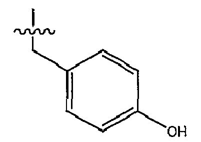 | OH | OH | 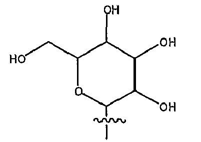 |
| AC-98-3 | 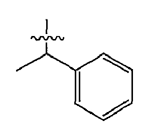 | 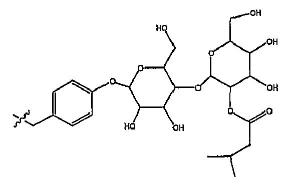 | OH | OH | 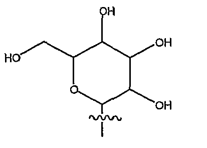 |
| AC-98-4 | 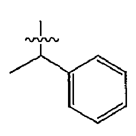 | 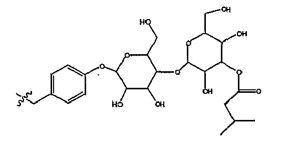 | OH | OH | 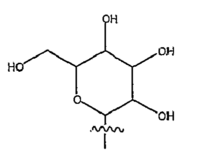 |
| AC-98-5 | 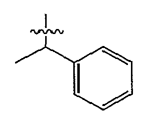 | 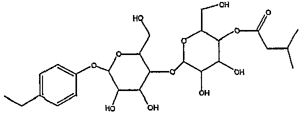 | OH | OH | 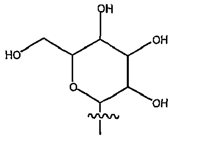 |

Certain compounds of the invention are prepared by fermentation means using modified strains of *Streptomyces hygroscopicus* selected from the group LL4600, LL4614, LL4666, LL4690, LL4728, LL4741, LL4742, LL4744, LL4773, LL4779, LL4783, LL4902, BD2, BD20 and BD70 and mutants thereof and include those of the formula 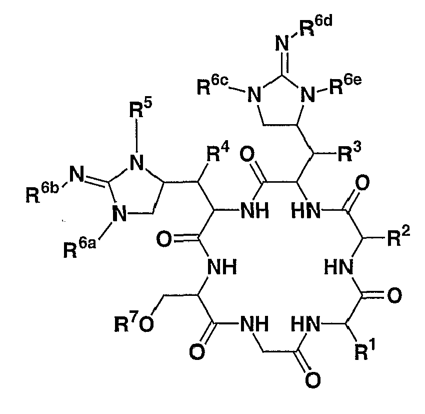 wherein:
R¹ is selected from: 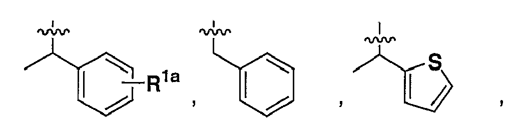 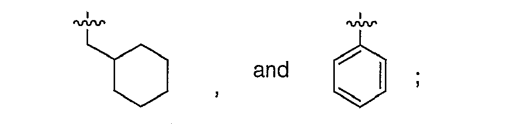
R^{1a} is H or halogen;
R² is a moiety 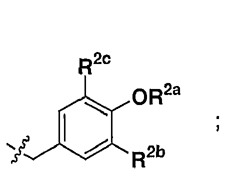
R^{2a} is selected from
H, 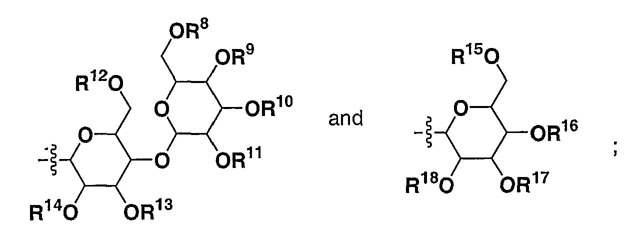
R2^{b} is H, F or NH₂;
R^{2c} is H;
R³ and R⁴ are independently H or OH;
R⁵ is H or 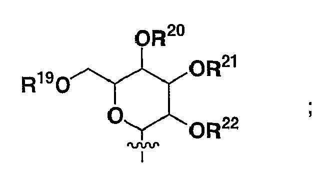
R^{6a}, R^{6b}, R^{6c}, R^{6d}, or R^{6e} are H;
R⁷ ,R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently H, or -C(O)-Y-Z ;
Y is a single bond ;
Z is straight or branched chain alkyl(C₁-C₂₀) or straight or branched chain alkenyl(C₂-C₂₀);
by directed biosynthetic fermentations through the addition of substrates of the formulae: straight or branched chain alkyl(C₁-C₂₀)CO₂H, straight or branched chain alkenyl(C₂-C₂₀)CO₂H, 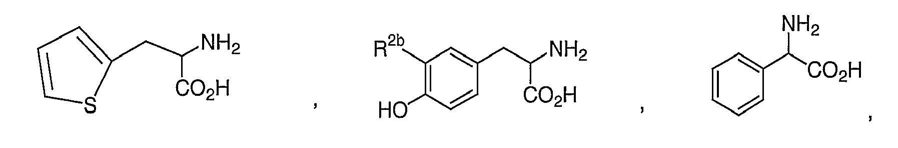 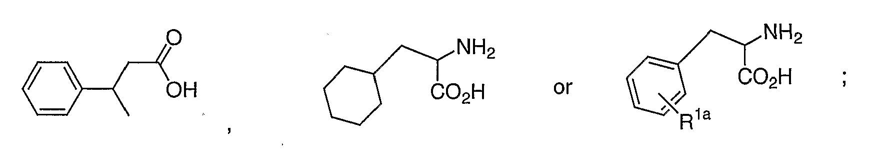 using controlled conditions in a suitable media, optionally containing mannose, under aerobic conditions and isolating the antibiotics which optionally may be further chemically modified. Multiple substrates may optionally be added.

For example cultivating *streptomyces hygroscopicus* LL4614 in the presence of p-chloro-DL-phenylalanine affords recoverable quantities of glycopeptide antibiotics cyclo[glycyl-4-chloro-β-methylphenylalanyl-O-[4-O-[3-O-(3-methylbutanoyl)hexopyranosyl]-hexopyranosyl]tyrosyl-3-(2-iminoimidazolidin-4-yl)seryl-3-(3-hexopyranosyl-2-iminoimidazolidin-4-yl)serylseryl] and cyclo[glycyl-4-chloro-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl].

A further aspect of the invention is to recover glycopeptide antibiotics of the invention wherein R⁷ ,R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are H by hydrolysis of mixtures where R⁷ ,R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are H or esters (-C(O)-Y-Z) by adding base which includes sodium hydroxide and the like or (3-[cyclohexylamino]-1-propanesulfonic acid)to the medium at about O° to about 25° C, preferred is about O° to about 4° C at a pH of about 8.0 to about 13.5, preferred about 11.5 to about 13.5 and recovering said antibiotic at a pH of about 1.8 to about 6.5 preferred is about 4.0 to about 6.0. The pH is adjusted from about 8.0 to about 13.5 to about 1.8 to about 6.5 with acids which include hydrochloric acid, acetic acid, propanoic acid and (3-[N-morpholino]propanesulfonic acid). Preferably the acid adjusted pH is about 4.0 to about 5.0. Optionally buffers may also be used to adjust pH. Optionally, mixtures where R⁷ ,R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are H or esters (-C(O)-Y-Z) may first be isolated from the medium and then hydrolyzed as described hereinabove.

Certain of the glycopeptide antibiotics of the invention are produced by fermentation of mutant derivative strains of *Streptomyces hygroscopicus* LL4600. These microorganisms listed in Table 1 are maintained in the culture collection of American Home Products, Wyeth-Ayerst Discovery, Pearl River, New York as culture numbers LL4600, LL4614, LL4666, LL4690, LL4728, LL4741, LL4742, LL4744, LL4773, LL4779, LL4780, LL4783, LL4902, BD2, BD20 and BD70. A viable culture of these new microorganisms is deposited under the Budapest Treaty with the Patent Culture Collection Laboratory, Northern Regional Research Center, U.S. Department of Agriculture, Peoria, Ill. 61604, and added to its permanent collection.

**Table 1**

| **Wyeth-Ayerst culture** **collection #** | **NRRL culture** **collection** |
|---|---|
| LL4600 | NRRL 30439 |
| LL4614 | NRRL 30440 |
| LL4666 | NRRL 30441 |
| LL4690 | NRRL 30442 |
| LL4728 | NRRL 30443 |
| LL4741 | NRRL 30444 |
| LL4742 | NRRL 30445 |
| LL4744 | NRRL 30446 |
| LL4773 | NRRL 30447 |
| LL4779 | NRRL 30448 |
| LL4780 | NRRL 30449 |
| LL4783 | NRRL 30450 |
| LL4902 | NRRL 30451 |
| BD2 | NRRL 30452 |
| BD20 | NRRL 30453 |
| BD70 | NRRL 30454 |

It is to be understood that the production of certain glycopeptide antibiotics of the invention by fermentation is not limited to the particular mutants defined above which are for illustrative purposes only. In fact, it is desired and intended to include the use of mutants as described herein and those produced by additional exposure of the above defined mutants to X-radiation, ultraviolet radiation, N'-methyl-N'-nitro-N-nitrosoguanidine, ethyl-methane sulfonate and the like. Strains presented are all derivatives of NRRL 3085. A culture stock designated LL4600 is derived from NRRL 3085 by colony purification and served as the starting point for the work described. Mutants accumulating biosynthetic intermediates, shunt metabolites or compounds not detected in LL4600 are derived by NTG mutagenesis of LL4600 or various industrial derivatives of LL4600.

### Mutagenesis

The described strains are obtained via N-methyl-N'-nitro-N-nitrosoguanidine (NTG) mutagenesis. Cells are grown in TSBG (Tryptic soy broth [Difco] supplemented with 20 g/L glucose) for 24-48 hours and then are sonicated for 5-40" to disperse mycelial pellets to variably-sized mycelial fragments. The sonicated suspension is pelleted, resuspended in fresh TSBG containing 50-1000 µg/mL NTG and dosed for 10-260 min at 30°C with shaking. The dosed cells are then pelleted, resuspended in fresh TSBG and grown overnight at 30°C. The overnight cells are then sonicated for 6-20" to disrupt mycelial pellets. The mutagenized cells are stored as a 20% glycerol stock at -70°C.

### Cultivation

Cultivation of mutant strains of *Streptomyces hygroscopicus* designated above may be carried out in a wide variety of liquid culture media. Media which are useful for the production of glycopeptide antibiotics include an assimilable source of carbon, such as dextrin, dextrose, sucrose, molasses, starch, glycerol, etc; an assimilable source of nitrogen such as protein, protein hydrolysate, polypeptides, amino acids, corn steep liquor, etc; and inorganic anions and cations, such as potassium, sodium, ammonium, calcium, sulfate, carbonate, phosphate, chloride, etc. Trace elements such as zinc, cobalt, iron, boron, molybdenum, copper, etc., are supplied as impurities of other constituents of the media. Aeration in tanks and bottles is supplied by forcing sterile air through or onto the surface of the fermenting medium. Aeration provides for aerobic fermentations. Further agitation in tanks is provided by a mechanical impeller. An antifoam agent such as polypropylene glycol may be added as needed.

### Culture preservation

Strains are preserved as frozen whole cells (frozen vegetative mycelia, FVM) prepared from cells grown for 24-48 hours in TSBG (Tryptic soy broth [Difco] supplemented with 20 g/L glucose). Glycerol is added to 20% and the cells are frozen at -70°C.

Inoculum Development Fermentation In Suitable Culture Media and Conditions Composition of suitable culture media used in the examples presented is as follows:

| **Composition of fermentation media** | | | | | | |
|---|---|---|---|---|---|---|
| **Component** | **BPM17** | **BPM17stat** | **BPM17stat gal** | **BPM17stat man** | **BPM27** | **BPM27-man** |
| Pharmamedia (Traders) | 10 g/L | 20 g/L | 20 g/L | 20 g/L | 20 g/L | 20 g/L |
| Glucose | 40 g/L | 60 g/L | 60 g/L | 60 g/L | 60 g/L | 60 g/L |
| Galactose | - | - | 20 g/L | - | - | - |
| Mannose | - | - | - | 2 g/L | 2 g/L | - |
| CaCO₃ (Mississippi Lime) | 5 g/L | 5 g/L | 5 g/L | 5 g/L | - | - |
| CaCO₃ (Gamaco) | - | - | - | - | 15 g/L | 15 g/L |

Fermentations are inoculated from cells grown in TSBG medium at 30°C for 24-48hr with shaking on a gyrorotary shaker. Shake-flask fermentations are performed at 30°C for 3 - 5 days on a gyro-rotary shaker operating at 250 rpm (2" stroke). Ten-liter fermentations are performed at 30°C for 3- 5days at 30°C, at 400-800 rpm with1 vvm airflow. Fermentation at 300 liters is similarly performed with agitation at 170-200 rpm. Antifoam, such as Macol P2000 is added to fermentor medium at 0.2-2.0%. Three hundred-liter fermentations with medium BPM17statgal employ galactose at 8 g/L. The strains described may be fermented in any of the media listed above beyond the specific examples herein described.

### Biotransformation Methods

Substrates are prepared as 5 mg/mL stock solutions in water and filter sterilized. The stock solution is added to BPM17statgal shake-flasks (each containing 25mL of medium) to give a final concentration of 100 mg/L. Substrate compounds are added at the time of inoculation. Fermentations are conducted for 3 days.

### Hydrolysis of Esters

Compounds of the invention, prepared by fermentation may be isolated as mixtures which include esters. Alternatively, and in particular, mixtures of esters wherein R⁷ ,R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently H or -C(O)-Y-Z ; Y is a single bond ; and Z is straight or branched chain alkyl(C₁-C₂₀) or straight or branched chain alkenyl(C₂-C₂₀) may optionally be hydrolyzed following fermentation and preferably before isolation of antibiotics by adding suitable bases to the broth which include: aqueous sodium hydroxide and (3-[cyclohexylamino]-1-propanesulfonic acid) CAPS and the like and adjusting the pH with hydrochloric acid, acetic acid or (3-[N-morpholino]propanesulfonic acid) MOPS and the like to afford products where R⁷ ,R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are H followed by recovery of the antibiotics.

### Analytical HPLC analysis of glycopeptide antibiotics

Cells are removed for fermentation broth by centrifugation. The clarified supernatant is applied to a wetted BAKERBOND™ spe carboxylic acid extraction column (catalog # 7211-03). Columns are washed with 50% aqueous methanol and eluted with acetonitrile/water/trifluoroacetic acid (70/30/0.5). The solvent is evaporated, and the residue is reconstituted in 0.2 - 0.4 mL methanol/water (1/1). In some instances supernatants are analyzed directly. Samples are analyzed using a Hewlett Packard model 1090 liquid chromatograph with photodiode array detection. The compounds are resolved by reverse phase chromatography using a YMC ODS-A 4.6 x 150 mm HPLC column, with a mobile phase of 10% acetonitrile: 0.01% trifluoroacetic acid (solvent A) and 50% acetonitrile : 0.01% trifluoroacetic acid (solvent B). A linear gradient from 0% B to 100% B in 22 min, with a flow rate of 1 mL/min, is used for elution. Novel metabolites are identified by the appearance of HPLC peaks which possess characteristic UV absorption spectra but displayed novel retention times. Relative retention times (RRT) are calculated by dividing the peak retention times of novel compounds of the invention by that of (Example 281a) Cyclo[glycyl-.β.-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]. Subsequent LC/MS analysis is then performed on fermentation extracts to determine molecular weights.

### LC/MS analysis of glycopeptide antibiotics from fermentations

The molecular weights of new glycopeptide antibiotics from fermentations are determined using a Hewlett-Packard API-electrospray LC/MS system with an HP 5989B Mass Spectrometer, HP 59987A API-Electrospray, HP 1090 series II HPLC and HP ChemStation data system with HP G1047A LC/MS software. Extracts are resolved by reverse phase HPLC as described above. UV detection is at 226 nm. The MS electrospray is performed in positive mode with a scan range of 400∼1700 m/z.

### General Procedure for the Isolation of Fermentation Products

Typical representative methods for the recovery and isolation of glycopeptide antibiotics of the invention from the fermentation broth include:
a) loading fermentation broth on polyacrylate resin, XAD-7, prewashed sequentially with methanol, acetone and water. Eluting the resin with 1:1 acetonitrile-water containing 0.1% trifluoroacetic acid, concentrating under reduced pressure to a small volume then extracting with 1:4 water-N,N-dimethylformamide and fractionating the residue of the evaporated extract by reverse phase HPLC on a C18 column by elution with a gradient of acetonitrile-water containing trifluoroacetic acid;
b) using method a) above and fractionating the residue by reverse phase HPLC on a C18 column by elution with a gradient of methanol-water containing trifluoroacetic acid;
c) loading fermentation broth on CG-61 resin, and eluting with 40%:60% acetonitrile-water containing 0.01 % trifluoroacetic acid, concentrating product fractions under reduced pressure and adjusting the pH to 12.5 with sodium hydroxide for 45 min to 3 h or until esters are hydrolyzed. Adjusting the pH to 3.0 with hydrochloric acid and collecting the antibiotic from acetonitrile;
d) adding diatomaceous earth to the fermentation, filtering and adjusting the pH of the filtrate to pH 12.8 with sodium hydroxide and holding for up to 3 h, neutralizing with acetic acid and loading on SP207 resin. Washing the resin with water, methanol and eluting with 1:1 methanol-water-3% acetic acid and collecting product from concentrated fractions with isopropanol-acetonitrile.
e) adding an equal volume of 50 mM CAPS buffer (pH 13) to the fermentation broth until esters have been hydrolyzed, adding three volumes of 1M MOPS buffer (pH 7.0) to neutralized the mixture and loading on a CG-71C column, eluting with 1:1 acetonitrile-water-0.05% trifluoroacetic acid and purifying antibiotic product fractions by reverse phase HPLC on a C18 column using a gradient of methanol-water-0.05% trifluoroacetic acid ; or
f) adding an equal volume of 50 mM CAPS buffer (pH 13) to the fermentation broth until esters are hydrolyzed, adding three volumes of 1M MOPS buffer (pH 7.0) to neutralized the mixture and separating antibiotic product by reverse phase HPLC on a C18 column by elution with 10%-90% acetonitrile-water containing 0.01% trifluoroacetic acid.

Further preparation of compounds of this invention is described below and is illustrated in the following Schemes.

Compounds of this invention may be prepared as shown in Scheme I by nitrations and halogenations of glycopeptide antibiotics 1 to give glycopeptide antibiotics 2 which may be further modified by herein described subsequent transformations. Halogenation of glycopeptide antibiotics 1 to afford glycopeptide antibiotics 2 may be achieved by methods known to those skilled in the art, including treatment with bromine, iodine, sodium hypochlorite, N-bromosuccinimide, N-chlorosuccinimide, N-iodosuccinimide, iodine monochloride, benzyltrimethylammonium dichloroiodate, and the like, in a solvent which include a concentrated mineral acid, such as aqueous hydrochloric acid, sulfuric acid, and the like, or a concentrated carboxylic acid such as acetic acid or trifluoroacetic acid, and the like, at temperatures about 0° to about 30°C. Nitration of glycopeptide antibiotics 1 to afford glycopeptide antibiotics 2 may be achieved by methods known to those skilled in the art, by treatment with nitrating reagents, which include metal nitrate salts including potassium nitrate in a solvent such as a concentrated mineral acid or a concentrated carboxylic acid such as acetic acid or trifluoroacetic acid, at temperatures about -20°C to about 30°C.

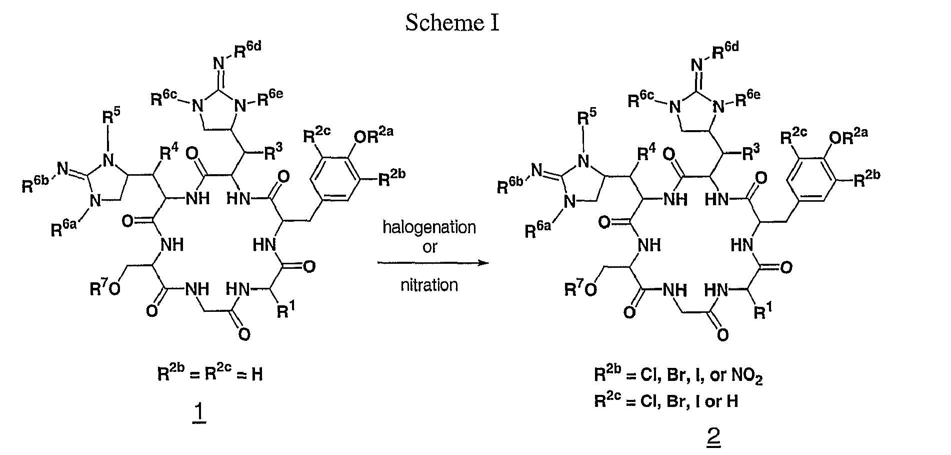

As further shown in Scheme II, compounds in which R^{2b} and/or R^{2c} of glycopeptide antibiotics 2 are bromine or iodine serve as precursors to compounds of formula 3 wherein R^{2b} and/or R^{2c} are alkyl(C₁-C₂₀), cycloalkyl(C₃-C₂₀), alkenyl(C₂-C₂₀), alkynyl(C₂-C₂₀), aryl and heteroaryl, all of which may be prepared by transition metal mediated coupling reactions, which include the palladium(0) mediated couplings of aryl bromides or iodides with organostannanes which include (R^{2b})₄Sn, (R^{2c})₄Sn, R^{2b}Sn(butyl)₃ or R^{2c}Sn(butyl)₃, and the like (Stille couplings; as described in Farina, V. and Roth, G.P. Advances in Metal-Organic Chemistry 1996, 5, 1- 53 and references therein), with organoboron compounds which include R^{2b}-(9-borabicyclo[3.3.1]nonane) (R^{2b}-9-BBN), R^{2c}-(9-borabicyclo[3.3.1]nonane) (R^{2c}-9-BBN), R^{2b}-B(OH)₂, R^{2c}-B(OH)₂, R^{2b}-B(O-alkyl(C₁-C₂₀))₂, R^{2c}-B(O-alkyl(C₁-C₂₀))₂,

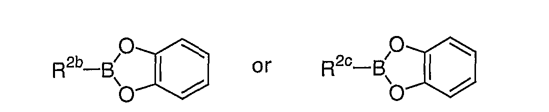

(Suzuki couplings; as described in Miyaura, N. and Suzuki, A. Chem. Rev. 1995, 95, 2457-2483 and references therein), or with alkenes or terminal alkynes (Heck couplings; as described in de Meijere, A. and Meyer, F.E. Angew. Chem. Int. Ed. Eng. 1994, 33, 2379-2411 and references therein). In those instances wherein the transition metal mediated coupling reaction is a palladium(0) mediated coupling of a terminal alkyne with glycopeptide antibiotics 2, wherein R^{2a} is H, the formation of products 3a containing a benzofuran moiety may also be produced.

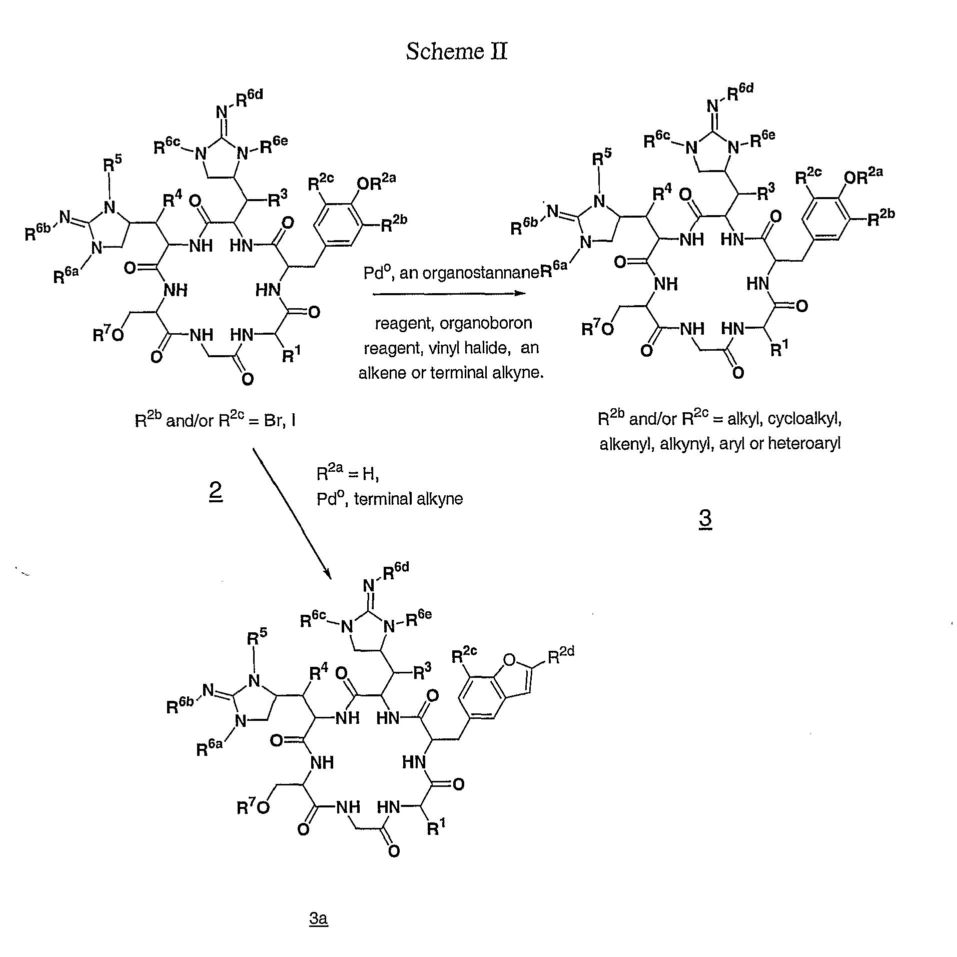

Scheme III describes the synthesis of amine 4 in which R^{2b} is -NH₂ which maybe prepared from the corresponding glycopeptide antibiotic 2 in which R^{2b} is -NO₂, by reduction using standard methods known to those skilled in the art, including hydrogenation under an atmosphere of hydrogen at pressures from about 1 to about 250 psi, over a suitable catalyst such as palladium or platinum, and the like, either alone or adsorbed onto a suitable support such as carbon, alumina or diatomaceous earth, in solvents such as water, methanol, ethanol, and the like, alone or in combination, in the presence or absence of a mineral or carboxylic acid, such as hydrochloric acid, sulfuric acid, or acetic acid, at temperatures from about 25°C to the reflux temperature of the solvent. Alternatively, amine 4 may also be prepared by the reduction of glycopeptide antibiotic 2 over metals such as iron or zinc in solvents such as water, methanol, ethanol, and the like, alone or in combination, in the presence of a mineral or carboxylic acid, such as hydrochloric acid, sulfuric acid, or acetic acid, at temperatures from ambient temperature to the reflux temperature of the solvent.

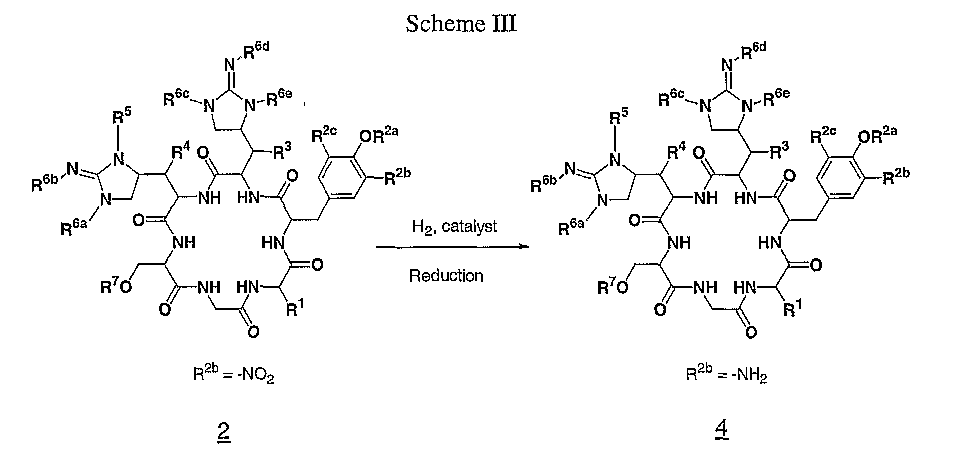

As further described in Scheme IV, amine 5 in which R^{2b} is -NR^{2f}R^{2g}, wherein R^{2f} is alkyl(C₁-C₂₀), cycloalkyl(C₃-C₂₀), alkenyl(C₃-C₂₀) or alkynyl(C₃-C₂₀) and R^{2g} is H, may be prepared from amine 4 in which R^{2b} is -NH₂, by methods known to those skilled in the art, such as by alkylation with an appropriate alkyl halide R^{2f}X, where X is Cl, Br, I, -O-tosylate, -O-mesylate, or -O-triflate, in the presence or absence of a suitable base. Amine 5 may be further alkylated in the case where R^{2g} is H to give disubstituted amine 6 where R^{2b} is -NR^{2f}R^{2g}, wherein R^{2f} and R^{2g} are independently alkyl(C₁-C₂₀), cycloalkyl(C₃-C₂₀), alkenyl(C₃-C₂₀) or alkynyl(C₃-C₂₀), by treatement with an appropriate alkyl halide R^{2g}X, where X is Cl, Br, I, -O-tosylate, -O-mesylate, or -O-triflate, in the presence or absence of a suitable base as above. Alternatively, amine 5 in which R^{2b} is -NR^{2f}R^{2g}, wherein R^{2f} is alkyl(C₁-C₂₀), cycloalkyl(C₃-C₂₀), alkenyl(C₃-C₂₀) or alkynyl(C₃-C₂₀) may be prepared from amine 4 in which R^{2b} is -NH₂, by methods known to those skilled in the art, such as by treatment with an aldehyde or keto form of the formula R^{2f}(R^{2f}(O)) and a reductant (reductive amination) which include but are not limited to sodium borohydride, sodium cyanoborohydride, and hydrogen and a catalyst selected from palladium and platinum, and the like. Amine 5 may undergo further reductive amination in the case where R^{2g} is H to give disubstituted amine 6 where R^{2b} is -NR^{2f}R^{2g}, wherein R^{2f} and R^{2g} are independently alkyl(C₁-C₂₀), cycloalkyl(C₃-C₂₀), alkenyl(C₃-C₂₀) or alkynyl(C₃-C₂₀), by treatment with an aldehyde or keto form of the formula R^{2g}(R^{2g}(O)) and a reductant as above. Alternatively, amine 5 in which R^{2b} and/or R^{2c} are -NR^{2f}R^{2g}, wherein R^{2f} is alkyl(C₁-C₂₀), cycloalkyl(C₃-C₂₀), alkenyl(C₃-C₂₀), alkynyl(C₃-C₂₀), aryl, or heteroaryl and R^{2g} is H, or amine 6, wherein R^{2b} and/or R^{2c} are -NR^{2f}R^{2g}, wherein R^{2f} and R^{2g} are independently alkyl(C₁-C₂₀), cycloalkyl(C₃-C₂₀), alkenyl(C₃-C₂₀), alkynyl(C₃-C₂₀), aryl, or heteroaryl, may be prepared from glycopeptide antibiotic 2 in which R^{2b} and/or R^{2c} are bromine or iodine, by methods known to those skilled in the art, such as by palladium(0) mediated amination with an appropriate amine R^{2f}NH₂ or R^{2f}R^{2g}NH, respectively (J. F. Hartwig, Angew. Chem. Int. Ed. 1998, 37, 2046-2067, and references therein).

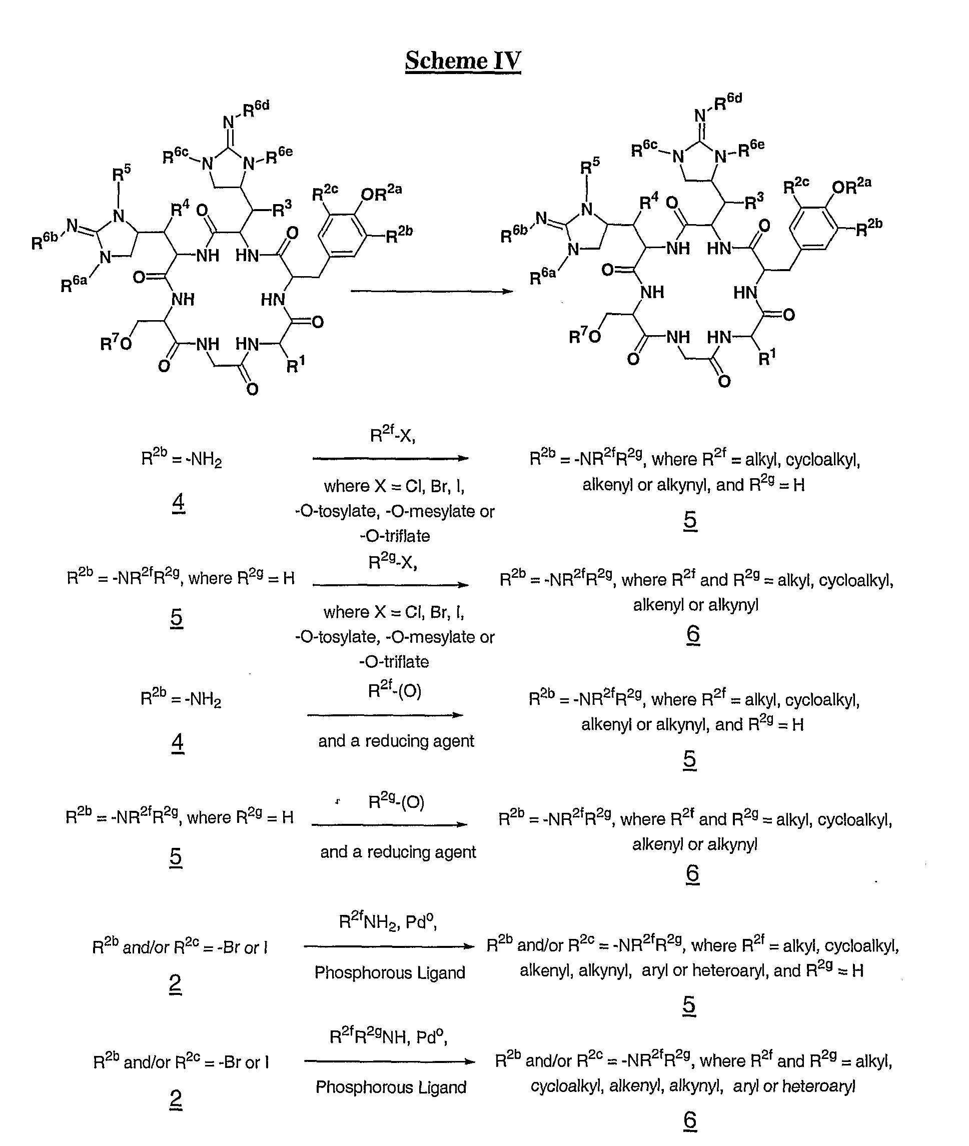

As shown in Scheme V, glycopeptide antibiotics 7 in which R^{2b} is -NR^{2f}R^{2g}, wherein R^{2g} is the group D-E-G, wherein D is -C(O)-, E is a single bond and G is H, alkyl(C₁-C₂₀), cycloalkyl(C₃-C₂₀), alkenyl(C₂-C₂₀), alkynyl(C₂-C₂₀), perfluoroalkyl(C₁-C₆), aryl or heteroaryl, may be prepared from amine 4 or amine 5 in which R^{2b} is -NR^{2f}R^{2g}, where R^{2f} is hereinbefore defined and R^{2g} is H, by methods known to those skilled in the art. Such methods include employing any of a variety of acylation reactions using a carboxylic acid halide G-C(O)-Cl, carboxylic acid anhydride (G-C(O))₂-O, or a carboxylic acid G-C(O)-OH in combination with an appropriate activating agent, such as 1,3-dicyclohexylcarbodiimide, 1-(3-dimethylaminopropyl)-3-ethylcarbodiimide hydrochloride, benzotriazol-1-yloxy-tris(dimethylamino)phosphonium hexafluorophosphate, O-benzotriazol-1-yl-N,N,N',N'-tetramethyluronium hexafluorophosphate, 1,1'-carbonyldiimidazole, and the like, in the presence or absence of a suitable base to give glycopeptide antibiotics 7.

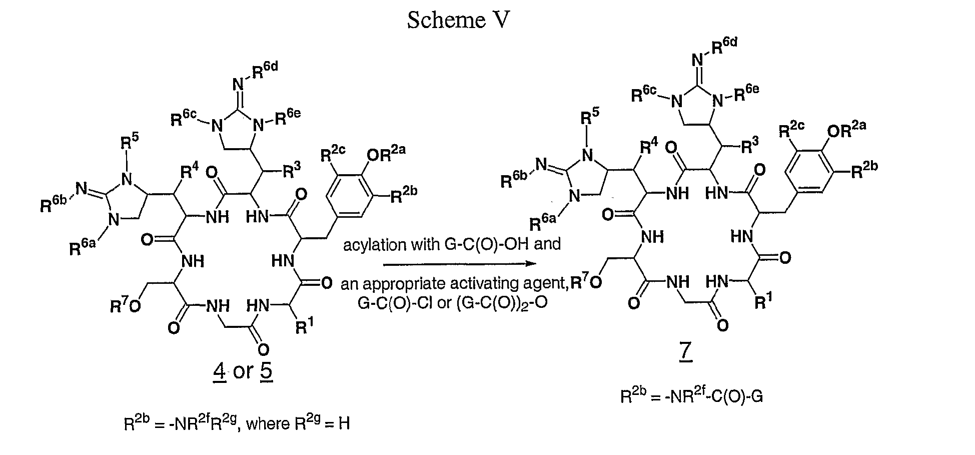

As described in Scheme VI, glycopeptide antibiotics 8 in which R^{2b} is -NR^{2f}R^{2g}, where R^{2g} is the group D-E-G as described above, wherein D is -C(S)-, E is a single bond and G is H, alkyl(C₁-C₂₀), cycloalkyl(C₃-C₂₀), alkenyl(C₂-C₂₀), alkynyl(C₂-C₂₀), perfluoroalkyl(C₁-C₆), aryl or heteroaryl, may be prepared from glycopeptide antibiotics 7 in which D is -C(O)- by methods known to those skilled in the art such as by treatment with 2,4-bis(4-methoxyphenyl)-1,3-dithia-2,4-diphosphetane-2,4-disulfide.

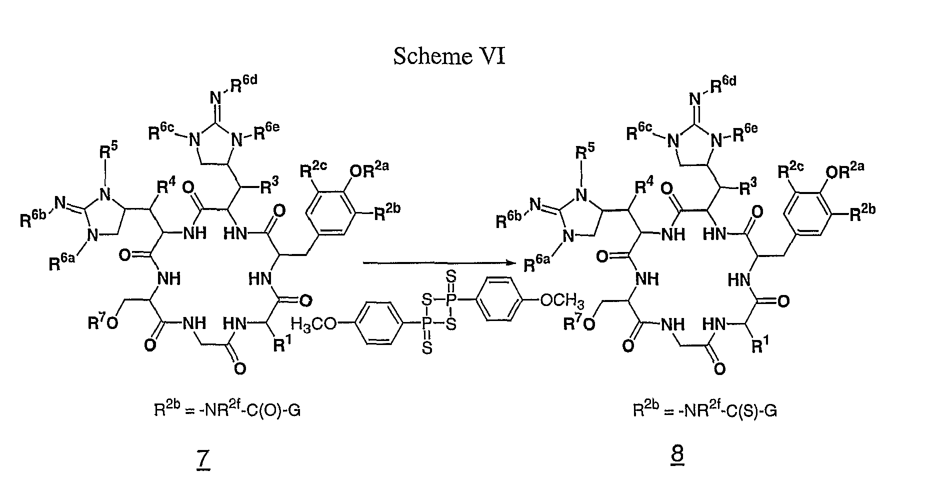

As described in Scheme VII, glycopeptide antibiotics 9 in which R^{2b} is -NR^{2f}R^{2g}, wherein R^{2g} is the group D-E-G, D is -S(O)₂-, E is a single bond and G is alkyl(C₁-C₂₀), cycloalkyl(C₃-C₂₀), alkenyl(C₂-C₂₀), alkynyl(C₂-C₂₀), perfluoroalkyl(C₁-C₆), aryl or heteroaryl, may be prepared from the corresponding primary amine 4 or secondary amine 5 in which R^{2b} is -NR^{2f}R^{2g}, where R^{2f} is hereinbefore defined and R^{2g} is H, by methods known to those skilled in the art, such as by treatment with an appropriate sulfonic acid halide G-S(O)₂-Cl or sulfonic acid anhydride (G-S(O)₂)₂O, in the presence or absence of a suitable base.

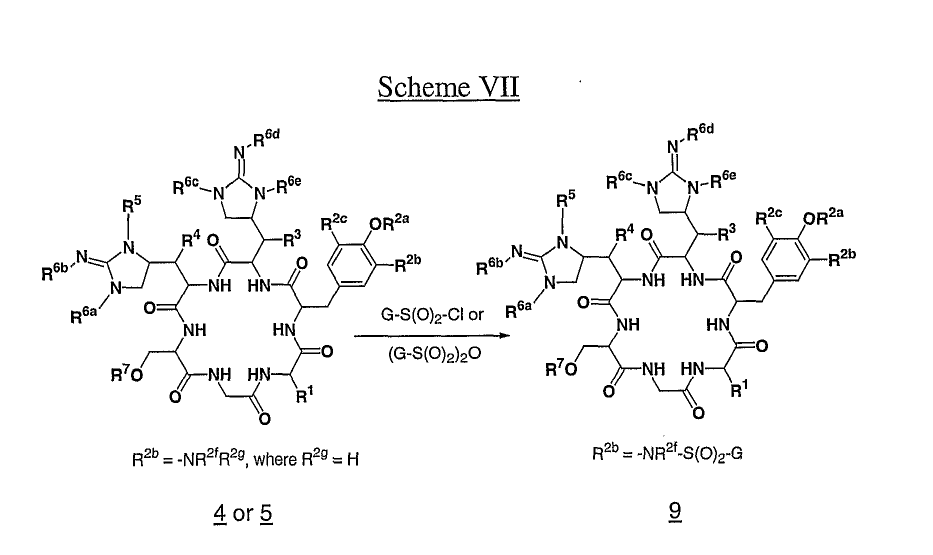

Glycopeptide antibiotics 10, in which R^{2b} is -NR^{2f}R^{2g}, wherein R^{2g} is the group wherein D is -C(O)-, E is -O- and G is alkyl(C₁-C₂₀), cycloalkyl(C₃-C₂₀), alkenyl(C₃-C₂₀), alkynyl(C₃-C₂₀), perfluoroalkyl(C₁-C₆), aryl or heteroaryl, may be prepared as shown in Scheme VIII from primary amine 4 or secondary amine 5 in which R^{2b} is -NR^{2f}R^{2g}, where R^{2f} is hereinbefore defined and R^{2g} is H, by methods known to those skilled in the art. Methods include treatment with an appropriate chloroformate G-O-C(O)-Cl, N-hydroxysuccinimide carbonate G-O-C(O)-OSu or, alternatively, by sequential treatment with phosgene or a phosgene equivalent such as triphosgene, 1,1'-carbonyldiimidazole, 1,1'-carbonyl-bis(1,2,4)-triazole, or a chloronitrophenylformate, and the like, followed by treatment with an alcohol G-OH, in the presence or absence of a suitable base to give glycopeptide antibiotics 10.

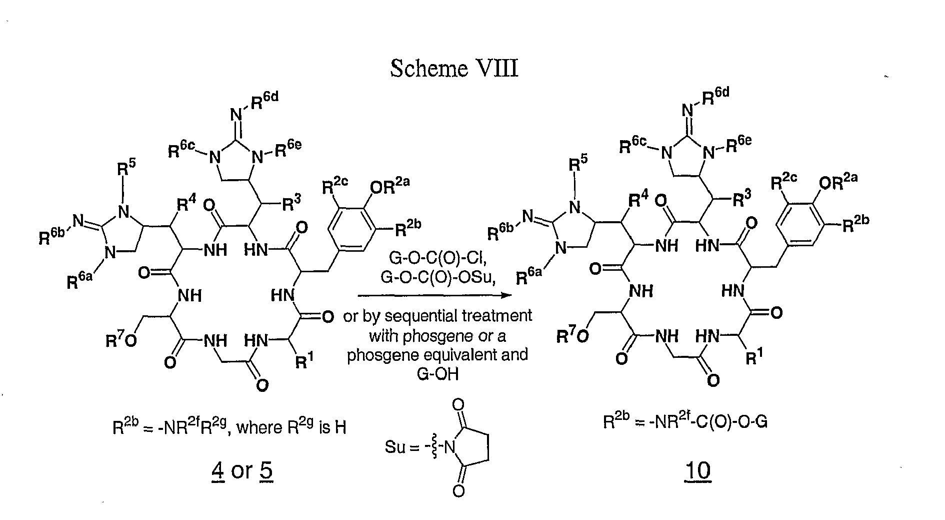

Glycopeptide antibiotics 11, in which R^{2b} is -NR^{2f}R^{2g}, wherein R^{2g} is the group D-E-G wherein D is -C(O)-, E is -NR^{2h}- and G is alkyl(C₁-C₂₀), cycloalkyl(C₃-C₂₀), alkenyl(C₃-C₂₀), alkynyl(C₃-C₂₀), perfluoroalkyl(C₁-C₆), aryl or heteroaryl, may be prepared as shown in Scheme IX from primary amine 4 or secondary amine 5 where R^{2b} is -NR^{2f}R^{2g}, where R^{2f} is hereinbefore defined and R^{2g} is H, by methods known to those skilled in the art. Methods include treatment with an appropriate isocyanate G-N=C=O or, alternatively, by sequential treatment with phosgene or a phosgene equivalent such as triphosgene, 1,1'-carbonyldiimidazole, 1,1'-carbonyl-bis(1,2,4)-triazole, or a chloronitrophenylformate, and the like, followed by treatment with a primary or secondary amine, G-NHR^{2h} in the presence or absence of a suitable base to give glycopeptide antibiotics 11.

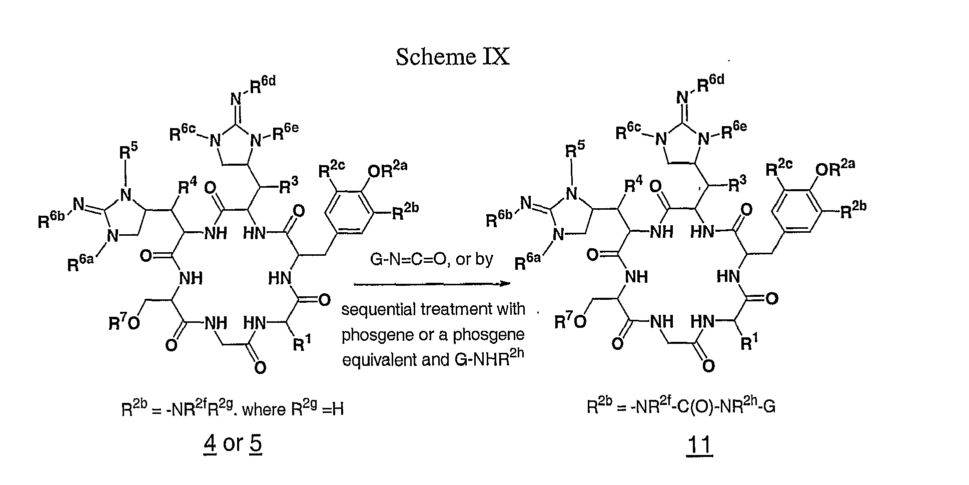

Glycopeptide antibiotics 12, in which R^{2b} is -NR^{2f}R^{2g}, wherein R^{2g} is the group D-E-G wherein D is -C(S)-, E is -O- and G is alkyl(C₁-C₂₀), cycloalkyl(C₃-C₂₀), alkenyl(C₃-C₂₀), alkynyl(C₃-C₂₀), perfluoroalkyl(C₁-C₆), aryl or heteroaryl, may be prepared as shown in Scheme X from primary amine 4 or secondary amine 5 in which R^{2b} is -NR^{2f}R^{2g}, where R^{2f} is hereinbefore defined and R^{2g} is H, by methods known to those skilled in the art. Methods include sequential treatment of primary amine 4 or secondary amine 5 with thiophosgene or a thiophosgene equivalent such as 1,1'-thiocarbonyldiimidazole or 1,1'-thiocarbonyl-bis(1,2,4)-triazole followed by treatment with an alcohol G-OH in the presence or absence of a suitable base to give glycopeptide antibiotics 12.

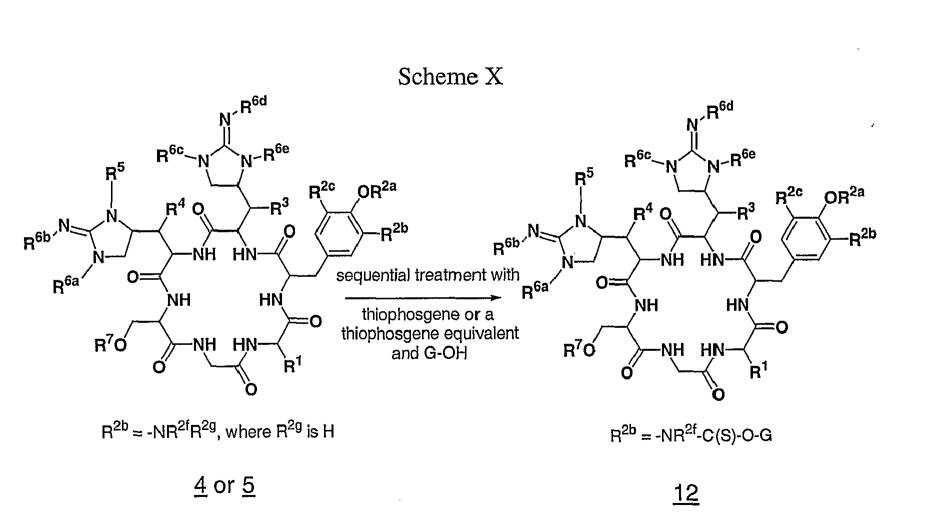

Glycopeptide antibiotics 13, in which R^{2b} is -NR^{2f}R^{2g}, wherein R^{2g} is the group D-E-G, D is -C(S)-, E is -NR^{2h}- and G is alkyl(C₁-C₂₀), cycloalkyl(C₃-C₂₀), alkenyl(C₃-C₂₀), alkynyl(C₃-C₂₀), perfluoroalkyl(C₁-C₆), aryl or heteroaryl, may be prepared as shown in Scheme XI from primary amine 4 or secondary amine 5 in which R^{2b} is -NR^{2f}R^{2g}, where R^{2g} is H and R^{2f} is hereinbefore defined by methods known to those skilled in the art. Methods include treatment of primary amine 4 or secondary amine 5 with an appropriate isothiocyanate G-N=C=S or, alternatively, by sequential treatment with thiophosgene or a thiophosgene equivalent such 1,1'-thiocarbonyldiimidazole or 1,1'-thiocarbonyl-bis(1,2,4)-triazole followed by treatment with a primary or secondary amine G-NHR^{2h} in the presence or absence of a suitable base to give glycopeptide antibiotics 13.

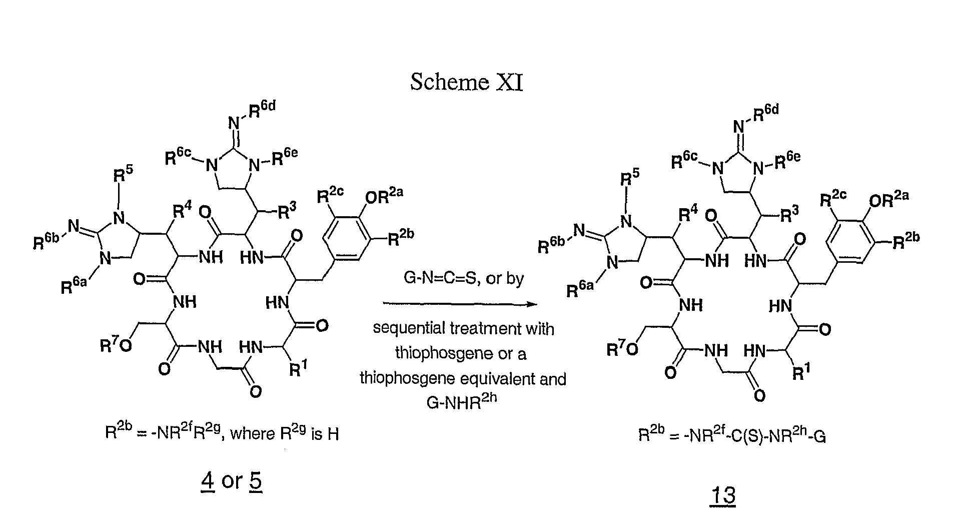

As shown in Scheme XII, glycopeptide antibiotics 14, wherein R^{2d} is alkyl(C₁-C₂₀), alkenyl(C₂-C₂₀), alkynyl(C₂-C₂₀), aryl or heteroaryl, may be prepared from amine 4 in which R^{2b} is -NH₂ by methods known to those skilled in the art. Methods include treatment with an appropriate alkenyl-, alkynyl-, aryl- or heteroaryl- aldehyde R^{2d}-CHO or aldehyde dialkyl-acetal R^{2d}-CH(O-alkyl(C₁-C₂₀))₂ and an excess of an oxidant, such as 2,3-dichloro-5,6-dicyano-1,4-benzoquinone or it's equivalent to give glycopeptide antibiotics 14.

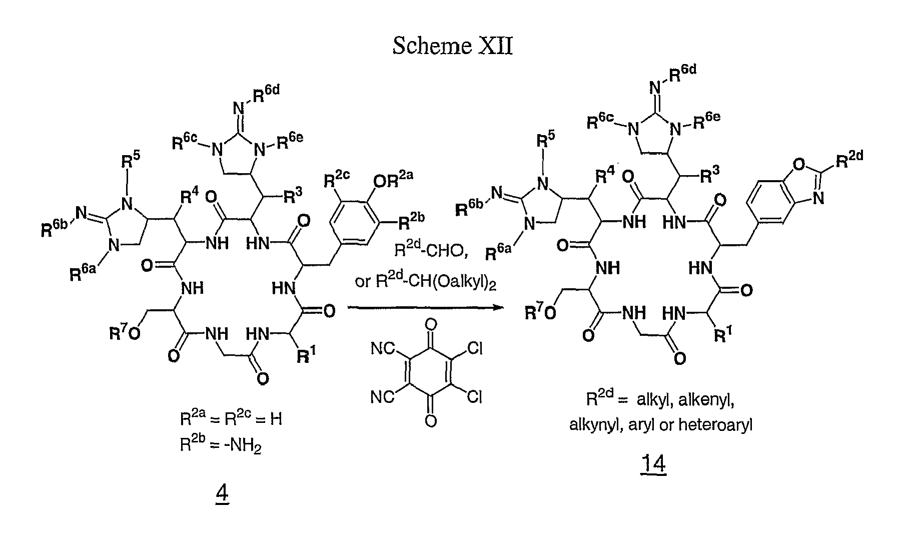

Glycopeptide antibiotics 15 wherein R^{2e} is O, may be prepared as shown in Scheme XIII from an amine 4 by methods known to those skilled in the art. Methods include treatment of amine 4 with phosgene or a phosgene equivalent such as triphosgene, 1,1'-carbonyldiimidazole, 1,1'-carbonyl-bis(1,2,4)-triazole, or a chloronitrophenylformate, in the presence or absence of a suitable base to give glycopeptide antibiotics 15. Alternatively, glycopeptide antibiotics 15 may be prepared from glycopeptide antibiotics 10, by treatment with a suitable base. 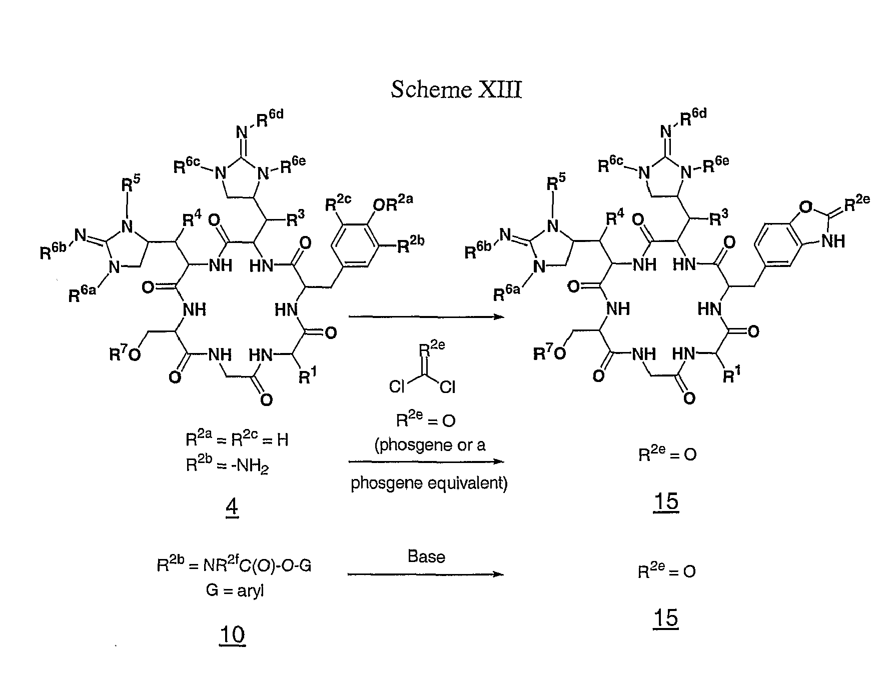 Glycopeptide antibiotics 16 wherein R^{2e} is S, may be prepared as shown in Scheme XTV from amine 4, by methods known to those skilled in the art. Methods include treatment of amine 4 with thiophosgene or a thiophosgene equivalent such as 1,1'-thiocarbonyldiimidazole or 1,1'-thiocarbonyl-bis(1,2,4)-triazole, in the presence or absence of a suitable base to give glycopeptide antibiotics 16. Alternatively, glycopeptide antibiotics 16 may be prepared from glycopeptide antibiotics 12 in which G is aryl, by treatment with a suitable base. 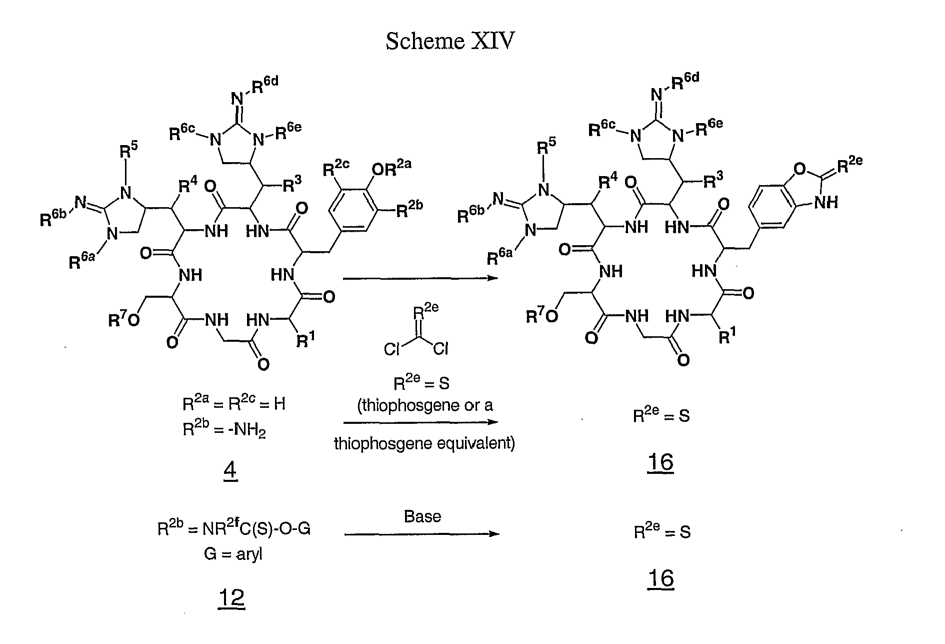 Glycopeptide antibiotics 17 in which R^{2d} is the group L-M, where L is -S- or -SCH₂C(O)-, and M is as described above may be prepared as shown in Scheme XV from glycopeptide antibiotics 16 in which R^{2e} is S, by methods known to those skilled in the art. Methods include treatment of glycopeptide antibiotics 16 with an appropriate alkylating agent M-X or M-C(O)-CH₂-X, where X is Cl, Br, or I, in the presence or absence of a suitable base to give glycopeptide antibiotics 17.

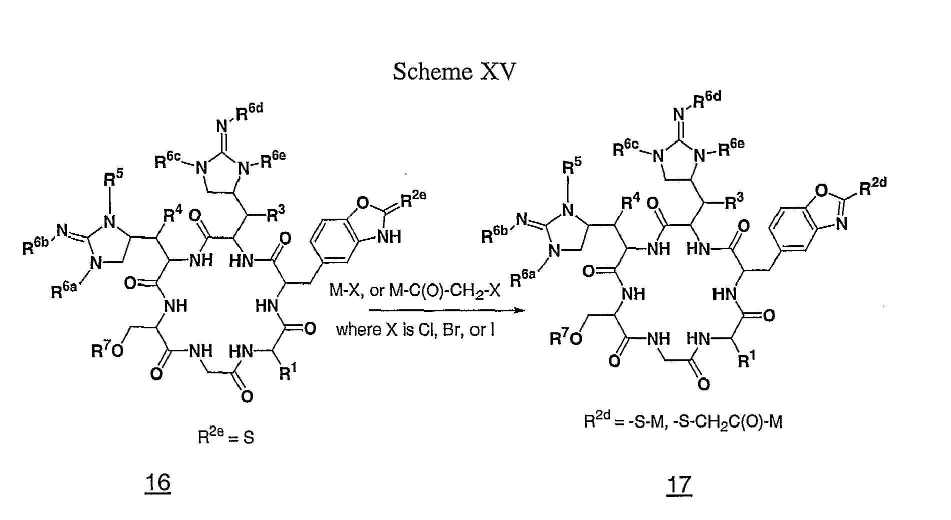

As shown in Scheme XVI, glycopeptide antibiotics 18 in which R^{2d} is the group L-M where L is -NH-, and M is as described above may be prepared from glycopeptide antibiotics 13 in which R^{2b} is -NR^{2f}R^{2g}, wherein R^{2f} is H and R^{2g} is the group D-E-G where D is -C(S)-, E is -NR^{2h}- and G is alkyl(C₁-C₂₀), cycloalkyl(C₃-C₂₀), alkenyl(C₃-C₂₀), alkynyl(C₃-C₂₀), aryl or heteroaryl by methods known to those skilled in the art. Methods include treatment of glycopeptide antibiotics 13 with a suitable mercury (II) salt, such a mercuric chloride, in the presence or absence of a suitable base to give glycopeptide antibiotics 18.

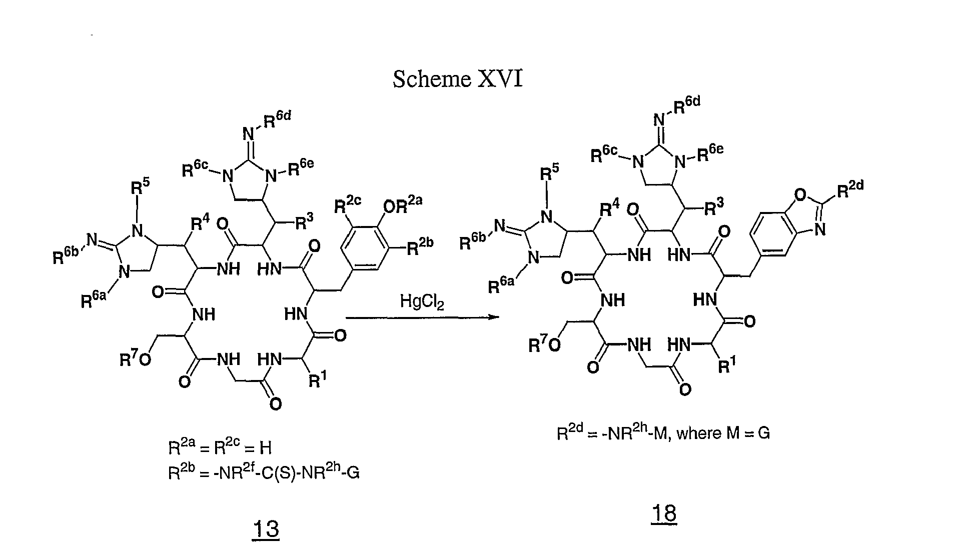

As shown in Scheme XVII, glycopeptide antibiotics 20, may be prepared from glycopeptide antibiotics 19, where R^{1a} is H;
R^{2a} is H or a moiety of the formulae: 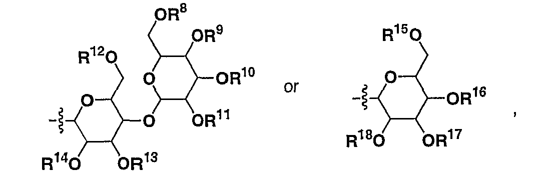 and R^{2b} and R²⁰ are H, by reduction under suitable reducing conditions which include hydrogenation under an atmosphere of hydrogen at pressures from about 1 to about 250 psi, over a suitable catalyst such as rhodium, either alone or adsorbed onto a suitable support such as carbon, alumina or diatomaceous earth in solvents such as water, methanol, ethanol, and the like, alone or in combination, in the presence or absence of a mineral or carboxylic acid, such as hydrochloric acid, sulfuric acid, or acetic acid, and the like, at temperatures from about 25°C to the reflux temperature of the solvent to give glycopeptide antibiotics 20. The extent of the hydrogenation may be controlled by variations in the reaction temperature, hydrogen pressure, amount of catalyst, composition of the catalyst and support, amount of acid additive and the reaction time.

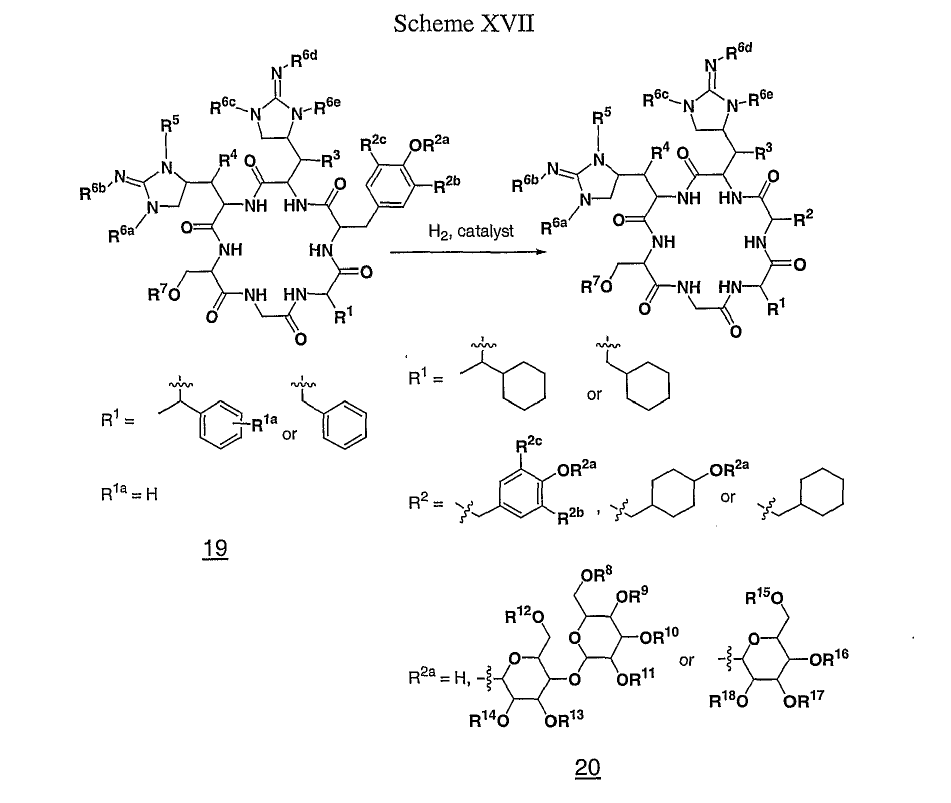

As described in Scheme XVIII, glycopeptide antibiotics 22, may be prepared from the corresponding glycopeptide antibiotics 21 as shown by methods known to those skilled in the art, such as by treatment with an aqueous mineral acid, such as hydrochloric acid, sulfuric acid, nitric acid, and the like, either alone or in a suitable solvent such as methanol, ethanol, water, N,N-dimethylformamide, dimethyl sulfoxide, and the like, at temperatures ranging from about 25°C to about 90°C. Alternatively, and when R⁸, R⁹, R¹⁰, R¹¹, R¹², R¹³, R¹⁴, R¹⁵, R¹⁶, R¹⁷ and R¹⁸ are H, such compounds may also be prepared by treatment with α-mannosidase enzymes, such as purified α-mannosidase derived from sources such as *Canavalia ensiformis* or *Prunus amygdalus* or crude α-mannosidase present in jack bean meal or almond meal, in buffered aqueous systems, preferably in 0.1M sodium acetate buffer at about pH 3.5 to about 6.5, in the presence or absence of added metal salts, such as zinc chloride, and in the presence or absence of added cosolvents.

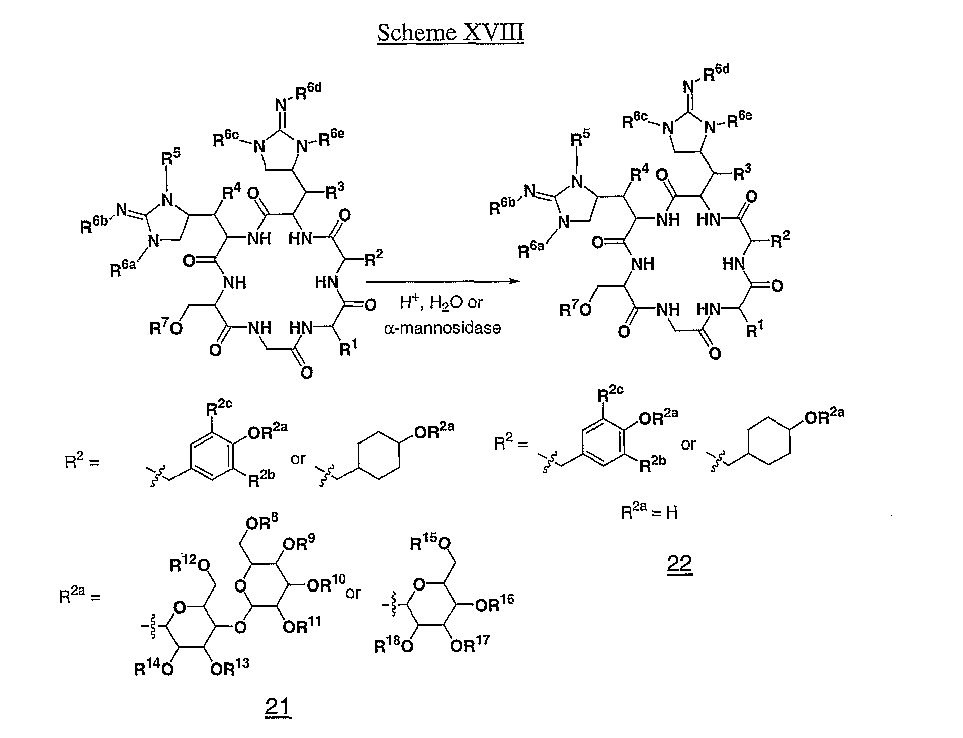

As described in Scheme XIX glycopeptide antibiotics 24, may be prepared from the corresponding glycopeptide antibiotics 23 by treatment with α-mannosidase enzymes, such as purified α-mannosidase readily derived from sources such as *Canavalia ensiformis* or *Prunus amygdalus* or crude a-mannosidase present in jack bean meal or almond meal, in buffered aqueous systems, preferably 0.1M sodium acetate buffer at pH about 3.5 to about 6.5, in the presence or absence of added metal salts such as zinc chloride, and in the presence or absence of added cosolvents.

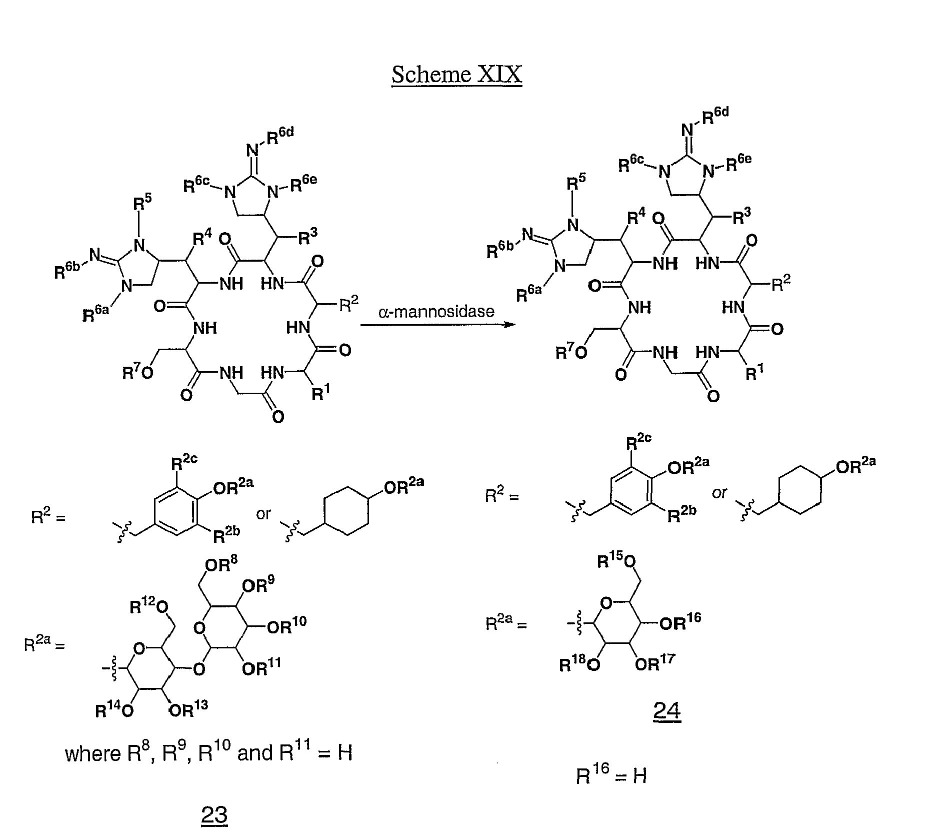

Glycopeptide antibiotics 26 in which R⁵ is H, may be prepared as described in Scheme XX from the corresponding glycopeptide antibiotics 25 as shown in which R¹⁹, R²⁰, R²¹ and R²² are H, by methods known to those skilled in the art, such methods include sequential treatment with sodium periodate, followed by a reductant such as sodium borohydride, followed by an aqueous mineral acid, such as hydrochloric acid, sulfuric acid, nitric acid, and the like, either alone or in a suitable solvent at temperatures ranging from about 25°C to about 90°C.

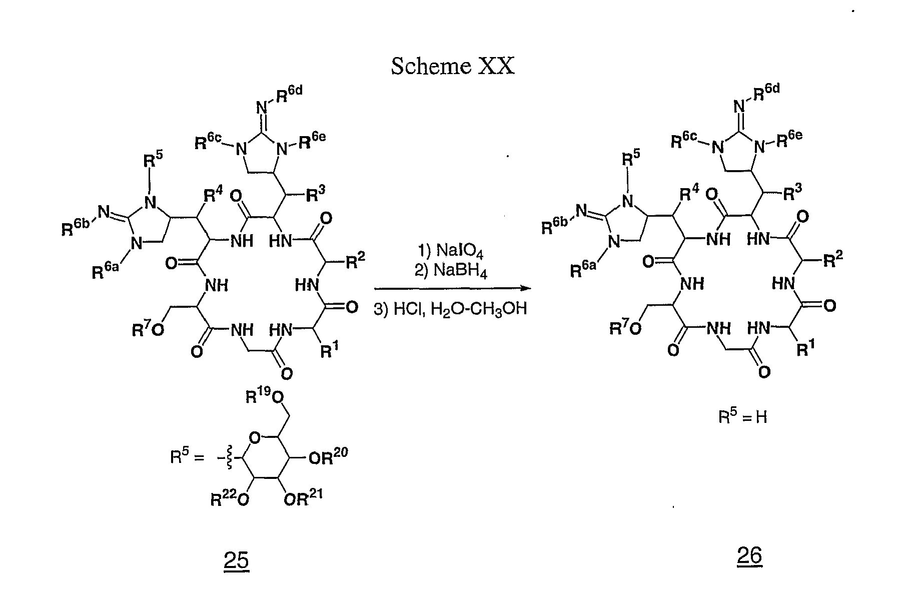

As shown in Scheme XXI, glycopeptide antibiotics 27 in which R⁵ is alkyl(C₁-C₂₀), cyaoalkyl(C₃-C₂₀), alkenyl(C₃-C₂₀) or alkynyl(C₃-C₂₀) may be prepared from the corresponding glycopeptide antibiotics 26 in which R⁵ is H, by methods known to those skilled in the art, such as by alkylation with an appropriate alkylating agent R⁵, where X is Cl, Br, I, -O-tosylate, -O-mesylate, or -O-triflate, in the presence or absence of a suitable base.

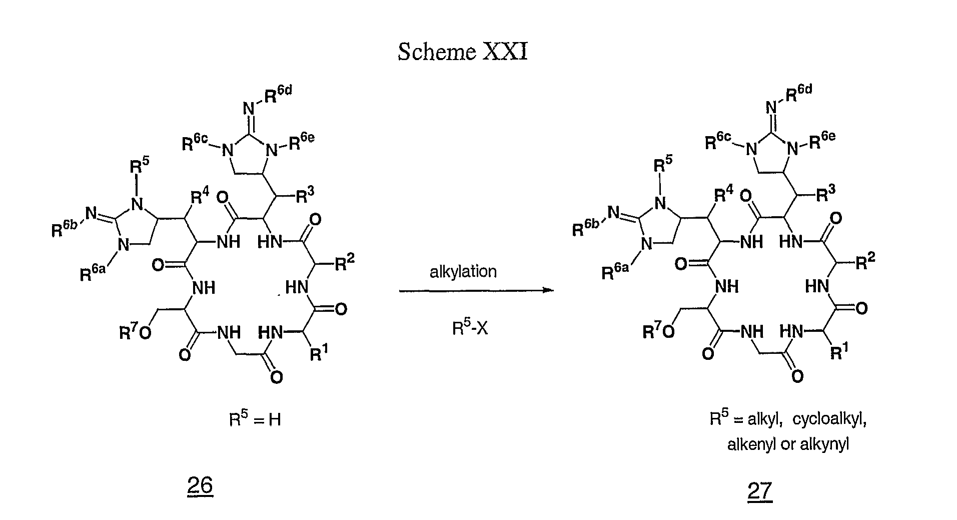

Glycopeptide antibiotics 28, in which R⁵ is -C(O)-Y-Z, wherein Y is a single bond and Z is H, alkyl(C₁-C₂₀), cycloalkyl (C₃-C₂₀), alkenyl(C₂-C₂₀), alkynyl(C₂-C₂₀), perfluoroalkyl(C₁-C₆), aryl or heteroaryl, may be prepared as shown in Scheme XXII from the corresponding compounds 26 in which R⁵ is H, by methods known to those skilled in the art, such as by employing any of a variety of acylation reactions using a carboxylic acid halide Z-C(O)-Cl, carboxylic acid anhydride (Z-C(O))₂-O, or a carboxylic acid Z-C(O)-OH in combination with an appropriate activating agent, such as 1,3-dicyclohexylcarbodiimide, 1-(3-dimethylaminopropyl)-3-ethylcarbodiimide hydrochloride, benzotriazol-1-yloxy-tris(dimethylamino)phosphonium hexafluorophosphate, O-benzotriazol-1-yl-N,N,N',N'-tetramethyluronium hexafluorophosphate, 1,1'-carbonyldiimidazole, and the like, in the presence or absence of a suitable base.

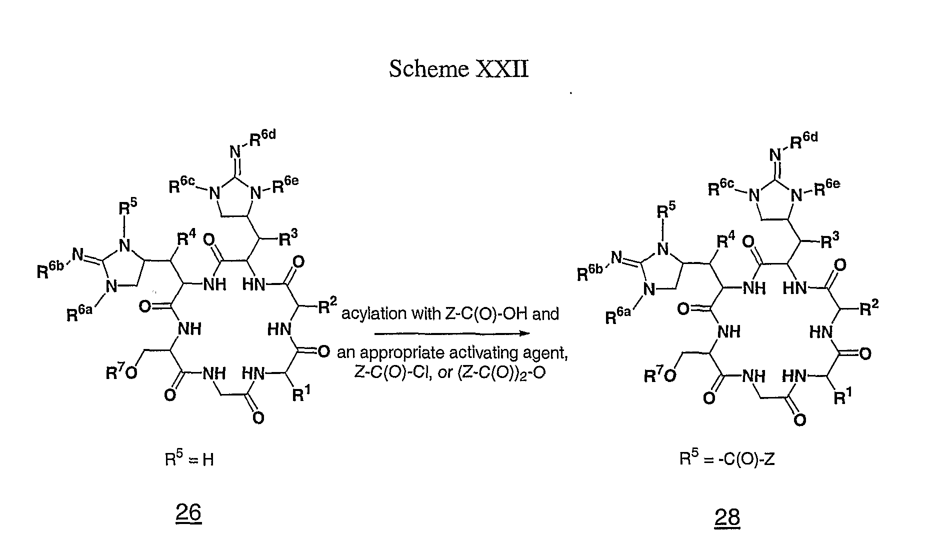

As described in Scheme XXIII glycopeptide antibiotics 29 in which R⁵ is -C(O)-Y-Z, wherein Y is -O- and Z is alkyl(C₁-C₂₀), cycloalkyl(C₃-C₂₀), alkenyl(C₃-C₂₀), alkynyl(C₃-C₂₀), perfluoroalkyl(C₁-C₆), aryl or heteroaryl, may be prepared from the corresponding glycopeptide antibiotics 26 in which R⁵ is H, by methods known to those skilled in the art, such as by treatment with an appropriate chloroformate Z-O-C(O)-Cl or N-hydroxysuccinimide carbonate Z-O-C(O)-OSu, and the like, in the presence or absence of a suitable base.

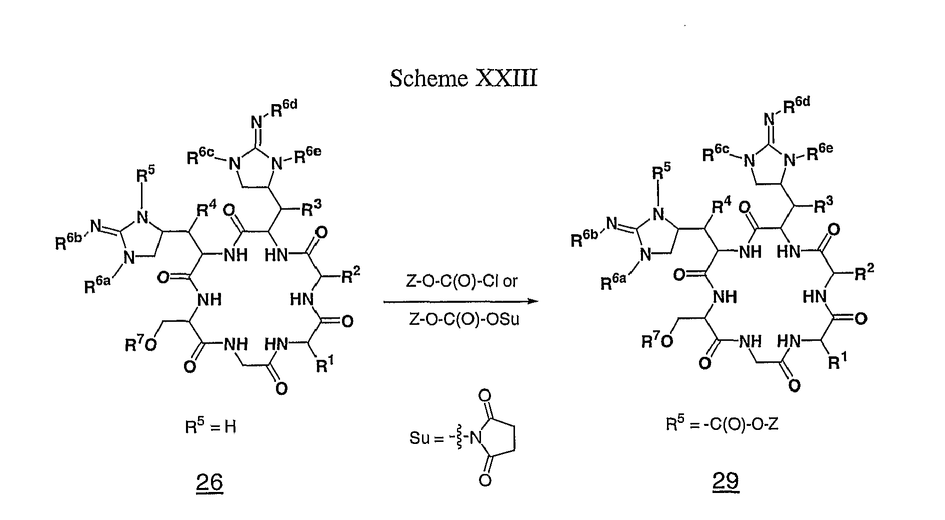

Glycopeptide antibiotics 30 in which R⁵ is -C(O)-Y-Z, wherein Y is -NR^{8a}-and Z is alkyl(C₁-C₂₀), cycloalkyl(C₃-C₂₀), alkenyl(C₃-C₂₀), alkynyl(C₃-C₂₀), perfluoroalkyl(C₁-C₆), aryl or heteroaryl, may be prepared as shown in Scheme XXIV from the corresponding glycopeptide antibiotics 26 in which R⁵ is H, by methods known to those skilled in the art, such as by treatment with appropriate isocyanate Z-N=C=O, or, alternatively, by sequential treatment with phosgene or a phosgene equivalent such as triphosgene, 1,1'-carbonyldiimidazole, 1,1'-carbonyl-bis(1,2,4)-triazole, or a chloronitrophenylformate, and the like, followed by treatment with a primary or secondary amine, Z-NHR^{8a} in the presence or absence of a suitable base. 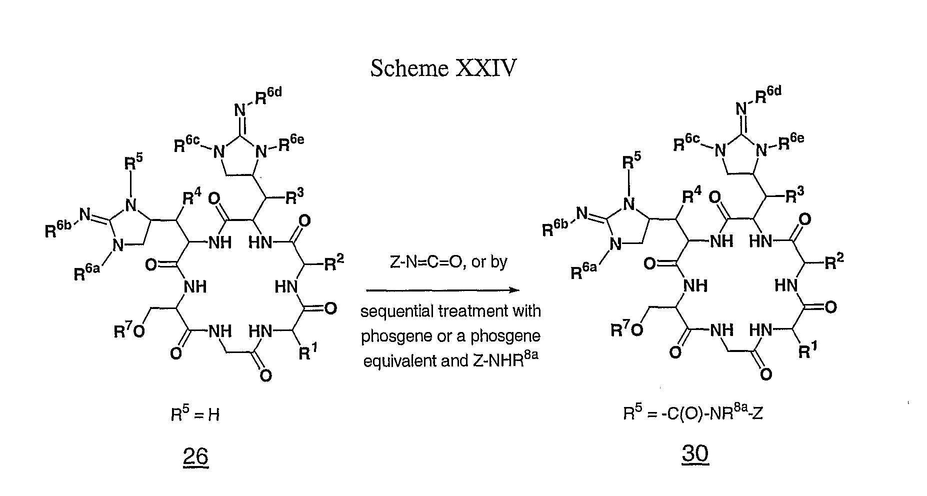 As shown in Scheme XXV, glycopeptide antibiotics 31 in which R⁵ is as shown may be prepared from the corresponding glycopeptide antibiotics 26 in which R⁵ is H, by methods known to those skilled in the art, such as by treatment with aryl halides, tosylates, and triflates, such as a 2-chloropyrimidine, a 2-chlorobenzoxazole, a 2-chlorobenzothiazole, a 4-chlorobenzopyrimidine, a 2-fluoronitrobenzene, a 4-fluoronitrobenzene, and the like, in the presence or absence of a suitable base.

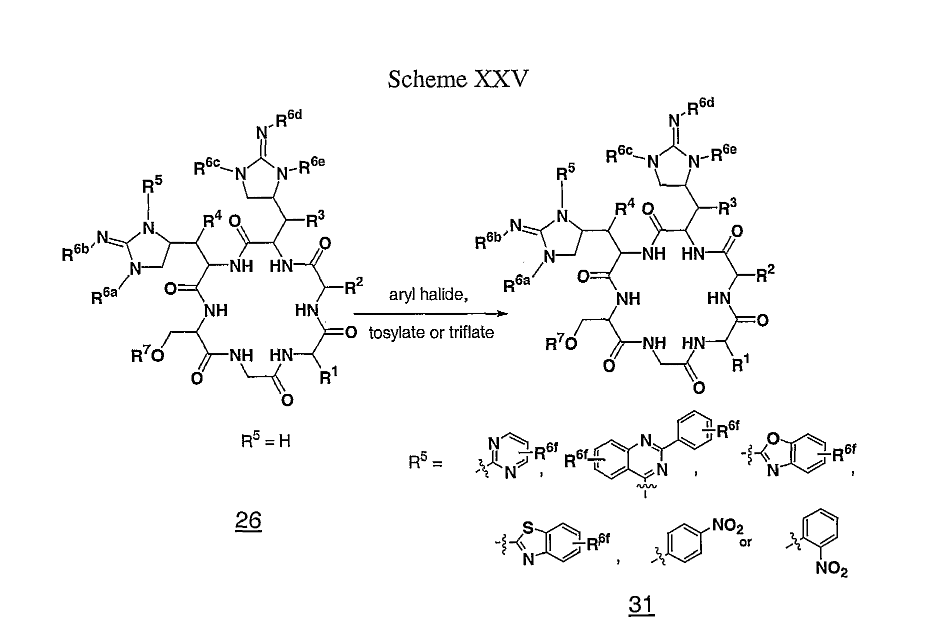

As shown in Scheme XXVI, glycopeptide antibiotics 33 in which R^{6a}, R^{6b}, R^{6c} , R^{6d} or R^{6e} are independently alkyl(C₁-C₂₀), cydoalkyl(C₃-C₂₀), alkenyl(C₃-C₂₀) or alkynyl(C₃-C₂₀) may be prepared from the corresponding glycopeptide antibiotics 32 in which at least one of R^{6a}, R^{6b} , R^{6c} , R^{6a} and R^{6e} are H, by methods known to those skilled in the art, such as by alkylation with an appropriate alkylating agent R^{6a}X, R^{6b}X, R^{6c}X, R^{6d}X or R^{6e}X where X is Cl, Br, I, -O-tosylate, -O-mesylate, or -O-triflate, in the presence or absence of a suitable base. As recognized by those skilled in the art, the extent of alkylation may be controlled by the stoichiometry of the alkylating agent as well as variations in the reaction temperature, and reaction time.

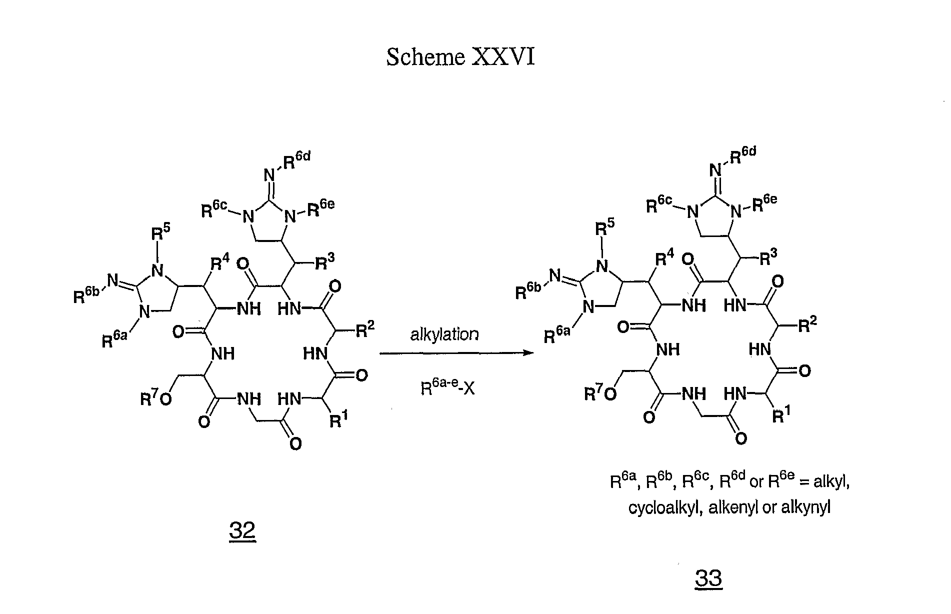

As shown in Scheme XXVII, glycopeptide antibiotics 34 in which R^{6a}, R^{6b}, R^{6c} , R^{6d} or R^{6e} are independently -C(O)-Y-Z, wherein Y is a single bond and Z is H, alkyl(C₁₋C₂₀), cycloalkyl(C₃-C₂₀), alkenyl(C₂-C₂₀), alkynyl(C₂-C₂₀), perfluoroalkyl(C₁-C₆), aryl or heteroaryl, may be prepared from the corresponding glycopeptide antibiotics 32 in which at least one of R^{6a} , R^{6b}, R^{6c} , R^{6d} and R^{6e} are H, by methods known to those skilled in the art, such as by employing any of a variety of acylation reactions using reagents such as a carboxylic acid halide Z-C(O)-Cl, carboxylic acid anhydride (Z-C(O))₂-O, or a carboxylic acid Z-C(O)-OH in combination with an appropriate activating agent, such as 1,3-dicyclohexylcarbodiimide, 1-(3-dimethylaminopropyl)-3-ethylcarbodiimide hydrochloride, benzotriazol-1-yloxy-tris(dimethylamino)phosphonium hexafluorophosphate, O-benzotriazol-1-yl-N,N,N',N'-tetramethyluronium hexafluorophosphate, 1,1'-carbonyldiimidazole, and the like, in the presence or absence of a suitable base. The extent of acylation may be controlled by variations in reagent stoichiometry, reaction temperature, and reaction time.

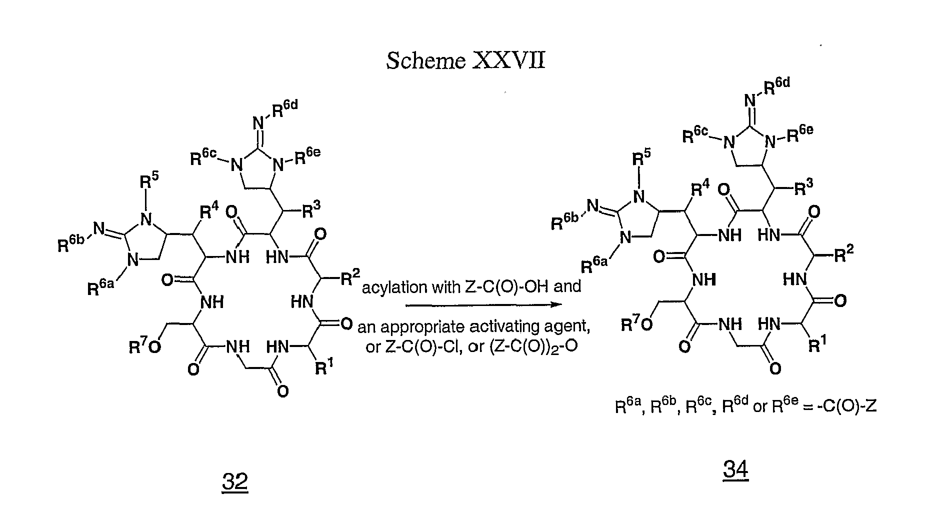

As shown in Scheme XXVIII glycopeptide antibiotics 35 in which R^{6a}, R^{6b}, R^{6c}, R^{6d} or R^{6e} are independently -C(O)-Y-Z, wherein Y is -0- and Z is alkyl(C₁-C₂₀), cycloalkyl(C₃-C₂₀), alkenyl(C₃-C₂₀), alkynyl(C₃-C₂₀), perfluoroalkyl(C₁-C₆), aryl or heteroaryl, may be prepared from the corresponding glycopeptide antibiotics 32 in which at least one of R^{6a}, R^{6b}, R^{6c}, R^{6a} and R^{6e} are H, by methods known to those skilled in the art, such as by treatment with reagents which include an appropriate chloroformate Z-O-C(O)-Cl or N-hydroxysuccinimide carbonate Z-O-C(O)-OSu, and the like, in the presence or absence of a suitable base. The extent of acylation may be controlled by variations in reagent stoichiometry, reaction temperature, and reaction time.

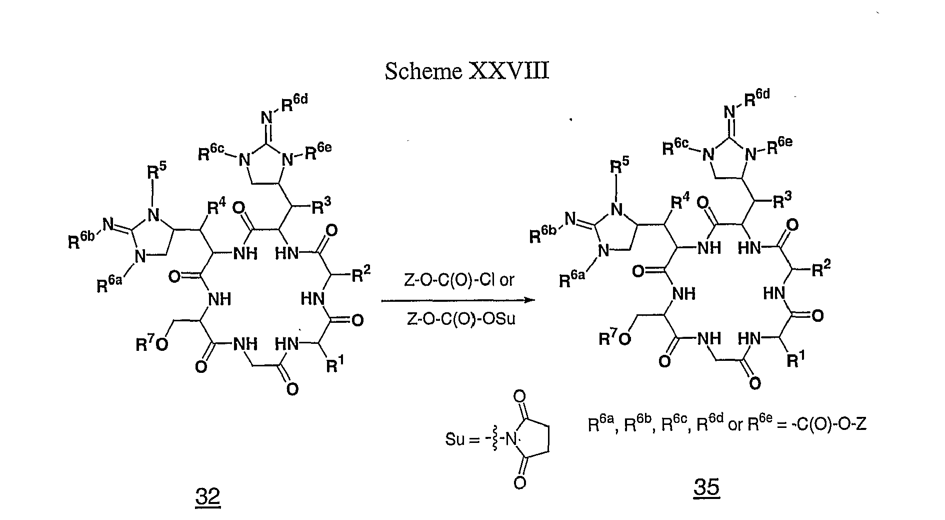

Glycopeptide antibiotics 36 in which R^{6a}, R^{6b}, R^{6c}, R^{6d} or R^{6e} are independently -C(O)-Y-Z, wherein Y is -NR^{8a}- and Z is alkyl(C₁-C₂₀), cycloalkyl(C₃-C₂₀), alkenyl(C₃-C₂₀), alkynyl(C₃-C₂₀), aryl or heteroaryl, may be prepared as shown in Scheme XXIX from the corresponding glycopeptide antibiotics 32 in which at least one of R^{6a}, R^{6b}, R^{6c} , R^{6d} and R^{6e} are H, by methods known to those skilled in the art, such as by treatment with reagents which include an appropriate isocyanate Z-N=C=O, or, alternatively, by sequential treatment with reagents which include phosgene or a phosgene equivalent such as triphosgene, 1,1'-carbonyldiimidazole, 1,1'-carbonyl-bis(1,2,4)-triazole, or a chloronitrophenylformate, and the like, followed by treatment with a primary or secondary amine, Z-NHR^{8a} in the presence or absence of a suitable base. The extent of acylation may be controlled by variations in reagent stoichiometry, reaction temperature, and reaction time.

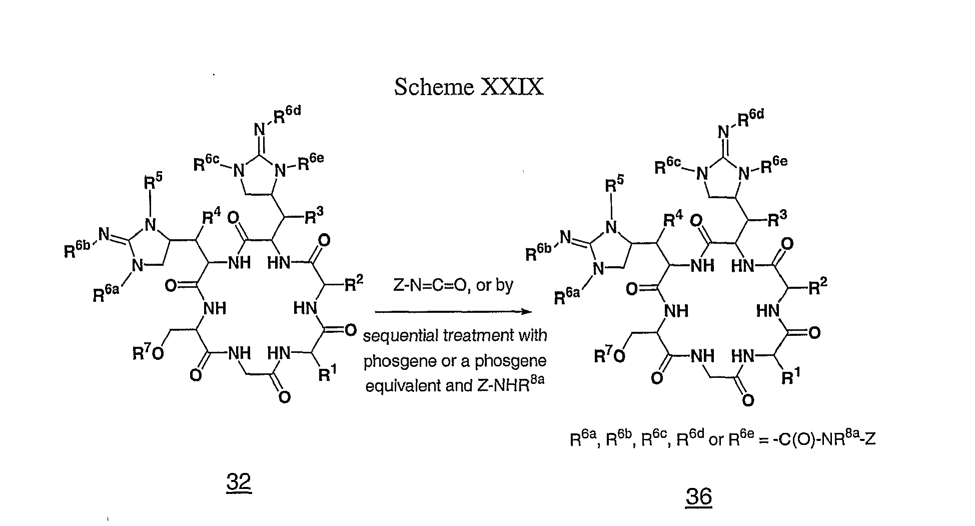

As shown in Scheme XXX, glycopeptide antibiotics 37 in which R^{6a}, R^{6b}, R^{6c}, R^{6d} and R^{6e} are independently as shown may be prepared from the corresponding glycopeptide antibiotics 32 in which at least one of R^{6a}, R^{6b}, R^{6c}, R^{6d} and R^{6e} are H, by methods known to those skilled in the art, such as by treatment with reagents which include appropriate aryl halides, tosylates, and triflates, such as a 2-chloropyrimidine, a 2-chlorobenzoxazole, a 2-chlorobenzothiazole, a 4-chlorobenzopyrimidine, a 2-fluoronitrobenzene, a 4-fluoronitrobenzene, and the like, in the presence or absence of a suitable base. The extent of alkylation may be controlled by variations in reagent stoichiometry, reaction temperature, and reaction time.

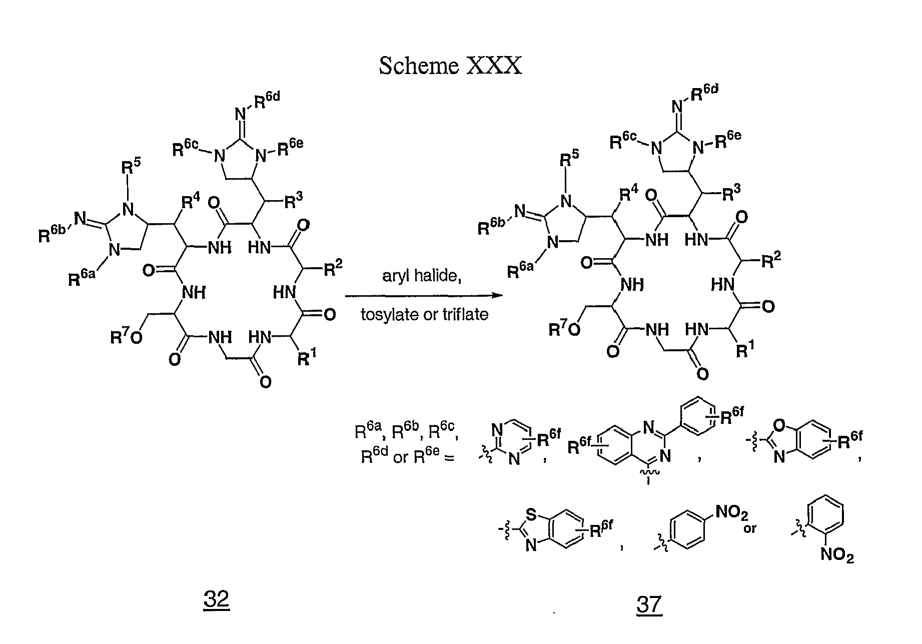

As shown in Scheme XXXI, glycopeptide antibiotics 39 in which R⁷ is -C(O)-Y-Z, wherein Y is a single bond and Z is H, alkyl(C₁-C₂₀), cycloalkyl(C₃-C₂₀), alkenyl(C₂-C₂₀), alkynyl(C₂-C₂₀), perfluoroalkyl(C₁-C₆), aryl or heteroaryl, may be prepared from the corresponding glycopeptide antibiotics 38 in which R⁷ is H, by methods known to those skilled in the art, such as by employing any of a variety of acylation reactions using a carboxylic acid halide Z-C(O)-Cl, carboxylic acid anhydride (Z-C(O))₂-O, or a carboxylic acid Z-C(O)-OH in combination with an appropriate activating agent, such as 1,3-dicyclohexylcarbodiimide, 1-(3-dimethylaminopropyl)-3-ethylcarbodiimide hydrochloride, benzotriazol-1-yloxy-tris(dimethylamino)phosphonium hexafluorophosphate, O-benzotriazol-1-yl-N,N,N',N'-tetramethyluronium hexafluorophosphate, 1,1'-carbonyldiimidazole, and the like, in the presence or absence of a suitable base.

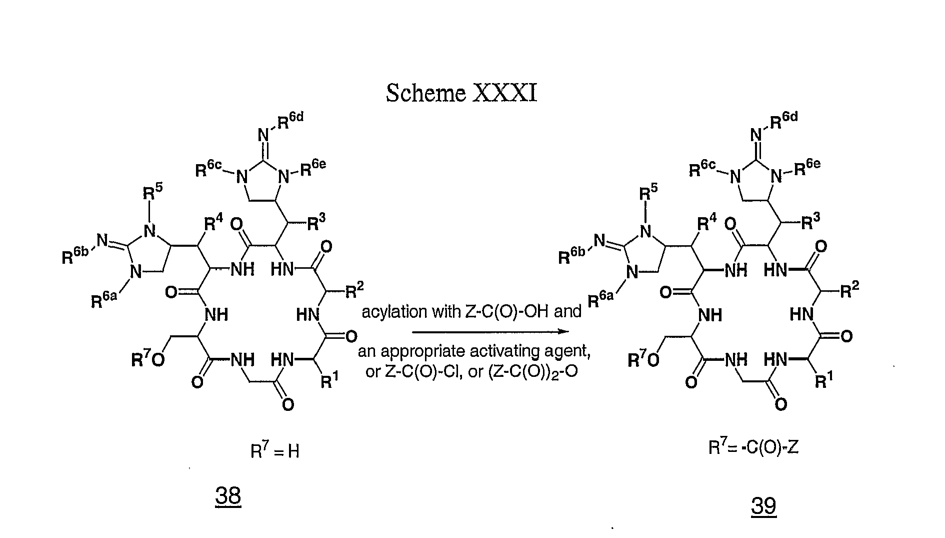

Glycopeptide antibiotics 40 in which R⁷ is -C(O)-Y-Z, wherein Y is -0- and Z is alkyl(C₁-C₂₀₎, cycloalkyl(C₃₋C₂₀), alkenyl(C₃-C₂₀), alkynyl(C₃-C₂₀), perfluoroalkyl(C₁-C₆), aryl or heteroaryl, may be prepared as shown in Scheme XXXII from the corresponding glycopeptide antibiotics 38 in which R⁷ is H, by methods known to those skilled in the art, such as by treatment with an appropriate chloroformate Z-O-C(O)-Cl or N-hydroxysuccinimide carbonate Z-O-C(O)-OSu or, alternatively, by sequential treatment with phosgene or a phosgene equivalent such as triphosgene, 1,1'-carbonyldiimidazole, 1,1'-carbonyl-bis(1,2,4)-triazole, or a chloronitrophenylformate, and the like, followed by treatment with an alcohol, Z-OH, in the presence or absence of a suitable base.

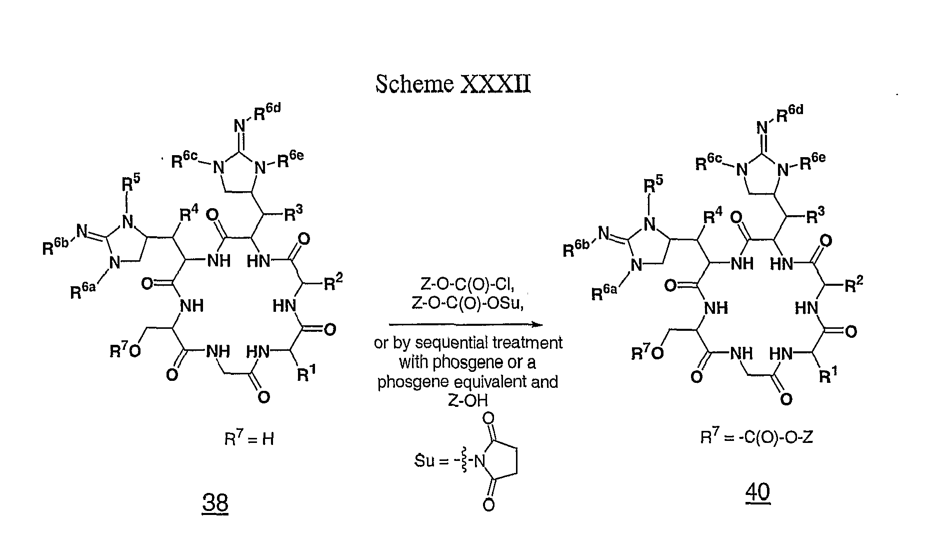

As shown in Scheme XXXIII glycopeptide antibiotics 41 in which R⁷ is -C(O)-Y-Z, wherein Y is -NR^{8a}- and Z is alkyl(C₁-C₂₀), cycloalkyl(C₃-C₂₀), allcenyl(C₃-C₂₀), alkynyl(C₃-C₂₀), perfluoroalkyl(C₁-C₆), aryl or heteroaryl, may be prepared from the corresponding compounds 38 in which R⁷is H, by methods known to those skilled in the art, such as by treatment with appropriate isocyanate Z-N=C=O or, alternatively, by sequential treatment with phosgene or a phosgene equivalent such as triphosgene, 1,1'-carbonyldiimidazole, 1,1'-carbonyl-bis(1,2,4)-triazole, or a chloronitrophenylformate, and the like, followed by treatment with an amine Z-NHR^{8a}, in the presence or absence of a suitable base.

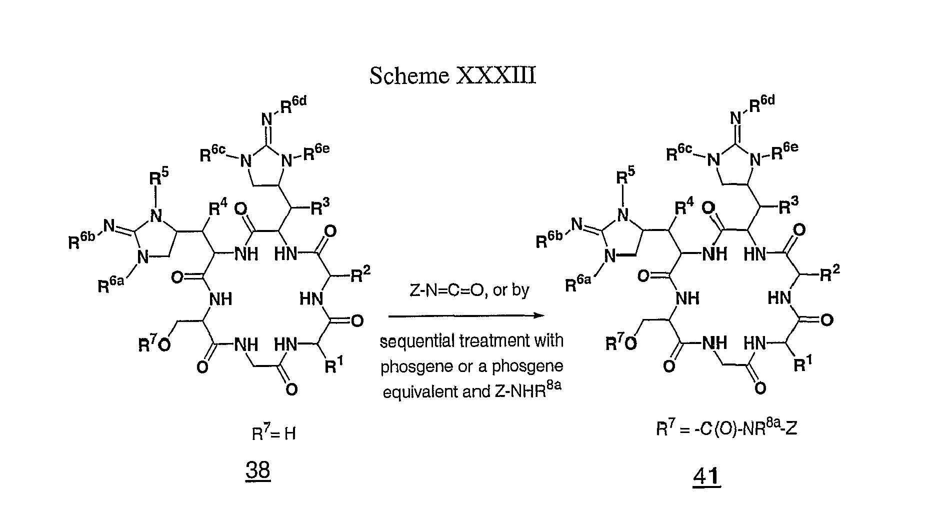

As shown in Scheme XXXIV glycopeptide antibiotics 42 in which R⁷ is alkyl(C₁-C₂₀), cycloalkyl(C₃-C₂₀), alkenyl(C₃-C₂₀) or alkynyl(C₃-C₂₀), may be prepared from the corresponding glycopeptide antibiotics 38 in which R⁷is H, by methods known to those skilled in the art, such as by alkylation with an appropriate alkylating agent R⁷-X, where X is Cl, Br, I, -O-tosylate, -O-mesylate, or -O-triflate, in the presence or absence of a suitable base.

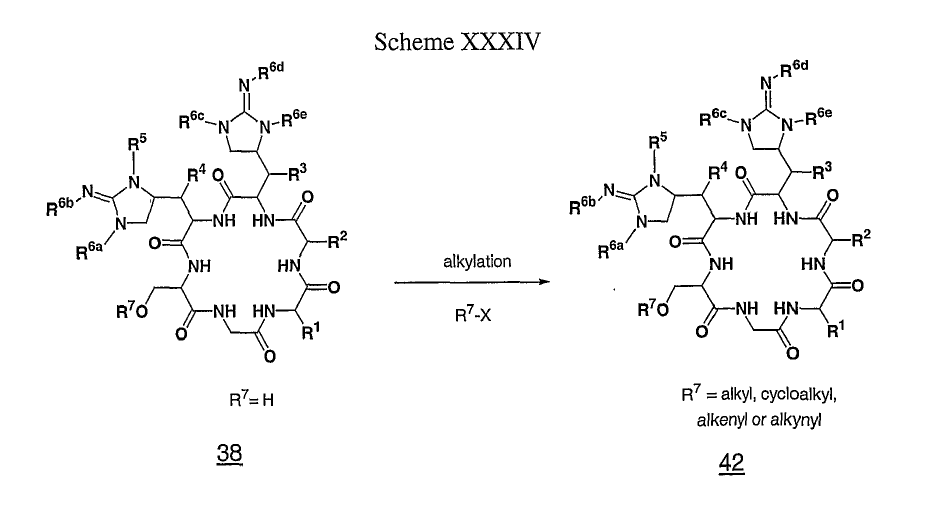

Glycopeptide antibiotics 43 in which R⁷ is trialkylsilyl may be prepared from the corresponding glycopeptide antibiotics 38 as shown in Scheme XXXV in which R⁷is H, by methods known to those skilled in the art, such as by treatment with an appropriate silylating agent, such as trimethylsilyl chloride, trimethylsilyl triflate, t-butyldimethylsilyl chloride, t-butyldimethylsilyl triflate, t-butyldiphenylsilyl triflate, and comparable silylating agents commonly used to protect alcohol functionality, in the presence or absence of a suitable base.

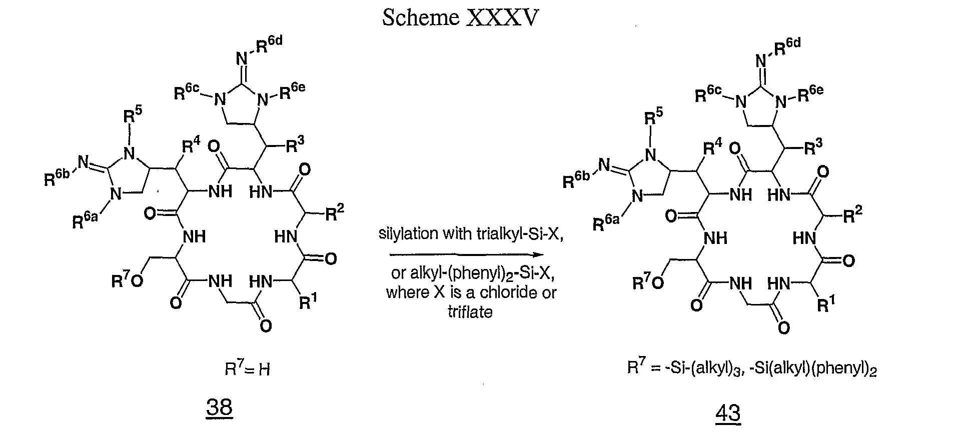

As shown in Scheme XXXVI glycopeptide antibiotics 45 in which R^{2a}, is as shown and R⁸, R⁹, R¹⁰, R¹¹, R¹², R¹³, R¹⁴, R¹⁵, R¹⁶, R¹⁷ or R¹⁸ are independently -C(O)-Y-Z, wherein Y is a single bond and Z is H, alkyl(C₁-C₂₀), cycloalkyl(C₃-C₂₀), alkenyl(C₂-C₂₀), alkynyl(C₂-C₂₀), perfluoroalkyl(C₁-C₆), aryl or heteroaryl, may be prepared from the corresponding glycopeptide antibiotics 44 in which R^{2a} is, as shown and at least one of R⁸, R⁹, R¹⁰, R¹¹, R¹², R¹³, R¹⁴, R¹⁵, R¹⁶, R¹⁷ and R¹⁸ are H, by methods known to those skilled in the art, such as by employing any of a variety of acylation reactions using reagents such as a carboxylic acid halide Z-C(O)-Cl, carboxylic acid anhydride (Z-C(O))₂-O, or a carboxylic acid Z-C(O)-OH in combination with an appropriate activating agent, such as 1,3-dicyclohexylcarbodiimide, 1-(3-dimethylaminopropyl)-3-ethylcarbodiimide hydrochloride, benzotriazol-1-yloxy-tris(dimethylamino)phosphonium hexafluorophosphate, O-benzotriazol-1-yl-N,N,N',N'-tetramethyluronium hexafluorophosphate, 1,1'-carbonyldiimidazole, and the like, in the presence or absence of a suitable base. The extent of acylation may be controlled by variations in reagent stoichiometry, reaction temperature, and reaction time.

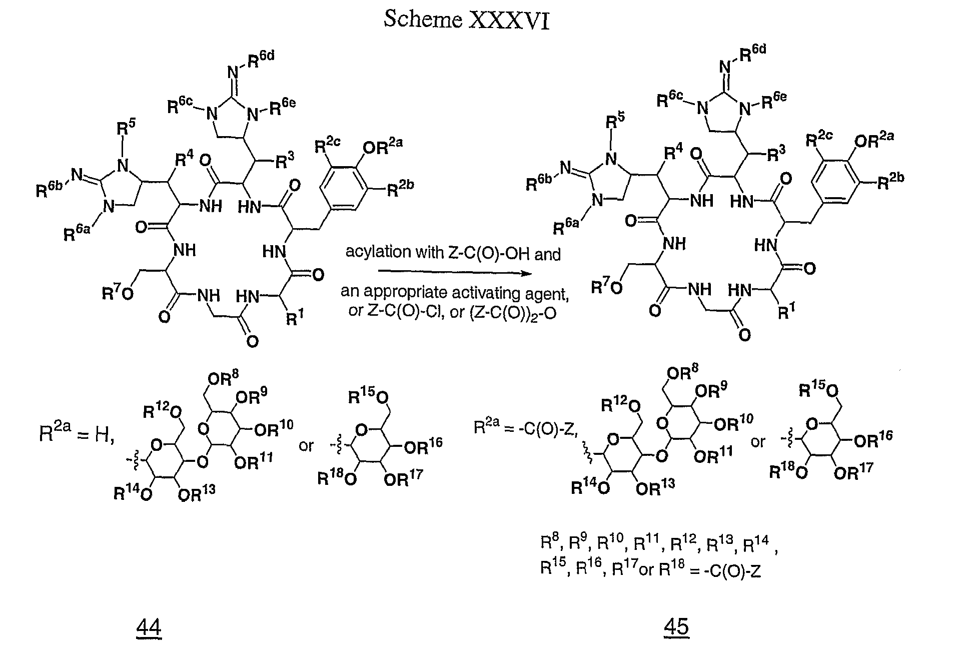

As shown in Scheme XXXVII glycopeptide antibiotics 46 in which R^{2a} is as shown and R⁸, R⁹, R¹⁰, R¹¹, R¹², R¹³, R¹⁴; R¹⁵, R¹⁶, R¹⁷ or R¹⁸ are independently -C(O)-Y-Z, wherein Y is -O- and Z is alkyl(C₁-C₂₀), cycloalkyl(C₃-C₂₀), alkenyl(C₃-C₂₀), alkynyl(C₃-C₂₀), perfluoroalkyl(C₁-C₆), aryl or heteroaryl, may be prepared from the corresponding glycopeptide antibiotics 44 in which R^{2a} is as shown and at least one of R⁸, R⁹, R¹⁰, R¹¹, R¹², R¹³, R¹⁴; R¹⁵, R¹⁶, R¹⁷ and R¹⁸ are H, by methods known to those skilled in the art, such as by treatment with reagents which include an appropriate chloroformate Z-O-C(O)-Cl or N-hydroxysuccinimide carbonate Z-O-C(O)-OSu or, alternatively, by sequential treatment with reagents such as phosgene or a phosgene equivalent such as triphosgene, 1,1'-carbonyldiimidazole, 1,1'-carbonyl-bis(1,2,4)-triazole, or a chloronitrophenylformate, and the like, followed by treatment with an alcohol Z-OH, in the presence or absence of a suitable base. The extent of reaction may be controlled by variations in reagent stoichiometry, reaction temperature, and reaction time.

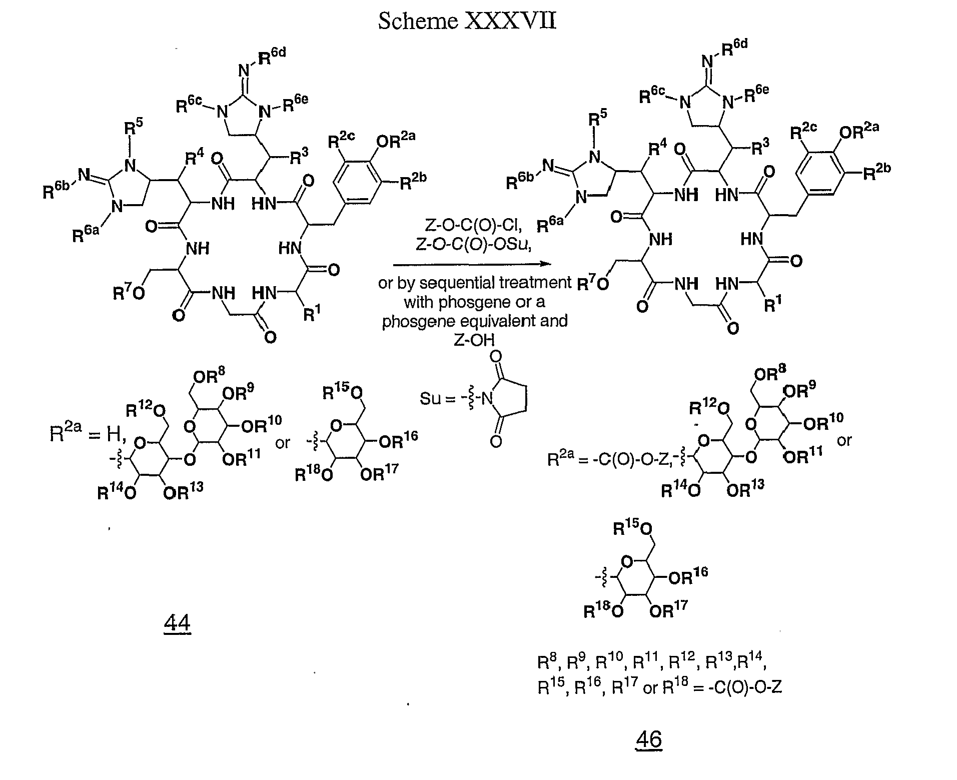

As shown in Scheme XXXVIII glycopeptide antibiotics 47 in which R^{2a} is as shown and R⁸, R⁹, R¹⁰, R¹¹, R¹², R¹³, R¹⁴; R¹⁵, R¹⁶, R¹⁷ and R¹⁸ are independently -C(O)-Y-Z, wherein Y is -NR^{8a}- and Z is alkyl(C₁-C₂₀), cycloalkyl(C₃-C₂₀), alkenyl(C₃-C₂₀), alkynyl(C₃-C₂₀), perfluoroalkyl(C₁-C₆), aryl or heteroaryl, may be prepared from the corresponding glycopeptide antibiotics 44 in which R^{2a} is as shown and at least one of R⁸, R⁹, R¹⁰, R¹¹, R¹², R¹³, R¹⁴; R¹⁵, R¹⁶, R¹⁷ and R¹⁸ are H, by methods known to those skilled in the art, such as by treatment with reagents which include an an appropriate isocyanate Z-N=C=O or, alternatively, by sequential treatment with reagents which include phosgene or a phosgene equivalent such as triphosgene, 1,1'-carbonyldiimidazole, 1,1'-carbonyl-bis(1,2,4)-triazole, or a chloronitrophenylformate, and the like, followed by treatment with an amine Z-NHR^{8a}, in the presence or absence of a suitable base. The extent of reaction may be controlled by variations in reagent stoichiometry, reaction temperature, and reaction time.

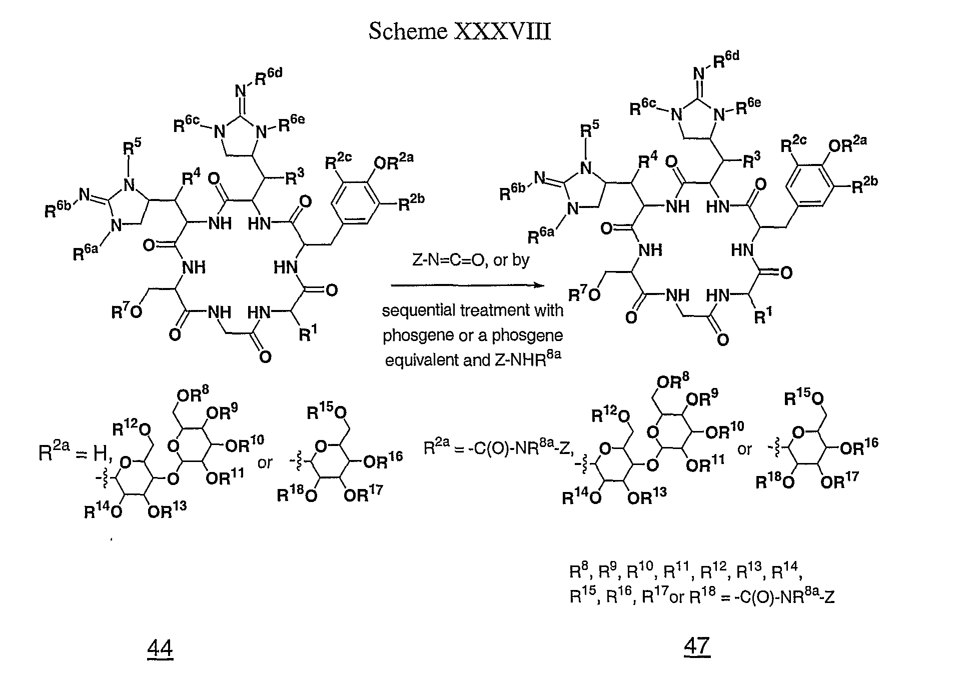

As shown in Scheme XXXIX glycopeptide antibiotics 49 in which R⁸ and R⁹, R⁹ and R¹⁰, R¹⁰ and R¹¹, R¹³ and R¹⁴, R¹⁵ and R¹⁶, R¹⁶ and R¹⁷, or R¹⁷ and R¹⁸ are independently joined forming moieties of the formula: 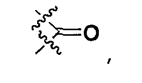 where n is an integer of from 1 to 3 may be prepared from the corresponding glycopeptide antibiotics 48 in which R⁸, R⁹, R¹⁰, R¹¹, R¹³, R¹⁴, R¹⁵, R¹⁶ R¹⁷ and R¹⁸ are H, by methods known to those skilled in the art, such as by treatment with reagents which include phosgene or a phosgene equivalent reagent such as triphosgene, 1,1'-carbonyldiimidazole, 1,1'-carbonyl-bis(1,2,4)-triazole, or a chloronitrophenylformate, in the presence or absence of a suitable base. The extent of the reaction may be controlled by variations in reagent stoichiometry, reaction temperature, and reaction time.

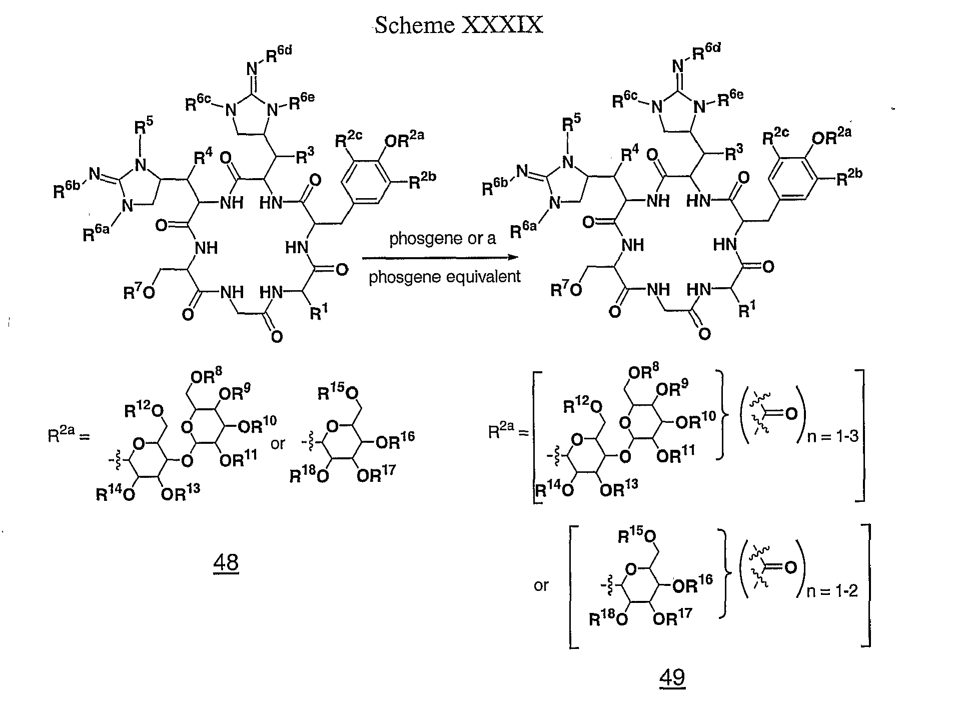

As shown in Scheme XL glycopeptide antibiotics 50 in which R⁸ and R⁹, R⁹ and R¹⁰, R¹⁰ and R¹¹, R¹³ and R¹⁴, R¹⁵ and R¹⁶, R¹⁶ and R¹⁷, or R¹⁷ and R¹⁸ are independently joined forming moieties of the formula: 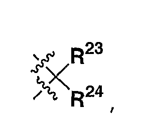 where n is an integer of from 1 to 3 may be prepared from the corresponding glycopeptide antibiotics 48 in which at least one pair of R⁸ and R⁹, R⁹ and R¹⁰, R¹⁰ and R¹¹, R¹³ and R¹⁴, R¹⁵ and R¹⁶, R¹⁶ and R¹⁷, or R¹⁷ and R¹⁸ are H, by methods known to those skilled in the art, such as by treatment with reagents which include an appropriate dialkyl acetal or dialkyl ketal of the formula R²³R²⁴-C(O-alkyl(C₁-C₂₀))₂, such as a dimethyl acetal or dimethyl ketal, in the presence of a suitable acid catalyst, such as hydrochloric acid, p-toluene sulfonic acid mono-hydrate, camphor sulfonic acid, pyridinium p-toluene sulfonate, Amberlyst, or an equivalent mineral acid, carboxylic acid, or sulfonic acid commonly used by those skilled in the art. The extent of the reaction may be controlled by variations in reagent stoichiometry, reaction temperature, and reaction time.

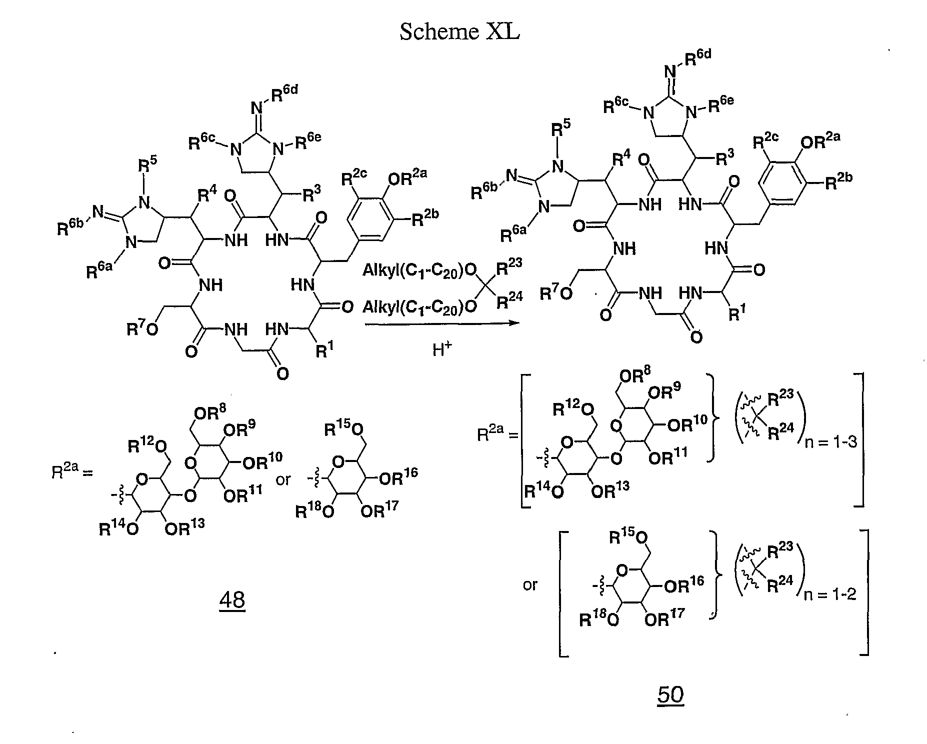

As shown in Scheme XLI, glycopeptide antibiotics 52 in which R⁸, R⁹, R¹⁰, R¹¹, R¹³, R¹⁴, R¹⁵, R¹⁶, R¹⁷ or R¹⁸ are independently -(CH₂)-alkenyl(C₂-C₂₀), -(CH₂)-alkynyl(C₂-C₂₀), -(CH₂)-aryl or -(CH₂)-heteroaryl, may be prepared from the corresponding glycopeptide antibiotics 51 prepared using the conditions described in Scheme XL (by treatment with a compound of the formula R²³R²⁴-C(O-alkyl(C₁-C₂₀))₂ where R²³ is H and R²⁴ is alkenyl(C₂-C₂₀), alkynyl(C₂-C₂₀), aryl, or heteroaryl), in which at least one pair of R⁸ and R⁹, R⁹ and R¹⁰, R¹⁰ and R¹¹, R¹³ and R¹⁴, R¹⁵ and R¹⁶, R¹⁶ and R¹⁷, and R¹⁷ and R¹⁸ are moieties of the formula: 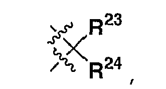 where R²³ is H and R²⁴ is alkenyl(C₂-C₂₀), alkynyl(C₂-C₂₀), aryl, or heteroaryl, and n is an integer of 1 to 3 by methods known to those skilled in the art, such as by treatment with a reductant and a protic acid or Lewis acid. Suitable combinations of reductants and acids include: sodium cyanoborohydride and trifluroacetic acid, sodium cyanoborohydride and hydrochloride acid, triethylsilane-trifluroacetic acid, borane-trimethylamine complex-aluminium chloride, borane-dimethylamine complex-boron trifluoride diethyl etherate, borane-dibutylboron triflate, or lithium aluminium hydride-aluminium chloride, and the like.

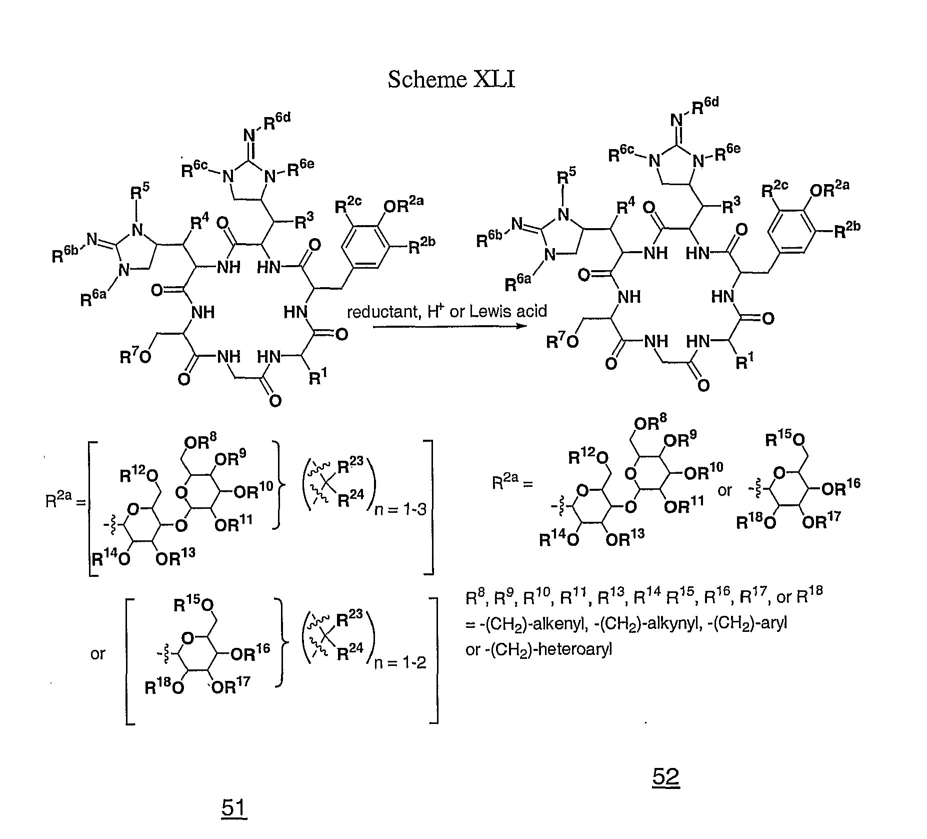

Alternatively, glycopeptide antibiotics 53 in which R^{2a} is as shown and R⁸, R⁹, R¹⁰, R¹¹, R¹², R¹³, R¹⁴, R¹⁵, R¹⁶, R¹⁷ or R¹⁸ are independently alkyl(C₁-C₂₀), cycloalkyl(C₃-C₂₀), alkenyl(C₃-C₂₀) or alkynyl(C₃-C₂₀) may be prepared as described in Scheme XLII from the corresponding glycopeptide antibiotics 44 in which R^{2a} is as shown, and at least one of R⁸, R⁹, R¹⁰, R¹¹, R¹², R¹³, R¹⁴, R¹⁵, R¹⁶, R¹⁷ and R¹⁸ are H, by methods known to those skilled in the art, such as by alkylation with an appropriate alkyling agent R⁸X, R⁹X, R¹⁰X, R¹¹X_{,} R¹²X, R¹³X, R¹⁴X, R¹⁵X, R¹⁶X, R¹⁷X and R¹⁸X, where X is Cl, Br, I, -O-tosylate, -O-mesylate, or -O-triflate, in the presence or absence of a suitable base.

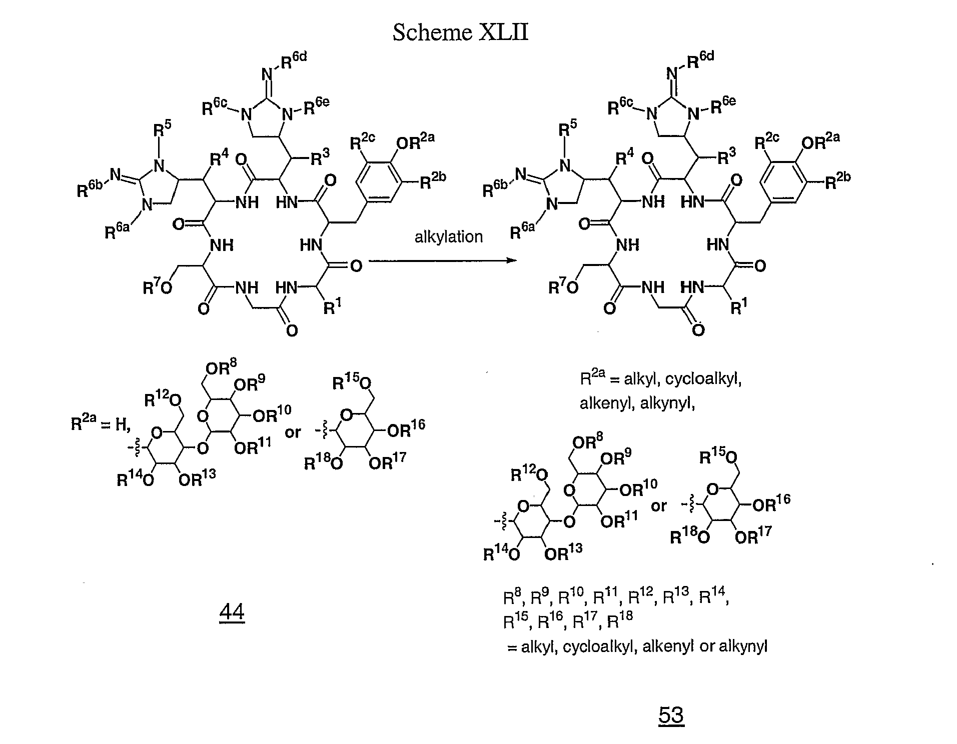

As shown in Scheme XLIII, glycopeptide antibiotics 55 in which R¹⁹, R²⁰ , R²¹ or R²² are independently -C(O)-Y-Z, wherein Y is a single bond and Z is H, alkyl(C₁-C₂₀), cycloalkyl(C₃-C₂₀), alkenyl(C₂-C₂₀), alkynyl(C₂-C₂₀), perfluoroalkyl(C₁-C₆), aryl or heteroaryl, may be prepared from the corresponding glycopeptide antibiotics 54 in which at least one of R¹⁹, R²¹ , R²¹ and R²² are H, by methods known to those skilled in the art, such as by employing any of a variety of acylation reactions using reagents such as a carboxylic acid halide Z-C(O)-Cl, carboxylic acid anhydride (Z-C(O))₂-O, or a carboxylic acid Z-C(O)-OH in combination with an appropriate activating agent, such as 1,3-dicyclohexylcarbodiimide, 1-(3-dimethylaminopropyl)-3-ethylcarbodiimide hydrochloride, benzotriazol-1-yloxy-tris(dimethylamino)phosphonium hexafluorophosphate, O-benzotriazol-1-yl-N,N,N',N'-tetramethyluronium hexafluorophosphate, 1,1'-carbonyldiimidazole, and the like, in the presence or absence of a suitable base. The extent of acylation may be controlled by variations in reagent stoichiometry, reaction temperature, and reaction time.

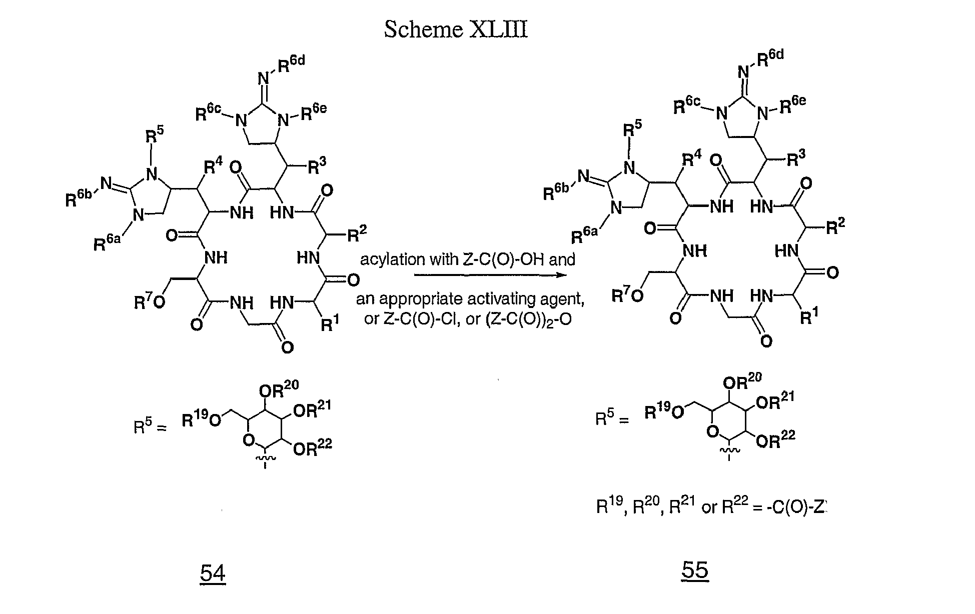

As shown in Scheme XLIV glycopeptide antibiotics 56 in which R¹⁹, R²⁰, R²¹ or R²² are independently -C(O)-Y-Z, wherein Y is -0- and Z is alkyl(C₁-C₂₀), cycloalkyl(C₃-C₂₀), alkenyl(C₃-C₂₀), alkynyl(C₃-C₂₀), perfluoroalkyl(C₁-C₆), aryl or heteroaryl, may be prepared from the corresponding glycopeptide antibiotics 54 in which at least one of R¹⁹, R²¹, R²¹ and R²² are H, by methods known to those skilled in the art, such as by treatment with reagents which include an appropriate chloroformate Z-O-C(O)-Cl or N-hydroxysuccinimide carbonate Z-O-C(O)-OSu or, alternatively, by sequential treatment with reagents which include phosgene or a phosgene equivalent such as triphosgene; 1,1'-carbonyldiimidazole, 1,1'-carbonyl-bis(1,2,4)-triazole, or a chloronitrophenylformate, and the like, followed by treatment with an alcohol Z-OH, in the presence or absence of a suitable base. The extent of acylation may be controlled by variations in reagent stoichiometry, reaction temperature, and reaction time.

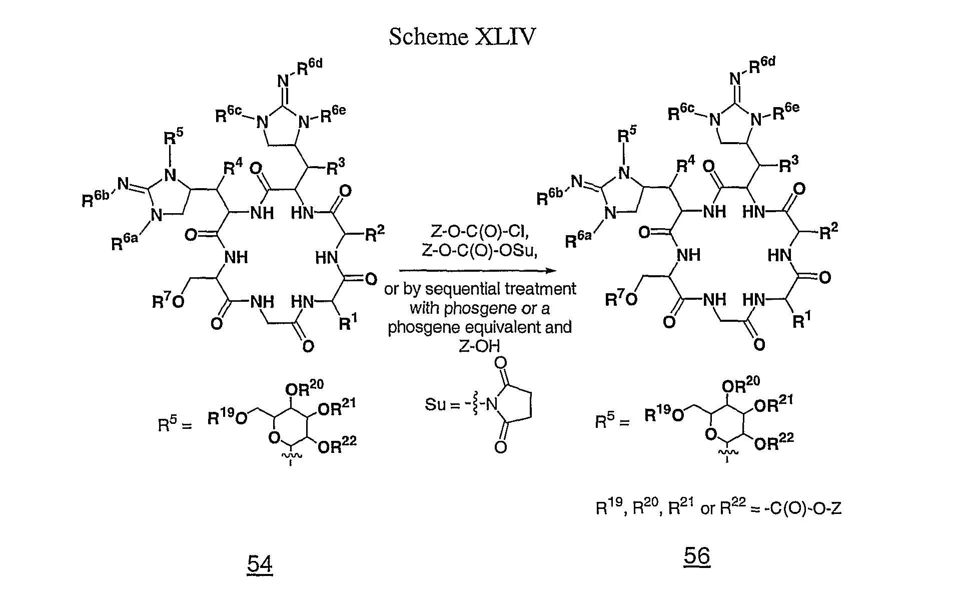

As described in Scheme XLV, glycopeptide antibiotics 57 in which R¹⁹, R²⁰, R²¹ and R²² are independently -C(O)-Y-Z, wherein Y is -NR^{8a}- and Z is alkyl(C₁-C₂₀), cycloalkyl(C₃-C₂₀), alkenyl(C₃-C₂₀), alkynyl(C₃-C₂₀), perfluoroalkyl(C₁-C₆), aryl or heteroaryl, may be prepared from the corresponding glycopeptide antibiotics 54 in which at least one of R¹⁹, R²⁰, R²¹ and R²² are H, by methods known to those skilled in the art, such as by treatment with reagents which include an appropriate isocyanate Z-N=C=O or, alternatively, by sequential treatment with reagents such as phosgene or a phosgene equivalent such as triphosgene, 1,1'-carbonyldiimidazole, 1,1'-carbonyl-bis(1,2,4)-triazole, or a chloronitrophenylformate, and the like, followed by treatment with an amine Z-NHR^{8a} in the presence or absence of a suitable base. The extent of acylation may be controlled by variations in reagent stoichiometry, reaction temperature, and reaction time.

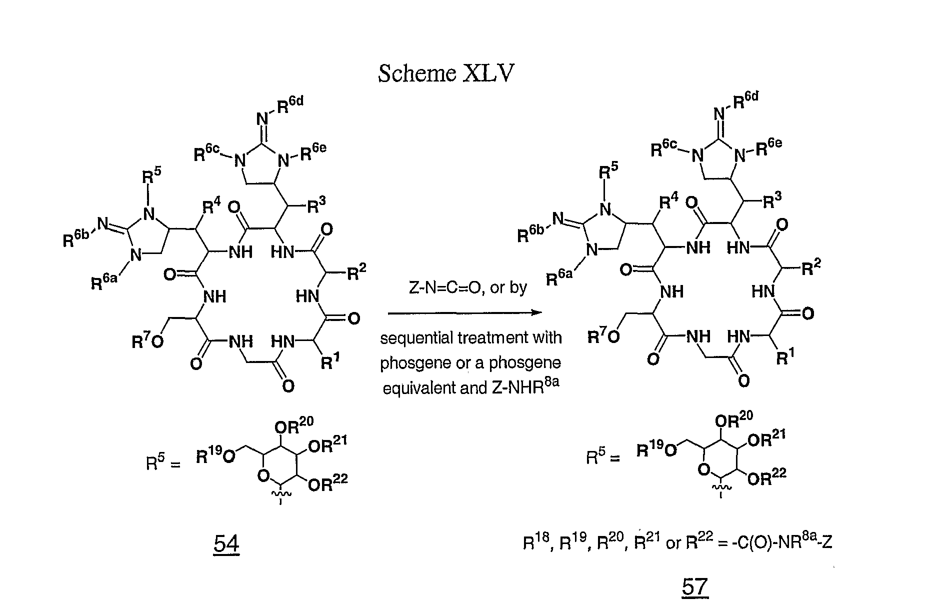

As shown in Scheme XLVI, glycopeptide antibiotics 58 in which R¹⁹ and R²⁰, R²⁰ and R²¹, or R²¹ and R²² are independently joined forming moieties of the formula: 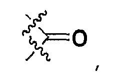 where n is an integer of 1 or 2 may be prepared from the corresponding glycopeptide antibiotics 54 in which at least one pair of R¹⁹ and R²⁰, R²⁰ and R²¹ and R²¹ and R²² are H, by methods known to those skilled in the art, such as by treatment with a reagent such as phosgene or a phosgene equivalent such as triphosgene, 1,1'-carbonyldiimidazole, 1,1'-carbonyl-bis(1,2,4)-triazole, or a chloronitrophenylformate, in the presence or absence of a suitable base. The extent of the reaction may be controlled by variations in reagent stoichiometry, reaction temperature, and reaction time.

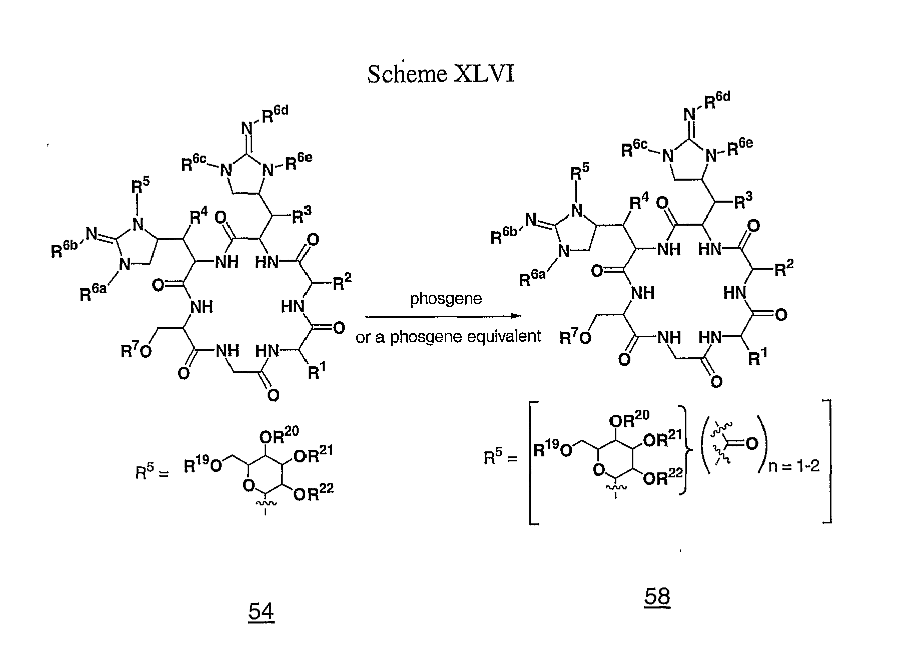

As shown in Scheme XLVII, glycopeptide antibiotics 59 in which R¹⁹ and R²⁰, R²⁰ and R²¹, or R²¹ and R²² are independently joined forming moieties of the formula: 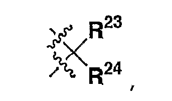 where n is an integer of 1 or 2, may be prepared from the corresponding glycopeptide antibiotics 54 in which at least one pair of R¹⁹ and R²⁰, R²⁰ and R²¹, and R²¹ and R²² are H, by methods known to those skilled in the art, such as by treatment with a dialkyl acetal or dialkyl ketal of the formula R²³R²⁴-C(O-alkyl(C₁-C₂₀))₂, such as a dimethyl acetal or dimethyl ketal, in the presence of a suitable acid catalyst, such as hydrochloric acid, p-toluene sulfonic acid mono-hydrate, camphor sulfonic acid, pyridinium p-toluene sulfonate, Amberlyst, or any equivalent mineral acid, carboxylic acid, or sulfonic acid commonly used by those skilled in the art. The extent of the reaction may be controlled by variations in reagent stoichiometry, reaction temperature, and reaction time.

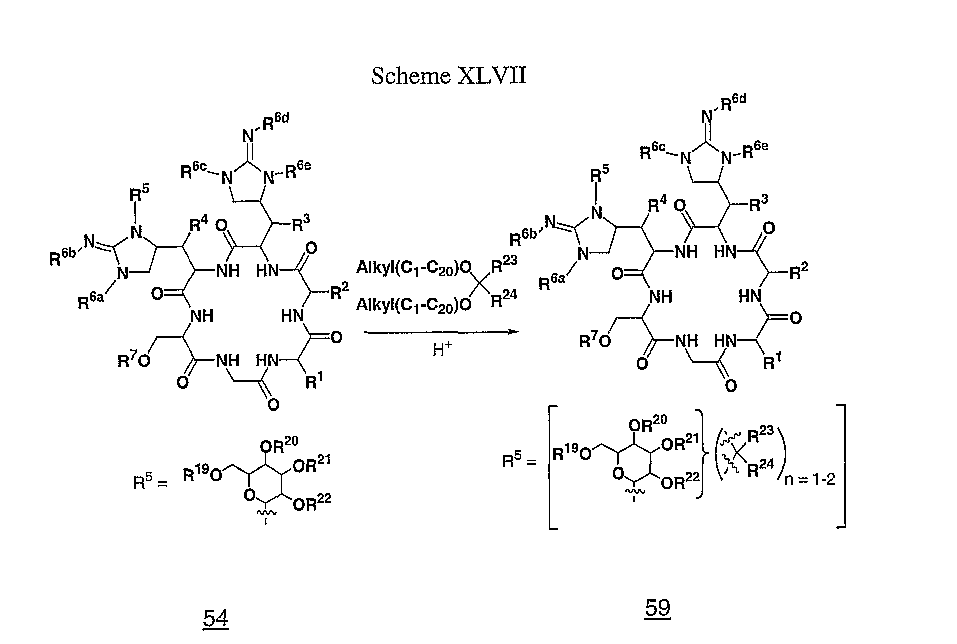

As described in Scheme XLVIII, glycopeptide antibiotics 61 in which R¹⁹, R²⁰, R²¹ and R²² are independently -(CH₂)-alkenyl(C₂-C₂₀), -(CH₂)-alkynyl(C₂-C₂₀), -(CH₂)-aryl or -(CH₂)-heteroaryl, may be prepared from the corresponding glycopeptide antibiotics 60 prepared using the conditions described in Scheme XLVII (by treatment with a compound of the formula R²³R²⁴-C(O-alkyl(C₁-C₂₀))₂ where R²³ is H and R²⁴ is alkenyl(C₂-C₂₀), alkynyl(C₂-C₂₀), aryl, or heteroaryl) in which at least one pair R¹⁹ and R²⁰, R²⁰ and R²¹, and R²¹ and R²² are moieties of the formula: 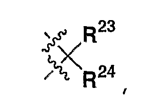 where R²³ is H and R²⁴ is alkenyl(C₂-C₂₀), alkynyl(C₂-C₂₀), aryl, or heteroaryl, where n is an integer of 1 or 2, by methods known to those skilled in the art, such as by treatment with a reductant and a protic acid or Lewis acid. Suitable combinations of reductants and acids include: sodium cyanoborohydride and trifluroacetic acid, sodium cyanoborohydride and hydrochloride acid, triethylsilane-trifluroacetic acid, borane-trimethylamine complex - aluminium chloride, borane-dimethylamine complex-boron trifluoride diethyl etherate, borane-dibutylboron triflate, or lithium aluminium hydride-aluminium chloride, and the like.

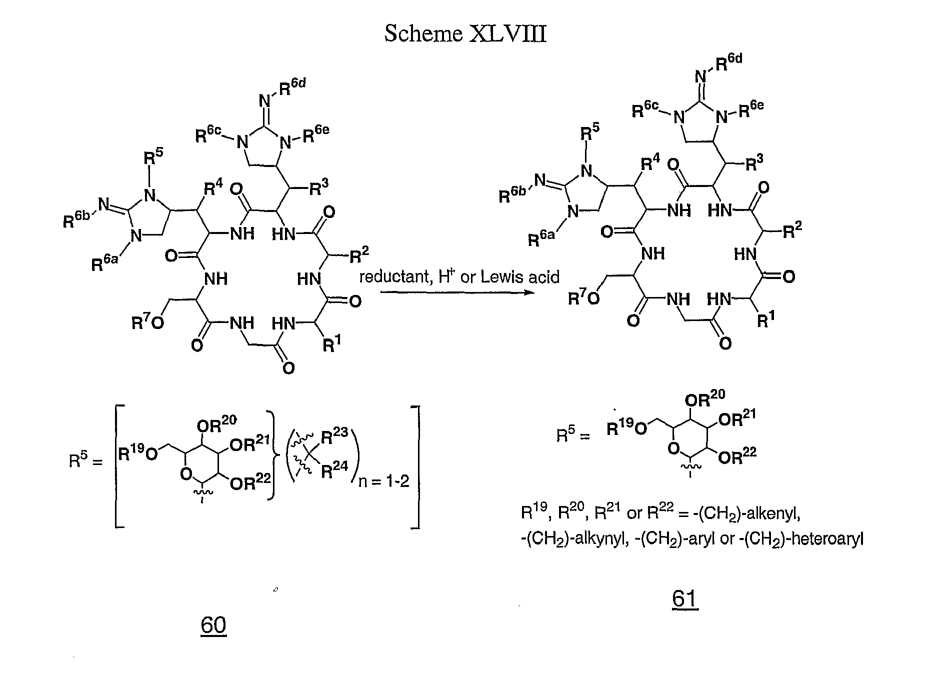

As described in Scheme XLIX, glycopeptide antibiotics 62 in which R¹⁹, R²⁰, R²¹ and R²² are independently alkyl(C₁-C₂₀), cycloalkyl(C₃-C₂₀),alkenyl(C₃-C₂₀) or alkynyl(C₃-C₂₀) may be prepared from the corresponding compounds 54 in which at least one of R¹⁹, R²⁰, R²¹ and R²² are H, by methods known to those skilled in the art, such as by alkylation with an appropriate alkylating agent R¹⁹X, R²⁰X , R²¹X or R²²X, where X is Cl, Br, I, -O-tosylate, -O-mesylate, or -O-triflate, in the presence or absence of a suitable base. The extent of the reaction may be controlled by variations in the stoichiometry of the alkylating agent, reaction temperature, and reaction time. 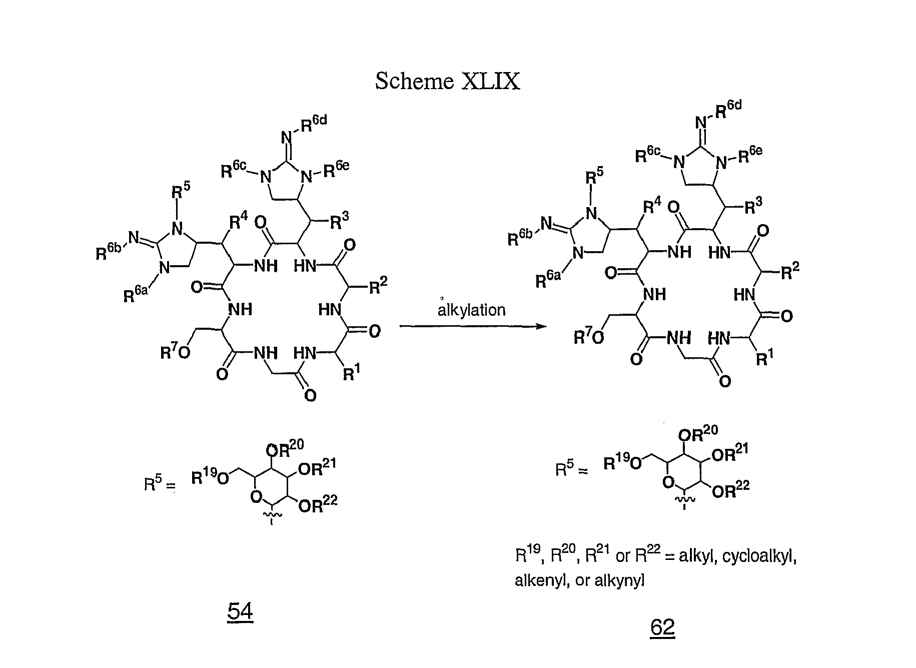 As described in Scheme XLX glycopeptide antibiotics 64 wherein R² and R^{2a} are as shown may be prepared by fermentation of glycopeptide antibiotics 63 wherein R² and R^{2a} are as shown in the presence of modified strains of *Streptomyes hygroscopicus* and in particular, strain LL4780.

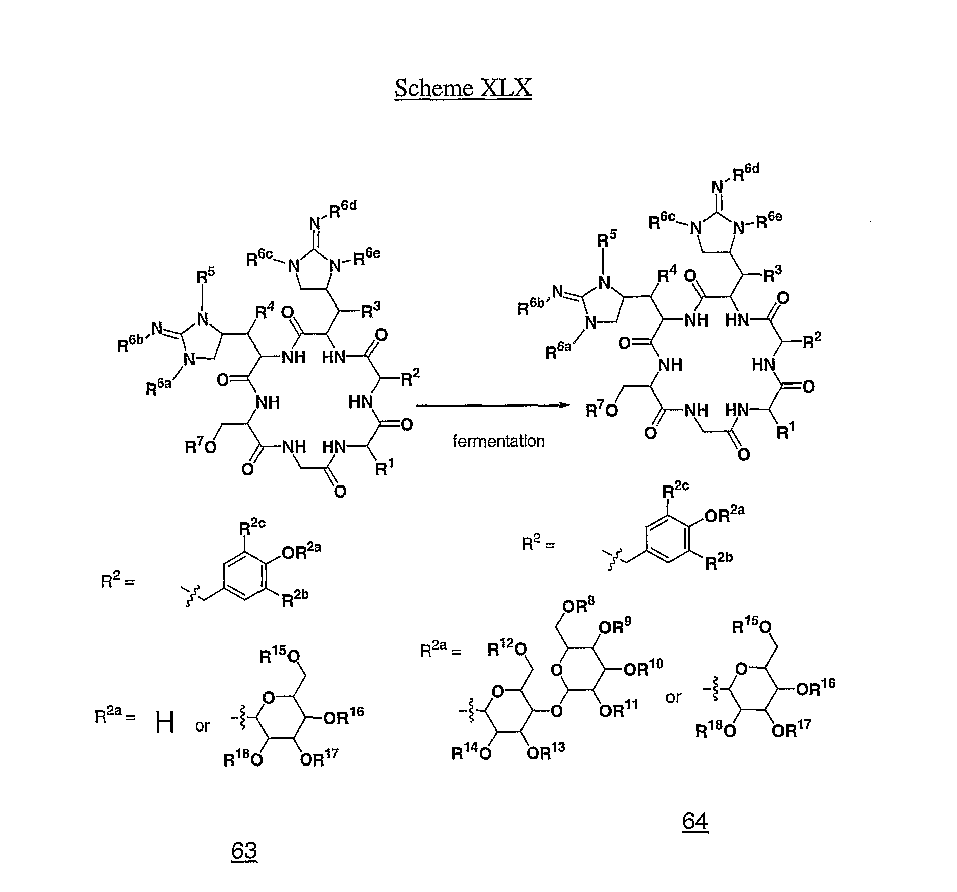

Generally, reactions described herein may be conducted in a solvent or solvents which are compatible with the reaction conditions contemplated, as is known by those skilled in the art, which include but are not limited to water, *N,N*-dimethylformamide, *N,N-*dimethylacetamide, dimethylsulfoxide, *N,N-*dimethyl propyleneurea, N-methylpyrrolidinone, and the like, at temperatures ranging from -15°C to the reflux temperature of the solvent.

Additionally, reactions are performed in a solvent appropriate to the reagents and materials employed and suitable for the transformation being effected. Various functionalities present on the molecule must be consistent with the chemical transformations proposed. This may necessitate judgement as to the order of synthetic steps and that substituents on the starting materials may be incompatible with some of the reaction conditions. Such restrictions to the substituents which are compatible with the reaction conditions will be apparent to one skilled in the art.

Suitable bases employed in the reactions as described in Schemes IV-XI, XIII-XVI, XXI-XXXIX, and XLII-XLVI include but are not limited to amine bases such as ammonia, triethylamine, *N,N*-diisopropylethylamine, pyridine, 2,6-di-tert-butyl pyridine, 1,5-diazabicyclo[4.3.0]non-5-ene, 1,8-diazabicyclo[5.4.0]undec-7-ene, and the like, carbonate bases such as sodium carbonate, potassium carbonate, cesium carbonate, and the like, hydroxide bases such as sodium hydroxide, potassium hydroxide, lithium hydroxide, barium hydroxide, and the like, or hydride bases such as sodium hydride, potassium hydride, calcium hydride, and the like.

Reactions may be monitored by reverse-phase thin-layer chromatography, electrospray mass spectrometry, analytical high-pressure liquid chromatography. (HPLC), liquid chromatography-mass spectrometry (LC-MS), and/or other analytical methods commonly employed by those skilled in the art.

Reaction products may be isolated by the removal of heterogeneous materials, if present, in the reaction vessel by filtration, followed by the removal of solvent by evaporation and/or by direct precipitation of the crude product upon addition of sufficient quantities of a co-solvent in which the product is minimally soluble, such as acetonitrile, acetone, methanol, ethanol, ethyl acetate, diethyl ether, and the like, followed by filtration or centrifugation.

Reaction products may be purified by reverse-phase preparative HPLC over commonly marketed reverse phase supports, such as the C18-coated silica ODS packing support marketed by the YMC corporation (currently a wholly-owned subsidiary of the Waters corporation), employing isocratic elution conditions, gradient elution conditions, or a combination of both isocratic and gradient elution conditions, using mixed solvent systems consisting of an organic solvent, such as methanol, acetonitrile, and the like, and water, and containing approximately 0.005-0.01 % by volume of trifluoroacetic acid, or, alternatively, approximately 0.01-0.05% by volume of acetic acid. In those instances wherein the isolated product exhibits limited stability in the acidic media of the elution solvents, such as those cases in which the product(s) is a (are) ketal(s) or aryl-acetal(s), it is advantageous to employ 0.01% of acetic acid and to neutralize the product-containing fractions to pH 6 (pH paper) by the addition of sufficient quantities of aqueous ammonium hydroxide. Excess ammonium acetate thus produced is removed from the final desired product following concentration of the product-containing fractions *in vacuo,* by lyophilization, or by washing with a solvent in which the product is minimally soluble, such as ethanol, 2-propanol, and the like.

Typically dialkyl acetals or dialkyl ketals of the formula R²³R²⁴-C(O-alkyl(C₁-C₂₀))₂, used in the hereinbefore described schemes include the dimethyl acetals of acetaldehyde, propionaldehyde, butyraldehyde, 3-methylbutyraldehyde, 3,3-dimethylbutyraldehyde, cyclopentanecarboxaldehyde, cyclohexanecarboxaldehyde, 2-ethyl-butyraldehyde, phenylacetaldehyde, 4-methoxyphenylacetaldehyde, 4-bromophenylacetaldehyde, 3-phenyl-propionaldehyde, 2-(N-benzyloxycarbonyl-4-piperidinyl)-acetaldehyde (prepared in two steps from 2-(4-piperidnyl)-ethanol by N-funtionalization and oxidation), 1-adamantylcarboxaldehyde, benzaldehyde, 3-(4-methylphenoxy)-benzaldehyde, 3-nitro-4-methoxy-benzaldehyde, 4-benzyloxybenzaldhyde, 3-benzyloxybenzaldhyde, 4-carboxymethylbenzaldehyde, 4-(2-propyl)benzaldehyde, 4-(1-propyl)benzaldehyde, 4-phenylbenzaldehyde, piperonal, 1-naphthaldehyde, 2-naphthaldehyde, 6-methoxy-2-naphthaldehyde, 4-methoxy-1-naphthaldehyde, and the like, and the dimethyl ketals of acetone, cyclopentanone, cyclohexanone, 2-methylcyclohexanone, 4-tert-butylcyclohexanone, 2,2-dimethylcyclohexanone, 2,5-dimethylcyclohexanone, 3,3,5,5-tetramethylcyclohexanone, 2-adamantanone, bicyclo[3.3.1]nonan-9-one, tetrahydrothiopyran-4-one, acetophenone, 4-fluoroacetophenone, (R)-camphor, (S)-camphor, carvone, and the like.

In those instances where the parent aldehyde or ketone is not commercially available, the aldehyde or ketone is prepared from readily accessible compounds by methods known to those skilled in the art, such as by oxidation of the corresponding alcohols, by Kornblum-type oxidation of the corresponding alkyl- or benzyl- bromides, by oxidation of aryl methanes, by benzylic bromination of aryl methanes followed by Kornblum oxidation of the resultant benzyl bromide or hydrolysis of the resultant benzylic dibromides, by reduction of the corresponding esters, carboxylic acids, or nitriles, or, in the case of alkoxy-substituted benzaldehydes, by alkylation of commerically available phenolic benzaldehydes or an appropriate precursor thereof, or, in the case of aryl- or heteroaryl-substituted benzaldehydes, by palladium-mediated couplings of bromo- or iodo-substituted benzaldehydes or an appropriate precursor thereof with aromatic or heteroaromatic boronic acids, or, in the case of acyl-substituted benzaldehydes, by Friedel-Crafts acylation of a benzaldehyde or an appropriate precursor thereof.

Aldehydes or ketones used in these reactions are easily converted to their corresponding dialkyl-acetals, typically dimethylacetals, by methods known to those skilled in the art, such as by reaction of the aldehyde or ketone with trimethylorthoformate (to prepare dimethyl acetals) in the presence of an acid catalyst, such as hydrochloric acid, sulfuric acid, p-toluenesulfonic acid, camphorsulfonic acid, immobilized sulfonic acid resins (anionic exchange resins)and the like, or, by reaction of the aldehyde or ketone with an alcohol in the presence of an acid catalyst such as those listed above while employing the use of a dehydrating agent such as molecular sieves or by employing the use of a Dean-Stark apparatus, or employing any other conditions that permit the efficient removal of water from the reaction.

### BIOLOGICAL ACTIVITY

### METHODS FOR IN VITRO ANTIBACTERIAL EVALUATION(Table 1)

The minimum inhibitory concentration (MIC), the lowest concentration of the antibiotic which inhibits growth of the test organism, is determined by the broth dilution method using Muller-Hinton II agar (Baltimore Biological Laboratories) following the recommendations of the National Committee for Clinical Laboratory Standards [Methods for dilution antimicrobial susceptibility tests for bacteria that grow aerobically, approved standard M7-A2, National Committee for Clinical Laboratory Standards, Villanova, Pa].

An inoculum level of 5x10⁵ CFU/mL, and a range of antibiotic concentrations (64-0.06 µg/mL) is used. The MIC is determined after the microtiter plates are incubated for 18 hours at 35°C in an ambient air incubator. The test organisms include a spectrum of Gram-positive bacteria comprised of *Staphylococcus* sp., *Streptococcus* sp. and *Enterococcus* sp. These organisms include recent clinical isolates that are resistant to methicillin, penicillin and/or vancomycin. The results of representative examples of the invention are given in Table 2.

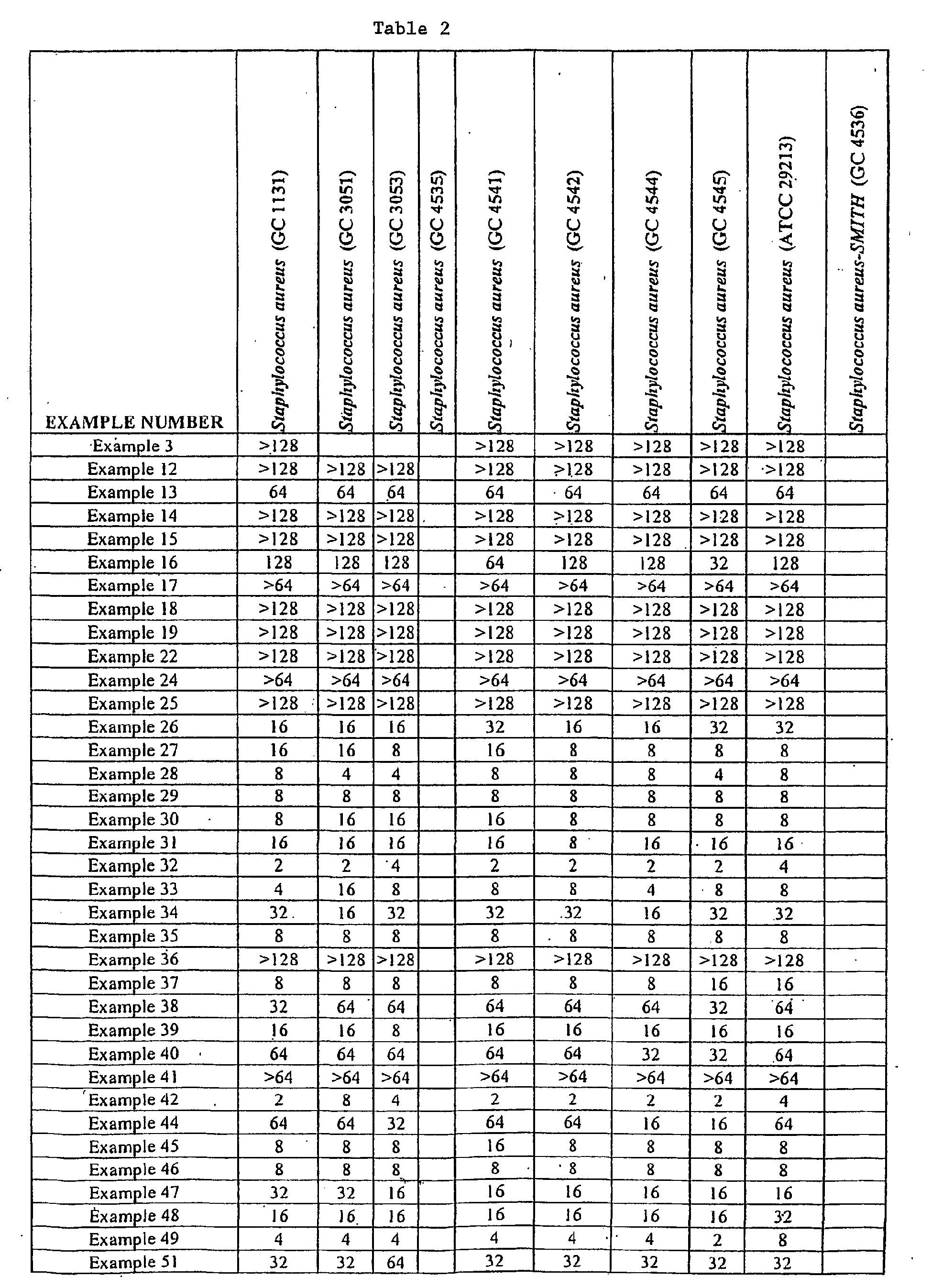

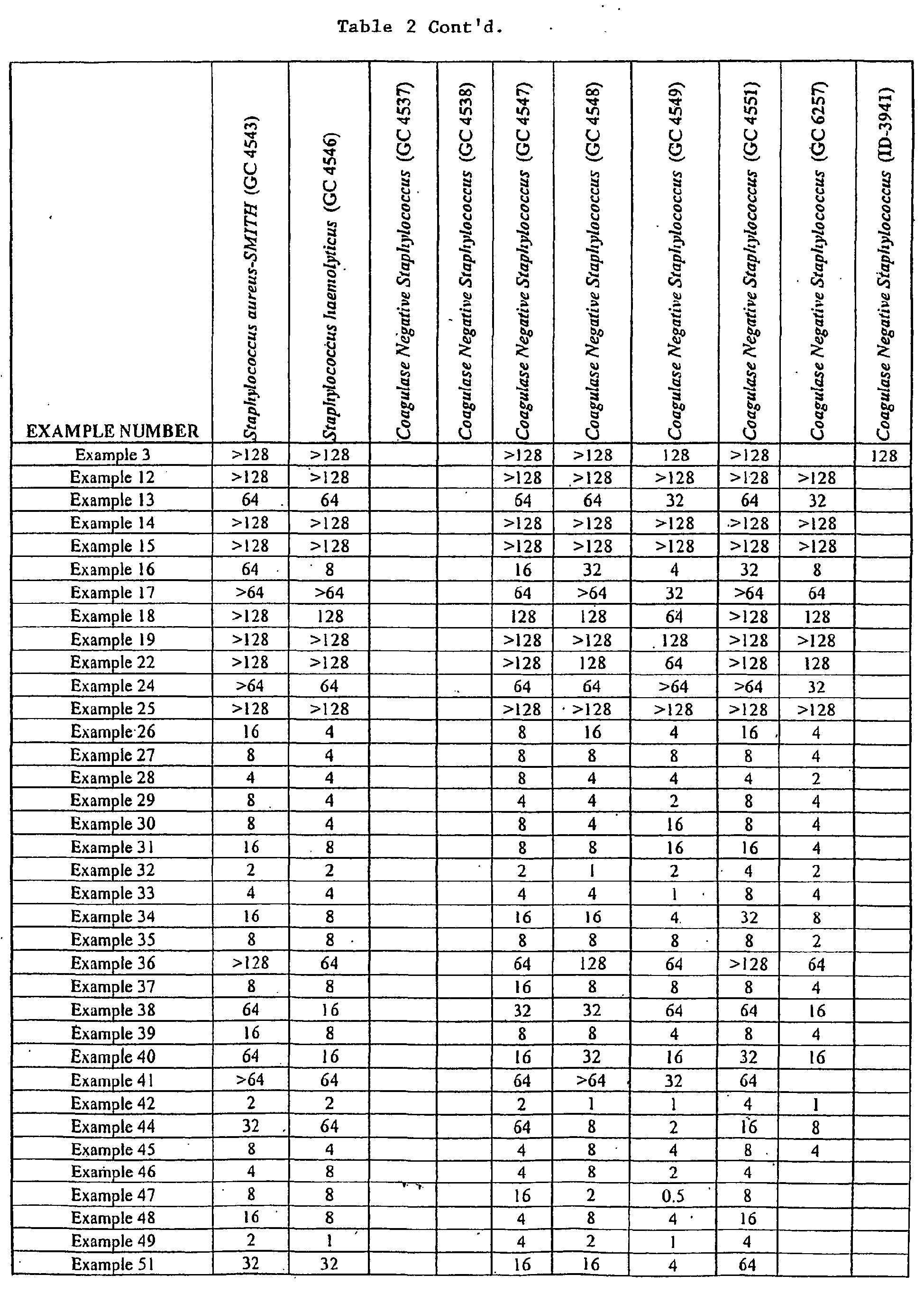

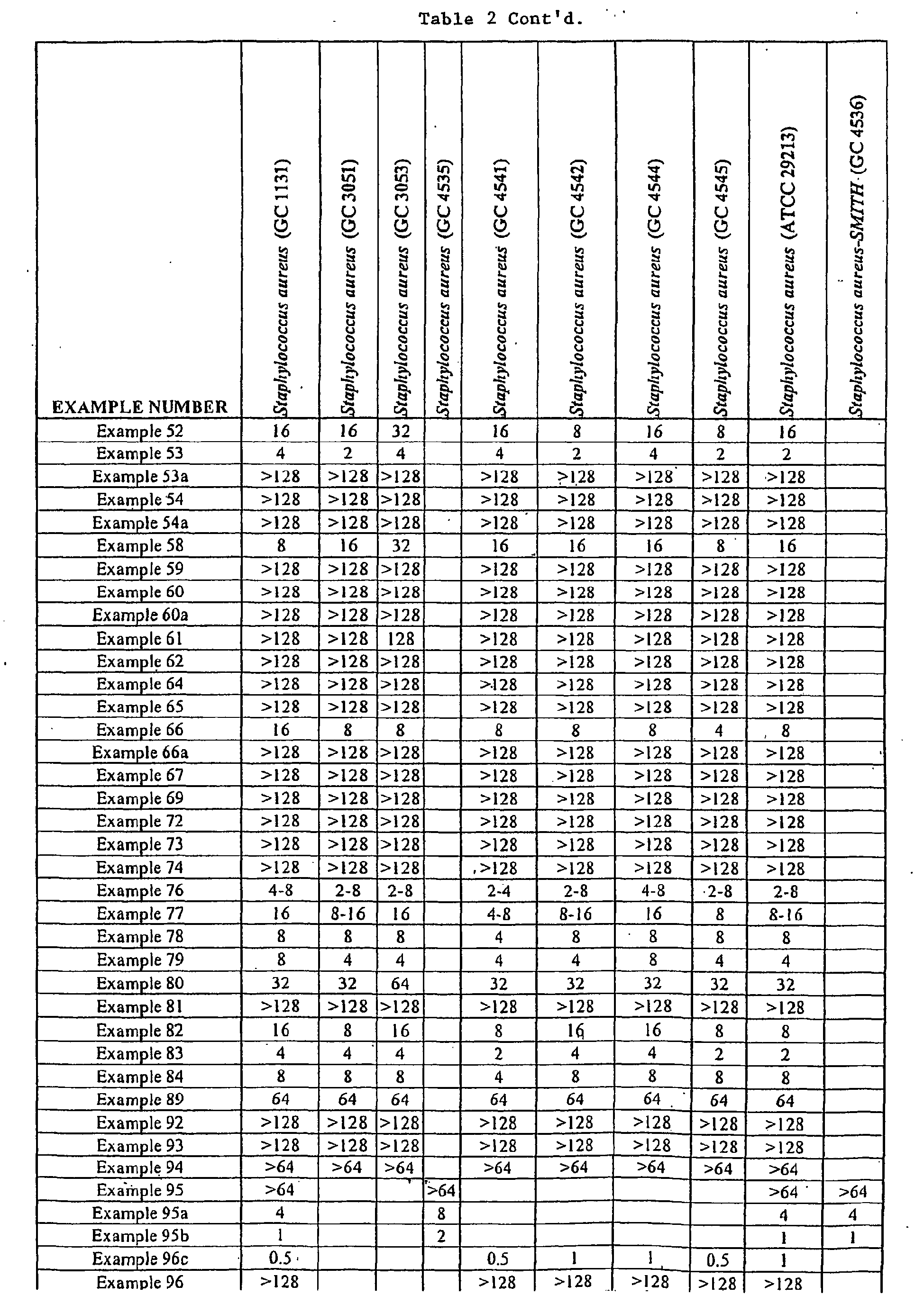

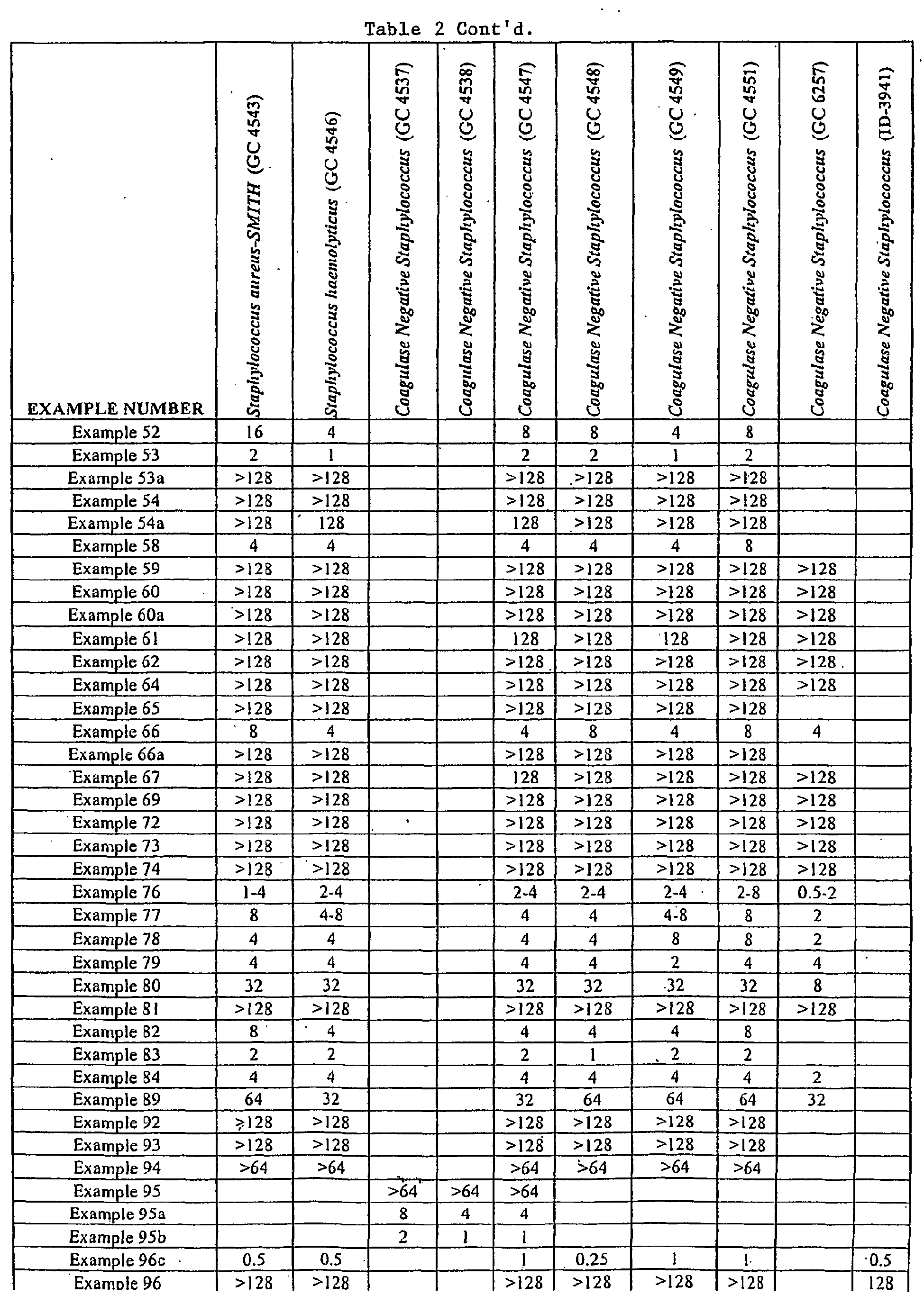

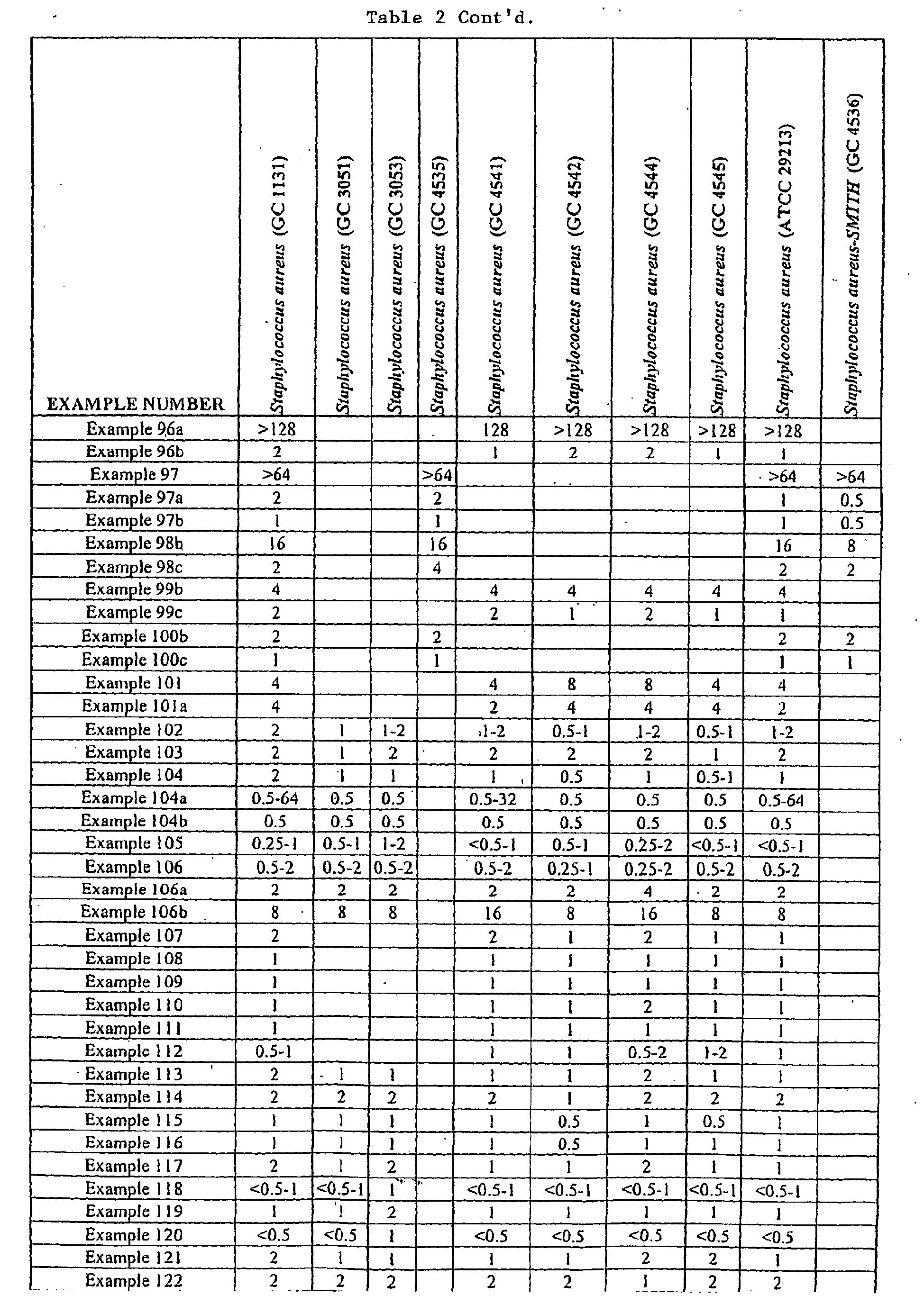

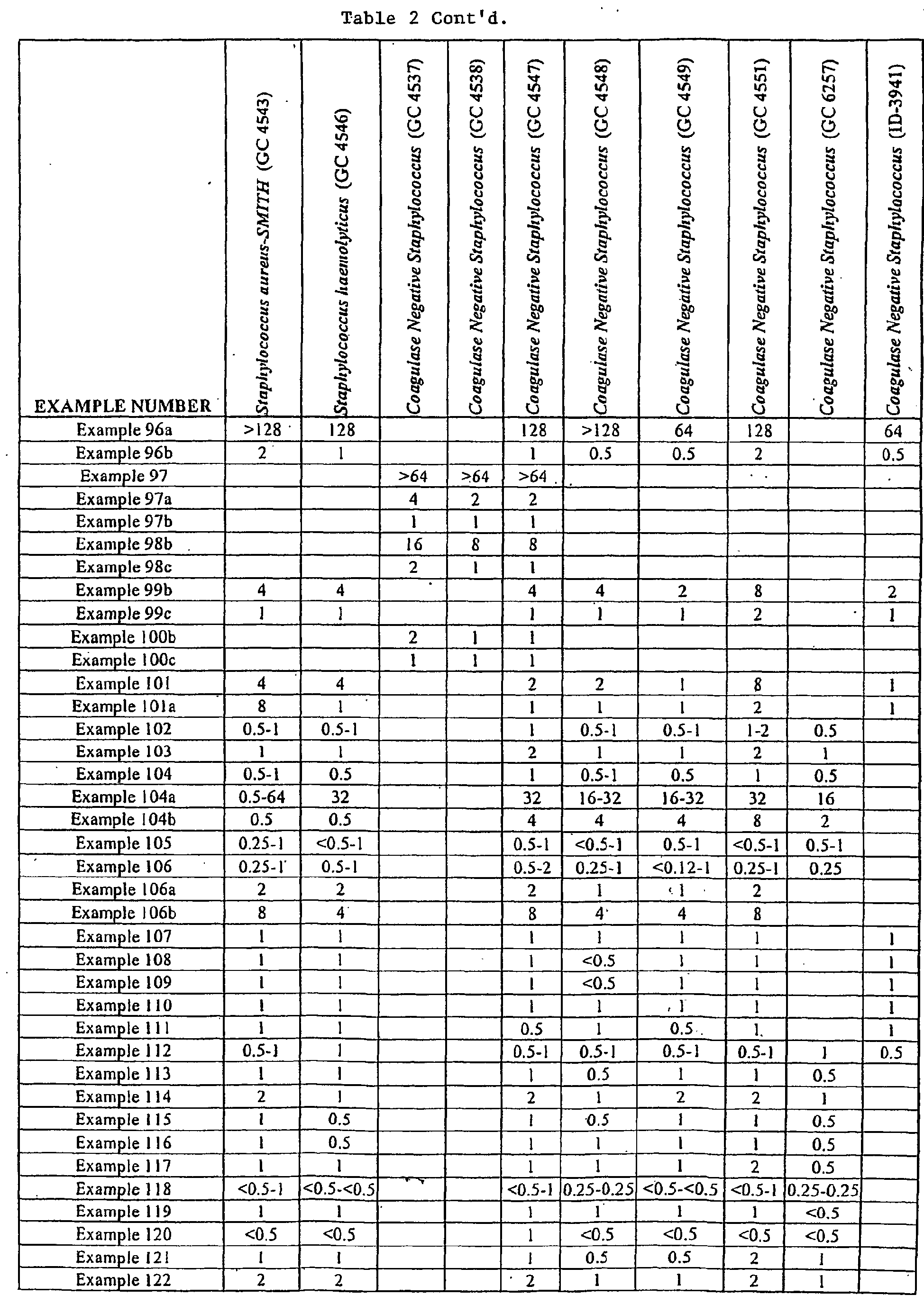

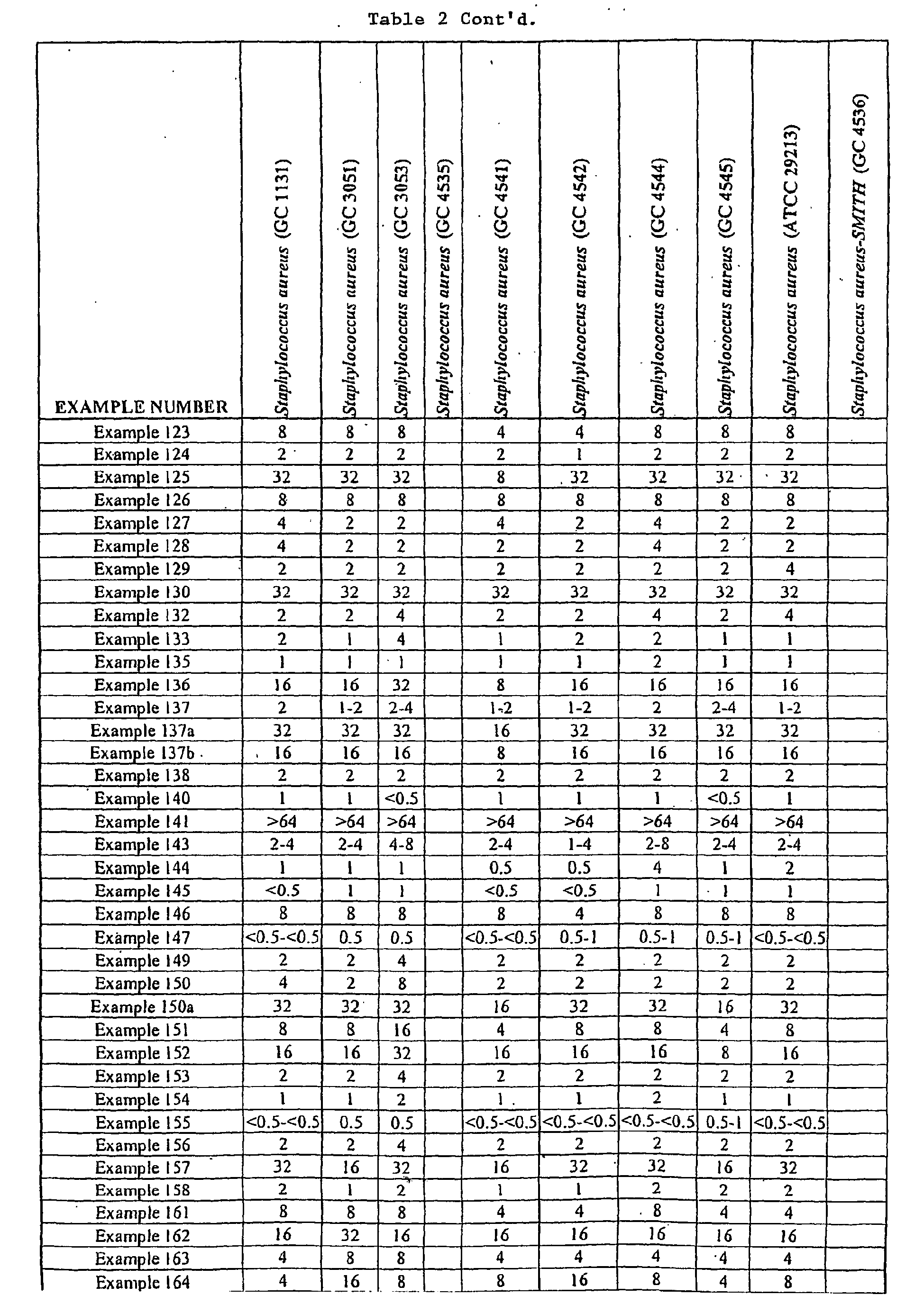

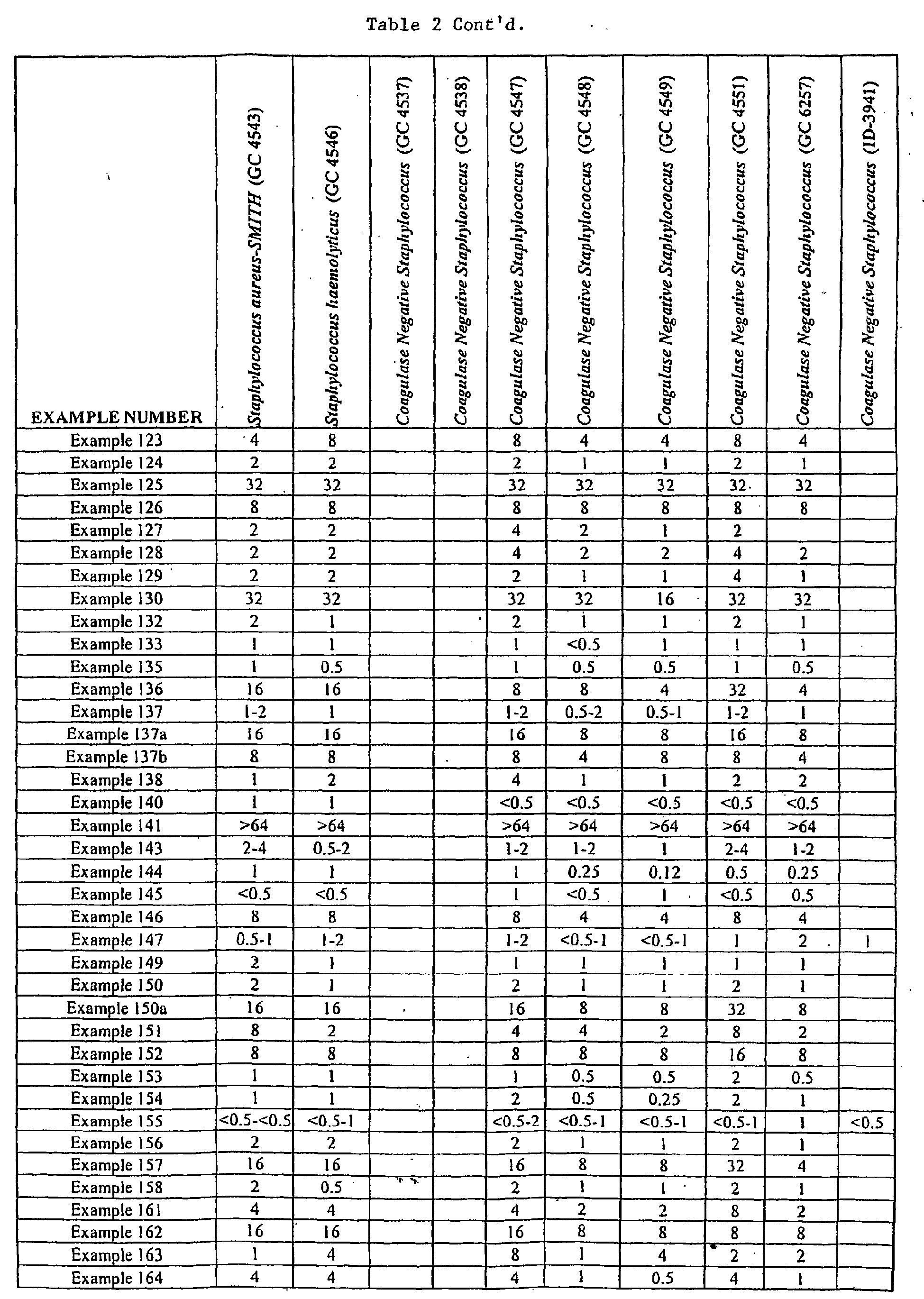

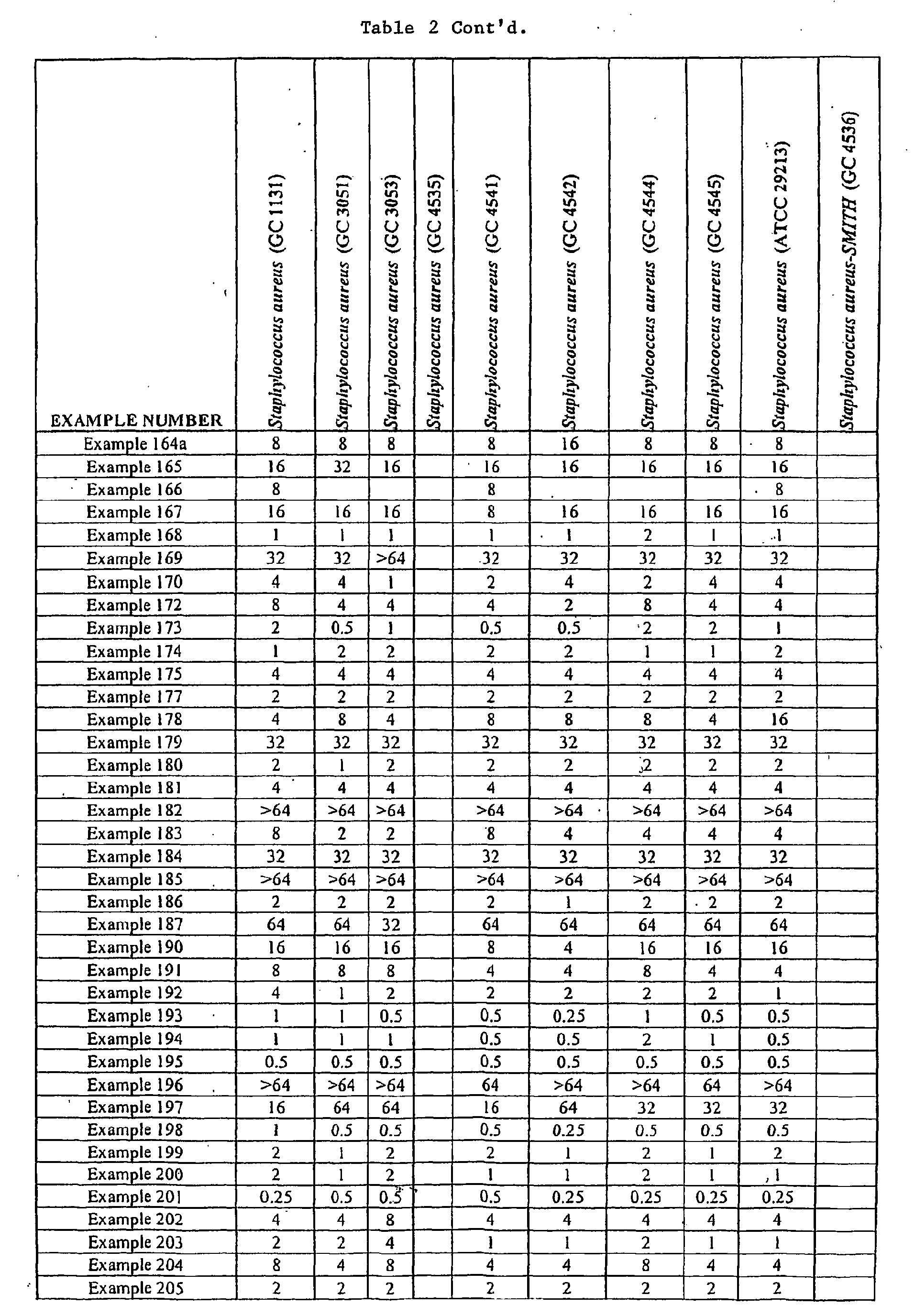

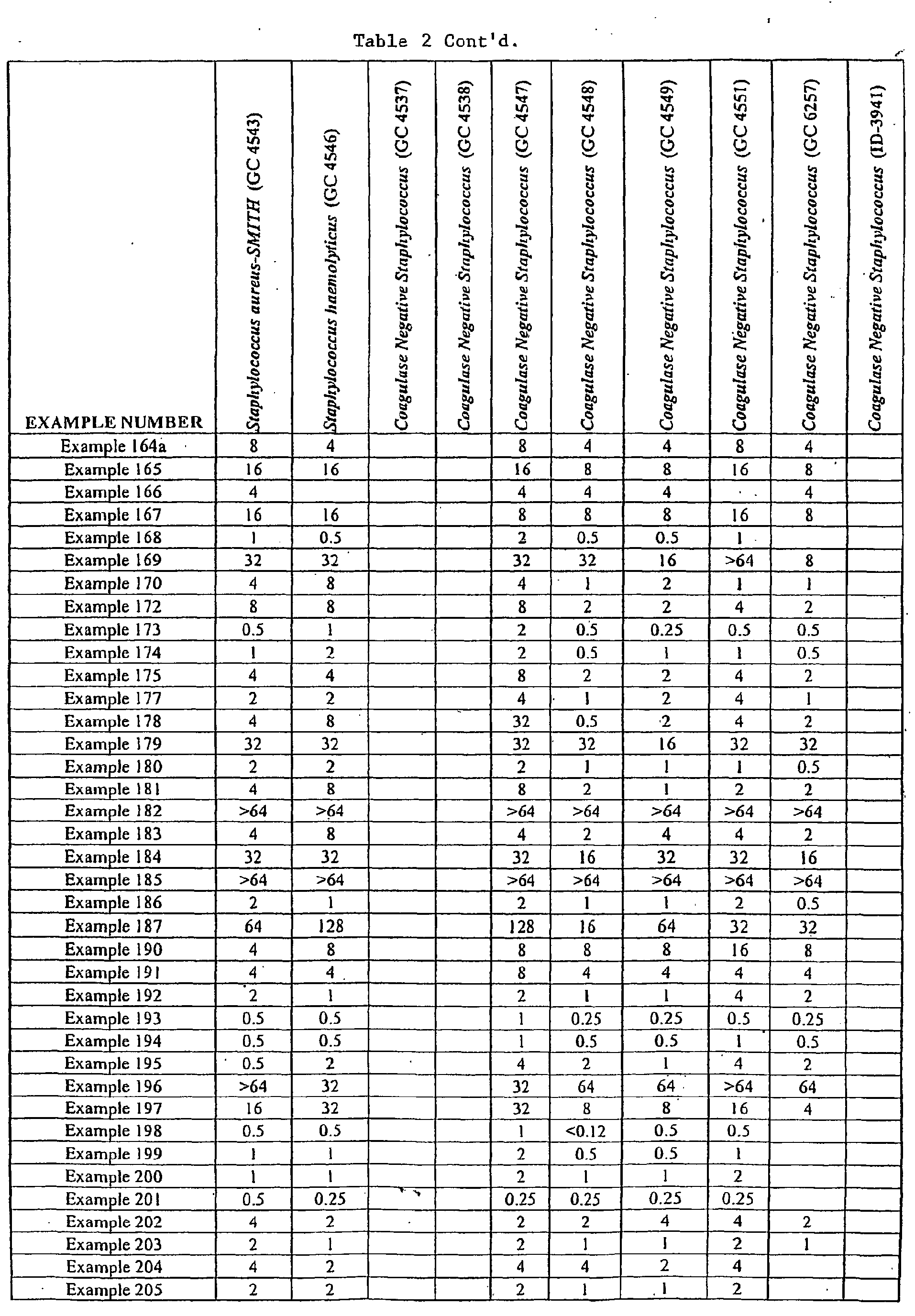

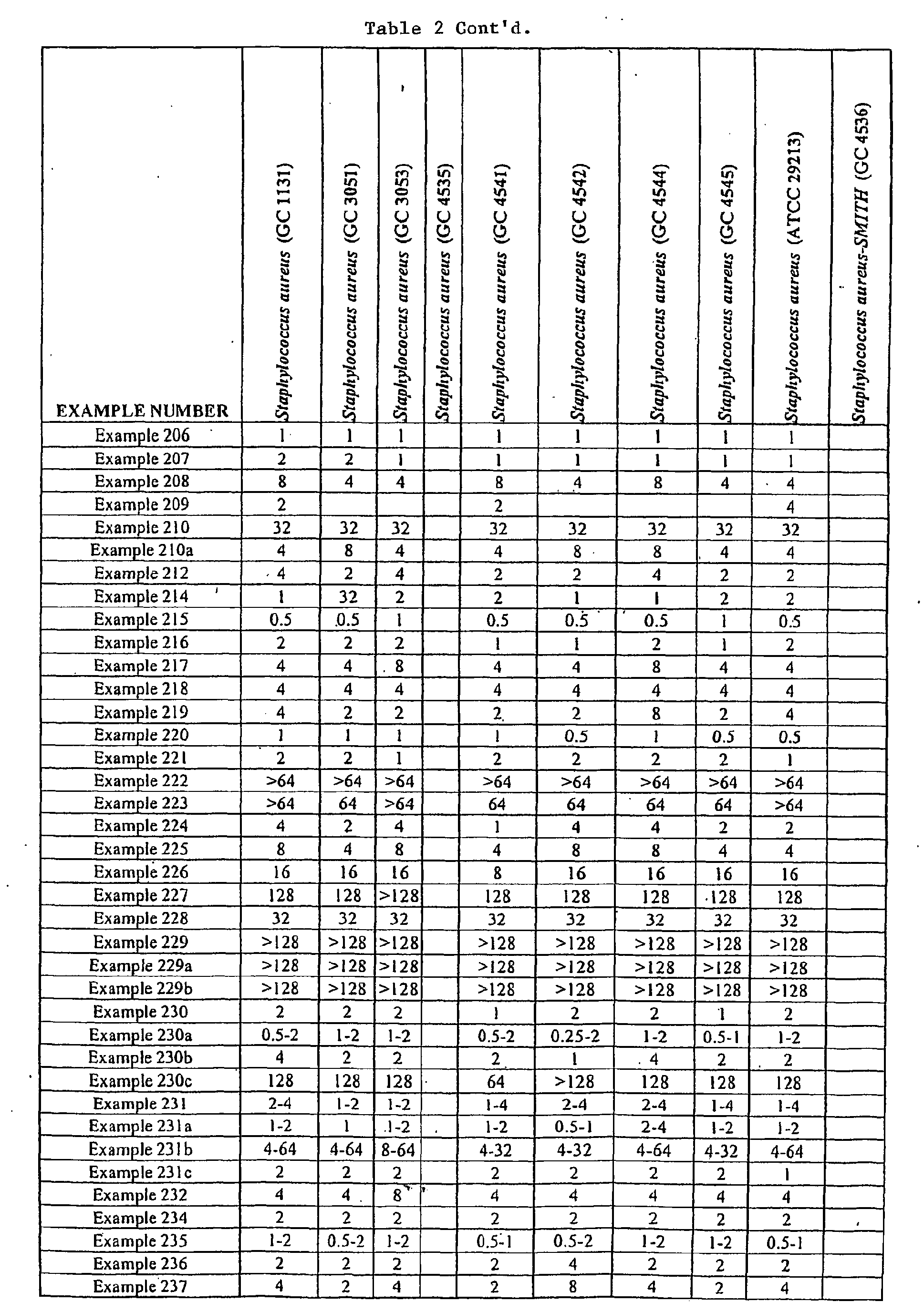

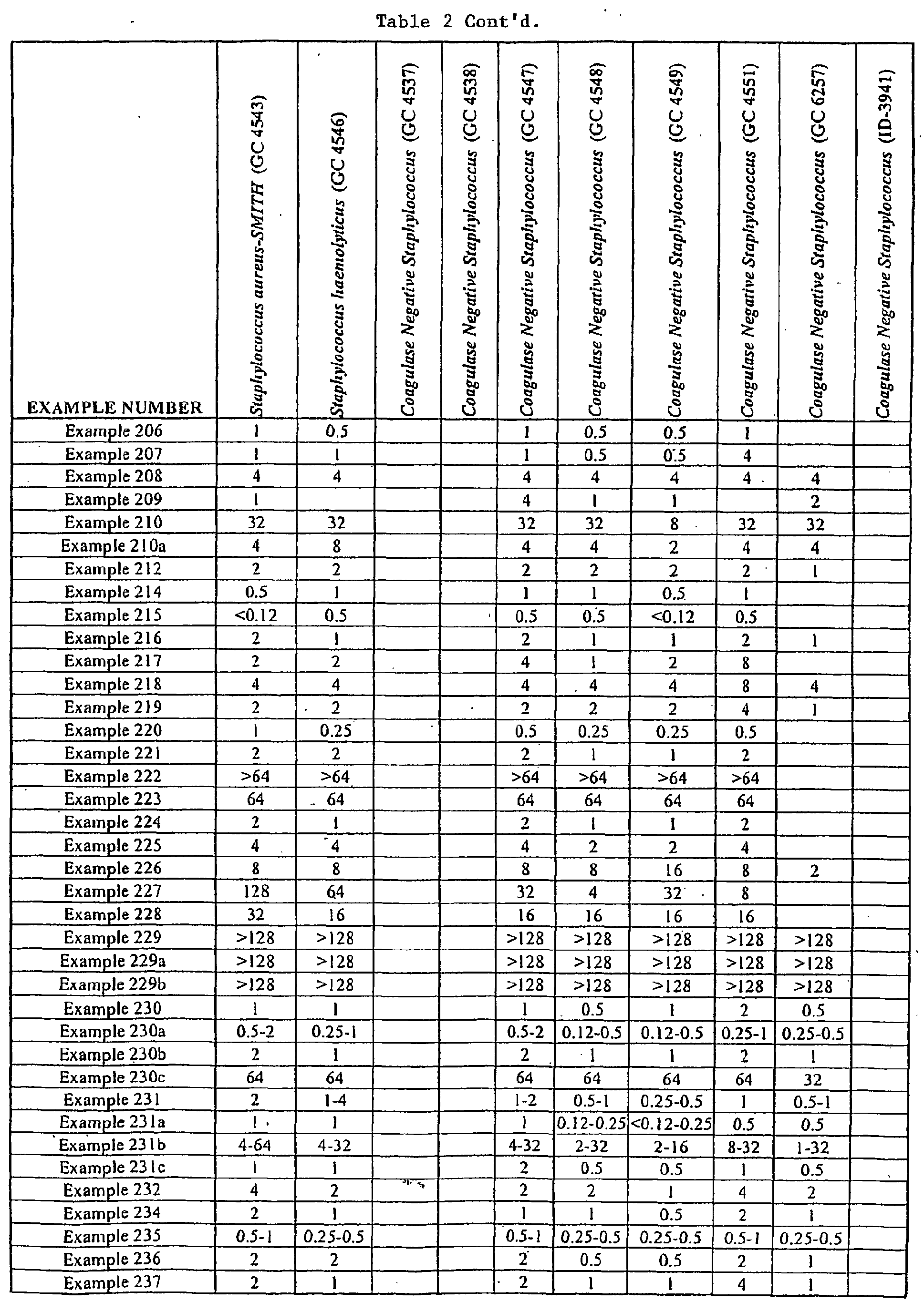

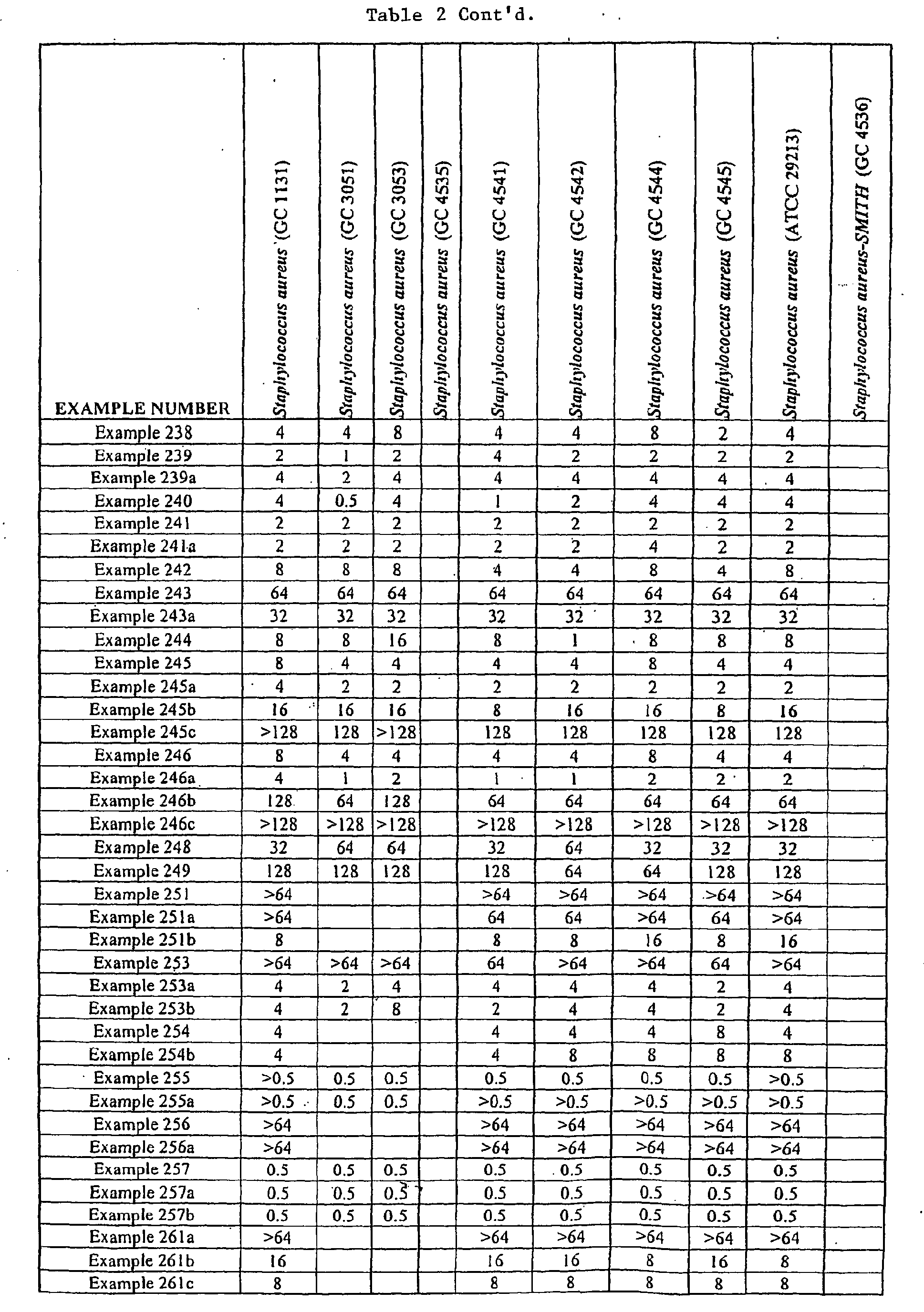

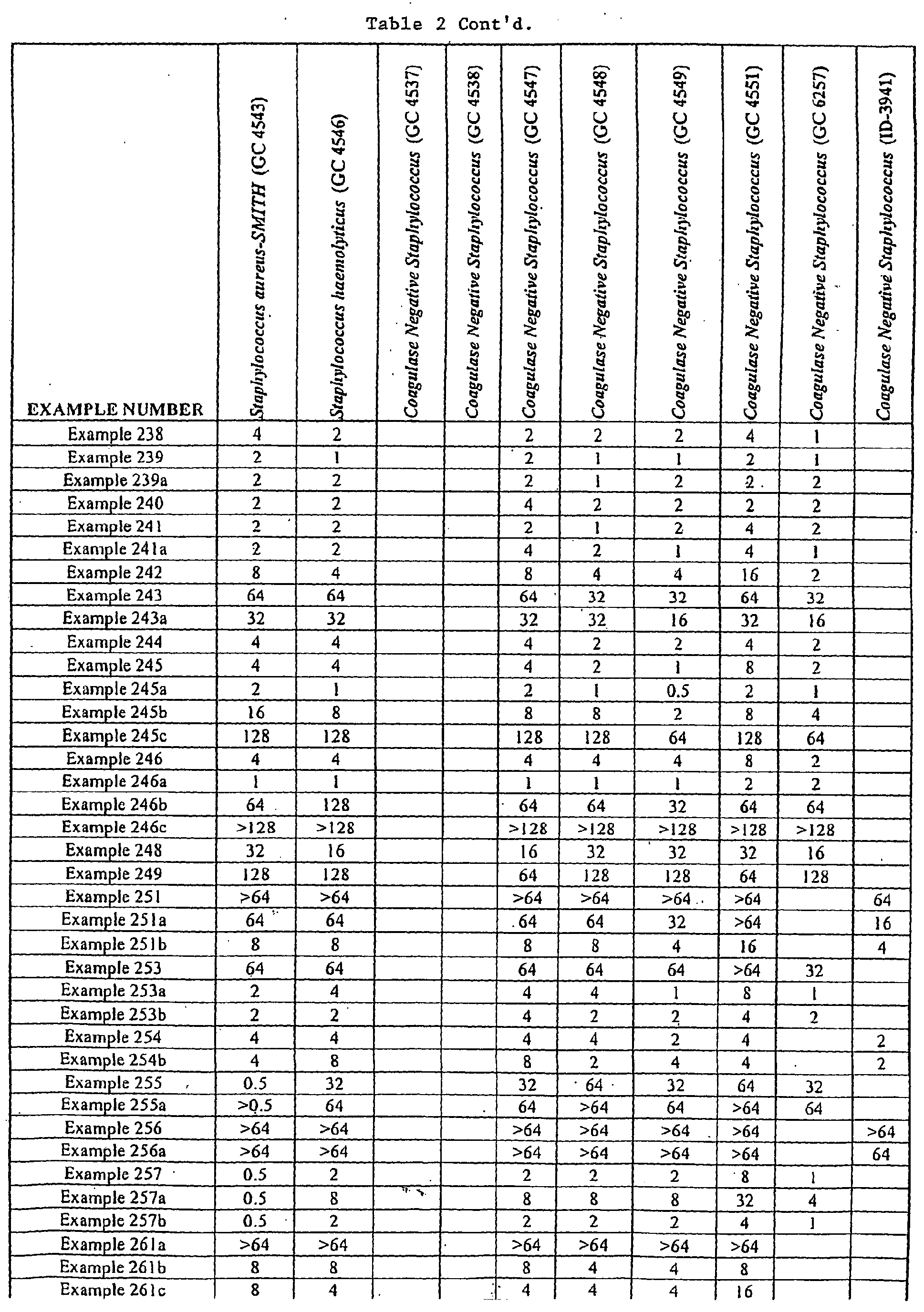

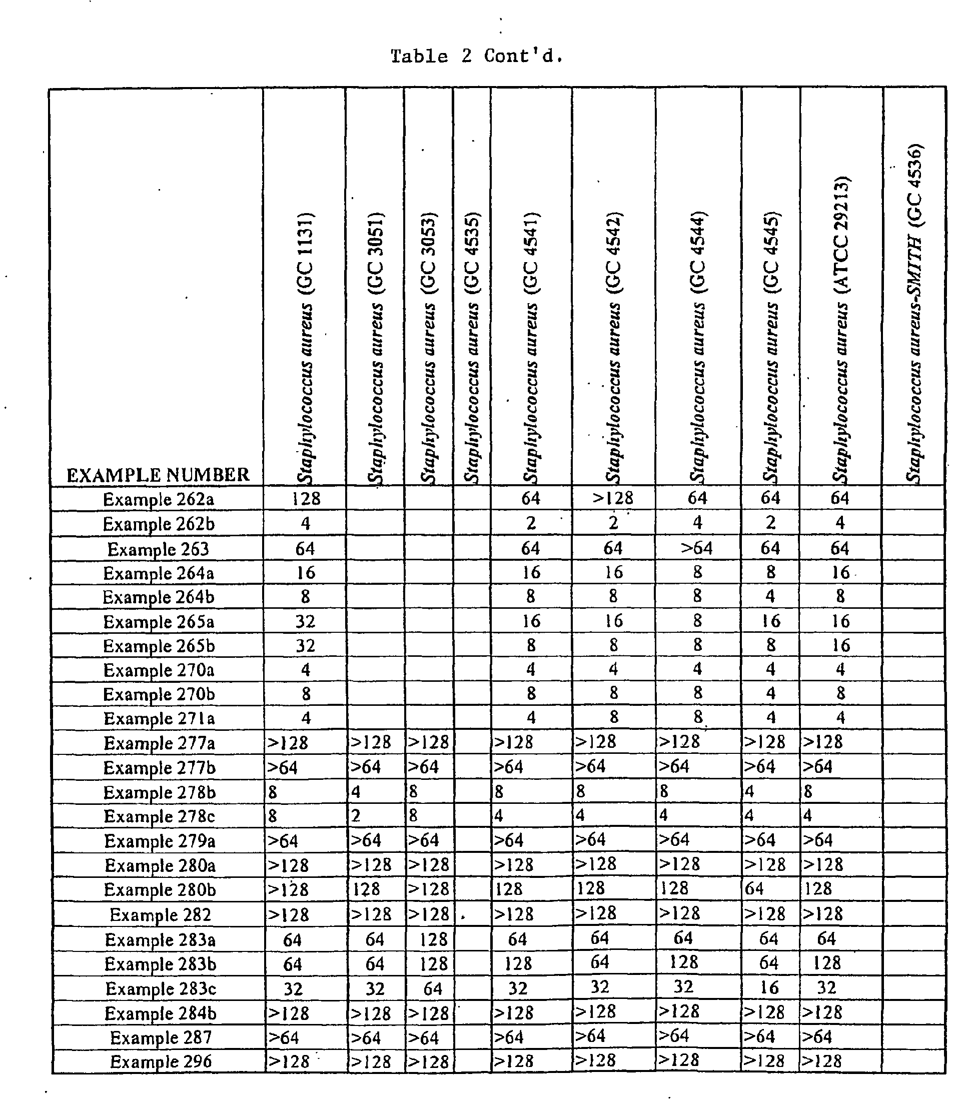

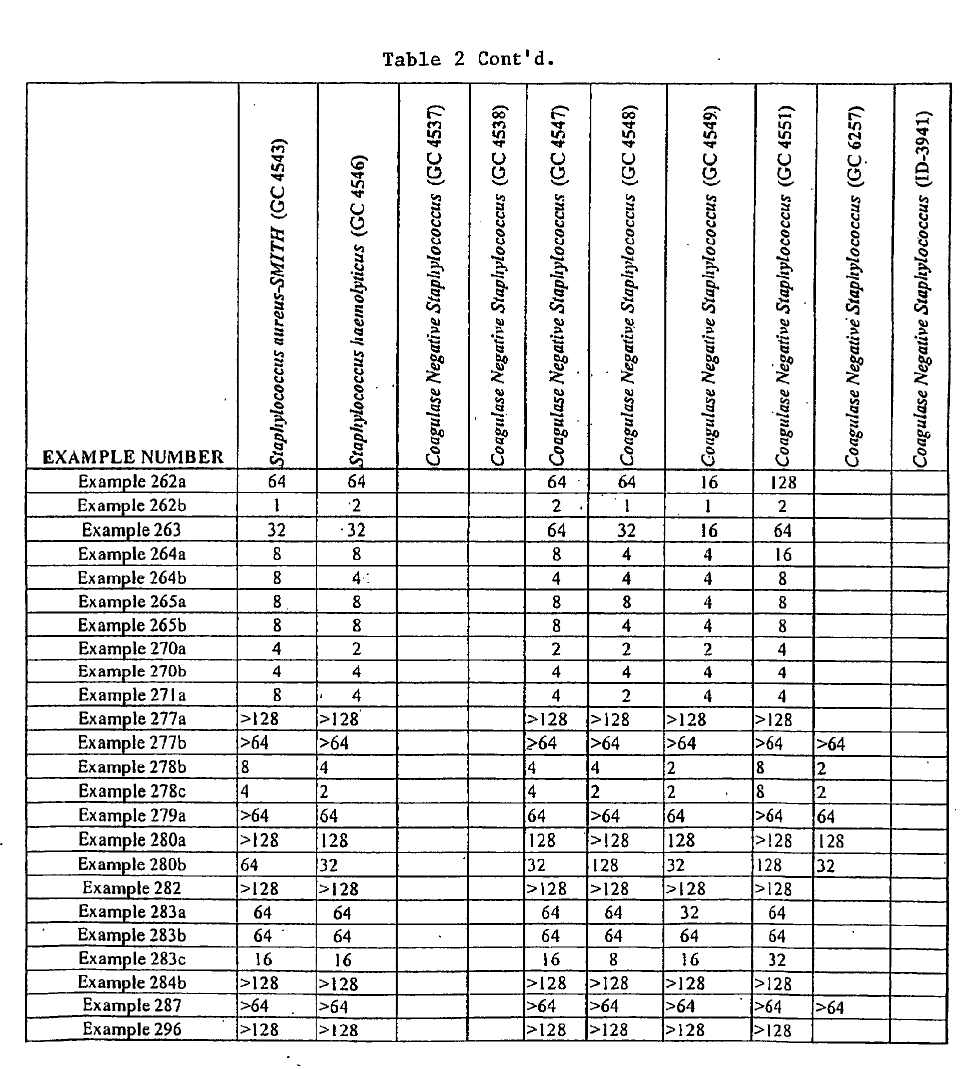

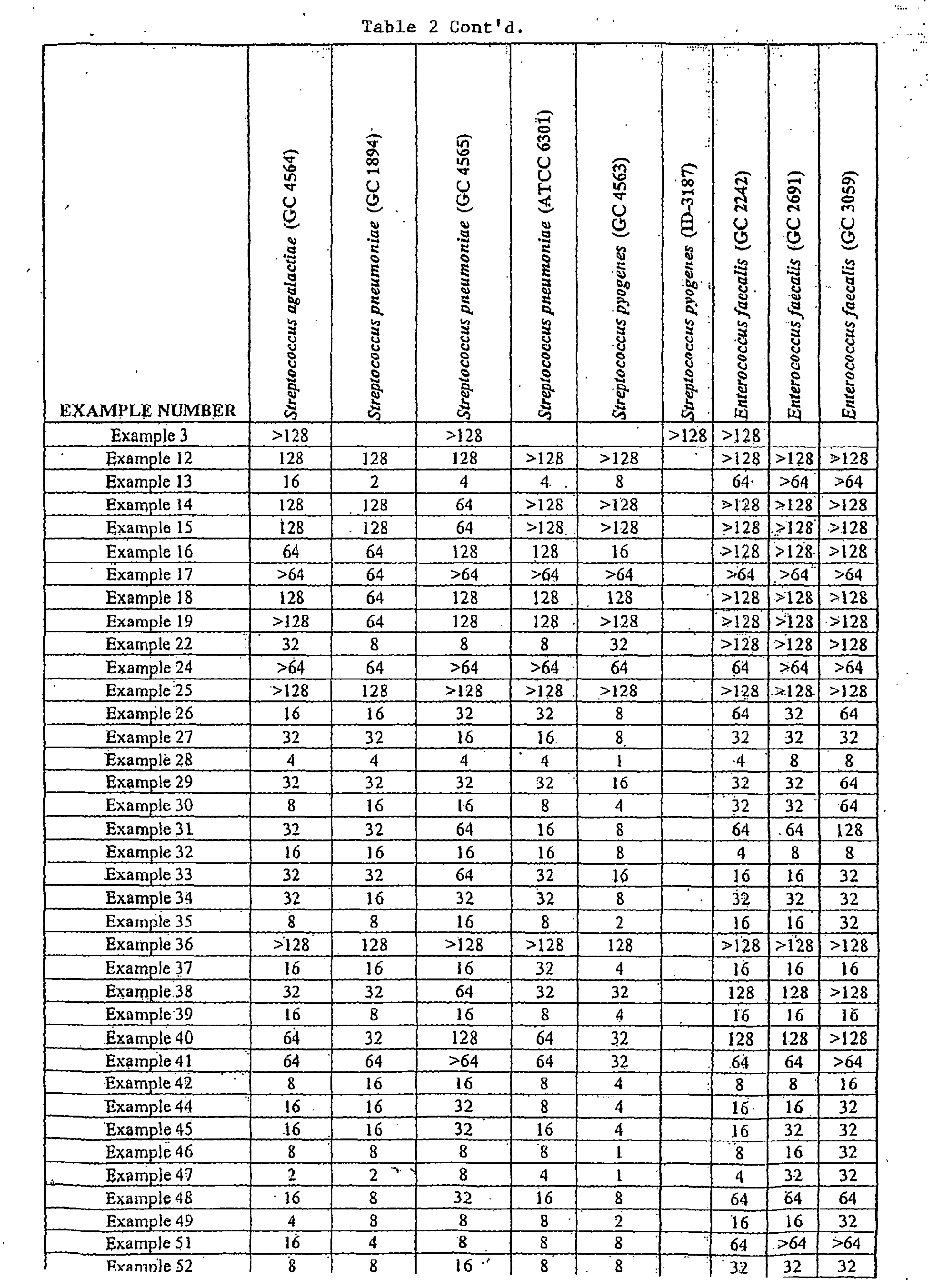

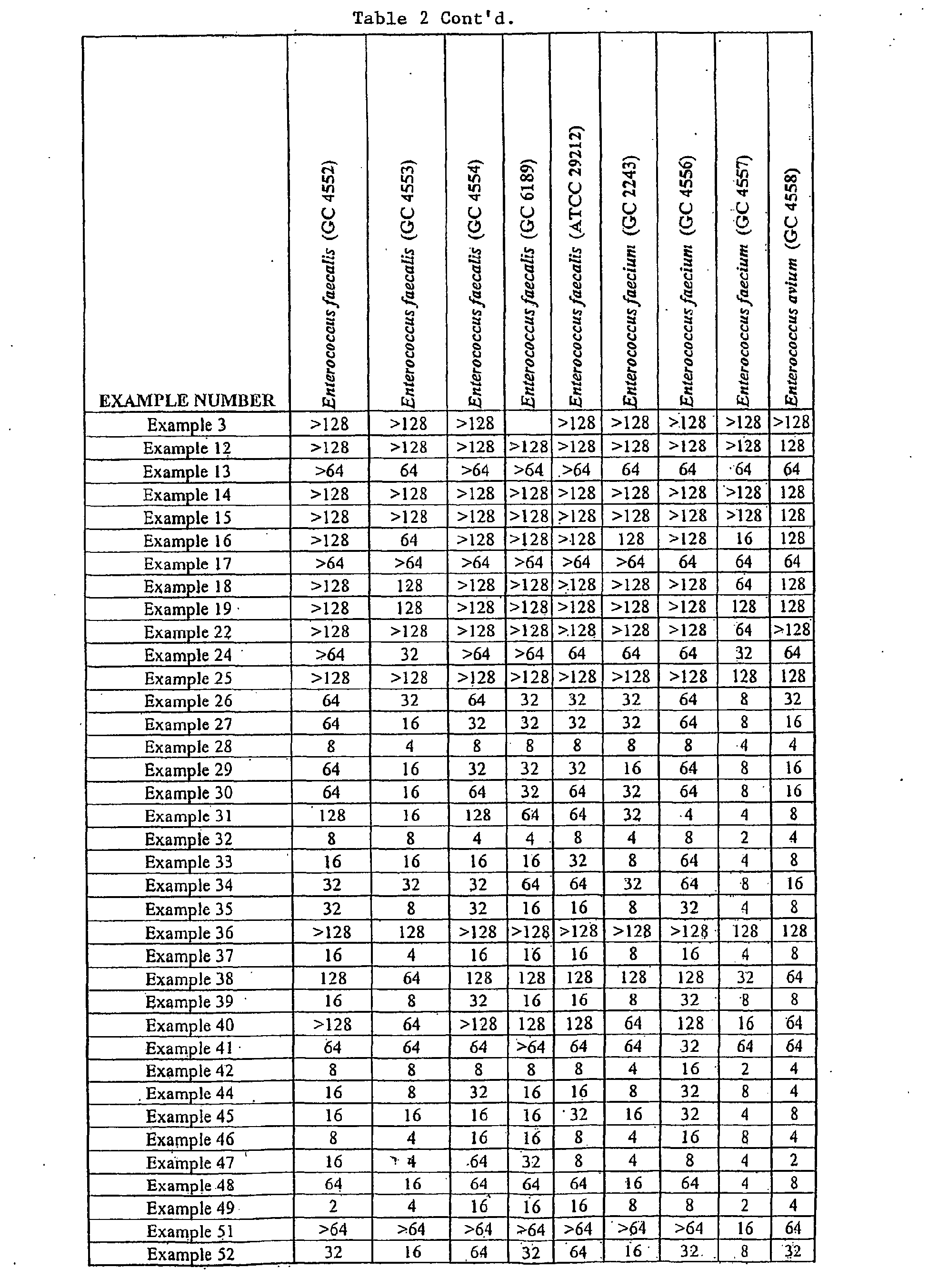

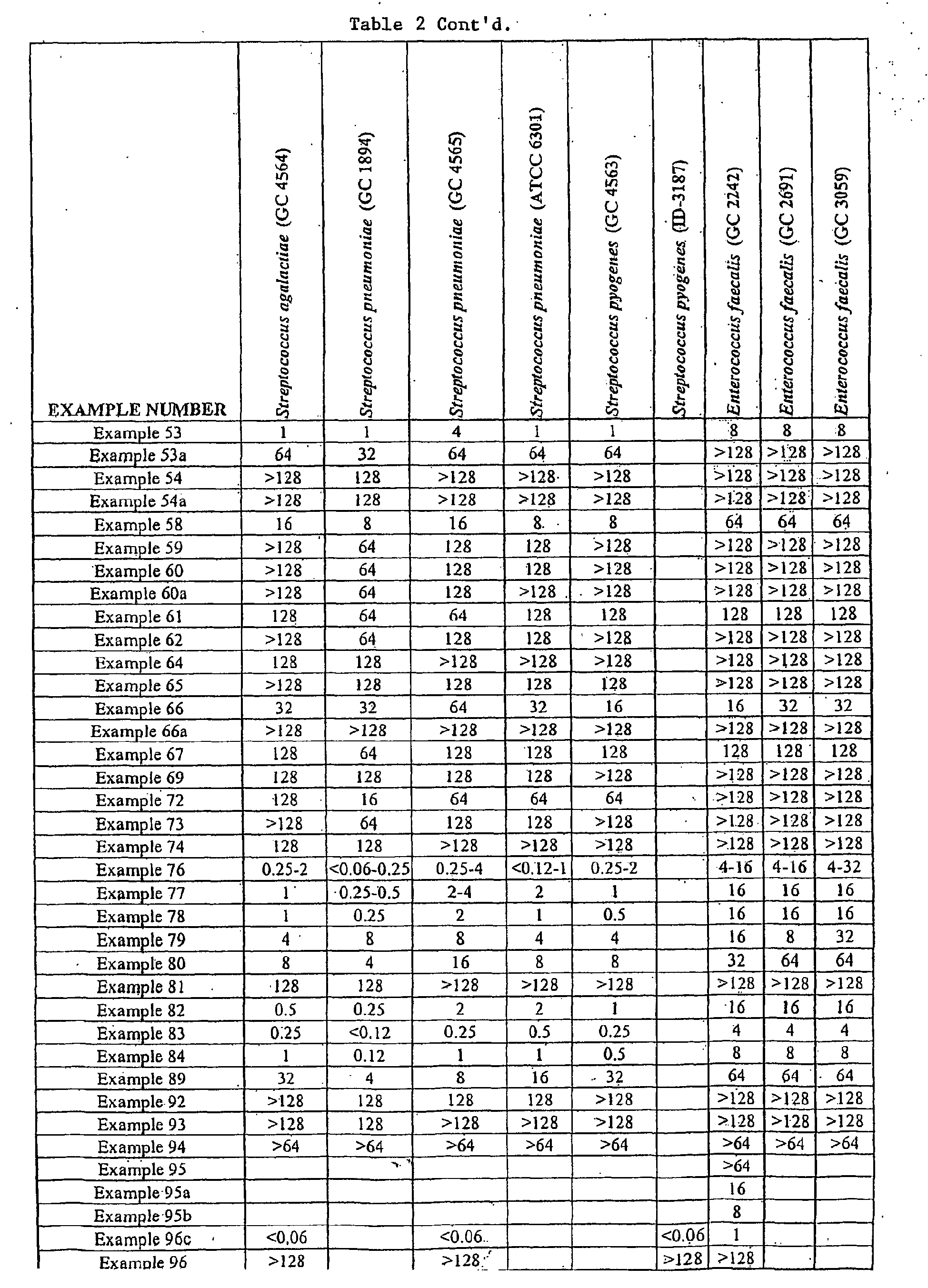

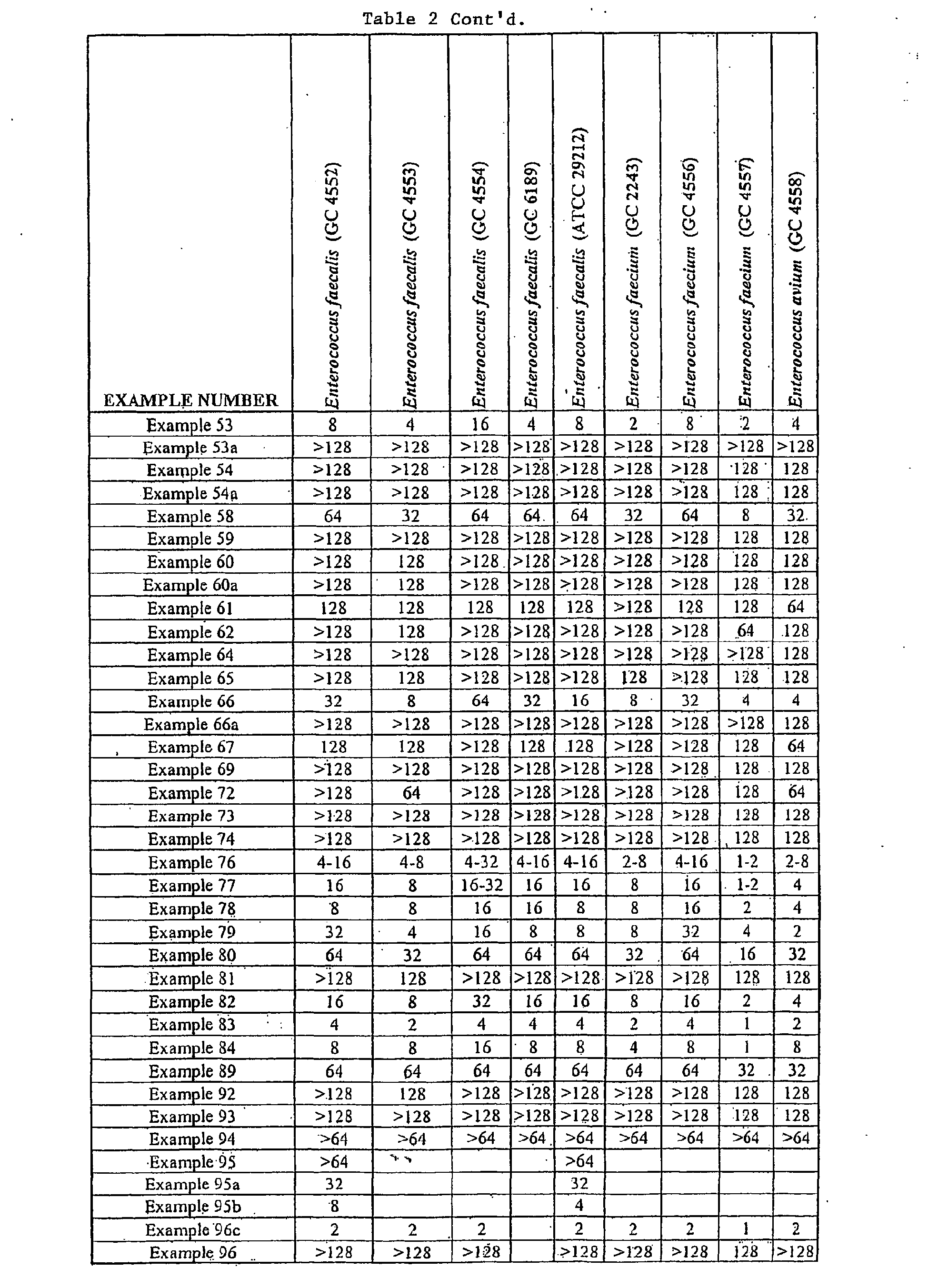

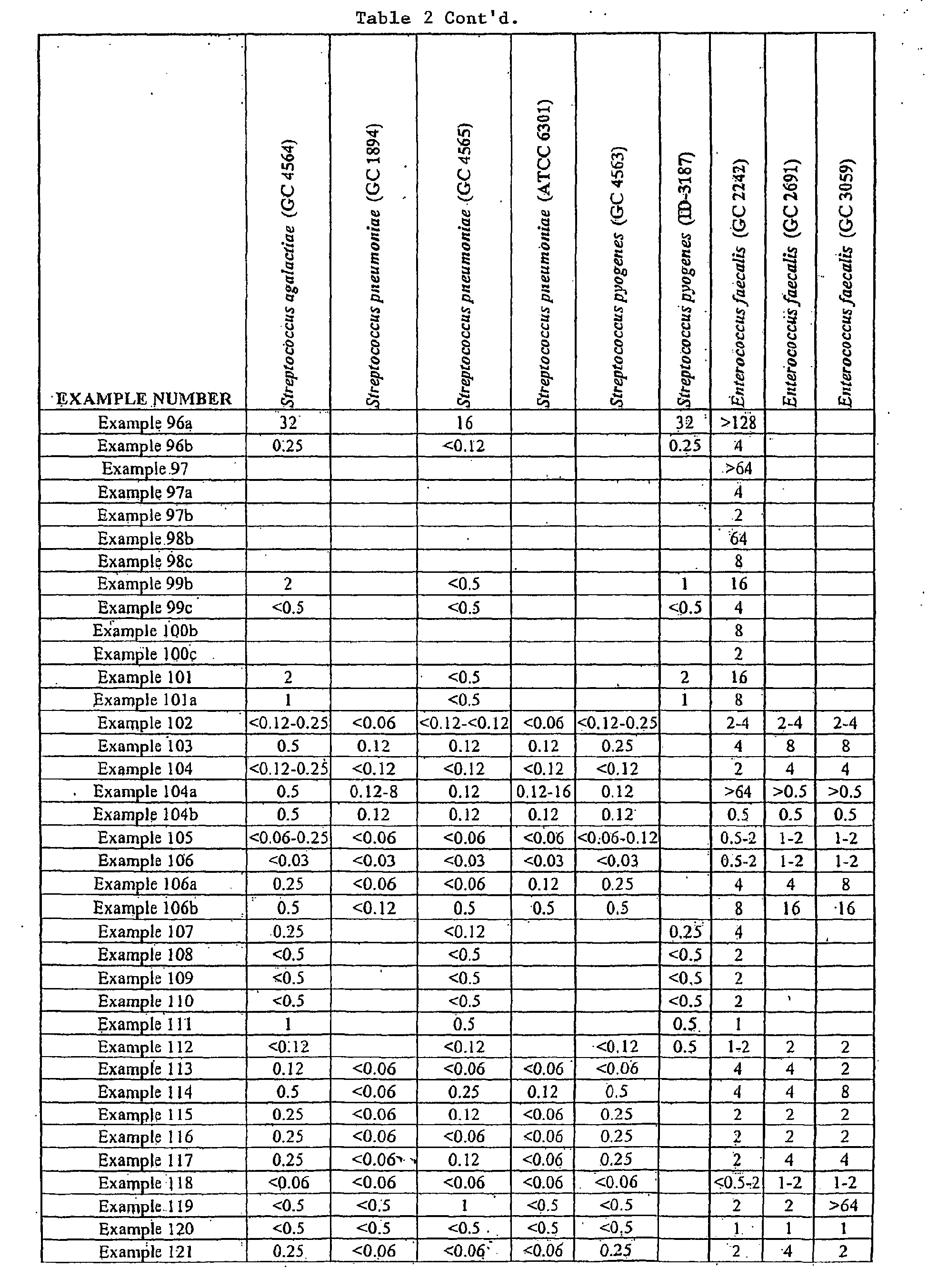

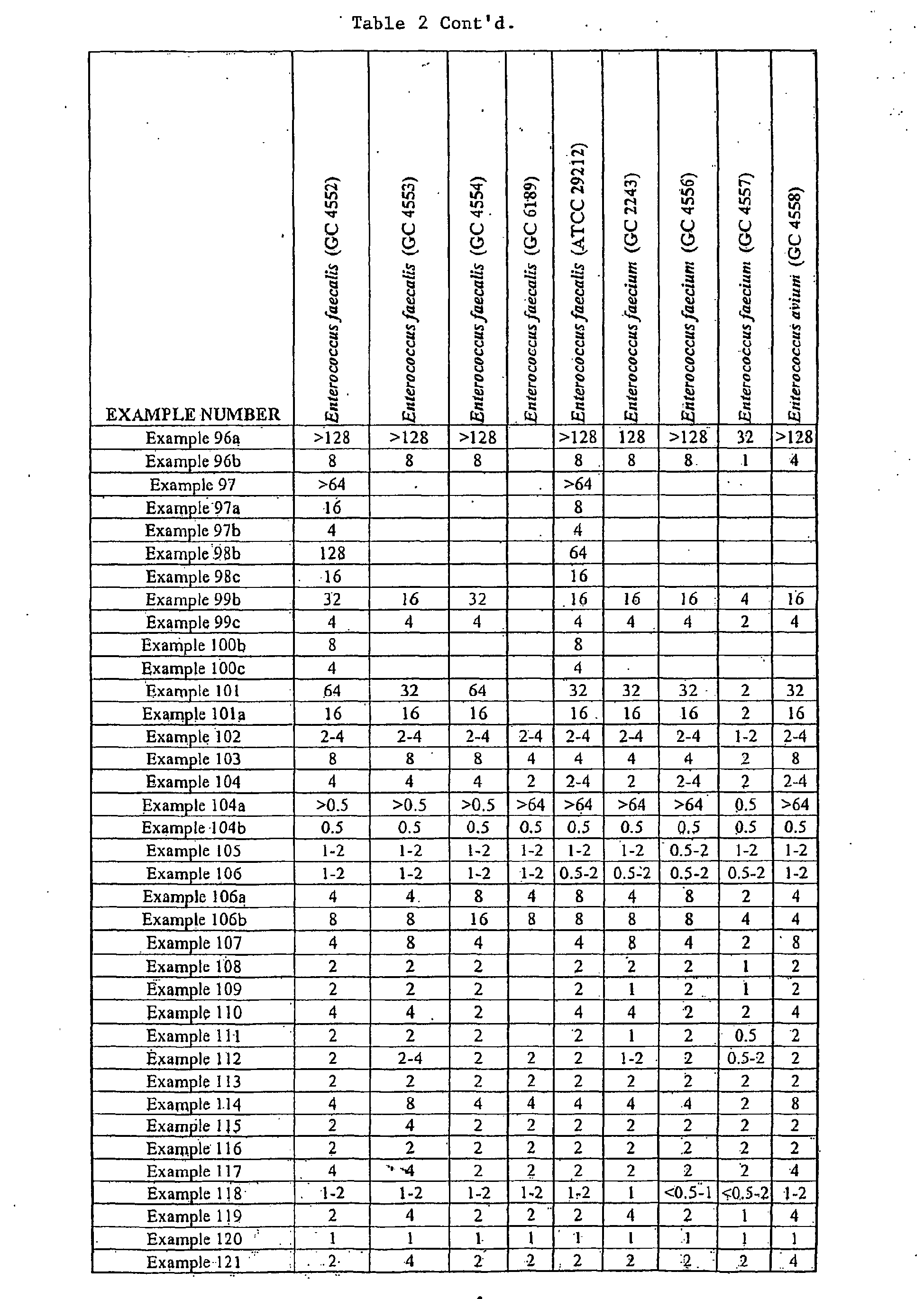

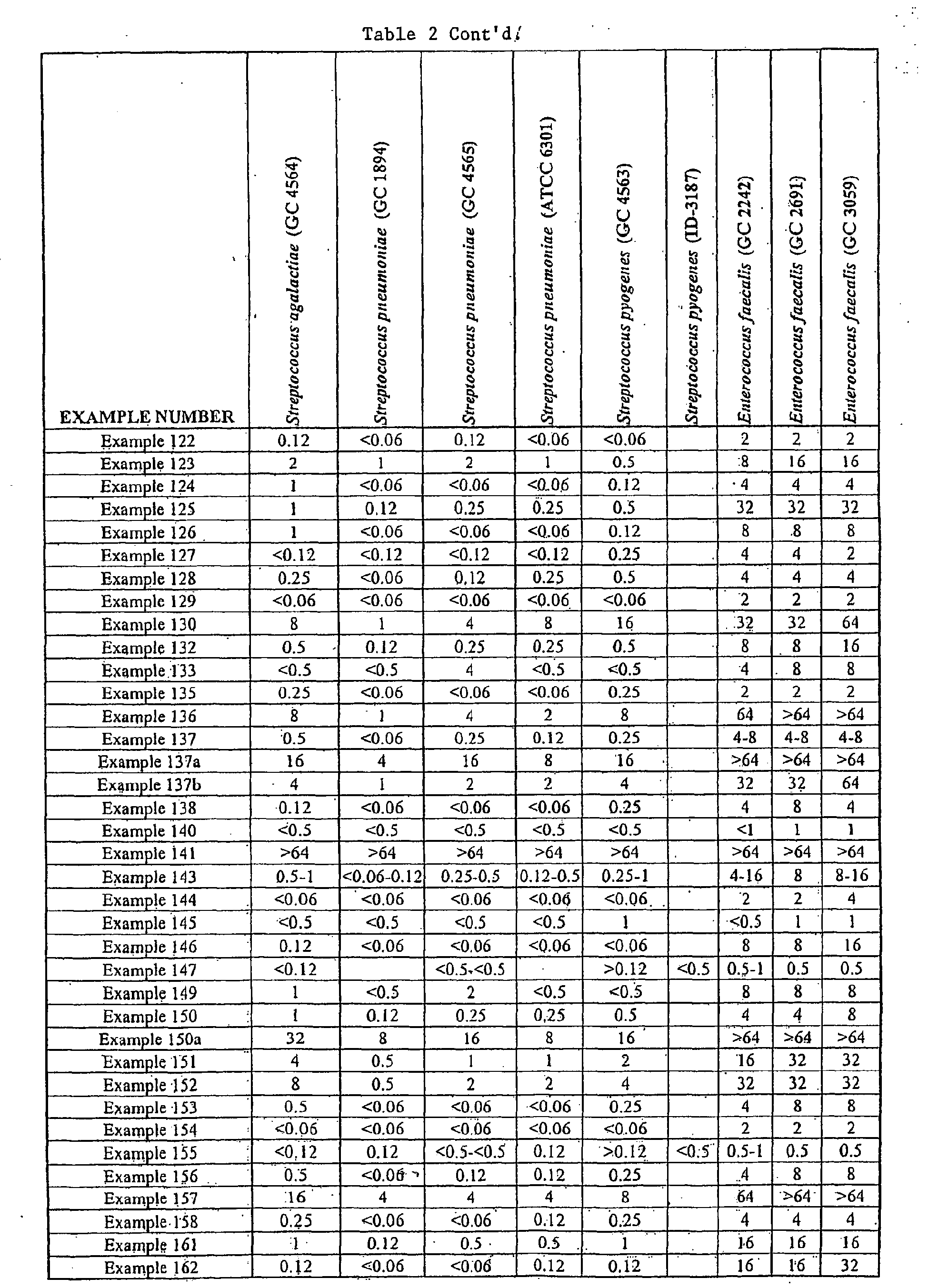

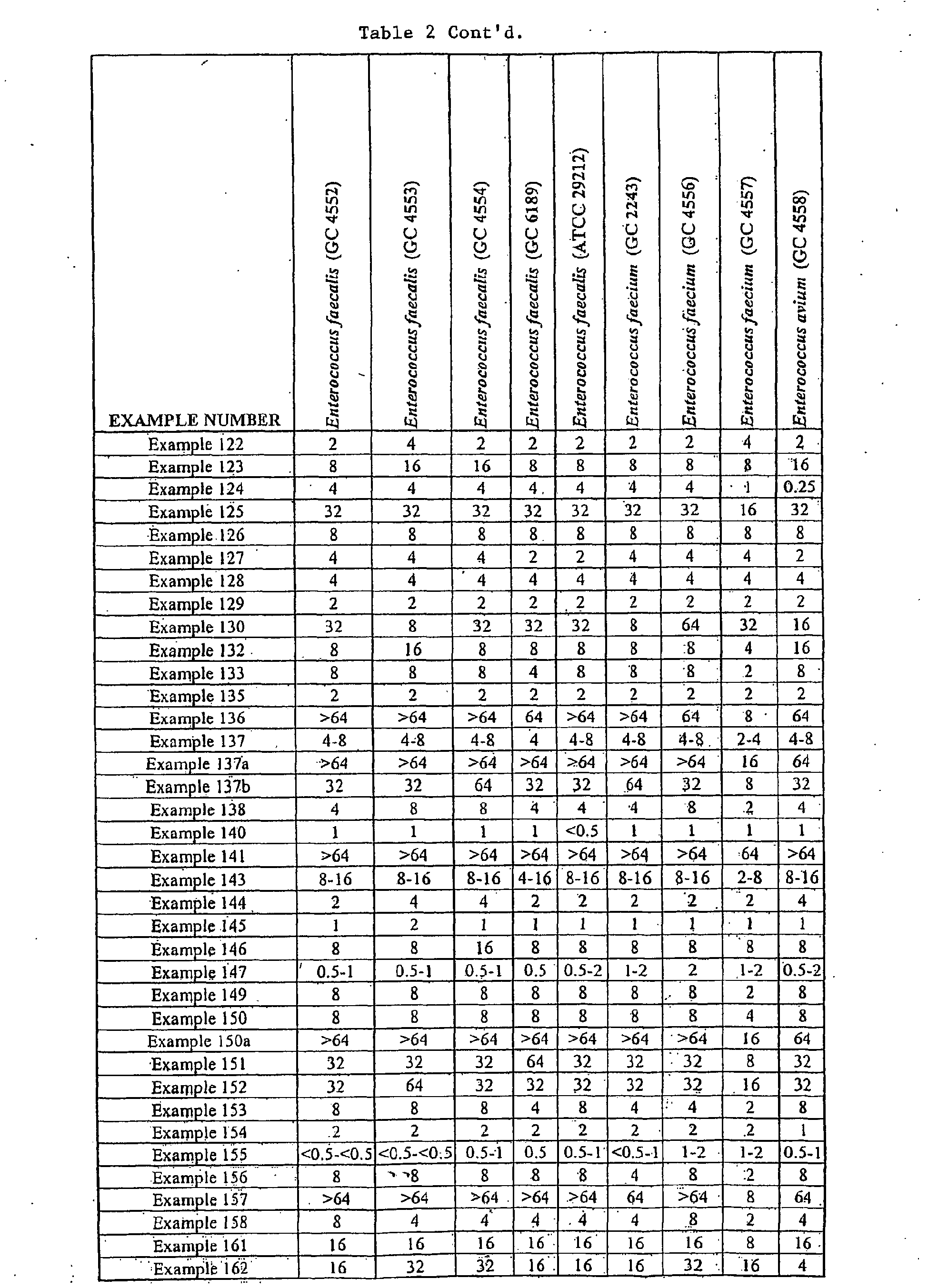

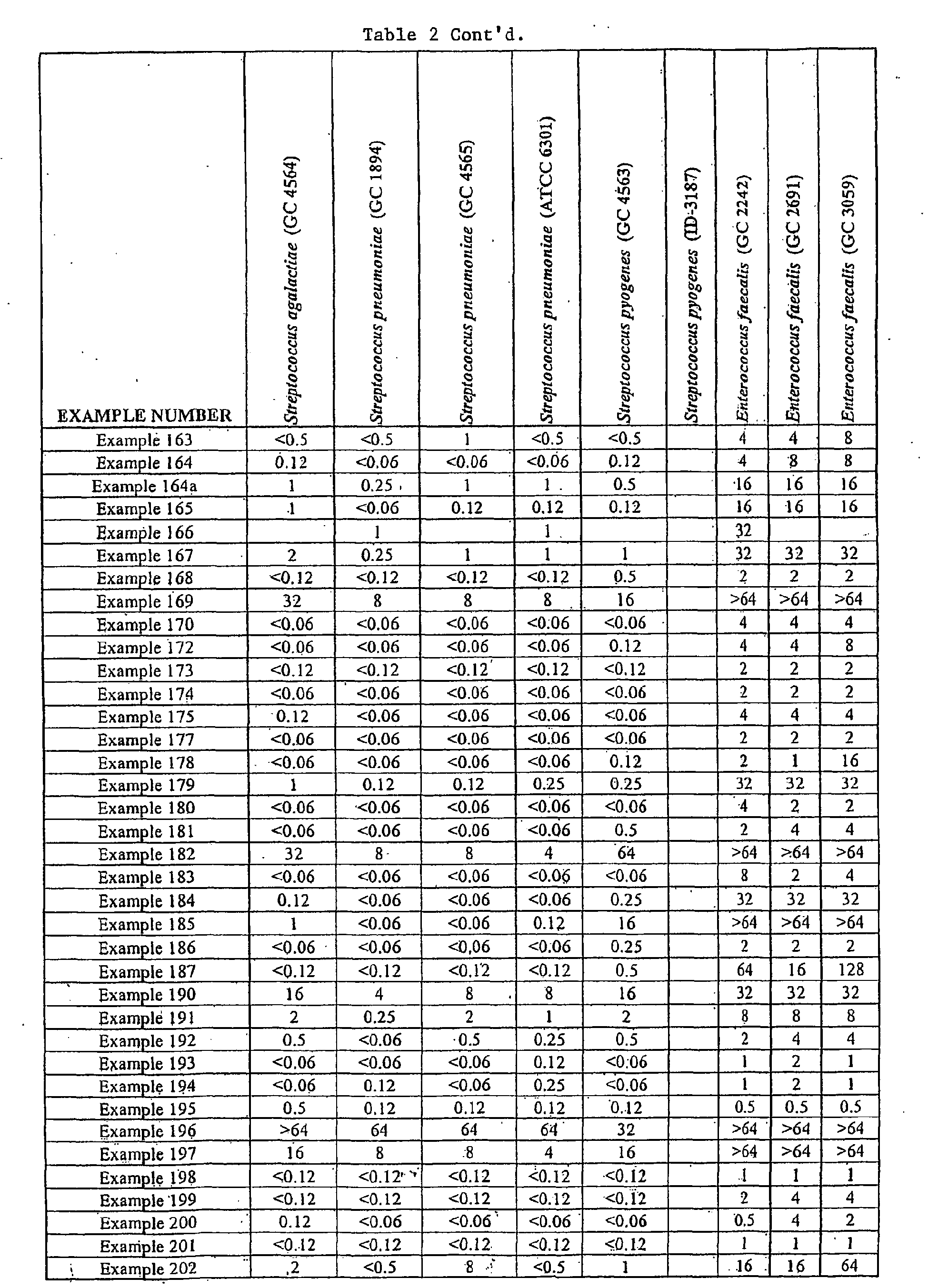

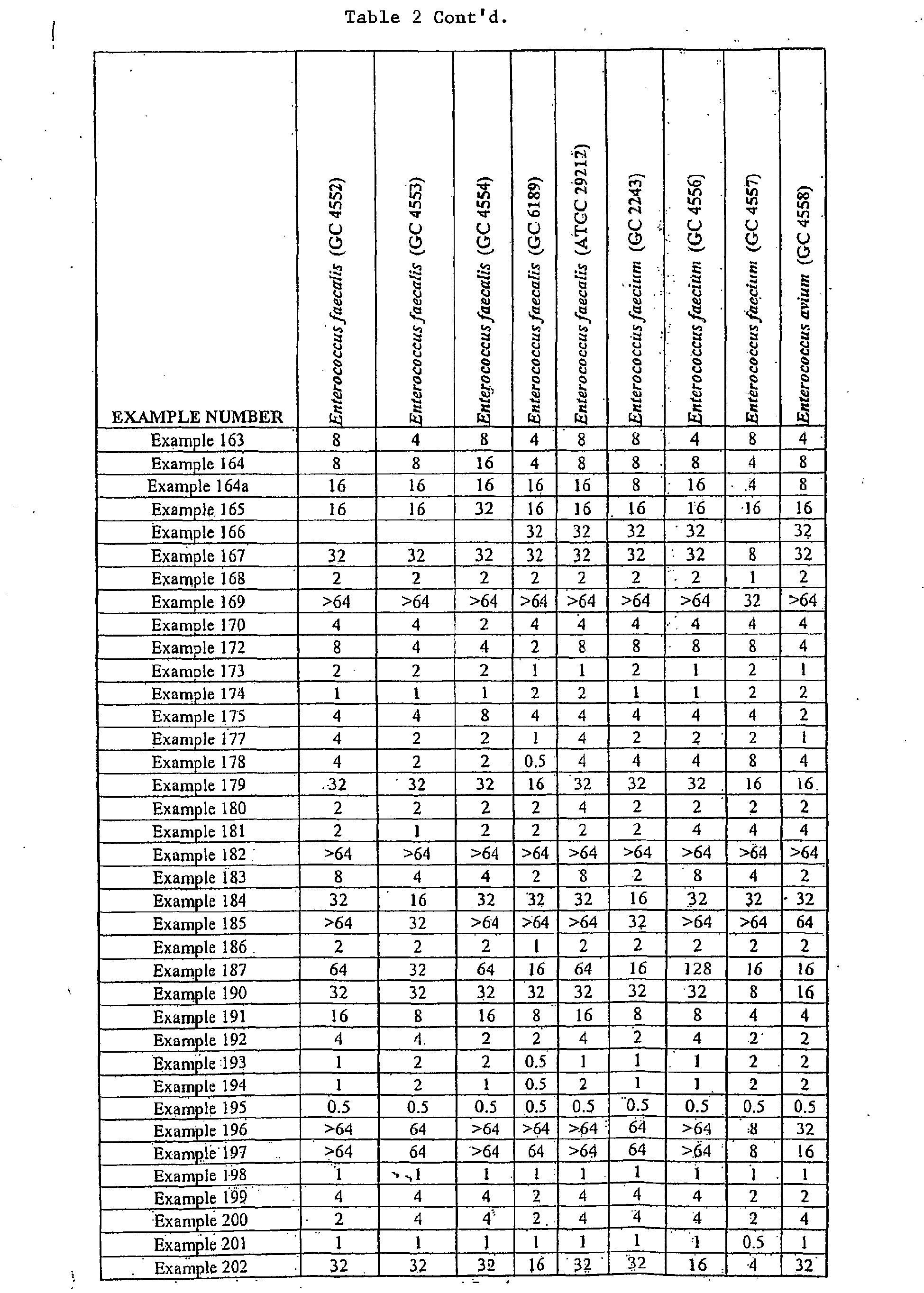

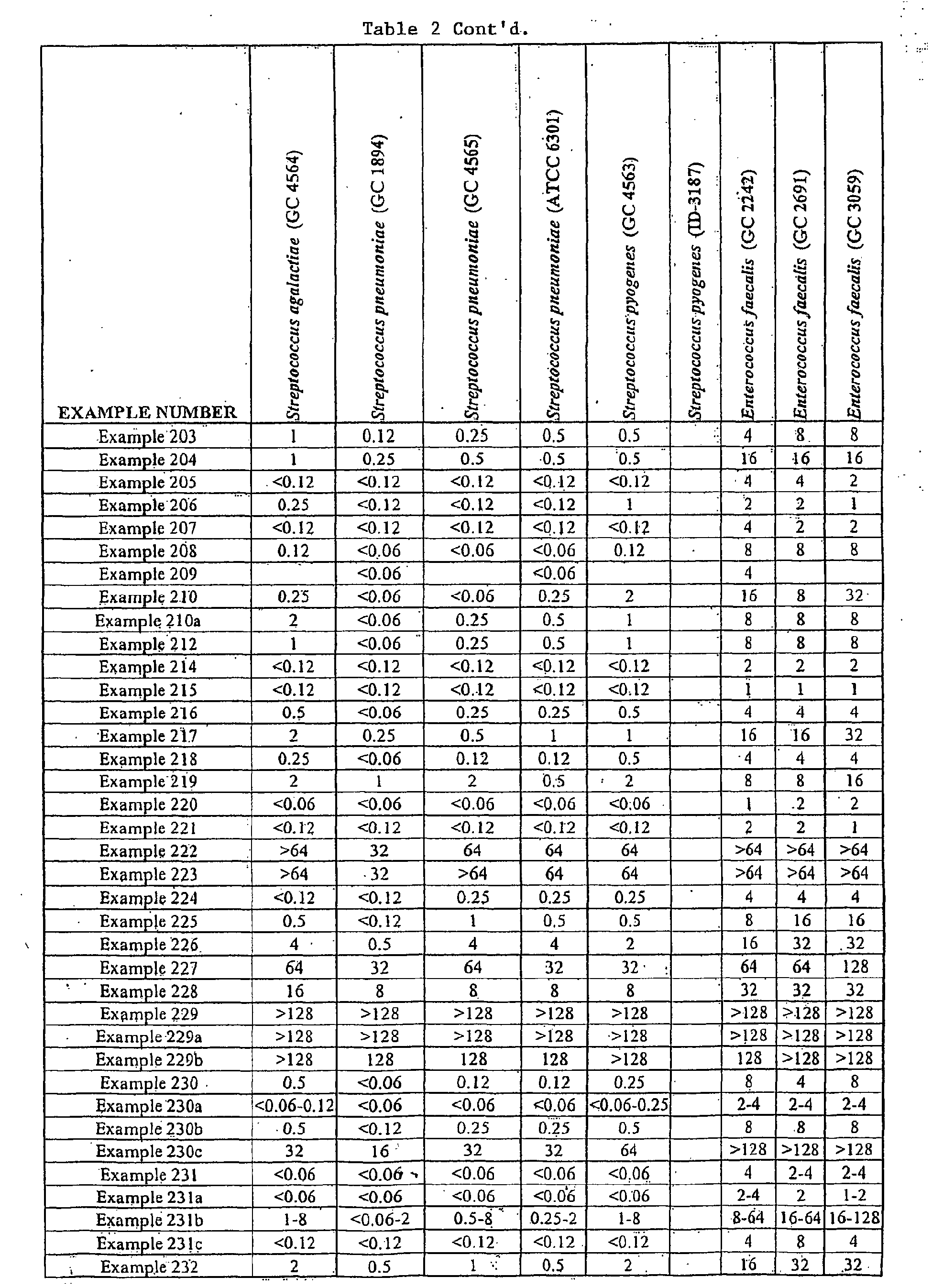

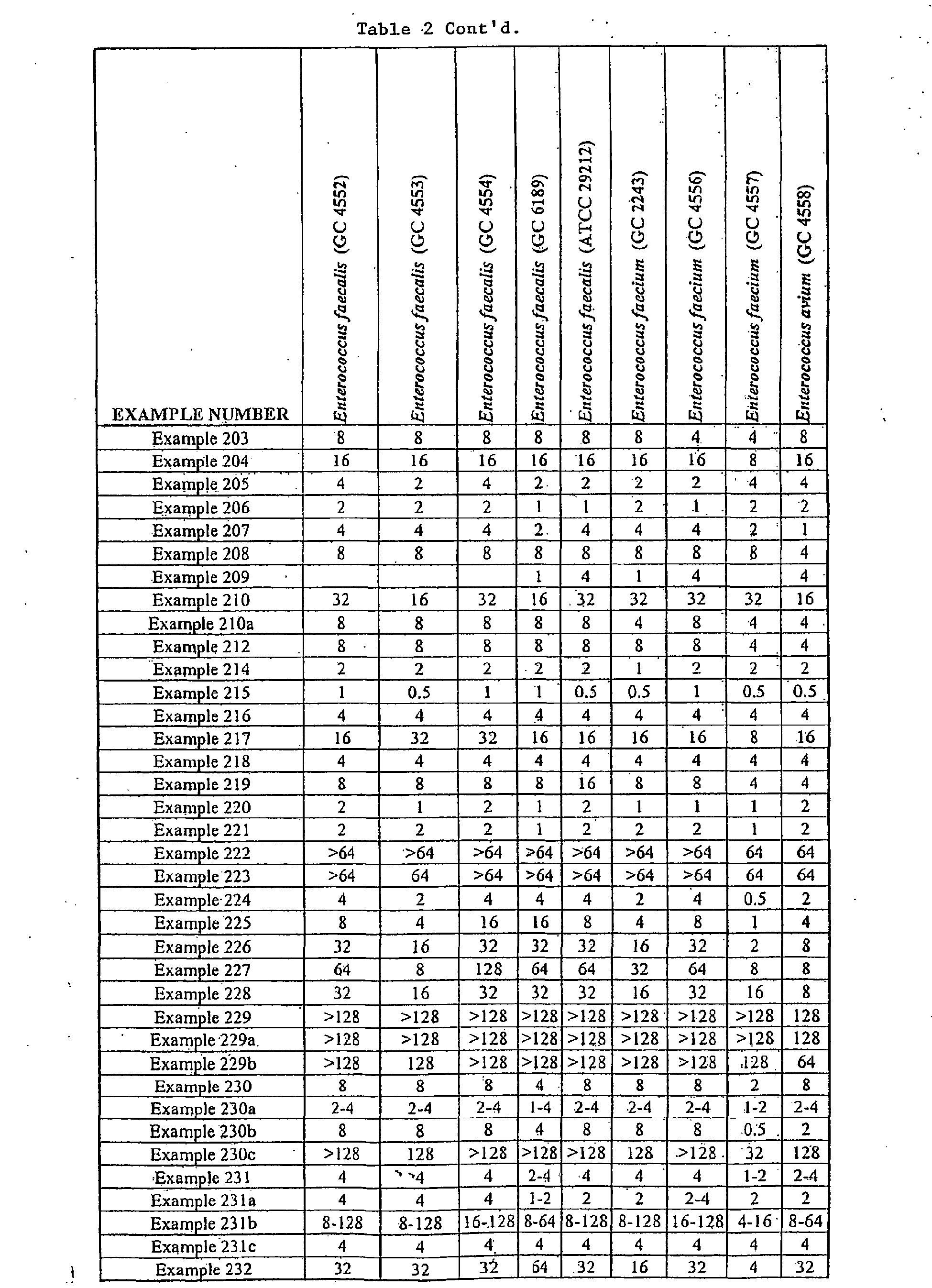

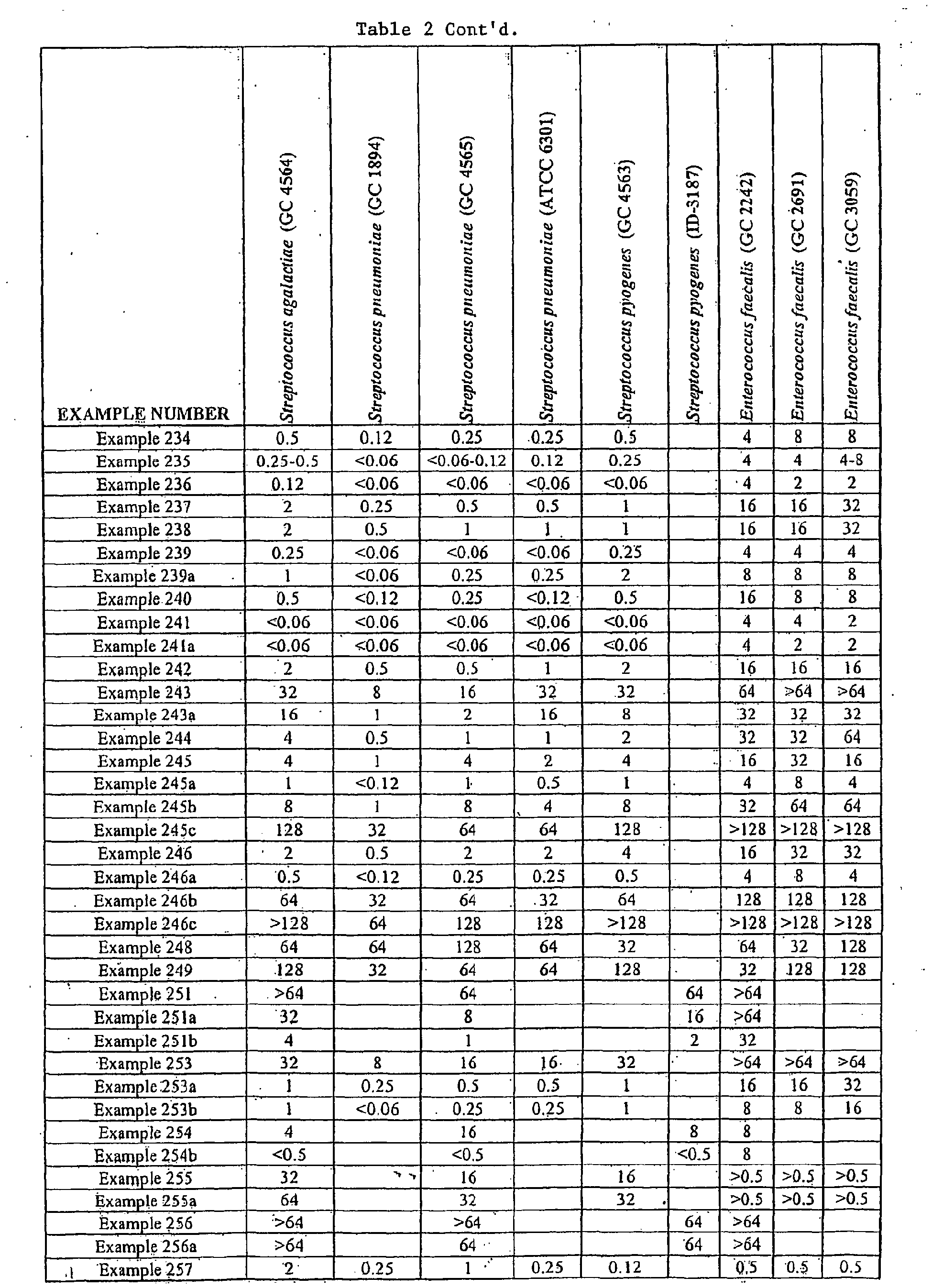

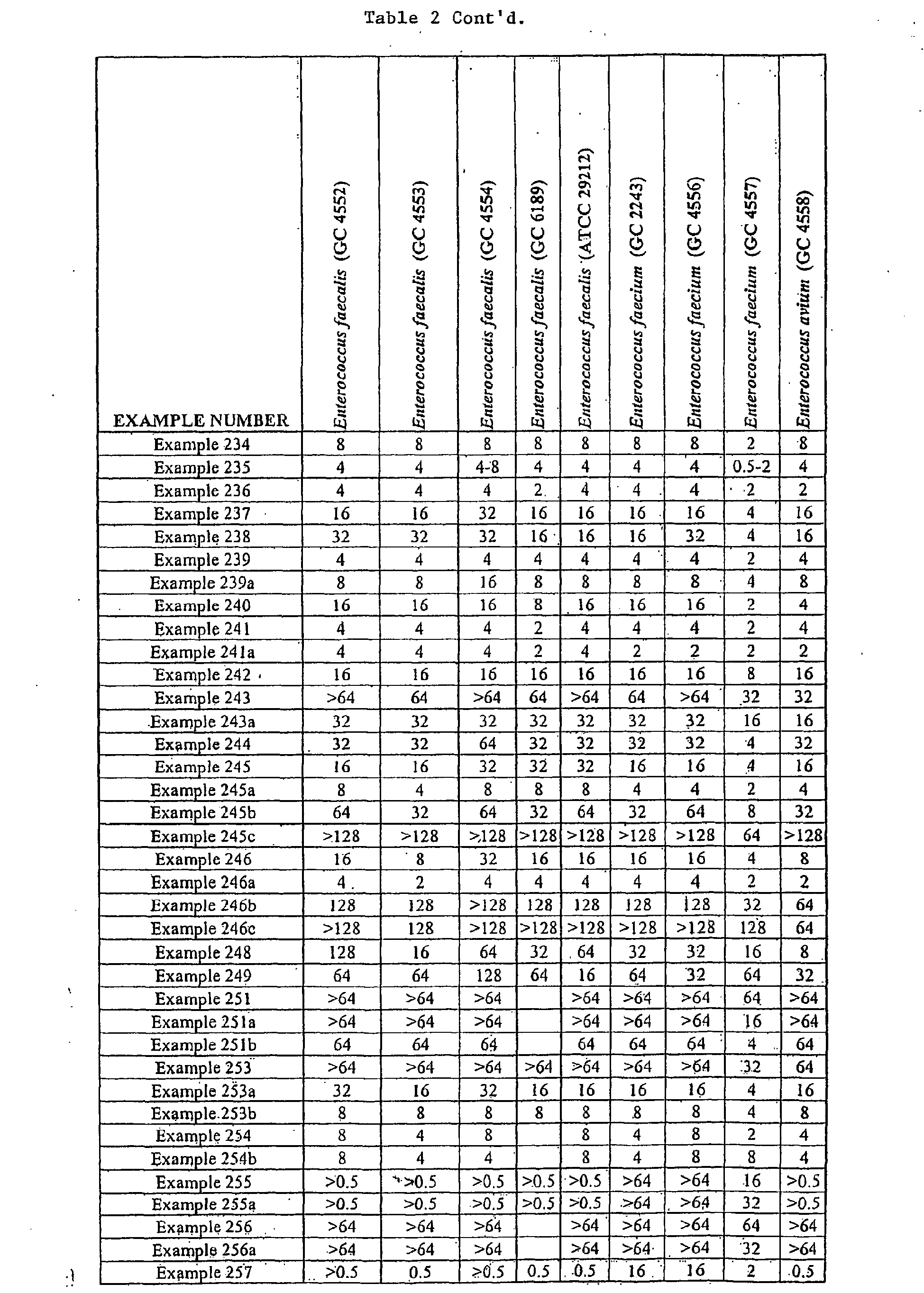

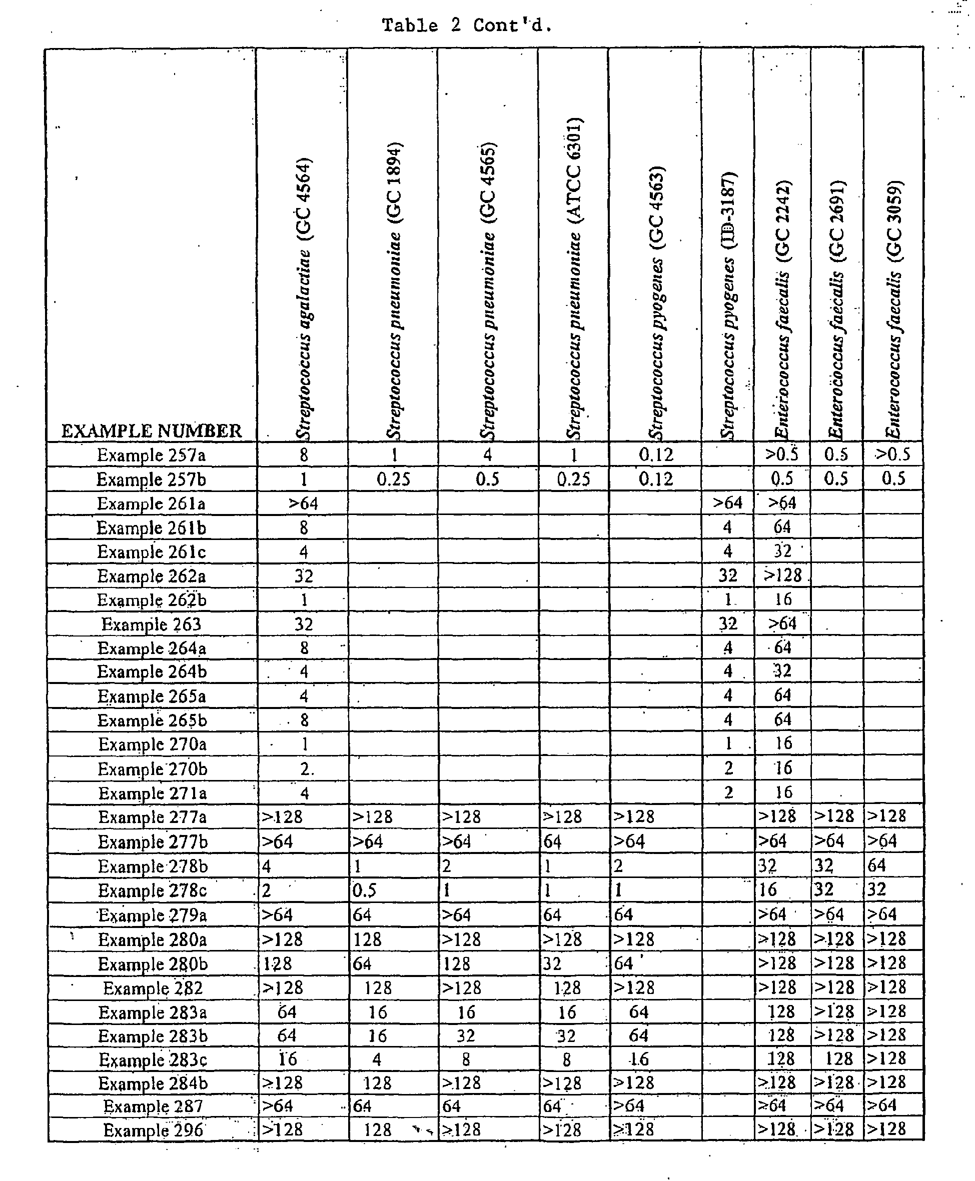

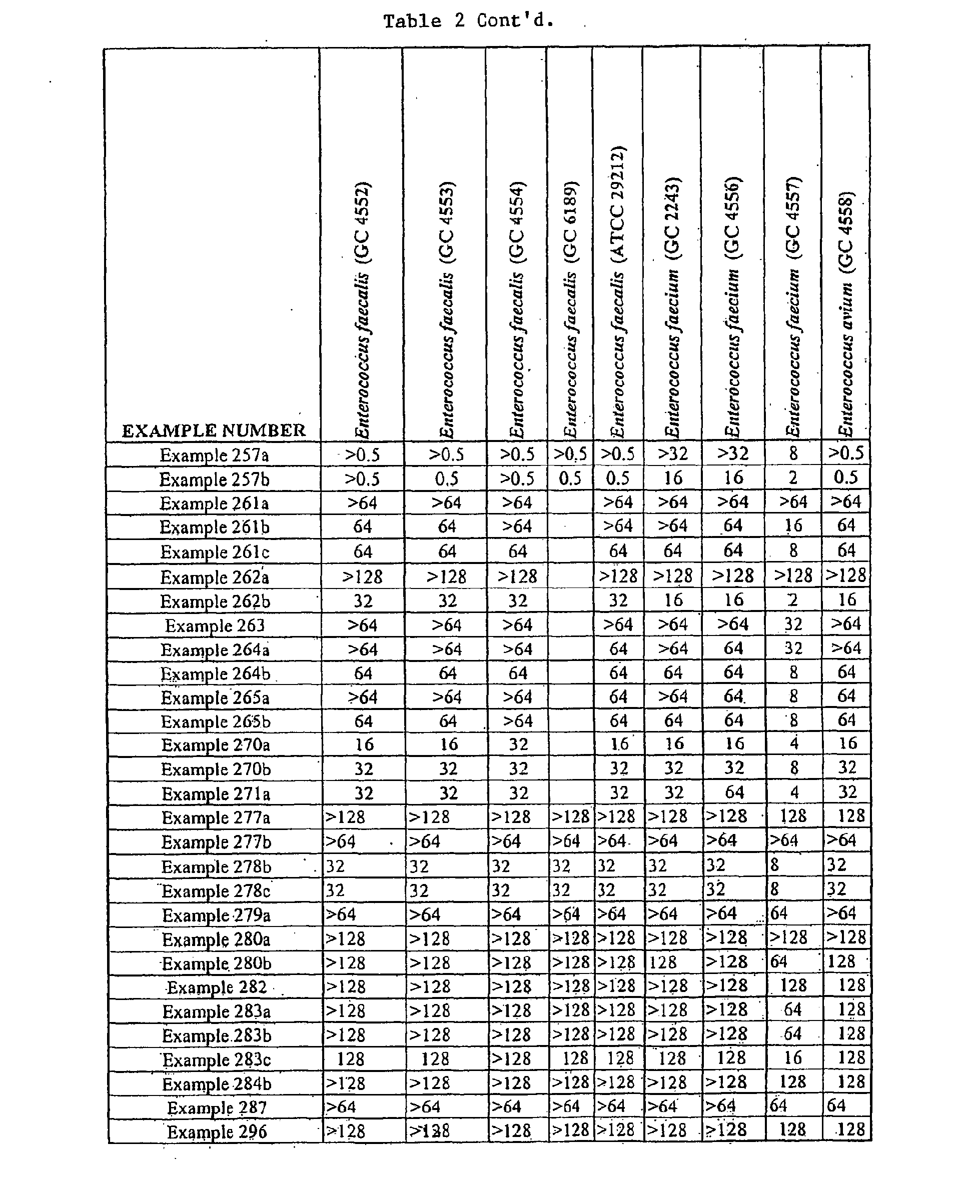

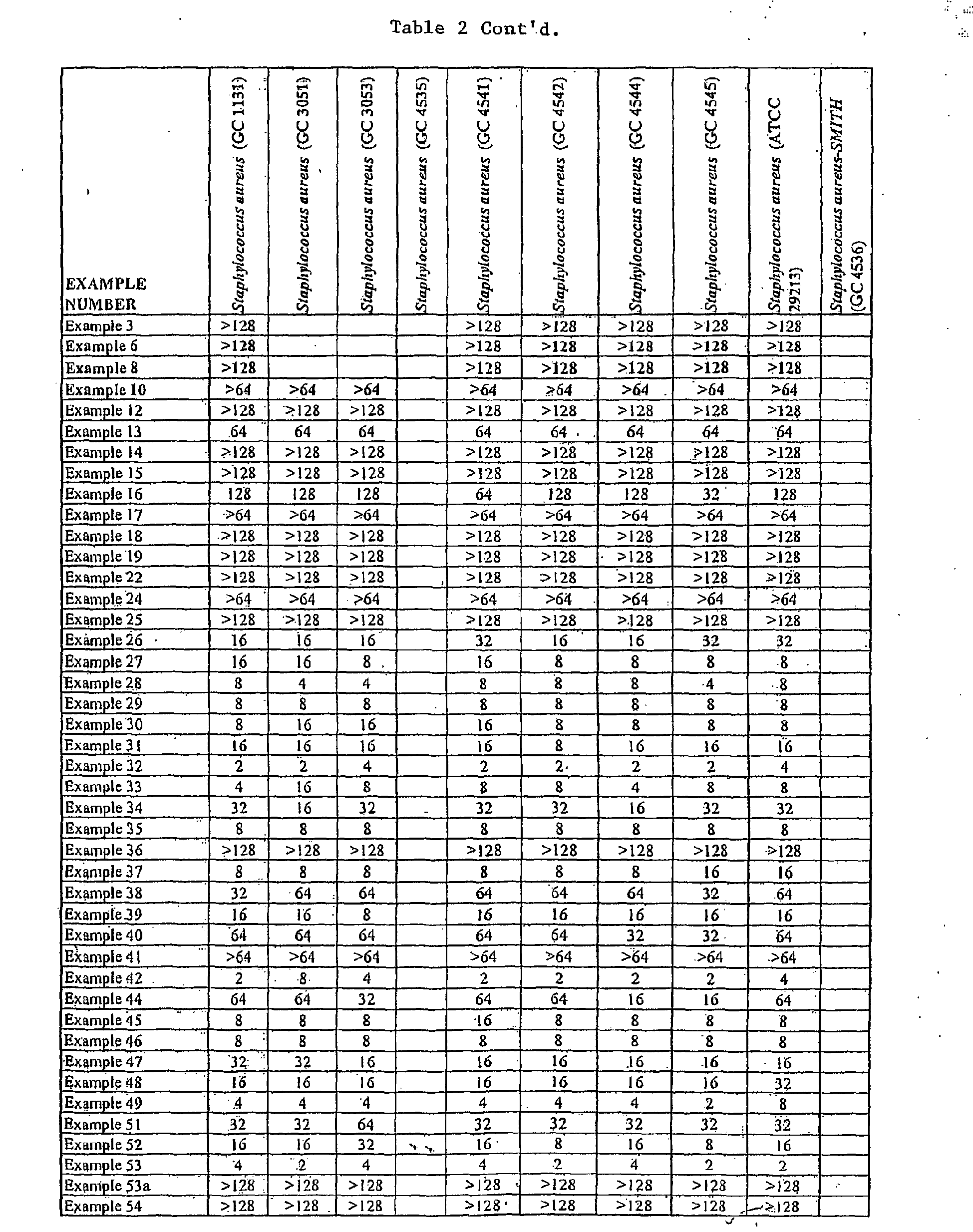

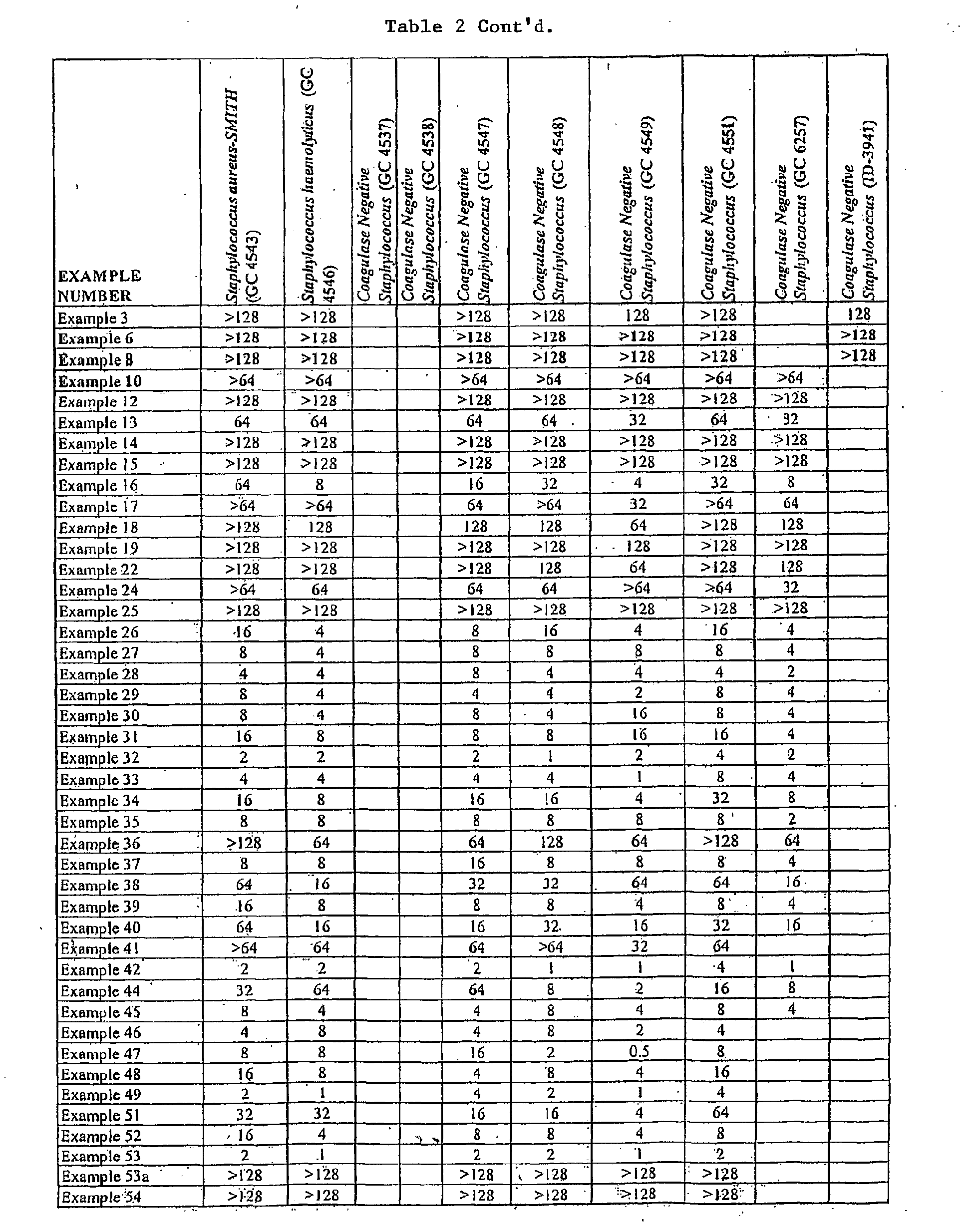

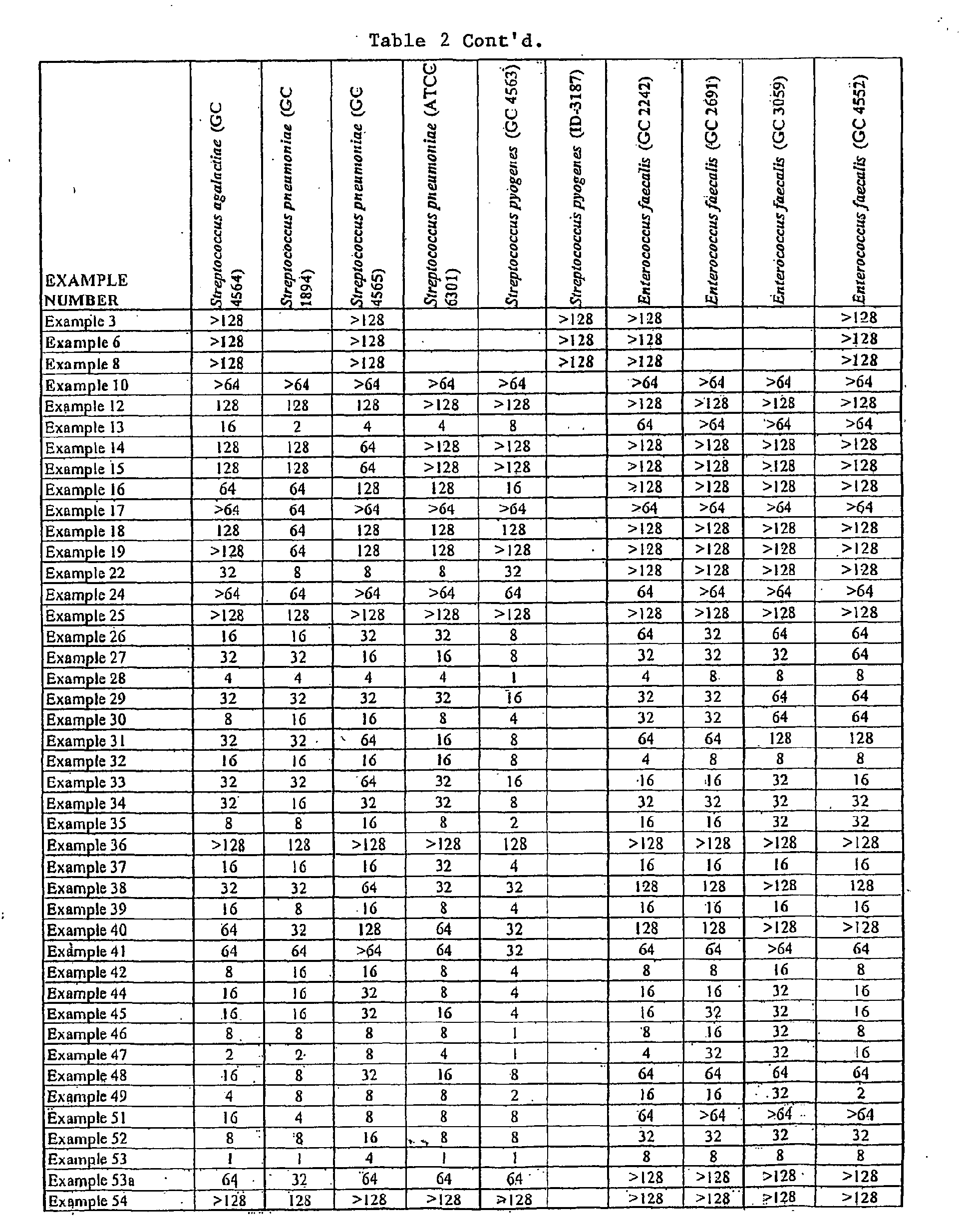

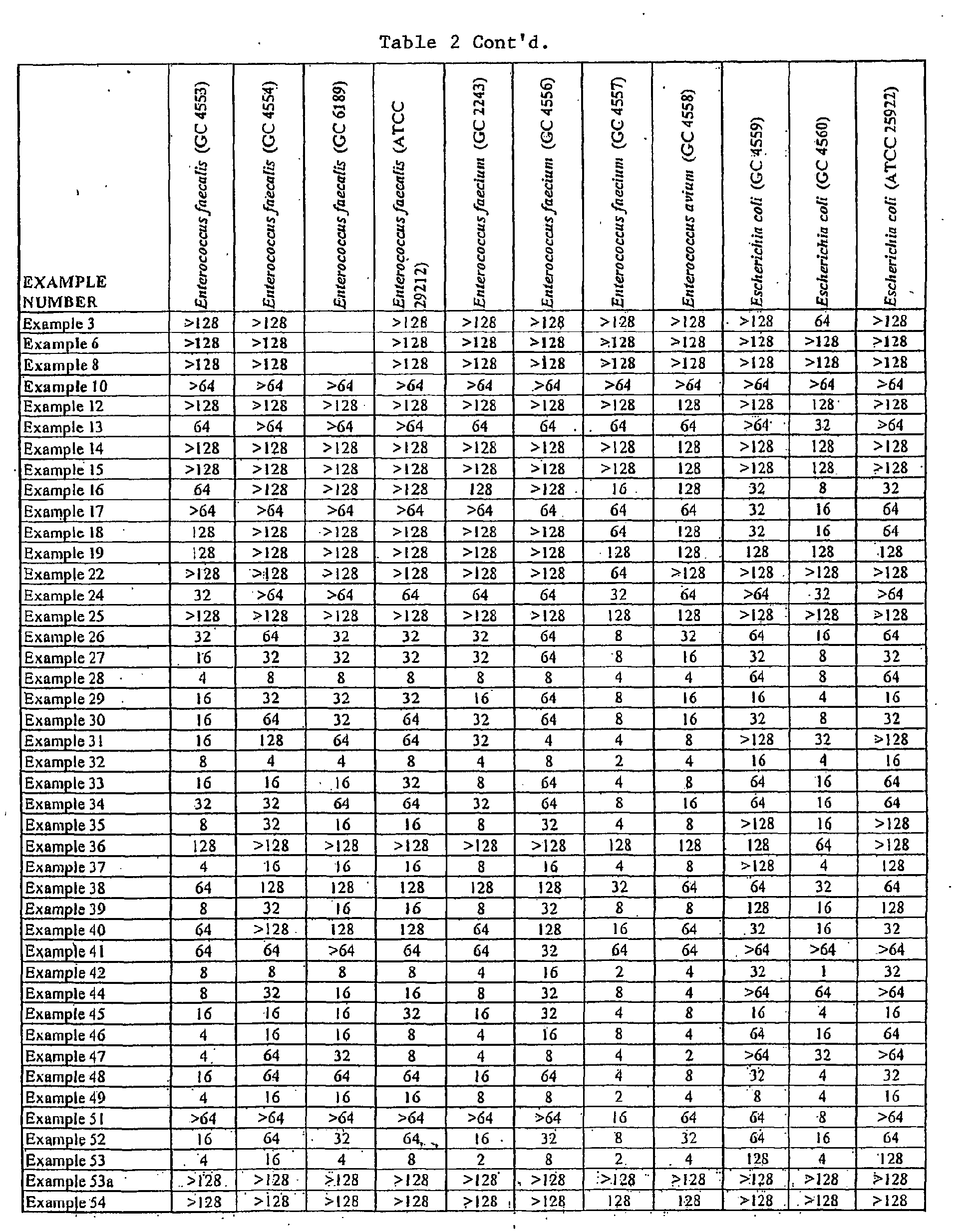

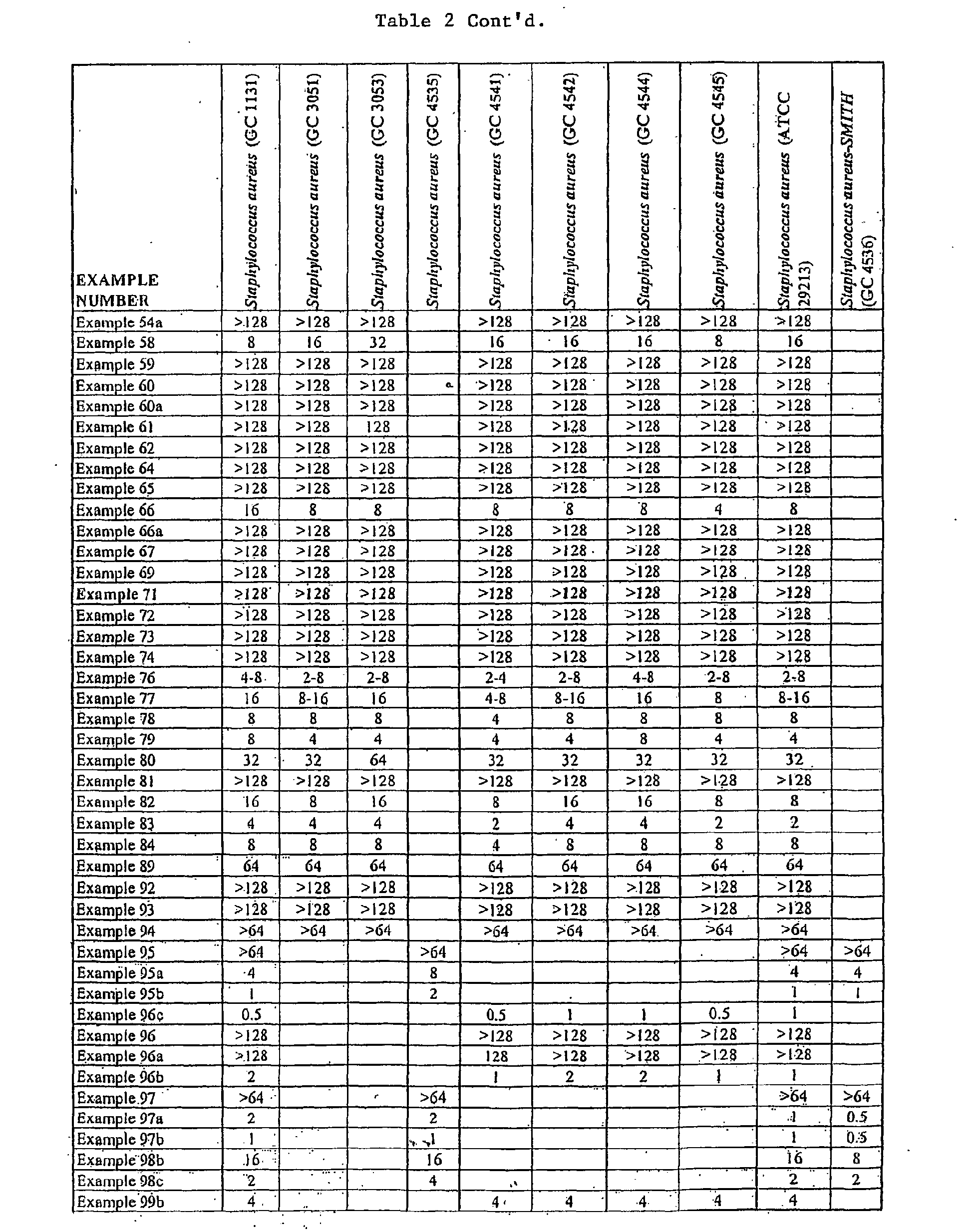

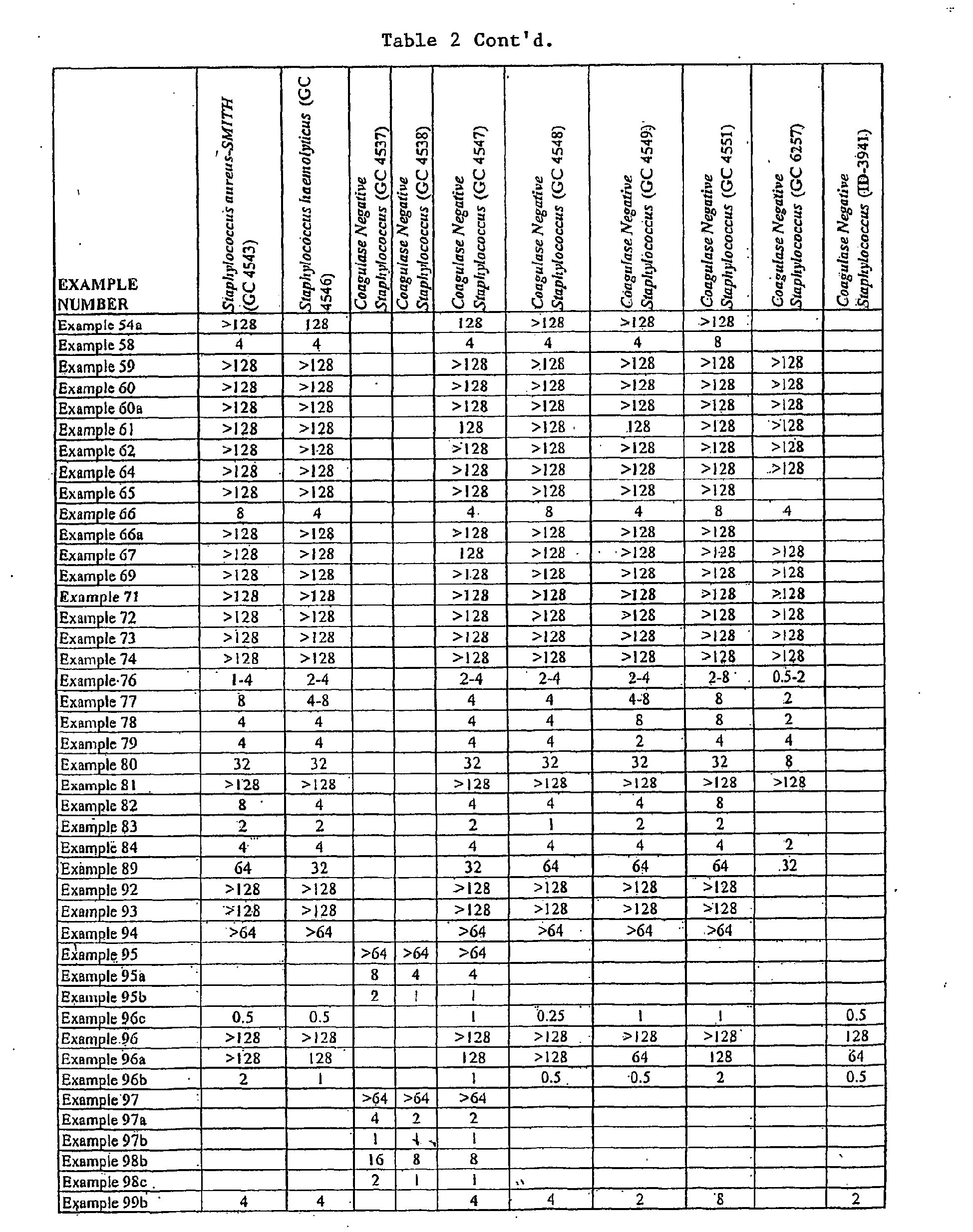

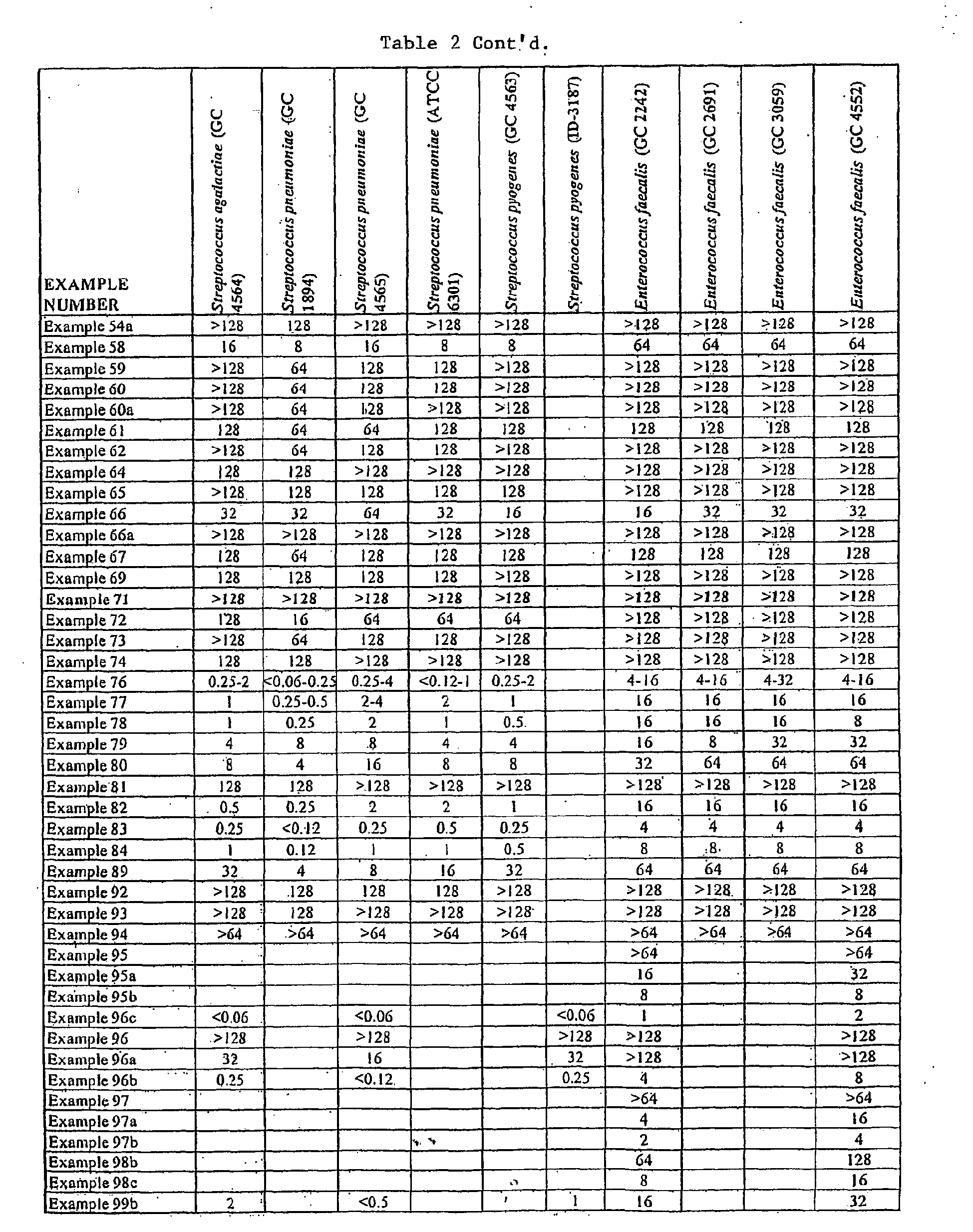

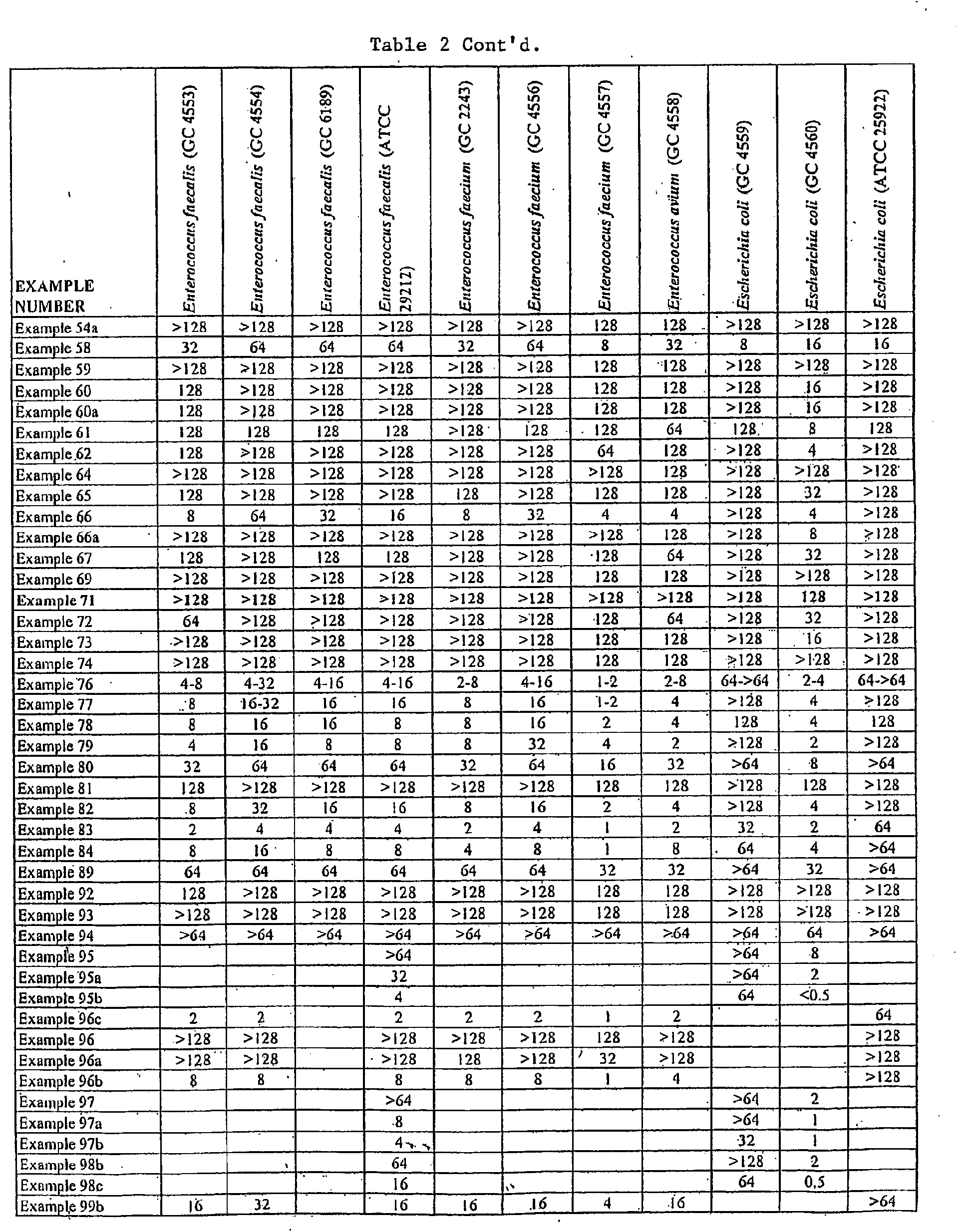

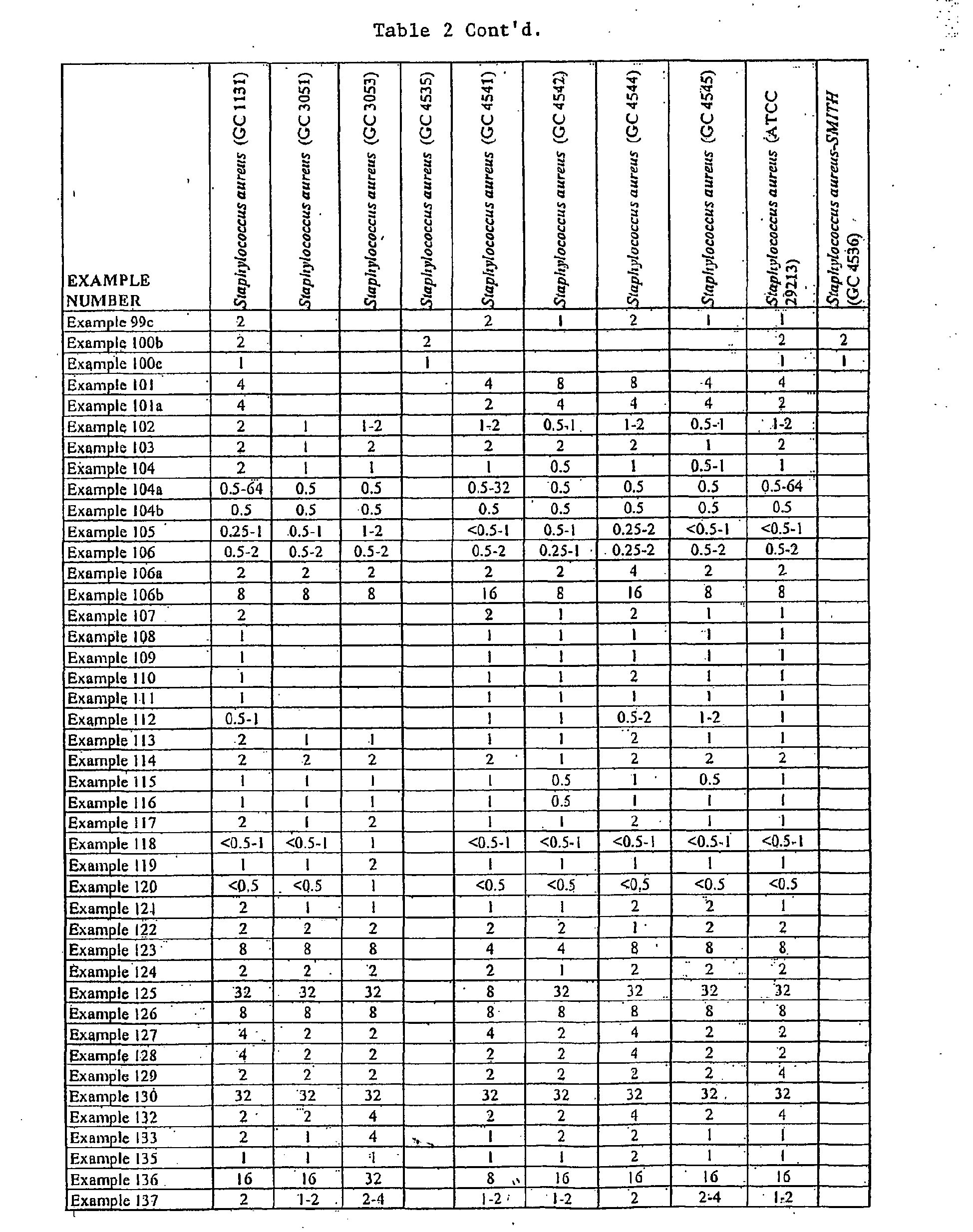

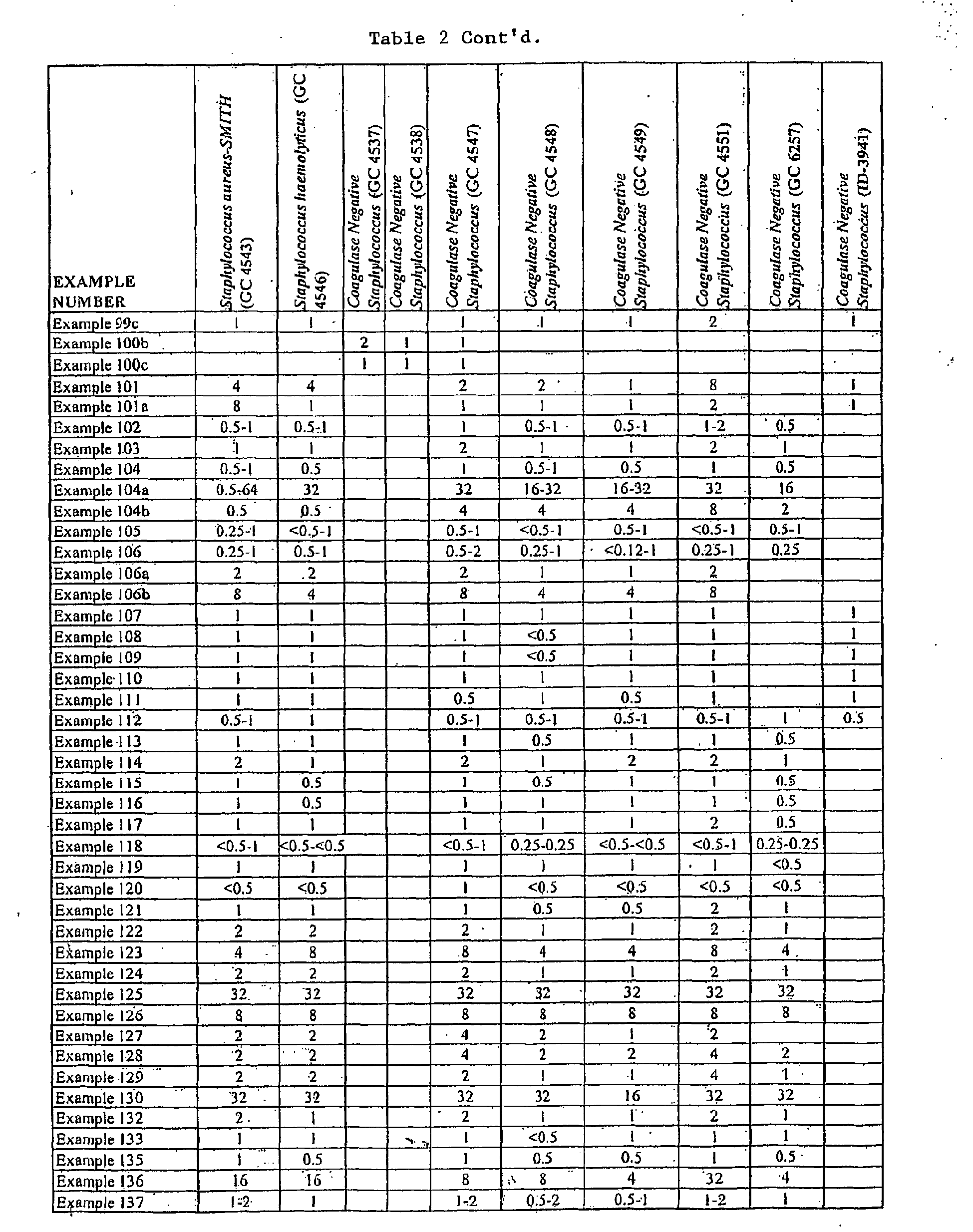

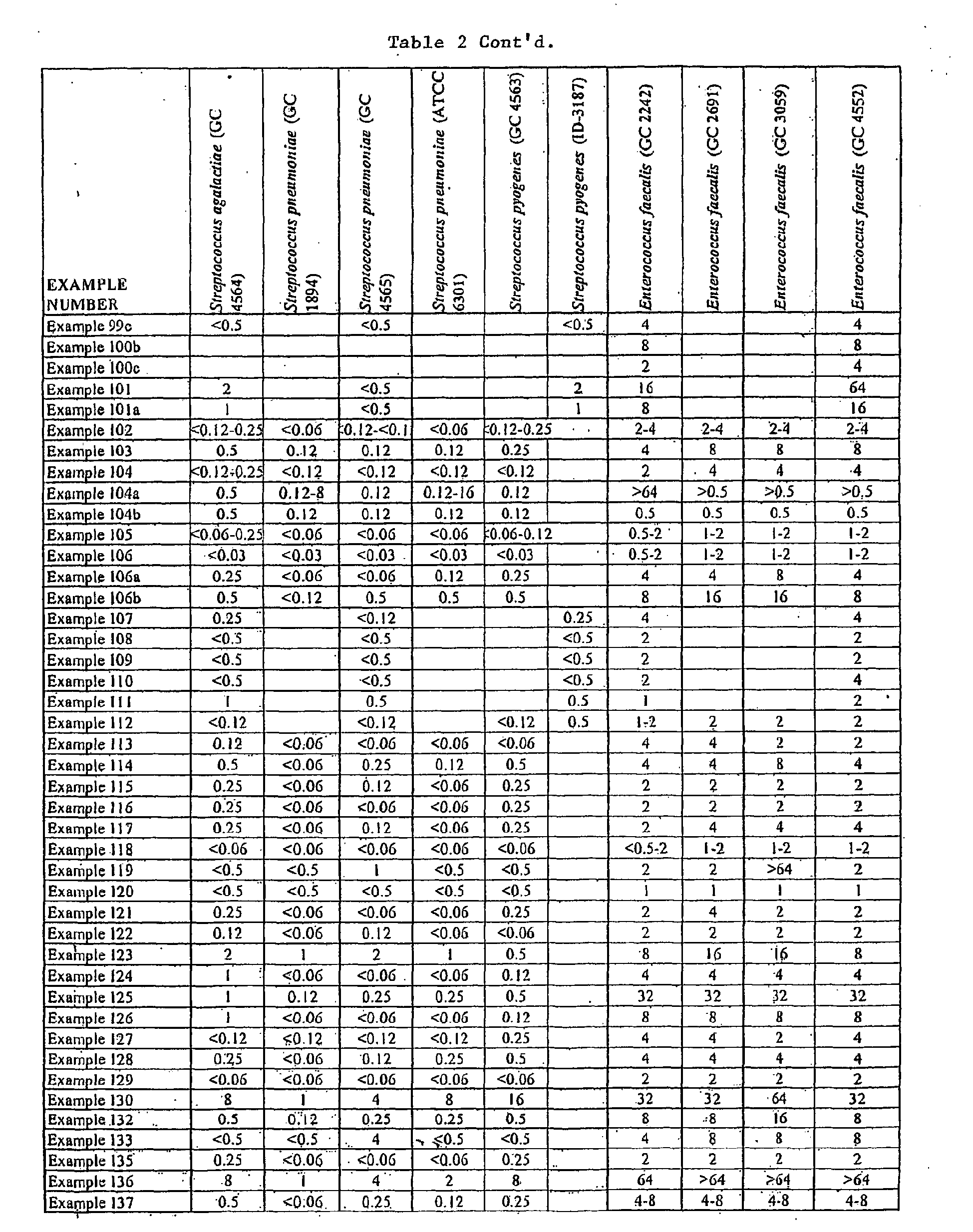

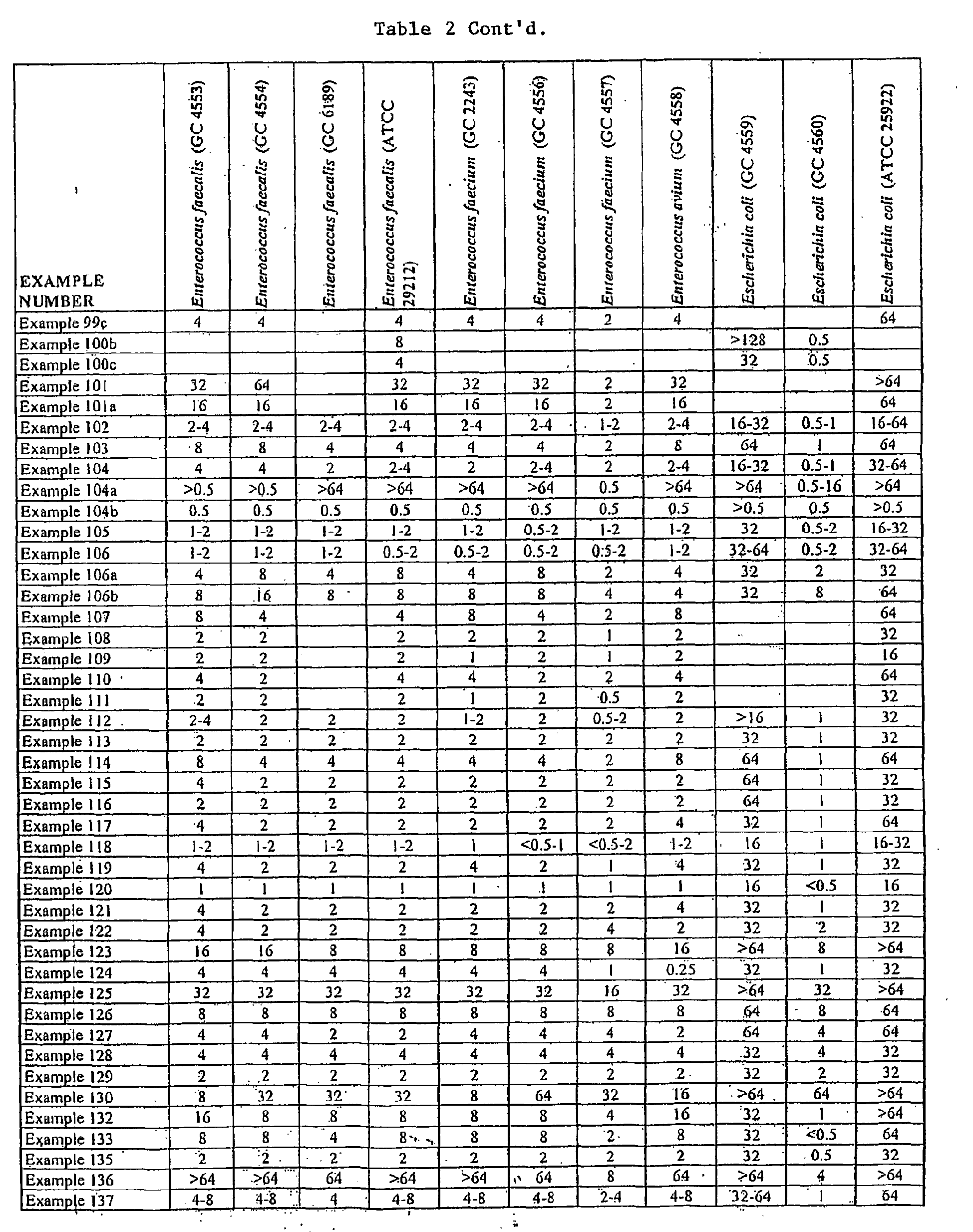

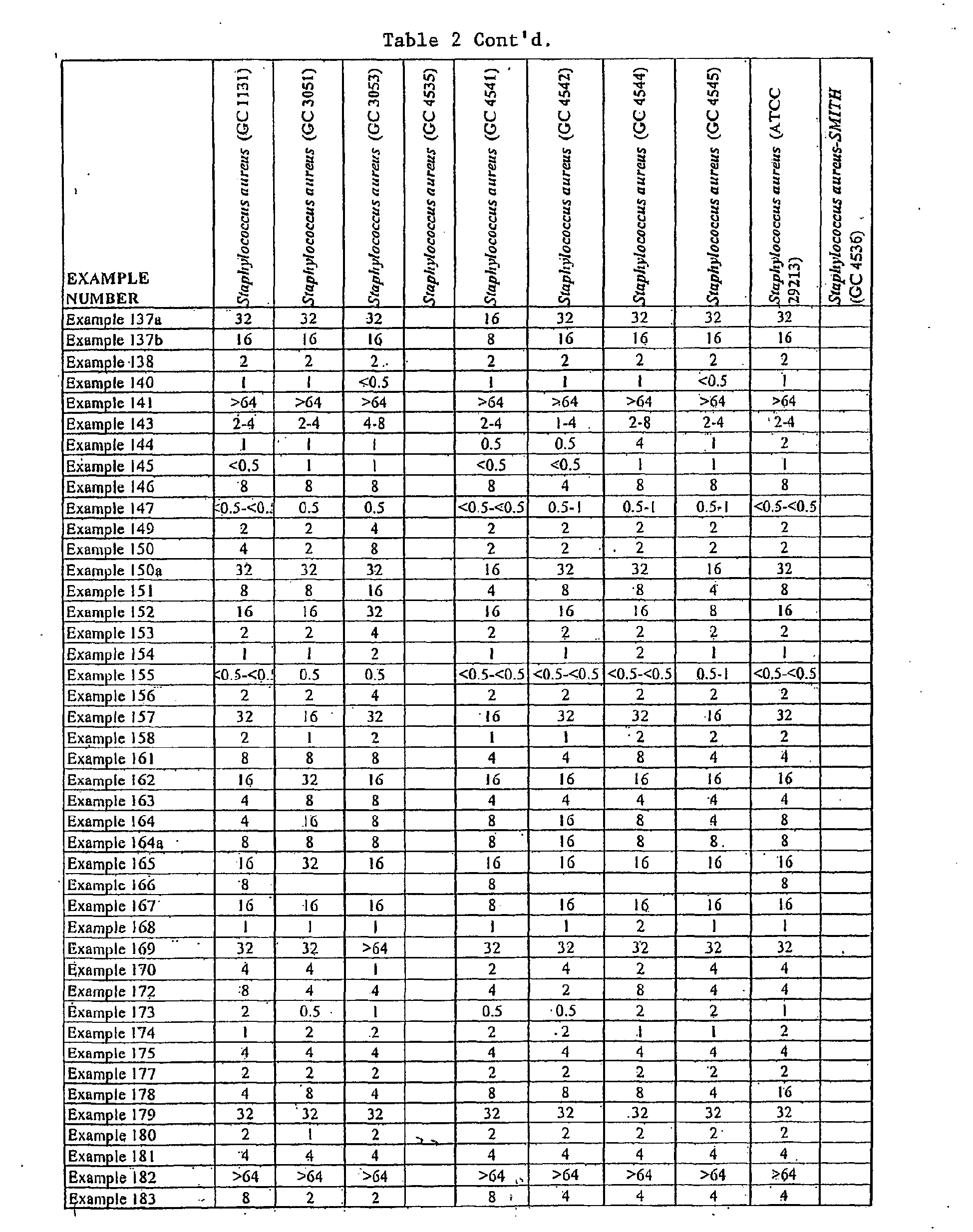

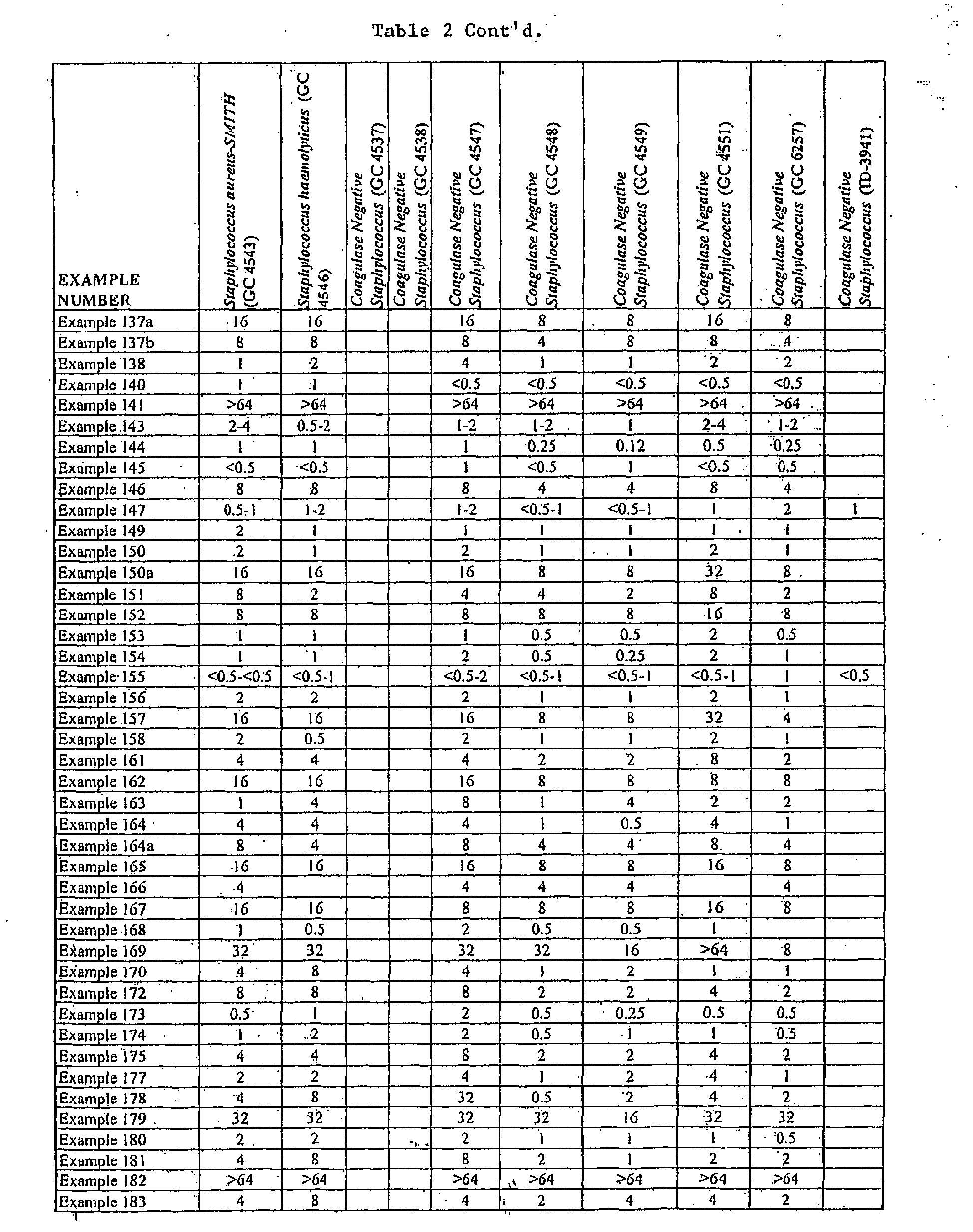

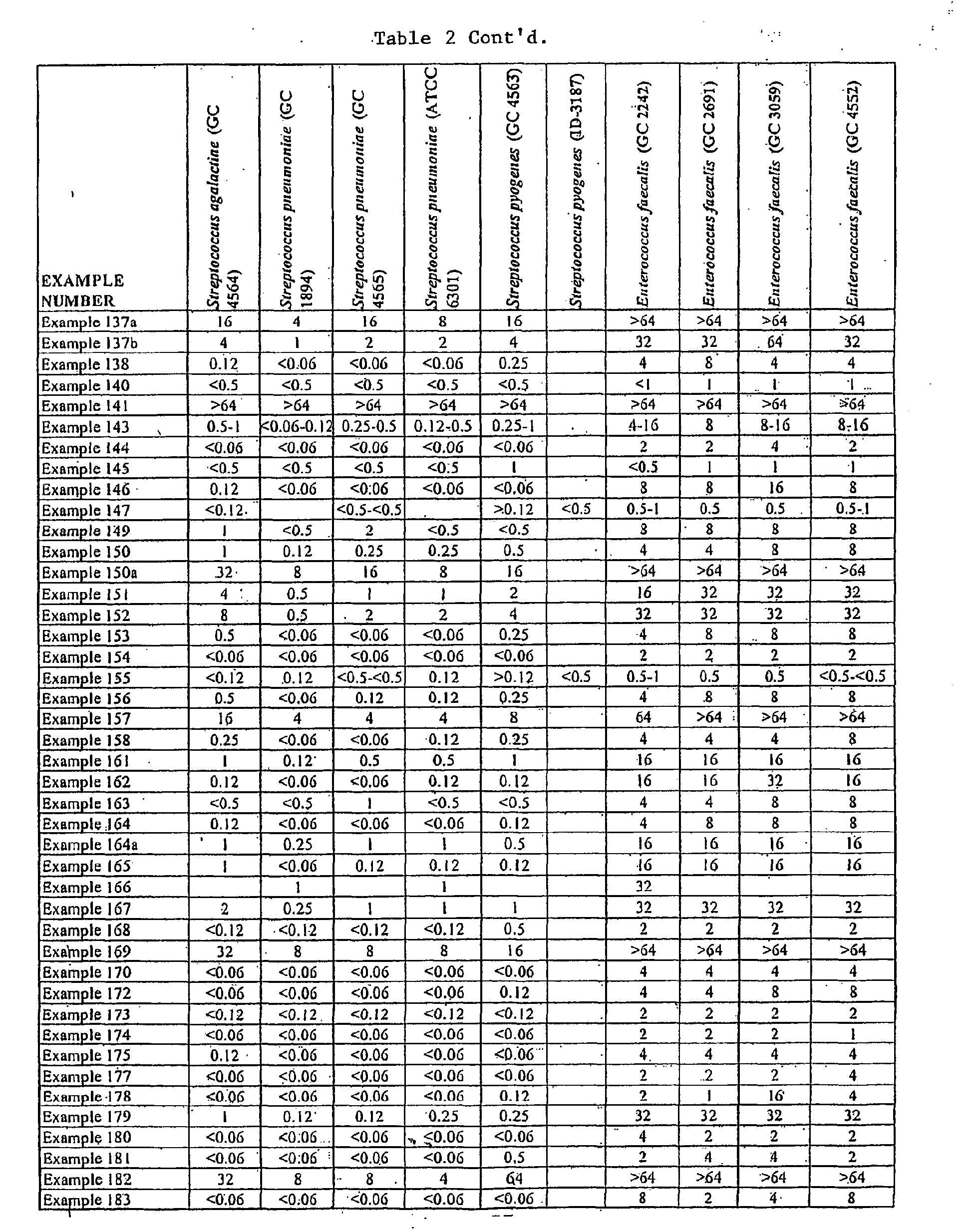

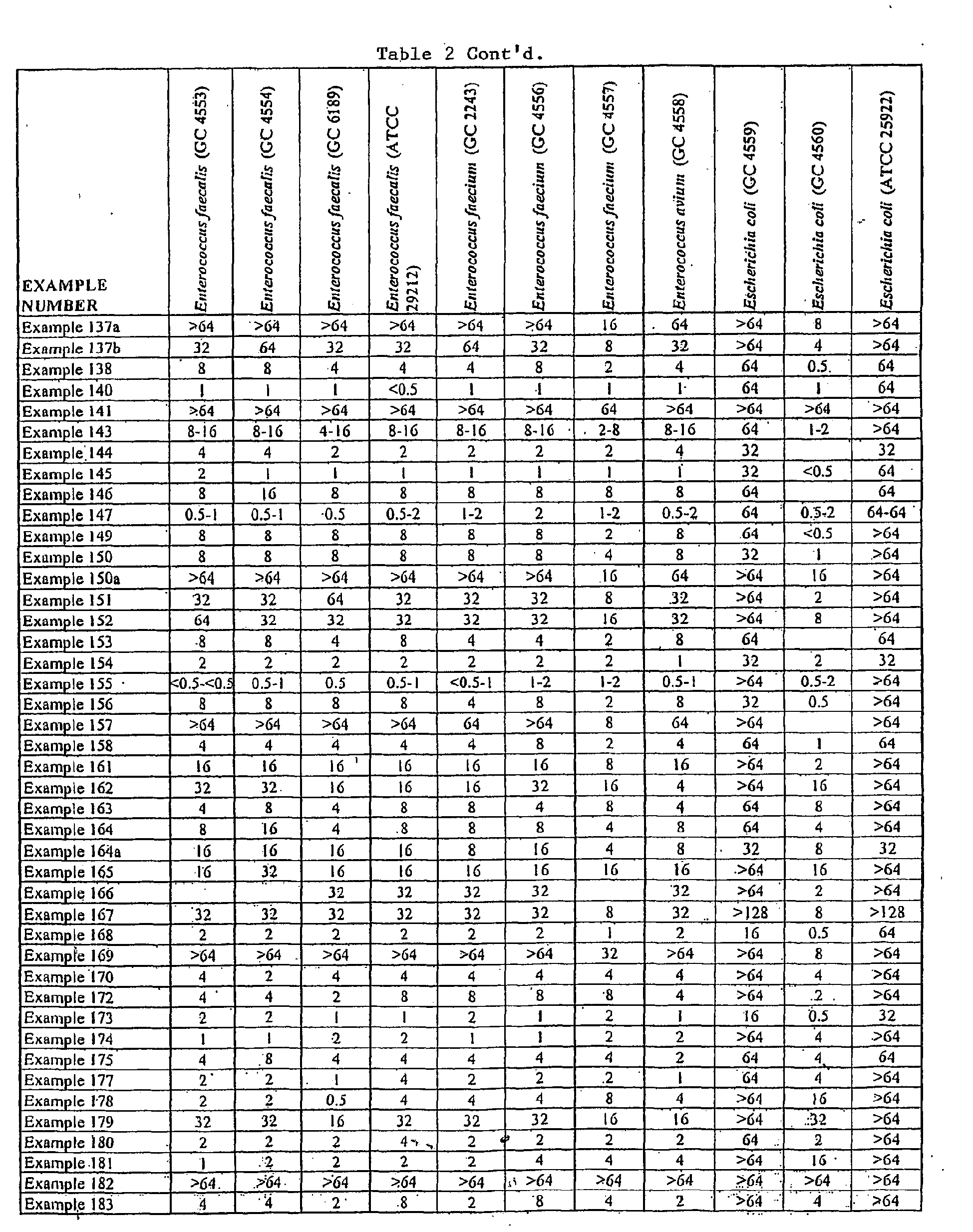

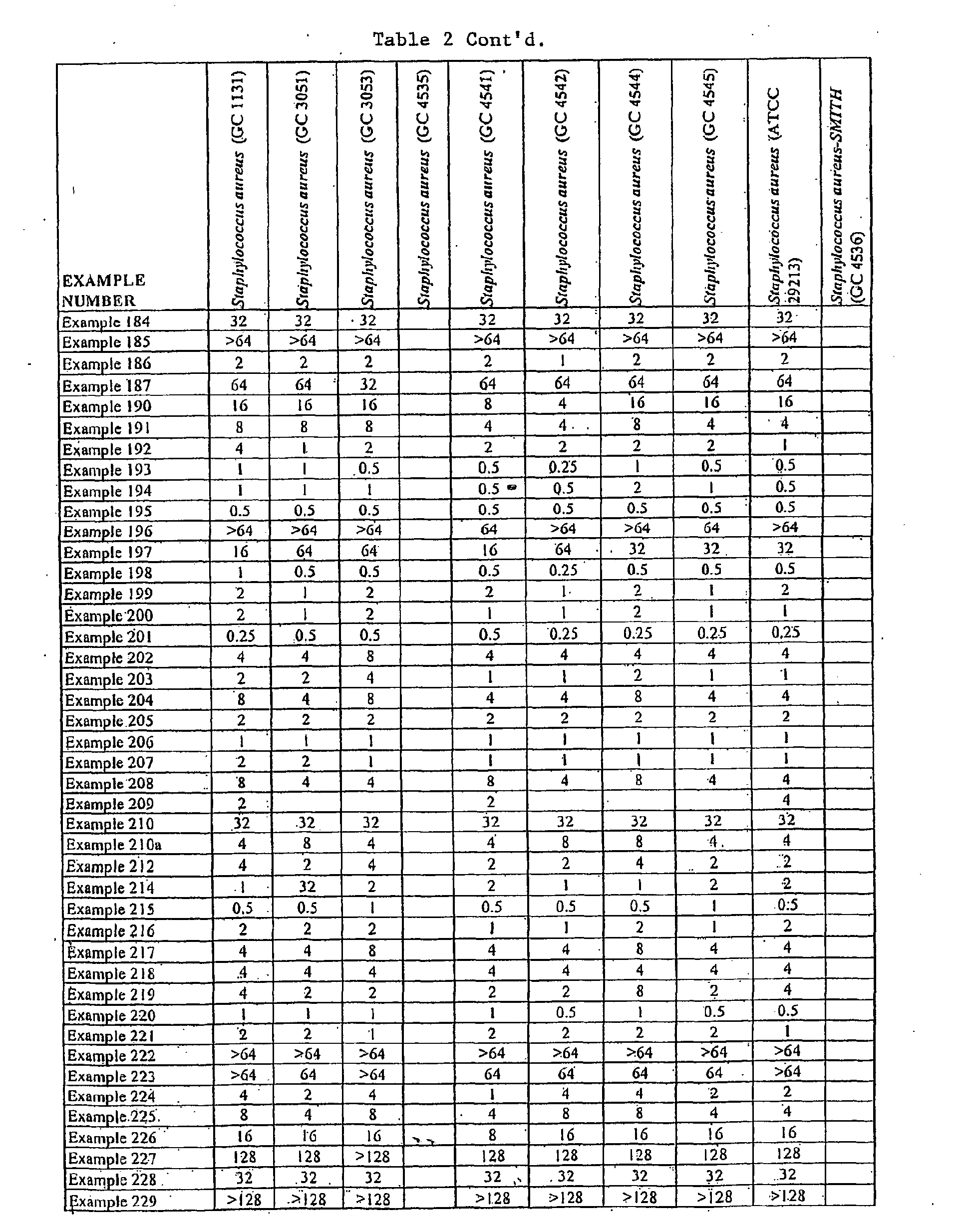

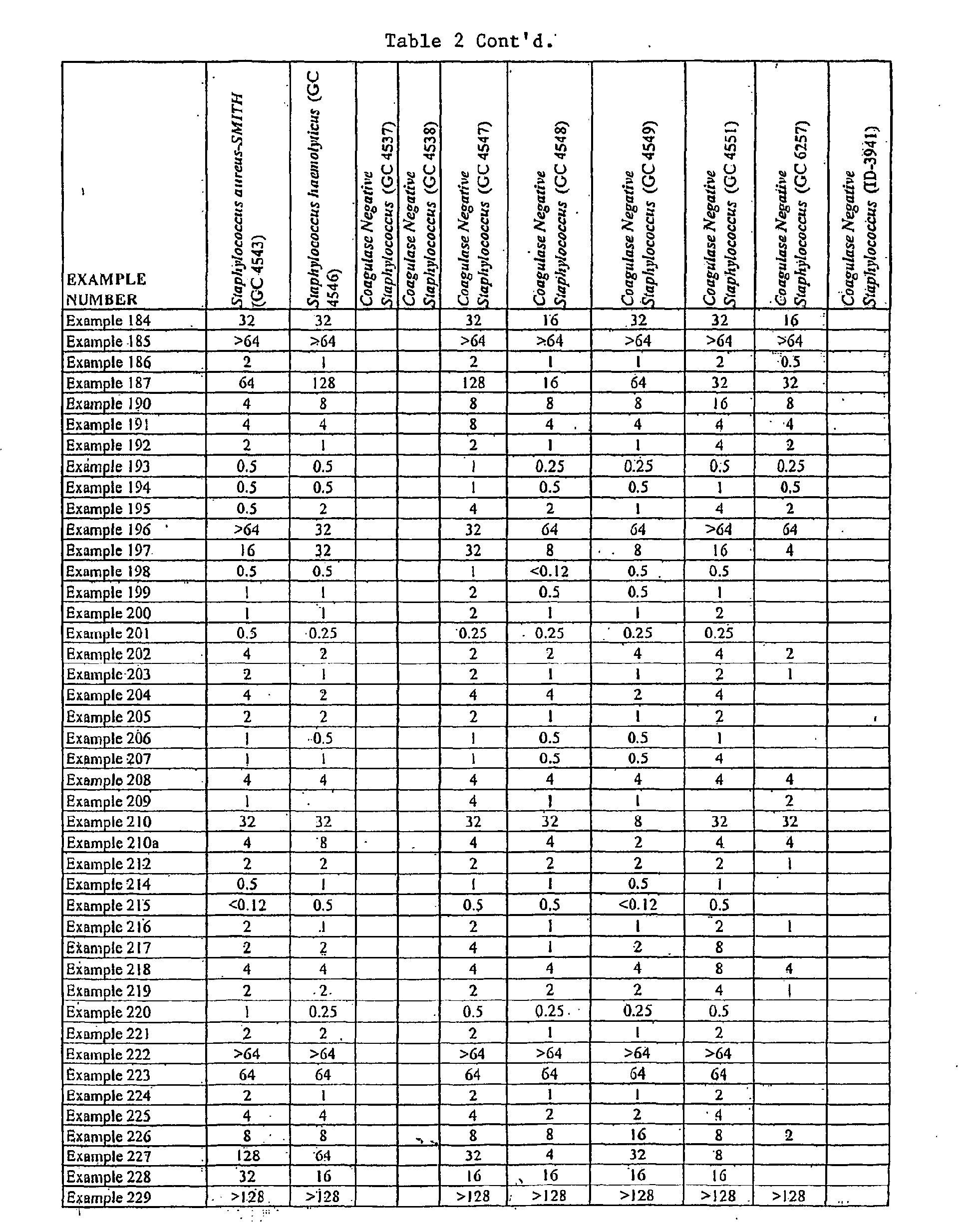

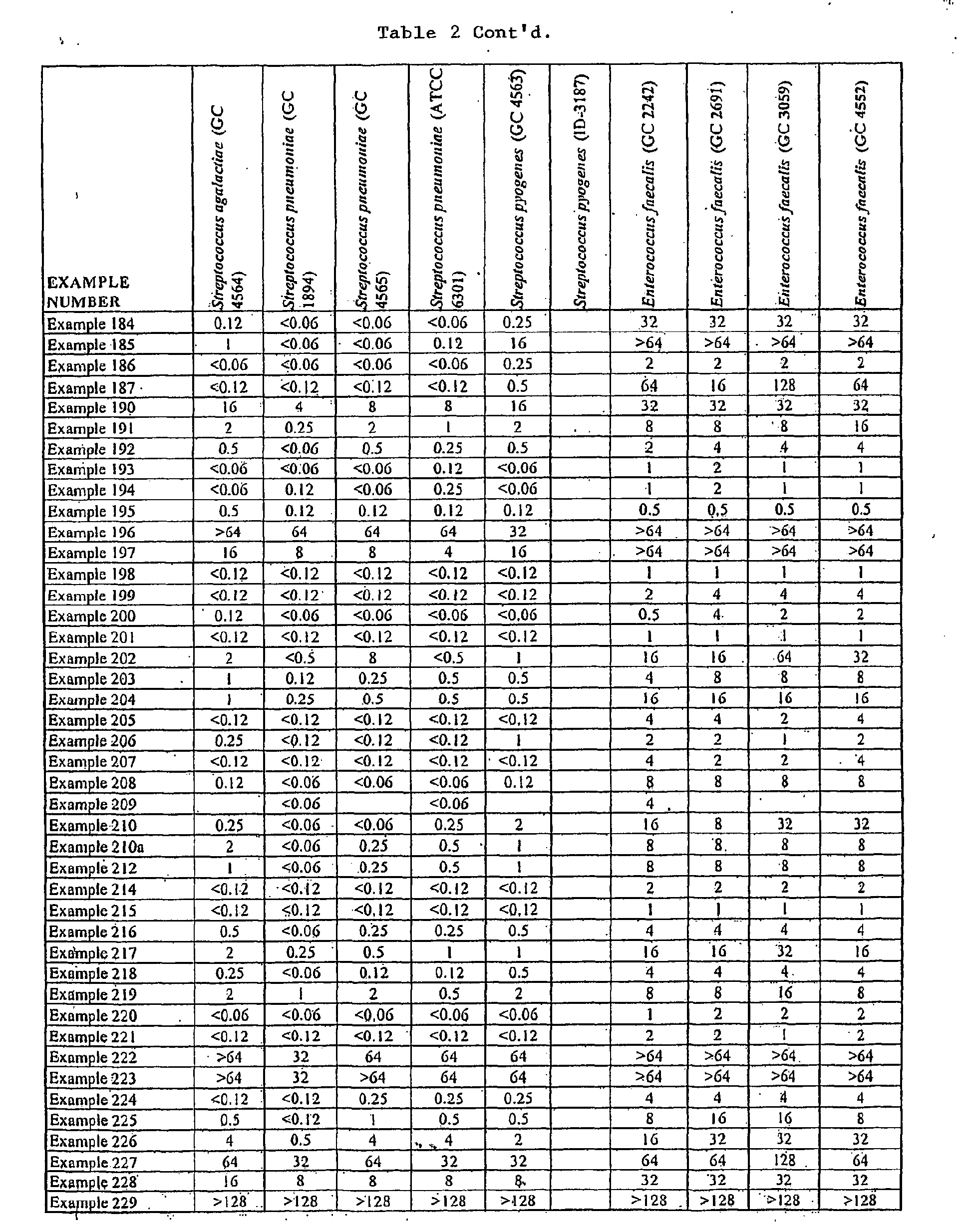

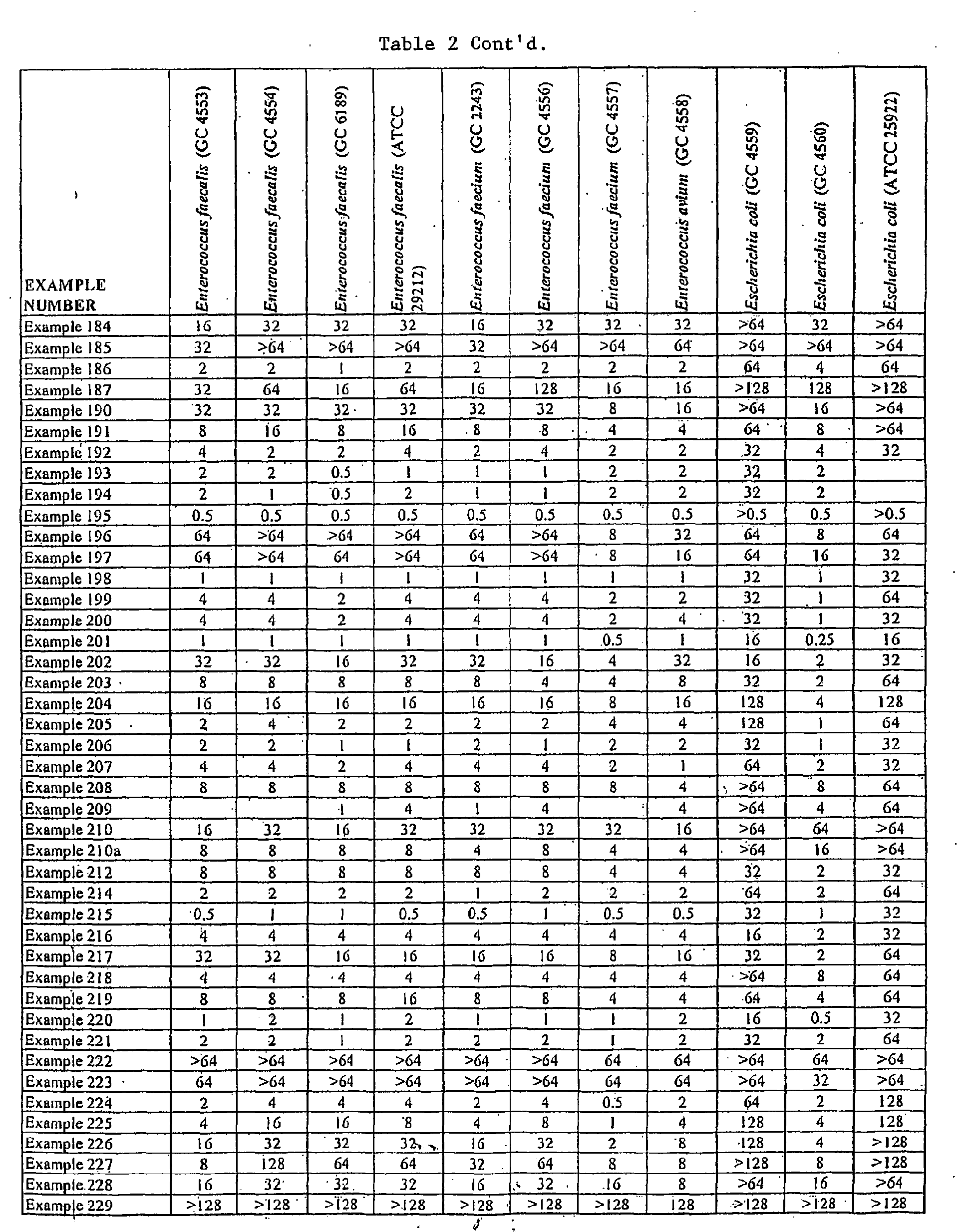

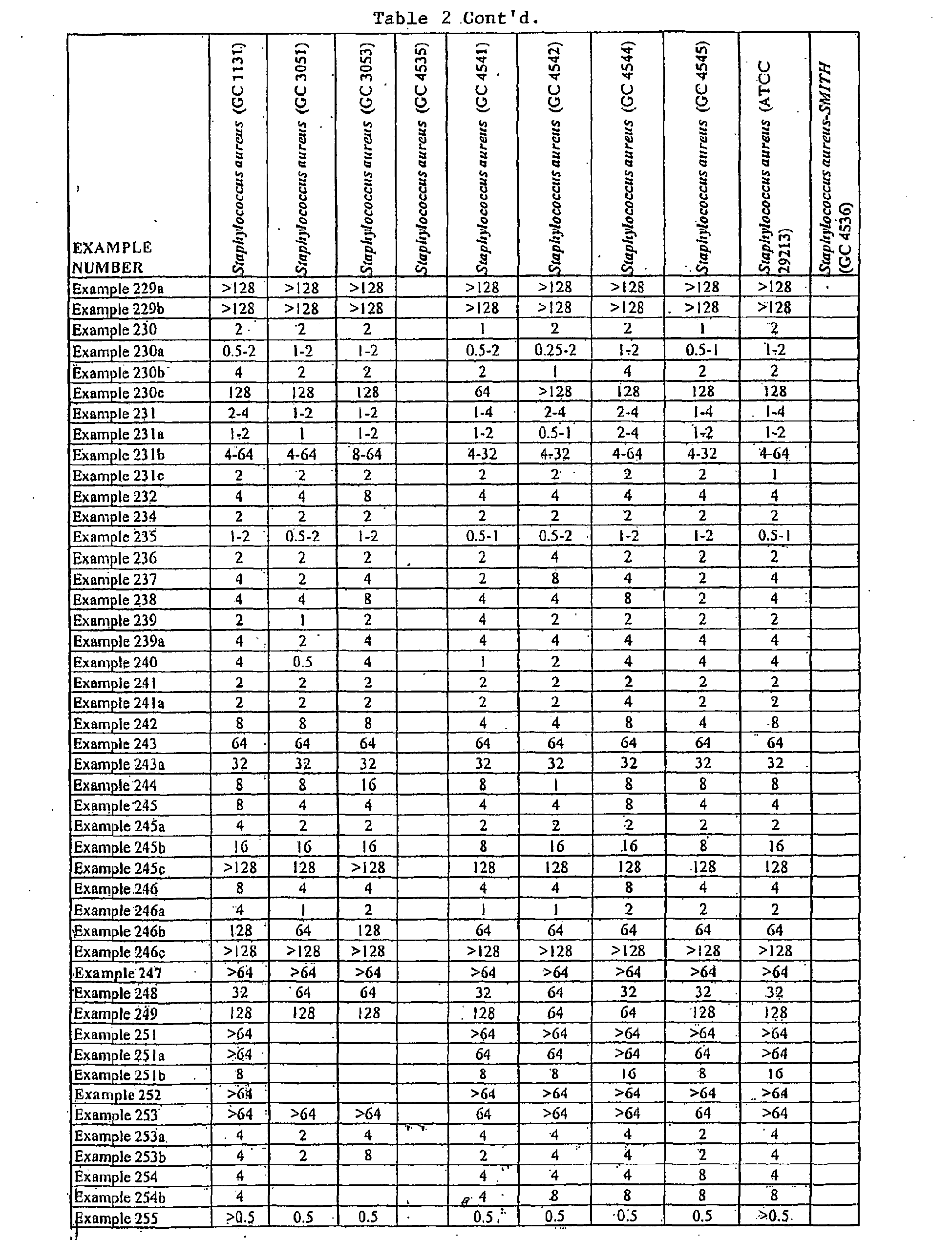

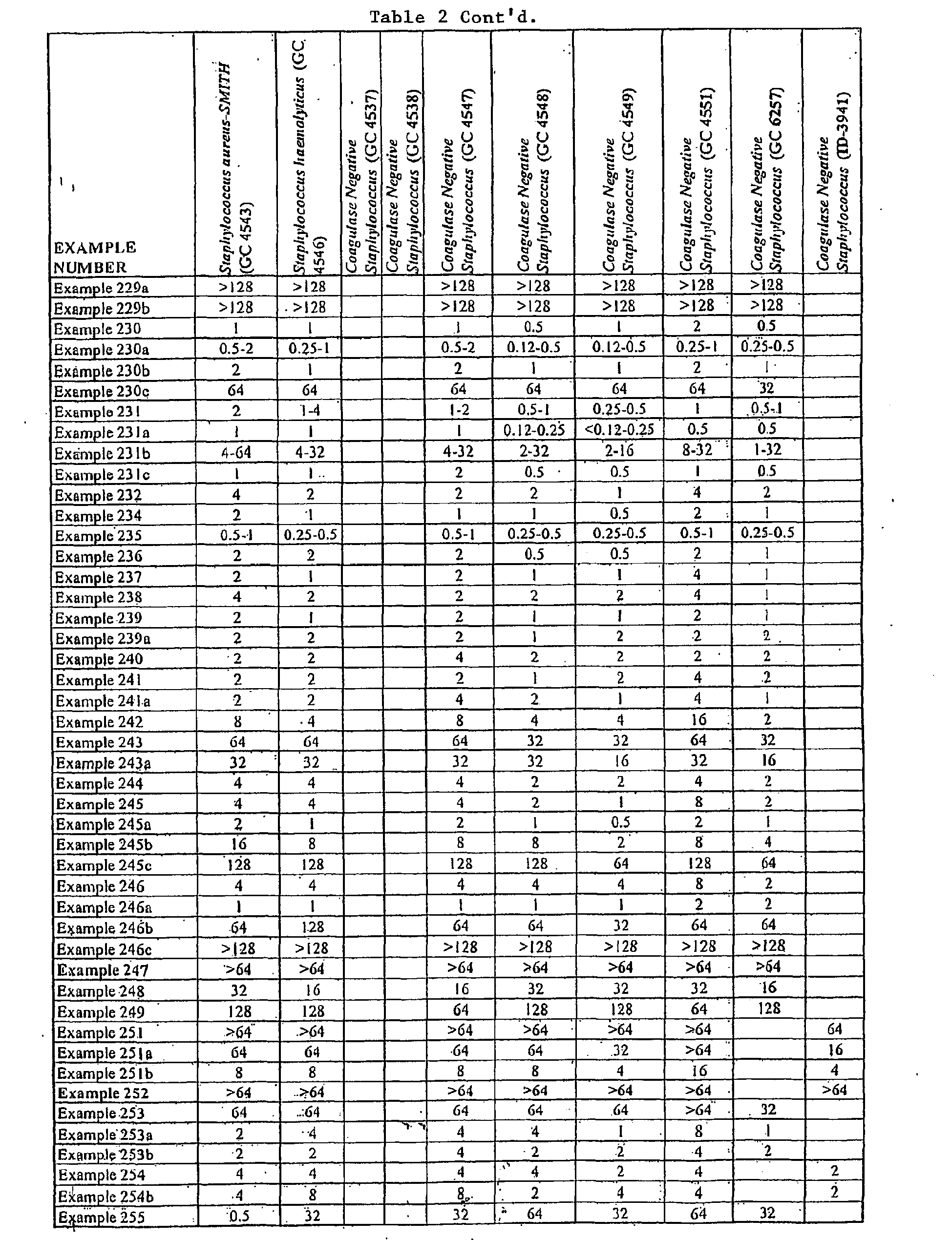

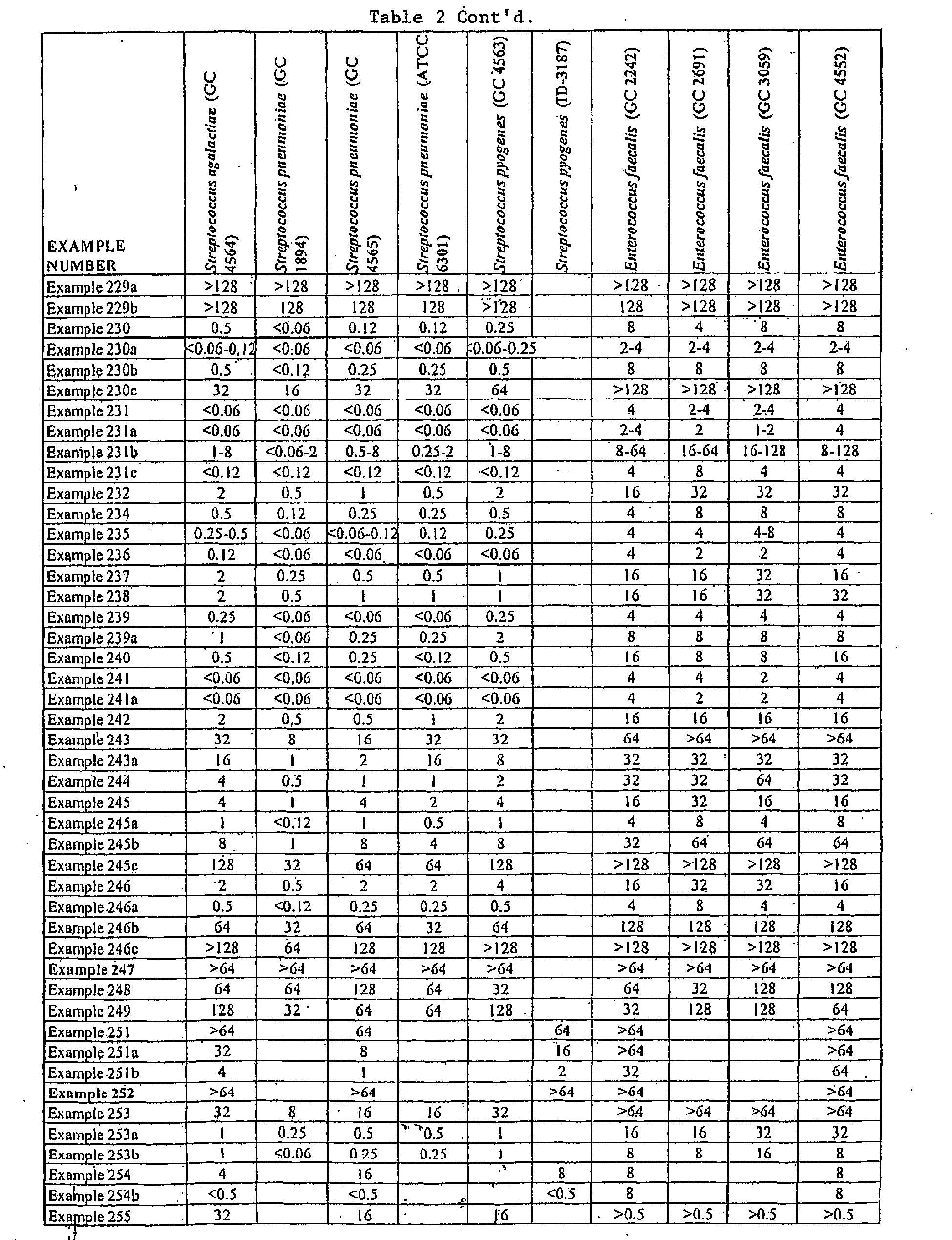

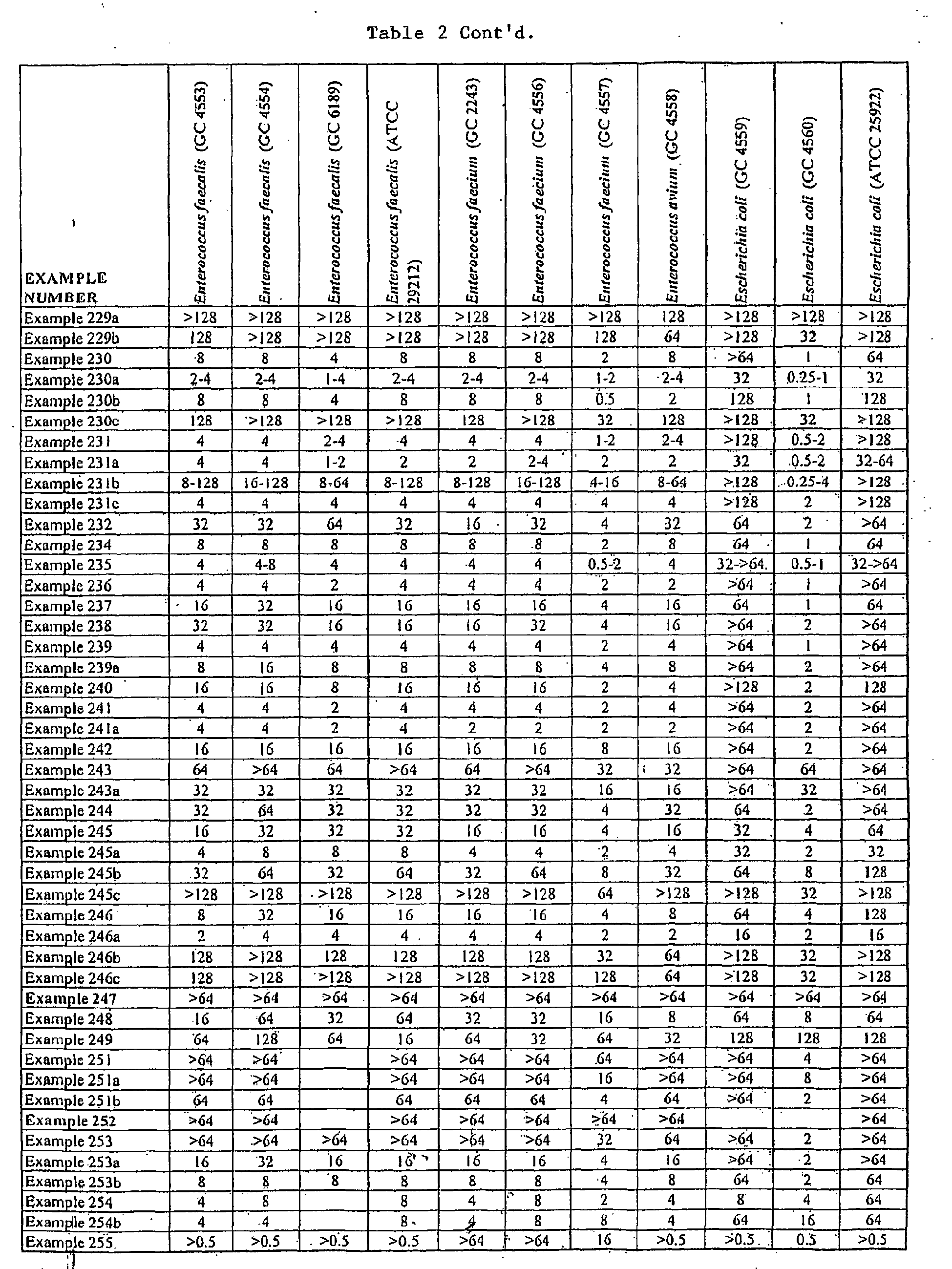

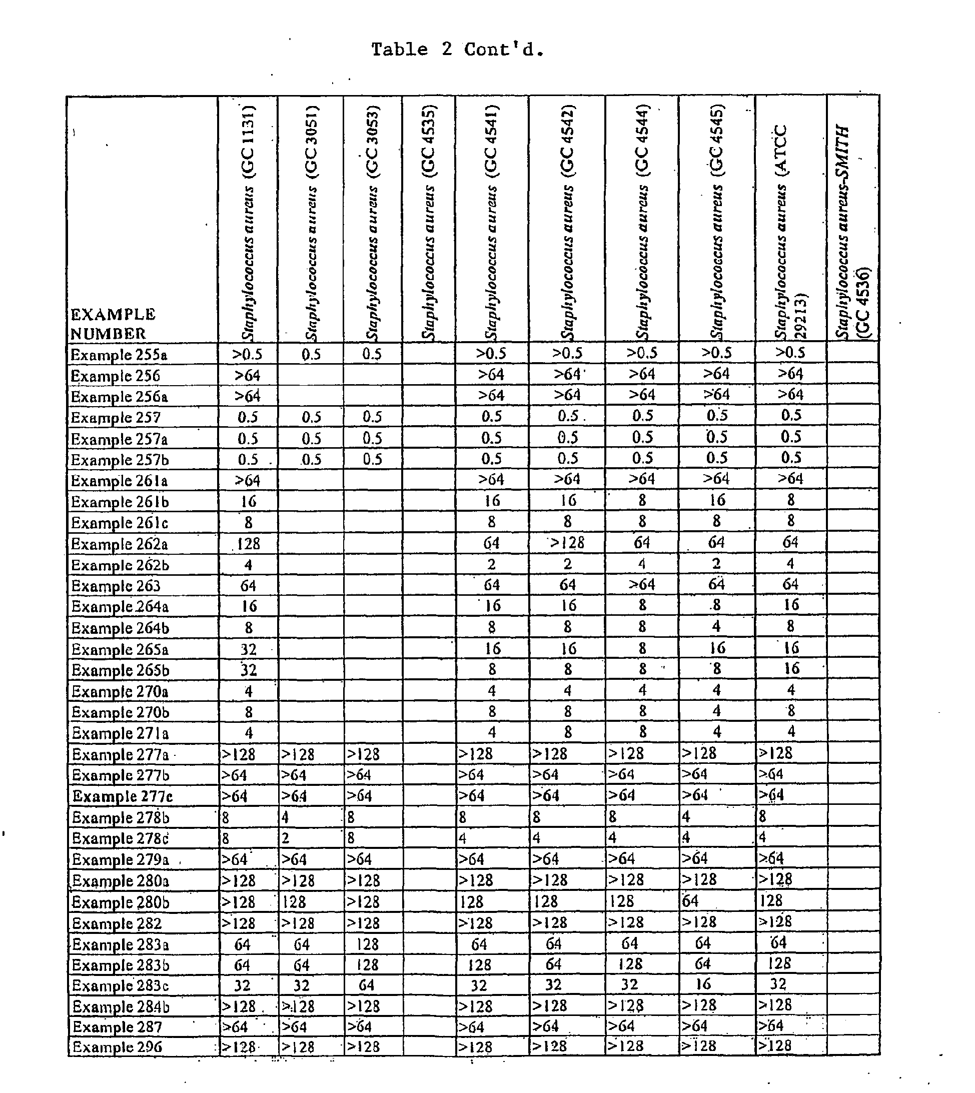

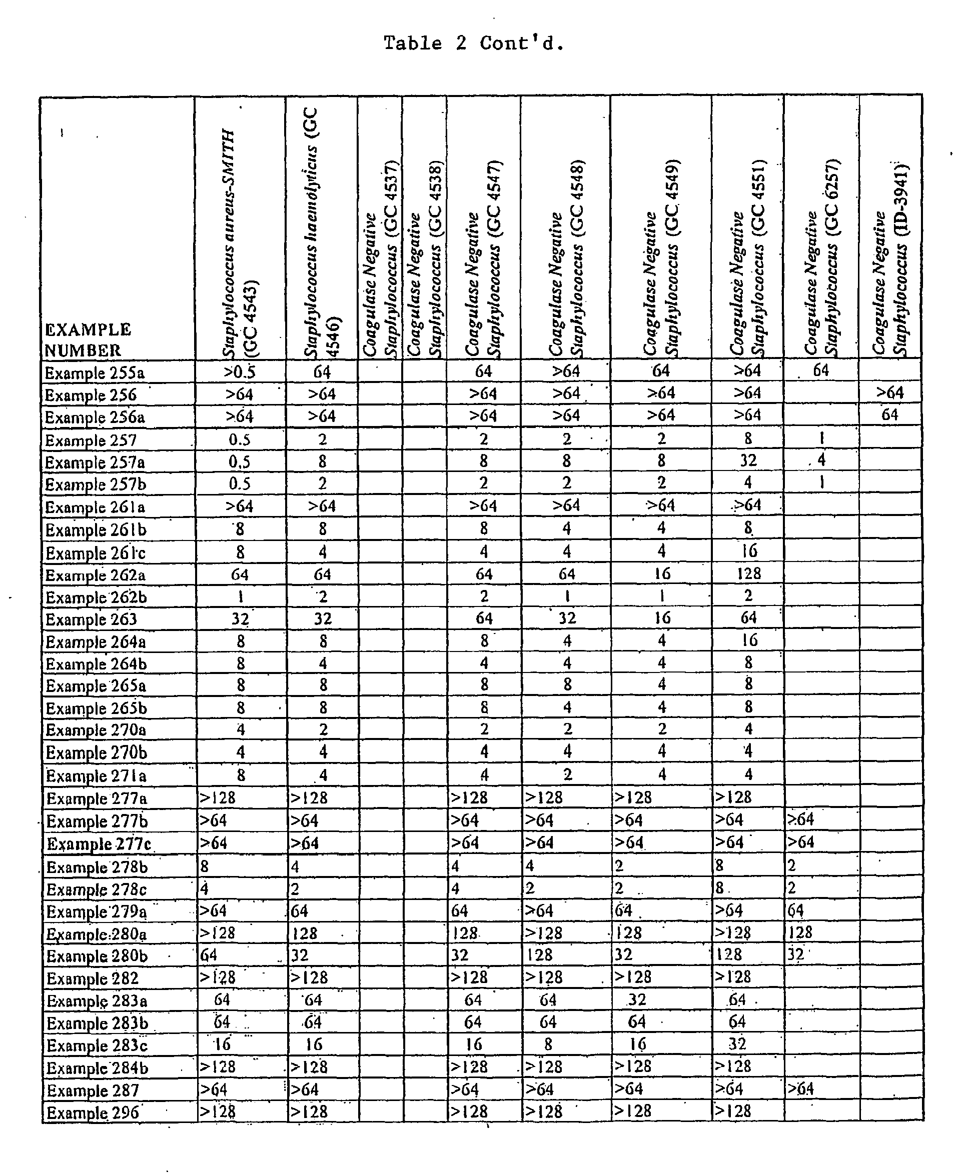

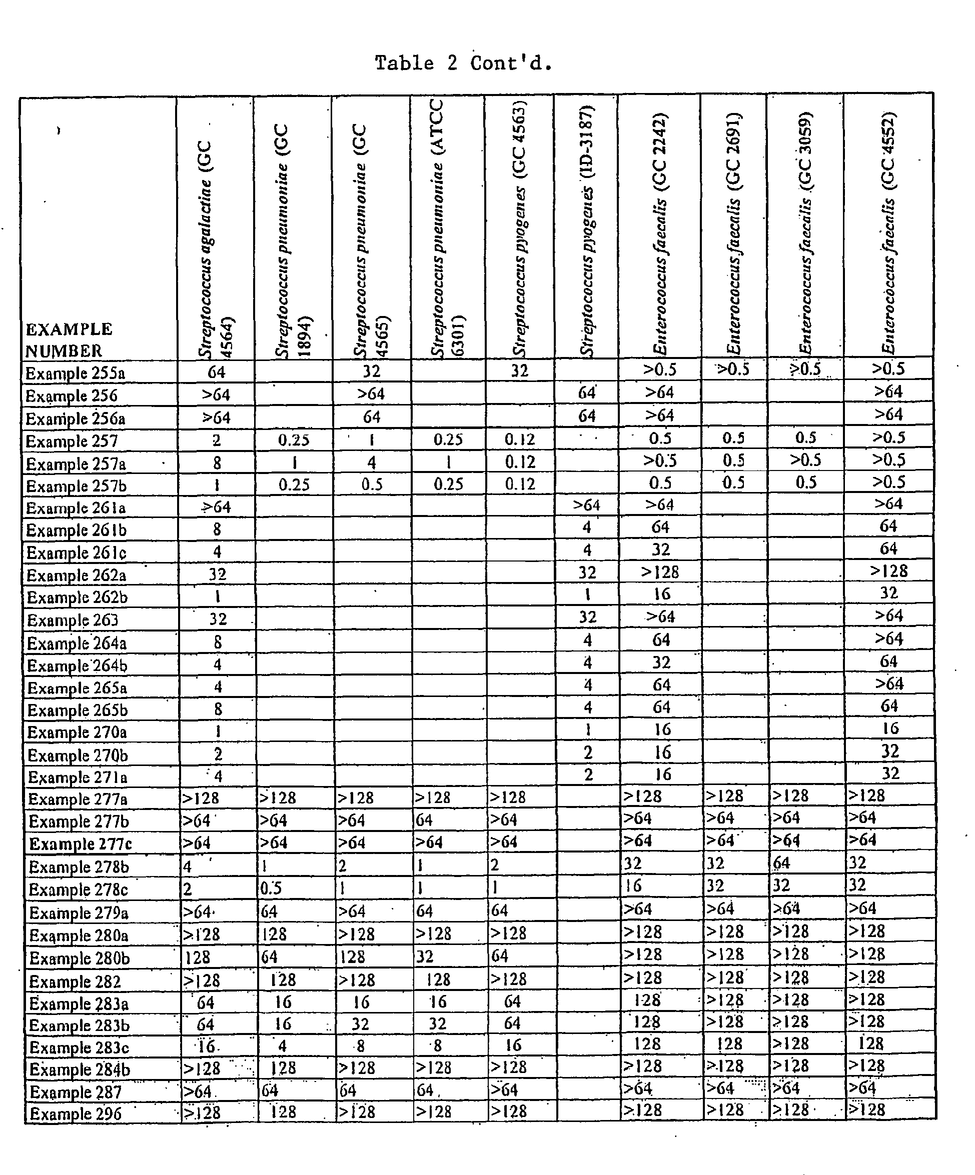

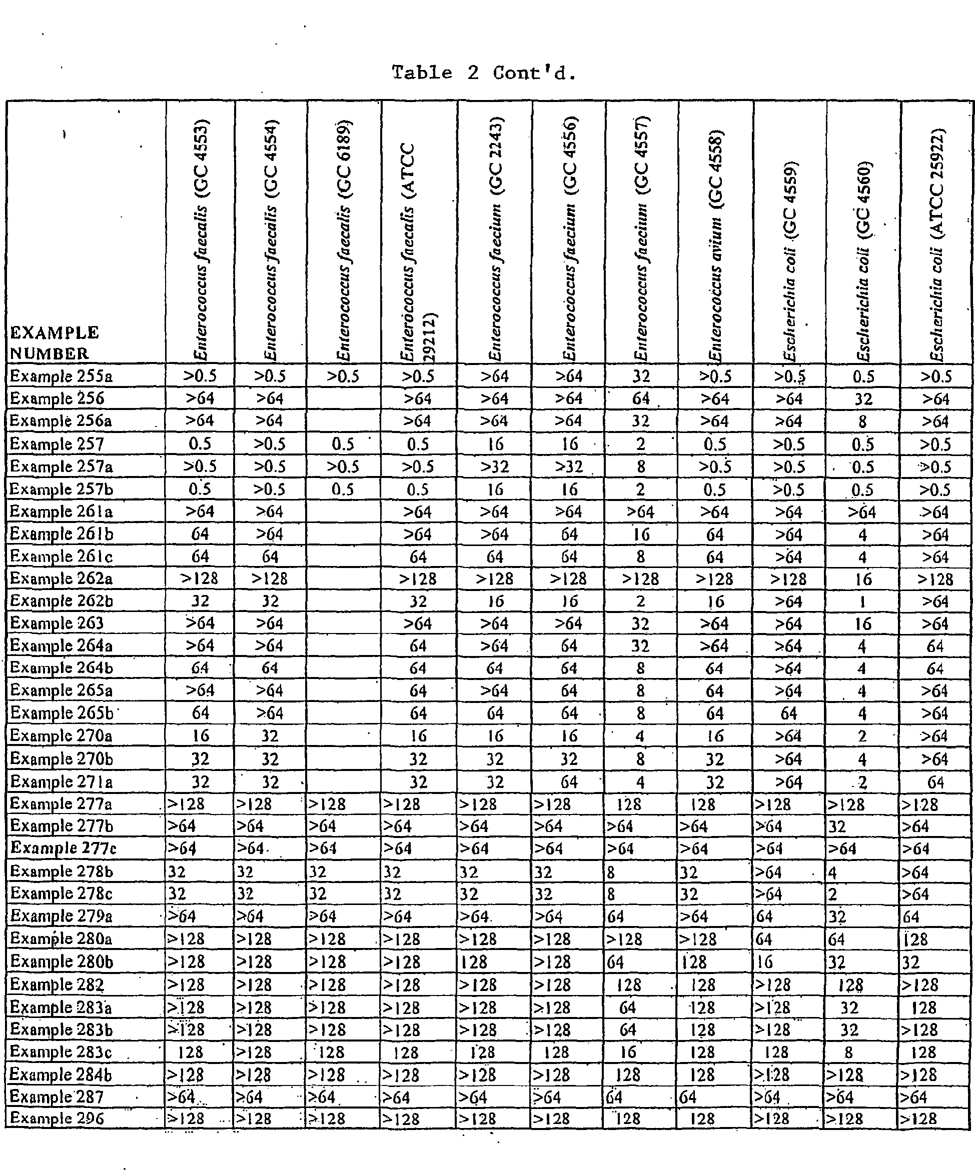

### Zone of Inhibition

### Protocol for Assay Plate Staphylococcus aureus

Inoculate 10 mL of Tryptic Soy Broth with a loopful of slant culture, *Staphylococcus aureus,* and place in a stationary rack at 37°C. After 16-18 hour incubation, the Optical Density (OD) of the culture is read @ A₆₀₀ nm, using a 1:10 dilution. OD should be between 0.1-0.3, and if not adjust with Tryptic Soy Broth. A 40 mL sample of Nutrient Agar, pH 6.8 is inoculated with 90 µl of *S. aureus* culture and poured into a sterile petri dish. When the agar has solidified, wells (5mm) are plated using an automated welling device. Wells are inoculated with 25 µl of extract and incubated overnight at 37°C. Zones of inhibition are measured and recorded the following morning. The results of representative examples of the invention are given in Table 3.

**Table 3**

| Example No. | Zone of Inhibition vs. *S. aureus*(*mm*) |
|---|---|
| 270c | 11 |
| 271c | 12 |
| 272b | 8 |
| | 8 |
| 273b | 8 |
| | 9 |
| 274a | 9 |
| 274b | 10 |

### Methods for in Vivo Antibacterial Evaluation:

The therapeutic effects of glycopeptide antibiotics of Formula I are determined against acute lethal infections with S. aureus (strain Smith), a penicillin resistant strain of S. pneumoniae (strain GC 1894), and a vancomycin resistant strain of E. fecalis (strain GC 6189 and GC 2246). Female mice, CD-1 (Charles River Laboratories), 20 +/- 2 gm, are challenged by intraperitoneal injection of sufficient bacteria (suspended in Trypticase Soy Broth or hog gastric mucin) to kill non-treated controls within 24-48 hr. Antibacterial agents, contained in 0.2 mL of saline or 5% dextrose solution, are administered intravenously 30 min. after infection. Five mice are treated at each dose level. The 7 day survival ratios from 3 separate tests are pooled for calculation of median effective dose (ED50). The results of representative examples of the invention are given in Table 4.

**Table 4**

| *In Vivo* Antibacterial Activity of Glycopeptide Antibiotics in Acute Lethal Infections in Mice | | | | |
|---|---|---|---|---|
| **EXAMPLE** | ***Enterococcus faecalis*** ***(GC 2246)*** | ***Enterococcus faecalis*** ***(GC 6189)*** | ***Staphylococcus aureus-SMITH*** ***(GC 4543)*** | ***Streptococcus pneumoni ae*** ***(GC 1894)*** |
| 28 | | | 8 | |
| 52 | | | >4 | |
| 55 | | | >8 | |
| 59 | | | >8 | |
| 60 | | | >8 | |
| 61 | | | >8 | |
| 62 | | | >8 | |
| 64 | | | >8 | |
| 65 | | | >8 | |
| 66 | | | >8 | |
| 66a | | | >4 | |
| 67 | | | >8 | |
| 69 | | | >8 | |
| 71 | | | >8 | |
| 72 | | | >8 | |
| 73 | | | >8 | |
| 74 | | | >8 | |
| 77 | | | 4 | |
| 78 | | | 2 | |
| 80 | | | >8 | |
| 81 | | | >8 | |
| 89 | | | >8 | |
| 95b | | | 4 | |
| 96c | 5.2 | | 0.29 | |
| 97b | | | 8 | |
| 99c | 4.3 | | 0.52 | |
| 102 | | 1.04 | 0.23-0.43 | |
| 103 | | | 0.28 | |
| 104 | | 0.39-0.63 | 0.19 | 0.22 |
| 105 | | 1.8-1.96 | 0.04-0.08 | 0.04 |
| 106 | | | | 0.07 |
| 111 | | 0.28 | 0.14 | 0.03 |
| 112 | | 0.19-0.28 | 0.07-0.08 | 0.02 |
| 113 | | 1.04 | 0.26 | |
| 115 | | 0.36 | 0.23 | |
| 116 | | 0.46 | 0.08-0.19 | |
| 117 | | 0.38 | 0.26-0.5 | |
| 119 | | 0.41 | 0.19 | |
| 120 | | | 0.06 | |
| 121 | | 0.5 | 0.32 | |
| 135 | | 0.26 | 0.07 | |
| 137 | | | 0.24 | |
| 141 | | | >8 | |
| 143 | | | 0.28 | |
| 154 | | | 0.1 | |
| 158 | | | 0.14 | |
| 174 | | | 0.08 | |
| 177 | | | 0.1 | |
| 180 | | | 0.05 | |
| 219 | | | 0.5 | |
| 220 | | | 0.19 | |
| 221 | | | 0.03 | |
| 230 | | | 0.12 | |
| 230a | | | 0.06 | |
| 231 | | | 0.25 | |
| 231a | | | 0.06 | |
| 235 | | | 0.59 | |
| 245 | | | 4 | |
| 245a | | | 1 | |
| 262b | | | 1.22 | |
| 263 | | | >8 | |
| 277a | | | >8 | |
| 277b | | | >8 | |
| 277c | | | >8 | |
| 280a | | | >8 | |
| 280b | | | >8 | |

### Table 5

### In Vivo Antibacterial Activity of Glycopeptide Antibiotics in Non-Lethal Thigh Infections in Mice

The therapeutic effects of glycopeptide antibiotics of the invention are also determined against a non-lethal thigh infection model in mice infected with S. aureus (strain Smith and PT 5679), a penicillin resistant strain of S. pneumoniae (strain GC 1894), a penicillin sensitive strain of S. pneumoniae (GC 6242), and a vancomycin resistant strain of E. fecalis (strain GC 6189). Female mice, CD-1 (Charles River Laboratories), 20 +/- 2 gm, are challenged by intramuscular injection of sufficient bacteria (suspended in Trypticase Soy Broth or hog gastric mucin) to cause biohazard class 1-2 (Biosafety in Microbiological and Medical Laboratories, HHS Publication NO (NIH) 88-8395, 3rd edition, 1993) infection in mice. Broth cultures of freshly plated bacteria are grown into log phase overnight to an optical density of 0.3 at 580 nm. After a 1:10 dilution into fresh broth, 0.1 mL (approximately 106 CFU) is injected intramuscularly into the thigh of each mouse. Antibacterial agents, contained in 0.2 mL of saline or 5% dextrose solution, are administered intravenously beginning 2 hr after infection. Two-fold serial dilutions of each antibiotic are administered at selected time intervals for up to 22 hr post-infection to achieve a range of drug concentrations in serum for a complete dose-response relationship from no effect to maximal effect. After 24 hr. animals are sacrificed, thighs are removed and homogenized in 10 mL of 0.85% iced saline. Duplicate aliquots are plated for serial dilutions to determine the bacterial population. Efficacy is calculated by subtracting the log₁₀CFU per thigh of untreated control mice just before therapy and at the end of therapy (24 hr) from the treated groups. The results of representative examples of the invention are given in Table 5.

**Table 5**

| *In Vivo* Antibacterial Activity of Glycopeptide Antibiotics in Mouse Thigh Infection | | | | | |
|---|---|---|---|---|---|
| **EXAMPLE NO.** | **Enterococcus faecalis (GC 6189)** | **Staphylococcus aureus (PT 5679)** | **Staphylococcus aureus-SMITH (GC 4543)** | **Streptococcus pneumoniae (GC 1894)** | **Streptococcus** **pneumoniae (GC 6242)** |
| 76 | | | 10.63 | | |
| 102 | 0.5 | | 2.03 | | |
| 104 | 1.22-1.8 | | 1.42 | | |
| 105 | 1 | 2.46 | 1.02-1.84 | | 0.53 |
| 106 | | | 0.59 | | |
| 111 | 0.48 | | 1.73-2 | | |
| 112 | 0.33-0.65 | | 1.57-1.95 | 0.13 | |
| 113 | | | 1.25 | | |
| 115 | 0.56-1.37 | | 1.22 | | |
| 116 | 1.21-1.65 | | 1.1-1.9 | | |
| 117 | 0.67 | | 2.53 | | |
| 119 | 1.39 | | 1.15 | | |
| 135 | 1.02 | | 1.27 | | |
| 137 | 1.19 | | 0.87 | | |

### Clinical pathology:

20-day-old CD-1 mice were divided into a vehicle control group (5 mice) and a treatment group (5 mice). Samples of glycopeptide antibiotics were administered at 20 mg/kg intravenously daily for 9 days. Animals were necropsied on day 10 Sera were analyzed for BUN (blood urea nitrogen), creatinine, AST (aspartic aminotransferase), and ALT (alanine aminotransferase) by routine, standard, automated methods using an Hitachi 747 chemistry analyzer. Results of representative examples of the invention are given in Table 6.

### Anatomic Pathology:

Samples of tissues from necropsied mice were formalin-fixed, paraffin-embedded, sectioned, stained with hematoxylin and eosin, and examined microscopically. Histopathologic findings were recorded. These findings were subjectively graded on a scale corresponding to slight, mild, moderate, marked and severe as compared to untreated controls.Results of representative examples of the invention are given in Table 6.

**Table 6**

| Clinical Chemistry and Anatomical Pathology Comparison in Mice | | | | | | |
|---|---|---|---|---|---|---|
| Results based on 20 mg/kg Intravenous Dose | | | | | | |
| | **Clinical Chemistry** **(Day 10) % of Control** | | | | **Anatomical** **Pathology** **(Day 10)** | |
| **Ex. No.** | **AST** | **ALT** | **BUN** | **Creatinine** | **Heart^{b}** | **Kidney** |
| 28 | No samples | | | | 0 | 0 |
| 52 | NS | NS | 211% | NS | 0 | +++ |
| 59 | NS | NS | NS | NS | 0 | 0 |
| 60 | NS | NS | NS | NS | 0 | 0 |
| 61 | NS | NS | +157% | +122% | 0 | +++ |
| 62 | NT^{*} | | | | NT* | NT* |
| 64 | NS | NS | NS | NS | 0 | 0 |
| 65 | +182% | +196% | +161% | NS | 0 | +++ |
| 66 | 147% | 182% | NS | NS | 0 | 0 |
| 66a | NS | NS | NS | NS | 0 | ++ |
| 67 | NS | NS | NS | NS | 0 | 0 |
| 69 | NS | NS | NS | NS | 0 | 0 |
| 71 | NS | NS | NS | NS | 0 | 0 |
| 72 | NS | NS | +122% | NS | 0 | ++ |
| 73 | NT^{*} | | | | NT* | NT* |
| 74 | NS | NS | NS | NS | 0 | 0 |
| 76 | NS | NS | NS | NS | 0 | ++ |
| 77 | NS | NS | NS | NS | 0 | 0 |
| 78 | NS | NS | NS | NS | 0 | ++ |
| 80 | NT* | | | | NT* | NT* |
| 81 | NS | NS | NS | NS | 0 | 0 |
| 102 | + 340% | + 672% | + 249% | + 100% | +++ | +++ |
| 104 | + 87% | NS | + 203% | + 65% | +++ | +++ |
| 105 | + 205% | + 480% | + 35% | NS | +++ | +++ |
| 106 | NS | NS | NS | NS | 0 | +/- |
| 141 | NS | NS | + 308% | + 113% | 0 | +++ |
| 219 | NS | NS | NS | NS | 0 | +++ |
| 220 | NS | NS | 149% | 121% | 0 | +++ |
| 221 | NS | NS | NS | NS | 0 | 0 |
| 230 | NS | NS | + 179% | + 124% | 0 | +++ |
| 230a | NS | NS | NS | NS | 0 | + |
| 245 | NS | NS | +160% | +138% | 0 | +++ |
| 245a | NS | NS | +212% | +133% | + | +++ |
| 263 | NS | NS | NS | +116% | + | ++ |
| 277a | NS | NS | +110% | NS | 0 | ++ |
| 277b | + 138% | + 164% | + 113% | NS | 0 | + |
| 277c | + 253% | NS | NS | NS | 0 | + |
| 280a | NS | NS | NS | NS | 0 | ++ |
| 280b | NT^{*} | | | | NT* | NT* |
| **^{a}. Clinical chemisitry**- Liver function tests included AST and ALT measurements. | | | | | | |
| - Renal function tests includes BUN and Creatinine measurements | | | | | | |
| -Degree of elevation reported as a % above untreated control animals. | | | | | | |
| **^{b}Anatomical Pathology**- Heart and kidneys were examined for microscopic histopathology | | | | | | |
| - Degree of severity and number of animals affected were scored by | | | | | | |
| 0, +, ++, or +++ as compared to untreated control animals. | | | | | | |
| NS-not significant change from controls. | | | | | | |
| NT*- animals did not survive treatment regimin. | | | | | | |

In therapeutic use, the compounds of this invention may be administered in the form of conventional pharmaceutical composition appropriate for the intended use as antibacterials. Such compositions may be formulated so as to be suitable for oral, parenteral or topical administration. The active ingredient may be combined in admixture with nontoxic pharmaceutical carrier may take a variety of forms, depending on the form of preparation desired for administration, i.e. oral, parenteral, or topical.

When the compounds are employed as antibacterials, they can be combined with one or more pharmaceutically acceptable carriers, for example, solvents, diluentsand the like, and may be administered orally in such forms as tablets, capsules, dispersible powders, granules, or suspensions containing, for example, from about 0.05 to 5% of suspending agent, syrups containing, for example, from about 10 to 50% of sugar, and elixirs containing for example, from about 20 to 50% ethanol and the like, or parenterally in the form of sterile injectable solutions or suspensions containing from about 0.05 to 5% suspending agent in an isotonic medium. Such pharmaceutical preparations may contain, for example, from about 25 to about 90% of the active ingredient in combination with the carrier, more usually between about 5% and 60% by weight.

An effective amount of compound from 0.001 mg/kg of body weight to 100.0 mg/kg of body weight should be administered one to five times per day via any typical route of administration including but not limited to oral, parenteral (including subcutaneous, intravenous, intramuscular, intrasternal injection or infusion techniques), topical or rectal, in dosage unit formulations containing conventional non-toxic pharmaceutically acceptable carriers, adjuvants and vehicles. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition of the host undergoing therapy.

Additionally, the antibacterially effective amount of the glycopeptide antibiotics of the invention may be administered at a dosage and frequency without inducing side effects commonly experienced with conventional antibiotic therapy which could include hypersensitivity, neuromuscular blockade, vertigo, photosensitivity, discoloration of teeth, hematologic changes, gastrointestinal disturbances, ototoxicity, and renal, hepatic, or cardiac impairment. Further the frequency and duration of dosage may be monitored to substantially limit harmful effects to normal tissues caused by administration at or above the antibacterially effective amount of the glycopeptide antibiotics of the invention.

These active compounds may be administered orally as well as by intravenous, intramuscular, or subcutaneous routes. Solid carriers include starch, lactose, dicalcium phosphate, microcrystalline cellulose, sucrose and kaolin, while liquid carriers include sterile water, polyethylene glycols, non-ionic surfactants and edible oils such as corn, peanut and sesame oils, as are appropriate to the nature of the active ingredient and the particular form of administration desired. Adjuvants customarily employed in the preparation of pharmaceutical compositions may be advantageously included, such as flavoring agents, coloring agents, preserving agents, and antioxidants, for example, vitamin E, ascorbic acid, BHT and BHA. These active compounds may also be administered parenterally or intraperitoneally. Solutions or suspensions of these active compounds as a free base or pharmacologically acceptable salt can be prepared in glycerol, liquid, polyethylene glycols and mixtures thereof in oils. Under ordinary conditions of storage and use,these preparations contain a preservative. The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacterial and fungi. The carrier can be a solvent or dispersion medium containing, for example, water, ethanol, polyol (e.g., glycerol, propylene glycol and liquid polyethylene glycol), suitable mixtures thereof, and vegetable oil.

The invention accordingly provides a pharmaceutical composition which comprises a compound of this invention in combination or association with a pharmaceutically acceptable carrier. In particular, the present invention provides a pharmaceutical composition which comprises an antibacterially effective amount of a compound of this invention and a pharmaceutically acceptable carrier.

The present invention further provides a method of treating bacterial infections in warm-blooded animals including man, which comprises administering to the afflicted warm-blooded animals an antibacterially effective amount of a compound or a pharmaceutical composition of a compound of the invention. The invention will be more fully described in conjunction with the following specific examples which are not to be construed as limiting the scope of the invention.

The following examples illustrate the preparation of the compounds of the invention by fermentation and synthetic procedures and as such are not to be considered as limiting the invention set forth in the claims appended hereto.

### Example 1

### (Cyclo[glycyl-β-methylphenylalanyl-3-bromo-O-(4-O-hexopyranosylhexopyranosyl)tyrosyl-3-(2-iminoimidazolidin-4-yl)seryl-3-(3-hexopyranosyl-2-iminoimidazolidin-4-yl)serlyseryl])

A stirred solution of the bis-trifluroacetate salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (152 mg) in trifluoroacetic acid (4 mL) is treated with N-bromosuccinimide (23 mg) and the reaction mixture is then stirred at room temperature for 2 h. Volatiles are then removed *in vacuo* and the resulting residue is triturated with ether, the solid collected and washed with ethyl acetate and then diethyl ether to give the crude monobromo-derivative of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]. The product is then purified by reverse phase HPLC. MS (+ES), *m*/*z*: 687 (M+2H)²⁺.

### Example 2

### (Cyclo[glycyl-β-methylphenylalanyl-3-bromotyrosyl-3-(2-iminoimidazolidin-4-yl)seryl-3-(3-hexopyranoxyl-2-iminoimidazolidin-4-yl)serylseryl])

A stirred solution the bis-hydrochloride salt of cyclo[glycyl-β-methylphenylalanyltyrosyl-3-(2-iminoimidazolidin-4-yl)seryl-3-(3-hexopyranosyl-2-iminoimidazolidin-4-yl)serylseryl] (21 mg) in trifluoroacetic acid (0.5 mL) is treated with 3 mg of bromine in 0.3 mL of acetic acid and the reaction is stirred at room temperature for 45 min, then poured into diethyl ether (8 mL). The resulting solid is collected by filtration and washed with diethyl ether to give 18 mg of the crude monobromo-derivative of cyclo[glycyl-β-methylphenylalanyltyrosyl-3-(2-iminoimidazolidin-4-yl)seryl-3-(3-hexopyranosyl-2-iminoimidazolidin-4-yl)serylseryl]. The product is then purified by reverse phase HPLC. MS (+ES), *m*/*z*: 525.1 (M+2H)²⁺.

### Example 3

### (Cyclo[glycyl-β-methylphenylalanyl-3,5-dibromotyrosyl-3-(2-iminoimidazolidin-4-yl)seryl-3-(3-hexopyranosyl-2-iminoimidazolidin-4-yl)serylseryl])

A stirred solution of the bis-hydrochloride salt of cyclo[glycyl-β-methylphenylalanyltyrosyl-3-(2-iminoimidazolidin-4-yl)seryl-3-(3-hexopyranosyl-2-iminoimidazolidin-4-yl)serylseryl] (31 mg) in trifluoroacetic acid (1 mL) is treated with N-bromosuccinimide (10 mg) and the reaction mixture is then stirred at room temperature for 2 h. Volatiles are then removed *in vacuo* and the resulting residue is triturated with ether, the solid collected and washed with ethyl acetate and then diethyl ether to give the crude dibromo-derivative of cyclo[glycyl-β-methylphenylalanyltyrosyl-3-(2-iminoimidazolidin-4-yl)seryl-3-(3-hexopyranosyl-2-iminoimidazolidin-4-yl)serylseryl]. The product is then purified by reverse phase HPLC. MS (+ES), *m*/*z*: 565 (M+2H)²⁺.

### Example 4

### (Cyclo[glycyl-β-methylphenylalanyl-3,5-dibromotyrosyl-3-(2-iminoimidazolidin-4-yl)seryl-3-(2-iminoimidazolidin-4-yl)serylseryl])

The title compound is prepared from cyclo[glycyl-β-methylphenylalanyltyrosyl-3-(2-iminoimidazolidin-4-yl)seryl-3-(2-iminoimidazolidin-4-yl)serylseryl] by the procedure described for example 3. MS (+ES), *m*/*z*: 484 (M+2H)²⁺.

### Example 5

### (Cyclo[glycyl-β-methylphenylalanyl-3-iodotyrosyl-3-(2-iminoimidazolidin-4-yl)seryl-3-(3-hexopyranosyl-2-iminoimidazolidin-4-yl)serylseryl])

A stirred solution of the bis-hydrochloride salt of cyclo[glycyl-β-methylphenylalanyltyrosyl-3-(2-iminoimidazolidin-4-yl)seryl-3-(3-hexopyranosyl-2-iminoimidazolidin-4-yl)serylseryl] (31 mg) in trifluoroacetic acid (1 mL) is treated with N-iodosuccinimide (12 mg) and the reaction mixture is then stirred at 4-8°C for 2 h. Volatiles are then removed *in vacuo* and the resulting residue is triturated with ether, the solid is collected by filtration and washed with ethyl acetate and then diethyl ether to give the crude monoiodo-derivative of cyclo[glycyl-β-methylphenylalanyltyrosyl-3-(2-iminoimidazolidin-4-yl)seryl-3-(3-hexopyranosyl-2-iminoimidazolidin-4-yl)serylseryl]. The product is then purified by reverse phase HPLC. MS (+ES), *m*/*z*: 549 (M+2H)²⁺.

### Example 6

### (Cyclo[glycyl-β-methylphenylalanyl-O-(4-O-hexopyranosylhexopyranosyl)-3-iodo-tyrosyl-3-(2-iminoimidazolidin-4-yl)seryl-3-(3-hexopyranosyl-2-iminoimidazolidin-4-yl)serylseryl])

The title compound is prepared by the procedure described for example 5 using the bis-trifluroacetate salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]. MS (+ES), *m*/*z*: 711.3 (M+2H)²⁺.

### Example 7

### (Cyclo[glycyl-β-methylphenylalanyl-O-(4-O-hexopyranosylhexopyranosyl)-3,5-diiodo-tyrosyl-3-(2-iminoimidazolidin-4-yl)seryl-3-(3-hexopyranosyl-2-iminoimidazolidin-4-yl)serylseryl])

A stirred solution of the bis-trifluroacetate salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (304 mg) in trifluoroacetic acid (10 mL) is treated with N-iodosuccinimide (99 mg) and the reaction mixture is then stirred at 4-8°C for 2 h. Volatiles are then removed *in vacuo* and the resulting residue is triturated with ether, the solid collected and washed with ethyl acetate and then diethyl ether to give the crude diiodo-derivative of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]. The product is then purified by reverse phase HPLC. MS (+ES), *m*/*z*: 774 (M+2H)²⁺.

### Example 8

### (Cyclo[glycyl-β-methylphenylalanyl-3-nitrotyrosyl-3-(2-iminoimidazolidin-4-yl)seryl-3-(3-hexopyranosyl-2-iminoimidazolidin-4-yl)serylscryl])

A stirred solution of the bis-hydrochloride salt of cyclo[glycyl-β-methylphenylalanyltyrosyl-3-(2-iminoimidazolidin-4-yl)seryl-3-(3-hexopyranosyl-2-iminoimidazolidin-4-yl)serylseryl] (4.62 g) in trifluoroacetic acid (10 mL) at 3°C is treated with potassium nitrate (492 mg) and the reaction mixture is then stirred with cooling in an ice bath for 45 min. Volatiles are then removed *in vacuo* and the resulting residue is triturated with ethanol to provide an amber solid, which is then collected by filtration and washed with ethanol and then diethyl ether to give the crude mono-nitro derivative of cyclo[glycyl-β-methylphenylalanyltyrosyl-3-(2-iminoimidazolidin-4-yl)seryl-3-(3-hexopyranosyl-2-iminoimidazolidin-4-yl)serylseryl]. The product is then purified by reverse phase HPLC. MS (+ES), *m*/*z*: 508 (M+2H)²⁺.

### Example 9

### (Cyclo[glycyl-β-methylphenylalanyl-3-nitrotyrosyl-3-(2-iminoimidazolidin-4-yl)seryl-3-(2-iminoimidazolidin-4-yl)serlylseryl])

The title compound is prepared from cyclo[glycyl-β-methylphenylalanyltyrosyl-3-(2-iminoimidazolidin-4-yl)seryl-3-(2-iminoimidazolidin-4-yl)serylseryl] by the procedure described for example 8. MS (+ES), *m*/*z*: 427.8 (M+2H)²⁺.

### Example 10

### (Cyclo[3-(2-iminoimidazolidin-4-yl)alanyl-3-(3-hexopyranosyl-2-iminoimidazolidin-4-yl)-alanylserylglycyl-β-methylphenylalanyl-3-nitrotyrosyl])

The title compound is prepared from cyclo[3-(2-iminoimidazolidin-4-yl)alanyl-3-(3-hexopyranosyl-2-iminoimidazolidin-4-yl)-alanylserylglycyl-β-methylphenylalanyltyrosyl] by the procedure described for example 8. MS (+ES), *m*/*z*: 492.9 (M+2H)²⁺.

### Example 11

### (Cyclo[glycyl-β-methylphenylalanyl-3-aminotyrosyl-3-(2-iminoimidazolidin-4-yl)seryl-3-(3-hexopyranosyl-2-iminoimidazolidin-4-yl)serylseryl])

A stirred solution of the crude bis-trifluroacetate salt of cyclo[glycyl-β-methylphenylalanyl-3-nitrotyrosyl-3-(2-iminoimidazolidin-4-yl)seryl-3-(3-hexopyranosyl-2-iminoimidazolidin-4-yl)serylseryl] (16 g) in 15% aqueous acetic acid (160 mL) and ethanol (10 mL) is hydrogenated under balloon pressure at room temperature over 10% palladium on carbon (1.2 g) for 3.5 h. The catalyst is then removed by filtration through diatomaceous earth, the filtrate is concentrated to a volume of ∼70 mL, then added to acetonitrile (450 mL) and the precipitated product is collected by filtration. The precipitate is then washed with acetonitrile and diethyl ether and air dried to give the desired product. MS (+ES), *m*/*z*: 493.9 (M+2H)²⁺.

### Example 12

### (Cyclo[glycyl-β-methylphenylalanyl-3-(dimethylamino)tyrosyl-3-(2-iminoimidazolidin-4-yl)-seryl-3-(3-hexopyranosyl-2-iminoimidazolidin-4-yl)serylseryl])

A stirred solution of the bis-trifluroacetate salt of cyclo[glycyl-β-methylphenylalanyl-3-aminotyrosyl-3-(2-iminoimidazolidin-4-yl)seryl-3-(3-hexopyranosyl-2-iminoimidazolidin-4-yl)serylseryl] (100 mg) in ethanol (10 mL) is treated with glacial acetic acid (1.5 mL) and aqueous formaldehyde (37%, 1 mL), and is then hydrogenated under balloon pressure at room temperature over 10% palladium on carbon for 1 hour. The catalyst is removed by filtration through diatomaceous earth, and the filtrate is concentrated to a volume of ∼4 mL. The concentrated filtrate is then added to acetonitrile (15 mL) and diethyl ether (30 mL) and the precipitated product is collected by filtration. The precipitate is then washed with acetonitrile and diethyl ether and air dried to give the desired product. MS (+ES), *m*/*z*: 507.8 (M+2H)²⁺.

### Example 13

### (Cyclo[glycyl-β-methylphenylalanyl-3-acetamidotyrosyl-3-(2-iminoimidazolidin-4-yl)seryl-3-(3-hexopyranosyl-2-iminoimidazolodin-4-yl)serylseryl])

A stirred solution of the bis-trifluroacetate salt of cyclo[glycyl-β-methylphenylalanyl-3-aminotyrosyl-3-(2-iminoimidazolidin-4-yl)seryl-3-(3-hexopyranosyl-2-iminoimidazolidin-4-yl)serylseryl] (100 mg) in neat acetic anhydride (0.2 mL) is stirred at room temperature for 45 min. Acetic anhydride is removed under a stream of nitrogen and the residue is triturated with diethyl ether. The resulting precipitate is collected by filtration, washed, and air dried. The product is then purified by reverse phase HPLC. MS (+ES), *m*/*z*: 515 (M+2H)²⁺.

### Example 14

### (Cyclo[glycyl-β-methylphenylalanyl-3-(propanamido)tyrosyl-3-(2-iminoimidazolidin-4-yl)seryl-3-(3-hexopyranosyl-2-iminoimidazolidin-4-yl)serylseryl])

A stirred solution of the bis-trifluroacetate salt of cyclo[glycyl-β-methylphenylalanyl-3-aminotyrosyl-3-(2-iminoimidazolidin-4-yl)seryl-3-(3-hexopyranosyl-2-iminoimidazolidin-4-yl)serylseryl] (100 mg) in tetrahydrofuran (0.2 mL) is treated with propionic anhydride (2 mL) and the reaction mixture is stirred at room temperature for 75 min. The reaction mixture is then triturated with acetonitrile and diethyl ether, and the resulting precipitate is collected by filtration, washed with diethyl ether and air dried. MS (+ES), *m*/*z*: 522.2 (M+2H)²⁺.

### Example 15

### (Cyclo[glycyl-β-methylphenylalanyl-3-(2-methylpropanamido)tyrosyl-3-(2-iminoimidazolidin-4-yl)seryl-3-(3-hexopyranosyl-2-iminoimidazolidin-4-yl)serylseryl])

The title compound is prepared by the procedure described for example 14 using isobutyric anhydride to give the corresponding isobutyryl amide derivative. MS (+ES), *m*/*z*: 528.9 (M+2H)²⁺.

### Example 16

### (Cyclo[glycyl-β-methylphenylalanyl-3-(heptanamido)tyrosyl-3-(2-iminoimidazolidin-4-yl)seryl-3-(3-hexopyranosyl-2-iminoimidazolidin-4-yl)serylseryl])

The title compound is prepared by the procedure described for example 14 using heptanoic anhydride to give the corresponding heptyl amide derivative. MS (+ES), *m*/*z*: 550.2 (M+2H)²⁺.

### Example 17

### (Cyclo[glycyl-β-methylphenylalanyl-3-(benzamido)tyrosyl-3-(2-iminoimidazolidin-4-yl)seryl-3-(3-hexopyranosyl-2-iminoimidazolidin-4-yl)serylseryl])

A stirred solution of the bis-trifluroacetate salt of cyclo[glycyl-β-methylphenylalanyl-3-aminotyrosyl-3-(2-iminoimidazolidin-4-yl)seryl-3-(3-hexopyranosyl-2-iminoimidazolidin-4-yl)serylseryl] (250 mg) in tetrahydrofuran (3 mL) is treated with benzoyl chloride (0.3 mL) and the reaction mixture is then stirred at room temperature for 45 min. Solvent is removed under a stream of nitrogen and the residue is triturated with diethyl ether. The resulting precipitate is collected by filtration, washed with diethyl ether and air dried. The product is then purified by reverse phase HPLC. MS (+ES), *m*/*z*: 545.9 (M+2H)²⁺.

### Example 18

### (Cyclo[glycyl-β-methylphenylalanyl-3-formamidotyrosyl-3-(2-iminoimidazolidin-4-yl)seryl-3-(3-hexopyranoxyl-2-iminoimidazolidin-4-yl)serylseryl])

A stirred suspension of the bis-trifluroacetate salt of cyclo[glycyl-β-methylphenylalanyl-3-aminotyrosyl-3-(2-iminoimidazolidin-4-yl)seryl-3-(3-hexopyranosyl-2-iminoimidazolidin-4-yl)serylseryl] (180 mg) in 88% formic acid (2.5 mL) at 0°C is treated with acetic anhydride (0.25 mL) and the reaction mixture is then stirred for 45 min. The reaction mixture is then diluted with diethyl ether and the resulting precipitate is collected by filtration, washed with diethyl ether and air dried. The product is then purified by reverse phase HPLC. MS (+ES), *m*/*z*: 508.5 (M+2H)²⁺.

### Example 19

### (Cyclo[glycyl-β-methylphenylalanyl-3-[[(4-methylphenoxy)carbonyl]amino]tyrosyl-3-(2-iminoimidazolidin-4-yl)seryl-3-(3-hexopyranosyl-2-iminoimidazolidin-4-yl)serylseryl])

A stirred suspension of the bis-trifluroacetate salt of cyclo[glycyl-β-methylphenylalanyl-3-aminotyrosyl-3-(2-iminoimidazolidin-4-yl)seryl-3-(3-hexopyranosyl-2-iminoimidazolidin-4-yl)serylseryl] (61 mg) in glacial acetic acid (5 mL) is treated dropwise with a solution of p-tolyl chloroformate (8.5 mg) in glacial acetic acid (0.5 mL) and the reaction mixture is then stirred at 70°C for 2 h. The reaction mixture is then diluted with diethyl ether and acetonitrile and the resulting precipitate is collected by filtration, washed with diethyl ether and air dried. The product is then purified by reverse phase HPLC. MS (+ES), *m*/*z*: 561 (M+2H)²⁺.

### Example 20

### (Cyclo[glycyl-β-methylphenylalanyl-3-[(methoxycarbonyl)amino]tyrosyl-3-(2-iminoimidazolidin-4-yl)seryl-3-(3-hexopyranosyl-2-iminoimidazolidin-4-yl)serylseryl])

The title compound is prepared by the procedure described for example 19 using methyl chloroformate to form the corresponding urethane derivative. MS (+ES), *m*/*z*: 523.5 (M+2H)²⁺.

### Example 21

### (Cyclo[glycyl-β-methylphenylalanyl-3-[(phenylmethoxycarbonyl)amino]tyrosyl-3-(2-iminoimidazolidin-4-yl)seryl-3-(3-hexopyranosyl-2-iminoimidazolidin-4-yl)serylseryl])

The title compound is prepared by the procedure described for example 19 using benzyl chloroformate to form the corresponding urethane derivative. MS (+ES), *m*/*z*; 561 (M+2H)²⁺.

### Example 22

### (Cyclo[3-(2,3-dihydro-2-oxo-1,3-benzoxazol-5-yl)alanyl-3-(2-iminoimidazolidin-4-yl)seryl-3-(3-hexopyranosyl-2-iminoimidazolidin-4-yl)serylserylglycyl-β-methylphenylalanyl])

A stirred suspension of the bis-trifluroacetate salt of cyclo[glycyl-β-methylphenylalanyl-3-aminotyrosyl-3-(2-iminoimidazolidin-4-yl)seryl-3-(3-hexopyranosyl-2-iminoimidazolidin-4-yl)serylseryl] (106 mg) in glacial acetic acid (9.5 mL) is treated dropwise with a solution of 4-chlorophenyl chloroformate (17 mg) in glacial acetic acid (0.5 mL) and the reaction mixture is then stirred at 70°C for 3 h. The reaction mixture is then diluted with diethyl ether and the resulting precipitate is collected by filtration, washed with diethyl ether and air dried. The resulting solid (71 mg) is dissolved in 0.1N NaOH (2 mL) and stirred at room temperature for 1 h. The solution is then diluted with acetonitrile and the resulting precipitate is collected by filtration, washed with acetonitrile then diethyl ether and air dried. The product is then purified by reverse phase HPLC. MS (+ES), *m*/*z*: 507 (M+2H)²⁺.

### Example 23

### (Cyclo[3-(2,3-dihydro-2-thio-1,3-benzoxazol-5-yl)alanyl-3-(2-iminoimidazolidin-4-yl)seryl-3-(3-hexopyranosyl-2-iminoimidazolidin-4-yl)serylserylslycyl-β-methylphenylalanyl])

A stirred solution of cyclo[glycyl-β-methylphenylalanyl-3-aminotyrosyl-3-(2-iminoimidazolidin-4-yl)seryl-3-(3-hexopyranosyl-2-iminoimidazolidin-4-yl)serylseryl] (177 mg) in N,N-dimethylformamide (3 mL) is treated with 1,1'-thiocarbonyldiimidazole (32 mg) and the mixture is stirred at room temperature for 1 h. The reaction mixture is then diluted with diethyl ether and the resulting precipitate is collected by filtration, washed with diethyl ether and air dried. The product is then purified by reverse phase HPLC. MS (+ES), *m*/*z*: 515 (M+2H)²⁺.

### Example 24

### (Cyclo[glycyl-β-methylphenylalanyl-3-[[[3,5-bis(trifluoromethyl)phenyl]carbamothioyl]-amino]-tyrosyl-3-(2-iminoimidazolidin-4-yl)seryl-3-(3-hexopyranosyl-2-iminoimidazolidin-4-yl)-serylseryl])

A stirred suspension of the bis-trifluroacetate salt of cyclo[glycyl-β-methylphenylalanyl-3-aminotyrosyl-3-(2-iminoimidazolidin-4-yl)seryl-3-(3-hexopyranosyl-2-iminoimidazolidin-4-yl)serylseryl] (135 mg) in THF (0.5 mL) is treated with 3,5-ditrifluoromethylphenyl isothiocyanate (0.6 mL) and the mixture is stirred at room temperature for 3 days. The reaction mixture is then diluted with diethyl ether and the resulting precipitate is collected by filtration, washed with diethyl ether and air dried. The product is then purified by reverse phase HPLC. MS (+ES), *m*/*z*: 629.2 (M+2H)²⁺.

### Example 25

### (Cyclo[3-[2-(4-carboxyphenyl)-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenylalanyl])

A stirred suspension of the bis-trifluroacetate salt of cyclo[glycyl-β-methylphenylalanyl-3-aminotyrosyl-3-(2-iminoimidazolidin-4-yl)seryl-3-(3-hexopyranosyl-2-iminoimidazolidin-4-yl)serylseryl] (121 mg) in N,N-dimethylformamide (5 mL) at room temperature is treated with 4-carboxybenzaldehyde (75 mg) and 2,3-dichloro-5,6-dicyano-1,4-benzoquinone (57 mg), and the reaction mixture is stirred for 2 h. The reaction mixture is then poured into a mixture of diethyl ether and acetonitrile, and the resulting solid is collected by filtration, washed with diethyl ether and air dried. The product is then purified by reverse phase HPLC. MS (+ES), *m*/*z*: 558.7 (M+2H)²⁺.

### Example 26

### (Cyclo[3-[2-(3-nitrophenyl)-1,3-benzoxazol-5-yl)alanyl-3-(2-iminoimidazolidin-4-yl)seryl-3-(3-hexopyranosyl-2-iminoimidazolidin-4-yl)serylserylglycyl-β-methylphenylalanyl])

The title compound is prepared by the procedure described for example 25 using the appropriate aldehyde to form the corresponding benzoxazole derivative. MS (+ES), *m*/*z*: 559.5 (M+2H)²⁺.

### Example 27

### (Cyclo[3-[2-(4-bromophenyl)-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenylalanyl])

The title compound is prepared by the procedure described for example 25 using the appropriate aldehyde to form the corresponding benzoxazole derivative. MS (+ES), *m*/*z*: 576.6 (M+2H)²⁺.

### Example 28

### (Cyclo[3-[2-[3-(4-methylphenoxy)phenyl]-1,3-benzoxazol-5-yl]alanyl-3-(2-iminoimidazolidin-4-yl)seryl-3-(3-hexopyranosyl-2-iminoimidazolidin-4-yl)serylserylglycyl-6-methylphenyl-alanyl])

The title compound is prepared by the procedure described for example 25 using the appropriate aldehyde to form the corresponding benzoxazole derivative. MS (+ES), *m*/*z*: 590.2 (M+2H)²⁺.

### Example 29

### (Cyclo[3-[2-[4-(dimethylamino)pheny]-1,3-benzoxazol-5-yl]alanyl-3-(2-iminoimidazolidin-4-yl)-seryl-3-(3-hexopyranosyl-2-iminoimidazolidin-4-yl)serylserylglycyl-β-methylphenyl-alanyl])

The title compound is prepared by the procedure described for example 25 using the appropriate aldehyde to form the corresponding benzoxazole derivative. MS (+ES), *m*/*z*: 558.6 (M+2H)²⁺.

### Example 30

### (Cyclo[3-[2-(3-fluorophenyl)-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenylalanyl]).

The title compound is prepared by the procedure described for example 25 using the appropriate aldehyde to form the corresponding benzoxazole derivative. MS (+ES), *m*/*z*: 545.8 (M+2H)²⁺.

### Example 31

### (Cyclo[3-[2-[4-(phenylmethoxy)phenyl]-1,3-benzoxazol-5-yl]alanyl-3-(2-iminoimidazolidin-4-yl)seryl-3-(3-hexopyranosyl-2-iminoimidazolidin-4-yl)serylserylglycyl-β-methyl-phenylalanyl])

The title compound is prepared by the procedure described for example 25 using the appropriate aldehyde to form the corresponding benzoxazole derivative. MS (+ES), *m*/*z*: 589.9 (M+2H)²⁺.

### Example 32

### (Cyclo[3-[2-(4-tert-butylphenyl)-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenylalanyl])

The title compound is prepared by the procedure described for example 25 using the appropriate aldehyde to form the corresponding benzoxazole derivative. MS (+ES), *m*/*z*: 565 (M+2H)²⁺.

### Example 33

### (Cyclo[3-[2-([1,1-biphenyl]-4-yl)-1,3-benzoxazol-5-yl]alanyl-3-(2-iminoimidazolidin-4-yl)-seryl-3-(3-hexopyranosyl-2-iminoimidazolidin-4-yl)serylserylalycyl-β-methylphenylalanyl])

The title compound is prepared by the procedure described for example 25 using the appropriate aldehyde to form the corresponding benzoxazole derivative. MS (+ES), *m*/*z*: 574.9 (M+2H)²⁺.

### Example 34

### (Cyclo[3-[2-(3,4,5-trimethoxyphenyl)-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenyl-alanyl])

The title compound is prepared by the procedure described for example 25 using the appropriate aldehyde to form the corresponding benzoxazole derivative. MS (+ES), *m*/*z*: 581.9 (M+2H)²⁺.

### Example 35

### (Cyclo[3-[2-[3-(4-methoxyphenoxy)phenyl]-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenyl-alanyl])

The title compound is prepared by the procedure described for example 25 using the appropriate aldehyde to form the corresponding benzoxazole derivative. MS (+ES), *m*/*z*: 597.9 (M+2H)²⁺.

### Example 36

### (Cyclo[3-[2-[2-(hexopyranosloxy)phenyl]-1,3-benzoxazol-5-yl]alanyl-3-(2-iminoimidazolidih-4-yl)seryl-3-(3-hexopyranosyl-2-iminoimidazolidin-4-yl)serylserylglycyl-β-methyl-phenylalanyl])

The title compound is prepared by the procedure described for example 25 using the appropriate aldehyde to form the corresponding benzoxazole derivative. MS (+ES), *m*/*z*: 625.6 (M+2H)²⁺.

### Example 37

### (Cyclo[3-[2-(9H-fluoren-2-yl)-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenylalanyl])

The title compound is prepared by the procedure described for example 25 using the appropriate aldehyde to form the corresponding benzoxazole derivative. MS (+ES), *m*/*z*: 581.6 (M+2H)²⁺.

### Example 38

### (Cyclo[3-[2-(3-furyl)-1,3-benzoxazol-5-y]alanyl-3-(2-iminoimidazolidin-4-yl)seryl-3-(3-hexopyranosyl-2-iminoimidazolidin-4-yl)serylserylglycyl-β-methylphenylalanyl])

The title compound is prepared by the procedure described for example 25 using the appropriate aldehyde to form the corresponding benzoxazole derivative. MS (+ES), *m*/*z*: 531.9 (M+2H)²⁺.

### Example 39

### (Cyclo[3-[2-(2,2-diphenylethenyl)-1,3-benzoxazol-5-yl]alanyl-3-(2-iminoimidazolidin-4-yl)seryl-3-(3-hexopyranosyl-2-iminoimidazolidin-4-yl)serylserylglycyl-6-methylphenylalanyl])

The title compound is prepared by the procedure described for example 25 using the appropriate aldehyde to form the corresponding benzoxazole derivative. MS (+ES), *m*/*z*: 588.1 (M+2H)²⁺.

### Example 40

### (Cyclo[3-[2-(2-methylprop-1-en-1-yl)-1,3-benzoxazol-5-yl]alanyl-3-(2-iminoimidazolidin-4-yl)seryl-3-(3-hexopyranosyl-2-iminoimidazolidin-4-yl)serylserylalycyl-β-methylphenyl-alanyl])

The title compound is prepared by the procedure described for example 25 using the appropriate aldehyde to form the corresponding benzoxazole derivative. MS (+ES), *m*/*z*: 526.1 (M+2H)²⁺.

### Example 41

### (Cyclo[3-[2-[4-(3-methylphenoxy)phenyl]benzoxazol-5-yl]alanyl-3-(2-iminoimidazolidin-4-yl)-seryl-3-(2-iminoimidazolidin-4-yl)serylserylglyeyl-β-methylphenylalanyl])

The title compound is prepared from cyclo[glycyl-β-methylphenylalanyltyrosyl-3-(2-iminoimidazolidin-4-yl)seryl-3-(2-iminoimidazolidin-4-yl)serylseryl] by the procedure described for example 25 using the appropriate aldehyde to form the corresponding benzoxazole derivative. MS (+ES), *m*/*z*: 508.7 (M+2H)²⁺.

### Example 42

### (Cyclo[3-[2-(9H-fluoren-2-yl)-1,3-benzoxazol-5-yl]alanyl-3-(2-iminoimidazolidin-4-yl)alanyl-3-(3-hexopyranosyl-2-iminoimidazolidin-4-yl)alanylserylglycyl-β-methylphenylalanyl])

The title compound is prepared from cyclo[3-(2-iminoimidazolidin-4-yl)alanyl-3-(3-hexopyranosyl-2-iminoimidazolidin-4-yl)-alanylserylglycyl-β-methylphenylalanyltyrosyl] by the procedure described for example 25 using the appropriate aldehyde to form the corresponding benzoxazole derivative. MS (+ES), *m*/*z*: 564.9 (M+2H)²⁺.

### Example 43

### (Cyclo[3-[2-(6-methoxynaphth-2-yl)-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenylalanyl])

The title compound is prepared from cyclo[3-(2-iminoimidazolidin-4-yl)alanyl-3-(3-hexopyranosyl-2-iminoimidazolidin-4-yl)-alanylserylglycyl-β-methylphenylalanyltyrosyl] by the procedure described for example 25 using the appropriate aldehyde to form the corresponding benzoxazole derivative. MS (+ES), *m*/*z*: 577.2 (M+2H)²⁺.

### Example 44

### (Cyclo[3-[[2-(2,3 4,6-tetra-O-benzoylhexopyranosyl)amino]-1,3-benzoxazol-5-yl]alanyl-3-(2-iminoimidazolidin-4-yl)seryl-3-(3-hexopyranosyl-2-iminoimidazolidin-4-yl)seryl-serylglycyl-β-methylphenylalanyl])

A suspension of the bis-trifluoroacetate salt of cyclo[glycyl-β-methylphenylalanyl-3-aminotyrosyl-3-(2-iminoimidazolidin-4-yl)seryl-3-(3-hexopyranosyl-2-iminoimidazolidin-4-yl)serylseryl] (300 mg) in THF (5 mL) is treated with 2,3,4,6-tetra-O-benzoyl-β-D-glucopyranosyl isothiocyanate (300 mg) and the mixture is stirred at room temperature for 11 days. The reaction mixture is then diluted with diethyl ether and the resulting precipitate is collected by filtration, washed with diethyl ether and air dried. The resulting solid is then dissolved in methanol (5 mL) and treated with mercuric chloride (350 mg) and the reaction mixture is stirred at room temperature for 18 h. Methanol is then evaporated under a gentle stream of nitrogen and the title compound is then isolated by reverse phase HPLC. MS (+ES), *m*/*z*: 795.9 (M+2H)²⁺.

### Example 45

### (Cyclo[3-[2-(benzylthio)-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenylalanyl])

A stirred solution of the bis-trifluoroacetate salt of cyclo[3-(2,3-dihydro-2-thio-1,3-benzoxazol-5-yl)alanyl-3-(2-iminoimidazolidin-4-yl)seryl-3-(3-hexopyranosyl-2-iminoimidazolidin-4-yl)serylserylglycyl-β-methylphenylalanyl] (100 mg) in N,N-dimethylformamide (0.6 mL) is treated with benzyl bromide (40 mg) and N,N-diisopropylethylamine (40 uL), and the mixture is stirred at room temperature for 30 min. The reaction mixture is then diluted with diethyl ether and the resulting precipitate is collected by filtration, washed with diethyl ether and air dried. The product is then purified by reverse phase HPLC. MS (+ES), *m*/*z*: 560 (M+2H)²⁺.

### Example 46

### (Cyclo[3-[2-[(naphthylmethyl)thio]-1,3-benzoxazol-5-yl]alanyl-3-(2-iminoimidazolidin-4-yl)-seryl-3-(3-hexopyranosyl-2-iminoimidazolidin-4-yl)serylserylglycyl-β-methylphenylalanyl])

The title compound is prepared by the procedure described for example 45 using 2-(bromomethyl)naphthalene to form the corresponding 2-thiobenzoxazole derivative. MS (+ES), *m*/*z*: 584.8 (M+2H)²⁺.

### Example 47

### (Cyclo[3-[2-[(4-phenylbenzyl)thio]-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenylalanyl])

The title compound is prepared by the procedure described for example 45 using 4-phenylbenzyl chloride to form the corresponding 2-thiobenzoxazole derivative. MS (+ES), *m*/*z*: 598 (M+2H)²⁺.

### Example 48

### (Cyclo[3-[2-[(2-oxo-2-phenylethyl)thio]-1,3-benzoxazol-5-yl]alanyl-3-(2-iminoimidazolidin-4-yl)-seryl-3-(3-hexopyranosyl-2-iminoimidazolidin-4-yl)serylserylglycyl-β-methylphenyl-alanyl])

A stirred solution of the bis-trifluoroacetate salt of cyclo[3-(2,3-dihydro-2-thio-1,3-benzoxazol-5-yl)alanyl-3-(2-iminoimidazolidin-4-yl)seryl-3-(3-hexopyranosyl-2-iminoimidazolidin-4-yl)serylserylglycyl-β-methylphenylalanyl] (100 mg) in N,N-dimethylformamide (3 mL) is treated with 2-bromo-acetophenone (200 mg), and the mixture is stirred at room temperature for 2 h. The reaction mixture is then diluted with diethyl ether and the resulting precipitate is collected by filtration, washed with diethyl ether and air dried. The product is then purified by reverse phase HPLC. MS (+ES), *m*/*z*: 573.7 (M+2H)²⁺.

### Example 49

### (Cyclo[3-[2-[[2-(4-chlorophenyl)-2-oxoethyl]thio]-1,3-benzoxazol-5-yl]alanyl-3-(2-iminoimidazolidin-4-yl)seryl-3-(3-hexopyranosyl-2-iminoimidazolidin-4-yl)serylserylglycyl-β-methylphenylalanyl])

The title compound is prepared by the procedure described for example 48 using 2-bromo-4'-chloroacetophenone to form the corresponding 2-thiobenzoxazole derivative. MS (+ES), *m*/*z*: 590.7 (M+2H)²⁺.

### Example 50

### (Cyclo[3-cyclohexylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylserylglycyl])

A stirred solution of the bis-hydrochloride salt of cyclo[glycylphenylalanyl-O-(4-O-hexopyranosylhexopyranosyl)tyrosyl-3-(2-iminomidazolidin-4-yl)seryl-3-(3-hexopyranosyl-2-iminoimidazolidin-4-yl)serylseryl] (10 g) in 10% aqueous acetic acid (300 mL) containing n-butanol (10 mL) is hydrogenated under balloon pressure over 3% rhodium on carbon (20 g) and monitored periodically by electrospray mass spectrometry until the majority of the starting material is consumed. Catalyst is removed by filtration through diatomaceous earth and the filtrate is concentrated in *vacuo* to provide a gum. This material is then triturated with 1:1 acetonitrile-diethyl ether (300 mL) and the resulting precipitate is filtered, washed with acetonitrile and diethyl ether, and air dried. MS (+ES), *m*/*z*: 644.3 (M+2H)²⁺.

### Example 51

### (Cyclo[3-cyclohexyl-2-aminobutanoyl-O-(4-O-hexopyranosylhexopyranosyl)tyrosyl-3-(2-iminoimidazolidin-4-yl)seryl-3-(3-hexopyranosyl-2-iminoimidazolidin-4-yl)-serylserylglycyl])

The title compound is prepared from cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] by the procedure described for example 50. The product is then purified by reverse phase HPLC. MS (+ES), *m*/*z*: 651.1 (M+2H)²⁺.

### Example 52

### (Cyclo[3-cyclohexylalanyl-3-cyclohexylalanyl-3-(2-iminoimidazolidin-4-yl)seryl-3-(3-hexopyranosyl-2-iminoimidazolidin-4-yl)serylserylglycyl])

### Example 52a

### (Cyclo[3-cyclohexylalanyl-3-[4-[(4-O-hexopyranosylhexopyranosyl)oxy]cyclohexyl]alanyl-3-(2-iminoimidazolidin-4-yl)seryl-3-(3-hexopyranosyl-2-iminoimidazolidin-4-yl)serylserylglycyl])

### Example 52b

### (Cyao(3-cyaohexylalanyl-3-[4-[(4-O-hexopyranosylhexopyranosyl)oxylcyohexyl]alanyl-3-(2-iminoimidazolidin-4-yl)seryl-3-(3-hexopyranosyl-2-iminoimidazolidin-4-yl)serylserylglycyl])

A solution of the bis-hydrochloride salt of cyclo[glycylphenylalanyl-O-(4-O-hexopyranosylhexopyranosyl)tyrosyl-3-(2-iminomidazolidin-4-yl)seryl-3-(3-hexopyranosyl-2-iminoimidazolidin-4-yl)serylseryl] (350 mg) in 10% aqueous acetic acid (10 mL) containing n-butanol (1 mL) is hydrogenated over 3% Rh/C (250 mg) in a Parr apparatus at an initial pressure of 50 psi and monitored periodically by electrospray mass spectrometry until the starting material has been completely converted to products of the desired molecular weight. Catalyst is removed by filtration through diatomaceous earth and the filtrate is concentrated *in vacuo* to provide a gum. This material is then subjected to reverse phase HPLC separation to provide the three title compounds. Cyclo[3-cyclohexylalanyl-3-cyclohexylalanyl-3-(2-iminoimidazolidin-4-yl)seryl-3-(3-hexopyranosyl-2-iminoimidazolidin-4-yl)serylserylglycyl]: MS (+ES), *m*/*z:* 477.1 (M+2H)²⁺; Cyclo[3-cyclohexylalanyl-3-[4-[(4-O-hexopyranosylhexopyranosyl)oxy]cyclohexyl]alanyl-3-(2-iminoimidazolidin-4-yl)seryl-3-(3-hexopyranosyl-2-iminoimidazolidin-4-yl)serylserylglycyl]: MS (+ES), *m*/*z:* 647.1 (M+2H)²⁺; Cyclo[3-cyclohexylalanyl-3-[4-[(4-O-hexopyranosylhexopyranosyl)oxy]cyclohexyl]alanyl-3-(2-iminoimidazolidin-4-yl)seryl-3-(3-hexopyranosyl-2-iminoimidazolidin-4-yl)serylserylglycyl]: MS (+ES), *m*/*z:* 647.2 (M+2H)²⁺.

### Example 53

### (Cyclo[3-cyclohexylalanyl-3-(2-iminoimidazolidin-4-yl)seryl-3-(3-hexopyranosyl-2-iminoimidazolidin-4-yl)serylserylglycyl-3-cyclohexyl-2-aminobutanoyl])

### Example 53a

### (Cyclo[3-[4-[(4-O-hexgpyranosylhexgpyranosyl)oxy]cyclohexyl]alanyl-3-(2-iminoimidazolidin-4-yl)seryl-3-(3-hexopyranosyl-2-iminoimidazolidin-4-yl)serylserylglycyl-3-cyclohexyl-2-aminobutanoyl])

The title compounds are prepared from cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] by the procedure described for example 52. Cyclo[3-cyclohexylalanyl-3-(2-iminoimidazolidin-4-yl)seryl-3-(3-hexopyranosyl-2-iminoimidazolidin-4-yl)serylserylglycyl-3-cyclohexyl-2-aminobutanoyl]: MS (+ES), *m*/*z:* 484 (M+2H)²⁺; Cyclo[3-[4-[(4-O-hexopyranosylhexopyranosyl)oxy]cyclohexyl]alanyl-3-(2-iminoimidazolidin-4-yl)seryl-3-(3-hexopyranosyl-2-iminoimidazolidin-4-yl)serylserylglycyl-3-cyclohexyl-2-aminobutanoyl]: MS (+ES), *m*/*z:* 654.1 (M+2H)²⁺.

### Example 54

### (Cyclo[3-(syn-4-hydroxycyclohexyl)alanyl-3-(2-iminoimidazolidin-4-yl)seryl-3-(3-hexopyranosyl-2-iminoimidazolidin-4-yl)serylserylglycyl-3-cyclohexyl-2-aminobutanoyl])

### Example 54a

### (Cyclo[3-(anti-4-hydroxycyclohexyl)alanyl-3-(2-iminoimidazolidin-4-yl)seryl-3-(3-hexopyranosyl-2-iminoimidazolidin-4-yl)serylserylglycyl-3-cyclohexyl-2-aminobutanoyl])

The title compounds are prepared from cyclo[glycyl-β-methylphenylalanyltyrosyl-3-(2-iminoimidazolidin-4-yl)seryl-3-(3-hexopyranosyl-2-iminoimidazolidin-4-yl)serylseryl] by the procedure described for example 52. Cyclo[3-cyclohexylalanyl-3-(2-iminoimidazolidin-4-yl)seryl-3-(3-hexopyranosyl-2-iminoimidazolidin-4-yl)serylserylglycyl-3-cyclohexyl-2-aminobutanoyl]: MS (+ES), *m*/*z*: 484 (M+2H)²⁺; Cyclo[3-(syn-4-hydroxycyclohexyl)alanyl-3-(2-iminoimidazolidin-4-yl)seryl-3-(3-hexopyranosyl-2-iminoimidazolidin-4-yl)serylserylglycyl-3-cyclohexyl-2-aminobutanoyl]: MS (+ES), *m*/*z:* 492.5 (M+2H)²⁺; Cyclo[3-(anti-4-hydroxycyclohexyl)alanyl-3-(2-iminoimidazolidin-4-yl)seryl-3-(3-hexopyranosyl-2-iminoimidazolidin-4-yl)serylserylglycyl-3-cyclohexyl-2-aminobutanoyl]: MS (+ES), *m*/*z:* 492.3 (M+2H)²⁺.

### Example 55

### (Cyclo[glycyl-β-methylphenylalanyltyrosyl-3-(2-iminoimidazolidin-4-yl)seryl-3-(3-hexopyranosyl-2-iminoimidazolidin-4-yl)serylseryl])

A stirred solution of the bis-hydrochloride salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (10 g) in dimethyl sulfoxide (80 mL) is treated with concentrated hydrochloric acid (37%, 5 mL) and the mixture is heated at 60°C for 20 hrs. The solution is then filtered to remove insoluble material and the filtrate is treated dropwise at 60°C with acetonitrile (180 mL) to precipitate the product. The mixture is cooled to room temperature and the precipitate is removed by filtration, the filter cake is washed with acetonitrile and diethyl ether and air dried to provide the product as a tan powder. MS (+ES), *m*/*z:* 486.4 (M+2H)²⁺.

### Example 56

### (Cyclo[glycylphenylalanyltyrosyl-3-(2-iminoimidazolidin-4-yl)seryl-3-(3-hexopyranosyl-2-iminoimidazolidin-4-yl)serylseryl])

The title compound is prepared from cyclo[glycylphenylalanyl-O-(4-O-hexopyranosylhexopyranosyl)tyrosyl-3-(2-iminomidazolidin-4-yl)seryl-3-(3-hexopyranosyl-2-iminoimidazolidin-4-yl)serylseryl] by the procedure described for example 55. MS (+ES), *m*/*z:* 479.5 (M+2H)²⁺.

### Example 57

### (Cyclo[glycyl-β-methylphenylalanyltyrosyl-3-(2-iminoimidazolidin-4-yl)seryl-3-(2-iminoimidazolidin-4-yl)serylserl])

To a suspension of the bis-hydrochloride salt of cyclo[glycyl-β-methylphenylalanyltyrosyl-3-(2-iminoimidazolidin-4-yl)seryl-3-(3-hexopyranosyl-2-iminoimidazolidin-4-yl)serylseryl] (1.5 g) in water (60 mL) is added sodium periodate (616 mg) and the mixture is stirred for 5 h. at room temperature. Methanol (45 mL) is added, followed by sodium borohydride (326 mg) and the mixture is stirred for 16 h. The volume of the solution is adjusted to 150 mL by the addition of methanol, and then concentrated hydrochloric acid (37%, 9 mL) is added and the solution is stirred at 60°C for 16 h. The solution is cooled and concentrated *in vacuo* to provide a gum, which is subjected to flash chromatography over a reverse phase support (MCI CHP20P) to provided the desired product. MS (+ES), *m*/*z:* 405.4 (M+2H)²⁺.

### Example 58

### (Cyclo[3-cyclohexylalanyl-3-cyclohexylalanyl-3-(2-iminoimidazolidin-4-yl)seryl-3-(2-iminoimidazolidin-4-yl)-serylserylglycl])

The title compound is prepared from cyclo[3-cyclohexylalanyl-3-cyclohexylalanyl-3-(2-iminoimidazolidin-4-yl)seryl-3-(3-hexopyranosyl-2-iminoimidazolidin-4-yl)serylserylglycyl] by the procedure described for example 57. MS (+ES), *m*/*z:* 396.4 (M+2H)²⁺.

### Example 59

### (Cyclo[glycyl-β-methylphenylalanyl-O-(4-O-hexopyranosylhexopyranosyl)tyrosyl-(2-iminoimidazolidin-4-yl)seryl-3-[1-benzyl-3-hexopyranosyl-2-iminoimidazolidin-4-yl]serylserl])

A stirred solution of the bis-hydrochloride salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (1.0 g) in dimethyl sulfoxide (25 mL) and water (4 mL) is treated with benzyl bromide (2.0 mL) and potassium carbonate (2.0 g) and the mixture is stirred for 2 h at room temperature. The reaction mixture is filtered and the filtrate is treated with acetonitrile (100 mL) to precipitate the crude product. The precipitate is filtered, washed with acetonitrile, and air-dried. The desired product is purified by reverse phase HPLC. MS (+ES), *m*/*z:* 693.3 (M+2H)²⁺.

### Example 60

### (Cyclo[glycyl-β-methylphenylalanyl-O-(4-O-hexopyranosylhexopyranosyl)tyrosyl-(2-iminoimidazolidin-4-yl)seryl-3-[1-(4-tert-butylbenzyl)-3-hexopyranosyl-2-iminoimidazolidin-4-yl]-serylseryl])

### Example 60a

### (Di-N-(4-tert-butylbenzyl)-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl])

A stirred solution of the bis-hydrochloride salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (0.5 g) in dimethyl sulfoxide (10 mL) and water (1 mL) is treated with 4-t-butylbenzyl bromide (0.8 g) and potassium carbonate (0.8 g) and the mixture is stirred for 3 h at room temperature. The reaction mixture is filtered and the filtrate is treated with acetonitrile (100 mL) to precipitate the crude products. The precipitate is filtered, washed with acetonitrile, and air-dried. The desired products are purified by reverse phase HPLC. Cyclo[glycyl-β-methylphenylalanyl-O-(4-O-hexopyranosylhexopyranosyl)tyrosyl-(2-iminoimidazolidin-4-yl)seryl-3-[1-(4-tert-butylbenzyl)-3-hexopyranosyl-2-iminoimidazolidin-4-yl]-serylseryl]: MS (+ES), *m*/*z:* 721.4 CM+2H)²⁺; Di-N-(4-tert-butylbenzyl)-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES), *m*/*z*: 794.5 (M+2H)²⁺.

### Example 61

### (Cyclo[glycyl-β-methylphenylalanyl-O-(4-O-hexopyranosylhexopyranosyl)tyrosyl-3-(2-iminoimidazolidin-4-yl)seryl-3-[3-hexopyranosyl-1-(12-hydroxydodecyl)-2-iminoimidazolidin-4-yl]-serylseryl])

A stirred solution of the bis-hydrochloride salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (1.0 g) in dimethyl sulfoxide (20 mL) and water (4 mL) is treated with 12-bromo-1-dodecanol (0.66 g) and potassium carbonate (0.8 g) and the mixture is stirred for 2 h at room temperature. The reaction mixture is filtered and the filtrate is treated with acetonitrile (100 mL) to precipitate the crude product. The precipitate is filtered, washed with acetonitrile, and air-dried. The desired product is purified by reverse phase HPLC. MS (+ES), *m*/*z*: 740.4 (M+2H)²⁺.

### Example 62

### (Cyclo[glycyl-β-methylphenylalanyl-O-(4-O-hexopyranosylhexopyranosyl)tyrosyl-3-[1,3-dibenzyl-2-(benzylimino)imidazolidin-4-yl]seryl-3-[1-benzyl-2-(benzylimino)-3-hexopyranosylimidazolidin-4-yl]serylseryl])

A stirred solution of the bis-hydrochloride salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (1.0 g) in dimethyl sulfoxide (25 mL) and water (4 mL) is treated with benzyl bromide (2.0 mL) and potassium carbonate (2.0 g) and the mixture is stirred for 16 h at room temperature. Additional benzyl bromide (1.0 mL), potassium carbonate (0.6 g) and water (5 mL) is then added and the mixture is stirred for 20 h. The reaction mixture is filtered and the filtrate is treated with acetonitrile (180 mL) to precipitate the crude product. The precipitate is filtered, washed with acetonitrile, and air-dried. The desired product is purified by reverse phase HPLC. MS (+ES), *m*/*z*: 873.4 (M+2H)²⁺.

### Example 63

### (Cyclo[glycyl-β-methylphenylalanyl-O-(4-O-hexopyranosylhexopyranosyl)tyrosyl-3-(2-iminoimidazolidin-4-yl)seryl-3-[1-butyl-3-hexopyranosyl-2-iminoimidazolidin-4-yl]serylseryl])

A stirred solution of the bis-hydrochloride salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (1.0 g) in dimethyl sulfoxide (20 mL) and water (2.0 mL) is treated with 1-bromobutane (0.24 mL) and potassium carbonate (0.6 g) and the mixture is stirred for 24 h at room temperature. The reaction mixture is filtered and the filtrate is treated with acetonitrile to precipitate the crude product. The precipitate is filtered, washed with acetonitrile, and air-dried. The desired product is purified by reverse phase HPLC. MS (+ES), *m*/*z*: 676.4 (M+2H)²⁺.

### Example 64

### (Cyclo[glycyl-β-methylphenylalanyl-O-(4-O-hexopyranosylhexopyranosyl)tyrosyl-3-(1,3-dimethyl-2-(methylimino)imidazolidin-4-yl)seryl-3-[3-hexopyranosyl-1-methyl-2-(methylimino)imidazolidin-4-yl]-serylseryl])

A stirred solution of the bis-hydrochloride salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (0.8 g) in dimethyl sulfoxide (15 mL) and water (1.5 mL) is treated with methyl iodide (0.35 mL) and potassium carbonate (1.0 g) and the mixture is stirred for 16 h at room temperature. Additional benzyl bromide (1.0 mL), potassium carbonate (0.6 g) and water (5 mL) is then added and the mixture is stired for 20 h. The reaction mixture is filtered and the filtrate is treated with acetonitrile (180 mL) to precipitate the crude product. The precipitate is filtered, washed with acetonitrile, and air-dried. The desired product is purified by reverse phase HPLC. MS (+ES), *m*/*z*: 697.6 (M)²⁺.

### Example 65

### (Cyclo[glycyl-β-methylphenylalanyl-O-(4-O-hexopyranosylhexopyranosyl)tyrosyl-3-(2-iminoimidazolidin-4-yl)seryl]-3-[3-hexgpyranosyl-2-imino-1-(2-phenylbenzyl)-imidazolidin-4-yl]-serylseryl])

The title compound is prepared by the procedure described for example 59 using the appropriate arylmethyl halide. MS (+ES), *m*/*z*: 731.6(M+2H)²⁺.

### Example 66

### (Cyclo[glycyl-β-methylphenylalanyl-O-(4-O-hexopyranosylhexopyranosyl)tyrosyl-3-[3-(4-phenylbenzyl)-2-[(4-phenylbenzyl)imino]imidazolidin-4-yl]seryl-3-[3-hexopyranosyl-2-imino-1-(4-phenylbenzyl)-imidazolidin-4-yl]serylseryl])

### Example 66a

### (Cyclo[glycyl-β-methylphenylalanyl-O-(4-O-hexopyranosylhexopyranosyl)tyrosyl-3-(2-iminoimidazolidin-4-yl)seryl]-3-[3-hexopyranosyl-2-imino-1-(4-phenylbenzyl)-imidazolidin-4-yl]-serylseryl])

The title compound is prepared by the procedure described for example 60 using the appropriate arylmethyl halide. Cyclo[glycyl-β-methylphenylalanyl-O-(4-O-hexopyranosylhexopyranosyl)tyrosyl-3-[3-(4-phenylbenzyl)-2-[(4-phenylbenzyl)imino]imidazolidin-4-yl]seryl-3-[3-hexopyranosyl-2-imino-1-(4-phenylbenzyl)-imidazolidin-4-yl]serylseryl]: MS (+ES), *m*/*z*: 897.6(M+2H)²⁺; Cyclo[glycyl-β-methylphenylalanyl-O-(4-O-hexopyranosylhexopyranosyl)tyrosyl-3-(2-iminoimidazolidin-4-yl)seryl]-3-[3-hexopyranosyl-2-imino-1-(4-phenylbenzyl)-imidazolidin-4-yl]-serylseryl]: MS (+ES), *m*/*z:* 731.6(M+2H)²⁺.

### Example 67

### (Cyclo[glycyl-β-methylphenylalanyl-O-(4-O-hexopyranosylhexopyranosyl)tyrosyl-3-(2-iminoimidazolidin-4-yl)seryl-3-[3-hexopyranosyl-2-imino-1-(2-naphthylmethyl)-imidazolidin-4yl]-serylseryl])

The title compound is prepared by the procedure described for example 59 using the appropriate arylmethyl halide. MS (+ES), *m*/*z:* 718.7(M+2H)²⁺.

### Example 68

### (Cyclo[glycyl-β-methylphenylalanyl-O-(4-O-hexopyranosylhexopyranosyl)tyrosyl-3-(2-iminoimidazolidin-4-yl)serl]-3-[3-hexopyranosyl-2-imino-1-(4-trifluoromethylbenzyl)-imidazolidin-4-yl]-serylseryl])

The title compound is prepared by the procedure described for example 59 using the appropriate arylmethyl halide. MS (+ES), *m*/*z:* 727.5(M+2H)²⁺.

### Example 69

### (Cycol[glycyl-β-methylphenylalanyl-O-(4-O-hexopyranosylhexopyranosyl)tyrosyl-3-(2-iminoimidazolidin-4-yl)seryl-3-[1-(4-carboxybenzyl)-2-imino-3-hexopyranosylimidazolidin-4-yl]-serylseryl])

### Example 69a

### (Di-N-(4-carboxybenzyl)-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl])

### Example 69b

### (Tri-N-(4-carboxybenzyl)-Cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl])

The title compounds are prepared by the procedure described for example 60 using the appropriate arylmethyl halide. Cyclo[glycyl-β-methylphenylalanyl-O-(4-O-hexopyranosylhexopyranosyl)tyrosyl-3-(2-iminoimidazolidin-4-yl)seryl-3-[1-(4-carboxybenzyl)-2-imino-3-hexopyranosylimidazolidin-4-yl]-serylseryl]: MS (+ES), *m*/*z:* 715.4(M+2H)²⁺; Di-N-(4-carboxybenzyl)-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES), *m*/*z:* 782.4(M+2H)²⁺; Tri-N-(4-carboxybenzyl)-cCyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES), *m*/*z*: 849.2(M+2H)²⁺.

### Example 70

### (Cyclo[glycyl-β-methylphenylalanyl-O-(4-O-hexopyranosylhexopyranosyl)tyrosyl-3-(2-iminoimidazolidin-4-yl)seryl-3-[3-hexopyranosyl-2-imino-1-(3-methyl-but-2-enyl)imidazolidin-4-yl]serylseryl])

The title compound is prepared by the procedure described for example 59 using the appropriate allylic halide. MS (+ES), *m*/*z:* 682.5(M+2H)²⁺.

### Example 71

### (Cyclo[glycyl-β-methylphenylalanyl-O-(4-O-hexopyranosylhexopyranosyl)tyrosyl-3-(2-iminoimidazolidin-4-yl)seryl-3-[1-heptyl-3-hexopyranosyl-2-iminoimidazolidin-4-yl]serylseryl])

The title compound is prepared by the procedure described for example 63 using the appropriate alkyl halide. MS (+ES), *m*/*z:* 697.4(M+2H)²⁺.

### Example 72

### (Cyclo[glycyl-β-methylphenylalanyl-O-(4-O-hexopyranosylhexopyranosyl)tyrosyl-3-(2-iminoimidazolidin-4-yl)seryl-3-[1-(10-carboxydecyl)-3-hexopyranosyl-2-iminoimidazolidin-4-yl]serylseryl])

### Example 72a

### (Di-N-(10-carboxydecyl)-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl])

The title compounds are prepared by the procedure described for example 61 using the appropriate alkyl halide. Cyclo[glycyl-β-methylphenylalanyl-O-(4-O-hexopyranosylhexopyranosyl)tyrosyl-3-(2-iminoimidazolidin-4-yl)seryl-3-[1-(10-carboxydecyl)-3-hexopyranosyl-2-iminoimidazolidin-4-yl]serylseryl]: MS (+ES), *m*/*z:* 740.7(M+2H)²⁺; Di-N-(10-carboxydecyl)-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES), *m*/*z:* 832.9(M+2H)²⁺.

### Example 73

### (Cycle[3-[1,3-benzyl-2-(benzylimino)imidazolidin-4-yl]alanyl-3-[1-benzyl-2-(benzylimino)-3-hexopyranosylimidazolidin-4-yl]alanylserylglycyl-β-methylphenylalanyl-O-(4-O-hexopyranosylhexopyranosyl)tyrosyl])

The title compound is prepared from cyclo[3-(2-iminoimidazolidin-4-yl)alanyl-3-(3-hexopyranosyl-2-iminoimidazolidin-4-yl)-alanylserylglycyl-β-methylphenylalanyl-O-(4-O-hexopyranosylhexopyranosyl)tyrosyl] by the procedure described for example 62 using the appropriate alkyl halide. MS (+ES), *m*/*z*: 857.9 (M+2H)²⁺.

### Example 74

### (Cyclo[3-[1,3-dimethyl-2-(methylimino)imidazolidin-4-yl]alanyl-3-[3-hexopyranosyl-1-methyl-2-(methylimino)imidazolidin-4-yl]alanylserylglycyl-β-methylphenylalanyl-O-(4-O-hexopyranosylhexopyranosyl)tyrosyl])

The title compound is prepared from cyclo[3-(2-iminoimidazolidin-4-yl)alanyl-3-(3-hexopyranosyl-2-iminoimidazolidin-4-yl)-alanylserylglycyl-β-methylphenylalanyl-O-(4-O-hexopyranosylhexopyranosyl)tyrosyl] by the procedure described for example 64. MS (+ES), *m*/*z:* 681.7(M)²⁺.

### Example 75

### (Cyclo[glycyl-β-methylphenylalanyl-O-(methyl)tyrosyl-3-(1,3-dimethyl-2-(methylimino)imidazolidin-4-yl)seryl-3-[3-hexopyranosyl-1-methyl-2-(methylimino)imidazolidin-4-yl]-serylseryl])

The title compound is prepared from cyclo[glycyl-β-methylphenylalanyltyrosyl-3-(2-iminoimidazolidin-4-yl)seryl-3-(3-hexopyranosyl-2-iminoimidazolidin-4-yl)serylseryl] by the procedure described for example 64. MS (+ES), *m*/*z:* 542.6(M)²⁺.

### Example 76

### (Cyclorslycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-[2-(benzylimino)-3-hexopyranoxylimidazolidin-4-yl]serylseryl])

The title compound is prepared from cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-(3-hexopyranosyl-2-iminoimidazolidin-4-yl)serylseryl] by the procedure described for example 59. MS (+ES) *m*/*z:* 759.4 (M+2H)²⁺.

### Example 77

### (Cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-[2-[1-(4-tert-butylbenzyl)-3-hexopyranosyl-2-imidazolidin-4-yl]serylseryl]

The title compound is prepared from cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-(3-hexopyranosyl-2-iminoimidazolidin-4-yl)serylseryl] by the procedure described for example 59 using the appropriate arylmethyl halide. MS (+ES) *m*/*z:* 787.7 (M+2H)²⁺.

### Example 78

### (Cyclo[glycyl-β-methylohenylalanyl-O-[4-O-[4,6-O-(2-adamantylidene)hexopyranosyl]hexopyranosyl]tyrosyl-3-(2-iminoimidazolidin-4-yl)seryl-3-[3-hexopyranosyl-2-imino-1-(2-naphthylmethyl)-imidazolidin-4-yl]serylseryl]

The title compound is prepared from cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-(3-hexopyranosyl-2-iminoimidazolidin-4-yl)serylseryl] by the procedure described for example 59 using the appropriate arylmethyl halide. MS (+ES) *m*/*z:* 784.6 (M+2H)²⁺.

### Example 79

### (Cyclo[glycyl-β-methylphenylalanyl-O-[4-O-[4,6-O-(2-adamantylidene)hexopyranosyl]hexopyranosyl]tyrosyl-3-[1,3-dibenzyl-2-(benzylimino)imidazolidin-4-yl]seryl-3-[1-benzyl-2-(benzylimino)-3-hexopyranosylimidazolidin-4-yl]serylseryl])

The title compound is prepared from cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-(3-hexopyranosyl-2-iminoimidazolidin-4-yl)serylseryl] by the procedure described for example 62 using the appropriate arylmethyl halide. MS (+ES) *m*/*z:* 939.9 (M+2H)²⁺.

### Example 80

### (Cyclo[3-(2-iminoimidazolidin-4-yl)alanyl-3-(1-benzyl-3-hexopyranosyl-2-iminoimidazolidin-4-yl)alanylserylglycyl-β-methylphenylalanyl-O-[4-0-[4,6-O-(2-adamantylidene)hexopyranosyl]hexopyranosyl]tyrosyl])

The title compound is prepared from cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[4,6-O-(2-adamantylidene)hexopyranosyl]-hexopyranosyl]tyrosyl] by the procedure described for example 59. MS (+ES) *m*/*z:* 743.6 (M+2H)²⁺.

### Example 81

### (Cyclo[glycyl-β-methylphenylalanyl-O-[4-O-[4,6-O-(2-adamantylidene)hexopyranosyl]hexopyranosyl]tyrosyl-3-[1,3-dimethyl-2-(methylimino)imidazolidin-4-yl]seryl-3-[3-hexopyranosyl-1-methyl-2-(methylimino)imidazolidin-4-yl]serylseryl])

The title compound is prepared from cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-(3-hexopyranosyl-2-iminoimidazolidin-4-yl)serylseryl] by the procedure described for example 64. MS (+ES) *m*/*z:* 763.6 (M)²⁺.

### Example 82

### (Cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-[1-[(3,7-dimethylocta-2,6-dien-1-yl)-imino]-3-hexopyranosylimidazolidin-4-yl]serylseryl])

The title compound is prepared from cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-(3-hexopyranosyl-2-iminoimidazolidin-4-yl)serylseryl] by the procedure described for example 59. MS (+ES) *m*/*z:* 782.5 (M+2H)²⁺.

### Example 83

### (Cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-[1-heptyl-3-hexopyranosyl-2-iminoimidazolidin-4-yl]serylseryl]

The title compound is prepared from cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-(3-hexopyranosyl-2-in-iminoimidazolidin-4-yl)serylseryl] by the procedure described for example 63 using the appropriate alkyl halide. MS (+ES) *m*/*z:* 763.5 (M+2H)²⁺.

### Example 84

### (Cyclo[glycyl-β-methylphenylalanyl-O-[4-O-[4,6-O-(2-adamantylidene)hexopyranosyl]-hexopyranosyl]tyrosyl-3-(2-iminoimidazolidin-4-yl)seryl-3-[3-hexopyranosyl-2-(hexylimino)imidazolidin-4-yl]serylseryl])

The title compound is prepared from cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-(3-hexopyranosyl-2-iminoimidazolidin-4-yl)serylseryl] by the procedure described for example 63 using the appropriate alkyl halide. MS (+ES) *m*/*z:* 756.5 (M+2H)²⁺.

### Example 85

### (Cyclo[3-[2-[2-(hexopyranosyloxy)phenyl]-1,3-benzoxazol-5-yl]alanyl-3-(2-iminoimidazolidin-4-yl)seryl-3-(1-benzyl-3-hexopyranosyl-2-iminoimidazolidin-4-yl)serylserylglycyl-β-methyl-phenylalanyl])

The title compound is prepared from cyclo[3-[2-[2-(hexopyranosyloxy)phenyl]-1,3-benzoxazol-5-yl]alanyl-3-(2-iminoimidazolidin-4-yl)seryl-3-(3-hexopyranosyl-2-iminoimidazolidin-4-yl)serylserylglycyl-β-methyl-phenylalanyl] by the procedure described for example 59. MS (+ES) *m*/*z:* 671.0 (M+2H)²⁺.

### Example 86

### (Cyclo[glycyl-β-methylphenylalanyl-O-(4-O-hexopyranosylhexopyranosyl)tyrosyl-3-(2-iminoimidazolidin-4-yl)seryl-3-(2-decanoylimino-3-hexopyranosylimidazolidin-4-yl)serylseryl])

A stirred solution of the bis-hydrochloride salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (0.1 g) in N,N-dimethylformamide (1.5 mL) and 2,4,6-collidine (1.2 mL) is treated with decanoic anhydride (0.07 mL) and the mixture is stirred for 30 h at room temperature. The reaction mixture is then concentrated *in vacuo* to provide a gum, which is triturated with ethyl acetate to precipitate the crude product. The precipitate is filtered, washed with ethyl acetate, and air-dried. The desired product is purified by reverse phase HPLC. MS (+ES), *m*/*z*: 725.2 (M+2H)²⁺.

### Example 87

### (Cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-(3-methyl-butyrylimino)-imidazolidin-4-yl)serylseryl])

The title compound is prepared by the procedure described for example 86 using the appropriate carboxylic acid anhydride. MS (+ES), *m*/*z:* 690.2 (M+2H)²⁺.

### Example 88

### (Cyclo[glycyl-β-methylphenylalanyl-O-(4-O-hexopyranosylhexopyranosyl)tyrosyl-3-(2-iminoimidazolidin-4-yl)seryl-3-(2-(2-ethyl-butyrylimino)-3-hexopyranosylimidazolidin-4-yl)serylseryl])

The title compound is prepared by the procedure described for example 86 using the appropriate carboxylic acid anhydride. MS (+ES), *m*/*z:* 697.2 (M+2H)²⁺.

### Example 89

### (Tri-N-[(phenylmethoxy)carbonyl]-cyclo[glycyl-β-methylphenylalanyltyrosyl-3-(2-iminoimidazolidin-4-yl)seryl-3-(3-hexopyranosyl-2-iminoimidazolidin-4-yl)serylseryl])

A stirred solution of the bis-acetate salt of cyclo[glycyl-β-methylphenylalanyltyrosyl-3-(2-iminoimidazolidin-4-yl)seryl-3-(3-hexopyranosyl-2-iminoimidazolidin-4-yl)serylseryl] (0.2 g) in dimethyl sulfoxide (1.5 mL) is treated with N,N-diisopropylethylamine (0.105 mL) and a solution of N-(benzyloxycarbonyloxy)-succinimide (160 mg) in dimethyl sulfoxide (0.3 mL) and the mixture is stirred for 16 h at room temperature. Acetonitrile (8 mL) is then added to precipitate the crude product. The precipitate is filtered, washed with acetonitrile and diethyl ether, and air-dried. The desired product is purified by reverse phase HPLC. MS (+ES), *m*/*z:* 687.3 (M+2H)²⁺.

### Example 90

### (N-[(Phenylmethoxy)carbonyl]-cyclo[glycyl-β-methylphenylalanyl-O-(4-O-hexopyranosylhexopyranosyltyrosyl-3-(2-iminoimidazolidin-4-yl)seryl-3-(3-hexopyranosyl-2-iminoimidazolidin-4-yl)serylseryl])

A stirred solution of the bis-hydrochloride salt of cyclo(glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (50 mg) in N,N-dimethylformamide (0.8 mL) is treated with N,N-diisopropylethylamine (0.013 mL) and N-(benzyloxycarbonyloxy)-succinimide (13 mg) and the mixture is stirred for 16 h at room temperature. Acetonitrile is then added to precipitate the crude product. The precipitate is filtered, washed with ethyl acetate, and air-dried. The desired product is purified by reverse phase HPLC. MS (+ES), *m*/*z:* 715.8 (M+2H)²⁺.

### Example 91

### (Tri-N-[(phenylmethoxy)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl])

The title compound is prepared from cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] by the procedure described for example 89. MS (+ES), *m*/*z:* 849.7 (M+2H)²⁺.

### Example 92

### (Cyclo[glycyl-β-methylphenylalanyl-O-(4-O-hexopyranosylhexopyranosyl)tyrosyl-3-[2-(pyrimidin-2-ylimino)imidazolidin-4-yl]seryl-3-[3-hexopyranosyl-2-imino-1-(pyrimidin-2-yl)imidazolidin-4-yl]serylseryl])

A stirred solution of the bis-hydrochloride salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (0.908 g) in dimethyl sulfoxide (20 mL) is treated with potassium carbonate (0.912 g) and 2-chloropyrimidine (0.756 g) and the mixture is stirred for 20 h at 50° C. The mixture is cooled to room temperature, decanted into acetonitrile (64 mL) and the resultant precipitate collected by centrifugation. The precipitate is resuspended in acetonitrile (64 mL) and recentrifuged. The resulting pale brown solid is purified by reverse phase HPLC. MS (+ES), *m*/*z:* 726.4 (M+2H)²⁺.

### Example 93

### (Cyclo[glycyl-β-methylphenylalanyl-O-(4-O-hexopyranosylhexolpyranosyl)tyrosyl-3-[2-(1,3-benzoxazol-2-ylimino)imidazolidin-4-yl]seryl-3-[1-(1,3-benzoxaxol-2-yl)-3-hexopyranosyl-2-iminoimidazolidin-4-yl]serylseryl])

A stirred solution of the bis-hydrochloride salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (0.908 g) in dimethyl sulfoxide (20 mL) is treated with potassium carbonate (0.912 g) and 2-chlorobenzoxazole (0.674 g) and the mixture is stirred for 4 h at room temperature. The reaction mixture is decanted into acetonitrile (64 mL) and the resultant precipitate collected by centrifugation. The precipitate is resuspended in acetonitrile (64 mL) and recentrifuged. The resulting pale brown solid is purified by reverse phase HPLC. MS (+ES), *m*/*z:* 765.3 (M+2H)²⁺.

### Example 94

### (Cyclo[glycyl-β-methylphenylalanyl-O-(4-O-hexopyranosylhexopyranosyl)tyrosyl-3-[2-(1,3-benzothiazol-2-ylimino)imidazolidin-4-yl]seryl-3-[1-(1,3-benzo-thiazoL-2-yl)-2-imino-3-hexopyranosylimidazolidin-4-yl]serylseryl])

A stirred solution of the bis-hydrochloride salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (0.24 g) in dimethyl sulfoxide (5 mL) is treated with potassium carbonate (0.24 g) and 2-chlorobenzothiazole (0.297 g) and the mixture is stirred for 5 days at 35° C. The mixture is cooled to room temperature, decanted into acetonitrile (64 mL) and the resultant precipitate collected by centrifugation. The precipitate is resuspended in acetonitrile (64 mL) and recentrifuged. The resulting pale brown solid is purified by reverse phase HPLC. MS (+ES), *m*/*z:* 781.2 (M+2H)²⁺.

### Example 95

### (Cyclo[glycyl-β-methylphenylalanyl-O-(4-O-hexopyranosylhexopyranosyl)tyrosyl-3-(2-iminoimidazolidin-4-yl)seryl-3-[3-(6-O-hexanoylhexopyranosyl)-2-iminoimidazolidin-4-yl]serylseryl])

### Example 95a

### (Cyclo[glycyl-β-methylphenylalanyl-O-(6-O-hexanoyl-4-O-hexopyranosylhexopyranosyl)-tyrosyl-3-(2-iminoimidazolidin-4-yl)seryl-3-(3-hexonyranosyl-2-iminoinudazolidin-4-yl)-serylseryl])

### Example 95b

### (Cyclo[glycyl-β-methylphenylalanyl-O-[4-O-(6-O-hexanoylhexopyranosyl)hexopyranosyl]-tyrosyl-3-(2-iminoimidazolidin-4-yl)seryl-3-(3-hexopyranosyl-2-iminoimidazolidin-4-yl)-serylseryl])

A stirred solution of the bis-trifluoroacetate salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (0.38 g) in N,N-dimethylformamide (50 mL) and pyridine (0.4 mL) is treated dropwise with a solution of hexanoic anhydride (0.25 g) in N,N-dimethylformamide (2 mL) and the reaction mixture is stirred for 16 h at room temperature. The reaction is then quenched by the addition of methanol (100 mL), stirred for 30 min, and the volatiles are removed *in vacuo.* The resulting residue is purified by reverse phase HPLC to provide the title compounds. Cyclo[glycyl-β-methylphenylalanyl-O-(4-O-hexopyranosylhexopyranosyl)tyrosyl-3-(2-iminoimidazolidin-4-yl)seryl-3-[3-(6-O-hexanoylhexopyranosyl)-2-iminoimidazolidin-4-yl]serylseryl]: MS (+ES), *m*/*z:* 697.4 (M+2H)²⁺; Cyclo[glycyl-β-methylphenylalanyl-O-(6-O-hexanoyl-4-O-hexopyranosylhexopyranosyl)-tyrosyl-3-(2-iminoimidazolidin-4-yl)seryl-3-(3-hexopyranosyl-2-iminoimidazolidin-4-yl)-serylseryl]: MS (+ES), *m*/*z:* 697.4 (M+2H)²⁺; Cyclo[glycyl-β-methylphenylalanyl-O-[4-O-(6-O-hexanoylhexopyranosyl)hexopyranosyl]-tyrosyl-3-(2-iminoimidazolidin-4-yl)seryl-3-(3-hexopyranosyl-2-iminoimidazolidin-4-yl)-serylseryl]: MS (+ES), *m*/*z:* 697.4 (M+2H)²⁺.

### Example 96

### (Cyclo[glycyl-β-methylphenylalanyl-O-(4-O-hexopyranosylhexopyranosyl)tyrosyl-3-(2-iminoimidazolidin-4-yl)seryl-3-[3-[6-O-(diphenylacetyl)hexopyranosyl]-2-iminoimidazolidin-4-yl]-serylseryl])

### Example 96a

### (Cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)seryl-O-(diphenylacetyl)-seryl])

### Example 96b

### (Cyclo[glycyl-β-methylphenylalanyl-O-[6-O-(diphenylacetyl)-4-O-hexopyranosylhexopyranosyl]tyrosyl-3-(2-iminoimidazolidin-4-yl)seryl-3-(3-hexopyranosyl-2-iminoimidazolidin-4-yl)serylseryl])

### Example 96c

### (Cyclorslycyl-β-methylphenylalanyl-O-[4-O-[6-O-(diphenylaceyl)hexopyranosyl]hexopyranosyl]tyrosyl-3-(2-iminoimidazolidin-4-yl)seryl-3-(3-hexopyranosyl-2-iminoimidazolidin-4-yl)serylseryl])

A stirred solution of the bis-trifluoroacetate salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (1.0 g) in N,N-dimethylformamide (100 mL) and pyridine (1.0 mL) is treated dropwise with a solution of diphenylacetyl chloride (0.8 g) in N,N-dimethylformamide (2 mL) at -5°C. The resulting mixture is warmed to room temperature and stirred for 1 h. The reaction is then quenched by the addition of methanol (100 mL), stirred for 30 min, and the volatiles are removed *in vacuo.* The resulting residue is purified by reverse phase HPLC to provide the title comounds. Cyclo[glycyl-β-methylphenylalanyl-O-(4-O-hexopyranosylhexopyranosyl)tyrosyl-3-(2-iminoimidazolidin-4-yl)seryl-3-[3-[6-O-(diphenylacetyl)hexopyranosyl]-2-iminoimidazolidin-4-yl]-serylseryl]: MS (+ES), *m*/*z:* 745.2 (M+2H)²⁺; Cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)seryl-O-(diphenylacetyl)-seryl]: MS (+ES), *m*/*z:* 745.4 (M+2H)²⁺; Cyclo[glycyl-β-methylphenylalanyl-O-[6-O-(diphenylacetyl)-4-O-hexopyranosylhexopyranosyl]tyrosyl-3-(2-iminoimidazolidin-4-yl)seryl-3-(3-hexopyranosyl-2-iminoimidazolidin-4-yl)serylseryl]: MS (+ES), *m*/*z:* 745.3 (M+2H)2⁺; Cyclo[glycyl-β-methylphenylalanyl-O-[4-O-[6-O-(diphenylacetyl)hexopyranosyl]hexopyranosyl]tyrosyl-3-(2-iminoimidazolidin-4-yl)seryl-3-(3-hexopyranosyl-2-iminoimidazolidin-4-yl)serylseryl]: MS (+ES), *m*/*z:* 745.4 (M+2H)²⁺.

### Example 97

### (Cyclo[glycyl-β-methylphenylalanyl-O-(4-O-hexopyranosylhexopyranosyl)tyrosyl-3-(2-iminoimidazolidin-4-yl)seryl-3-β-(6-O-heptanoylhexopyranosyl)-2-iminoimidazolidin-4-yl]seryl-seryl])

### Example 97a

### (Cyclo[glycyl-β-methylphenylalanyl-O-(6-O-heptanoyl-4-O-hexopyranosylhexopyranosyl)-tyrosyl-3-(2-iminoimidazolidin-4-yl)seryl-3-(3-hexopyranosyl-2-iminoimidazolidin-4-yl)-serylseryl])

### Example 97b

### (Cyclo[glycyl-β-methylphenylalanyl-O-[4-O-(6-O-heptanoylhexopyranosyl)hexopyranosyl]-tyrosyl-3-(2-iminoimidazolidin-4-yl)seryl-3-(3-hexopyranosyl-2-iminoimidazolidin-4-yl)-serylseryl])

The title compounds are prepared by the procedure described for example 95 using the appropriate carboxylic acid anhydride. Cylo[glycyl-β-methylphenylalanyl-O-(4-O-hexopyranosylhexopyranosyl)tyrosyl-3-(2-iminoimidazolidin-4-yl)seryl-3-[3-(6-O-heptanoylhexopyranosyl)-2-iminoimidazolidin-4-yl]seryl-seryl]: MS (+ES) *m*/*z:* 704.4 (M+2H)²⁺; Cyclo[glycyl-β-methylphenylalanyl-O-(6-O-heptanoyl-4-O-hexopyranosylhexopyranosyl)-tyrosyl-3-(2-iminoimidazolidin-4-yl)seryl-3-(3-hexopyranosyl-2-iminoimidazolidin-4-yl)-serylseryl]: MS (+ES) *m*/*z:* 704.4 (M+2H)²⁺; Cyclo[glycyl-β-methylphenylalanyl-O-[4-O-(6-O-heptanoylhexopyranosyl)hexopyranosyl]-tyrosyl-3-(2-iminoimidazolidin-4-yl)seryl-3-(3-hexopyranosyl-2-iminoimidazolidin-4-yl)-serylseryl]: MS (+ES) *m*/*z:* 704.4 (M+2H)²⁺.

### Example 98

### (Cyclo[glycyl-β-methylphenylalanyl-O-(4-O-hexopyranosylhexopyranosyl)tyrosyl-3-(2-iminoimidazolidin-4-yl)seryl-3-(3-(6-O-(phenylacetyl)hexopyranosyl-2-iminoimidazolidin-4-yl-serylseryl])

### Example 98a

### (Cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl-seryl-O-(phenylacetyl)seryl]

### Example 98b

### (Cyclo[glycyl-β-methylphenylalanyl-O-[4-O-hexopyranosyl-6-O-(phenylacetyl)hexopyranosyl]-tyrosyl-3-(2-iminoimidazolidin-4-yl)seryl-3-(3-hexopyranosyl-2-iminoimidazolidin-4-yl-serylseryl])

### Example 98c

### (Cyclo[glycyl-β-methylphenylalanyl-O-[4-O-[6-O-(phenylacetyl)hexopyranosyl]hexopyranosyl]-tyrosyl-3-(2-iminoimidazolidin-4-yl)seryl-3-(3-hexopyranosyl-2-iminoimidazolidin-4-yl)-serylseryl])

The title compounds are prepared by the procedure described for example 96 using the appropriate carboxylic acid chloride. Cyclo[glycyl-β-methylphenylalanyl-O-(4-O-hexopyranosylhexopyranosyl)tyrosyl-3-(2-iminoimidazolidin-4-yl)seryl-3-(3-(6-O-(phenylacetyl)hexopyranosyl-2-iminoimidazolidin-4-yl-serylseryl]: MS (+ES) *m*/*z:* 707.4 (M+2H)²⁺; Cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl-seryl-O-(phenylacetyl)seryl]: MS (+ES) *m*/*z:* 707.4 (M+2H)²⁺; Cyclo[glycyl-β-methylphenylalanyl-O-[4-O-hexopyranosyl-6-O-(phenylacetyl)hexopyranosyl]-tyrosyl-3-(2-iminoimidazolidin-4-yl)seryl-3-(3-hexopyranosyl-2-iminoimidazolidin-4-yl-serylseryl]: MS (+ES) *m*/*z:* 707.4 (M+2H)²⁺; Cyclo[glycyl-β-methylphenylalanyl-O-[4-O-[6-O-(phenylacetyl)hexopyranosyl]hexopyranosyl]-tyrosyl-3-(2-iminoimidazolidin-4-yl)seryl-3-(3-hexopyranosyl-2-iminoimidazolidin-4-yl)-serylseryl]: MS (+ES) *m*/*z:* 707.2 (M+2H)²⁺.

### Example 99

### (Cyclo[glycyl-β-methylphenylalanyl-O-[4-O-[6-O-(2-propylpentanoyl)hexopyranosyl]-hexopyranosyl]tyrosyl-3-(2-iminoimidazolidin-4-yl)seryl-3-[3-(6-O-(2-propylpentanoyl)-hexopyranosyl)-2-iminoimidazolidin-4-yl]serylseryl])

### Example 99a

### (Cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)seryl-O-(2-propylpentanoyl)-seryl])

### Example 99b

### (Cyclo[glycyl-β-methylphenylalanyl-O-[4-O-hexopyranosyl-6-O-(2-propylpentanoyl)hexopyranosyl]tyrosyl-3-(2-iminoimidazolidin-4-yl)seryl-3-(3-hexopyranosyl-2-iminoimidazolidin-4-yl)serylseryl])

### Example 99c

### (Cyclo[glycyl-β-methylphenylalanyl-O-[4-O-[6-O-(2-propylpentanoyl)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compounds are prepared by the procedure described for example 96 using the appropriate carboxylic acid chloride. Cylo[glycyl-β-methylphenylalanyl-O-[4-O-[6-O-(2-propylpentanoyl)hexopyranosyl]-hexopyranosyl]tyrosyl-3-(2-iminoimidazolidin-4-yl)seryl-3-[3-(6-O-(2-propylpentanoyl)-hexopyranosyl)-2-iminoimidazolidin-4-yl]serylseryl]: MS (+ES) *m*/*z:* 711.2 (M+2H)²⁺; Cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)seryl-O-(2-propylpentanoyl)-seryl]: MS (+ES) *m*/*z:* 711.2 (M+2H)²⁺; Cyclo[glycyl-β-methylphenylalanyl-O-[4-O-hexopyranosyl-6-O-(2-propylpentanoyl)hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 711.3 (M+2H)²⁺; Cyclo[glycyl-β-methylphenylalanyl-O-[4-O-[6-O-(2-propylpentanoyl)hexopyranosyl]-hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 711.3 (M+2H)²⁺.

### Example 100

### (Cyclo[glycyl-β-methylphenylalanyl-O-(4-O-hexopyranosylhexopyranosyl)tyrosyl-3-(2-iminoimidazolidin-4-yl)seryl-3-[3-(6-O-(3-cyclopentylpropanoyl)-hexopyranosyl)-2-iminoimidazolidin-4-yl]serylseryl])

### Example 100a

### (Cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)seryl-O-(3-cyclopentylpropanoyl)seryl])

### Example 100b

### (Cyclo[glycyl-β-methylphenylalanyl-O-[6-O-(3-cyclopentylpropanoyl)-4-O-hexopyranosyl-hexopyranosyl]tyrosyl-3-(2-iminoimidazolidin-4-yl)seryl-3-(3-hexopyranosyl-2-iminoimidazolidin-4-yl)serylseryl])

### Example 100c

### (Cylo[glycyl-β-methylphenylalanyl-O-[4-O-(6-O-(3-cyclopentylpropanoyl)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compounds are prepared by the procedure described for example 96 using the appropriate carboxylic acid chloride. Cyclo[glycyl-β-methylphenylalanyl-O-(4-O-hexopyranosylhexopyranosyl)tyrosyl-3-(2-iminoimidazolidin-4-yl)seryl-3-[3-(6-O-(3-cyclopentylpropanoyl)-hexopyranosyl)-2-iminoimidazolidin-4-yl]serylseryl]: MS (+ES) *m*/*z:* 710.3 (M+2H)²⁺; Cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)seryl-O-(3-cyclopentylpropanoyl)seryl]: MS (+ES) *m*/*z:* 710.3 (M+2H)²⁺; Cyclo[glycyl-β-methylphenylalanyl-O-[6-O-(3-cyclopentylpropanoyl)-4-O-hexopyranosyl-hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 710.4 (M+2H)²⁺; Cyclo[glycyl-β-methylphenylalanyl-O-[4-O-[6-O-(3-cyclopentylpropanoyl)hexopyranosyl]-hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 710.4 (M+2H)²⁺.

### Example 101

### (Cyclo[glycyl-β-methylphenylalanyl-O-[4-O-hexopyranosyl-6-O-[(phenylmethoxy)carbonyl]-hexopyranosyl]tyrosyl-3-(2-iminoimidazolidin-4-yl)seryl-3-(3-hexopyranosyl-2-iminoimidazolidin-4-yl)serylseryl])

### Example 101a

### (Cyclo[glycyl-β-methylphenylalanyl-O-[4-O-[6-O-[(phenylmethoxy)carbonyl]hexopyranosyl]-hexopyranosyl]tyrosyl-3-(2-iminoimidazolidin-4-yl)seryl-3-(3-hexopyranosyl-2-iminoimidazolidin-4-yl)serylseryl])

A stirred solution of the bis-trifluoroacetate salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (80 mg) in pyridine (3 mL) is treated with benzyl chloroformate (15 µl). After stirring at room temperature for 20 hr, additional 10 µl of benzyl chloroformate is added. The mixture is stirred for 3 days at room temperature and the volatiles are then removed *in vacuo.* The residue is triturated with ethyl acetate (3 mL), and the resulting precipitate is collected by filtration, washed with ethyl acetate, dried under vacuum to give a brown solid. The solid is then purified by reverse phase HPLC to provide the title compounds; Cyclo[glycyl-β-methylphenylalanyl-O-[4-O-hexopyranosyl-6-O-[(phenylmethoxy)carbonyl]-hexopyranosyl]tyrosyl-3-(2-iminoimidazolidin-4-yl)seryl-3-(3-hexopyranosyl-2-iminoimidazolidin-4-yl)serylseryl]: MS (+ES), *m*/*z*: 715.3 (M+2H)²⁺; Cyclo[glycyl-β-methylphenylalanyl-O-[4-O-[6-O-[(phenylmethoxy)carbonyl]hexopyranosyl]-hexopyranosyl]tyrosyl-3-(2-iminoimidazolidin-4-yl)seryl-3-(3-hexopyranosyl-2-iminoimidazolidin-4-yl)serylseryl]: MS (+ES), *m*/*z*: 715.3 (M+2H)²⁺.

### Example 102

### (Cyclo[glycyl-β-methylphenylalanyl-O-[4-O-(4,6-O-phenethylidenehexopyranosyl)-hexopyranosyl]tyrosyl-3-(2-iminoimidazolidin-4-yl)seryl-3-(3-hexopyranosyl-2-iminoimidazolidin-4-yl)serylseryl])

A stirred solution of the bis-trifluoroacetate salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (0.5 g) in N,N-dimethylformamide (35 mL) is treated with phenylacetaldehyde dimethyl acetal (0.165 g) and p-toluene sulfonic acid mono-hydrate (0.15 g) and the mixture is stirred at 60- 65°C for 12 h. The reaction mixture is then directly separated by reverse phase HPLC to provide the title compound. MS (+ES), *m*/*z*: 699.3 (M+2H)²⁺.

### Example 103

### (Cyclo[glycyl-β-methylphenylalanyl-O-[4-O-[4,6-O-(4-methoxyphenethylidene)hexopyranosyl]-hexopryranosyl]tyrosyl-3-(2-iminoimidazolidin-4-yl)seryl-3-(3-hexopyranosyl-2-iminoimidazolidin-4-yl)serylseryl])

A stirred solution of the bis-hydrochloride salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (0.5 g) in N,N-dimethylformamide (5 mL) and dimethyl sulfoxide (5 mL) is treated with 4-methoxyphenylacetaldehyde dimethyl acetal (0.215 g) and p-toluene sulfonic acid mono-hydrate (0.15 g) and the mixture is stirred at 60- 65°C for 12 h. The reaction mixture is then directly separated by reverse phase HPLC to provide the title compound. MS (+ES), *m*/*z*: 714.4 (M+2H)²⁺.

### Example 104

### (Cyclo[glycyl-β-methylphenylalanyl-O-[4-O-(4,6-O-benzylidenehexopyranosyl)hexopyranosyl]-tyrosyl-3-(2-iminoimidazolidin-4-yl)seryl-3-(3-hexopyranosyl-2-iminoimidazolidin-4-yl)-serylseryl])

### Example 104a

### (Cyclo[glycyl-β-methylphenylalanyl-O-[4-O-(2,3-O-benzylidenehexopyranosyl)hexopyranosyl]-tyrosyl-3-(2-iminoimidazolidin-4-yl)seryl-3-(3-hexopyranosyl-2-iminoimidazolidin-4-yl)-serylseryl])

### Example 104b

### (Cyclo[glycyl-β-methylphenylalanyl-O-[4-O-(2,3-O-benzylidenehexopyranosyl)hexopyranosyl]-tyrosyl-3-(2-iminoimidazolidin-4-yl)seryl-3-(3-hexopyranosyl-2-iminoimidazolidin-4-yl)-serylseryl])

A stirred solution of the bis-hydrochloride salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (20.9g, 15.2 mmol) in dimethylsufloxide (100 mL) is treated with benzaldehyde dimethyl acetal (3.7 mL) and hydrogen chloride (1 mL of 4.0M solution in 1,4-dioxane) and the mixture is heated at 50°C for 24 h. The reaction mixture is cooled to room temperature and acetonitrile (300 mL) is added to precipitate the crude products. The precipitate is collected by filtration, washed with acetonitrile (3 x 100 mL), and dried. The products are then purified by reverse phase HPLC. Cyclo[glycyl-β-methylphenylalanyl-O-[4-O-(4,6-O-benzylidenehexopyranosyl)hexopyranosyl]-tyrosyl-3-(2-iminoimidazolidin-4-yl)seryl-3-(3-hexopyranosyl-2-iminoimidazolidin-4-yl)-serylseryl]: MS (+ES), *m*/*z*: 692.4 (M+2H)²⁺; Cyclo[glycyl-β-methylphenylalanyl-O-[4-O-(2,3-O-benzylidenehexopyranosyl)hexopyranosyl]-tyrosyl-3-(2-iminoimidazolidin-4-yl)seryl-3-(3-hexopyranosyl-2-iminoimidazolidin-4-yl)-serylseryl]: MS (+ES), *m*/*z*: 692.4 (M+2H)²⁺; Cyclo[glycyl-β-methylphenylalanyl-O-[4-O-(2,3-O-benzylidenehexopyranosyl)hexopyranosyl]-tyrosyl-3-(2-iminoimidazolidin-4-yl)seryl-3-(3-hexopyranosyl-2-iminoimidazolidin-4-yl)-serylseryl]: MS (+ES), *m*/*z*: 692.4 (M+2H)²⁺.

### Example 105

### (Cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

A stirred solution of the bis-hydrochloride salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (30.5 g, 22.3 mmol) in dimethyl sulfoxide (110 mL) is treated with adamantanone dimethyl ketal (8.0 g) and hydrogen chloride (1.6 mL of 4.0 M in 1,4-dioxane). The mixture is heated at 50°C for 1 h before 10 mL of DMSO is removed *in vacuo.* The resulting solution is directly subjected to purification by reverse phase HPLC. MS (+ES), *m*/*z*: 714.4 (M+2H)²⁺.

### Example 106

### (Cyclo[3-cyclohexylalanyl-O-[4-O-[4,6-O-(2-adamantylidene)hexopyranosyl]hexopyranosyl]-tyrosyl-3-(2-iminoimidazolidin-4-yl)seryl-3-(3-hexopyranosyl-2-iminoimidazolidin-4-yl)-serylserylglycyl])

### Example 106a

### (Cyclo[3-cyclohexylalanyl-O-[4-O-[2,3-O-(2-adamantylidene)hexopyranosyl]hexopyranosyl]-tyrosyl-3-(2-iminoimidazolidin-4-yl)seryl-3-(3-hexopyranosyl-2-iminoimidazolidin-4-yl)-serylserylglycyl])

### Example 106b

### (Cyclo[3-cyclohexylalanyl-O-[4-O-[2,3:4,6-di-O-(2-adamantylidene)hexopyranosyl]hexopyranosyl]tyrosyl-3-(2-iminoimidazolidin-4-yl)seryl-3-(3-hexopyranosyl-2-iminoimidazolidin-4-yl)-serylserylglycyl])

A stirred solution of the bis-acetate salt of cyclo[3-cyclohexylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylserylglycyl] (8.2 g) in dimethyl sulfoxide (110 mL) is treated with adamantanone dimethyl ketal (3.0 g) and hydrogen chloride (5 mL of 4.0 M in 1,4-dioxane). The mixture is heated at 50°C for 2 h and then cooled and neutralized (pH paper) by the addition of N,N-diisopropylethylamine (1 mL). Acetonitrile (400 mL) is added to precipitate the crude products, which are collected by filtration and washed with acetonitrile, diethyl ether, and air dried. The resulting solids are subjected to purification by reverse phase HPLC. Cyclo[3-cyclohexylalanyl-O-[4-O-[4,6-O-(2-adamantylidene)hexopyranosyl]hexopyranosyl]-tyrosyl-3-(2-iminoimidazolidin-4-yl)seryl-3-(3-hexopyranosyl-2-iminoimidazolidin-4-yl)-serylserylglycyl]: MS (+ES), *m*/*z:* 710.6 (M+2H)²⁺; Cyclo[3-cyclohexylalanyl-O-[4-O-[2,3-O-(2-adamantylidene)hexopyranosyl]hexopyranosyl]-tyrosyl-3-(2-iminoimidazolidin-4-yl)seryl-3-(3-hexopyranosyl-2-iminoimidazolidin-4-yl)-serylserylglycyl]: MS (+ES), *m*/*z*: 710.6 (M+2H)²⁺; Cyclo[3-cyclohexylalanyl-O-[4-O-[2,3:4,6-di-O-(2-adamantylidene)hexopyranosyl]hexopyranosyl]tyrosyl-3-(2-iminoimidazolidin-4-yl)seryl-3-(3-hexopyranosyl-2-iminoimidazolidin-4-yl)-serylserylglycyl]: MS (+ES), *m*/*z*: 776.5 (M+2H)²⁺.

### Example 107

### (Cyclo[glycyl-β-methylphenylalanyl-O-[4-O-[4,6-O-(3-methylbutylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 102 using the appropriate dimethyl acetal. MS (+ES) *m*/*z*: 682.2 (M+2H)²⁺.

### Example 108

### (Cyclo[glycyl-β-methylphenylalanyl-O-[4-O-(4,6-O-hexylidenehexopyranosyl)hexopyranosyl]-tyrosyl-3-(2-iminoimidazolidin-4-yl)seryl-3-(3-hexopyranosyl-2-iminoimidazolidin-4-yl)-serylseryl])

The title compound is prepared by the procedure described for example 102 using the appropriate dimethyl acetal. MS (+ES) *m*/*z*: 689.4 (M+2H)²⁺.

### Example 109

### (Cyclo[glycyl-β-methylphenylalanyl-O-[4-O-(4,6-O-octylidenehexo-pyranosyl)hexopyranosyl]-tyrosyl-3-(2-iminoimidazolidin-4-yl)seryl-3-(3-hexopyranosyl-2-iminoimidazolidin-4-yl)-serylseryl])

The title compound is prepared by the procedure described for example 102 using the appropriate dimethyl acetal. MS (+ES) *m*/*z:* 703.3 (M+2H)²⁺.

### Example 110

### (Cyclo[glycyl-β-methylphenylalanyl-O-[4-O-[4,6-O-(3,3-dimethylbutylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 102 using the appropriate dimethyl acetal. MS (+ES) *m*/*z*: 689.1 (M+2H)²⁺.

### Example 111

### (Cyclo[glycyl-β-methylphenylalanyl-O-[4-O-[4,6-O-(2-methylpentylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 102 using the appropriate dimethyl acetal. MS (+ES) *m*/*z:* 689.3 (M+2H)²⁺.

### Example 112

### (Cvclo[glycyl-β-methylphenylalanyl-O-[4-O-[4,6-O-(cylohexylmethylene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 102 using the appropriate dimethyl acetal. MS (+ES) *m*/*z*: 695.3 (M+2H)²⁺.

### Example 113

### (Cyclo[glycyl-β-methylphenylalanyl-O-[4-O-[4,6-O-[2-[1-[(Rhenylmethoxy)carbonyl]piperidin-4-yl]ethylidene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 102 using the appropriate dimethyl acetal. MS (+ES) *m*/*z*: 769.8 (M+2H)²⁺.

### Example 114

### (Cyclo[glycyl-β-methylphenylalanyl-O-[4-O-[4,6-O-(4-butoxy-4-oxobutylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 102 using the appropriate di-n-butyl acetal. MS (+ES) *m*/*z*: 718.4 (M+2H)²⁺.

### Example 115

### (Cyclo[glycyl-β-methylphenylalanyl-O-[4-O-[4,6-O-(cyclohex-3-en-1-ylmethylene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 102 using the appropriate dimethyl acetal. MS (+ES) *m*/*z*: 694.4 (M+2H)²⁺.

### Example 116

### (Cyclo[glycyl-β-methylphenylalanyl-O-[4-O-[4,6-O-(2-ethylbutylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 102 using the appropriate dimethyl acetal. MS (+ES) *m*/*z*: 689.4 (M+2H)²⁺.

### Example 117

### (Cyclo[glycyl-β-methylphenylalanyl-O-[4-O-[4,6-O-(cyclopentylmethylene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 102 using the appropriate dimethyl acetal. MS (+ES) *m*/*z*: 688.3 (M+2H)²⁺.

### Example 118

### (Cyclo[glycyl-β-methylphenylalanyl-O-[4-O-[4,6-O-(4-bromophenethylidene)hexopyranosyl]-hexopyranosyl]tyrosyl-3-(2-iminoimidazolidin-4-yl)seryl-3-(3-hexpyranosyl-2-iminoimidazolidin-4-yl)serylseryl])

The title compound is prepared by the procedure described for example 103 using the appropriate dimethyl acetal. MS (+ES) *m*/*z:* 738.8 (M+2H)²⁺.

### Example 119

### (Cyclo[glycyl-β-methylphenylalanyl-O-[4-O-[4,6-O-(4-methylphenethylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 103 using the appropriate dimethyl acetal. MS (+ES) *m*/*z*: 706.3 (M+2H)²⁺.

### Example 120

### (Cyclo[glycyl-β-methylphenylalanyl-O-[4-O-[4,6-O-(4-chlorophenethylidene)hexopyranosyl]-hexopyranosyl]tyrosyl-3-(2-iminoimidazolidin-4-yl)seryl-3-(3-hexopylanosyl-2-iminoimidazolidin-4-yl)serylseryl])

The title compound is prepared by the procedure described for example 103 using the appropriate dimethyl acetal. MS (+ES) *m*/*z:* 716.4 (M+2H)²⁺.

### Example 121

### (Cyclo[glycyl-β-methylphenylalanyl-O-[4-O-[4,6-O-(4-fluorophenethylidene)hexopyranosyl]-hexolpyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 103 using the appropriate dimethyl acetal. MS (+ES) *m*/*z:* 708.3 (M+2H)²⁺.

### Example 122

### (Cyclo[glycyl-β-methylphenylalanyl-O-[4-O-[4,6-O-(2-cyclohexylethylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 702 (M+2H)²⁺.

### Example 123

### (Cyclo[glycyl-β-methylphenylalanyl-O-[4-O-[4,6-O-(5-methylhex-4-en-1-ylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 695.3 (M+2H)²⁺.

### Example 124

### (Cyclo[glycyl-β-methylphenylalanyl-O-[4-O-[4,6-O-(3-phenylpropylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 706.3 (M+2H)²⁺.

### Example 125

### (Cyclo[glycyl-β-methylphenylalanyl-O-[4-O-[4,6-O-(2,6-dimethylhept-5-en-1-yidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 709 (M+2H)²⁺.

### Example 126

### (Cyclo[glycyl-β-methylphenylalanyl-O-[4-O-[4,6-O-(3,7-dimethyloct-6-en-1-ylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 716.1 (M+2H)²⁺.

### Example 127

### (Cyclo[glycyl-β-methylphenlalanyl-O-[4-O-[4,6-O-(1-adamantylmethylene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 721.4 (M+2H)²⁺.

### Example 128

### (Cyclo[glycyl-β-methylphenylalanyl-O-[4-O-[2,3-O-[2-(1-adamantyl)ethylidene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 728.4 (M+2H)²⁺.

### Example 129

### (Cyclo[glycyl-β-methylphenylalanyl-O-[4-O-[4,6-O-[2-(1-adamantyl)ethylidene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 728.8 (M+2H)²⁺.

### Example 130

### (Cyclo[glycyl-β-methylphenylalanyl-O-[4-O-[4,6-O-(3-methoxycholan-24-ylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 826.4 (M+2H)²⁺.

### Example 131

### (Cyclo[glycyl-β-methylphenylalanyl-O-[4-O-(4,6-O-(4-phenylbenzylidene)-hexopyranosyl)hexopyranosyl]-tyrosyl-3-(2-iminoimidazolidin-4-yl)seryl-3-(3-hexopyranosyl-2-iminoimidazolidin-4-yl)-serylseryl])

The title compound is prepared by the procedure described for example 104 using the appropriate dimethyl acetal. MS (+ES) *m*/*z:* 690.2 (M+2H)²⁺.

### Example 132

### (Cyclo[glycyl-β-methylphenylalanyl-O-[4-O-[4,6-O-(2-thienylmethylene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 696 (M+2H)²⁺.

### Example 133

### (Cyclo[glycyl-β-methylphenylalanyl-O-[4-O-[4,6-O-(3-thienylmethylene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 695 (M+2H)²⁺.

### Example 134

### (Cyclo[glycyl-β-methylphenylalanyl-O-[4-O-[4,6-O-(4-methylbenzylidene)hexopyranosyl]-hexopyranosyl]-tyrosyl-3-(2-iminoimidazolidin-4-yl)seryl-3-(3-hexopyranosyl-2-iminoimidazolidin-4-yl)-serylseryl])

The title compound is prepared by the procedure described for example 104 using the appropriate dimethyl acetal. MS (+ES) *m*/*z:* 699.2 (M+2H)²⁺.

### Example 135

### (Cyclo[glycyl-β-methylphenylalanyl-O-[4-O-[4,6-O-(3-methylbenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 699 (M+2H)²⁺.

### Example 136

### (Cyclo[glycyl-β-methylphenylalanyl-O-[4-O-[4,6-O-(3-hydroxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 700 (M+2H)²⁺.

### Example 137

### (Cyclo[glycyl-β-methylphenylalanyl-O-[4-O-[4,6-O-(3-methoxybenzylidene)hexopyranosyl]-hexopyranosyl]tyrosyl-2-(2-iminoimidazolidin-4-yl)seryl-3-(3-hexopyranosyl-2-iminoimidazolidin-4-yl)serylseryl])

### Example 137a

### (Cyclo[glycyl-β-methylphenylalanyl-O-[4-O-[2,3-O-(3-methoxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

### Example 137b

### (Cyclo[glycyl-β-methylphenylalanyl-O-[4-O-[2,3-O-(3-methoxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compounds are prepared by the procedure described for example 104 using the appropriate dimethyl acetal. Cyclo[glycyl-β-methylphenylalanyl-O-[4-O-[4,6-O-(3-methoxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 707.3 (M+2H)²⁺; Cyclo[glycyl-β-methylphenylalanyl-O-[4-O-[2,3-O-(3-methoxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 707.3 (M+2H)²⁺; Cyclo[glycyl-β-methylphenylalanyl-O-[4-O-[2,3-O-(3-methoxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 707.3 (M+2H)²⁺.

### Example 138

### (Cyclo[glycyl-β-methylphenylalanyl-O-[4-O-[4,6-O-(3-chlorobenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 709.2 (M+2H)²⁺.

### Example 139

### (Cyclo[glycyl-β-methylphenylalanyl-O-[4-O-[4,6-O-(4-isopropylbenzylidene)hexopyranosyl]-hexopyranosyl]tyrosyl-3-(2-iminoimidazolidin-4-yl)seryl-3-(3-hexopyranosyl-2-iminoimidazolidin-4-yl)serylsery])

The title compound is prepared by the procedure described for example 104 using the appropriate dimethyl acetal. MS (+ES) *m*/*z:* 713.4 (M+2H)²⁺.

### Example 140

### (Cyclo[glycyl-β-methylphenylalanyl-O-[4-O-[4,6-O-(4-propylbenzylidene)hexopyranosyl]-hexopyranosyl]tyrosyl-3-(2-iminoimidazolidin-4-yl)seryl-3-(3-hexopylanosyl-2-iminoimidazolidin-4-yl)serylseryl])

The title compound is prepared by the procedure described for example 104 using the appropriate dimethyl acetal. MS (+ES) *m*/*z:* 713.3 (M+2H)²⁺.

### Example 141

### (Cyclo[glycyl-β-methylphenylalanyl-O-[4-O-[4,6-O-(4-carboxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 714.6 (M+2H)²⁺.

### Example 142

### (Cyclo[glycyl-β-methylphenylalanyl-O-[4-O-[4,6-O-(4-ethoxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 714.3 (M+2H)²⁺.

### Example 143

### (Cyclo[glycyl-β-methylphenylalanyl-O-[4-O-[4,6-O-(1,3-benzodioxol-5-ylmethylene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 714.3 (M+2H)²⁺.

### Example 144

### (Cyclo[glycyl-β-methylphenylalanyl-O-[4-O-[4,6-O-[4-(methylthio)benzylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 715 (M+2H)²⁺.

### Example 145

### (Cyclo[glycyl-β-methylphenylalanyl-O-[4-O-[4,6-O-(2-nahthylmethylene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 717.3 (M+2H)²⁺.

### Example 146

### (Cyclo[glycyl-β-methylphenylalanyl-O-[4-O-[4,6-O-[3-(2-methylprop-1-en-1-yl)benzylidene]-hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 719.2 (M+2H)²⁺.

### Example 147

### (Cyclo[glycyl-β-methylphenylalanyl-O-[4-O-[4,6-O-(4-tert-butylbenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 720.4 (M+2H)²⁺.

### Example 148

### (Cyclo[glycyl-β-methylphenylalanyl-O-[4-O-[4,6-O-(4-propyloxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 721.3 (M+2H)²⁺.

### Example 149

### (Cyclo[glycyl-β-methylphenylalanyl-O-[4-O-[4,6-O-[(2,3-dihydro-1,4-benzodioxan-6-yl)-methylene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 721 (M+2H)²⁺.

### Example 150

### (Cyclo[glycyl-β-methylphenylalanyl-O-[4-O-[4,6-O-(3,5-dimethoxybenzylidene)hexopyranosyl]-hexopyranosyl]tyrosyl-3-(2-iminoimidazolidin-4-yl)seryl-3-(3-hexopylanosyl-2-iminoimidazolidin-4-yl)serylseryl])

### Example 150a

### (Cyclo[glycyl-β-methylphenylalanyl-O-[4-O-[2,3-O-(3,5-dimethoxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compounds are prepared by the procedure described for example 104 using the appropriate dimethyl acetal. Cyclo[glycyl-β-methylphenylalanyl-O-[4-O-[4,6-O-(3,5-dimethoxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 722.3 (M+2H)²⁺; Cyclo[glycyl-β-methylphenylalanyl-O-[4-O-[2,3-O-(3,5-dimethoxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 722.3 (M+2H)²⁺.

### Example 151

### (Cyclo[glycyl-β-methylphenylalanyl-O-[4-O-[4,6-O-(3-methyl-4-nitrobenzylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 722.5 (M+2H)²⁺.

### Example 152

### (Cyclo[glycyl-β-methylphenylalanyl-O-[4-O-[4,6-O-(3-ethoxy-4-methoxybenzylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 729.2 (M+2H)²⁺.

### Example 153

### (Cyclo[glycyl-β-methylphenylalanyl-O-[4-O-[4,6-O-(3-methoxy-4-nitrobenzylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 729.5 (M+2H)²⁺.

### Example 154

### (Cyclo[glycyl-β-methylphenylalanyl-O-[4-O-[4,6-O-(3-phenylbenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 730.2 (M+2H)²⁺.

### Example 155

### (Cyclo[glycyl-β-methylphenylalanyl-O-(4-O-hexopyranosylhexopyranosyl)tyrosyl-3-[2-[(2,2-dimethylpropanoyl)imino]imidazolidin-4-yl]seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 730.3 (M+2H)²⁺.

### Example 156

### (Cyclo[glycyl-β-methylphenylalanyl-O-[4-O-[4,6-O-[3-(3-pyridyl)benzylidene]hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 730.8 (M+2H)²⁺.

### Example 157

### (Cyclo[glycyl-β-methylphenylalanyl-O-[4-O-[4,6-O-[4-(methylsulfonyl)benzylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 731 (M+2H)²⁺.

### Example 158

### (Cyclo[glycyl-β-methylphenylalanyl-O-[4-O-[4,6-O-[(6-chloro-1,3-benzodioxol-5-yl)-methylene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 731.2 (M+2H)²⁺.

### Example 159

### (Cyclo[glycyl-β-methylphenylalanyl-O-[4-O-[4,6-O-[(6-methoxy-2-naphthyl)-methylene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 732.3 (M+2H)²⁺.

### Example 160

### (Cyclo[glycyl-β-methylphenylalanyl-O-[4-O-[2,3-O-[(6-methoxy-2-naphthyl)-methylene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 732.5 (M+2H)²⁺.

### Example 161

### (Cyclo[glycyl-β-methylphenylalanyl-O-[4-O-[4,6-O-[1-(acetylindol-3-yl)methylene]hexopyranosy]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 732.8 (M+2H)²⁺.

### Example 162

### (Cyclo[glycyl-β-methylphenylalanyl-O-[4-O-[4,6-O-[3-(3-thienyl)benzylidene]hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 733.3 (M+2H)²⁺.

### Example 163

### (Cyclo[glycyl-β-methylphenylalanyl-O-[4-O-[4,6-O-[4-(2-thienyl)benzylidene]hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 733.3 (M+2H)²⁺.

### Example 164

### (Cyclo[glycyl-β-methylphenylalanyl-O-[4-O-[4,6-O-[4-(3-thienyl)benzylidene]hexopyranosyl]-hexopyranosyl]tyrosyl-3-(2-iminoimidazolidin-4-yl)seryl-3-(3-hexopyranosyl-2-iminoimidazolidin-4-yl)serylseryl])

### Example 164a

### (Cyclo[glycyl-β-methylphenylalanyl-O-[4-O-[2,3-O-[4-(3-thienyl)benzylidene]hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compounds are prepared by the procedure described for example 104 using the appropriate dimethyl acetal. Cyclo[glycyl-β-methylphenylalanyl-O-[4-O-[4,6-O-[4-(3-thienyl)benzylidene]hexopyranosyl]-hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 733.3 (M+2H)²⁺; Cyclo[glycyl-β-methylphenylalanyl-O-[4-O-[2,3-O-[4-(3-thienyl)benzylidene]hexopyranosyl]-hexopyranosyl]tyrosyl-3-(2-iminoimidazolidin-4-yl)seryl-3-(3-hexopyranosyl-2-iminoimidazolidin-4-yl)serylseryl]: MS (+ES) m/*z:* 733.3 (M+2H)²⁺.

### Example 165

### (Cyclo[glycyl-β-methylphenylalanyl-O-[4-O-[4,6-O-[3-[(3-methylbut-2-en-1-yl)oxyl-benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 734 (M+2H)²⁺.

### Example 166

### (Cyclo[glycyl-β-methylphenylalanyl-O-[4-O-[4,6-O-[3-(carboxymethoxy)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 736.2 (M+2H)²⁺.

### Example 167

### (Cyclo[glycyl-β-methylphenylalanyl-O-[4-O-[4,6-O-(3-ethoxy-4-nitrobenzylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 736.8 (M+2H)²⁺.

### Example 168

### (Cyclo[glycyl-β-methylphenylalanyl-O-[4-O-[4,6-O-[4-(methylthio)-3-nitrobenzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 737.73 (M+2H)²⁺.

### Example 169

### (Cyclo[glycyl-β-methylphenylalanyl-O-[4-O-[4,6-O-(4-hydroxy-3-methoxy-4-nitrobenzylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 737.5 (M+2H)²⁺.

### Example 170

### (Cyclo[glycyl-β-methylphenylalanyl-O-[4-O-[4,6-O-(3-phenoxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 738 (M+2H)²⁺.

### Example 171

### (Cyclo[glycyl-β-methylphenylalanyl-O-[4-O-[4,6-O-(4-phenoxybenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 738.3 (M+2H)²⁺.

### Example 172

### (Cyclo[glycyl-β-methylphenylalanyl-O-[4-O-[4,6-O-[4-(2-phenylethenyl)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 743.4 (M+2H)²⁺.

### Example 173

### (Cyclo[glycyl-β-methylphenylalanyl-O-[4-O-[4,6-O-(4-benzoylbenzylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 744.2 (M+2H)²⁺.

### Example 174

### (Cyclo[glycyl-β-methylphenylalanyl-O-[4-O-[4,6-O-[4-(4-methylphenoxy)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 745 (M+2H)²⁺.

### Example 175

### (Cyclo[glycyl-β-methylphenylalanyl-O-[4-O-[4,6-O-[3-(phenylmethoxy)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 745 (M+2H)²⁺.

### Example 176

### (Cyclo[glycyl-β-methylphenylalanyl-O-[4-O-[4,6-O-[4-(phenylmethoxy)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 745.3 (M+2H)²⁺.

### Example 177

### (Cyclo[glycyl-β-methylphenylalanyl-O-[4-O-[4,6-O-[3-(4-chlorophenyl)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 747.3 (M+2H)²⁺.

### Example 178

### (Cyclo[glycyl-β-methylphenylalanyl-O-[4-O-[4,6-O-[4-(4-chlorophenyl)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 747.3 (M+2H)²⁺.

### Example 179

### (Cyclo[glycyl-β-methylphenylalanyl-O-[4-O-[4,6-O-[3-(1,1,2,2-tetrafluoroethoxy)benzylidene]-hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 750 (M+2H)²⁺.

### Example 180

### (Cyclo[glycyl-β-methylphenylalanyl-O-[4-O-[4,6-O-[3-(4-methoxyphenoxy)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 753 (M+2H)²⁺.

### Example 181

### (Cyclo[glycyl-β-methylphenylalanyl-O-[4-O-[4,6-O-[4-(4-tert-butylphenoxy)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 766 (M+2H)²⁺.

### Example 182

### (Cyclo[glycyl-β-methylphenylalanyl-O-[4-O-[4,6-O-[3-[(4-nitrophenyl)methoxy]benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 768.1 (M+2H)²⁺.

### Example 183

### (Cyclo[glycyl-β-methylphenylalanyl-O-[4-O-[4,6-O-[4-[(3,7-dimethylocta-2,6-dien-1-yl)oxy]-benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 768 (M+2H)²⁺.

### Example 184

### (Cyclo[glycyl-β-methylphenylalanyl-O-[4-O-[4,6-O-[3-[3-(trifluoromethyl)phenoxy]-benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 772 (M+2H)²⁺.

### Example 185

### (Cyclo[glycyl-β-methylphenylalanyl-O-[4-O-[4,6-O-[[3-(3,7,11-trimethyldodeca-2,6,10-trien-1-yl)oxy]benzylidene]hexopyranosyl]hexopyranosyl]tyrosyl-3-(2-iminoimidazolidin-4-yl)-seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 802 (M+2H)²⁺.

### Example 186

### (Cyclo[glycyl-β-methylphenylalanyl-O-[4-O-[4,6-O-[4-(4-iodobenzoyl)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 807.2 (M+2H)²⁺.

### Example 187

### (Cyclo[glycyl-β-methylphenylalanyl-O-[4-O-[4,6-O-[4-(3,4,5-triiodobenzoyl)benzylidene]-hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 933.4 (M+2H)²⁺.

### Example 188

### (Cyclo[glycyl-β-methylphenylalanyl-O-[4-O-[4,6-O-(3,3-diphenylprop-2-enylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 743.2 (M+2H)²⁺.

### Example 189

### (Cyclo[glycyl-β-methylphenylalanyl-O-[4-O-[4,6-O-(prop-2-enylidene)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl acetal. MS (+ES) *m*/*z:* 667.4 (M+2H)²⁺.

### Example 190

### (Cyclor3-(2-iminoimidazolidin-4-yl)alanyl-3-(3-hexopyranosyl-2-iminoimidazolidin-4-yl)-alanylserylglycyl-β-methylphenylalanyl-O-[4-O-[4,6-O-[(6-methoxy-2-naphthyl)-methylenelhexopyranosyl]hexopyranosyl]tyrosyl])

The title compound is prepared from cyclo[3-(2-iminoimidazolidin-4-yl)alanyl-3-(3-hexopyranosyl-2-iminoimidazolidin-4-yl)-alanylserylglycyl-β-methylphenylalanyl-O-(4-0-hexopyranosylhexopyranosyl)tyrosyl] by the procedure described for example 104 using the appropriate dimethyl acetal. MS (+ES) *m*/*z:* 716.3 (M+2H)²⁺.

### Example 191

### (Cyclo[3-(2-iminoimidazolidin-4-yl)alanyl-3-(3-hexopyranosyl-2-iminoimidazolidin-4-yl)-alanylserylglycyl-β-methylhenylalanyl-O-[4-O-[4,6-O-[4-(phenylmethoxy)benzylidene]-hexopyranosyl]hexopyranosyl]tyrosyl])

The title compound is prepared from cyclo[3-(2-iminoimidazolidin-4-yl)alanyl-3-(3-hexopyranosyl-2-imnoimidazolidin-4-yl)-alanylserylglycyl-β-methylphenylalanyl-O-(4-O-hexopyranosylhexopyranosyl)tyrosyl] by the procedure described for example 104 using the appropriate dimethyl acetal. MS (+ES) *m*/*z:* 729.6 (M+2H)²⁺.

### Example 192

### (Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[4,6-O-[3-(4-methoxyphenoxy)benzylidene]-hexopyranosyl]hexopyranosyl]tyrosyl])

The title compound is prepared from cyclo[3-(2-iminoimidazolidin-4-yl)alanyl-3-(3-hexopyranosyl-2-iminoimidazolidin-4-yl)-alanylserylglycyl-β-methylphenylalanyl-O-(4-O-hexopyranosylhexopyranosyl)tyrosyl] by the procedure described for example 104 using the appropriate dimethyl acetal. MS (+ES) *m*/*z:* 737.4 (M+2H)²⁺.

### Example 193

### (Cyclo[glycyl-4-chloro-β-methylphenylalanyl-O-[4-O-[4,6-O-(cyclohexylmethylene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared from cyclo[glycyl-4-chloro-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] by the procedure described for example 104 using the appropriate dimethyl acetal. MS (+ES) *m*/*z:* 712.3 (M+2H)²⁺.

### Example 194

### (Cyclo[glycyl-4-chloro-β-methylphenylalanyl0O-[4-O-[4,6-O-(2-methylpentylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared from cyclo[glycyl-4-chloro-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] by the procedure described for example 104 using the appropriate dimethyl acetal. MS (+ES) *m*/*z:* 706.2 (M+2H)²⁺.

### Example 195

### (Cyclo[glycyl-4-chloro-β-methylphenylalanyl-O-[4-O-(4,6-O-benzylidenehexopyranosyl)-hexopyranosyl]tyrosyl-3-(2-iminoimidazolidin-4-yl)seryl-3-(3-hexopyranosyl-2-iminoimidazolidin-4-yl)serylseryl])

The title compound is prepared from cyclo[glycyl-4-chloro-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] by the procedure described for example 104 using the appropriate dimethyl acetal. MS (+ES) *m*/*z:* 709.5 (M+2H)²⁺.

### Example 196

### (Cyclo[glycyl-β-methylphenylalanyl-O-[4-O-(4,6-O-benzylidenehexopyranosyl)hexopyranosyl]-3-iodotyrosyl-3-(2-iminoimidazolidin-4-yl)seryl-3-(3-hexopyranosyl-2-iminoimidazolidin-4-yl)-serylseryl])

The title compound is prepared from cyclo[glycyl-β-methylphenylalanyl-O-(4-O-hexopyranosylhexopyranosyl)-3-iodo-tyrosyl-3-(2-iminoimidazolidin-4-yl)seryl-3-(3-hexopyranosyl-2-iminoimidazolidin-4-yl)serylseryl] by the procedure described for example 104 using the appropriate dimethyl acetal. MS (+ES) *m*/*z:* 755 (M+2H)²⁺.

### Example 197

### (Cyclo[glycyl-β-methylphenylalanyl-O-[4-O-[4,6-O-[3-(4-methylphenoxy)benzylidene]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared from cyclo[glycyl-β-methylphenylalanyl-O-(4-O-hexopyranosylhexopyranosyl)-3-iodo-tyrosyl-3-(2-iminoimidazolidin-4-yl)seryl-3-(3-hexopyranosyl-2-iminoimidazolidin-4-yl)serylseryl] by the procedure described for example 104 using the appropriate dimethyl acetal. MS (+ES) *m*/*z:* 808 (M+2H)²⁺.

### Example 198

### (Cyclo[3-cyclohexylalanyl-O-[4-O-[4,6-O-(3-phenylpropylidene)hexopyranosyl]hexopyranosyl]tyrosyl-3-(2-iminoimidazolidin-4-yl)seryl-3-(3-hexopyranosyl-2-iminoimidazolidin-4-yl)serylserylglycyl]

The title compound is prepared from cyclo[3-cyclohexylalanyl-O-(4-O-hexopyranosylhexopyranosyl)tyrosyl-3-(2-iminoimidazolidin-4-yl)seryl-3-(3-hexopyranosyl-2-imnoimidazolidin-4-yl)serylserylglycyl] by the procedure described for example 104 using the appropriate dimethyl acetal. MS (+ES) *m*/*z:* 702.3 (M+2H)²⁺.

### Example 199

### (Cyclo[3-cyclohexylalanyl-O-[4-O-(4,6-O-benzylidenehexopyranosyl)hexopyranosyl]tyrosyl-3-(2-iminoimidazolidin-4-yl)seryl-3-(3-hexonyranosyl-2-iminoimidazolidin-4-yl)-seryl-serylglycyl])

The title compound is prepared from cyclo[3-cyclohexylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylserylglycyl] by the procedure described for example 104 using the appropriate dimethyl acetal. MS (+ES) *m*/*z:* 688.1 (M+2H)²⁺.

### Example 200

### (Cyclo[3-cyclohexylalanyl-O-[4-O-[4,6-O-phenethylidene-hexopyranosyl]hexopyranosyl]tyrosyl-3-(2-iminoimidazolidin-4-yl)seryl-3-(3-hexopyranosyl-2-iminoimidazolidin-4-yl)serylserylglycyl])

The title compound is prepared from cyclo[3-cyclohexylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylserylglycyl] by the procedure described for example 104 using the appropriate dimethyl acetal. MS (+ES) *m*/*z:* 695.2 (M+2H)²⁺.

### Example 201

### (Cyclo[3-cyclohexyl-2-aminobutanoyl-O-[4-O-[4,6-O-benzylidenehexopyranosyl]hexopyranosyl]tyrosyl-3-(2-iminoimidazolidin-4-yl)seryl-3-(3-hexopyranosyl-2-iminoimidazolidin-4-yl)serylserylglycyl])

The title compound is prepared from cyclo[3-cyclohexyl-2-aminobutanoyl-O-(4-O-hexopyranosylhexopyranosyl)tyrosyl-3-(2-iminoimidazolidin-4-yl)seryl-3-(3-hexopyranosyl-2-iminoimidazolidin-4-yl)-serylserylglycyl] by the procedure described for example 104 using the appropriate dimethyl acetal. MS (+ES) *m*/*z:* 695.1 (M+2H)²⁺.

### Example 202

### (Cyclo[glycyl-4-chloro-β-methylphenylalanyl-O-[4-O-(4,6-O-cyclopentylidenehexopyranosyl)-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl ketal. MS (+ES) *m*/*z:* 681.2 (M+2H)²⁺.

### Example 203

### (Cyclo[glycyl-β-methylphenylalanyl-O-[4-O-(4,6-O-cyclohexylidene-4-O-hexopyranosyl)hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl ketal. MS (+ES) *m*/*z:* 688.2 (M+2H)²⁺.

### Example 204

### (Cyclo[glycyl-β-methylphenylalanyl-O-[4-O-[4,6-O-(2-methylcyclohexylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl ketal. MS (+ES) *m*/*z:* 695.3 (M+2H)²⁺.

### Example 205

### (Cyclo[glycyl-β-methylphenylalanyl-O-[4-O-[4,6-O-(2,2-dimethylcyclohexylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl ketal. MS (+ES) *m*/*z:* 702.3 (M+2H)²⁺.

### Example 206

### (Cyclo[glycyl-β-methylphenylalanyl-O-[4-O-[4,6-O-(2,6-dimethylcylohexylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl ketal. MS (+ES) *m*/*z:* 702.4 (M+2H)²⁺.

### Example 207

### (Cyclo[glycyl-β-methylphenylalanyl-O-[4-O-[4,6-O-(3,3,5,5-tetramethylcyclohexylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl ketal. MS (+ES) *m*/*z:* 716.3 (M+2H)²⁺.

### Example 208

### (Cyclo[glycyl-β-methylphenylalanyl-O-[4-O-[4,6-O-(4-tert-butylcylohexylidene)hexopyranosyl]hexopyranosyl]tyrosyl-3-(2-iminoimidazolidin-4-yl)seryl-3-(3-hexopyranosyl-2-iminoimidazolidin-4-yl)serylseryl]

The title compound is prepared by the procedure described for example 104 using the appropriate dimethyl ketal. MS (+ES) *m*/*z:* 716 (M+2H)²⁺.

### Example 209

### (Cyclo[glycyl-β-methylphenylalanyl-O-[4-O-(4,6-O-cyclododecylidenehexopyranosyl)-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl ketal. MS (+ES) *m*/*z:* 730.4 (M+2H)²⁺.

### Example 210

### (Cyclo[glycyl-β-methylphenylalanyl-O-[4-O-(4,6-O-cyclotridecylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl ketal. MS (+ES) *m*/*z:* 737.2 (M+2H)²⁺.

### Example 210a

### (Cyclo[glycyl-β-methylphenylalanyl-O-[4-O-(2,3-O-cyclotridecylidenehexopyanosyl)hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl ketal. MS (+ES) *m*/*z:* 737.2 (M+2H)²⁺.

### Example 211

### (Cyclo[glycyl-β-methylphenylalanyl-O-[4-O-[4,6-O-(bicylo-[3.2.1]oct-2-ylidene)-hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl ketal. MS (+ES) *m*/*z:* 701.1 (M+2H)²⁺.

### Example 212

### (Cyclo[glycyl-β-methylphenylalanyl-O-[4-O-(4,6-O-bicyclo[3.3.1]non-9-ylidenehexopyranosyl)hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl ketal. MS (+ES) *m*/*z:* 708.7 (M+2H)²⁺.

### Example 213

### (Cyclo[glycyl-β-methylphenylalanyl-O-[4-O-(4,6-O-tricyclo[5.2.1.0(2,6)]decan-2-ylidenehexopyranosyl)hexopyranosyl]tyrosyl-3-(2-iminoimidazolidin-4-yl)seryl-3-(3-hexopyranosyl-2-iminoimidazolidin-4yl)serylseryl])

The title compound is prepared by the procedure described for example 104 using the appropriate dimethyl ketal. MS (+ES) *m*/*z:* 714.3 (M+2H)²⁺.

### Example 214

### (Cyclo[glycyl-β-methylphenylalanyl-O-[4-O-[4,6-O-(1,7,7-trimethylbicyclo[2.2.1]hept-2-ylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl ketal. MS (+ES) *m*/*z:* 715.2 (M+2H)²⁺.

### Example 215

### (Cyclo[glycyl-β-methylphenylalanyl-O-[4-O-[4,6-O-(1,7,7-trimethylbicyclo[2.2.1]hept-2-ylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl ketal. MS (+ES) *m*/*z:* 715.3 (M+2H)²⁺.

### Example 216

### (Cyclo[glycyl-β-methylphenylalanyl-O-[4-O-[4,6-O-(4-fluoro-α-methylbenzylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl ketal. MS (+ES) *m*/*z:* 708.4 (M+2H)²⁺.

### Example 217

### (Cyclo[glycyl-β-methylphenylalanyl-O-[4-O-[4,6-O-(tetrahydrothiopyran-4-ylidene)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 104 using the appropriate dimethyl ketal. MS (+ES) *m*/*z:* 697.4 (M+2H)²⁺.

### Example 218

### (Cyclo[glycyl-β-methylphenylalanyl-O-[4-O-[4,6-O-(2-adamantylidene)-3-O-(3-methyl-butanoyl)-hexopyranosyl]hexopyranosyl]tyrosyl-3-(2-iminoimidazolidin-4-yl)-seryl-3-(3-hexopyranosyl-2-iminoimidazolidin-4-yl)serylseryl])

The title compound is prepared from cyclo[glycyl-β-methylphenylalanyl-O-[4-O-[3-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl] by the procedure described for example 104 using the appropriate dimethyl ketal. MS (+ES) *m*/*z:* 756.4 (M+2H)²⁺.

### Example 219

### (Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[4,6-O-(2-adamantylidene)hexopyranosyl]-hexopyranosyl]tyrosyl])

The title compound is prepared from cyclo[3-(2-iminoimidazolidin-4-yl)alanyl-3-(3-hexopyranosyl-2-iminoimidazolidin-4-yl)-alanylserylglycyl-β-methylphenylalanyl-O-(4-O-hexopyranosylhexopyranosyl)tyrosyl] by the procedure described for example 104 using the appropriate dimethyl ketal. MS (+ES) *m*/*z:* 698.9 (M+2H)²⁺.

### Example 220

### (Cyclo[glycyl])henylalanyl-O-[4-O-[4,6-O-(2-adamantylidene)hexopyranosyl]hexopyranosyl]-tyrosyl-3-(2-iminoimidazolidin-4-yl)seryl-3-(3-hexopyranosyl-2-iminoimidazolidin-4-yl]-serylseryl])

The title compound is prepared from cyclo[glycylphenylalanyl-O-(4-O-hexopyranosylhexopyranosyl)tyrosyl-3-(2-iminomidazolidin-4-yl)seryl-3-(3-hexopyranosyl-2-iminoimidazolidin-4-yl)serylseryl] by the procedure described for example 104 using the appropriate dimethyl ketal. MS (+ES) *m*/*z:* 707.4 (M+2H)²⁺.

### Example 221

### (Cyclo[3-cyclohexyl-2-aminobutanoyl-O-[4-O-[4,6-O-(2-adamantylidene)hexopyranosyl]hexopyranosyl]tyrosyl-3-(2-iminoimidazolidin-4-yl)seryl-3-(3-hexopyranosyl-2-iminoimidazolidin-4-yl)serylserylglycyl])

The title compound is prepared from cyclo[3-cyclohexyl-2-aminobutanoyl-O-(4-O-hexopyranosylhexopyranosyl)tyrosyl-3-(2-iminoimidazolidin-4-yl)seryl-3-(3-hexopyranosyl-2-iminoimidazolidin-4-yl)-serylserylglycyl] by the procedure described for example 104 using the appropriate dimethyl ketal. MS (+ES) *m*/*z:* 717.2 (M+2H)²⁺.

### Example 222

### (Cyclo[3-cyclohexylalanyl-3-[4-[[4-O-[4,6-O-(2-adamantylidene)hexopyranosyl]hexopyranosyl]oxy]cyclohexyl]alanyl-3-(2-iminoimidazolidin-4-yl)seryl-3-(3-hexopyranosyl-2-iminoimidazolidin-4-yl)serylserylglycyl])

The title compound is prepared from cyclo[3-cyclohexylalanyl-3-[4-[(4-O-hexopyranosylhexopyranosyl)oxy]cyclohexyl]alanyl-3-(2-iminoimidazolidin-4-yl)seryl-3-(3-hexopyranosyl-2-iminoimidazolidin-4-yl)serylserylglycyl] by the procedure described for example 104 using the appropriate dimethyl ketal. MS (+ES) *m*/*z:* 713.3 (M+2H)²⁺.

### Example 223

### (Cyclo[3-cyclohexylalanyl-3-[4-[[4-O-[4,6-O-(2-adamantylidene)hexopyranosyl]hexopyranosyl]oxy]cyclohexyl]alanyl-3-(2-iminoimidazolidin-4-yl)seryl-3-(3-hexopyranosyl-2-iminoimidazolidin-4-yl)serylserylglycyl])

The title compound is prepared from cyclo[3-cyclohexylalanyl-3-[4-[(4-O-hexopyranosylhexopyranosyl)oxy]cyclohexyl]alanyl-3-(2-iminoimidazolidin-4-yl)seryl-3-(3-hexopyranosyl-2-iminoimidazolidin-4-yl)serylserylglycyl] by the procedure described for example 104 using the appropriate dimethyl ketal. MS (+ES) *m*/*z:* 713.2 (M+2H)²⁺_{.}

### Example 224

### Cyclo[glycyl-β-methylphenylalanyl-O-[4-O-[4,6-O-(2-adamantylidene)hexopranosyl]hexopyranosyl]tyrosyl-3-(2-iminoimidazolidin-4-yl)seryl-3-(1-butyl-3-hexopyranosyl-2-iminoimidazolidin-4-yl)serylseryl])

The title compound is prepared from cyclo[glycyl-β-methylphenylalany]-O-(4-O-hexopyranosylhexopyranosyl)tyrosyl-3-(2-iminoimidazolidin-4-yl)seryl-3-[1-butyl-3-hexopyranosyl-2-iminoimidazolidin-4-yl]serylseryl] by the procedure described for example 104 using the appropriate dimethyl ketal. MS (+ES) *m*/*z:* 742.6 (M+2H)²⁺.

### Example 225

### (Cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-[3-hexopyranosyl-2-imino-1-(3-methylbut-2-en-1-yl)imidazolidin-4-yl]serylseryl])

The title compound is prepared from cyclo[glycyl-β-methylphenylalanyl-O-(4-O-hexopyranosylhexopyranosyl)tyrosyl-3-(2-iminoimidazolidin-4-yl)seryl-3-[3-hexopyranosyl-2-imino-1-(3-methyl-but-2-enyl)imidazolidin-4-yl]serylseryl] by the procedure described for example 104 using the appropriate dimethyl ketal. MS (+ES) *m*/*z:* 748.5 (M+2H)²⁺.

### Example 226

### (Cyclo[glycyl-β-methylphenylalanyl-O-[4-O-[4,6-O-(2-adamantylidene)hexopyranosyl]hexopyranosyl]tyrosyl-3-(2-iminoimidazolidin-4-yl)seryl-3-[3-hexopyranosyl-2-imino-1-[4-(trifluoromethyl)-benzyl]imidazolidin-4-yl]serylseryl]

The title compound is prepared from cyclo[glycyl-β-methylphenylalanyl-O-(4-O-hexopyranosylhexopyranosyl)tyrosyl-3-(2-iminoimidazolidin-4-yl)seryl]-3-[3-hexopyranosyl-2-imino-1-(4-trifluoromethylbenzyl)-imidazolidin-4-yl]-serylseryl] by the procedure described for example 104 using the appropriate dimethyl ketal. MS (+ES) *m*/*z:* 793.5 (M+2H)²⁺.

### Example 227

### Di-N-(2-(1,3-benzoxazolyl)-cyclo[glycyl-β-methylphenyalanyl-O-[4-O-[4,6-O-(2-adamantylidene)hexopyranosyl]hexopyranosyl]tyrosyl-3-[2-iminoimidazolidin-4-yl]seryl-3-[3-hexopyranosyl-2-iminoimidazolidin-4-yl]serylseryl]

The title compound is prepared from cyclo[glycyl-β-methylphenylalanyl-O-(4-O-hexopyranosylhexopyranosyl)tyrosyl-3-[2-(pyrimidin-2-ylimino)imidazolidin-4-yl]seryl-3-[3-hexopyranosyl-2-imino-1-(pyrimidin-2-yl)imidazolidin-4-yl]serylseryl] by the procedure described for example 104 using the appropriate dimethyl ketal. MS (+ES) *m*/*z*: 831.1 (M+2H)²⁺.

### Example 228

### Di-N-(2-(pyrimidinyl)-cyclo[glycyl-β-methylphenylalanyl-O-[4-O-[4,6-O-(2-adamantylidene)hexopyranosyl]hexopyranosyl]tyrosyl-3-[2-iminoimidazolidin-4-yl]seryl-3-[3-hexopyranosyl-2-iminoimidazolidin-4-yl]serylseryl]

The title compound is prepared from cyclo[glycyl-β-methylphenylalanyl-O-(4-O-hexopyranosylhexopyranosyl)tyrosyl-3-[2-(1,3-benzoxazol-2-ylimino)imdazolidin-4-yl]seryl-3-[1-(1,3-benzoxaxol-2-yl)-3-hexopyranosyl-2-iminoimidazolidin-4-yl]serylseryl] by the procedure described for example 104 using the appropriate dimethyl ketal. MS (+ES) *m*/*z:* 792.2 (M+2H)²⁺.

### Example 229

### (Cyclo[glyycyl-β-methylphenylalanyl-O-(2,3-O-isopropylidene-4-O-hexopyranosylhexopyranosyl)tyrosyl-3-(2-iminoimidazolidin-4-yl)seryl-3-[2-imino-(2,3-O-isopropylidene-hexopyranosyl)imidazolidin-4-yl]serylseryl])

### Example 229a

### (Cyclo[glycyl-β-methylphenylalanyl-O-[2,3-O-isopropylidene-4-o-(2,3-isopropylidenehexopyranosyl)hexopyranosyl]tyrosyl-3-(2-iminoimidazolidin-4-yl)seryl-3-[2-imino-3-(2,3-O-isopropylidenehexopyranosyl)imidazolidin-4-yl]serylseryl])

### Example 229b

### (Cyclo[glycyl-β-methylphenylalanyl-O-[2,3-O-isopropylidene-4-O-(2,3:4,6-di-O-isopropylidene-hexopyranosyl)hexopyranosyl]tyrosyl-3-(2-in-imoimidazolidin-4-yl)seryl-3-[2-imino-(2,3-O-isopropylidenehexopyranosyl)imidazolidin-4-yl]serylseryl])

A stirred solution of the bis-hydrochloride salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (0.908 g) in N,N-dimethylformamide (16 mL) is treated with 2,2-dimethoxypropane (5 mL) and hydrogen chloride (0.15 mL of a 4.0 M solution in 1,4-dioxane) and the mixture is stirred for 3 days at room temperature. The resultant solution is concentrated *in vacuo* to approximately one-third of the original volume, added to acetonitrile (32 mL) and the resultant precipitate collected by centrifugation. The precipitate is resuspended in acetonitrile (32 mL) and recentrifuged. The products are then purified by reverse phase HPLC. Cyclo[glycyl-β-methylphenylalanyl-O-(2,3-O-isopropylidene-4-O-hexopyranosylhexopyranosyl)tyrosyl-3-(2-iminoimidazolidin-4-yl)seryl-3-[2-imino-(2,3-O-isopropylidene-hexopyranosyl)imidazolidin-4-yl]serylseryl]: MS (+ES) *m*/*z:* 688.6 (M+2H)²⁺; Cyclo[glycyl-β-methylphenylalanyl-O-[2,3-O-isopropylidene-4-O-(2,3-isopropylidenehexopyranosyl)hexopyranosyl]tyrosyl-3-(2-iminoimidazolidin-4-yl)seryl-3-[2-imino-3-(2,3-O-isopropylidenehexopyranosyl)imidazolidin-4-yl]serylseryl]: MS (+ES) *m*/*z:* 708.5 (M+2H)²⁺; Cyclo[glycyl-β-methylphenylalanyl-O-[2,3-O-isopropylidene-4-O-(2,3:4,6-di-O-isopropylidene-hexopyranosyl)hexopyranosyl]tyrosyl-3-(2-iminoimidazolidin-4-yl)seryl-3-[2-imino-(2,3-O-isopropylidenehexopyranosyl)imidazolidin-4-yl]serylseryl]: MS (+ES) *m*/*z:* 728.6 (M+2H)²⁺_{.}

### Example 230

### (Cyclo[glycyl-β-methylphenylalanyl-O-[4-O-[6-O-[(6-methoxy-2-nahthyl)methyl]hexopyranosyl]hexopyranosyl]tyrosyl-3-(2-iminoimidazolidin-4-yl)seryl-3-(3-hexopyranosyl-2-iminoimidazolidin-4-yl)serylseryl])

### Example 230a

### (Cyclo[glycyl-β-methylphenlalanyl-O-[4-O-[4-O-[(6-methoxy-2-naphthyl)methyl]hexoyranosyl]hexopyranosyl]tyrosyl-3-(2-iminoimidazolidin-4-yl)seryl-3-(3-hexopyranosyl-2-iminoimidazolidin-4-yl)serylseryl])

### Example 230b

### (Cyclo[glycyl-β-methylphenylalanyl-O-[4-O-[3-O-[(6-methoxy-2-naphthyl)methyl]hexopyranosyl]hexopyranosyl]tyrosyl-3-(2-iminoimidazolidin-4-yl)seryl-3-(3-hexopyranosyl-2-iminoimidazolidin-4-yl)serylseryl])

### Example 230c

### (Cyclo[glycyl-β-methylphenylalanyl-O-[4-O-[2-O-[(6-methoxy-2-naphthyl)methyl]hexopyranosyl]hexopyranosyl]tyrosyl-3-(2-iminoimidazolidin-4-yl)seryl-3-(3-hexopyranosyl-2-iminoimidazolidin-4-yl)serylseryl])

A stirred solution of the bis-hydrochloride salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (5.46 g) in dimethyl sulfoxide (25 mL) is treated with 6-methoxy-2-naphthaldehyde dimethyl acetal (1.856 g) and hydrogen chloride (1 mL of 1.0M solution in diethyl ether), and the mixture is heated at 50°C for 1 h. The reaction mixture is then cooled to room temperature and poured into water (100 mL) containing dilute sodium hydroxide solution. The pH of the solution is then adjusted to pH 10, and the resulting precipitates are filtered and washed with a mixture of acetonitrile and diethyl ether (1:1). The crude acetal thus obtained is air dried and used in the next step without further purification. An ice cold solution of trifluoroacetic acid (3.192g) in N,N-dimethylformamide (5 mL) is added dropwise to a cooled (0°C) mixture containing the above acetal derivative (2.04 g) and sodium cyanoborohydride (0.879g) in N,N-dimethylformamide (20 mL). The mixture is then stirred for 24 h and gradually warmed to room temperature, and is then poured into water (50 mL). A dilute solution of sodium hydroxide is added to adjust to pH 10, the resulting solid is filtered, washed several times with water, then acetonitrile and dried. This solid is then purified by reverse phase HPLC to provide the title compounds. Cyclo[glycyl-β-methylphenylalany]-O-[4-O-[6-O-[(6-methoxy-2-naphthyl)methyl]hexopyranosyl]hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 733.2 (M+2H)²⁺; Cyclo[glycyl-β-methylphenylalanyl-O-[4-O-[4-O-[(6-methoxy-2-naphthyl)methyl]hexopyranosyl]hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 733.4 (M+2H)²⁺; Cyclo[glycyl-β-methylphenylalanyl-O-[4-O-[3-O-[(6-methoxy-2-naphthyl)methyl]hexopyranosyl]hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z*: 733.3 (M+2H)²⁺; Cyclo[glycyl-β-methylphenylalanyl-O-[4-O-[2-O-[(6-methoxy-2-naphthyl)methyl]hexopyranosyl]hexopyranosyl]tyrosyl-3-(2-iminoimidazolidin-4-yl)seryl-3-(3-hexopyranosyl-2-iminoimidazolidin-4-yl)serylseryl]: MS (ES) *m*/*z*: 733.2 (M+2H)²⁺_{.}

### Example 231

### (Cyclo[glycyl-β-methylphenylalanyl-O-[4-O-[6-O-[4-(phenylmethoxy)benzyl]hexopyranosyl]-hexopyranosyl]tyrosyl-3-(2-iminoimidazolidin-4-yl)seryl-3-(3-hexopyranosyl-2-iminoimidazolidin-4-yl)serylseryl])

### Example 231a

### (Cyclo[glycyl-β-methylphenylalanyl-O-[4-O-[4-O-[4-(phenylmethoxy)benzyl]hexopyranosyl]-hexopyranosyl]tyrosyl-3-(2-iminoimidazolidin-4-yl)seryl-3-(3-hexopyranosyl-2-iminoimidazolidin-4-yl)serylseryl])

### Example 231b

### (Cyclo[glycyl-β-methylphenylalanyl-O-[4-O-[2-O-[4-(phenylmethoxy)benzyl]hexopyranosyl]-hexopyranosyl]tyrosyl-3-(2-iminoimidazolidin-4-yl)seryl-3-(3-hexopyranosyl-2-iminoimidazolidin-4-yl)serylseryl])

### Example 231c

### (Cylo[glycyl-β-methylphenylalanyl-O-[4-O-[3-O-[4-(phenylmethoxy)benzyl]hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compounds are prepared by the procedure described for example 230 using the appropriate dimethyl acetal. Cyclo[glycyl-β-methylphenylalanyl-O-[4-O-[6-O-[4-(phenylmethoxy)benzyl]hexopyranosyl]-hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 746.4 (M+2H)²⁺; Cyclo[glycyl-β-methylphenylalanyl-O-[4-O-[4-O-[4-(phenylmethoxy)benzyl]hexopyranosyl]-hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 746.3 (M+2H)²⁺; Cyclo[glycyl-β-methylphenylalanyl-O-[4-O-[2-O-[4-(phenylmethoxy)benzyl]hexopyranosyl]-hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 746.7 (M+2H)²⁺: Cyclo[glycyl-β-methylphenylalanyl-O-[4-O-[3-O-[4-(phenylmethoxy)benzyl]hexopyranosyl]-hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 746.7 (M+2H)²⁺.

### Example 232

### (Cyclo[glycyl-β-methylphenylalanyl-O-[4-O-[6-O-(4-methoxybenzyl)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 230 using the appropriate dimethyl acetal. MS (+ES) *m*/*z:* 708.3 (M+2H)²⁺.

### Example 233

### (Cyclo[glycyl-β-methylphenylalanyl-O-[4-O-[6-O-(4-ethoxybenzyl)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 230 using the appropriate dimethyl acetal. MS (+ES) *m*/*z:* 715.4 (M+2H)²⁺.

### Example 234

### (Cyclo[glycyl-β-methylphenylalanyl-O-[4-O-O6-O-(4-methylbenzyl)hexopyranosyl]hexopyranosyl]tyrosyl-3-(2-iminoimidazolidin-4-yl)seryl-3-(3-hexol)yranosyl-2-iminoimidazzolidin-4-yl)serylseryl])

The title compound is prepared by the procedure described for example 230 using the appropriate dimethyl acetal. MS (+ES) *m*/*z:* 700.2 (M+2H)²⁺.

### Example 235

### (Cyclo[glycyl-β-methylphenylalanyl-O-[4-O-[6-O-(4-propoxybenzyl)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 230 using the appropriate dimethyl acetal. MS (+ES) *m*/*z:* 722.3 (M+2H)²⁺.

### Example 236

### (Cyclo[glycyl-β-methylphenylalanyl-O-[4-O-[6-O-(4-phenoxybenzyl)hexopyranosyl]hexopylanosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 230 using the appropriate dimethyl acetal. MS (+ES) *m*/*z:* 739.2 (M+2H)²⁺.

### Example 237

### (Cyclo[glycyl-β-methylphenylalanyl-O-[4-O-6-O-(3-fluoro-4-methoxybenzyl)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 230 using the appropriate dimethyl acetal. MS (+ES) *m*/*z:* 717.3 (M+2H)²⁺.

### Example 238

### (Cyclo[glycyl-β-methylphenylalanyl-O-[4-O-[6-O-(3-ethoxy-4-methoxybenzyl)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 230 using the appropriate dimethyl acetal. MS (+ES) *m*/*z:* 730.3 (M+2H)²⁺.

### Example 239

### (Cyclo[gycyl-β-methylphenylalanyl-O-[4-O-[6-O-[4-methoxy-3-(phenylmethoxy)benzyl]-hexopyranosyl]hexopyranosyl]tyrosyl-3-(2-iminoimidazolidin-4-yl)seryl-3-(3-hexopyranosyl-2-iminoimidazolidin-4-yl)serylseryl])

### Example 239a

### (Cyclo[glycyl-β-methylphenylalanyl-O-[4-O-[4-O-[4-methoxy-3-(phenylmethoxy)benzyl]-hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compounds are prepared by the procedure described for example 230 using the appropriate dimethyl acetal. Cyclo[glycyl-β-methylphenylalanyl-O-[4-O-[6-O-[4-methoxy-3-(phenylmethoxy)benzyl]-hexopyranosyl]hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 761.3 (M+2H)²⁺; Cyclo[glycyl-β-methylphenylalanyl-O-[4-O-[4-O-[4-methoxy-3-(phenylmethoxy)benzyl]-hexopyranosyl]hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 761.8 (M+2H)²⁺.

### Example 240

### (Cyclo[glycyl-β-methylphenylalanyl-O-[4-O-[6-O-[(4-methoxy-1-naphthyl)methyl]hexo-pyranosyl]hexopyranosyl]tyrosyl-3-(2-iminoimidazolidin-4-yl)seryl-3-(3-hexopryanosyl-2-iminoimidazolidin-4-yl)serylseryl])

### Example 240a

### (Cyclo[glycyl-β-methylphenylalanyl-O-[4-O-[4-O-[(4-methoxy-1-naphthyl)methyl]hexo-pyranosyl]hexopyranosyl]tyrosyl-3-(2-iminoimidazolidin-4-yl)seryl-3-(3-hexopyranosyl-2-iminoimidazolidin-4-yl)serylseryl])

### Example 240b

### (Cyclo[glycyl-β-methylphenylalanyl-O-[4-O-[2-O-[(4-methoxy-1-naphthyl)methyl]hexo-pyranosyl]hexopyranosyl]tyrosyl-3-(2-iminoimidazolidin-4-yl)seryl-3-(3-hexopyranosyl-2-iminoimidazolidin-4-ly)serylseryl])

The title compounds are prepared by the procedure described for example 230 using the appropriate dimethyl acetal. Cyclo[glycyl-β-methylphenylalanyl-O-[4-O-[6-O-[(4-methoxy-1-naphthyl)methyl]hexo-pyranosy]lhexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 733.6 (M+2H)²⁺; Cyclo[glycyl-β-methylphenylalanyl-O-[4-O-[4-O-[(4-methoxy-1-naphthyl)methyl]hexo-pyranosy]lhexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 733.5 (M+2H)²⁺; Cyclo[glycyl-β-methylphenylalanyl-O-[4-O-[2-O-[(4-methoxy-1-naphthyl)methyl]hexo-pyranosy]lhexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 733.4 (M+2H)²⁺.

### Example 241

### (Cyclo[glycyl-β-methylphenylalanyl-O-[4-O-[6-O-(3,3-diphenylprop-2-en-1-yl)hexopyranosyl]-hexopyranosyl]tyrosyl-3-(2-iminoimidazolidin-4-yl)seryl-3-(3-hexopyranosyl-2-iminoimidazolidin-4-yl)serylseryl])

### Example 241a

### (Cyclo[glycyl-β-methylphenylalanyl-O-[4-O-[4-O-(3,3-diphenylprop-2-en-1-yl)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compounds are prepared by the procedure described for example 230 using the appropriate dimethyl acetal. Cyclo[glycyl-β-methylphenylalanyl-O-[4-O-[6-O-(3,3-diphenylprop-2-en-1-yl)hexopyranosyl]-hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 744.4 (M+2H)²⁺; Cyclo[glycyl-β-methylphenylalanyl-O-[4-O-[4-O-(3,3-diphenylprop-2-en-1-yl)hexopyranosyl]-hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 744.7 (M+2H)²⁺.

### Example 242

### (Cyclo[glycyl-β-methylphenylalanyl-O-[4-O-[6-O-(3-phenylprop-2-en-1-yl)hexopyranosyl]-hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 230 using the appropriate dimethyl acetal. MS (+ES) *m*/*z:* 706.3 (M+2H)²⁺.

### Example 243

### (Cyclo[glycyl-β-methylphenylalanyl-O-[4-O-[6-O-[3-[4-(dimethylamino)phenyl]prop-2-en-1-yl]hexopyranosyl]hexopyranosyl]tyrosyl-3-(2-iminoimidazolidin-4-yl)seryl-3-(3-hexopyranosyl-2-iminoimidazolidin-4-yl)serylseryl])

### Example 243a

### (Cyclo[glycyl-β-methylphenylalanyl-O-[4-O-[4-O-[3-[4-(dimethylamino)phenyl]prop-2-en-1-yl]hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compounds are prepared by the procedure described for example 230 using the appropriate dimethyl acetal. Cyclo[glycyl-β-methylphenylalanyl-O-[4-O-[6-O-[3-[4-(dimethylamino)phenyl]prop-2-en-1-yl]hexopyranosyl]hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 728.0 (M+2H)²⁺; Cyclo[glycyl-β-methylphenylalanyl-O-[4-O-[4-O-[3-[4-(dimethylamino)phenyl]prop-2-en-1-yl]hexopyranosyl]hexopyranosyl]tyrosyl-3-(2-iminoimidazolidin-4-yl)seryl-3-(3-hexopyranosyl-2-iminoimidazolidin-4-yl)serylseryl]: MS (+ES) *m*/*z:* 728.0 (M+2H)²⁺.

### Example 244

### (Cyclo[glycyl-β-methylphenylalanyl-O-[4-O-[6-O-(2-thienylmethyl)hexopyranosyl]hexopyranosyl]tyrosyl-3-(2-iminoimidazolidin-4-yl)seryl-3-(3-hexopyranosyl-2-iminoimidazolidin-4-yl)serylseryl])

The title compound is prepared by the procedure described for example 230 using the appropriate dimethyl acetal. MS (+ES) *m*/*z:* 696.3 (M+2H)²⁺.

### Example 245

### (Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[6-O-[(6-methoxy-2-naphthyl)methyl]-hexopyranosyl]hexopyranosyl]tyrosyl])

### Example 245a

### (Cyclo[3-(2-iminoimidazolidin-4-yl)alanyl-3-(3-hexopranosyl-2-iminoimidazolidin-4-yl)-alanylserylglycyl-β-methylphenylalanyl-O-[4-O-[4-O-[(6-methoxy-2-naphthyl)methyl]-hexopyranosyl]hexopyranosyl]tyrosyl])

### Example 245b

### (Cyco[3-(2-iminoimidazolidin-4-yl)alanyl-3-(3-hexopyranosyl-2-iminoimidazolidin-4-yl)-alanylserylglycyl-β-methylphenylalanyl-O-[4-O-[3-O-[(6-methoxy-2-naphthyl)methyl]-hexopyranosyl]hexopyranosyl]tyrosyl])

### Example 245c

### (Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[2-O-[(6-methoxy-2-naphthyl)methyl]-hexopyranosyl]hexopyranosyl]tyrosyl])

The title compounds are prepared from cyclo[3-(2-iminoimidazolidin-4-yl)alanyl-3-(3-hexopyranosyl-2-iminoimidazolidin-4-yl)-alanylserylglycyl-β-methylphenylalanyl-O-(4-O-hexopyranosylhexopyranosyl)tyrosyl] by the procedure described for example 230 using the appropriate dimethyl acetal. Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[6-O-[(6-methoxy-2-naphthyl)methyl]-hexopyranosyl]hexopyranosyl]tyrosyl]: MS (+ES) *m*/*z:* 717.6 (M+2H)²⁺; Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[4-O-[(6-methoxy-2-naphthyl)methyl]-hexopyranosyl]hexopyranosyl]tyrosyl]: MS (+ES) *m*/*z:* 717.7 (M+2H)²⁺; Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[3-O-[(6-methoxy-2-naphthyl)methyl]-hexopyranosyl]hexopyranosyl]tyrosyl]: MS (+ES) *m*/*z:* 717.7 (M+2H)²⁺: Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[2-O-[(6-methoxy-2-naphthyl)methyl]-hexopyranosyl]hexopyranosyl]tyrosyl]: MS (+ES) *m*/*z:* 717.7 (M+2H)²⁺.

### Example 246

### (Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[6-O-[4-(phenylmethoxy)benzyl]hexopyranosyl]hexopyranosyl]tyrosyl])

### Example 246a

### (Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[4-O-[4-(phenylmethoxy)benzyl]hexopyranosyl]hexopyranosyl]tyrosyl])

### Example 246b

### (Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[3-O-[4-(phenylmethoxy)benzyl]hexopyranosyl]hexopyranosyl]tyrosyl])

### Example 246c

### (Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[2-O-[4-(phenylmethoxy)benzyl]hexopyranosyl]hexopyranosyl]tyrosyl])

The title compounds are prepared from cyclo[3-(2-iminoimidazolidin-4-yl)alanyl-3-(3-hexopyranosyl-2-iminoimidazolidin-4-yl)-alanylserylglycyl-β-methylphenylalanyl-0-(4-0-hexopyranosylhexopyranosyl)tyrosyl] by the procedure described for example 230 using the appropriate dimethyl acetal. Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[6-O-[4-(phenylmethoxy)benzyl]hexopyranosyl]hexopyranosyl]tyrosyl]: MS (+ES) *m*/*z:* 717.6 (M+2H)²⁺; Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[4-O-[4-(phenylmethoxy)benzyl]hexopyranosyl]hexopyranosyl]tyrosyl]: MS (+ES) *m*/*z:* 717.7 (M+2H)²⁺; Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[3-O-[4-(phenylmethoxy)benzyl]hexopyranosyl]hexopyranosyl]tyrosyl]: MS (+ES) *m*/*z:* 717.7 (M+2H)²⁺: Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[2-O-[4-(phenylmethoxy)benzyl]hexopyranosyl]hexopyranosyl]tyrosyl]: MS (+ES) *m*/*z:* 717.7 (M+2H)²⁺.

### Example 247

### (Cyclo[glcyl-β-methylphenylalanyltyrosyl-3-(2-iminoimidazolidin-4-yl)seryl-3-(2,3:4,6-diisopropylidenehexopyranosyl)-2-iminoimidazolidin-4-ylserylseryl])

A stirred solution of the bis-hydrochloride salt of cyclo[glycyl-β-methylphenylalanyltyrosyl-3-(2-iminoimidazolidin-4-yl)seryl-3-(3-hexopyranosyl-2-iminoimidazolidin-4-yl)serylseryl] (2.85 g) in N,N-dimethylformamide (20 mL) is treated with 2,2-dimethoxypropane (5.8 mL) and p-toluenesulfonic acid mono-hydrate (35 mg) and the mixture is stirred for 16 h at room temperature. Water (1 mL) is then added, the mixture is stirred for 30 min at room temperature, and then acetonitrile (100 mL) is added dropwise to precipitate the product. After stirring for 45 min at 0°C, the product is collected by filtration, and is then washed with acetonitrile, diethyl ether, and air dried. MS (+ES) *m*/*z:* 526.6 (M+2H)²⁺.

### Example 248

### (Cyclo[glycyl-β-methylphenylalanyltyrosyl-3-(2-iminoimidazolidin-4-yl)seryl-3-[3-(2,3:4,6-di-O-isopropylidenehexopyranosyl)-2-iminoimidazolidin-4-yl]seryl-O-(tertbutyldimethylsilyl)seryl])

A stirred solution of the bis-hydrochloride salt of cyclo[glycyl-β-methylphenylalanyltyrosyl-3-(2-iminoimidazolidin-4-yl)seryl-3-(2,3:4,6-diisopropylidenehexopyranosyl)-2-iminoimidazolidin-4-ylserylseryl] (2.25 g) in N,N-dimethylformamide (15 mL) containing 2,6-lutidine (4 mL) is treated dropwise at -45°C with a solution of t-buytyldimethylsilyl triflate in N,N-dimethylformamide (2 M, 5 mL) and the mixture is stirred for 2 h at -45°C. Methanol (1.5 mL) is added, and then diethyl ether (200 mL) is added to the reaction mixture. Stirring is stopped and the resulting oil is permitted to settle. The solvents are decanted and an additional portion of diethyl ether (200 mL) is added. After stirring briefly, and then allowing the oil to settle, the solvents are again decanted. Acetonitrile (175 mL) is added to precipitate the product, which is collected by filtration and then washed with acetonitrile, diethyl ether, and air dried. MS (+ES) *m*/*z:* 583.6 M+2H)²⁺.

### Example 249

### (Tri-N-[(phenylmethoxy)carbonyl]-cyclo[glycyl-β-methylphenylalanyltyrosyl-3-(2-iminoimidazolidin-4-yl)seryl-3-[3-(2,3:4,6-di-O-isopropylidenehexopylanosyl)-2-iminoimidazolidin-4-yl]seryl-O-(tert-butyldimethylsilyl)seryl])

A stirred solution of cyclo[glycyl-β-methylphenylalanyltyrosyl-3-(2-iminoimidazolidin-4-yl)seryl-3-[3-(2,3:4,6-di-O-isopropylidenehexopyranosyl)-2-iminoimidazolidin-4-yl]seryl-O-(tert-butyldimethylsilyl)seryl] (1.97 g) in N,N-dimethylformamide (15 mL) containing N,N-diisopropylethylamine (1 mL) is treated with N-(benzyloxycarbonyloxy)-succinimide (1.48 g) and the mixture is stirred for 16 h at room temperature. The mixture is then diluted with ethyl acetate and washed twice with saturated aqueous sodium bicarbonate, twice with water, and then with saturated aqueous sodium chloride. The organic phase is then dried over anhydrous sodium sulfate, filtered and concentrated to provide an oil. This oil is dissolved in a minimal amount of dichloromethane and treated with hexanes to precipitate the product. The product is then collected by filtration, washed with hexanes, and dried. MS (+ES) *m*/*z:* 784.6 (M+2H)²⁺.

### Example 250

### (Tetra-N-[(phenylmethoxy)carbonyl]-cyclo[glycyl-β-methylphenylalanyltyrosyl-3-(2-iminoimidazolidin-4-yl)seryl-3-(2-iminoimidazolidin-4-yl)seryl-O-(tert-butyldimethylsilyl)seryl])

A stirred solution of the bis-hydrochloride salt of cyclo[glycyl-β-methylphenylalanyltyrosyl-3-(2-iminoimidazolidin-4-yl)seryl-3-(2-iminoimidazolidin-4-yl)serylseryl] (590 mg) in N,N-dimethylformamide (6 mL) containing 2,6-lutidine (0.5 mL) is treated dropwise at -45°C with t-buytyldimethylsilyl triflate (0.62 mL) and the mixture is stirred for 16 h and then gradually warmed to room temperature. Methanol (0.25 mL) is added, the mixture is stirred for 10 min, and then N,N-diisopropylethylamine (0.66 mL) and N-(benzyloxycarbonyloxy)-succinimide (0.84 g) are sequentially added. The mixture is stirred for 2 h, and then additional N,N-diisopropylethylamine (0.33 mL) and N-(benzyloxycarbonyloxy)-succinimide (0.33 g) are added. After 2 more hs, the mixture is diluted with ethyl acetate and washed twice with saturated aqueous sodium bicarbonate, twice with water, and then with saturated aqueous sodium chloride. The organic phase is then dried over anhydrous sodium sulfate, filtered and concentrated to provide an oil. This oil is chromatographed over silica gel to provide the title compound. MS (+ES) *m*/*z:* 730.9 (M+2H)²⁺.

### Example 251

### (α-O-[(Pentylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl])

### Example 251a

### (β-O-[(Pentylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl])

### Example 251b

### (γ-O-[(Pentylamino)carbonyl]-cyclo[glycyl-β-methylphenylalanyl-O-(4-O-hexopyranosylhexopyranosyl)tyrosyl-3-(2-iminoimidazoyidin-4-yl)seryl-3-(3-hexopyranosyl-2-iminoimidazolidin-4-yl)serylseryl])

A solution of the bis-trifluoroacetate salt of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (250 mg) in N,N-dimethylformamide (1.6 mL) at 0°C is treated dropwise with a 1M solution of N,N'-carbonyldiimidazole in N,N-dimethylformamide (0.25 mL) and the reaction is stirred for 2 h at 0°C. Isoamyl amine (0.16 mL) is added and the reaction is stirred overnight at approximately 4°C. Acetonitrile (∼2 mL) is then added to precipitate the products. Solids are collected by filtration, washed with acetonitrile and ether, air dried, and then purified by reverse phase HPLC. α-O-[(Pentylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]:MS (+ES) *m*/*z:* 704.8 (M+2H)²⁺; β-O-[(Pentylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES) *m*/*z:* 704.8 (M+2H)²⁺; γ-O-[(Pentylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES) *m*/*z:* 704.6 (M+2H)²⁺.

### Example 252

### (α-O-[(Butylamino)carbonyl]-cyclo[glycyl-β-methylphenylalanyl-O-(4-O-hexopyranosylhexopyranosyl)trosyl-3-(2-iminoimidazolidin-4-yl)seryl-3-(3-hexopyranosyl-2-iminoimidazolidin-4-yl)serylseryl])

### Example 252a

### (β-O-[(Butylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl])

The title compounds are prepared by the procedure described for example 251 using the appropriate alkyl amine. α-O-[(Butylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES) *m*/*z:* 697.8 (M+2H)²⁺; β-O-[(Butylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES) *m*/*z:* 697.9 (M+2H)²⁺.

### Example 253

### (α-O-[(Heptylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl])

### Example 253a

### (β-O-[(Heptylamino)carbonyl]-cyclorglycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl])

### Example 253b

### (γ-O-[(Heptylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl])

The title compounds are prepared by the procedure described for example 251 using the appropriate alkyl amine. α-O-[(Heptylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES) *m*/*z:* 718.8 (M+2H)²⁺; β -O-[(Heptylamino)carbonyl]cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES) *m*/*z:* 718.9 (M+2H)²⁺; γ-O-[(Heptylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES) *m*/*z:* 718.9 (M+2H)²⁺.

### Example 254

### (α-O-[(Dodecylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl])

### Example 254a

### (β-O-[(Dodecylamino)carbonyl]-cyclo[glycyl-β-methylphentlalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl])

### Example 254b

### (γ-O-[(Dodecylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl])

The title compounds are prepared by the procedure described for example 251 using the appropriate alkyl amine. α-O-[(Dodecylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl): MS (+ES) *m*/*z:* 753.8 (M+2H)²⁺; β-O-[(Dodecylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES) *m*/*z:* 754.0 (M+2H)²⁺; γ-O-[(Dodecylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES) *m*/*z:* 753.9 (M+2H)²⁺.

### Example 255

### (α-O-[(N-Methyl-2-methylpropylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopranosyl-2-iminoimidazolidin-4-yl)serylseryl])

### Example 255a

### (β-O-[(N-Methyl-2-methylpropylamino)carbonyl]-cyclo[glycyl-β-methylphenylalanyl-O-(4-O-hexopyranosylhexopyranosyl)tyrosyl-3-(2-iminoimidazohdin-4-yl)seryl-3-(3-hexopyranosyl-2-infinoimidazolidin-4-yl)serylseryl])

The title compounds are prepared by the procedure described for example 251 using the appropriate alkyl amine. α-O-[(N-Methyl-2-methylpropylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES) *m*/*z:* 705.0 (M+2H)²⁺; β-O-[(N-Methyl-2-methylpropylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES) *m*/*z:* 704.8 (M+2H)²⁺.

### Example 256

### (α-O-[(Cyclohexylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl])

### Example 256a

### (β-O-[(Cyclohexylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl])

The title compounds are prepared by the procedure described for example 251 using the appropriate alkyl amine. α-O-[(Cyclohexylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES) *m*/*z:* 710.7 (M+2H)²⁺; β-O-[(Cyclohexylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES) *m*/*z:* 710.9 (M+2H)²⁺.

### Example 257

### (α-O-[(Benzylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseyl])

### Example 257a

### (β-O-[(Benzylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl])

### Example 257b

### (γ-O-[(Benzylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl])

The title compounds are prepared by the procedure described for example 251 using the appropriate alkyl amine. α-O-[(Benzylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES) *m*/*z:* 714.8 (M+2H)²⁺; β-O-[(Benzylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES) *m*/*z:* 714.8 (M+2H)²⁺; γ-O-[(Benzylamino)carbonyl]-cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]: MS (+ES) *m*/*z:* 714.8 (M+2H)²⁺.

### Example 258

### SEED PREPARATION

A seed medium of the following formulation is prepared:

| | |
|---|---|
| Bacto tryptic soy broth¹ (dehydrated) | 3% |
| Dextrose | 2% |
| Distilled water | |

| | |
|---|---|
| ¹(Soybean-casein digest medium, Difco Laboratories, Detroit MI) | |

The seed medium is prepared by adding a filter-sterilized dextrose solution to the tryptic soy broth after autoclaving. Fifty mL of seed medium in a 250 mL Erlenmeyer flask is inoculated with cells from a mutant strain (see table below) cultured on ATCC agar medium #172 (ATCC Media Handbook, 1^{st} edition, 1984). Sufficient inoculum from the agar culture is used to provide a turbid seed after 24 -36 h. of growth. The seed is incubated at 30°C, 200 rpm using a gyro-rotary shaker with a 2 inch throw, for 24 - 36 h. To prepare inoculum for fermentors, 1 liter of the above seed medium in a 2.8 liter Fernbach flask is inoculated with 5 mL of cryo-preserved seed culture and is incubated as above.

### Example 259

### DIRECTED BIOSYNTHESIS OF GLYCOPEPTIDE ANTIBIOTICS

A fermentation medium of the following formulation is prepared:

| | |
|---|---|
| Pharmamedia² | 2% |
| Calcium carbonate | 0.5% |
| Dextrose | 4% |
| Distilled water | |

| | |
|---|---|
| ²(Cottonseed flour, Traders Oil Mill Co., Fort Worth, TX Trader's Guide to Fermentation Media Formulation, 1980) | |

This medium is prepared by adding a filter-sterilized dextrose solution to autoclaved Pharmamedia™/calcium carbonate broth. A production medium volume of 50 mL in a 250 mL Erlenmeyer flask is supplemented to a final concentration of 2 mM of a selected amino acid or 16 mM of a selected fatty acid. This flask is then inoculated with 1 mL of seed culture prepared as described in Example 258. Production fermentations are incubated at 30°C, 250 rpm, 2 in. throw for up to 7 days. For large-scale production of glycopeptide antibiotics of the invention, 15L glass jar fermentors are prepared with 10L of the above production medium supplemented with the selected amino acid or fatty acid. Fermentors are inoculated with 200 mL of seed culture and are incubated at 30°C, 800 rpm with an air flow rate of 10L per minute.

### Example 260a

### ISOLATION AND IDENTIFICATION OF GLYCOPEPTIDE ANTIBIOTICS

The supernatant of a fermentation broth, obtained by centrifugation at 3000 X g, is loaded on a column containing pre-washed polyarylate absorbant resin XAD-7 at approximately a 1:10 resin/supernatant ratio (v/v) for a typical extraction. The column is then sequentially washed with water, methanol, and water (each with ∼2 column volumes). The crude glycopeptide antibiotics are eluted by 1:1 acetonitrile in water containing 0.05-0.1% trifluoroacetic acid (2-3 column volumes). Upon evaporation under reduced pressure, the residue is redissolved in water or water/methanol mixture and subjected to reverse phase high performance liquid chromatography (HPLC) to afford the pure antibiotics. Typically the HPLC is performed with C18 reverse phase columns (YMC ODS-A, 120A pore size) using mixtures of acetonitrile or methanol in water containing small amounts of trifluoroacetic acid to control the acidity in the range of pH 3.5 and 5.5

The purified antibiotics are then subjected to structural determination by spectroscopic analysis. The molecular weights are usually determined by electrospray ionization mass spectrometry (ESI MS) measured with a Finnigan LCQ instrument in positive mode. The structures are determined by interpretation of 1-D and 2-D nuclear magnetic resonance (NMR) spectral data in 1:1 D₂O/CD₃OD, including ¹H-¹H COSY, TOCSY and ¹H-¹³C HMBC, and HMQC data. New antibiotics discerned by the above described conditions are shown in the Examples 261a, 261b, 261c, 262a, 262b, 263, 264a, 264b, 265a, 265b, 270a, 270b, 271a, 275, 277a, 277b, 277c, 278a, 278b, 278c, 278d, 279a, 279b, 280a, 280b, 282, 283a, 283b, 283c, 284b and 296.

### Example 260b

### DETECTION OF GLYCOPEPTIDE ANTIBIOTICS BY HPLC AND LC/MS

Fermentation broth is centrifuged (3000 X g), and the supernatant is applied to a wetted BAKERBOND™ spe carboxylic acid extraction column (catalog # 7211-03). Columns are washed with 50% aqueous methanol and are eluted with acetonitrile/water/trifluoroacetic acid (70/30/0.5). The solvent is evaporated, and the residue is reconstituted in 0.2 mL methanol/water (2/8). Samples are analyzed by HPLC, and the production of new glycopeptide antibiotics of the invention is monitored. Reverse phase HPLC is performed using a Hewlett Packard model 1090M liquid chromatograph with photodiode array detection, a YMC ODS-A 4.6 x 150 mm HPLC column, and a mobile phase of 0.02% trifluoroacetic acid in water (solvent A) and acetonitrile (solvent B). A linear gradient from 5%B to 34%B in 15 min, with a flow rate of 1 mL/min is used for elution. Relative retention times (RRT) are calculated by dividing the peak retention times of the new glycopeptide antibiotics prepared herein by that of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]. The incorporation of amino acids or fatty acids into the glycopeptide antibiotics is indicated by liquid chromatography/mass spectrometry (LC/MS) analysis of fermentation extracts and a comparison of the molecular weights of new peaks with the predicted molecular weight of glycopeptide antibiotics containing the amino acid or fatty acid analogs.

The molecular weights of new glycopeptide antibiotics of the invention are determined using a Hewlett-Packard APCI-electrospray LC/MS system with an HP 5989B Mass Spectrometer, HP 59987A APCI-Electrospray, HP 1090 series II HPLC and HP ChemStation data system with HP G1047A LC/MS software. Extracts are resolved by reverse phase HPLC as described above. UV detection is at 226 nm. The ESI MS is performed in positive mode. New glycopeptide antibiotics discerned by the above described conditions are shown in the Examples 262c, 264c, 265c, 266a, 266b, 266c, 267a, 267b, 267c, 268, 269a, 269b, 269c, 270c, 271b, 271c, 272a, 272b, 273a, 273b, 274a, 274b, 276a and 276b.

### Examples 261a, 261b and 261c

### GLYCOPEPTIDE ANTIBIOTIC INCORPORATING p-FLUORO-DL-PHENYLALANINE

### Example 261a

### Cyclo[glycyl-4-fluoro-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]

### Example 261b

### Cyclo[glycyl-4-fluoro-β-methylphenylalanyl-O-[4-O-[2-O-(3-methylbutanoyl)hexopyranosyl]-hexopyranosyl]tyrosyl-3-(2-iminoimidazolidin-4-yl)seryl-3-(3-hexopyranosyl-2-imino-imidazolidin-4-yl)serylseryl]

### Example 261c

### Cyclo[glycyl-4-fluoro-β-methylphenylalanyl-O-[4-O-[3-O-(3-methylbutanoyl)hexopyranosyl]-hexopyranosyl]tyrosyl-3-(2-iminoimidazolidin-4-yl)seryl-3-(3-hexopyranosyl-2-iminoimidazolidin-4-yl)serylseryl]

Approximately 10L of supernatant of a 7-day fermentation of strain LL4690 supplemented with p-fluoro-DL-phenylalanine, conducted as described in Examples 258 and 259, is loaded on a column containing polyacrylate resin XAD-7 (1L). The XAD-7 resin is washed sequentially with methanol (2L), acetone (2L), and water (4L) before packing in the column. After loading, the column is sequentially washed with water (2L), methanol (2L), and water (2L) followed by elution with 1:1 acetonitrile in water containing 0.1% trifluoroacetic acid (3L). The acidic acetonitrile/water fraction is concentrated under reduced pressure to a small volume, and the residue after extraction with 1:4 water/dimethylformamide is fractionated by reverse phase high performance liquid chromatography (HPLC) on a C18 column (YMC ODS-A, 10 micron particle size, 70 x 500 mm). The mobile phase consists of a gradient from 14 to 50% by volume of acetonitrile in water with 0.02% trifluoroacetic acid over 55 min at a flow rate of 100 mL per min, and the effluent is monitored by UV absorbance at 226 nm. Peak fractions with retention times of approximately 26 min, 33 min, and 36 min are collected to afford Examples 261a, 261b, and 261c, respectively, upon e

### Example (261a)

a) Molecular Weight: MS(ESI) [M+2H]²⁺ = M/Z 657.2 (m. w. =1312)
b) The structure is consistent with the Proton Magnetic Resonance Spectral data (300 MHz, CD₃OD/D₂O 1:1)

### Example (261b)

a) Molecular Weight: MS(ESI) [M+2H]²⁺ = M/Z 699.2 (m. w. =1396)
b) The structure is consistent with the Proton Magnetic Resonance Spectral data (300 MHz, CD₃OD/D₂O 1:1)

### Example (261c)

a) Molecular Weight: MS(ESI) [M+2H]²⁺ = M/Z 699.3 (m. w. = 1396)
b) The structure is consistent with the Proton Magnetic Resonance Spectral data (300 MHz, CD₃OD/D₂O 1:1)

### Examples 262a, and 262b

### GLYCOPEPTIDE ANTIBIOTICS INCORPORATING p-CHLORO-DL-PHENYLALANINE

### Example 262a

### Cyclo[glycyl-4-chloro-β-methylphenlalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]

### Example 262b

### Cyclo[glycyl-4-chloro-β-methylphenylalanyl-O-[4-O-[3-O-[3-methylbutanoyl)hexopyranosyl]-hexopyranosyl]tyrosyl-3-(2-iminoimidazolidin-4-yl)seryl-3-(3-hexopranosyl-2-iminoimidazolidin-4-yl)serylseryl]

Approximately 3L of supernatant from a 7-day fermentation of strain LL4614 supplemented with p-chloro-DL-phenylalanine, conducted as described in Examples 258 and 259, is processed using XAD-7 column chromatography and HPLC as described in Examples 261a, 261b, and 261c. Peak fractions A and B with respective HPLC retention times at approximately 26 and 36 min are concentrated under reduced pressure and the concentrates are further purified by reverse phase HPLC on a C18 column (YMC ODS-A, 8 micron particle size, 20 x 250 mm). Further HPLC chromatography of fraction A, using a mobile phase consisting of a gradient from 30 to 60% by volume of methanol in water with 0.02% trifluoroacetic acid over 20 min at a flow rate of 20 mL per min, afforded 262a at approximately 16 min, while chromatography of fraction B, using a gradient solvent system from 45 to 60% of methanol in water with 0.02% trifluoroacetic acid over 20 min, afforded 262b at approximately 18 min upon evaporation.

### Example (262a)

a) Molecular Weight: MS(ESI) [M+2H]²⁺ = M/Z 665.2 (m. w. = 1328)
b) The structure is consistent with the Proton Magnetic Resonance Spectral data (300 MHz, CD₃OD/D₂O 1:1)

### Example (262b)

a) Molecular Weight: MS(ESI) [M+2H]²⁺ = M/Z 707 (m. w. = 1412)
b) The structure is consistent with the Proton Magnetic Resonance Spectral data (300 MHz, CD₃OD/D₂O 1:1)

### Example 262c

### GLYCOPEPTIDE ANTIBIOTIC INCORPORATING p-CHLORO-DL-PHENYLALANINE

### Cyclo[glycyl-4-chloro-β-methylphenlyalanyl-O-[4-O-[2-O-(3-methylbutanoyl)hexopyranosyl]-hexopyranosyl]tyrosyl-3-(2-iminoimidazolidin-4-yl)seryl-3-(3-hexopyranosyl-2-iminoimidazolidin-4-yl)serylseryl]

A 50 mL fermentation of strain LL4690 supplemented with p-chloro-DL-phenylalanine is performed as described in Examples 258 and 259. A sample is removed and centrifuged to remove the cells. The supernatant is analyzed by analytical HPLC and LC/MS as described in Example 260b. A new HPLC peak containing glycopeptide antibiotic 262c with a molecular weight that is predicted if p-chloro-DL-phenylalanine is incorporated in place of phenylalanine is identified.

### Example (262c)

Molecular Weight: MS(ESI) [M+2H]²⁺ = M/Z 707 (m. w. = 1412)

### Example 263

### GLYCOPEPTIDE ANTIBIOTICS INCORPORATING (S)-(-)-alpha-AMINOCYCLOHEXANE-PROPIONIC ACID OR PRODUCED IN THE PRESENCE OF (S)-(-)-alpha-AMINOCYCLOHEXANE-PROPIONIC ACID

### Cyclo[3-cyclohexylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylserylglycyl]

Approximately 10L of supernatant of a 7-day fermentation of strain LL4728 supplemented with (S)-(-)-alpha-aminocyclohexane-propionic acid, conducted as described in Examples 258 and 259, is loaded onto an XAD-7 column (1.2L). The column is sequentially washed with water (4L), methanol (3L), and water (3L) followed by elution with 1:1 acetonitrile in water containing 0.15% trifluoroacetic acid (4L). The acidic acetonitrile/water fraction is concentrated under reduced pressure to a small volume and lyophilized to dryness affording a brown powder. The brown powder is then extracted with 1:4 water/dimethylformamide, and the extract is fractionated by reverse phase HPLC on a C18 column (YMC ODS-A, 10 micron particle size, 70 x 500 mm). The mobile phase consists of a gradient from 12 to 40% by volume of acetonitrile in water with 0.02% trifluoroacetic acid over 40 min at a flow rate of 100 mL per min. The peak fraction with a retention time of approximately 29 min is concentrated and re-purified by reverse phase HPLC on another C18 column (MetaChem ODS3, 5 micron particle size, 20 x 250 mm). The mobile phase is a gradient solvent system from 20 to 50% by volume of acetonitrile in water with 0.02% trifluoroacetic acid over 41 min at a flow rate of 7 mL per min. The peak fraction at approximately 41 min affords Example 263 upon evaporation.

### Example (263)

a) Molecular Weight: MS(ESI) [M+2H]²⁺ = M/Z 644.4 (m. w. = 1286)
b) The structure is consistent with the Proton Magnetic Resonance Spectral data (300 MHz, CD₃OD/D₂O 1:1)

### Examples 264a and 264b

### GLYCOPEPTIDE ANTIBIOTICS INCORPORATING m-FLUORO-DL-PHENYLALANINE

### Example 264a

### Cyclo[glycyl-3-fluoro-β-methylphenylalanyl-O-[4-O-[2-O-(3-methylbutanoyl)hexopyranosyl]-hexopyranosyl]tyrosyl-3-(2-iminoimidazolidin-4-yl)selyl-3-(3-hexopyranosyl-2-iminoimidazolidin-4-yl)serylseryl]

### Example 264b

### Cyclo[glycyl-3-fluoro-β-methylphenlyalanyl-O-[4-O-[3-O-(3-methylbutanoyl)hexopyranosyl]-hexopyranosyl]tyrosyl-3-(2-iminoimidazolidin-4-yl)seryl-3-(3-hexopyranosyl-2-iminoimidazolidin-4-yl)serylseryl]

Approximately 10L of supernatant from a 7-day fermentation of strain LL4690 supplemented with m-fluoro-DL-phenylalanine, conducted as described in Examples 258 and 259, is processed using an XAD-7 column, and the fraction concentrate that contained the glycopeptide antibiotics is extracted with 1:4 water/dimethylformamide, as described in Example 261. The extract is fractionated by reverse phase HPLC on a C18 column (YMC ODS-A, 10 micron particle size, 70 x 500). The mobile phase is a gradient solvent system from 20 to 35% acetonitrile in water with 0.02% trifluoroacetic acid over 55 min at flow rate of 100 mL per min. The peak fractions with retention times at approximately 38 and 46 min afford Examples 264a and 264b, respectively, upon evaporation of volatiles.

### Example (264a)

a) Molecular Weight: MS(ESI) [M+2H]²⁺ = M/Z 699.4 (m. w. = 1396)
b) The structure is consistent with the Proton Magnetic Resonance Spectral data (300 MHz, CD₃OD/D₂O 1:1)

### Example (264b)

a) Molecular Weight: MS(ESI) [M+2H]²⁺ = M/Z 699.4 (m. w. = 1396)
b) The structure is consistent with the Proton Magnetic Resonance Spectral data (300 MHz, CD₃OD/D₂O 1:1)

### Example 264c

### GLYCOPEPTIDE ANTIBIOTICS INCORPORATING m-FLUORO-DL-PHENYLALANINE

### Cyclo[glycyl-3-fluoro-β-methylphenylalanyl-O-(4-O-hexopyranosylhexopryanosyl)tyrosyl-3-(2-iminoimidazolidin-4-yl)seryl-3-(3-hexopyranosyl-2-iminoimidazolidin-4-yl)serylseryl]

A 50 mL fermentation of strain LL4690 supplemented with m-fluoro-DL-phenylalanine is performed as described in Examples 258 and 259. A sample is removed and centrifuged to remove the cells. The supernatant is analyzed by analytical HPLC and LC/MS as described in Example 260b. A new HPLC peak containing glycopeptide antibiotic 264c with a molecular weight that is predicted if m-fluoro-DL-phenylalanine is incorporated in place of phenylalanine is identified.

### Example (264c)

Molecular Weight: MS(ESI) [M+2H]²⁺ = M/Z 657 (m. w. = 1312)

### Examples 265a and 265b

### GLYCOPEPTIDE ANTIBIOTICS INCORPORATING 3-(2-THIENYL)-DL-ALANINE

### Cyclo[3-(2-thienyl)-2-aminobutanoyl-O-[4-O-[2-O-(3-methylbutanoyl)hexopyranosyl]hexopyranosyl]tyrosyl-3-(2-iminoimidazolidin-4-yl)seryl-3-(3-hexopylanosyl-2-iminoimidazolidin-4-yl)serylserylglycyl]

### Example 265b

### Cyclo[3-(2-thienyl)-2-aminobutanoyl-O-[4-O-[3-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylserylglycyl]

Approximately 10L of supernatant from a 7-day fermentation of strain LL4690 supplemented with 3-(2-thienyl)-DL-alanine, conducted as described in Examples 258 and 259, is processed using an XAD-7 column. The fraction concentrate containing the glycopeptide antibiotics is extracted with 1:4 water/dimethylformamide, as described in Example 261. The extract is fractionated by reverse phase HPLC on a C18 column (YMC ODS-A, 10 micron particle size, 70 x 500). The mobile phase is a gradient solvent system from 20 to 35% acetonitrile in water with 0.02% trifluoroacetic acid over 55 min at flow rate of 100 mL per min. The peak fractions with retention times at approximately 27 and 33 min afford Examples 265a and 265b, respectively, upon evaporation of volatiles.

### Example (265a)

a) Molecular Weight: MS(ESI) [M+2H]²⁺ = M/Z 693.4 (m. w. = 1384)
b) The structure is consistent with the Proton Magnetic Resonance Spectral data (300 MHz, CD₃OD/D₂O 1:1)

### Example (265b)

a) Molecular Weight: MS(ESI) [M+2H]²⁺ = M/Z 693.4 (m. w. = 1384)
b) The structure is consistent with the Proton Magnetic Resonance Spectral data (300 MHz, CD₃OD/D₂O 1:1)

### EXAMPLE 265c

### GLYCOPEPTIDE ANTIBIOTICS INCORPORATING 3-(2-THIENYL)-DL-ALANINE

### Cyclo[3-(2-thienyl)-2-aminobutanoyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylserylglycyl]

A 50 mL fermentation of strain LL4690 supplemented with 3-(2-thienyl)-DL-alanine is performed as described in Examples 258 and 259. A sample is centrifuged to remove the cells. The supernatant is analyzed by analytical HPLC and LC/MS as described in Example 260b. A new HPLC peak containing glycopeptide antibiotic 265c with a molecular weight that is predicted if 3-(2-thienyl)-DL-alanine is incorporated in place of phenylalanine is identified.

### Example (265c)

Molecular Weight: MS(ESI) [M+2H]²⁺ = M/Z 651 (m. w. = 1300)

### Example 266a, 266b and 266c

### GLYCOPEPTIDE ANTIBIOTICS INCORPORATING m-FLUORO-DL-TYROSINE

### Example 266a

### Cyclo[glycyl-β-methylphenylalanyl-3-fluoro-O-(4-O-hexopyranosylhexopyranosyl)tyrosyl-3-(2-iminoimidazohdin-4-yl)seryl-3-(3-hexopyranosyl-2-iminoimidazolidin-4-yl)serylseryl]

### Example 266b

### Cyclo[glycyl-β-methylphenylalanyl-3-fluoro-O-[4-O-[2-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl]

### Example 266c

### Cyclo[glycyl-β-methylphenylalanyl-3-fluoro-O-[4-O-[3-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl]

A 50 mL fermentation of strain LL4690 supplemented with m-fluoro-DL-tyrosine is performed as described in Examples 258 and 259. A sample is centrifuged to remove the cells. The supernatant is analyzed by analytical HPLC and LC/MS as described in Example 260b. New HPLC peaks containing glycopeptide antibiotics 266a, 266b, and 266c with molecular weights predicted if m-fluoro-DL-tyrosine is incorporated in place of tyrosine are identified.

### Example (266a)

Molecular Weight: MS(ESI) [M+2H]²⁺ = M/Z 657 (m. w. = 1312)

### Example (266b)

Molecular Weight: MS(ESI) [M+2H]²⁺ = M/Z 699 (m. w. = 1396)

### Example (266c)

Molecular Weight: MS(ESI) [M+2H]²⁺ = M/Z 699 (m. w. = 1396)

### Example 267a, 267b and 267c

### GLYCOPEPTIDE ANTIBIOTICS INCORPORATING 3-AMINO-L-TYROSINE

### Example 267a

### Cyclo[glycyl-β-methylphenylalanyl-3-amino-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]

### Example 267b

### Cyclo[glycyl-β-methylphenylalanyl-3-amino-O-[4-O-[2-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl]

### Example 267c

### Cyclo[glycyl-β-methylphenylalanyl-3-amino-O-[4-O-[3-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl]

A 50 mL fermentation of strain LL4728 supplemented with 3-amino-L-tyrosine is performed as described in Examples 258 and 259. A sample is centrifuged to remove the cells. The supernatant is analyzed by analytical HPLC and LC/MS as described in

### Example 260b

New HPLC peaks containing glycopeptide antibiotics 267a, 267b, and 267c with molecular weights predicted if 3-amino-L-tyrosine is incorporated in place of tyrosine are identified.

### Example (267a)

Molecular Weight: MS(ESI) [M+2H]²⁺ = M/Z 655.5 (m. w. = 1309)

### Example (267b)

Molecular Weight: MS(ESI) [M+2H]²⁺ = M/Z 697.5 (m. w. = 1393)

### Example (267c)

Molecular Weight: MS(ESI) [M+2H]²⁺ = M/Z 697.5 (m. w. = 1393)

### Example 268

### GLYCOPEPTIDE ANTIBIOTICS INCORPORATING S-(+)-PHENYLGLYCINE

### Cyclo[glycyl-phenylglycyl-O-[4-O-[2-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl]

A 50 mL fermentation of strain LL4728 supplemented with S-(+)-phenylglycine is performed as described in Examples 258 and 259. A sample is centrifuged to remove the cells. The supernatant is analyzed by analytical HPLC and LC/MS as described in Example 260b. A new HPLC peak containing glycopeptide antibiotic 268 with a molecular weight predicted if S-(+)-phenylglycine is incorporated in place of phenylalanine is identified.

### Example (268)

Molecular Weight: MS(ESI) [M+2H]²⁺ = M/Z 676 (m. w. = 1350)

### Example 269a, 269b and 269c

### GLYCOPEPTIDE ANTIBIOTICS INCORPORATING O-CHLORO-PHENYLALANINE

### Example 269a

### Cyclo[glycyl-2-chloro-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]

### Example 269b

### Cyclo[glycyl-2-chloro-β-methylphenylalanyl-O-[4-O-[2-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl]

### Example 269c

### Cyclo[glycyl-2-chloro-β-methylphenylalanyl-O-[4-O-[3-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)seryl

A 50 mL fermentation of strain LL4728 supplemented with o-chloro-phenylalanine is performed as described in Examples 258 and 259. A sample is centrifuged to remove the cells. The supernatant is analyzed by analytical HPLC and LC/MS as described in Example 260b. New HPLC peaks containing glycopeptide antibiotics 269a, 269b, and 269c with molecular weights predicted if o-chloro-phenylalanine is incorporated in place of phenylalanine are identified.

### Example (269a)

Molecular Weight: MS(ESI) [M+2H]²⁺ = M/Z 665 (m. w. = 1328)

### Example (269b)

Molecular Weight: MS(ESI) [M+2H]²⁺ = M/Z 707 (m. w. = 1412)

### Example (269c)

Molecular Weight: MS(ESI) [M+2H]²⁺ = M/Z 707 (m. w. = 1412)

### Examples 270 a and 270b

### GLYCOPEPTIDE ANTIBIOTICS INCORPORATING 4-METHYL-VALERIC ACID

### Example 270a

### Cyclo[glycyl-β-methylphenylalanyl-O-[4-O-[2-O-(4-methylpentanoyl)hexopyranosyl]-hexopyranosyl]tyrosyl-3-(2-iminoimidazzolidin-4-yl)seryl-3-(3-hexopyranosyl-2-iminoimidazolidin-4-yl)serylseryl]

### Example 270b

### Cyclo[glycyl-β-methylphenylalanyl-O-[4-O-[6-O-acetyl-3-O-(4-methylpentanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl]

Approximately 45L of supernatant from a 7-day fermentation of strain LL-4690 supplemented with 4-methyl-valeric acid, conducted as described in Examples 258 and 259, is processed using an XAD-7 column. The fraction concentrate containing glycopeptide antibiotics is extracted with 1:4 water/dimethylformamide, as described in example 261. The extract is fractionated by reverse phase HPLC on a C18 column (YMC ODS-A, 10 micron particle size, 70 x 500). The mobile phase consists of a gradient solvent system from 10 to 40% acetonitrile in water with 0.02% trifluoroacetic acid over 62 min at flow rate of 100 mL per min. The broad fraction with a retention time of approximately 58 min is concentrated, and the residue is re-purified on a C18 column (MetaChem ODS3, 5 micron particle size, 20 x 250 mm). The mobile phase is a stepwise solvent system of 21% acetonitrile in water over the first 45 min and 23% acetonitrile in water over the next 25 min, both with 0.02% trifluoroacetic acid and at a flow rate of 7 mL per min. The peak fractions at approximately 19 and 63 min afford 270a and 270b upon evaporation of volatiles.

### Example (270a)

a) Molecular Weight: MS(ESI) [M+2H]²⁺ = M/Z 697.5 (m. w. = 1392)
b) The structure is consistent with the Proton Magnetic Resonance Spectral data (300 MHz, CD₃OD/D₂O 1:1)

### Example (270b)

a) Molecular Weight: MS(ESI) [M+2H]²⁺ = M/Z 718.5 (m. w. = 1434)
b) The structure is consistent with the Proton Magnetic Resonance Spectral data (300 MHz, CD₃OD/D₂O 1:1)

### Example 270 c

### GLYCOPEPTIDE ANTIBIOTICS INCORPORATING 4-METHYL-VALERIC ACID

### Cyclo[glycyl-β-methylphenylalanyl-O-[4-O-[4-O-(4-methylpentanoyl)hexopyranosyl]-hexo-pyranosyl]tyrosyl-3-(2-iminoimidazolidin-4-yl)seryl-3-(3-hexopyranosyl-2-iminoimidazolidin-4-yl)serylseryl]

A 50 mL fermentation of strain LL4666 supplemented with 4-methyl-valeric is performed as described in Examples 258 and 259. A sample is centrifuged to remove the cells. The supernatant is analyzed by analytical HPLC and LC/MS as described in Example 260b. A new HPLC peak containing glycopeptide antibiotic 270c with a molecular weight predicted if 4-methyl-valeric acid is incorporated in place of isovaleric acid is identified.

### Example (270c)

Molecular Weight: MS(ESI) [M+2H]²⁺ = M/Z 697 (m. w. = 1392)

### Example 271a

### GLYCOPEPTIDE ANTIBIOTICS INCORPORATING 3-METHYL-VALERIC ACID

### Cyclo[glycyl-β-methylphenylalanyl-O-[4-O-[3-O-(3-methylpentanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl]

Approximately 10L of supernatant from a 7-day fermentation of strain LL-4690 supplemented with 3-methyl-valeric acid, conducted as described in Examples 258 and 259, is processed using an XAD-7 column. The fraction concentrate that contained glycopeptide antibiotics is extracted with 1:4 water/dimethylformamide, as described in example 261. The extract is fractionated by reverse phase HPLC on a C18 column (YMC ODS-A, 10 micron particle size, 70 x 500). The mobile phase consisted of a gradient solvent system from 20 to 40% acetonitrile in water with 0.02% trifluoroacetic acid over 55 min at flow rate of 100 mL per min. The peak fraction at approximately 43 min affords Example 271a upon evaporation.

### Example (271 a)

a) Molecular Weight: MS(ESI) [M+2H]²⁺ = M/Z 697 (m. w. = 1392)
b) The structure is consistent with the Proton Magnetic Resonance Spectral data (300 MHz, CD₃OD/D₂O 1:1)

### Example 271b and 271c

### GLYCOPEPTIDE ANTIBIOTICS INCORPORATING 3-METHYL-VALERIC ACID

### Example 271b

### Cyclo[glycyl-β-methylohenylalanyl-O-[4-O-[6-O-acetyl-3-O-(3-methylpentanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl]

### Example 271c

### Cyclo[glycyl-β-methylphenylalanyl-O-[4-O-[4-O-(3-methylpentanoyl)hexopyranosyl]-hexopyranosyl]tyrosyl-3-(2-iminoimidazolidin-4-yl)seryl-3-(3-hexopyranosyl-2-iminoimidazolidin-4-yl)serylseryl]

Fermentations in 50mL volumes of strains LL4690 or LL4666 supplemented with 3-methyl-valeric acid are performed as described in Examples 258 and 259. A sample is centrifuged to remove the cells. The supernatant is analyzed by analytical HPLC and LC/MS as described in Example 260b. New HPLC peaks containing glycopeptide antibiotics 271b and 271c with molecular weights predicted if 3-methyl-valeric acid is incorporated in place of isovaleric acid are identified.

### Example (271b)

Molecular Weight: MS(ESI) [M+2H]²⁺ = M/Z 718.5 (m. w. = 1434)

### Example (271c)

Molecular Weight: MS(ESI) [M+2H]²⁺ = M/Z 697 (m. w. = 1392)

### EXAMPLE 272a and 272b

### GLYCOPEPTIDE ANTIBIOTICS CONTAINING BUTYRATE INSTEAD OF ISOVALERATE

### Example 272a

### Cyclo[glycyl-β-methylphenylalanyl-O-[4-O-(2-O-butanoyl-hexopyranosyl)-hexopyranosyl]tyrosyl-3-(2-iminoimidazolidin-4-yl)seryl-3-(3-hexopyranosyl-2-iminoimidazolidin-4-yl)serylseryl]

### Example 272b

### Cyclo[glycyl-β-methylphenylalanyl-O-[4-O-(3-O-butanoyl-hexopyranosyl)-hexopyranosyl]tyrosyl-3-(2-iminoimidazolidin-4-yl)seryl-3-(3-hexopyranosyl-2-iminoimidazolidin-4-yl)serylseryl]

A 50 mL fermentation of strain LL-4690 supplemented with 3-phenyl-butyric acid is performed as described in Examples 258 and 259. A sample is centrifuged to remove the cells. The supernatant is analyzed by analytical HPLC and LC/MS as described in Example 260b. New HPLC peaks containing glycopeptide antibiotics 272a and 272b with molecular weights predicted if butyric acid is incorporated in place of isovaleric acid are identified.

### Example (272a)

Molecular Weight: MS(ESI) [M+2H]²⁺ = M/Z 683 (m. w. = 1364)

### Example (272b)

Molecular Weight: MS(ESI) [M+2H]²⁺ = M/Z 683 (m. w. = 1364)

### Example 273a and 273b

### GLYCOPEPTIDE ANTIBIOTICS CONTAINING HEXANOIC ACID INSTEAD OF ISOVALERIC ACID

### Example 273a

### Cyclo[glycyl-β-methylphenylalanyl-O-[4-O-(2-O-hexanoyl-hexopyranosyl)-hexopyranosyl]tyrosyl-3-(2-iminoimidazolidin-4-yl)seryl-3-(3-hexopyranosyl-2-iminoimidazolidin-4-yl)serlseryl]

### Example 273b

### Cyclo[glycyl-β-methylphenylalanyl-O-[4-O-(3-O-hexanoyl-hexopyranosyl)-hexopyranosyl]tyrosyl-3-(2-iminoimidazolidin-4-yl)seryl-3-(3-hexopyranosyl-2-iminoimidazolidin-4-yl)serylseryl]

A 50 mL fermentation of strain LL-4690 supplemented with 2-methyl-hexanoic acid is performed as described in Examples 258 and 259. A sample is centrifuged to remove the cells. The supernatant is analyzed by analytical HPLC and LC/MS as described in Example 260b. New HPLC peaks containing glycopeptide antibiotics 273a and 273b with molecular weights predicted if hexanoic acid is incorporated in place of isovaleric acid are identified.

### Example (273a)

Molecular Weight: MS(ESI) [M+2H]²⁺ = M/Z 697 (m. w. = 1392)

### Example (273b)

Molecular Weight: MS(ESI) [M+2H]²⁺ = M/Z 697 (m. w. = 1392)

### Example 274a and 274b

### GLYCOPEPTIDE ANTIBIOTICS INCORPORATING n-HEPTANOIC ACID

### Example 274a

### Cyclo[glycyl-β-methylphenylalanyl-O-[4-O-(2-O-heptanoyl-hexopyranosyl)-hexopyranosyl]tyrosyl-3-(2-iminoimidazolidin-4-yl)seryl-3-(3-hexopyranosyl-2-iminoimidazolidin-4-yl)serylseryl]

### Example 274b

### Cyclo[glycyl-β-methylphenylalanyl-O-[4-O-(3-O-heptanoyl-hexopyranosyl)-hexopyranosyl]tyrosyl-3-(2-iminoimidazolidin-4-yl)seryl-3-(3-hexopyranosyl-2-iminoimidazolidin-4-yl)serylseryl]

A 50 mL fermentation of strain LL-4690 supplemented with n-heptanoic acid is performed as described in Examples 258 and 259. A sample is removed and centrifuged to remove the cells. The supernatant is analyzed by analytical HPLC and LC/MS as described in Example 260b. New HPLC peaks containing glycopeptide antibiotics 274a and 274b with molecular weights predicted if n-heptanoic acid is incorporated in place of isovaleric acid are identified.

### Example (274a)

Molecular Weight: MS(ESI) [M+2H]²⁺ = M/Z 704 (m. w. = 1406)

### Example (274b)

Molecular Weight: MS(ESI) [M+2H]²⁺ = M/Z 704 (m. w. =1406)

### Example 275

### Cyclo[glycyl-β-methylphnylalanyl-O-[4-O-[6-O-acetyl-3-O-(3-methylbutanoyl)hexopyranosyl]hexopyranosyl]tyrosyl-3-(2-iminoimidazolidin-4-yl)seryl-3-(3-hexopyranosyl-2-iminoinlidazolidin-4-yl)serylseryl]

Strain LL4614 is fermented for 4 days in medium BPM17. The supernatant from 7 L of fermentation broth is applied to a column containing XAD-7 resin. The column is sequentially washed with water (2 L), methanol (2 L) and water (2 L) followed by elution with 1:1 acetonitrile in water containing 0.1% trifluoroacetic acid (3L). The acetonitrile/water fraction is concentrated under reduced pressure to a small volume and the residue is applied to a reverse phase HPLC column (ODS-A, 10 micron particle size, 70 x 500 mm). The column is developed with a gradient of from 14 to 50% acetonitrile in water with 0.02% trifluoroacetic acid over 55 min at a flow rate of 100 mL per minute. The fraction with a retention time of approximately 37 min is collected and evaporated to afford (Example 275) MS (+ES) *m*/*z:* 711.4 (M+2H)²⁺.

### Example 276a

### Cyclo[glycyl-β-methylphenylalanyl-O-[4-O-[6-O-acetyl-hexopyranosyl]hexopyranosyl]tyrosyl-3-(2-iminoimidazolidin-4-yl)seryl-3-(3-hexopyranosyl-2-iminoimidazolidin-4-yl)serylseryl]

### Example 276b

### Cyclo[glycyl-β-methylphenylalanyl-O-[4-O-[6-O-acetyl-2-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryll

Strain LL4614 is fermented for 5 days in BPM17. Analysis of fermentation extracts prepared with carboxylic acid extraction columns by LC/MS showed the expected presence of an HPLC peak at RRT 1.79 with an associated molecular weight of 1420 which is cyclo[glycyl-β-methylphenylalanyl-O-[4-O-[6-O-acetyl-3-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl] (example 275) MS (+ES) *m*/*z:* 711.4 (M+2H)²⁺. The analysis also indicates the presence of compounds with masses of 1337 and 1420 with relative retention times of 1.17 and 1.73. The compound eluting at RRT1.17 is the first title compound (Example 276a) MS (+ES) *m*/*z* 669 (M+2H)²⁺. The compound eluting at RRT 1.73 is the second title compound (Example 276b) MS (+ES) *m*/*z:* 711 (M+2H)²⁺.

### Example 277a

### Cyclo[3-(2-iminoimidazolidin-4-yl)alanyl-3-(3-hexopyranosyl-2-iminoimidazolidin-4-yl)-alanylserylglycyl-β-methylphenylalanyl-O-(4-O-hexopyranosylhexopyranosyl)tyrosyl]

### Example 277b

### Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[2-O-(3-methylbutanoyl)hexopyranosyl]-hexopyranosyl]tyrosyl]

### Example 277c

### Cyclo[3-(2-iminoimidazolidin-4-yl)alanyl-3-(3-hexopyranosyl-2-iminoimidazolidin-4-yl)-alanylserylglycyl-β-methylphenylalanyl-O-[4-O-[3-O-(3-methylbbtanoyl)hexorpyranosyl]-hexopyranosyl]tyrosyl]

Strain LL4641 is fermented for 5 days in medium BPM17. Three peaks are observed at RRT 1.04, 1.47 and 1.55. To afford isolation of these compounds, clarified fermentation broth (8.5 L) is applied to a column containing XAD7 resin. The column is sequentially washed with water (2 L), methanol (2 L) and water (2 L). The column is then eluted with acetonitrile-water (50:50) containing 0.5% TFA. The acetonitrile/water solution is evaporated to a small volume under reduced pressure, and the residue is fractionated by reverse phase HPLC on a C18 column (ODS-A, 10 micron particle size, 70 x 500 mm). The mobile phase employed is a gradient from 14 to 50% acetonitrile in water with 0.02% trifluoroacetic acid over 55 min at a flow rate of 100 mL per min. Fractions eluting at approximately 25 min, 31 min, and 34 min are collected and after evaporation of solvents, afford (Example 277a) MS (+ES) *m*/*z*: 632.2 (M+2H)²⁺, (Example 277b) MS (+ES) *m*/*z:* 674.2 (M+2H)²⁺, and (Example 277c) MS (+ES) *m*/*z:* 674.1 (M+2H)²⁺.

Alternatively, five-day shake-flask fermentations of strain LL4783 are performed in medium BPM17statgal. LC/MS analysis of fermentation extracts prepared via carboxylic acid extraction columns shows the presence of compounds with RRTs of 1.04, 1.47 and 1.55with associated molecular weights of 1262, 1346 and 1346. The data indicates that mutant LL4783 is accumulating (Example 277a) MS (+ES) *m*/*z:* 632.2 (M+2H)²⁺, (Example 277b) MS (+ES) *m*/*z:* 674.2 (M+2H)²⁺, and (Example 277c) MS (+ES) *m*/*z:* 674.1 (M+2H)²⁺.

### Example 278a

### Cyclo[glycyl-β-methylphenylalanyl-O-hexopyranosyltyrosyl-3-(2-iminoimidazolidin-4-yl)-seryl-3-(2-iminoimidazolidin-4-yl)serylseryl]

### Example 278b

### Cyclo[glycyl-β-methylphenylalanyl-O-[4-O-[4-O-(2-methylbut-2-enoyl)hexopyranosyl]-hexopyranosylityrosyl-3-(2-iminoimidazolidin-4-yl)seryl-3-(3-hexopyranosyl-2-imino-imidazolidin-4-yl)serylseryl]

### Example 278c

### Cyclo[glycyl-β-methylphenylalanyl-O-[4-O-[4-O-(2-methylbutanoyl)hexopyranosyl]hexopyranosyl]tyrosyl-3-(2-iminoimidazolidin-4-yl)seryl-3-(3-hexopyranosyl-2-iminoimidazohdin-4-yl)serylseryl]

### Example 278d

### Cyclo[glycyl-β-methylphenylalanyl-O-[4-O-[3-O-(4-methylpentanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl]

### Example 278e

### Cyclo[glycyl-β-methylphenylalanyl-O-[4-O-[4-O-(3-methylbutanoyl)hexopyranosyl]hexopyranosyl]tyosyl-3-(2-iminoimidazolidin-4-yl)seryl-3-(3-hexopyranosyl-2-iminoimidazolidin-4-yl)serylseryl]

Preliminary shake-flask fermentations of strain LL4666 are conducted in medium BPM17. HPLC and LC/MS analysis are then conducted on concentrates of fermentation broth prepared with carboxylic acid concentration columns. A metabolite with RRT 1.82 and molecular weight of 1378 is observed and identified as cyclo[glycyl-β-methylphenylalanyl-O-[4-O-[4-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl] (Example 278e) MS (+ES) *m*/*z:* 690 (M+2H)²⁺.

Five other separable metabolites, four with RRTs of >1.85, and one with RRT ∼ 1.20, are observed which display molecular weights of 1392, 1392, 1376, 1378 and 970. Larger volume fermentations are then conducted to afford the isolation of these five compounds.

Multiple shake-flask fermentations of mutant LL4666 are carried out for 5 days in medium BPM17. The multiple shake-flasks are then pooled to yield 17 L of fermentation broth. The pooled broth is centrifuged and the supernatant is loaded onto a pretreated XAD-7 column (1 L). The column is sequentially washed with water (2 L), methanol (2 L) and water (2 L) to remove media components and pigments. The compounds are then eluted with 1:1 acetonitrile in water containing 0.1% trifluoroacetic acid (3 L). The acidic acetonitrile/water solution is evaporated to a small volume under reduced pressure, and the residue is fractionated by reverse phase HPLC on a C18 column (ODS-A, 10 micron particle size, 70 x 500 mm). The mobile phase consists of a gradient from 14 to 50% acetonitrile in water with 0.02% trifluoroacetic acid over 55 min at a flow rate of 100 mL per min.

The small peak eluting at approximately 27 min and the broad peak at approximately 38 min are both collected and concentrated under reduced pressure. The residue from the former peak is subjected to further separation by HPLC on a C18 column (ODS-A, 8 micron particle size, 20 x 250 mm). The mobile phase consists of a gradient from 10 to 60% acetonitrile in water with 0.02% trifluoroacetic acid over 60 min at a flow rate of 20 mL per minute. The fraction eluting at approximately 25 min affords (Example 278a) MS (+ES) *m*/*z;* 486.2 (M+2H)²⁺.

The residue of the latter peak is found to be a rather complicated mixture by HPLC analysis and a part of it (∼1/5) is subjected to further separation by HPLC on a C18 column (YMC ODS-basic, 5 micron particle size, 10 x 250 mm). The mobile phase consists of a gradient from 26.5 to 28.8% acetonitrile in water with 0.02% trifluoroacetic acid over 15 min at a flow rate of 4 mL per minute. One fraction eluting at approximately 13.0 min was found to be identical to cyclo[glycyl-β-methylphenylalanyl-O-[4-O-[3-O-(3-methylpentanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl] (Example 271a) MS (+ES) *m*/*z:* 697 (M+2H)²⁺.

The fractions eluting at approximately 8.6, 9.7, 10.2 and 11.5 min are collected to afford (Example 278b) MS (+ES) *m*/*z:* 689.4 (M+2H)²⁺, (Example 278c) MS (+ES) *m*/*z:* 690.1 (M+2H)²⁺ and (Example 278d) MS (+ES) *m*/*z:* 697.4 (M+2H)²⁺.

### Example 279a

### Cyclo[glycylphenylalanyltyrosyl-3-(2-iminoimidazolidin-4-yl)seryl-3-(3-hexopyranosyl-2-iminoimidazolidin-4-yl)serylseryl]

### Example 279b

### Cyclo[glycyl-β-methylphenylalanytyrosyl-3-(2-iminoimidazolidin-4-yl)selyl-3-(3-hexopyranosyl-2-iminoimidazolidin-4-yl)serylseryl]

Shake-flask fermentations of strain LL4779 are conducted in medium BPM17statgal for 5 days. HPLC and LC/MS analysis of the fermentation supernatants and concentrates prepared using carboxylic acid extraction columns indicate the presence of an HPLC peak at RRT 1.29 with an associated molecular weight of 970 which is cyclo[glycyl-β-methylphenylalanyltyrosyl-3-(2-iminoimidazolidin-4-yl)seryl-3-(3-hexopyranosyl-2-iminoimidazolidin-4-yl)serylseryl] (Example 279b) MS (+ES) *m*/*z:* 486 (M+2H)²⁺_{.}

A second peak is observed at RRT 1.19 displaying a molecular weight of 956.To afford isolation of the RRT 1.19 compound, LL4779 is fermented at the 300 L scale in medium BPM17statgal. Clarified broth is loaded onto a column containing XAD-7 resin, which is then washed with two column volumes of methanol-water (50:50) and eluted with 8 column volumes of methanol-water-trifluoroacetic acid (50:50:0.2). Finally, the resin is washed with 4 column volumes of water. Fractions and washes are analyzed and the active fractions and washes are pooled, neutralized to pH 4-6 and concentrated to 3-4 L. A portion of the crude concentrated extract (0.5 L) is centrifuged and the supernatant is added into 1.5 L acetonitrile to afford a precipitate. The precipitate is collected by centrifugation, re-dissolved in water and loaded onto a C18 reversed-phase silica gel flash column (200 g). The column is eluted sequentially with water (1 L), 10% acetonitrile/90% water with 0.1% hydrochloric acid (0.5 L) and 15% acetonitrile/85%water with 0.1% hydrochloric acid. The 10% acetonitrile eluate is collected and evaporated to 200 mL to enrich for the RRT 1.19 component. The concentrated solution is loaded onto a C18 reversed-phase silica gel flash column (100 g) and eluted with water (0.5 L), 9% acetonitrile/91% water with 0.1% hydrochloric acid (1L), 10% acetonitrile/90% water with 0.1% hydrochloric acid (2L) sequentially, and the eluates are collected at 200 mL per fraction. Fraction-2 and fraction-3 are collected and combined, and after solvent evaporation, afford (Example 279a) MS (+ES) *m*/*z:* 479 (M+2H)²⁺.

### Example 280a

### Cyclor[glycylphenylalanyltyrosyl-3-(2-iminoimidazolidin-4-yl)seryl-3-(2-iminoimidazolidin-4-yl)serylseryl]

### Example 280b

### Cyclo[glycyl-β-methylphenylalanyltyrosyl-3-(2-iminoimidazolidin-4-yl)seryl-3-(2-iminoimidazolidin-4-yl)serylseryl]

Strain LL4744 is fermented in medium BPM17statgal for 5 days. HPLC and LC/MS analysis of fermentation supernatants and concentrates prepared using carboxylic acid extraction columns show the presence of two peaks displaying relative retention times of 1.17 and 1.30 with molecular weights of 794 and 808. To isolate these compounds, clarified broth (300L) is loaded onto a column containing XAD7 resin. The resin is washed with two column volumes of methanol-water (50:50), eluted with 8 column volumes of methanol-water-trifluoroacetic acid (50:50:0.2) and finally washed with 4 column volumes of water. The active fractions and washes are pooled, the pH adjusted to 4-6 with sodium hydroxide and concentrated to 3-4 L.

A portion of the resulting crude oil (1.4 L) is centrifuged. The supernatant is collected, and after adjusting the pH to 7.0, loaded onto a pre-treated BAKERBOND carboxylic acid silica gel flash column (250 g). The column is eluted with water (2.5 L), 40% acetonitrile/60% water with 0.1% hydrochloric acid (1L), and 70% acetonitrile/30% water with 0.5% trifluoroacetic acid (1.5 L), sequentially.

The aqueous acetonitrile eluates are combined and evaporated to obtain a crude mixture of the RRT 1.17 and 1.30 compounds. This mixture is dissolved in water and loaded onto a reversed-phase C18 silica gel flash column (210 g) for further separation. The column is washed with water (0.5 L), 10% acetonitrile/90% water containing 0.1% trifluoroacetic acid (1 L), and 15% acetonitrile/85% water containing 0.1% trifluoroacetic acid (2 L). All eluates are collected at 100 mL per fraction. Fractions 9 to 12 are combined and solvents are evaporated under reduced pressure to afford (Example 280a) MS (+ES) *m*/*z:* 398 (M+2H)²⁺. Fractions 15 to 20 are combined and solvents are evaporated under reduced pressure to afford (Example 280b) MS (+ES) *m*/*z:* 405 (M+2H)²⁺.

Alternatively, the title compounds are prepared by fermentation of mutants LL4742 or LL4902 in media BPM17, BPM17stat or BPM17statgal. HPLC and LC/MS analysis of fermentation supernatants and concentrates prepared using carboxylic acid extraction columns show that strains LL4742 and LL4902 are both producing two compounds which display relative retention times of 1.17 and 1.30 with molecular weights of 794 and 808 which are (Example 280a) MS (+ES) *m*/*z:* 398 (M+2H)²⁺ and (Example 280b) MS (+ES) *m*/*z:* 405 (M+2H)²⁺.

Alternatively, strain BD2 is fermented for 5 days in medium BPM17statgal. HPLC and LC/MS analysis of fermentation supernatants and concentrates prepared using carboxylic acid extraction columns show the presence of a single peak at RRT 1.17 with an associated molecular weight of 794, indicating the production of (Example 280a) MS (+ES) *m*/*z:* 398 (M+2H)²⁺.

Alternatively, strains BD20 or BD70 are fermented for 5 days in medium BPM27-man. HPLC and LC/MS analysis of fermentation supernatants and concentrates prepared using carboxylic acid extraction columns show the presence of a single peak at RRT 1.17 with an associated molecular weight of 794, indicating the production of (Example 280a) MS (+ES) *m*/*z:* 398 (M+2H)²⁺.

### Example 281a

### Cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl],

### Example 281b

### Cyclor[glycyl-β-methylphenylalanyl-O-[4-O-[2-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl]

### Example 281c

### Cyclo[glycyl-β-methylphenylalanyl-O-[4-O-[3-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl]

Shake-flask fermentations of strains LL4742 and LL4902 are conducted in medium BPM17statman. Supernatants and concentrates are prepared from 5 day fermentation broth and analyzed by HPLC and LC/MS. Strains LL4742 and LL4902 fermented in BPM17statman accumulate metabolites with RRTs of 1.00, 1.43 and 1.64, displaying molecular weights of 1294, 1378 and 1378. The data indicate that mannose supplementation of strains LL4742 and LL4902 restores their ability to produce cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl (Example 281a) MS (+ES) *m*/*z:* 648 (M+2H)²⁺], cyclo[glycyl-β-methylphenylalanyl-O-[4-O-[2-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl] (Example 281b) MS (+ES) *m*/*z:* 690 (M+2H)²⁺, and cyclo[glycyl-β-methylphenylalanyl-O-[4-O-[3-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl] (Example 281c) MS (+ES) *m*/*z:* 690 (M+2H)²⁺_{.}

### Example 282

### Cyclo[glycylphenylalanyl-O-(4-O-hexopyranosylhexopyranosyl)tyrosyl-3-(2-iminomidazolidin-4-yl)seryl-3-(3-hexopyranosyl-2-iminoimidazolidin-4-yl)serylseryl]

Strain BD2 is fermented in BPM17statman for 5 days. HPLC and LC/MS analysis of fermentation supernatants and carboxylic acid concentrates indicate the accumulation of three peaks with novel retention times. The major component shows an RRT of 0.86 and a molecular weight of 1280. To afford isolation of the major component at RRT 0.86, cell free supernatant (7 L) is applied to a column containing CG-61 resin (600 mL). The column is washed with water (1 L) and eluted with 40% acetonitrile/ 60% water/ 0.01 % TFA (2.5 L). Fractions containing the title compound are collected and pooled. After evaporation of the solvents, the pH of the resulting concentrate is adjusted to 12.5 with NaOH and held for 45 min to hydrolyze esters. The pH of the concentrate is then adjusted to 3.0 with HCl and acetonitrile (5 volumes) is added. The resulting precipitated material is collected, washed with acetonitrile and dried to afford the title compound, (Example 282) MS (+ES) *m*/*z:* 641 (M+2H)²⁺. Alternatively, strains BD20 or BD70 are fermented for 5 days in medium BPM27. HPLC and LC/MS analysis of fermentation supernatants and concentrates prepared using carboxylic acid extraction columns show the presence of a major metabolite at RRT 0.86 with an associated molecular weight of 1280, indicating the production of (Example 282) MS (+ES) *m*/*z:* 641 (M+2H)²⁺.

Alternatively, strain BD70 is fermented for 5 days in medium BPM27 at the 300 L scale. Diatomaceous earth (4 kg) is added and the broth is filtered. The filtrate is then cooled to 4°C, adjusted to pH 12.8 with sodium hydroxide and held for 3 hours. The filtrate is then neutralized with acetic acid and loaded onto a 30 L column of SP207 resin. The resin is washed with 4 bed volumes of water, 4 bed volumes of methanol and eluted with 6 bed volumes of 50/50 methanol/water with 3% acetic acid. The eluate is concentrated to about 4 L by evaporation under vacuum. The concentrate is then precipitated by the addition of 3 volumes of isopropyl alcohol and one volume of acetonitrile. The resulting precipitate is filtered, washed with methanol and dried under vacuum to afford the title compound, (Example 282) MS (+ES) *m*/*z*: 641 (M+2H)²⁺_{.}

### Example 283a

### Isolation of Cyclo[glycylphenylalanyl-O-[4-O-[6-O-(2-methylpropanoyl)-hexopyranosyl]hexopyranosyl]tyrosyl-3-(2-iminoimidazolidin-4-yl)seryl-3-(3-hexopyranosyl-2-iminoimidazolidin-4-yl)serylseryl]

### Example 283b

### Cyclo[glycylphenylalanyl-O-[4-O-[3-O-(2-methylpropanoyl)-hexopyranosyl]hexopyranosyl]tyrosyl-3-(2-iminoimidazolidin-4-yl)seryl-3-(3-hexopyranosyl-2-iminoimidazolidin-4-yl)serylseryl]

### Example 283c

### Cyclo[glycylphenylalanyl-O-[4-O-[6-O-(3-methylbutanoyl)-hexopyranosyl]hexopyranosyl]tyrosyl-3-(2-iminoimidazolidin-4-yl)seryl-3-(3-hexopyranosyl-2-iminoimidazolidin-4-yl)serylseryl]

Strain BD20 is fermented in medium BPM27 for 5 days. HPLC and LC/MS analysis of fermentation supernatants indicates that a series of peaks are produced in addition to the expected major component, cyclo[glycylphenylalanyl-O-(4-O-hexopyranosylhexopyranosyl)tyrosyl-3-(2-iminomidazolidin-4-yl)seryl-3-(3-hexopyranosyl-2-iminoimidazolidin-4-yl)serylseryl] (Example 282) MS (+ES) *m*/*z:* 641 (M+2H)²⁺_{.}

The compounds were observed to elute in two clusters with central RRTs of approximately 1.35 and 1.55. The three most prominent peaks observed show RRTs of 1.34, 1.38 and 1.52, with apparent molecular weights of 1350.6, 1350.6 and 1364.6.

To afford isolation of the compounds, strain BD20 is fermented in medium BPM27 for 5 days at the 300 L scale. The clarified broth from the fermentation is applied to XAD7 resin, eluted and fractions containing the compounds of interest are identified and concentrated as in example 279. A portion of the resulting crude oil (0.3 L) is centrifuged and the supernatant loaded onto a pre-treated C18 reverse phase silica gel flash column (250 g). The column is washed sequentially with water (1 L), 10% acetonitrile/90% water with 0.1% trifluoroacetic acid (3 L), 12.5 % acetonitrile/87.5% water with 0.1% trifluoroacetic acid (6 L), and 15% acetonitrile/85% water with 0.1% trifluoroacetic acid (6 L). Column eluates are then collected at 400 mL/fraction. Fractions 13 to 17 are combined to produce a crude mixture which is further fractionated by preparative HPLC on a YMC ODS-A column using a gradient of 12% to 30% acetonitrile in water with 0.01% trifluoroacetic acid over 60 min at a flow rate of 20 mL per minute. The appropriate fractions are collected and solvents are evaporated to afford (Example 283a) MS (+ES) *m*/*z:* 676.5 (M+2H)²⁺ and (Example 283b) MS (+ES) *m*/*z:* 676.5 (M+2H)²⁺.

Fractions 30 to 33 from the C18 reverse phase silica gel flash column elution are combined and evaporated to dryness. The resulting crude material is further purified on a C18 reverse phase flash column (100 g) via elution with 12.5% acetonitrile/87.5% water containing 0.01% TFA to afford (Example 283c) MS (+ES) *m*/*z:* 683.3 (M+2H)²⁺.

### Example 284a

### Cyclo[3-(2-iminoimidazolidin-4-yl)alanyl-3-(3-hexopyranosyl-2-iminoimidazolidin-4-yl)-serylserylglycyl-β-methylphenylalanyl-O-(4-O-hexopyranosylhexopyranosyl)tyrosyl]

### Example 284b

### Cyclo[3-(3-hexopyranosyl-2-iminoimidazolidin-4-yl)-alanylserylglycyl-β-methylphenylalanyl-O-(4-O-hexopyranosylhexopyranosyl)tyrosyl-3-(2-iminoimidazolidin-4-yl)seryl]

### Example 284c

### Cyclo[3-(2-iminoimidazolidin-4-yl)alanyl-3-(3-hexopyranosyl-2-iminoimidazolidin-4-yl)-alanylserylglycylphenylalanyl-O-(4-O-hexopyranosylhexopyranosyl)tyrosyl]

Strain BD20 is fermented in medium BPM27 for 5 days. An equal volume of 50 mM CAPS buffer (pH 13) is mixed with broth to hydrolyze ester components. After 5 min incubation at room temperature, three volumes of 1M MOPS buffer (pH 7.0) is added to neutralize the mixture, which is then filtered and analyzed by modified HPLC and LC/MS procedures. The modified chromatographic system employs a YMC ODS-AQ 4.6 x 250 mm HPLC column. The chromatography is performed in the isocratic mode at 1.5 mL/min at 40°C for 60 min employing a mobile phase of 10% acetonitrile:90% water:0.01% trifluoroacetic acid. Relative retention times (RRT) for metabolites showing characteristic UV absorption spectra are calculated by dividing the peak retention times of compounds by that of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (Example 281a). LC/MS analysis is performed using an analogous chromatographic system.

The analysis of the saponified broth reveals the presence of a major component at RRT 0.62 with an associated molecular weight of 1280, which is cyclo[glycylphenylalanyl-O-(4-O-hexopyranosylhexopyranosyl)tyrosyl-3-(2-iminomidazolidin-4-yl)seryl-3-(3-hexopyranosyl-2-iminoimidazolidin-4-yl)serylseryl] (Example 282) MS (+ES) *m*/*z:* 641 (M+2H)²⁺.

Three additional components are also detected. Two of the additional compounds show RRTs of 0.66 and 0.73 with associated molecular weights of 1264 and 1248 and are, respectively, (Example 284a) MS (+ES) *m*/*z:* 633 (M+2H)²⁺ and (Example 284c) MS (+ES) *m*/*z:* 625 (M+2H)².

The third compound displays an RRT of 0.71 with an associated molecular weight of 1264. To afford isolation of the RRT 0.71 compound, strain BD20 is fermented in shake-flasks at 30°C for 5 days in a modified BPM27 fermentation medium. The modified formulation employs the reported BPM27 recipe with the following changes: the addition of 20 g/L galactose and 60 mg/L FeSO₄ 7H₂O. Pharmamedia is batched at 60 g/L rather than the normal 20 g/L.

The resulting fermentation broth (3.8 L) is centrifuged and the supernatant loaded onto a pretreated CG-71C column (0.4 L) which is sequentially washed with water (0.4 L), 0.1% sodium chloride (0.8 L) and then eluted with 50% acetonitriole/50% water containing 0.05% trifluroacetic acid (1 L). The acidic aqueous acetonitrile eluate is collected and concentrated to a small volume (0.1 L). This concentrated solution is loaded onto a C18 reverse phase silica gel flash column (140 g), washed with water (0.5 L), and then eluted with a gradient of aqueous methanol (18% methanol / 82% water to 25% methanol / 75% water containing 0.05% trifluroacetic acid, total 4 L). All eluates are collected at 250 mL per fraction. Fractions 8 to 16 are combined and the solvent evaporated under reduced pressure. The resulting crude material is further purified by preparative HPLC on a YMC ODS-A column using a gradient of from 10.8% to 14.0% acetonitrile / water containing 0.01% TFA at a flow rate of 18 mL per minute to afford (Example284b) MS (+ES) *m*/*z*: 633.4 (M+2H)²⁺.

### Example 285a

### Cyclo[glycylphenylalanyl-O-[4-O-[3-O-(3-methylbutanoyl)-hexopyranosyl]hexopyranosyl]tyrosyl-3-(2-iminoimidazolidin-4-yl)seryl-3-(3-hexopylanosyl-2-iminoimidazolidin-4-yl)serylseryl]

### Example 285b

### Cyclo[glycylphenylalanyl-O-[4-O-[3-O-(3-methylbutanoyl)-hexopyranosyl]hexopyranosyl]tyrosyl-3-(2-iminoimidazolidin-4-yl)seryl-3-(3-hexopyranosyl-2-iminoimidazolidin-4-yl)serylseryl

LL4780 mediated biotransformations are performed as described in the experimental biotransformation methods section. LL4780 processes the exogenously added metabolites cyclo[glycyl-β-methylphenylalanyltyrosyl-3-(2-iminoimidazolidin-4-yl)seryl-3-(3-hexopyranosyl-2-iminoimidazolidin-4-yl)serylseryl] (Example 279b) or cyclo[glycyl-β-methylphenylalanyltyrosyl-3-(2-iminoimidazolidin-4-yl)seryl-3-(2-iminoimidazolidin-4-yl)serylseryl] (Example 280b) to a mixture of metabolites exhibiting RRTS of 1.00, 1.43 and 1.64 with associated molecular weights of 1294, 1378 and 1378 which are cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (Example 281a) MS (+ES) *m*/*z:* 648(M+2H)²⁺], cyclo[glycyl-β-methylphenylalanyl-O-[4-O-[2-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl] (Example 281b) MS (+ES) *m*/*z:* 690 (M+2H)²⁺, and cyclo[glycyl-β-methylphenylalanyl-O-[4-O-[3-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl]) (Example 281c) MS (+ES) *m*/*z:* 690 (M+2H)²⁺.

The addition of cyclo[glycylphenylalanyltyrosyl-3-(2-iminoimidazolidin-4-yl)seryl-3-(2-iminoimidazolidin-4-yl)serylseryl] (Example 280a) to fermentations of mutant LL4780 resulted in the formation of biotransformation products at RRT 0.86, 1.33 and 1.55 with associated molecular weights of 1280, 1364 and 1364. The RRT 0.86 product is cyclo[glycylphenylalanyl-O-(4-O-hexopyranosylhexopyranosyl)tyrosyl-3-(2-iminomidazoli din-4-yl)seryl-3-(3-hexopyranosyl-2-iminoimidazolidin-4-yl)serylseryl]_(Example 282) MS (+ES) *m*/*z:* 641 (M+2H)²⁺.

The two compounds observed at RRT 1.33 and 1.55 are (Example 285a) MS (+ES) *m*/*z:* 683 (M+2H)²⁺ and (Example 285b) MS (+ES) *m*/*z:* 683 (M+2H)²⁺.

### Example 286

### Cyclo[glycyl-β-methylphenylalanyl-O-hexopyranosyl-2-(nitro)tyrosyl-3-(2-iminoimidazolidin-4-yl)seryl-3-(3-hexopyranosyl-2-iminoimidazolidin-4-yl)serylseryl]

(Cyclo[glycyl-β-methylphenylalanyl-3-nitrotyrosyl-3-(2-iminoimidazolidin-4-yl)seryl-3-(3-hexopyranosyl-2-iminoimidazolidin-4-yl)serylseryl]) (Example 8) is added to multiple shake-flask fermentations of strain LL4780 as described in the biotransformation methods section. Three-day fermentation broth is collected and pooled, yielding 4.0 L. Concentrates of a small sample of the pooled fermentation broth are analyzed by LC/MS which indicates the appearance of a peak at RRT 1.17 with an associated molecular weight of 1178.

For purification, 4 L of broth is centrifuged and the supernatant loaded onto a pretreated CG-71C column (0.2 L). The column is sequentially washed with water (0.2 L), 0.1% aqueous sodium chloride solution (0.5 L), 50% acetonitriole/50% water with 0.1% hydrochloric acid (0.7 L), and methanol (0.5 L). The acidic aqueous acetonitrile eluates are combined and evaporated to a small volume (0.05 L). The residue is added into acetonitrile/methanol (150:50) to afford a crude precipitate (120 mg) which is then dissolved in 50 mL water and loaded on a C18 reversed-phase silica gel flash column (150 g) for further purification. The flash column is eluted with water (0.5 L), and a gradient of aqueous acetonitrile (10% acetonitrile/water to 15% acetonitrile/water with 0.1% hydrochloric acid). Eluates are collected at 400 mL per fraction. Fractions15 to 24 are combined and the solvents are evaporated under reduced pressure to afford (Example 286) MS (+ES) *m*/*z*: 590 (M+2H)²⁺.

### Example 287

### Cylo[glycyl-β-methylphenylalanyl-2-amino-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]

(Cyclo[glycyl-β-methylphenylalanyl-3-aminotyrosyl-3-(2-iminoimidazolidin-4-yl)seryl-3-(3-hexopyranosyl-2-iminoimidazolidin-4-yl)serylseryl]) (Example 11) is added to multiple shake-flask fermentations of strain LL4780 as described in the biotransformation methods section. Three-day fermentation broth is collected and pooled, yielding 4.25 L. Concentrates of a small sample of the pooled fermentation broth are prepared using carboxylic acid extraction columns which are then analyzed by LC/MS. The analysis indicates the appearance of a peak at RRT 0.94 with an associated molecular weight of 1310.

For isolation, 4 L of broth is centrifuged and the supernatant is loaded onto a pretreated BAKERBOND carboxylic acid silica gel column (200 g). The column is sequentially washed with water (0.8 L) and 70% acetonitrile/30% water with 0.5% trifluoroacetic acid (0.8 L). The acidic acetonitrile eluates are evaporated to 50 mL and added into acetonitrile/methanol (150:50) to obtain a crude precipitate which is then dissolved in water. This crude material is then purified by reverse phase HPLC employing a mobile phase gradient from 5% to 26% of acetonitrile in water with 0.01% trifluoroacetic acid applied over 35 min at a flow rate of 8 mL per minute. An HPLC peak with a retention time of approximately 10.3 min is collected. Evaporation of solvents under reduced pressure affords (Example 287) MS (+ES) *m*/*z:* 656 (M+2H)²⁺_{.}

### Example 288

### Example 281a

### Cyclo[glycyl-β-methylphenlyalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]

### Example 281b

### Cyclo[glycyl-β-methylphenylalanyl-O-[4-O-[2-O-(3-methylbutanoyl)hexopyranosyl]hexopyranosyl]tyrosyl-3-(2-iminoimidazolidin-4-yl)seryl-3-(3-hexopyranosyl-2-iminoimidazolidin-4-yl)serylseryl]

### Example 281c

### Cyclo[glycyl-β-methylphenylalanyl-O-[4-O-[3-O-(3-methylbutanoyl)Hexopyl]anosyl]hexopyranosyl]tyrosyl-3-(2-iminoimidazolidin-4-yl)seryl-3-(3-hexopyranosyl-2-immoimidazolidin-4-yl)serylseryl]

Strain LL4773 is fermented in medium BPM17statgal for 5 days. Supernatant samples prepared from the final fermentation broth are analyzed by HPLC and LC/MS. Three prominent HPLC peaks are observed at RRT 1.00, RRT 1.43 and RRT 1.64 with associated molecular weights of 1294, 1378 and 1378. The compounds produced are identical to cyclo[glycyl-β-methylphenylalanyl-O-(4-O-hexopyranosylhexopyranosyl) tyrosyl-3-(2-iminoimidazolidin-4-yl)seryl-3-(3-hexopyranosyl-2-iminoimidazolidin-4-yl)serylseryl] (Example 281a) MS (+ES) *m*/*z:* 648 (M+2H)²⁺, cyclo[glycyl-β-methylphenylalanyl-O-[4-O-[2-O-(3-methylbutanoyl)hexopyranosyl]hexopyranosyl] tyrosyl-3-(2-iminoimidazolidin-4-yl)seryl-3-(3-hexopyranosyl-2-iminoimidazolidin-4-yl)serylseryl]_(Example 281b) MS (+ES) *m*/*z:* 690 (M+2H)²⁺ and cyclo[glycyl-β-methylphenylalanyl-O-[4-O-[3-O-(3-methylbutanoyl)hexopyranosyl]hexopyranosyl] tyrosyl-3-(2-iminoimidazolidin-4-yl)seryl-3-(3-hexopyranosyl-2-iminoimidazolidin-4-yl)serylseryl] (Example 281c) MS (+ES) *m*/*z:* 690 (M+2H)²⁺.

To afford isolation of cyclo[glycyl-β-mcthylphenylalanyl-O-(4-O-hexopyranosyl hexopyranosyl)tyrosyl-3-(2-iminoimidazolidin-4-yl)seryl-3-(3-hexopyranosyl-2-iminoimidazolidin-4-yl)serylseryl] (Example 281 a), strain LL4773 is fermented at the 300L scale in medium BPM17statgal for 5 days. Diatomaceous earth (4 kg) is added and the broth is filtered. The filtrate is then loaded onto a 60 L column of XAD7 resin. The column is then washed with one bed volume of water and eluted with 6 bed volumes of 50/50 methanol/ water with 0.1 % TFA. The eluate is then concentrated to about 4L by evaporation under vacuum. The resulting crude material is mixed with 4L methanol and the pH is adjusted to 12.8 with sodium hydroxide. After one hour, the pH is adjusted to 1.8 with HCl, 500 g of Diatomaceous earth is added and the mixture is filtered. Acetone (12 L) is then added to the filtrate, the mixture is stirred briefly then allowed to settle overnight to form a precipitate, which is then filtered, washed with methanol (2 L) and dried in a vacuum oven at 30°C to afford cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (Example 281a) MS (+ES) *m*/*z:* 648 (M+2H)²⁺.

### Example 289

### Jack bean α-mannosidase catalyzed conversion of Cyclo[glycyl-β-methylphenylalanyl-O-(4-O-hexopyranosylhexopyranosyl)tyrosyl-3-2-iminoimidazolidin-4-yl)seryl-3-(3-hexopyranosyl-2-iminoimidazolidin-4-yl)serylseryl] (Example 281a), To Cyclorglycyl-β-methylphenylalanyltyrosyl-3-(2-iminoimidazolidin-4-yl)seryl-3-(3-hexopyranosyl-2-iminoimidazolidin-4-yl)serylseryl] (Example 279b)

Jack bean α-mannosidase is added to a solution of Example (281a) Cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]. (0.60 g) in 0.1M pH 5 sodium acetate buffer (200 mL, 0.02M in ZnCl₂) and the solution stirred at room temperature for 18 h. The mixture is adjusted to pH 7 then centrifuged. The supernatant is passed through an XAD-7 column (eluting with a solvent system of 1:1:0.001 water:acetonitrile: trifluoroacetic acid) and the fractions collected. The solid is dissolved in 5% aq. acetic acid solution and the pH adjusted to 7. This solution is filtered and the filtrate passed through an XAD-7 column (eluting with a solvent system of 1:1:0.001 water:acetonitrile: trifluoroacetic acid). The product fractions are combined with those above and further purified by elution through a C18 reverse phase preparative column (eluting with a solvent gradient of 6:1:0.0014 to 1:1:0.0014 water: acetonitrile: trifluoroacetic acid) to give the bis(trifluoroacetate) (316 mg) product of the Example as an off-white solid.

### Example 290

### Jack bean α-mannosidase catalyzed conversion of Cyclo[glycyl-β-methylphenylalanyl-O-(4-O-hexopyranosylhexouyranosyl)trosyl-3-(2-iminoimidazolidin-4-yl)seryl-3-(3-hexopyranosyl-2-iminoimidazolidin-4-yl)serylseryl] (Example 281a) To Cycloralycyl-β-methylphenylalanyltyrosyl-3-(2-iminoimidazolidin-4-yl)seryl-3-(3-hexopyranosyl-2-iminoimidazolidin-4-yl)serylseryl]

### (Example 279b)

Jack bean meal (0.60 g)) is added to a solution of Cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]bis(trifluoroacetate) (0.60 g) in 0.1M pH 5 sodium acetate buffer (200 mL, 0.02M in ZnCl₂) and the solution stirred at room temperature for 18 h. The mixture is filtered through diametaceous earth then purified by chromatography through XAD-7 (eluting with a solvent system of 1:1:0.001 water:acetonitrile: trifluoroacetic acid) then C18 reverse phase (eluting with a solvent gradient of 6:1:0.0014 to 1:1:0.0014 water: acetonitrile: trifluoroacetic acid) columns to give the product of the Example (312 mg) as an off-white solid [MS (+ES), *m*/*z* 486 (M)²⁺].

### Example 291

### Almond Meal Fraction Mediated Preparation of Cyclo[glycyl-β-methylphenylalanyl-O-hexopyranosyltyrosyl-3-(2-iminoimidazolidin-4-yl)-seryl-3-(3-hexopyranosyl-2-iminoimidazolidin-4-yl)serylseryl]

Almond meal (2.5 g) is suspended in 0.1M pH 5 sodium acetate buffer (200 mL) and the suspension stirred for ca. 1 h and centrifuged. The supernatant is adjusted to pH 5 by the addition of acetic acid then recentrifuged. Cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl]bis(trifluoroacetate)bis(trifluoroacetate) (2 g) is added to 133 mL of the supernatant and the resultant solution stirred at room temperature for 18h. The mixture is evaporated to dryness, resuspended in N,N-dimethylformamide, filtered and the filtrate purified by chromatography on a C18 reverse phase preparative column (eluting with a solvent gradient of 6:1:0.0014 to 7:3:0.002 water: acetonitrile: trifluoroacetic acid) to give the product of the Example as the bis(trifluoroacetate)salt (48 mg) as an off-white solid [MS (+ES), *m*/*z* 567 (M)²

The following compounds are hydrolyzed using the procedure of Example 289 with jack bean α-mannosidase and the substrate as listed:

### Example 292

### Cyclo[3-cyclohexylalanyltyrosyl-3-(2-iminoimidazolidin-4-yl)seryl-3-(3-hexopyranosyl-2-iminoimidazolidin-4-yl)serylserylglycyl]

Substrate (Example 50): Cyclo[3-cyclohexylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylserylglycyl]

### Example 292

| Molecular Formula | C₄₁H₆₂N₁₂O₁₅ |
|---|---|
| MS(ESI) [M+2H]²⁺ | 482.2 |
| MW | 962 |

### Example 293

### Cyclo[3-(2-iminoimidazolidin-4-yl)alanyl-3-(3-hexopyranosyl-2-iminoimidazolidin-4-yl)-alanylserylglycyl-.β.-methylphenylalanyltyrosyl]

Substrate (Example 277a): Cyclo[3-(2-iminoimidazolidin-4-yl)alanyl-3-(3-hexopyranosyl-2-iminoimidazolidin-4-yl)-alanylserylglycyl-β-methylphenylalanyl-O-(4-O-hexopyranosylhexopyranosyl)tyrosyl]

### Example 293

| Molecular Formula | C₄₂H₅₈N₁₂O₁₃ |
|---|---|
| MS(ESI) [M+2H]⁺ | 470.4 |
| MW | 938 |

### Example 294

### Cyclo[3-cyclohexyl-2-aminobutanoyl-tyrosyl-3-(2-iminoimidazolidin-4-yl)seryl-3-(3-hexopyranosyl-2-iminoimidazolidin-4-yl)-serylserylglycyl]

Substrate (Example 51): Cyclo[3-cyclohexyl-2-aminobutanoyl-O-(4-O-hexopyranosylhexopyranosyl)tyrosyl-3-(2-iminoimidazolidin-4-yl)seryl-3-(3-hexopyranosyl-2-iminoimidazolidin-4-yl)-serylserylglycyl]

### Example 294

| Molecular Formula | C₄₂H₆₄N₁₂O₁₅ |
|---|---|
| MS(ESI) [M+2H]²⁺ | 489.3 |
| MW | 976 |

### Example 295

### Cyclo[glycyl-β-methylphenylalanyl-O-(2,3-O-isopropylidene-hexopyranosyl)tyrosyl-3-(2-iminoimidazolidin-4-yl)seryl-3-[2-imino-(2,3-O-isopropylidene-hexopyranosyl)imidazolidin-4-yl]serylseryl]

Substrate (Example 229):Cyclo[glycyl-β-methylphenylalanyl-O-(2,3-O-isopropylidene-4-O-hexopyranosylhexopyranosyl)tyrosyl-3-(2-iminoimidazolidin-4-yl)seryl-3-[2-imino-(2,3-0-isopropylidene-hexopyranosyl)imidazolidin-4-yl]serylseryl]

### Example 295

| Molecular Formula | C₅₄H₇₆N₁₂O₂₀ |
|---|---|
| MS(ESI) [M+2H]²⁺ | 607.5 |
| MW | 1212 |

### Example 296

### Cyclo[glycylphenylalanyl-O-(4-O-hexopyranosylhexopyranosyl)tyrosyl-3-(2-iminoimidazolidin-4-yl)seryl-3-(2-iminoimidazolidin-4-yl)serylseryl]

Strain BD20 is fermented at the 300 L scale for 5 days in medium BPM27. A clarified broth from the fermentation is then applied to a XAD7 resin column, eluted and fractions collected. The active fractions are pooled and concentrated by evaporation and then saponified, precipitated and dried as in Example 282. Analysis by HPLC and LC/MS, as described in Example 284, shows the expected presence of a peak at RRT 0.62 with an associated molecular weight of 1280 which is cyclo[glycylphenylalanyl-O-(4-O-hexopyranosylhexopyranosyl)tyrosyl-3-(2-iminomidazolidin-4-yl)seryl-3-(3-hexopyranosyl-2-iminoimidazolidin-4-yl)serylseryl] (Example 282) MS (+ES) *m*/*z*: 641 (M+2H)²⁺.

Analysis also reveals the presence of a related component at RRT 0.59, with a molecular mass of 1118.5. To afford isolation of the RRT 0.59 compound, 2 grams of dried precipitate is dissolved in 50 mL of water and applied to a pre-treated Sephadex LH-20 column. The column is eluted with water and 50 mL fractions are collected. Fractions 6 and 7 are combined and lyophilized to produce a crude mixture which is further fractionated by preparative HPLC on a YMC ODS-A column, employing a shallow gradient of acetonitrile/water (98% water/2% acetonitrile/.01% TFA to 92% water /8% acetonitrile/.01 % TFA in 60 min) to yield (Example 296) MS (+ES) *m*/*z*: 560.5 (M+2H)²⁺.

### Example 297

### Cyclo[glycyl-β-methylphenylalanyl-O-(4-O-hexopyranosylhexopyranosyl)tyrosyl-3-(2-iminoimidazolidin-4-yl)seryl-3-(2-iminoimidazolidin-4-yl)serylseryl]

Strain LL4773 is fermented in medium BPM27 for 2 days. A supernatant from the resulting fermentation broth is saponified, neutralized and analyzed by HPLC and LC/MS as in example 284. The analysis indicates a major component displaying a RRT of 1.0 and an associated molecular weight of 1294 which represents the expected production of cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] (Example 281a) MS (+ES) *m*/*z:* 648 (M+2H)²⁺.

Additionally, a component is noted at RRT 0.97 with an associated molecular weight of 1132. The observed chromatographic and spectral properties, and molecular weight for the RRT 0.97 compound, indicate it to be (Example 297) MS (+ES) *m*/*z*: 567 (M+2H)²⁺.

As described in Table 7 for Examples of the Invention, Analytical HPLC is performed over a 5 um, 120A, 4.6 x 150 mm YMC ODS-A column, with UV detection (215 and 254 nm) employing gradient elution of increasing concentrations of water in acetonitrile, each containing 0.02% trifluoroacetic acid, at a flow rate of 1 mL/min. Retention times are reported relative to the retention time for cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl](Example 281a) and retention times for this standard are shown in parentheses after each of the elution methods shown below:

**Table 7**

| Analytical HPLC retention times reported relative to the retention time for cyclo[glycyl-β-methylphenylalanyl-O-(4-O-hexopyranosylhexopyranosyl)tyrosyl-3-(2-iminoimidazolidin-4-yl)seryl-3-(3-hexopyranosyl-2-iminoimidazolidin-4-yl)serylseryl] for representative Examples of the Invention | | | |
|---|---|---|---|
| **EXAMPLE** | **MOLECULAR FORMULA** | **RELATIVE RETENTION TIME** | **ELUTION METHOD** |
| Example 1 | C₅₄H₇₇BrN₁₂O₂₅ | 1.16 | e |
| Example 2 | C₄₂H₅₇BrN₁₂O₁₅ | | |
| Example 3 | C₄₂H₅₆Br₂N₁₂O₁₅ | 1.57 | e |
| Example 4 | C₃₆H₄₆Br₂N₁₂O₁₀ | | |
| Example 5 | C₄₂H₅₇IN₁₂O₁₅ | 1.49 | e |
| Example 6 | C₅₄H₇₇IN₁₂O₂₅ | 1.18 | e |
| Example 7 | C₅₄H₇₆I₂N₁₂O₂₅ | | |
| Example 8 | C₄₂H₂₇N₁₃O₁₇ | 1.47 | e |
| Example 9 | C₃₆H₄₇N₁₃O₁₂ | | |
| Example 10 | C₄₂H₅₇N₁₃O₁₅ | | |
| Example 11 | C₄₂H₅₉N₁₃O₁₅ | | |
| Example 12 | C₄₄H₆₃N₁₃O₁₅ | 0.88 | e |
| Example 13 | C₄₄H₆₁N₁₃O₁₆ | 1.49 | e . |
| Example 14 | C₄₅H₆₃N₁₃O₁₆ | 1.23 | e |
| Example 15 | C₄₆H₆₅N₁₃O₁₆ | 1.38 | e |
| Example 16 | C₄₉H₇₁N₁₃O₁₆ | 1.78 | e |
| Example 17 | C₄₉H₆₃N₁₃O₁₆ | 1.64 | e |
| Example 18 | C₄₃H₅₉N₁₃O₁₆ | 1.24 | g |
| Example 19 | C₅₀H₆₅N₁₃O₁₇ | 1.71 | e |
| Example 20 | C₄₄H₆₁N₁₃O₁₇ | 1.36 | e |
| Example 21 | C₅₀H₆₅N₁₃O₁₇ | 1.33 | e |
| Example 22 | C₄₃H₅₇N₁₃O₁₆ | 1.26 | e |
| Example 23 | C₄₂H₅₇N₁₃O₁₅S | | |
| Example 24 | C₅₁H₆₂F₆N₁₄O₁₅S | 2.02 | e |
| Example 25 | C₅₀H₆₁N₁₃O₁₇ | 2.09 | e |
| Example 26 | C₄₉H₆₀N₁₄O₁₇ | | |
| Example 27 | C₄₉H₆₀BrN₁₃O₁₅ | 2.00 | e |
| Example 28 | C₅₆H₆₇N₁₃O₁₆ | 2.01 | e |
| Example 29 | C₅₁H₆₆N₁₄O₁₅ | 2.03 | e |
| Example 30 | C₄₉H₆₀FN₁₃O₁₅ | 1.63 | e |
| Example 31 | C₅₆H₆₇N₁₃O₁₆ | 2.57 | e |
| Example 32 | C₅₃H₆₉N₁₃O₁₅ | 2.76 | e |
| Example 33 | C₅₅H₆₅N₁₃O₁₅ | 1.63 | e |
| Example 34 | C₅₂H₆₇N₁₃O₁₈ | 1.76 | e |
| Example 35 | C₅₆H₆₇N₁₃O₁₇ | 1.85 | g |
| Example 36 | C₅₅H₇₁N₁₃O₂₁ | 2.08 | e |
| Example 37 | C₅₆H₆₅N₁₃O₁₅ | 1.24 | g |
| Example 38 | C₄₇H₅₉N₁₃O₁₆ | 1.63 | g |
| Example 39 | C₅₇H₆₇N₁₃O₁₅ | 1.83 | e |
| Example 40 | C₄₇H₆₃N₁₃O₁₅ | 2.49 | e |
| Example 41 | C₅₀H₅₇Ni₃O₁₁ | 2.62 | e |
| Example 42 | C₅₆H₆₅N₁₃O₁₃ | 1.46 | e |
| Example 43 | C₅₄H₆₅N₁₃O₁₆ | 1.51 | e |
| Example 44 | C₇₇H₈₄N₁₄O₂₄ | 2.41 | e |
| Example 45 | C₅₀H₆₃N₁₃O₁₅S | 1.83 | e |
| Example 46 | C₅₄H₆₅N₁₃O₁₅S | 1.68 | h |
| Example 47 | C₅₆H₆₇N₁₃O₁₅S | 4.64 | a |
| Example 48 | C₅₁H₆₃N₁₃O₁₆S | 2.03 | d |
| Example 49 | C₅₁H₆₂CIN₁₃O₁₆S | 2.10 | e |
| Example 50 | C₅₃H₈₂N₁₂O₂₅ | 1.05 | f |
| Example 51 | C₅₄H₈₄N₁₂O₂₅ | 1.16 | f |
| Example 52 | C₄₁H₆₈N₁₂O₁₄ | 1.97 | f |
| Example 52a | C₅₃H₈₈N₁₂O₂₅ | 1.04 | f |
| Example 52b | C₅₃H₅₈N₁₂O₂₅ | 0.83 | f |
| Example 53 | C₄₂H₇₀N₁₂O₁₄ | 1.97 | e |
| Example 53a | C₅₄H₉₀N₁₂O₂₅ | 1.29 | e |
| Example 54 | C₄₁H₆₈N₁₂O₁₄ | 1.46 | e |
| Example 54a | C₄₁H₆₈N₁₂O₁₄ | 1.59 | e |
| Example 55 | C₄₂H₅₈N₁₂O₁₅ | 1.15 | e |
| Example 56 | C₄₁H₅₆N₁₂O₁₅ | | |
| Example 57 | C₃₆H₄₈N₁₂O₁₀ | | |
| Example 58 | C₃₅H₅₈N₁₂O₉ | 1.68 | e |
| Example 59 | C₆₁H₈₄N₁₂O₂₅ | 1.05 | e |
| Example 60 | C₆₅H₉₂N₁₂O₂₅ | 1.37 | e |
| Example 60a | C₇₆H₁₀₆N₁₂O₂₅ | 1.74 | e |
| Example 61 | C₆₆H₁₀₂N₁₂O₂₆ | 1.46 | e |
| Example 62 | C₈₉H₁₀₈N₁₂O₂₅ | 1.82 | e |
| Example 63 | C₅₈H₈₆N₁₂O₂₅ | 0.89 | e |
| Example 64 | C₅₉H₈₈N₁₂O₂₅-₂CH₃I | 0.99 | f |
| Example 65 | C₆₇H₈₈N₁₂O₂₅ | 1.05 | e |
| Example 66 | C₉₃H₁₀₈N₁₂O₂₅ | 1.88 | e |
| Example 66a | C₆₇H₈₈N₁₂O₂₅ | 1.18 | e |
| Example 67 | C₆₅H₈₆N₁₂O₂₅ | 1.25 | e |
| Example 68 | C₆₂H₈₃F₃N₁₂O₂₅ | 1.00 | e |
| Example 69 | C₆₂H₈₄N₁₂O₂₇ | 0.85 | e |
| Example 69a | C₇₂H₉₀N₁₂O₂₉ | 1.17 | e |
| Example 69b | C₇₈H₉₆N₁₂O₃₁ | 1.56 | e |
| Example 70 | C₅₉H₈₆N₁₂O₂₅ | 0.92 | f |
| Example 71 | C₆₁H₉₂N₁₂O₂₅ | 1.40 | e |
| Example 72 | C₆₅H₉₈N₁₂O₂₇ | 1.34 | e |
| Example 72a | C₇₆H₁₁₈N₁₂O₂₉ | 2.31 | e |
| Example 73 | C₈₉H₁₀₈N₁₂O₂₃ | 1.83 | e |
| Example 74 | C₅₉H₈₈N₁₂O₂₃-₂CH₃I | 1.02 | f |
| Example 75 | C₄₈H₇₀N₁₂O₁₅-₂CH₃I | 1.43 | e |
| Example 76 | C₇₁H₉₆N₁₂O₂₅ | 1.82 | e |
| Example 77 | C₇₅H₁₀₄N₁₂O₂₅ | 1.71 | e |
| Example 78 | C₇₅H₉₈N₁₂O₂₅ | 1.81 | e |
| Example 79 | C₉₉H₁₂₀N₁₂O₂₅ | 2.35 | e |
| Example 80 | C₇₁H₉₆N₁₂O₂₃ | 1.56 | e |
| Example 81 | C₆₉H₁₀₀N₁₂O₂₅-₂CH₃I | 1.70 | e |
| Example 82 | C₇₄H₁₀₆N₁₂O₂₅ | 1.86 | e |
| Example 83 | C₇₁H₁₀₄N₁₂O₂₅ | 1.86 | e |
| Example 84 | C₇₀H₁₀₂N₁₂O₂₅ | 1.90 | e |
| Example 85 | C₆₂H₇₇N₁₃O₂₁ | 1.54 | e |
| Example 86 | C₆₄H₉₆N₁₂O₂₆ | 2.22 | e |
| Example 87 | C₅₉H₈₆N₁₂O₂₆ | 1.30 | e |
| Example 88 | C₆₀H₈₈N₁₂O₂₆ | 2.08 | e |
| Example 89 | C₆₆H₇₆N₁₂O₂₁ | 2.28 | e |
| Example 90 | C₆₂H₈₄N₁₂O₂₇ | 1.14 | e |
| Example 91 | C₇₈H₉₆N₁₂O₃₁ | 1.83 | e |
| Example 92 | C₆₂H₈₂N₁₆O₂₅ | 1.09 | i |
| Example 93 | C₆₈H₈₄N₁₄O₂₇ | 1.45 | f |
| Example 94 | C₆₈H₈₄N₁₄O₂₅S₂ | 1.40 | i |
| Example 95 | C₆₀H₈₈N₁₂O₂₆ | | |
| Example 95a | C₆₀H₈₈N₁₂O₂₆ | | |
| Example 95b | C₆₀H₈₈N₁₂O₂₆ | | |
| Example 96c | C₆₈H₈₈N₁₂O₂₆ | | |
| Example 96 | C₆₈H₈₈N₁₂O₂₆ | | |
| Example 96a | C₆₈H₈₈N₁₂O₂₆ | | |
| Example 96b | C₆₈H₈₈N₁₂O₂₆ | | |
| Example 97 | C₆₁H₉₀N₁₂O₂₆ | | |
| Example 97a | C₆₁H₉₀N₁₂O₂₆ | | |
| Example 97b | C₆₁H₉₀N₁₂O₂₆ | | |
| Example 98 | C₆₂H₈₄N₁₂O₂₆ | | |
| Example 98a | C₆₂H₈₄N₁₂O₂₆ | | |
| Example 98b | C₆₂H₈₄N₁₂O₂₆ | | |
| Example 98c | C₆₂H₈₄N₁₂O₂₆ | | |
| Example 99 | C₆₂H₉₂N₁₂O₂₆ | | |
| Example 99a | C₆₂H₉₂N₁₂O₂₆ | | |
| Example 99b | C₆₂H₉₂N₁₂O₂₆ | | |
| Example 99c | C₆₂H₉₂N₁₂O₂₆ | | |
| Example 100 | C₆₂H₉₀N₁₂O₂₆ | | |
| Example 100a | C₆₂H₉₀N₁₂O₂₆ | | |
| Example 100b | C₆₂H₉₀N₁₂O₂₆ | | |
| Example 100c | C₆₂H₉₀N₁₂O₂₆ | | |
| Example 101 | C₆₂H₈₄N₁₂O₂₇ | 1.40 | e |
| Example 101a | C₆₂H₈₄N₁₂O₂₇ | 1.44 | e |
| Example 102 | C₆₂H₈₄N₁₂O₂₅ | 1.92 | g |
| Example 103 | C₆₃H₈₆N₁₂O₂₆ | 1.94 | g |
| Example 104 | C₆₁H₈₂N₁₂O₂₅ | 1.55 | e |
| Example 104a | C₆₁H₈₂N₁₂O₂₅ | 1.50 | e |
| Example 104b | C₆₁H₈₂N₁₂O₂₅ | 1.53 | e |
| Example 105 | C₆₄H₉₀N₁₂O₂₅ | 1.77 | e |
| Example 106 | C₆₃H₉₄N₁₂O₂₅ | 1.78 | e |
| Example 106a | C₆₃H₉₄N₁₂O₂₅ | | |
| Example 106b | C₇₃H₁₀₆N₁₂O₂₅ | 1.95 | e |
| Example 107 | C₅₉H₈₆N₁₂O₂₅ | 1.81 | g |
| Example 108 | C₆₀H₈₈N₁₂O₂₅ | 2.05 | g |
| Example 109 | C₆₂H₉₂N₁₂O₂₅ | 2.43 | g |
| Example 110 | C₆₀H₈₈N₁₂O₂₅ | 1.97 | g |
| Example 111 | C₆₀H₈₈N₁₂O₂₅ | 2.05 | g |
| Example 112 | C₆₁H₈₈N₁₂O₂₅ | 2.05 | g |
| Example 113 | C₆₉H₉₅N₁₃O₂₇ | 2.25 | g |
| Example 114 | C₆₂H₉₀N₁₂O₂₇ | 1.98 | G |
| Example 115 | C₆₁H₈₆N₁₂O₂₅ | 1.95 | G |
| Example 116 | C₆₀H₈₈N₁₂O₂₅ | 2.02 | G |
| Example 117 | C₆₀H₈₆N₁₂O₂₅ | 1.86 | G |
| Example 118 | C₆₂H₈₃BrN₁₂O₂₅ | 2.16 | ' G |
| Example 119 | C₆₃H₈₆N₁₂O₂₅ | 2.11 | G |
| Example 120 | C₆₂H₈₃CIN₁₂O₂₅ | 2.16 | G |
| Example 121 | C₆₂H₈₃FN₁₂O₂₅ | 2.00 | G |
| Example 122 | C₆₂H₉₀N₁₂O₂₅ | 1.93 | E |
| Example 123 | C₆₁H₈₈N₁₂O₂₅ | 1.53 | E |
| Example 124 | C₆₃H₈₆N₁₂O₂₅ | 1.53 | E |
| Example 125 | C₆₃H₉₂N₁₂O₂₅ | 1.80 | E |
| Example 126 | C₆₄H₉₄N₁₂O₂₅ | 1.88 | e |
| Example 127 | C₆₅H₉₂N₁₂O₂₅ | 1.98 | e |
| Example 128 | C₆₆H₉₄N₁₂O₂₅ | 2.05 | e |
| Example 129 | C₆₆H₉₄N₁₂O₂₅ | 2.09 | e |
| Example 130 | C₇₉H₁₁₈N₁₂O₂₆ | 2.93 | e |
| Example 131 | C₅₉H₈₆N₁₂O₂₆ | 1.68 | e |
| Example 132 | C₅₉H₈₀N₁₂O₂₅S | 1.78 | e |
| Example 133 | C₅₉H₈₀N₁₂O₂₅S | 1.41 | e |
| Example 134 | C₆₂H₈₄N₁₂O₂₅ | | |
| Example 135 | C₆₂H₈₄N₁₂O₂₅ | 1.51 | e |
| Example 136 | C₆₁H₈₂N₁₂O₂₆ | 1.26 | e |
| Example 137 | C₆₂H₈₄N₁₂O₂₆ | 1.45 | e |
| Example 137a | C₆₂H₈₄N₁₂O₂₆ | 1.42 | e |
| Example 137b | C₆₂H₈₄N₁₂O₂₆ | 1.43 | e |
| Example 138 | C₆₁H₈₁ClN₁₂O₂₅ | 1.55 | e |
| Example 139 | C₆₄H₈₈N₁₂O₂₅ | | |
| Example 140 | C₆₄H₈₈N₁₂O₂₅ | 1.96 | e |
| Example 141 | C₆₂H₈₂N₁₂O₂₇ | 1.14 | e |
| Example 142 | C₆₃H₈₆N₁₂O₂₆ | | |
| Example 143 | C₆₂H₈₂N₁₂O₂₇ | 1.43 | e |
| Example 144 | C₆₂H₈₄N₁₂O₂₅S | 1.67 | e |
| Example 145 | C₆₅H₈₄N₁₂O₂₅ | 1.81 | e |
| Example 146 | C₆₅H₈₈N₁₂O₂₅ | 2.78 | e |
| Example 147 | C₆₅H₉₀N₁₂O₂₅ | 1.99 | e |
| Example 148 | C₆₄H₈₈N₁₂O₂₆ | | |
| Example 149 | C₆₃H₈₄N₁₂O₂₇ | 1.41 | e |
| Example 150 | C₆₃H₈₆N₁₂O₂₇ | 1.49 | e |
| Example 150a | C₆₃H₈₆N₁₂O₂₇ | 1.48 | e |
| Example 151 | C₆₁H₈₁N₁₃O₂₈ | 1.40 | e |
| Example 152 | C₆₄H₈₈N₁₂O₂₇ | 1.56 | e |
| Example 153 | C₆₂H₈₃N₁₃O₂₈ | 1.59 | e |
| Example 154 | C₆₇H₈₆N₁₂O₂₅ | 1.95 | e |
| Example 155 | C₆₇H₈₆N₁₂O₂₅ | 1.95 | e |
| Example 156 | C₆₆H₈₅N₁₃O₂₅ | 1.14 | e |
| Example 157 | C₆₂H₈₄N₁₂O₂₇S | 1.21 | e |
| Example 158 | C₆₂H₈₁ClN₁₂O₂₇ | 1.50 | e |
| Example 159 | C₆₆H₈₆N₁₂O₂₆ | | |
| Example 160 | C₆₆H₈₆N₁₂O₂₆ | | |
| Example 161 | C₆₅H₈₅N₁₃O₂₆ | 1.55 | e |
| Example 162 | C₆₅H₈₄N₁₂O₂₅S | 1.71 | e |
| Example 163 | C₆₅H₈₄N₁₂O₂₅S | 1.93 | e |
| Example 164 | C₆₅H₈₄N₁₂O₂₅S | 1.76 | e |
| Example 164a | C₆₅H₈₄N₁₂O₂₅S | 1.66 | e |
| Example 165 | C₆₆H₉₀N₁₂O₂₆ | 1.77 | e |
| Example 166 | C₆₄H₈₆N₁₂O₂₈ | 1.57 | e |
| Example 167 | C₆₃H₈₅N₁₃O₂₈ | 1.64 | e |
| Example 168 | C₆₂H₈₃N₁₃O₂₇S | 1.73 | e |
| Example 169 | C₆₂H₈₃N₁₃O₂₉ | 1.44 | e |
| Example 170 | C₆₇H₈₆N₁₂O₂₆ | 1.78 | e |
| Example 171 | C₆₇H₈₆N₁₂O₂₆ | | |
| Example 172 | C₆₉H₈₈N₁₂O₂₅ | 2.11 | e |
| Example 173 | C₆₈H₈₆N₁₂O₂₆ | 1.83 | e |
| Example 174 | C₆₈H₈8N₁₂O₂₆ | 2.16 | e |
| Example 175 | C₆₈H₈₈N₁₂O₂₆ | 1.78 | e |
| Example 176 | C₆₈H₈₈N₁₂O₂₆ | | |
| Example 177 | C₆₇H₈₅ClN₁₂O₂₅ | 2.09 | e |
| Example 178 | C₆₇H₈₅ClN₁₂O₂₅ | 2.12 | e |
| Example 179 | C₆₃H₈₂F₄N₁₂O₂₆ | 1.66 | e |
| Example 180 | C₆₈H₈₈N₁₂O₂₇ | 1.87 | e |
| Example 181 | C₇₁H₉₄N₁₂O₂₆ | 2.11 | e |
| Example 182 | C₆₈H₈₇N₁₃O₂₈ | 1.91 | e |
| Example 183 | C₇₁H₉₈N₁₂O₂₆ | 2.12 | e |
| Example 184 | C₆₈H₈₅F₃N₁₂O₂₆ | 1.94 | e |
| Example 185 | C₇₆H₁₀₆N₁₂O₂₆ | 2.65 | e |
| Example 186 | C₆₈H₈₅IN₁₂O₂₆ | 2.05 | e |
| Example 187 | C₆₈H₈₃I₃N₁₂O₂₆ | 2.35 | e |
| Example 188 | C₆₉H₈₈N₁₂O₂₅ | | |
| Example 189 | C₅₇H₈₀N₁₂O₂₅ | 1.35 | e |
| Example 190 | C₆₆H₈₆N₁₂O₂₄ | | |
| Example 191 | C₆₈H₈₈N₁₂O₂₄ | | |
| Example 192 | C₆₈H₈₈N₁₂O₂₅ | 1.84 | e |
| Example 193 | C₆₁H₈₇ClN₁₂O₂₅ | 2.24 | g |
| Example 194 | C₆₀H₈₇ClN₁₂O₂₅ | 2.21 | g |
| Example 195 | C₆₁H₈₁ClN₁₂O₂₅ | 1.71 | e |
| Example 196 | C₆₁H₈₁IN₁₂O₂₅ | 1.59 | e |
| Example 197 | C₆₈H₈₇IN₁₂O₂₆ | 1.94 | e |
| Example 198 | C₆₂H₉₀N₁₂O₂₅ | 1.67 | e |
| Example 199 | C₆₀H₈₆N₁₂O₂₅ | 1.53 | e |
| Example 200 | C₆₁H₈₈N₁₂O₂₅ | | |
| Example 201 | C₆₁H₈₈N₁₂O₂₅ | 1.61 | e |
| Example 202 | C₅₉H₈₄N₁₂O₂₅ | 1.33 | e |
| Example 203 | C₆₀H₈₆N₁₂O₂₅ | 1.42 | e |
| Example 204 | C₆₁H₈₈N₁₂O₂₅ | 1.51 | e |
| Example 205 | C₆₂H₉₀N₁₂O₂₅ | 1.72 | e |
| Example 206 | C₆₂H₉₀N₁₂O₂₅ | 1.76 | e |
| Example 207 | C₆₄H₉₄N₁₂O₂₅ | 1.86 | e |
| Example 208 | C₆₄H₉₄N₁₂O₂₅ | 2.06 | e |
| Example 209 | C₆₆H₉₈N₁₂O₂₅ | 2.15 | e |
| Example 210 | C₆₇H₁₀₀N₁₂O₂₅ | 2.24 | e |
| Example 210a | C₆₇H₁₀₀N₁₂O**₂₅** | 2.20 | e |
| Example 211 | C₆₂H₈₈N₁₂O₂₅ | | |
| Example 212 | C₆₃H₉₀N₁₂O₂₅ | | |
| Example 213 | C₆₄H₉₀N₁₂O₂₅ | | |
| Example 214 | C₆₄H₉₂N₁₂O₂₅ | 1.85 | e |
| Example 215 | C₆₄H₉₂N₁₂O₂₅ | 1.85 | e |
| Example 216 | C₆₂H₈₃FN₁₂O₂₅ | 1.51 | e |
| Example 217 | C₅₉H₈₄N₁₂₀₂₅S | | |
| Example 218 | C₆₉H₉₈N₁₂O₂₆ | 2.35 | e |
| Example 219 | C₆₄H₉₀N₁₂O₂₃ | 1.74 | e |
| Example 220 | C₆₃H₈₈N₁₂O₂₅ | 1.79 | e |
| Example 221 | C₆₄H₉₆N₁₂O₂₅ | 1.84 | e |
| Example 222 | C₆₃H₁₀₀N₁₂O₂₅ | 1.70 | e |
| Example 223 | C₆₃H₁₀₀N₁₂O₂₅ | 1.53 | e |
| Example 224 | C₆₈H₉₈N₁₂O₂₅ | 1.69 | e |
| Example 225 | C₆₉H₉₈N₁₂O₂₅ | 1.68 | e |
| Example 226 | C₇₂H₉₅F₃N₁₂O₂₅ | 1.87 | e |
| Example 227 | C₇₈H₉₆N₁₄O₂₇ | 1.87 | e |
| Example 228 | C₇₂H₉₄N₁₆O₂₅ | 1.63 | e |
| Example 229 | C₆₀H₈₆N₁₂O₂₅ | 1.18 | e |
| Example 229a | C₆₃H₉₀N₁₂O₂₅ | 1.51 | e |
| Example 229b | C₆₆H₉₄N₁₂O₂₅ | 1.67 | e |
| Example 230 | C₆₆H₈₈N₁₂O₂₆ | 1.64 | e |
| Example 230a | C₆₆H₈₈N₁₂O₂₆ | 1.70 | e |
| Example 230b | C₆₆H₈₈N₁₂O₂₆ | 1.61 | e |
| Example 230c | C₆₆H₈₈N₁₂O₂₆ | 1.53 | e |
| Example 231 | C₆₈H₉₀N₁₂O₂₆ | 1.85 | e |
| Example 231a | C₆₈H₉₀N₁₂O₂₆ | 1.93 | e |
| Example 231b | C₆₈H₉₀N₁₂O₂₆ | 1.78 | e |
| Example 231c | C₆₈H₉₀N₁₂O₂₆ | 1.72 | e |
| Example 232 | C₆₂H₈₆N₁₂O₂₆ | 1.45 | e |
| Example 233 | C₆₃H₈₈N₁₂O₂₆ | | |
| Example 234 | C₆₂H₈₆N₁₂O₂₅ | 1.56 | e |
| Example 235 | C₆₄H₉₀N₁₂O₂₆ | 1.71 | e |
| Example 236 | C₆₇H₈₈N₁₂O₂₆ | 1.83 | e |
| Example 237 | C₆₂H₈₅FN₁₂O₂₆ | 1.47 | e |
| Example 238 | C₆₄H₉₀N₁₂O₂₇ | 1.55 | e |
| Example 239 | C₆₉H₉₂N₁₂O₂₇ | 1.71 | e |
| Example 239a | C₆₉H₉₂N₁₂O₂₇ | 1.77 | e |
| Example 240 | C₆₆H₈₈N₁₂O₂₆ | 1.77 | d |
| Example 240a | C₆₆H₈₈N₁₂O₂₆ | 2.26 | b |
| Example 240b | C₆₆H₈₈N₁₂O₂₆ | 1.95 | b |
| Example 241 | C₆₉H₉₀N₁₂O₂₅ | | |
| Example 241a | C₆₉H₉₀N₁₂O**₂₅** | | |
| Example 242 | C₆₃H₈₆N₁₂O₂₅ | 1.62 | e |
| Example 243 | C₆₅H₉₁N₁₃O₂₅ | 1.20 | e |
| Example 243a | C₆₅H₉₁N₁₃O₂₅ | 1.23 | e |
| Example 244 | C₅₉H₈₂N₁₂O₂₅S | 1.38 | e |
| Example 245 | C₆₆H₈₈N₁₂O₂₄ | 1.60 | e |
| Example 245a | C₆₆H₈₈N₁₂O₂₄ | 1.67 | e |
| Example 245b | C₆₆H₈₈N₁₂O₂₄ | 1.58 | e |
| Example 245c | C₆₆H₈₈N₁₂O₂₄ | 1.54 | e |
| Example 246 | C₆₈H₉₀N₁₂O₂₄ | 1.73 | e |
| Example 246a | C₆₈H₉₀N₁₂O₂₄ | 1.80 | e |
| Example 246b | C₆₈H₉₀N₁₂O₂₄ | 1.67 | e |
| Example 246c | C₆₈H₉₀N₁₂O₂₄ | 1.60 | e |
| Example 247 | C₄₈H₆₆N₁₂O₁₅ | | |
| Example 248 | C₅₄H₈₀N₁₂O₁₅Si | 1.84 | e |
| Example 249 | C₇₈H₉₈N₁₂O₂₁Si | | |
| Example 250 | C₇₄H₈₆N₁₂O₁₈Si | 2.62 | c |
| Example 251 | C₅₉H₈₇N₁₃O₂₆ | 1.35 | e |
| Example 251a | C₅₉H₈₇N₁₃O₂₆ | 1.43 | e |
| Example 251b | C₅₉H₈₇N₁₃O₂₆ | 1.49 | e |
| Example 252 | C₅₉H₈₇N₁₃O₂₆ | 1.27 | e |
| Example 252a | C₅₉H₈₇N₁₃O₂₆ | 1.39 | e |
| Example 253 | C₆₂H₉₃N₁₃O₂₆ | 1.60 | e |
| Example 253a | C₆₂H₉₃N₁₃O₂₆ | 1.63 | e |
| Example 253b | C₆₂H₉₃N₁₃O₂₆ | 1.68 | e |
| Example 254 | C₆₇H₁₀₃N₁₃O₂₆ | 2.26 | e |
| Example 254a | C₆₇H₁₀₃N₁₃O₂₆ | 2.32 | e |
| Example 254b | C₆₇H₁₀₃N₁₃O₂₆ | 2.51 | e |
| Example 255 | C₆₀H₈₉N₁₃O₂₆ | 1.60 | e |
| Example 255a | C₆₀H₈₉N₁₃O₂₆ | 1.63 | e |
| Example 256 | C₆₇H₁₀₃N₁₃O₂₆ | 1.26 | e |
| Example 256a | C₆₇H₁₀₃N₁₃O₂₆ | 1.39 | e |
| Example 257 | C₆₂H₈₅N₁₃O₂₆ | 1.42 | e |
| Example 257a | C₆₂H₈₅N₁₃O₂₆ | 1.45 | e |
| Example 257b | C₆₂H₈₅N₁₃O₂₆ | 1.49 | e |
| Example 261a | C₅₄H₇₇FN₁₂O₂₅ | 1.08 | k |
| Example 261b | C₅₉H₈₅FN₁₂O₂₆ | 1.51 | k |
| Example 261c | C₅₉H₈₅FN₁₂O_{**2**6} | 1.73 | k |
| Example 262a | C₅₄H₇₇CLN₁₂O₂₅ | 1.26 | k |
| Example 262b | C₅₉H₈₅CLN₁₂O₂₆ | 1.86 | k |
| Example 262c | C₅₉H₈₅ClN₁₂O₂₆ | 1.61 | k |
| Example 263 | C₅₃H₈₂N₁₂O₂₅ | 1.21 | k |
| Example 264a | C₅₉H₈₅FN₁₂O₂₆ | 1.57 | k |
| Example 264b | C₅₉H₈₅FN₁₂O₂₆ | 1.77 | k |
| Example 264c | C₅₄H₇₇FN₁₂O₂₅ | 1.11 | k |
| Example 265a | C₅₇H₈₄N₁₂O₂₆S | 1.37 | k |
| Example 265b | C₅₇H₈₄N₁₂O₂₆S | 1.60 | k |
| Example 265c | C₅₂H₇₆N₁₂O₂₅S | 0.91 | k |
| Example 266a | C₅₄H₇₇FN₁₂O₂₅ | 1.06 | k |
| Example 266b | C₅₉H₈₅FN₁₂O₂₆ | 1.56 | k |
| Example 266c | C₅₉H₈₅FN₁₂O₂₆ | 1.77 | k |
| Example 267a | C₅₄H₇₉N₁₃O₂₅ | 0.84 | k |
| Example 267b | C₅₉H₈₇N₁₃O₂₆ | 1.22 | k |
| Example 267c | C₅₉H₈₇N₁₃O₂₆ | 1.46 | k |
| Example 268 | C₅₇H₈₂N₁₂O₂₆ | 1.35 | k |
| Example 269a | C₅₄H₇₇ClN₁₂O₂₅ | 1.20 | k |
| Example 269b | C₅₉H₈₅ClN₁₂O₂₆ | 1.67 | k |
| Example 269c | C₅₉H₈₅ClN₁₂O₂₆ | 1.83 | k |
| Example 270a | C₆₀H₈₈N₁₂O₂₆ | 1.66 | k |
| Example 270a | C₆₂H₉₀N₁₂O₂₇ | 1.90 | k |
| Example 270a | C₆₀H₈₈N₁₂O₂₆ | 2.02 | k |
| Example 271a | C₆₀H₈₈N₁₂O₂₆ | >1.85 | g |
| Example 271b | C₆₂H₉₀N₁₂O₂₇ | 2.00 | k |
| Example 271c | C₆₀H₈₈N₁₂O₂₆ | 2.06 | k |
| Example 272a | C₅₈H₈₄N₁₂O₂₆ | 1.38 | k |
| Example 272b | C₅₈H₈₄N₁₂O₂₆ | 1.61 | k |
| Example 273a | C₆₀H₈₈N₁₂O₂₆ | 1.64 | k |
| Example 273b | C₆₀H₈₈N₁₂O₂₆ | 1.89 | k |
| Example 274a | C₆₁H₉₀N₁₂O₂₆ | 1.93 | k |
| Example 274b | C₆₁H₉₀N₁₂O₂₆ | 2.13 | k |
| Example 275 | C₆₁H₈₈N₁₂O₂₇ | 1.79 | g |
| Example 276a | C₅₆H₈₀N₁₂O₂₆ | 1.17 | g |
| Example 276b | C₆₁H₈₈N₁₂O₂₇ | 1.73 | g |
| Example 277a | C₅₄H₇₈N₁₂O₂₃. | 1.04 | g |
| Example 277b | C₅₉H₈₆N₁₂O₂₄ | 1.47 | g |
| Example 277c | C₅₉H₈₆N₁₂O₂₄ | 1.55 | g |
| Example 278a | C₄₂H₅₈N₁₂O₁₅ | 1.20 | g |
| Example 278b | C₅₉H₈₄N₁₂O₂₆ | >1.85 | g |
| Example 278c | C₅₉H₈₆N₁₂O₂₆ | >1.85 | g |
| Example 278d | C₆₀H₈₈N₁₂O₂₆ | >1.85 | g |
| Example 278e | C₅₉H₈₆N₁₂O₂₆ | 1.82 | g |
| Example 279a | C₄₁H₅₆N₁₂O₁₅ | 1.19 | g |
| Example 279b | C₄₂H₅₈N₁₂O₁₅ | 1.29 | g |
| Example 280a | C₃₅H₄₆N₁₂O₁₀ | 1.17 | g |
| Example 280b | C₃₆H₄₈N₁₂O₁₀ | 1.30 | g |
| Example 281a | C₅₄H₇₈N₁₂O₂₅ | 1.00 | g |
| Example 281b | C₅₉H₈₆N₁₂O₂₆ | 1.43 | g |
| Example 281c | C₅₉H₈₆N₁₂O₂₆ | 1.64 | g |
| Example 282 | C₅₃H₇₆N₁₂O₂₅ | 0.86 | g |
| Example 283a | C₅₇H₈₂N₁₂O₂₆ | 1.34 | g |
| Example 283b | C₅₇H₈₂N₁₂O₂₆ | 1.38 | g |
| Example 283c | C₅₈H₈₄N₁₂O₂₆ | 1.52 | g |
| Example 284a | C₅₃H₇₆N₁₂O₂₄ | 0.66 | j* |
| Example 284b | C₅₃H₇₆N₁₂O₂₄ | 0.71 | j* |
| Example 284c | C₅₃H₇₆N₁₂O₂₃ | 0.73 | j* |
| Example 285a | C₅₈H₈₄N₁₂O₂₆ | 1.33 | g |
| Example 285b | C₅₈H₈₄N₁₂O₂₆ | 1.55 | g |
| Example 286 | C₄₈H₆₇N₁₃O₂₂ | 1.17 | g |
| Example 287 | C₅₄H₇₉N₁₃O₂₅ | 0.94 | g |
| Example 296 | C₄₇H₆₆N₁₂O₂₀ | 0.59 | j* |
| Example 297 | C₄₈H₆₈N₁₂O₂₀ | 0.97 | j* |
| a) Gradient elution of 20-80% acetonitrile in water over 20 minutes (2.14 min) | | | |
| b) Gradient elution of 15-55% acetonitrile in water over 15 minutes (3.81 min) | | | |
| c) Gradient elution of 10-90% acetonitrile in water over 15 minutes (4.60 min) | | | |
| d) Gradient elution of 10-90% acetonitrile in water over 20 minutes (5.02 min) | | | |
| e) Gradient elution of 10-60% acetonitrile in water over 15 minutes (5.31 min) | | | |
| f) Gradient elution of 10-40% acetonitrile in water over 15 minutes (6.30 min) | | | |
| g) Gradient elution of 10-50% acetonitrile in water over 22 minutes (6.30 min) | | | |
| h) Gradient elution of 0-50% acetonitrile in water over 15 minutes (8.44 min) | | | |
| i) Gradient elution of 10-20% acetonitrile in water over 15 minutes (9.45 min) | | | |
| j) *Isocratic elution as described in example 284 | | | |

1. A process for producing a compound of the formula: 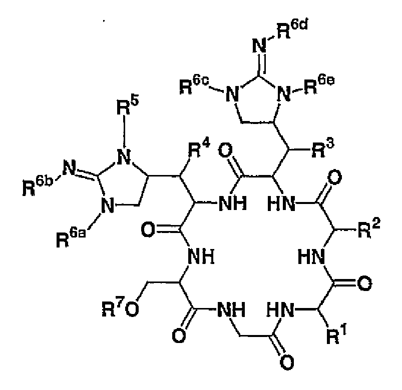 wherein:
   R¹ is selected from: 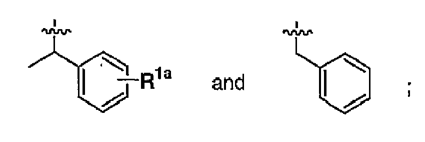
   R^{1a} is H;
   R² is a moiety 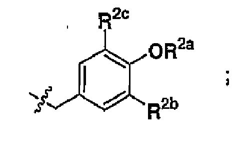
   R^{2a} is selected from
   H, 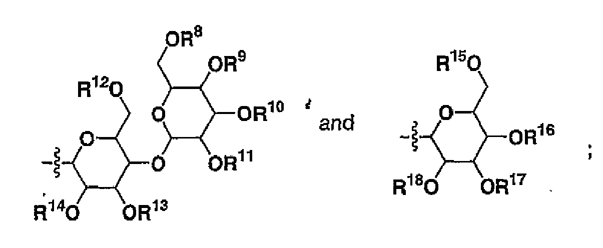
   R^{2b} is H;
   R^{2c} is H;
   R³ and R⁴ are independently H or OH;
   R⁵ is H or 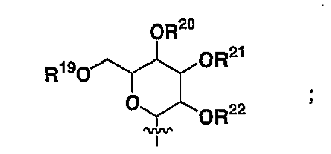
   R^{6a}, R^{6b}, R^{6c}, R^{6d}, or R^{6e} are H;
   R⁷,R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently
   H, or -C(O)-Y-Z;
   Y is a single bond;
   Z is straight or branched alkyl(C₁-C₂₀) or straight or branched alkenyl(C₂-C₂₀); which process comprises:
      cultivating a glycopeptide antibiotic producing strain of *Streptomyces hygroscopicus* selected from the group LL4600, LL4614, LL4666, LL4690, LL4728, LL4741, LL4742, LL4744, LL4773, LL4779, LL4783, LL4902, BD2, BD20 and BD70 or a mutant thereof under aerobic conditions, in a suitable culture medium until a recoverable amount of said antibiotic is formed in said medium and recovering said antibiotic therefrom.
2. A process according to 1 in which R⁷, R¹², R¹³, R¹⁴, R¹⁵, R¹⁶, R¹⁷, R¹⁸, R¹⁹, R²⁰, R²¹ and R²² are H; and
   Z is straight or branched alkyl(C₁-C₉) or straight or branched alkenyl(C₂-C₁₀);
3. A process according to 1 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₉) or straight or branched alkenyl(C₂-C₁₀); and R³ and R⁴ are H.
4. A process according to 1 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₂₀) or straight or branched alkenyl(C₂-C₂₀);
   and
   R³ and R⁴ are OH.
5. A process according to 4 in which the glycopeptide antibiotic produced has the structure selected from the group 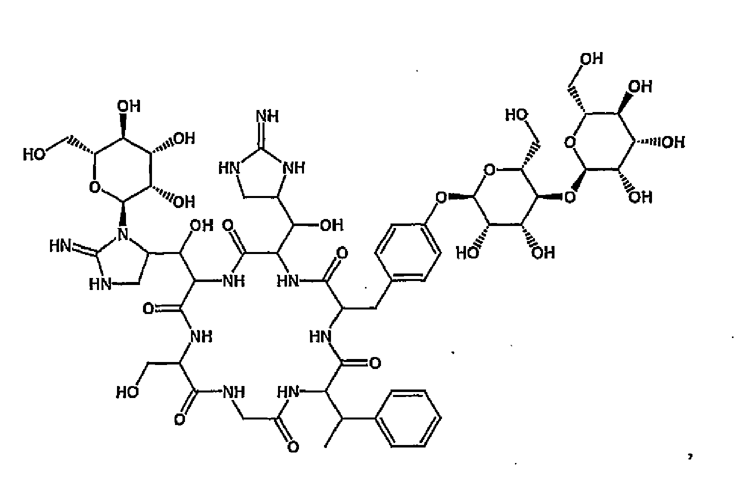 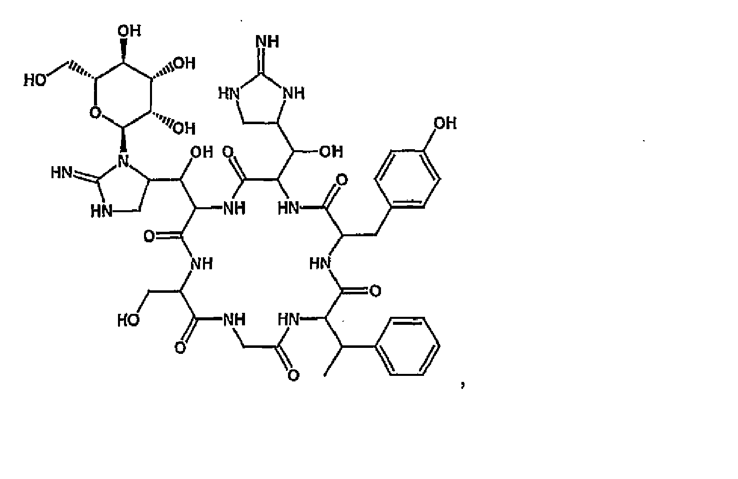 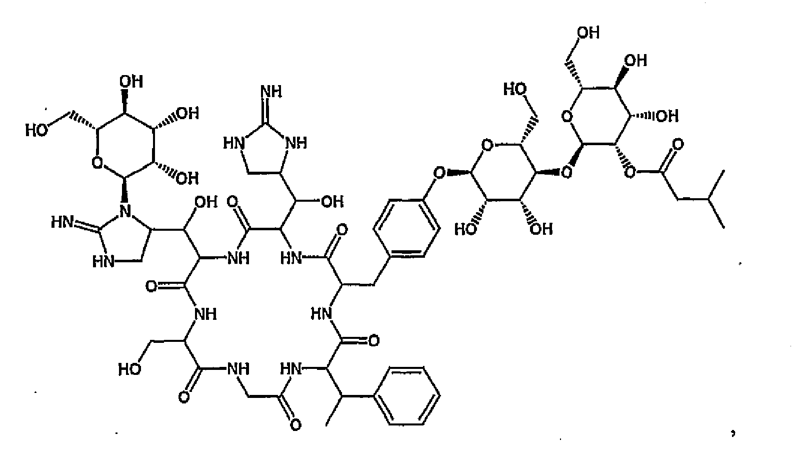 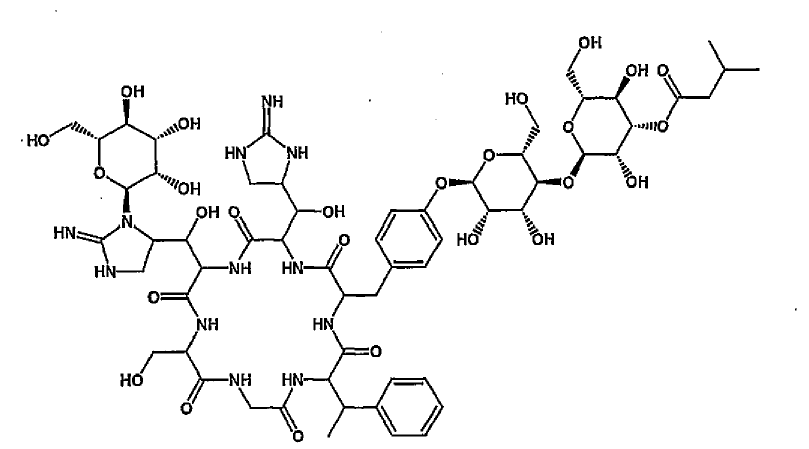 and 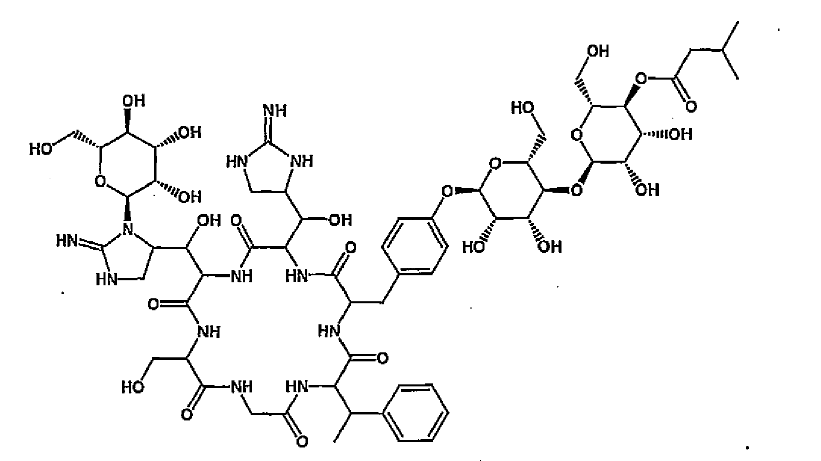
6. A process for producing a compound of the formula: 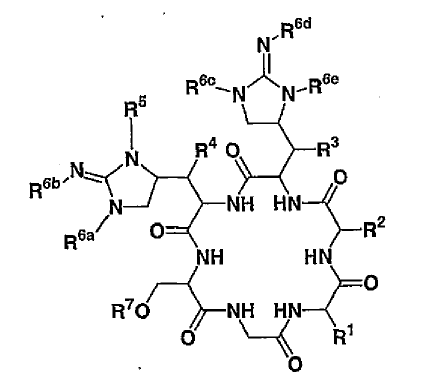 wherein:
   R¹ is selected from: 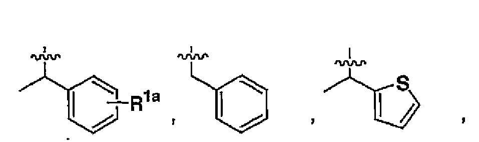 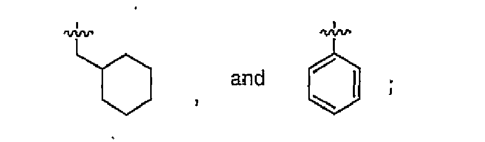
   R^{1a} is H or halogen;
   R² is a moiety 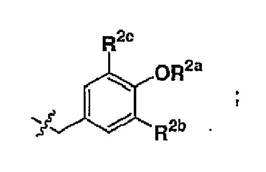
   R^{2a} is selected from
   H, 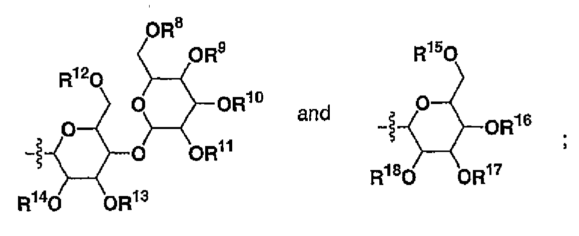
   R^{2b} is H;
   R^{2c} is H;
   R³ and R⁴ are independently H or OH;
   R⁵ is H or 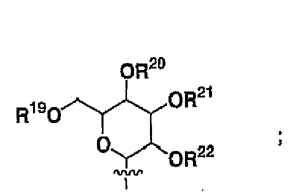
   R^{6a}, R^{6b}, R^{6c}, R^{6d}, or R^{6e} are H;
   R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently
   H, or -C(O)-Y-Z ;
   Y is a single bond;
   Z is straight or branched alkyl(C₁-C₂₀) or straight or branched alkenyl(C₂-C₂₀);
   which process comprises:
      cultivating a glycopeptide antibiotic producing strain of *Streptomyces hygroscopicus* selected from the group LL4600, LL4614, LL4666, LL4690, LL4728, LL4741, LL4742, LL4744, LL4773, LL4779, LL4783, LL4902, BD2, BD20 and BD70 or a mutant thereof under aerobic conditions, in a suitable culture medium in the presence of a substrate 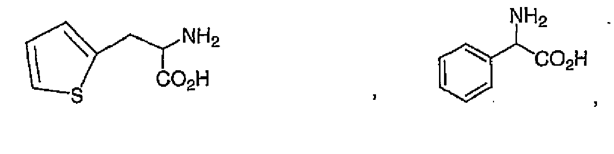 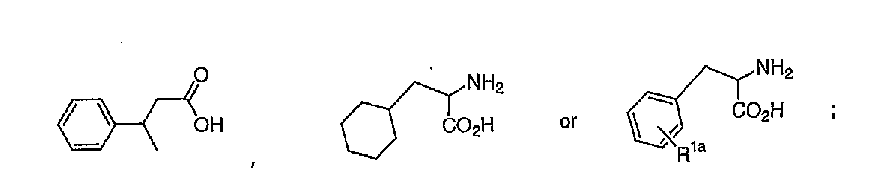 until a recoverable amount of said antibiotic is formed in said medium and recovering said antibiotic therefrom.
7. The process according to 6 wherein R¹ is 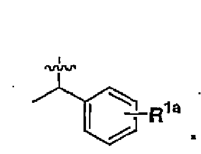
8. The process according to 6 wherein R¹ is 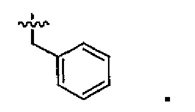
9. The process according to 6 wherein R¹ is 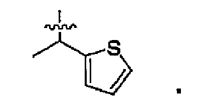
10. The process according to 6 wherein R¹ is 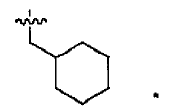
11. The process according to 6 wherein R¹ is 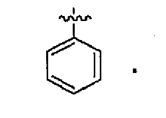
12. A process according to 6 in which R⁷, R¹², R¹³, R¹⁴, R¹⁵, R¹⁶, R¹⁷, R¹⁸, R¹⁹, R²⁰, R²¹ and R²² are H; and
   Z is straight or branched alkyl(C₁-C₉) or straight or branched alkenyl(C₂-C₁₀);
13. A process according to 6 in which R⁷, R¹², R¹³, R¹⁴, R¹⁵, R¹⁶,R¹⁷, R¹⁸, R¹⁹, R²⁰, R²¹ and R²² are H;
   Z is straight or branched alkyl(C₁-C₉) or straight or branched alkenyl(C₂-C₁₀); and R³ and R⁴ are H.
14. A process according to 6 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₉) or straight or branched alkenyl(C₂-C₁₀);and R³ and R⁴ are OH.
15. A process for producing a compound of the formula: 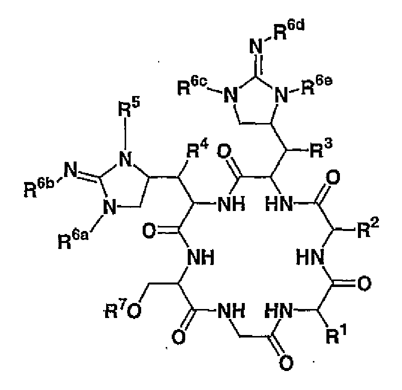 wherein:
   R¹ is selected from: 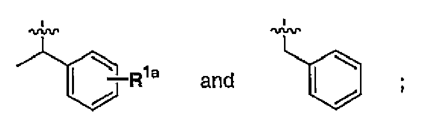
   R^{1a} is H;
   R² is a moiety 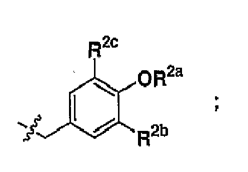
   R^{2a} is selected from
   H, 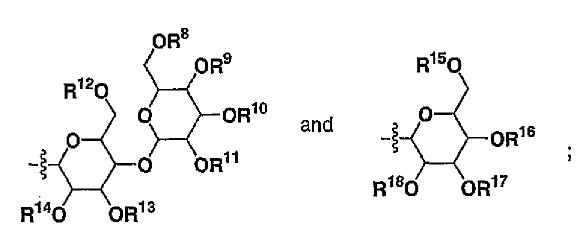
   R^{2b} is H;
   R^{2c} is H;
   R³ and R⁴ are independently H or OH;
   R⁵ is H or 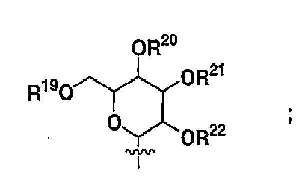
   R^{6a}, R^{6b}, R^{6c}, R^{6d}, or R^{6e} are H;
   R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently
      H, or -C(O)-Y-Z ;
   Y is a single bond;
   Z is straight or branched alkyl(C₁-C₂₀) or straight or branched alkenyl(C₂-C₂₀); which process comprises:
      cultivating a glycopeptide antibiotic producing strain of *Streptomyces hygroscopicus* selected from the group LL4600, LL4614, LL4666, LL4690, LL4728, LL4741, LL4742, LL4744, LL4773, LL4779, LL4783, LL4902, BD2, BD20 and BD70 or a mutant thereof under aerobic conditions, in a suitable culture medium and in the presence of a substrate ZCO₂H until a recoverable amount of said antibiotic is formed in said medium and recovering said antibiotic therefrom.
16. A process according to 15 in which R⁷, R¹², R¹³, R¹⁴, R¹⁵, R¹⁶, R¹⁷, R¹⁸, R¹⁹, R²⁰, R²¹ and R²² are H; and
   Z is straight or branched alkyl(C₁-C₉) or straight or branched alkenyl(C₂-C₁₀);
17. A process according to 15 in which R⁷, R¹², R¹³, R¹⁴, R¹⁵, R¹⁶, R¹⁷, R¹⁸, R¹⁹, R²⁰, and R²² are H;
   Z is straight or branched alkyl(C₁-C₉) or straight or branched alkenyl(C₂-C₁₀);and R³ and R⁴ are H.
18. A process according to 15 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₉) or straight or branched alkenyl(C₂-C₁₀);and R³ and R⁴ are OH.
19. A process for producing a compound of the formula: 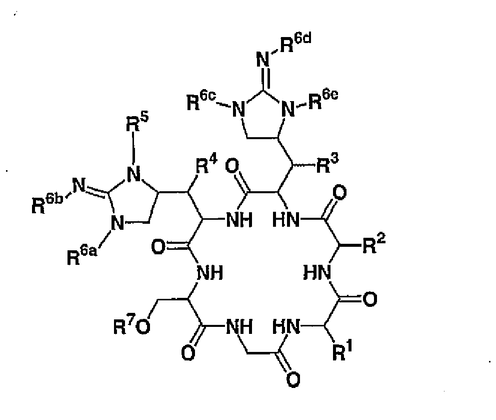 wherein:
   R¹ is selected from: 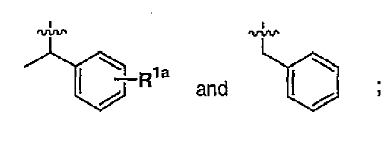
   R^{1a} is H;
   R² is a moiety 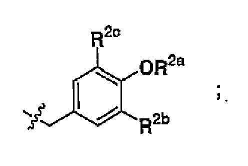
   R^{2a} is selected from 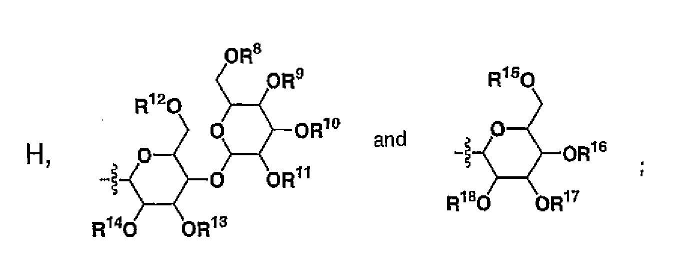
   R^{2b} is H, F, or NH₂;
   R^{2c} is H;
   R³ and R⁴ are independently H or OH;
   R⁵ is H or 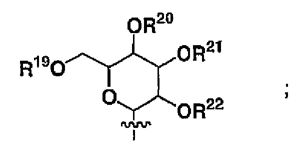
   R^{6a}, R^{6b}, R^{6c}, R^{6d}, or R^{6e} are H;
   R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently
      H, or -C(O)-Y-Z ;
   Y is a single bond ;
   Z is straight or branched alkyl(C₁-C₂₀) or straight or branched alkenyl(C₂-C₂₀);
   which process comprises:
      cultivating a glycopeptide antibiotic producing strain of *Streptomyces hygroscopicus* selected from the group LL4600, LL4614, LL4666, LL4690, LL4728, LL4741, LL4742, LL4744, LL4773, LL4779, LL4783, LL4902, BD2, BD20 and BD70 or a mutant thereof under aerobic conditions, in a suitable culture medium in the presence of a substrate 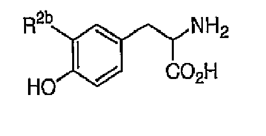 until a recoverable amount of said antibiotic is formed in said medium and recovering said antibiotic therefrom.
20. A process according to 19 in which R⁷, R¹², R³³, R¹⁴, R¹⁵, R¹⁶, R¹⁷, R¹⁸, R¹⁹, R²⁰, R²¹ and R²² are H; and
   Z is straight or branched alkyl(C₁-C₉) or straight or branched alkenyl(C₂-C₁₀);
21. A process according to 19 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₉) or straight or branched alkenyl(C₂-C₁₀);and R³ and R⁴ are H.
22. A process according to 19 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₉) or straight or branched alkenyl(G₂-C₁₀);and
   R³ and R⁴ are OH.
23. A process for producing a compound of the formula: 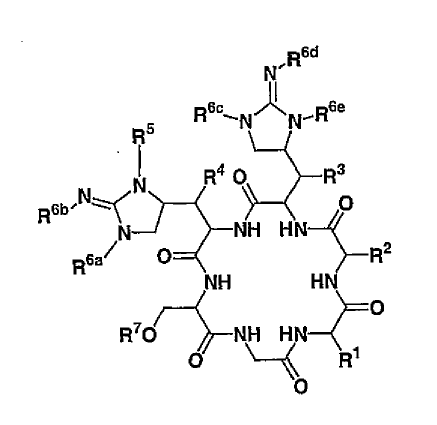 wherein:
   R¹ is selected from: 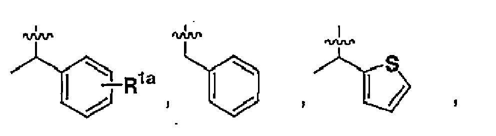 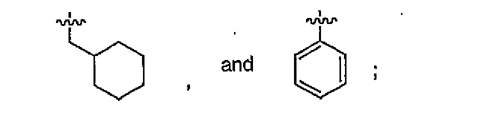
   R^{1a} is H or halogen;
   R² is a moiety 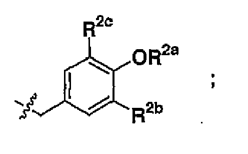
   R^{2a} is selected from
   H, 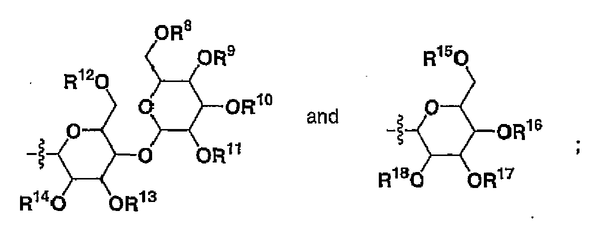
   R^{2b} is H;
   R^{2c} is H; R^{2c} is H;
   R³ and R⁴ are independently H or OH;
   R⁵ is H or 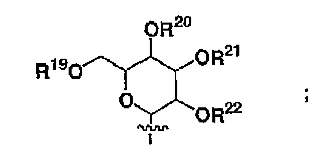
   R^{6a}, R^{6b}, R^{6c}, R^{6b}, or R^{6e} are H;
   R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently
      H, or -C(O)-Y-Z ;
   Y is a single bond ;
      Z is straight or branched alkyl(C₁-C₂₀) or straight or branched alkenyl(C₂-C₂₀);
   which process comprises:
      cultivating a glycopeptide antibiotic producing strain of *Streptomyces hygroscopicus* selected from the group LL4600, LL4614, LL4666, LL4690, LL4728, LL4741, LL4742, LL4T73, LL4783, LL4902, BD2, BD20 and BD70 or a mutant thereof under aerobic conditions, in a suitable culture medium in the presence of substrates 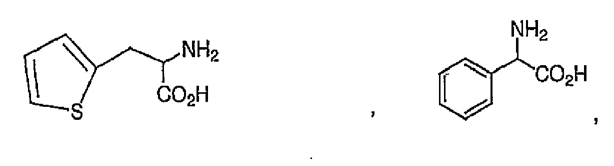 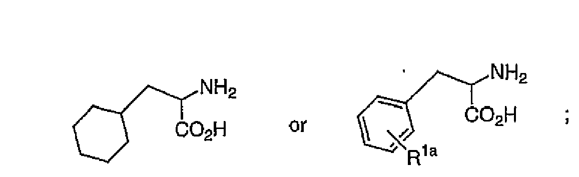 and ZCO₂H until a recoverable amount of said antibiotic is formed in said medium and recovering said antibiotic therefrom.
24. The process according to 23 wherein R¹ is 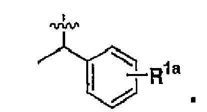
25. The process according to 23 wherein R¹ is 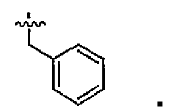
26. The process according to 23 wherein R¹ is 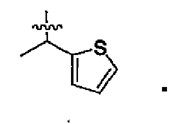
27. The process according to 23 wherein R¹ is 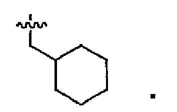
28. The process according to 23 wherein R¹ is 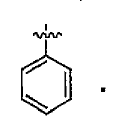
29. A process according to 23 in which R⁷, R¹², R¹³, R¹⁴, R¹⁵, R¹⁶, R¹⁷, R¹⁸, R¹⁹, R²⁰, R²¹ and R²² are H; and
   Z is straight or branched alkyl(C₁-C₉) or straight or branched alkenyl(C₂-C₁₀).
30. A process according to 23 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₉) or straight or branched alkenyl(C₂-C₁₀);and R³ and R⁴ are H.
31. A process according to 23 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₉) or straight or branched alkenyl(C₂-C₁₀);and
   R³ and R⁴ are OH.
32. A process for producing a compound of the formula: 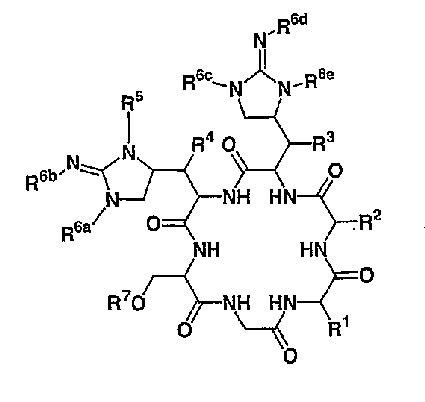 wherein:
   R¹ is selected from: 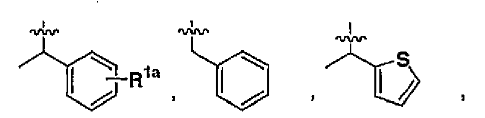 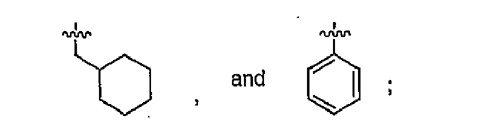
   R^{1a} is H or halogen;
   R² is a moiety 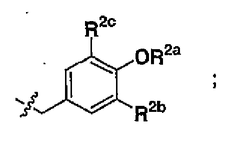
   R^{2a} is selected from
   H, 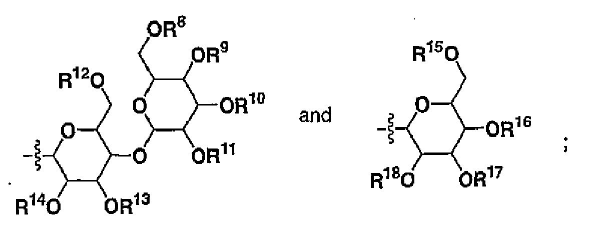
   R^{2b} is H;
   R^{2c} is H;
   R³ and R⁴ are independently H or OH;
   R⁵ is H or 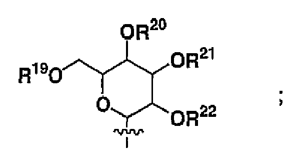
   R^{6a}, R^{6b}, R^{6c}, R^{6d}, or R^{6e} are H;
   R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently
      H, or -C(O)-Y-Z ;
   Y is a single bond;
      Z is straight or branched alkyl(C₁-C₂₀) or straight or branched alkenyl(C₂-C₂₀);
   which process comprises:
      cultivating a glycopeptide antibiotic producing strain of *Streptomyces hygroscopicus* selected from the group LL4600, LL4614, LL4666, LL4690, LL4728, LL4741, LL4742, LL4773, LL4783, LL4902, BD2, BD20 and BD70 or a mutant thereof under aerobic conditions, in a suitable culture medium in the presence of substrates 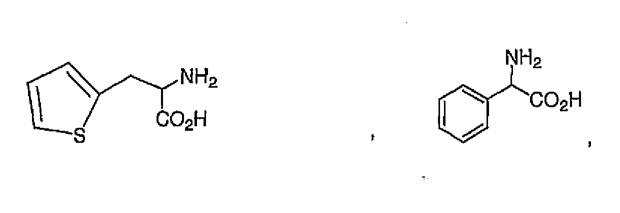 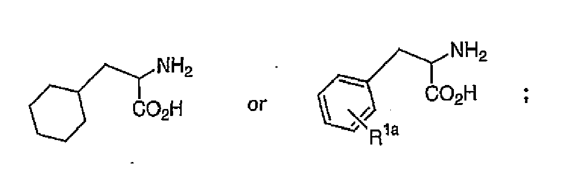 and 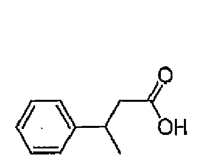 until a recoverable amount of said antibiotic is formed in said medium and recovering said antibiotic therefrom.
33. The process according to 32 wherein R¹ is 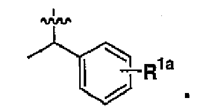
34. The process according to 32 wherein R¹ is 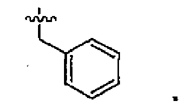
35. The process according to 32 wherein R¹ is 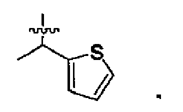
36. The process according to 32 wherein R¹ is 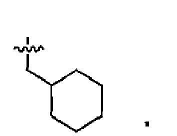
37. The process according to 32 wherein R¹ is 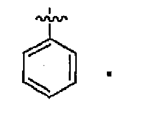
38. A process according to 32 in which R⁷, R¹², R¹³, R¹⁴, R¹⁵, R¹⁶, R¹⁷, R¹⁸, R¹⁹, R²⁰, R²¹ and R²² are H; and
   Z is straight or branched alkyl(C₁-C₉) or straight or branched alkenyl(C₂-C₁₀).
39. A process according to 32 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₉) or straight or branched alkenyl(C₂-C₁₀);and
   R³ and R⁴ are H.
40. A process according to 32 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₉) or straight or branched alkenyl(C₂-C₁₀);and
   R³ and R⁴ are OH.
41. A process for producing a compound of the formula: 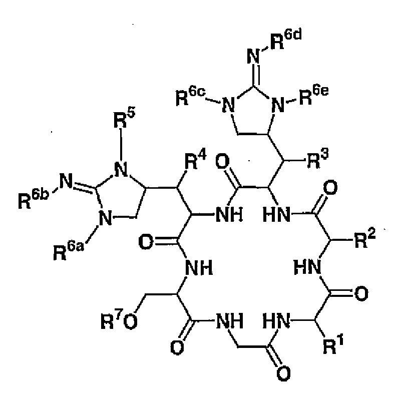 wherein:
   R¹ is selected from: 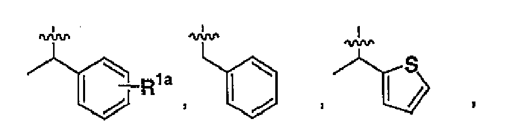 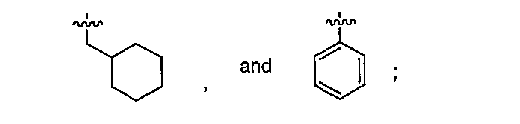
   R^{1a} is H or halogen;
   R² is a moiety 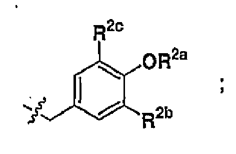
   R^{2a} is selected from
   H, 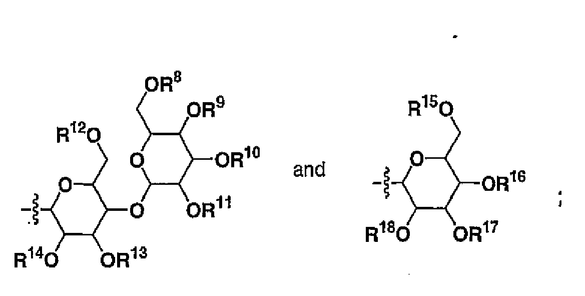
   R^{2b} is H, F, or NH₂;
   R^{2c} is H;
   R³ and R⁴ are independently H or OH;
   R⁵ is H or 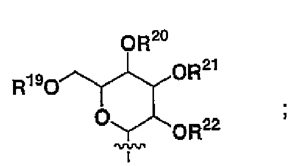
   R^{6a}, R^{6b}, R^{6c}, R^{6d}, or R^{6e} are H;
   R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently
      H, or -C(O)-Y-Z ;
   Y is a single bond;
      Z is straight or branched alkyl(C₁-C₂₀) or straight or branched alkenyl(C₂-C₂₀); which process comprises:
      cultivating a glycopeptide antibiotic producing strain of *Streptomyces hygroscopicus* selected from the group LL4600, LL4614, LL4666, LL4690, LL4728, LL4741, LL4742, LL4773, LL4783, LL4902, BD2, BD20 and BD70 or a mutant thereof under aerobic conditions, in a suitable culture medium in the presence of substrates 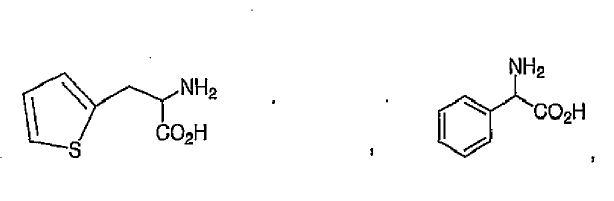 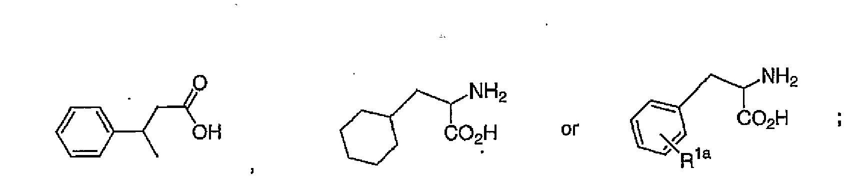 and 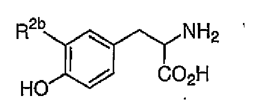 until a recoverable amount of said antibiotic is formed in said medium and recovering said antibiotic therefrom.
42. The process according to 41 wherein R¹ is 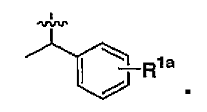
43. The process according to 41 wherein R¹ is 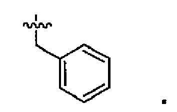
44. The process according to 41 wherein R¹ is 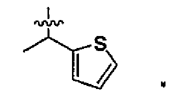
45. The process according to 41 wherein R¹ is 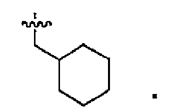
46. The process according to 41 wherein R¹ is 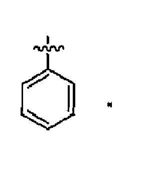
47. A process according to 41 in which R⁷, R¹², R¹³, R¹⁴, R¹⁵, R¹⁶, R¹⁷, R¹⁸, R¹⁹, R²⁰, R²¹ and R²² are H; and
   Z is straight or branched alkyl(C₁-C₉) or straight or branched alkenyl(C₂-C₁₀);
48. A process according to 41 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₉) or straight or branched alkenyl(C₂-C₁₀);and
   R³ and R⁴ are H.
49. A process according to 41 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₉) or straight or branched alkenyl(C₂-C₁₀);and
   R³ and R⁴ are OH.
50. A process for producing a compound of the formula: 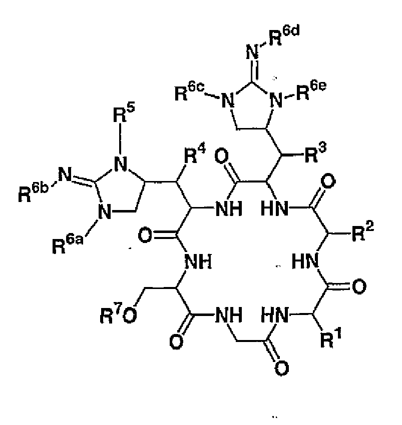 wherein:
   R¹ is selected from: 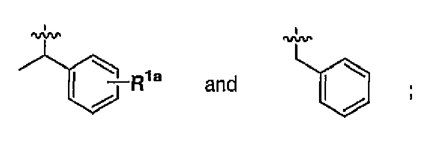
   R^{1a} is H;
   R² is a moiety 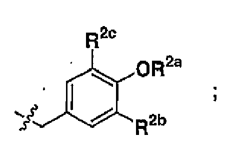
   R^{2a} is selected from
   H, 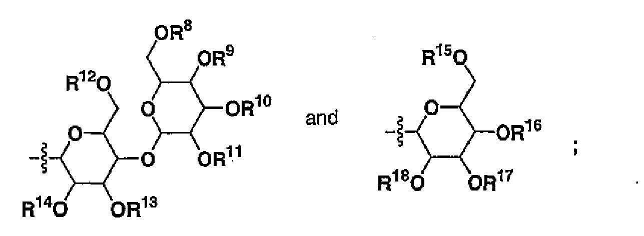
   R^{2b} is H, F or NH₂;
   R^{2c} is H;
   R³ and R⁴ are independently H or OH;
   R⁵ is H or 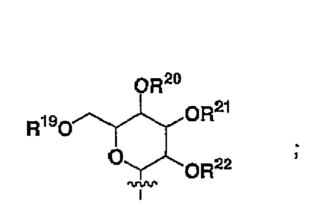
   R^{6a}, R^{6b}, R^{6c}, R^{6d}, or R^{6e} are H;
   R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently
      H, or -C(O)-Y-Z;
   Y is a single bond;
      Z is straight or branched alkyl(C₁-C₂₀) or straight or branched alkenyl(C₂-C₂₀);
   which process comprises:
      cultivating a glycopeptide antibiotic producing strain of *Streptomyces hygroscopicus* selected from the group LL4600, LL4614, LL4666, LL4690, LL4728, LL4741, LL4742, LL4773, LL4783, LL4902, BD2, BD20 and BD70 or a mutant thereof under aerobic conditions, in a suitable culture medium in the presence of substrates 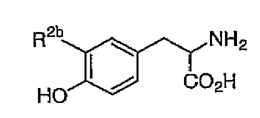 and ZCO₂H
      until a recoverable amount of said antibiotic is formed in said medium and recovering said antibiotic therefrom.
51. The process according to 50 wherein R¹ is 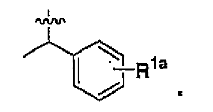
52. The process according to 50 wherein R¹ is 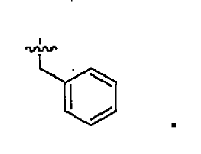
53. A process according to claim 50 in which R⁷, R¹², R¹³, R¹⁴, R¹⁵, R¹⁶, R¹⁷, R¹⁸, R¹⁹, R²⁰, R²¹ and R²² are H; and
   Z is straight or branched alkyl(C₁-C₉) or straight or branched alkenyl(C₂-C₁₀).
54. A process according to claim 50 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₉) or straight or branched alkenyl(C₂-C₁₀);and
   R³ and R⁴ are H.
55. A process according to claim 50 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₉) or straight or branched alkenyl(C₂₋C₁₀);and
   R³ and R⁴ are OH.
56. A process for producing a compound of the formula: 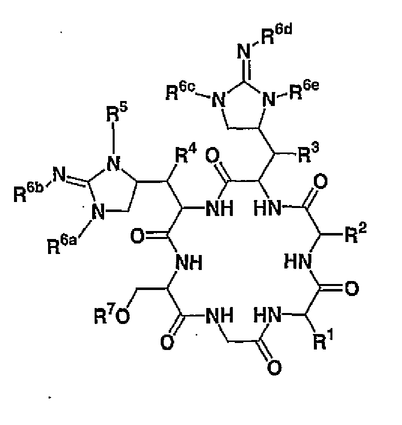 wherein:
   R¹ is selected from: 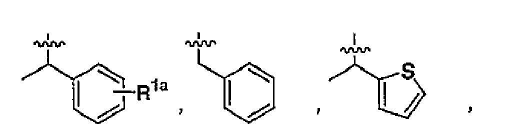 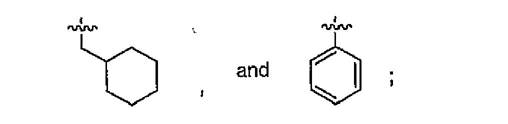
   R^{1a} is H or halogen;
   R² is a moiety 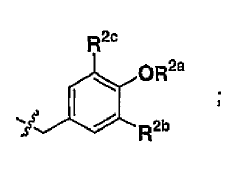
   R^{2a} is selected from
   H, 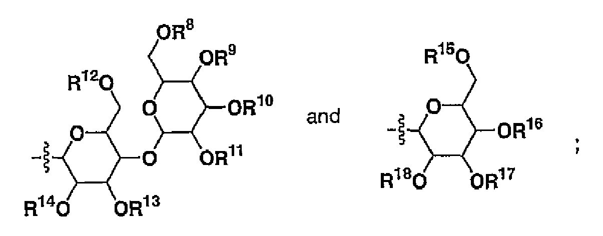
   R^{2b} is H, F,or NH₂;
   R^{2c} is H;
   R³ and R⁴ are independently H or OH;
   R⁵ is H or 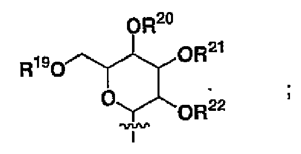
   R^{6a}, R^{6b}, R^{6c}, R^{6d}, or R^{6e} are H;
   R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently
      H, or -C(O)-Y-Z ;
   Y is a single bond;
      Z is straight or branched alkyl(C₁-C₂₀) or straight or branched alkenyl(C₂-C₂₀);
   which process comprises cultivating a glycopeptide antibiotic producing strain of *Streptomyces hygroscopicus* selected from the group LL4600, LL4614, LL4666, LL4690, LL4728, LLA741, LL4742, LL4773, LL4783, LL4902, BD2, BD20 and BD70 or a mutant thereof under aerobic conditions, in a suitable culture medium in the presence of substrates 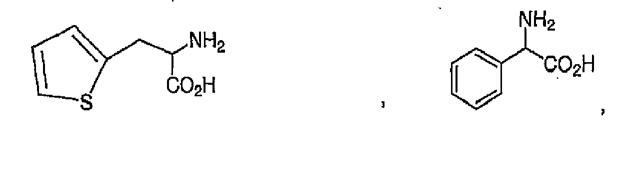 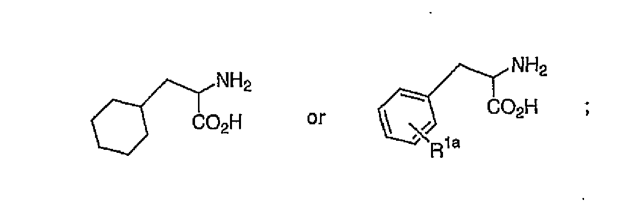 and 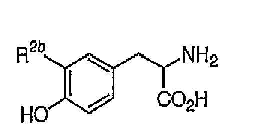 and ZCO₂H
      until a recoverable amount of said antibiotic is formed in said medium and recovering said antibiotic therefrom.
57. The process according to 56 wherein R¹ is 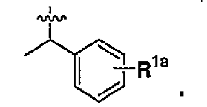
58. The process according to 56 wherein R¹ is 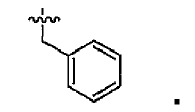
59. The process according to 56 wherein R¹ is 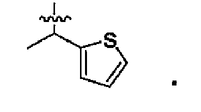
60. The process according to 56 wherein R¹ is 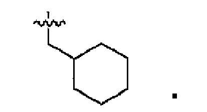
61. The process according to 56 wherein R¹ is 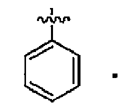
62. A process according to 56 in which R⁷, R¹², R¹³, R¹⁴, R¹⁵, R¹⁶, R¹⁷, R¹⁸, R¹⁹, R²⁰, R²¹ and R²² are H; and
   Z is straight or branched alkyl(C₁-C₉) or straight or branched alkenyl(C₂-C₁₀).
63. A process according to 56 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₉) or straight or branched alkenyl(C₂-C₁₀);and
   R³ and R⁴ are H.
64. A process according to 56 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₉) or straight or branched alkenyl(C₂-C₁₀);
   and
   R³ and R⁴ are OH.
65. A process for producing a compound of the formula: 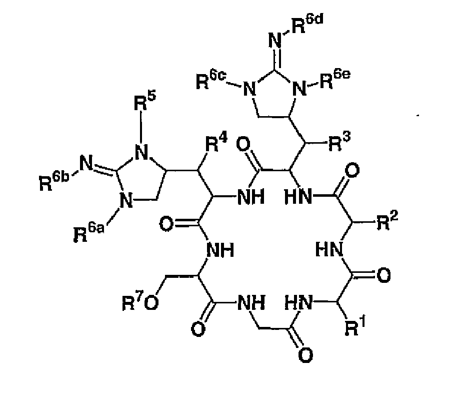 wherein:
   R¹ is selected from: 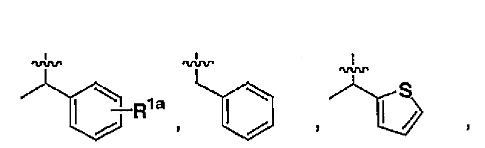 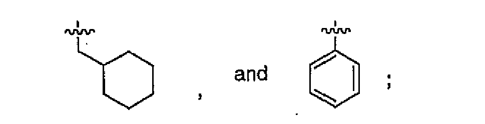
   R^{1a} is H or halogen;
   R² is a moiety 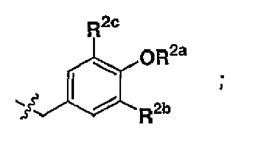
   R^{2a} is selected from
   H, 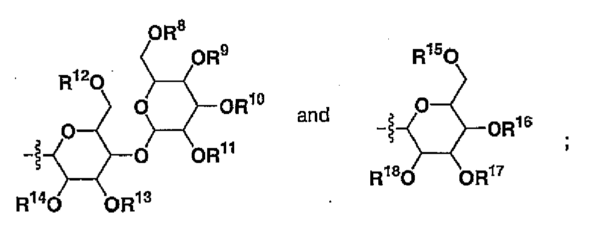
   R^{2b} is H, F, or NH₂;
   R^{2c} is H;
   R³ and R⁴ are independently H or OH;
   R⁵ is H or 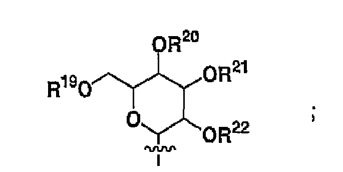
   R^{6a}, R^{6b}, R^{6c}, R^{6d}, or R^{6e} are H;
   R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently
      H, or -C(O)-Y-Z;
   Y is a single bond;
      Z is straight or branched alkyl(C₁-C₂₀) or straight or branched alkenyl(C₂-C₂₀); which process comprises:
      cultivating a glycopeptide antibiotic producing strain of *Streptomyces hygroscopicus* selected from the group LL4600, LL4614, LL4666, LL4690, LL4728, LL4741, LL4742, LL4773, LL4783, LL4902, BD2, BD20 and BD70 or a mutant thereof under aerobic conditions, in a suitable culture medium in the presence of substrates 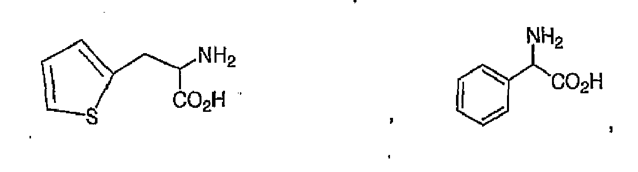 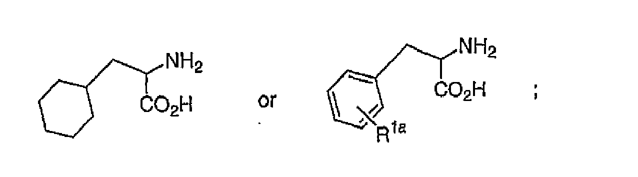 and 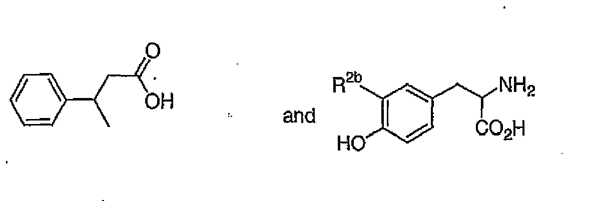 until a recoverable amount of said antibiotic is formed in said medium and recovering said antibiotic therefrom.
66. The process according to 65 wherein R¹ is 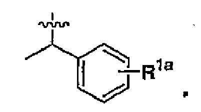
67. The process according to 65 wherein R¹ is 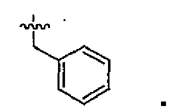
68. The process according to 65 wherein R¹ is 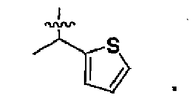
69. The process according to 65 wherein R¹ is 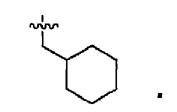
70. The process according to 65 wherein R¹ is 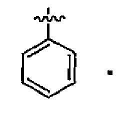
71. A process according to 65 in which R⁷, R¹², R¹³, R¹⁴, R¹⁵, R¹⁶, R¹⁷, R¹⁸, R¹⁹, R²⁰, R²¹ and R²² are H; and
   Z is straight or branched alkyl(C₁-C₉) or straight or branched alkenyl(C₂-C₁₀);
72. A process according to 65 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₉₎ or straight or branched alkenyl(C₂-C₁₀);and
   R³ and R⁴ are H.
73. A process according to claim 65 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₉) or straight or branched alkenyl(C₂-C₁₀);and
   R³ and R⁴ are OH.
74. A process for producing a compound of the formula: 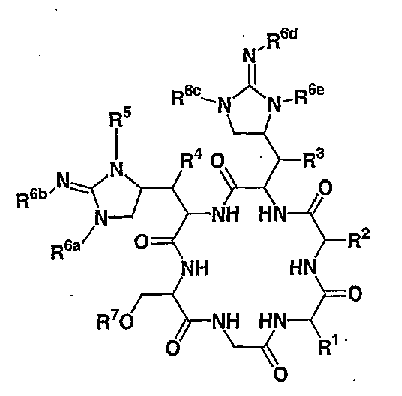 wherein:
   R¹ is selected from 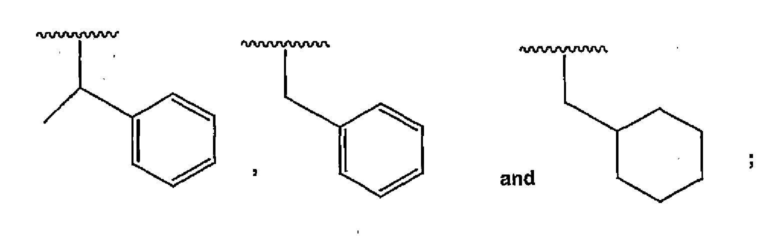
   R² is a moiety 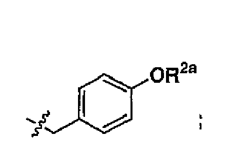
   R^{2a} is selected from
   H, 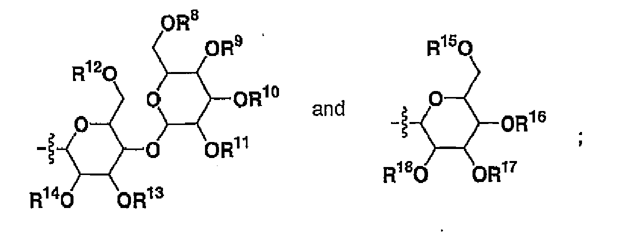
   R^{2b} is H;
   R^{2c} is H;
   R³ and R⁴ are independently H or OH;
   R⁵ is H or 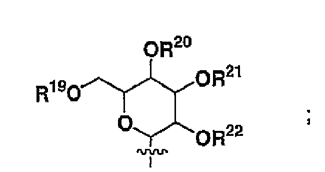
   R^{6a}, R^{6b}, R^{6c}, R^{6d}, or R^{6e} are H;
   R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ and R¹² are independently
      H, or -C(O)-Y-Z;
      Y is a single bond;
      Z is straight or branched alkyl(C₁-C₂₀) or straight or branched alkenyl(C₂-C₂₀);
      which process comprises cultivating a glycopeptide antibiotic producing strain of *Streptomyces hygroscopicus* selected from the group LL4600, LL4614, LL4666, LL4690, LL4728, LL4741, LL4742, LL4744, LL4773, LL4779, LL4783, LL4902, BD2, BD20 and BD70 or a mutant thereof under aerobic conditions, in a suitable culture medium in the presence of a substrate 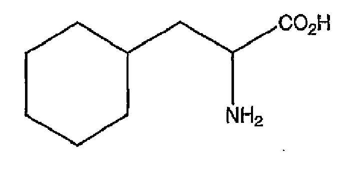 until a recoverable amount of said antibiotic is formed in said medium and recovering said antibiotic from said medium.
75. A process according to 74 in which R⁷, R¹², R¹³, R¹⁴, R¹⁵, R¹⁶, R¹⁷, R¹⁸, R¹⁹, R²⁰, R²¹ and R²² are H; and
   Z is straight or branched alkyl(C₁-C₉) or straight or branched alkenyl(C₂-C₁₀).
76. A process according to 74 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₉) or straight or branched akenyl(C₂-C₁₀); and R³ and R⁴ are H.
77. A process according to 74 in which R⁷, R¹², R¹³, R¹⁴, R¹⁵, R¹⁶, R¹⁷, R¹⁸, R¹⁹, R²⁰, R²¹ and R²² are H;
   Z is straight or branched alkyl(C₁-C₉) or straight or branched alkenyl(C₂-C₁₀);
   and
   R³ and R⁴ are OH.
78. A process according to 74 in which R¹ is 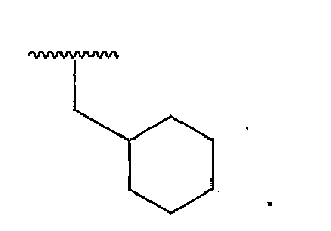
79. A process for producing of a compound of the formula: 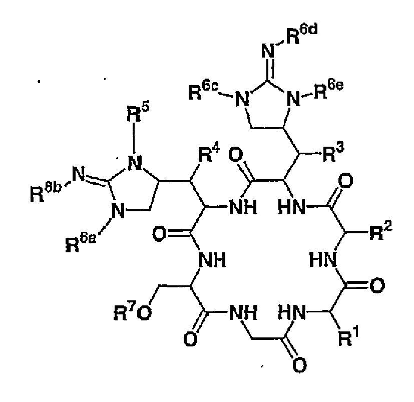 wherein:
   R¹ is selected from: 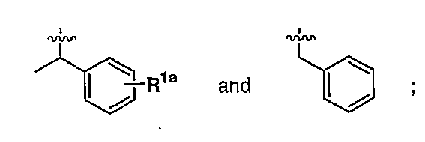
   R^{1a} is H;
   R² is a moiety 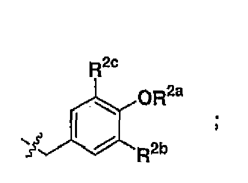
   R^{2a} is H;
   R^{2b} is H;
   R^{2c} is H;
   R³ and R⁴ are independently H or OH;
   R⁵ is H;
   R^{6a}, R^{6b}, R^{6c}, R^{6d}, or R^{6e} are H;
   R⁷ is H;
      which process comprises:
      cultivating a glycopeptide antibiotic producing strain of *Streptomyces hygroscopicus* selected from the group LL4742, LL4744, LL4902, BD2, BD20 and BD70 or a mutant thereof under aerobic conditions, in a suitable culture medium until a recoverable amount of said antibiotic is formed in said medium and recovering said antibiotic therefrom.
80. A process for producing of a compound of the formula: 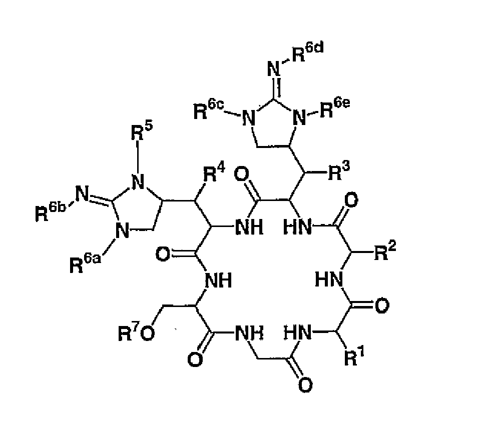 wherein:
   R¹ is selected from: 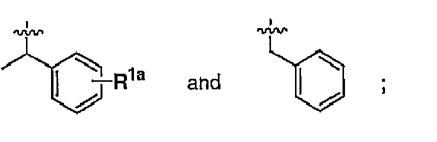
   R^{1a} is H;
   R² is a moiety 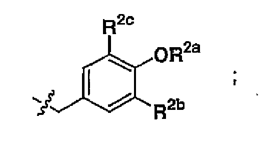
   R^{2a} is selected from
   H, 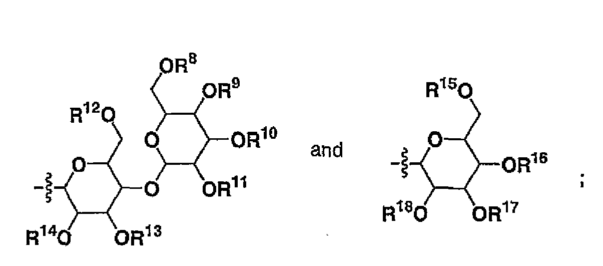
   R^{2b} is H;
   R^{2c} is H;
   R³ and R⁴ are independently H or OH;
   R⁵ is H or 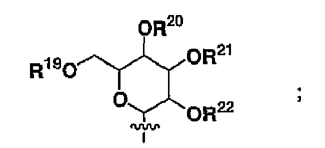
   R^{6a}, R^{6b}, R^{6c}, R^{6d}, or R^{6e} are H;
   R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently
      H, or -C(O)-Y-Z;
   Y is a single bond;
      Z is straight or branched alkyl(C₁-C₂₀) or straight or branched alkenyl(C₂-C₂₀); which process comprises:
      cultivating a glycopeptide antibiotic producing strain of *Streptomyces hygroscopicus* selected from the group LL4742, LL4902, BD2, BD20 and BD70 or a mutant thereof under aerobic conditions, in the presence of mannose in a suitable culture medium until a recoverable amount of said antibiotic is formed in said medium and recovering said antibiotic therefrom.
81. A process for producing a compound of the formula: 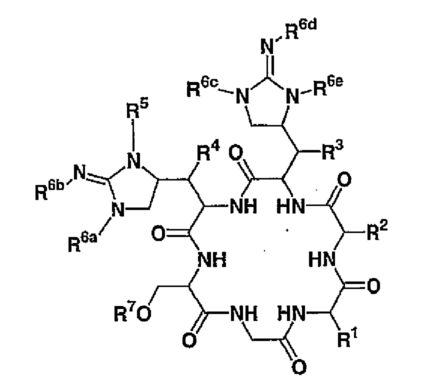 wherein:
   R¹ is selected from: 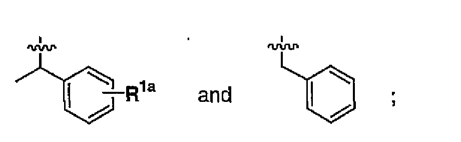
   R^{1a} is H;
   R² is a moiety 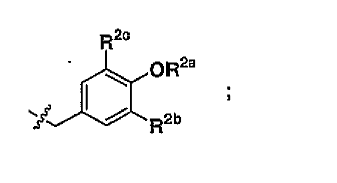
   R^{2a} is selected from
   H, 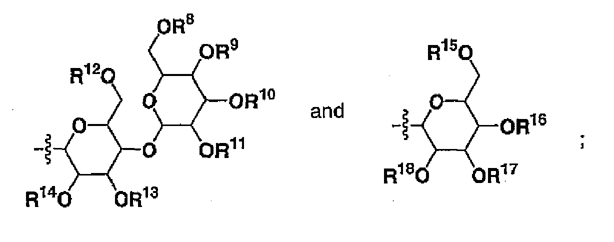
   R^{2b} is H;
   R^{2c} is H;
   R³ and R⁴ are independently H or OH;
   R⁵ is H or 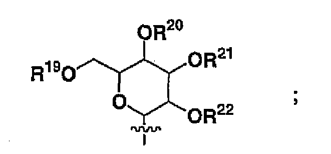
   R^{6a}, R^{6b}, R^{6c}, R^{6d}, or R^{6e} are H;
   R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are H; which process comprises:
      cultivating a glycopeptide antibiotic producing strain of *Streptomyces hygroscopicus* selected from the group LL4600, LL4614, LL4666, LL4690, LL4728, LL4741, LL4742, LL4744, LL4773, LL4779, LL4783, LL4902, BD2, BD20 and BD70 or a mutant thereof under aerobic conditions, in a suitable culture medium until a recoverable amount of said antibiotic is formed in said medium and while maintaining the temperature between about 0° C to about 25° C, hydrolyzing at a pH of about 8.0 to about 13.5 and recovering said antibiotic at a pH of about 1.8 to about 6.5.
82. The process of 81 wherein the hydrolyzing step is accomplished with sodium hydroxide.
83. The process of 81 wherein the hydrolyzing step is accomplished with (3-[cyclohexylamino]-1-propanesulfonic acid).
84. The process of 81 wherein the pH is adjusted from about 8.0 to about 12.5 with acetic acid or propanoic acid to about 4.0 to about 5.0.
85. The process of 81 wherein the pH is adjusted from about 8.0 to about 12.5 with hydrochloric acid to about 1.8 to about 5.0.
86. The process of 81 wherein the pH is adjusted with (3-[N-morpholino]propanesulfonic acid) to about 6.0 to about 6.5.
87. A process for producing a compound of the formula 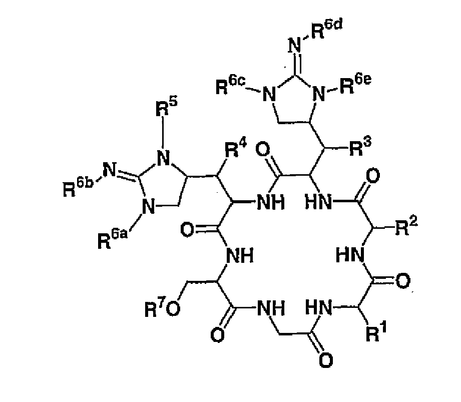 wherein:
   R¹ is selected from: 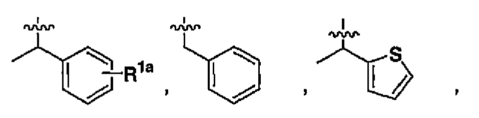 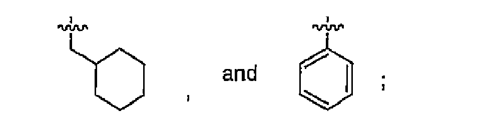
   R^{1a} is H or halogen;
   R² is a moiety 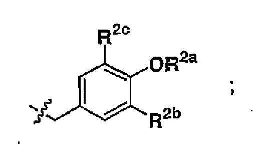
   R^{2a} is selected from H, 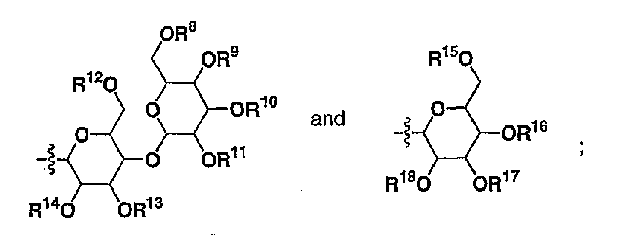
   R^{2b} is H, F,or NH₂;
   R^{2c} is H;
   R³ and R⁴ are independently H or OH;
   R⁵ is H or 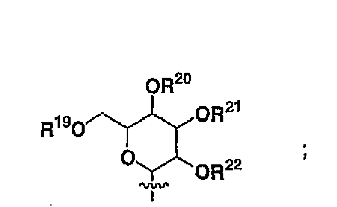
   R^{6a}, R^{6b}, R^{6c}, R^{6d}, or R^{6c} are H;
   R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, and R²² are independently
      H, or -C(O)-Y-Z;
   Y is a single bond;
      Z is straight or branched alkyl(C₁-C₂₀) or straight or branched alkenyl(C₂-C₂₀); which process comprises:
      cultivating a glycopeptide antibiotic producing strain of *Streptomyces hygroscopicus* LL4780, or a mutant thereof under aerobic conditions, in a suitable culture medium in the presence of a substrate: 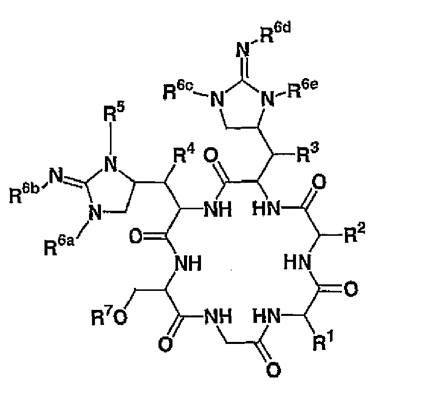 wherein:
      R¹ is selected from: 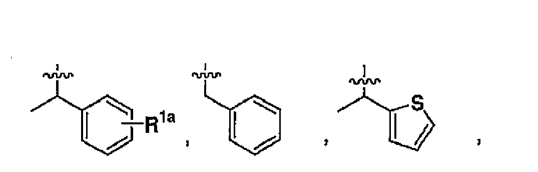 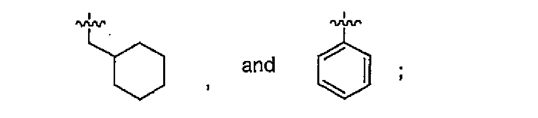
      R^{1a} is H or halogen;
      R² is a moiety 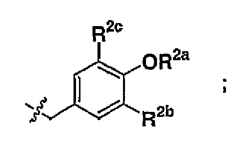
      R^{2a} is H and 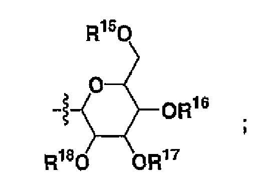
      R^{2b} is H, F, or NH₂;
      R^{2c} is H;
      R³ and R⁴ are independently H or OH;
      R⁵ is H or 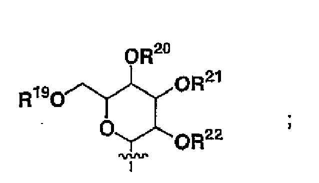
      R^{6a}, R^{6b}, R^{6c}, R^{6d}, or R^{6e} are H;
   until a recoverable amount of said antibiotic is formed in said medium and recovering said antibiotic therefrom.
88. A process for the production of a compound of the formula: 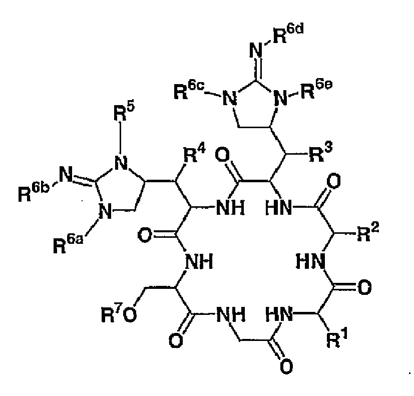 wherein:
   R¹ is 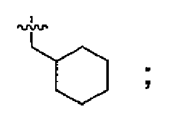
   R² is a moiety 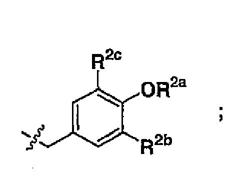
   R^{2a} is 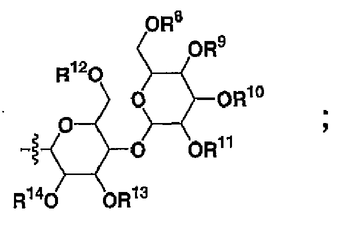
   R^{2b} is H;
   R^{2c} is H;
   R³ and R⁴ are independently H or OH;
   R⁵ is H or 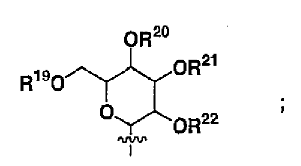
   R^{6a}, R^{6b}, R^{6c}, R^{6d}, or R^{6e} are H;
   R⁷, R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are H;
   the process comprising cultivating a glycopeptide antibiotic producing strain of *Streptomyces hygroscopicus* which is selected from the group LL4600, LL4614, LL4666, LL4690, LL4728, LL4741, LL4742, LL4744, LL4773, LL4779, LL4783, LL4902, BD2, BD20 and BD70 or a mutant thereof under aerobic conditions, in a suitable culture medium in the presence of alpha-aminocyclohexane-propionic acid until a recoverable amount of said antibiotic is formed in said medium and recovering said antibiotic therefrom.
89. A microorganism *Streptomyces hygroscopicus* LL4600, or a mutant thereof.
90. A microorganism *Streptomyces hygroscopicus* LL4614, or a mutant thereof.
91. A microorganism *Streptomyces hygroscopicus* LL4666, or a mutant thereof.
92. A microorganism *Streptomyces hygroscopicus* LL4690, or a mutant thereof.
93. A microorganism *Streptomyces hygroscopicus* LL4728, or a mutant thereof.
94. A microorganism *Streptomyces hygroscopicus* LL4741, or a mutant thereof.
95. A microorganism *Streptomyces hygroscopicus* LL4742, or a mutant thereof..
96. A microorganism *Streptomyces hygroscopicus* LL4744, or a mutant thereof.
97. A microorganism *Streptomyces hygroscopicus* LL4773, or a mutant thereof.
98. A microorganism *Streptomyces hygroscopicus* LL4779, or a mutant thereof.
99. A microorganism *Streptomyces hygroscopicus* LL4780, or a mutant thereof.
100. A microorganism *Streptomyces hygroscopicus* LL4783, or a mutant thereof.
101. A microorganism *Streptomyces hygroscopicus* LL4902, or a mutant thereof.
102. A microorganism *Streptomyces hygroscopicus* BD2. or a mutant thereof.
103. A microorganism *Streptomyces hygroscopicus* BD20, or a mutant thereof.
104. A microorganism *Streptomyces hygroscopicus* BD70, or a mutant thereof.
105. A process for producing glycopeptides of the formula 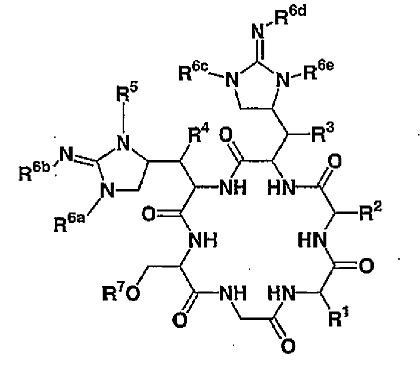 wherein:
   R¹ is a moiety selected from: 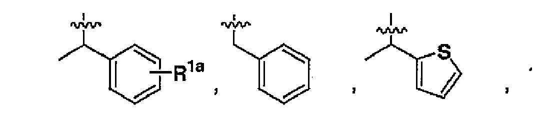 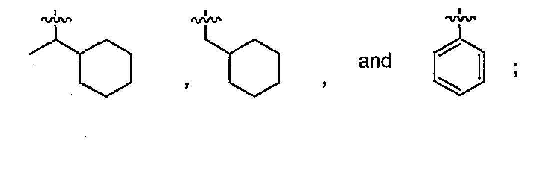
   R^{1a} is H or halogen;
   R² is a moiety selected from: 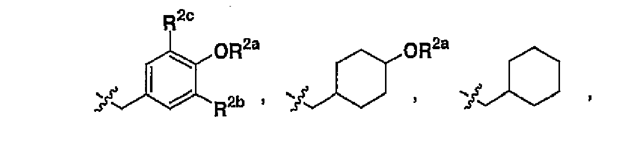 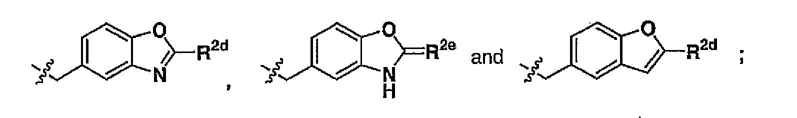
   provided when R² is selected from the moieties: 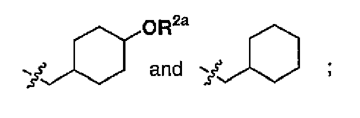
   that R¹ is selected from the moieties: 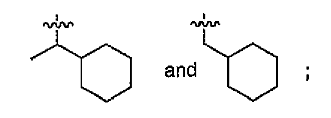
   R^{2a} is selected from H, alkyl(C₁-C₂₀), cycloalkyl(C₃-C₂₀), alkenyl(C₃-C₂₀), alkynyl(C₃-C₂₀), the group -C(O)-Y-Z, and a moiety selected from: 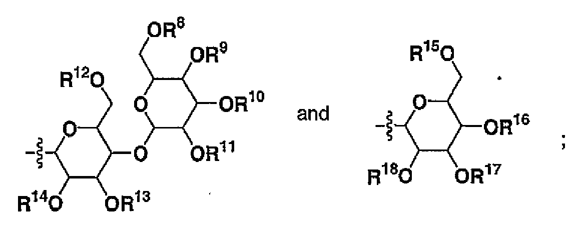
   Y is selected from a single bond, -O- and -NR^{8a}-;
   Z is selected from alkyl(C₁-C₂₀), cycloalkyl(C₃-C₂₀), alkenyl(C₃-C₂₀),
   alkynyl(C₃-C₂₀), aryl and heteroaryl;
   and when Y is a single bond then Z is also selected from H, alkenyl(C₂-C₂₀) and alkynyl (C₂-C₂₀);
   R^{2b} and R^{2c} are independently selected from H, halogen, alkyl(C₁-C₂₀), cycloalkyl(C₃-C₂₀), alkenyl(C₂-C₂₀), alkynyl(C₂-C₂₀), aryl, heteroaryl, -NH₂, -NR^{2f}R^{2g}, and -NO₂, provided when R^{2b} is -NO₂, that R^{2c} must be H;
   R^{2d} is selected from alkyl (C₁-C₂₀), alkenyl(C₂-C₂₀), alkynyl(C₂-C₂₀), aryl, heteroaryl, and when R² is 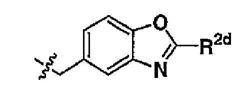
   R^{2d} may also be the group L-M, wherein;
   L is selected from -NH-, -S-, -SCH₂C(O)-, and a group of the formula: 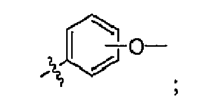
   M is selected from alkyl(C₁-C₂₀), cycloalkyl(C₃-C₂₀), alkenyl(C₃-C₂₀) and alkynyl(C₃-C₂₀); and when:
      a) L is not -S-, M may also be aryl or heteroaryl;
      b) L is -SCH₂C(O)-, M may also be H, alkenyl(C₂-C₂₀) or alkynyl(C₂-C₂₀); or
      c) L is -NH- or a group of the formula: 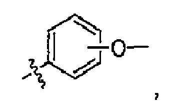
   M may also be a moiety of the formula: 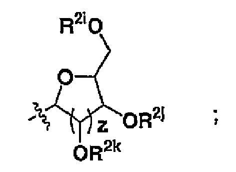
   z is an integer of 1 or 2;
   R²ⁱ, R^{2j} and R^{2k} are independently selected from H, alkyl(C₁-C₂₀), cycloalkyl(C₃-C₂₀), alkenyl(C₃-C₂₀), alkynyl(C₃-C₂₀), acyl, -Si(alkyl(C₁-C₂₀))₃, -Si(alkyl(C₁-C₂₀))₂(aryl), -Si(alkyl(C₁-C₂₀))(aryl)₂, -Si(aryl)₃, and aroyl;
   R^{2e} is S or 0;
   R^{2f} is selected from H, alkyl(C₁-C₂₀), cycloalkyl(C₃-C₂₀), alkenyl(C₃-C₂₀), alkynyl(C₃-C₂₀), aryl and heteroaryl;
   R^{2g} is H, alkyl(C₁-C₂₀), cycloalkyl(C₃-C₂₀), alkenyl(C₃-C₂₀), alkynyl(C₃-C₂₀), aryl and heteroaryl, and the group D-E-G, wherein;
   D is selected from -C(O)-, -C(S)- and -S(O)₂-;
   E is selected from a single bond, and, when D is -C(O)- or -C(S)-, E is also selected from -0- and -NR^{2h}-;
   G is selected from alkyl(C₁-C₂₀), cycloalkyl(C₃-C₂₀), alkenyl(C₃-C₂₀), alkynyl(C₃-C₂₀), aryl and heteroaryl;
   and when:
      a) E is a single bond, then G may also be alkenyl(C₂-C₂₀) or alkynyl(C₂-C₂₀); or
      b) D is -C(O)- or -C(S)- and E is a single bond, then G may also be H;
   R^{2f} and R^{2g} may optionally when taken together with the nitrogen atom to which each is attached form a monocyclic ring having three to seven atoms independently selected from the elements C, N, O and S where said monocyclic ring optionally contains up to three nitrogen atoms, up to two oxygen atoms and up to two sulfur atoms provided said monocyclic ring does not contain -0-0-, -S-S- or -S-O- bonds;
   R^{2h} is selected from H, alkyl(C₁-C₂₀), cycloalkyl(C₃-C₂₀), alkenyl(C₃-C₂₀), alkynyl(C₃-C₂₀), aryl and heteroaryl;
   R^{2h} and G may optionally when taken together with the nitrogen atom to which each is attached form a monocyclic ring having three to seven atoms independently selected from the elements C, N, O and S where said monocyclic ring optionally contains up to three nitrogen atoms, up to two oxygen atoms and up to two sulfur atoms provided said monocyclic ring does not contain -0-0-, -S-S- or -S-O- bonds;
   R³ and R⁴ are independently H, OH, -Si(alkyl(C₁-C₂₀))₃, -Si(alkyl(C₁-C₂₀))₂(aryl), -Si(alkyl(C₁-C₂₀))(aryl)₂, -Si(aryl)₃ or the group -C(O)-Y-Z;
   R⁵ is selected from H, alkyl(C₁-C₂₀), cycloalkyl(C₃-C₂₀), alkenyl(C₃-C₂₀), alkynyl(C₃-C₂₀), the group -C(O)-Y-Z and moieties of the formulae; 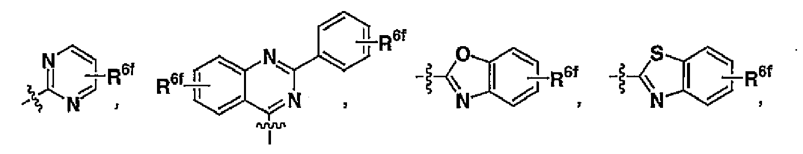 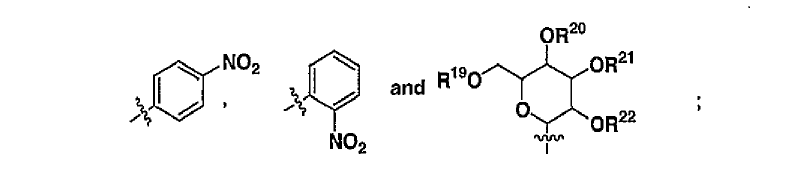
   R^{6a}, R^{6b}, R^{6c}, R^{6d} and R^{6e} are independently selected from H, alkyl(C₁-C₂₀), cycloalkyl(C₃-C₂₀), alkenyl(C₃-C₂₀), alkynyl(C₃-C₂₀), the group -C(O)-Y-Z and moieties of the formulae; 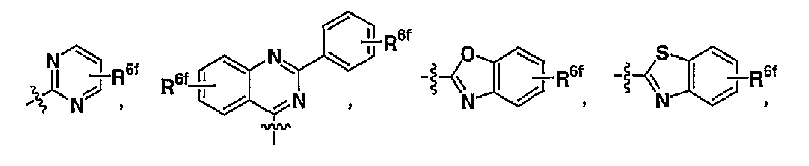 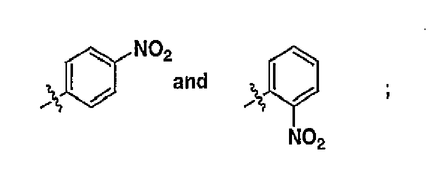
   R^{6f} is selected from H, alkyl(C₁-C₂₀), cycloallcyl(C₃-C₂₀), alkenyl(C₃-C₂₀), alkynyl(C₃-C₂₀), aryl, heteroaryl, halogen, hydroxy, alkaxy(C₁-C₂₀), aryloxy, amino, monoalkyl(C₁-C₂₀)amino, dialkyl(C₁-C₂₀)amino, carboxy, carboxyalkyl(C₁-C₂₀), carboxyaryl and carboxyamido;
   R⁷ is selected from H, alkyl(C₁-C₂₀), cycloalkyl(C₃-C₂₀), alkenyl(C₃-C₂₀), alkynyl(C₃-C₂₀), -Si(alkyl(C₁-C₂₀))₃, -Si(alkyl(C₁-C₂₀))₂(aryl), -Si(alkyl(C₁-C₂₀))(aryl)₂, -Si(aryl)₃ and the group -C(O)-Y-Z;
   R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently selected from H, alkyl(C₁-C₂₀), cycloalkyl(C₃-C₂₀), alkenyl(C₃-C₂₀), alkynyl(C₃-C₂₀), -Si(alkyl(C₁-C₂₀))₃, -Si(alkyl(C₁-C₂₀))₂(aryl), -Si(alkyl(C₁-C₂₀))(aryl)₂, -Si(aryl)₃ and the group -C(O)-Y-Z or, optionally, R⁸ and R⁹, R⁹ and R¹⁰, R¹⁰ and R¹¹, R¹³ and R¹⁴, R¹⁵ and R¹⁶, R¹⁶ and R¹⁷, R¹⁷ and R¹⁸, R¹⁹ and R²⁰, R²⁰ and R²¹ or R²¹ and R²² may independently be taken together forming moieties of the formulae: 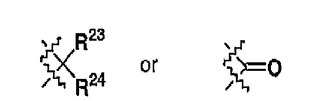 provided when;
      a) R⁸ and R⁹ are so joined, R⁹ and R¹⁰ may not be so joined;
      b) R⁹ and R¹⁰ are so joined, R⁸ and R⁹ and R¹⁰ and R¹¹ may not be so joined;
      c) R¹⁵ and R¹⁶ are so joined, R¹⁶ and R¹⁷ may not be so joined;
      d) R¹⁶ and R¹⁷ are so joined, R¹⁵ and R¹⁶, R¹⁷ and R¹⁸ may not be so joined;
      e) R¹⁹ and R²⁰ are so joined, R²⁰ and R²¹ may not be so joined;
      f) R²⁰ and R²¹ are so joined, R¹⁹ and R²⁰, and R²¹ and R²² may not be so joined;
   R^{8a} is selected from H, alkyl(C₁-C₂₀), cycloalkyl(C₃-C₂₀), alkenyl(C₃-C₂₀), alkynyl(C₃-C₂₀), aryl and heteroaryl;
   R^{8a} and Z may optionally when taken together with the nitrogen atom to which each is attached form a monocyclic ring having three to seven atoms independently selected from the elements C, N, O and S where said monocyclic ring optionally contains up to three nitrogen atoms, up to two oxygen atoms and up to two sulfur atoms provided said monocyclic ring does not contain -O-O-, -S-S- or -S-O- bonds;
   R²³ and R²⁴ are independently selected from H, alkyl(C₁-C₂₀), cycloalkyl(C₃-C₂₀), alkenyl(C₂-C₂₀), alkynyl(C₂-C₂₀), aryl, and heteroaryl;
   R²³ and R²⁴ may optionally when taken together with the carbon atom to which each is attached form carbocyclic, monocyclic, fused, bridged, spirocyclic or polycyclic rings from three to twenty ring atoms optionally selected from the elements C, N, 0 and S where said monocyclic ring optionally contains up to three nitrogen atoms, up to two oxygen atoms and up to two sulfur atoms provided said monocyclic ring does not contain -O-O-, -S-S- or -S-O- bonds;
   which process comprises:
   treating a compound of the formula: 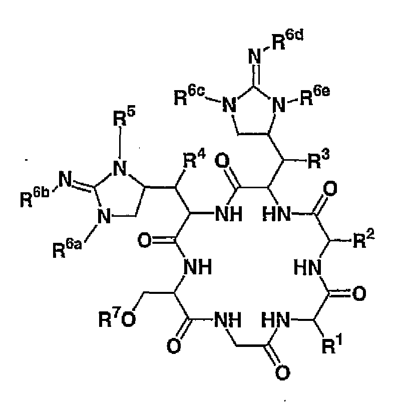 wherein:
   R² is selected from 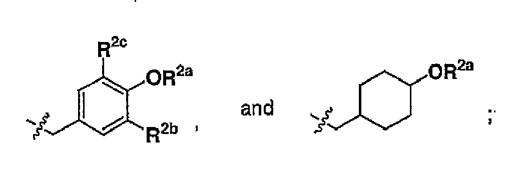
   R^{2a} is selected from 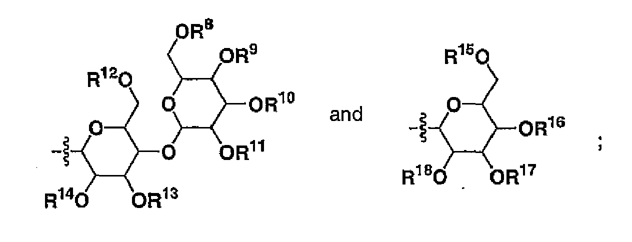 with an α-mannosidase in an aqueous buffer at about pH 3.5 to about 6.5 and isolating said glycopeptide therefrom.

## Claims

1. A compound of the formula: 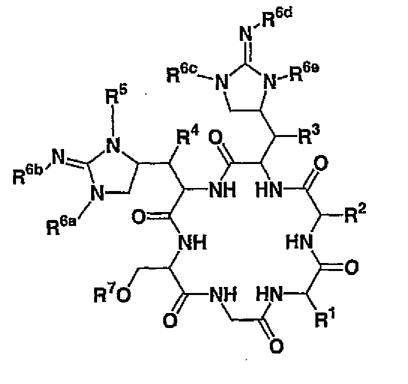 wherein:
R¹ is a moiety selected from: 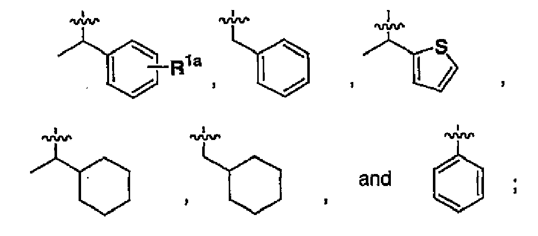
R^{1a} is H or halogen;
R² is a moiety selected from: 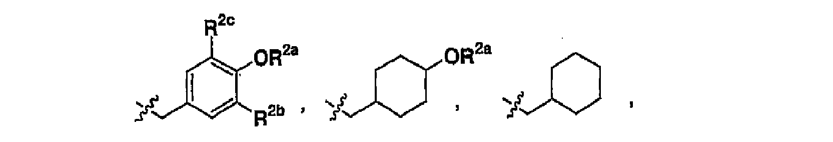 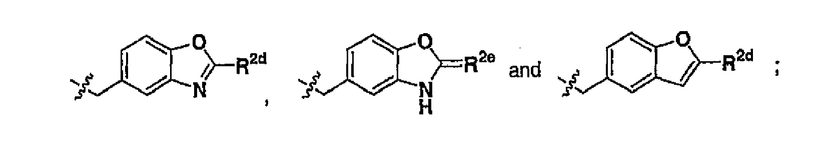 provided when R² is selected from the moieties: 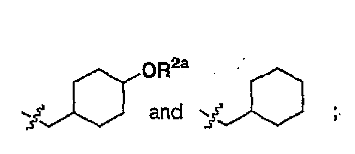 that R¹ is selected from the moieties: 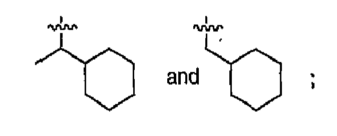
R^{2a} is selected from H, alkyl(C₁-C₂₀), cycloalkyl(C₃-C₂₀), alkenyl(C₃-C₂₀), alkynyl(C₃-C₂₀), the group -C(O)-Y-Z, and a moiety selected from: 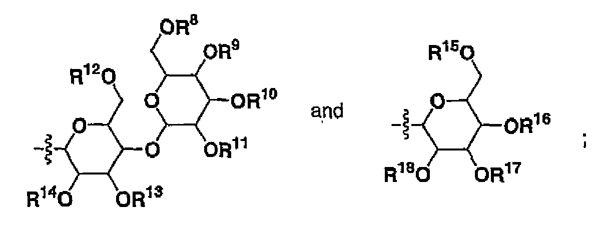
Y is selected from a single bond, -0- and -NR^{8a}-;
Z is selected from alkyl(C₁-C₂₀), cycloalkyl(C₃-C₂₀), alkenyl(C₃-C₂₀), alkynyl(C₃-C₂₀), aryl and heteroaryl;
and when Y is a single bond then Z is also selected from H, alkenyl(C₂-C₂₀) and alkynyl (C₂-C₂₀);
R^{2b} and R^{2c} are independently selected from H, halogen, alkyl(C₁-C₂₀), cycloakyl(C₃-C₂₀), alkenyl(C₂-C₂₀), alkynyl(C₂-C₂₀), aryl, heteroaryl, -NH₂, -NR^{2f}R^{2g}, and -NO₂, provided when R^{2b} is NO₂, that R^{2c} must be H;
R^{2d} is selected from alkyl (C₁-C₂₀), alkellyl(C₂-C₂₀), alkynyl(C₂-C₂₀), aryl, heteroaryl, and when R² is 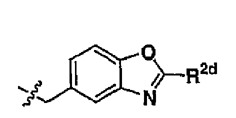
R^{2d} may also be the group L-M, wherein;
L is selected from -NH-, -S-, -SCH₂C(O)-, and a group of the formula: 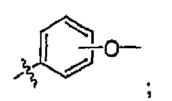
M is selected from alkyl(C₁-C₂₀), cycloalkyl(C₃-C₂₀), alkenyl(C₃-C₂₀) and alkynyl(C₃-C₂₀);
and when:
a) L is not -S-, M may also be aryl or heteroaryl;
b) L is -SCH₂C(O)-, M may also be H, alkenyl(C₂-C₂₀) or alkynyl(C₂-C₂₀); or
c) L is -NH- or a group of the formula: 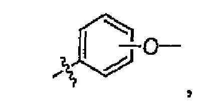
M may also be a moiety of the formula: 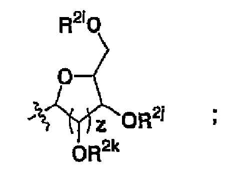
z is an integer of 1 or 2;
R²ⁱ, R^{2j} and R^{2k} are independently selected from H, alkyl(C₁-C₂₀), cycloalkyl(C₃-C₂₀), alkenyl(C₃-C₂₀), alkynyl(C₃-C₂₀), acyl, -Si(alkyl(C₁-C₂₀))₃, -Si(alkyl(C₁-C₂₀))₂(aryl), -Si(alkyl(C₁-C₂₀))(aryl)₂, -Si(aryl)₃, and aroyl;
R^{2e} is S or O;
R^{2f} is selected from H, alkyl(C₁-C₂₀), cycloalkyl(C₃-C₂₀), alkenyl(C₃-C₂₀), alkynyl(C₃-C₂₀), aryl and heteroaryl;
R^{2g} is H, alkyl(C₁-C₂₀), cycloalkyl(C₃-C₂₀), alkenyl(C₃-C₂₀), alkynyl(C₃-C₂₀), aryl and heteroaryl, and the group D-E-G, wherein;
D is selected from -C(O)-, -C(S)- and -S(O)₂-;
E is selected from a single bond, and, when D is -C(O)- or -C(S)-, E is also selected from -O- and -NR^{2h}-;
G is selected from alkyl(C₁-C₂₀), cycloalkyl(C₃-C₂₀), alkenyl(C₃-C₂₀), alkynyl(C₃-C₂₀), aryl and heteroaryl;
and when:
a) E is a single bond, then G may also be alkenyl(C₂-C₂₀) or alkynyl(C₂-C₂₀); or
b) D is -C(O)- or -C(S)- and E is a single bond, then G may also be H;
R^{2f} and R^{2g} may optionally when taken together with the nitrogen atom to which each is attached form a monocyclic ring having three to seven atoms independently selected from the elements C, N, 0 and S where said monocyclic ring optionally contains up to three nitrogen atoms, up to two oxygen atoms and up to two sulfur atoms provided said monocyclic ring does not contain -0-0-, -S-S- or -S-O- bonds;
R^{2h} is selected from H, alkyl(C₁-C₂₀), cycloalkyl(C₃-C₂₀), alkenyl(C₃-C₂₀), alkynyl(C₃-C₂₀), aryl and heteroaryl;
R^{2h} and G may optionally when taken together with the nitrogen atom to which each is attached form a monocyclic ring having three to seven atoms independently selected from the elements C, N, 0 and S where said monocyclic ring optionally contains up to three nitrogen atoms, up to two oxygen atoms and up to two sulfur atoms provided said monocyclic ring does not contain -0-0-, -S-S- or -S-O- bonds;
R³ and R⁴ are independently H, OH, -Si(alkyl(C₁-C₂₀))₃, -Si(alkyl(C₁-C₂₀))₂(aryl), -Si(alkyl(C₁-C₂₀))(aryl)₂, -Si(aryl)₃ or the group -C(O)-Y-Z;
R⁵ is selected from H, alkyl(C₁-C₂₀), cycloalkyl(C₃-C₂₀), alkenyl(C₃-C₂₀), alkynyl(C₃-C₂₀), the group -C(O)-Y-Z and moieties of the formulae; 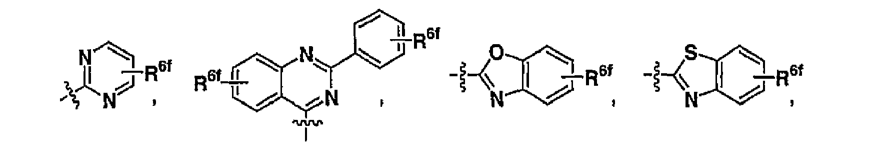 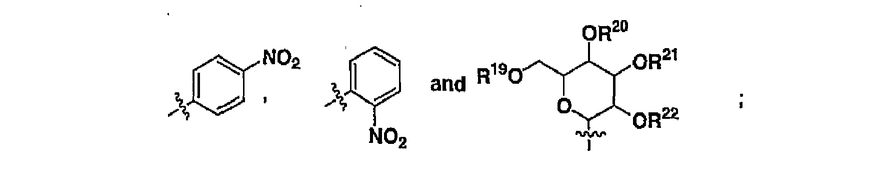
R^{6a}, R^{6b}, R^{6c}, R^{6d} and R^{6e} are independently selected from H, alkyl(C₁-C₂₀), cycloalkyl(C₃-C₂₀), alkenyl(C₃-C₂₀), alkynyl(C₃-C₂₀), the group -C(O)-Y-Z and moieties of the formulae; 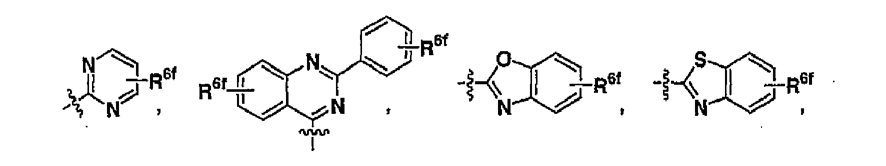 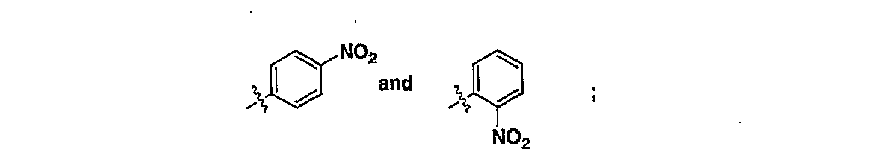
R^{6f} is selected from H, alkyl(C₁-C₂₀), cycloalkyl(C₃-C₂₀), alkenyl(C₃-C₂₀), alkynyl(C₃-C₂₀), aryl, heteroaryl, halogen, hydroxy, alkoxy(C₁-C₂₀), aryloxy, amino, monoalkyl(C₁-C₂₀)amino, dialkyl(C₁-C₂₀)amino, carboxy, carboxyalkyl(C₁-C₂₀), carboxyaryl and carboxyamido;
R⁷ is selected from H, alkyl(C₁-C₂₀), cycloalkyl(C₃-C₂₀), alkenyl(C₃-C₂₀), alkynyl(C₃-C₂₀), -Si(alkyl(C₁-C₂₀))₃, -Si(alkyl(C₁-C₂₀))₂(aryl), -Si(alkyl(C₁-C₂₀))(aryl)₂, -Si(aryl)₃ and the group -C(O)-Y-Z;
R⁸, R⁹, R¹⁰, R¹¹, R¹², R¹³, R¹⁴, R¹⁵, R¹⁶, R¹⁷, R¹⁸, R¹⁹, R²⁰, R²¹ and R²² are independently selected from H, alkyl(C₁-C₂₀), cycloalkyl(C₃-C₂₀), alkenyl(C₃-C₂₀), alkynyl(C₃-C₂₀), -Si(alkyl(C₁-C₂₀))₃, -Si(alkyl(C₁-C₂₀))₂(aryl), -Si(alkyl(C₁-C₂₀))(aryl)₂, -Si(aryl)₃ and the group -C(O)-Y-Z or, optionally,
R⁸ and R⁹, R⁹ and R¹⁰, R¹⁰ and R¹¹, R¹³ and R¹⁴, R¹⁵ and R¹⁶, R¹⁶ and R¹⁷, R¹⁷ and R¹⁸, R¹⁹ and R²⁰, R²⁰ and R²¹ or R²¹ and R²² may independently be taken together forming moieties of the formulae: 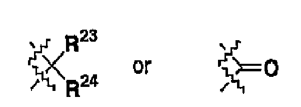 provided when;
a) R⁸ and R⁹ are so joined, R⁹ and R¹⁰ may not be so joined;
b) R⁹ and R¹⁰ are so joined, R⁸ and R⁹ and R¹⁰ and R¹¹ may not be so joined;
c) R¹⁵ and R¹⁶ are so joined, R¹⁶ and R¹⁷ may not be so joined;
d) R¹⁶ and R¹⁷ are so joined, R¹⁵ and R¹⁶, R¹⁷ and R¹⁸ may not be so joined;
e) R¹⁹ and R²⁰ are so joined, R²⁰ and R²¹ may not be so joined;
f) R²⁰ and R²¹ are so joined, R¹⁹ and R²⁰, and R²¹ and R²² may not be so joined;
R^{8a} is selected from H, alkyl(C₁-C₂₀), cycloalkyl(C₃-C₂₀), alkenyl(C₃-C₂₀), alkynyl(C₃-C₂₀), aryl and heteroaryl;
R^{8a} and Z may optionally when taken together with the nitrogen atom to which each is attached form a monocyclic ring having three to seven atoms independently selected from the elements C, N, O and S where said monocyclic ring optionally contains up to three nitrogen atoms, up to two oxygen atoms and up to two sulfur atoms provided said monocyclic ring does not contain -O-O-, -S-S- or -S-O- bonds;
R²³ and R²⁴ are independently selected from H, alkyl(C₁-C₂₀), cycloalkyl(C₃-C₂₀), alkenyl(C₂-C₂₀), alkynyl(C₂-C₂₀), aryl, and heteroaryl;
R²³ and R²⁴ may optionally when taken together with the carbon atom to which each is attached form carbocyclic, monocyclic, fused, bridged, spirocyclic or polycyclic rings from three to twenty ring atoms optionally selected from the elements C, N, 0 and S where said monocyclic ring optionally contains up to three nitrogen atoms, up to two oxygen atoms and up to two sulfur atoms provided said monocyclic ring does not contain -0-0-, -S-S- or -S-O- bonds;
provided when:
R¹ is 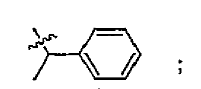
R³ and R⁴ are -OH;
R⁵ is 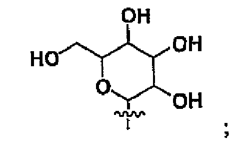 and
R^{2b}, R^{2c}, R^{6a}, R^{6b}, R^{6c}, R^{6d}, R^{6e}, R⁷, are H;
that:
R^{2a} is not H or moieties selected from the formulae: 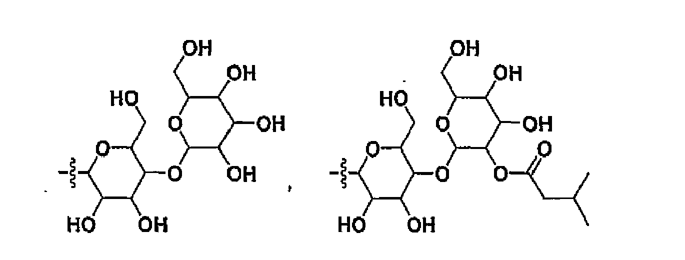 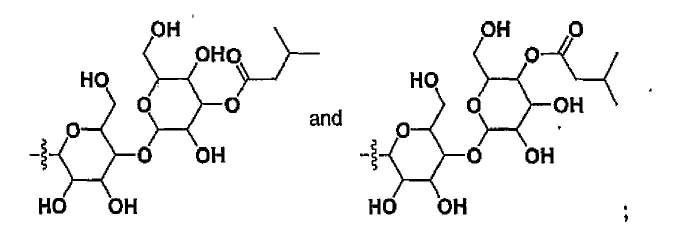 or a pharmaceutically acceptable salt thereof.
